# EUROPEAN PATENT SPECIFICATION

(11) **EP 2 590 996 B1**
(45) Date of publication and mention of the grant of the patent: **25.05.2016**
(21) Application number: 11767621.3
(22) Date of filing: 06.07.2011
(51) Int. Cl.: C12N 15/82, C07K 14/415, A01H 5/10

(54) **GLUCOSINOLATE TRANSPORTER PROTEIN AND USES THEREOF**
GLUCOSINOLAT-TRANSPORTERPROTEIN UND VERWENDUNGEN DAVON
PROTÉINE TRANSPORTRICE DE GLUCOSINOLATE ET SES UTILISATIONS

(30) Priority: 08.07.2010 US 362390 P; 12.07.2010 EP 10075299
(43) Date of publication of application: 15.05.2013
(73) Proprietor: Bayer CropScience NV, 1831 Diegem (BE); University of Copenhagen, 1017 Copenhagen K (DK)
(72) Inventor: DENOLF, Peter, B-9620 Velzeke (BE); OPSOMER, Christiane, B-8620 Nieuwpoort (BE); HALKIER, Barbara, Ann, DK-1366 Copenhagen K. (DK); NOUR-ELDIN AUIS, Hussam, Hassan, DK-1319 Copenhagen K. (DK); ANDERSEN, Tonni, Grube, DK-2000 Frederiksberg (DK); MADSEN, Svend, Roesen, DK-2100 Copenhagen Ø (DK); JØRGENSEN, Morten, Egevang, DK-2300 Copenhagen S (DK)
(86) International application number: PCT/EP2011/004565
(87) International publication number: WO 2012/004013

(56) References cited:
- WO-A2-2008/023263
- NOUR-ELDIN HUSSAM HASSAN ET AL: "Piecing together the transport pathway of aliphatic glucosinolates", PHYTOCHEMISTRY REVIEWS, vol. 8, no. 1, January 2009 (2009-01), pages 53-67, XP002667356, ISSN: 1568-7767
- NOUR-ELDIN HUSSAM H ET AL: "Screening for plant transporter function by expressing a normalized Arabidopsis full-length cDNA library in Xenopus oocytes", PLANT METHODS, BIOMED CENTRAL, LONDON, GB, vol. 2, no. 1, 27 October 2006 (2006-10-27), page 17, XP021025936, ISSN: 1746-4811, DOI: 10.1186/1746-4811-2-17 cited in the application
- DATABASE Geneseq [Online] 7 August 2008 (2008-08-07), "Arabidopsis thaliana amino acid sequence SEQ ID 36913.", XP002667357, retrieved from EBI accession no. GSP:ARM37541 Database accession no. ARM37541 & US 2007/214517 A1 (ALEXANDROV NICKOLAI [US] ET AL) 13 September 2007 (2007-09-13)
- CHEN S ET AL: "UPDATE ON GLUCOSINOLATE METABOLISM AND TRANSPORT", PLANT PHYSIOLOGY AND BIOCHEMISTRY, GAUTHIER-VILLARS, PARIS, FR, vol. 39, no. 9, 1 September 2001 (2001-09-01), pages 743-758, XP001085172, ISSN: 0981-9428, DOI: 10.1016/S0981-9428(01)01301-8
- ELLERBROCK BRYAN LJ ET AL: "Contribution of glucosinolate transport to Arabidopsis defense responses.", PLANT SIGNALING & BEHAVIOR JUL 2007 LNKD- PUBMED:19704682, vol. 2, no. 4, July 2007 (2007-07), pages 282-283, XP002667358, ISSN: 1559-2324

## Description

### Field of the invention

The present invention relates to the field of agricultural products, especially crop plants and parts thereof having a modified glucosinolate content. Provided are methods to alter the glucosinolate (GSL) content in plants, in particular in specific plant parts, by reducing glucosinolate transporter protein (GTR) activity in plants or parts thereof. Such modification of GTR activity may be achieved through down-regulation of GTR gene expression or GTR protein activity. In particular, methods are provided to decrease GSL content of plant seed and meal thereof, as well as methods to increase GSL content in green plant tissue, of *Brassicales* plants, particularly of *Brassicaceae* plants such as oilseed forms of *Brassica* spp. (including e.g. *B*. *napus, B. juncea* and *B*. *carinata*), *B. oleracea, B. rapa,* cruciferous salads (including e.g. *Eruca sativa* and *Diplotaxis tenuifolia)* and *Raphanus* (including e.g. *Raphanus sativa*).

### Background of the invention

The *Brassicales* or *Capparales* order of plants, including the *Brassicaceae* or *Cruciferae* family, includes many cultivars that have provided mankind with a source of condiments, vegetables, forage crops, and the economically important crops rapeseed *(Brassica napus* and *Brassica campestris* or *rapa*) and mustard *(Brassica juncea*).

A striking and characteristic chemical property of these plants is their high content of glucosinolates, amino acid-derived natural plant products containing a thioglucose and a sulfonated oxime. These sulphur-containing secondary metabolites, although considered non-toxic per se, are important because of the multiplicity of physiologically active products, such as nitriles, epithionitriles, oxazolidine-2-thiones, thiocyanates and isothiocyanates, derived from them upon cleavage by the hydrolytic enzyme myrosinase (thioglucoside glucohydrolase; EC 3.2.3.1) upon plant damage (Halkier and Gershenzon, 2006, Annual Review of Plant Biology 57: 303-333).

For plants, the glucosinolate/myrosinase system protects against herbivore attacks, and is implicated in host-plant recognition by specialized predators. For humans, glucosinolates (or rather their hydrolysis products) have received increased attention as cancer-preventive agents, flavor compounds, and potential biopesticides.

Glucosinolates are present in all parts of the plant. The level of glucosinolates varies in different tissues at different developmental stages (Fieldsend and Milford, 1994, Ann. Appl. Biol. 124: 531-542; Porter et al., 1991, Ann. Appl. Biol. 118: 461-467) and is affected by external factors such as growth conditions (Zhao et al., 1993, J. Sci. Food Agric. 63: 29-37; Zhao et al., 1994, J. Sci. Food Agric. 64, 295-304), wounding (Bodnaryk, 1992, Phytochemistry 31: 2671-2677), fungal infection (Doughty et al., 1991, Ann. Appl. Biol. 118: 469-477), actual and simulated insect damage (Bodnaryk, 1992, Phytochemistry 31: 2671-2677; Koritsas et al., 1991, Ann. Appl. Biol. 118: 209-221), and other forms of stress (Mithen, 1992, Euphytica 63: 71-83). Consistent with a prominent function in plant defense, the highest glucosinolate concentrations are found in reproductive organs, including seeds, siliques, flowers and developing inflorescences, followed by young leaves, the root system and fully expanded leaves.

Glucosinolates are known to be transported in the plant from maternal tissue across several apoplastic barriers from leaves and siliques via the phloem and into embryos, where they accumulate to high levels (reviewed in Nour-Eldin and Halkier, 2009, Phytochem Rev 8: 53-67). The molecular mechanism for this transport is unknown. The transport properties of glucosinolates within plants are of interest as identification of the mechanism of transport could, for example, lead to lower levels being obtained in seeds and seed meal thereof.

Methods of plant transporter discovery are described based on homology and yeast functional complementation. However, these methods represent a limitation when identifying transporters with no known homologues or when no auxotrophic yeast strains can be engineered. Nour-Eldin (Ph. D. thesis, University of Copenhagen, Faculty of Life Sciences, Department of Plant Biology, Plant Biochemistry Laboratory, 2007, 72 p.) describes a method of plant transporter discovery based on a functional genomics approach. A normalized library of *Arabidopsis* secondary metabolite transporters was constructed and screened in a high-throughput manner in *Xenopus* oocytes. The transporter library was screened for uptake towards the aliphatic glucosinolate 4-methylthiobytyl (4-MTB) glucosinolate. Three *Arabidopsis* glucosinolate transporter genes were identified. Two of them (At3g47960 and At1g18880; hereinafter referred to as ATGTR1 and 3) belong to the nitrate/peptide transporter family, while the third gene (At1g71880; known as AtSUC1) belongs to the sucrose transporter family. The *Arabidopsis* sucrose transporter family contains 9 genes (Williams et al., 2000, Trends Plant Sci 5: 283-290), which have been shown to be broad specific and transport a variety of β-glucosides (Chandran et al., 2003, J Biol Chem 278: 44320-44325). AtSUC1, 2, 5, 8 and 9 were shown to transport 4-MTB in *Xenopus* oocytes at varying efficiencies (Nour-Eldin, 2007, *supra*). Seeds of AtSUC9 T-DNA knockout lines showed a significant reduction in four types of glucosinolates, whereas AtSUC1 knockout lines showed no difference in seed glucosinolate content (Andersen et al., 2nd Conference on Glucosinolates, May 24-27, 2009). Two additional AtGTR genes (At5g62680 and At1g69870; hereinafter referred to as AtGTR2 and AtGTR5) were identified based on homology and subsequently also shown to transport 4-MTB into *Xenopus* oocytes (Nour-Eldin, 2007, *supra*).

Among the oilseed crops currently dominating the world market, rapeseed stands out for two important reasons; its high levels of oil with excellent nutritional properties for humans, and a protein-rich seedcake meal, ideal for animal feed. Interest in reducing the levels of glucosinolates in seed results from the presence of bitter-tasting, toxic and goitrogenic degradation products which limit the incorporation of rape meal into non-ruminant animal feed (Thomson and Hughes, 1986, In Oilseed rape. Edited by Scarisbrick and Daniels. Collins, London, U.K. pp. 32-82).

Approaches to reduce glucosinolate levels in seed have thus far been through blocking biosynthetic pathways. Often, however, this approach is accompanied by adverse effects on plant fitness due to e.g. increased susceptibility to biotic or abiotic stresses.

In the 1970s, traditional breeding generated a multiple-loci-dependent *B. napus* cultivar with reduced glucosinolate content in all parts of the plant, including the seeds. This "00" ("double low") variety and its descendants have subsequently become the most widely grown rapeseed cultivars across the northern hemisphere. The prolonged selection bottleneck caused by this single source has, however, created a limited genetic diversity for future *B. napus* breeding programs and limits interspecific hybridization. This poses a serious problem for *B. napus* breeders striving to improve yield and disease resistance as well as to introduce novel traits such as drought tolerance through interspecific hybridizations.

A further problem with "00" varieties is that the seeds, although low in glucosinolates, are not free of them. Pressed seed cake obtained from "00" varieties after the oil has been extracted will typically contain less than 18-24 micromoles of total glucosinolates (GSL) per gram of dry weight (as compared to traditional rapeseed meal that contains 120-150 µmol of total GSL per gram). For use in compound feed, palatability to ruminants sets the level of total GSL permitted at no more than 10-15 micromoles per gram of dry weight, meaning an animal feed could in theory be compounded almost entirely of "00" pressed seed cake if the seed cake is at the lower level of GSL content. However, it has recently been found that poultry and pigs are both much more sensitive to levels of GSLs than ruminants, and more than 2-4 micromoles GSL per gram of dry weight in the feed can severely affect reproductive efficiencies in these animals. A truly "zero GSL" variety would be of significant commercial advantage to animal feed compounders, producers of pressed seed cake and the growers by increasing quantities of seed cake that can be included in compound feeds and removing the need for continual monitoring of GSL levels in their products.

These and other problems are solved as hereinafter described in the different embodiments, examples and claims.

### Summary of the invention

The present invention relates to plants and plant parts, such as seed, seed meal, green plant tissue and root tissues, with modified total glucosinolate (GSL) content, and relates to methods to produce plants and plant parts with modified total GSL content by modification of functional glucosinolate transport protein (GTR) activity. In particular, methods are provided to produce seed with decreased total GSL content. The methods are also suitable to produce green plant tissue, such as leaf tissue, with increased total GSL content by reduction of functional GTR activity. Further provided is the use of GTR-encoding nucleic acid molecules to obtain modified GSL content in plants and plant parts. Plants and plant parts with modified functional GTR activity are suitable for use, for example, in cancer-prevention, in pest management or in animal feeding.

In a first aspect of the invention reduction of functional GTR activity may be achieved through down-regulation of GTR gene expression. In one embodiment of the invention, a method is provided to modify total GSL content of plants and plant parts by introduction of an RNA molecule capable of down-regulating *GTR* gene expression, e.g. through introduction of a chimeric nucleic acid construct comprising a nucleotide region which upon expression yields such RNA molecule.

*GTR* gene expression can be down-regulated by introducing an RNA molecule comprising part of a GTR-encoding nucleotide sequence or a homologous sequence or by introducing a chimeric DNA encoding such RNA molecule. *GTR* gene expression can also be down-regulated by introducing an antisense RNA molecule comprising a nucleotide sequence complementary to at least part of a GTR-encoding nucleotide or homologous sequence, or by introducing a chimeric DNA encoding such RNA molecule. *GTR* gene expression can also be down-regulated by introducing a double-stranded RNA molecule comprising a sense and an antisense RNA region corresponding to and respectively complementary to at least part of a GTR gene sequence, which sense and antisense RNA region are capable of forming a double stranded RNA region with each other. Further, *GTR* gene expression can be down-regulated by introduction of a microRNA molecule (which may be processed from a pre-microRNA molecule) capable of guiding the cleavage of GTR mRNA. Again, microRNA molecules may be conveniently introduced into plant cells through expression from a chimeric DNA molecule encoding such miRNA, pre-miRNA or primary miRNA transcript.

In another embodiment of the invention, a method is provided to modify total GSL content of plants or plant parts by down-regulation of *GTR* gene expression through alteration of the nucleotide sequence of the endogenous *GTR* gene by generating a mutant *gtr* allele comprising one or more mutations in its nucleic acid sequence, whereby the mutations result in a significantly reduced amount of functional GTR protein in the cell *in vivo,* such as e.g. alterations in regulatory signals including promoter sequence, intron processing signals, untranslated leader and trailer sequence or polyadenylation signal sequences.

Reduction of functional GTR activity may occur at the level of the GTR activity. A suitable method to modify total GSL content of plants and plant parts is through introduction of a chimeric nucleic acid construct encoding a protein capable of down-regulating GTR protein activity. GTR protein activity may be down-regulated by expression of a dominant negative *GTR* gene. Further, GTR protein activity may be down-regulated by expression of a GTR-inactivating antibody. Functional GTR activity may also be modulated by changing the phosphorylation/deposphorylation status of GTR proteins. This can be achieved by using variant alleles of GTR encoding genes, whereby the phosphorylation sites are modified, as hereinafter described.

In another embodiment of the invention, a method is provided to modify total GSL content of plants or plant parts by down-regulation of GTR protein activity through alteration of the nucleotide sequence of the endogenous *GTR* gene by generating a mutant *gtr* allele comprising one or more mutations in its nucleic acid sequence, whereby the mutations result in a significantly reduced amount of functional GTR protein in the cell *in vivo,* e.g. through alterations in the coding region introducing insertions, deletions or substitutions of amino acids, truncations of the encoded protein or splice site mutations.

### Brief description of the figures

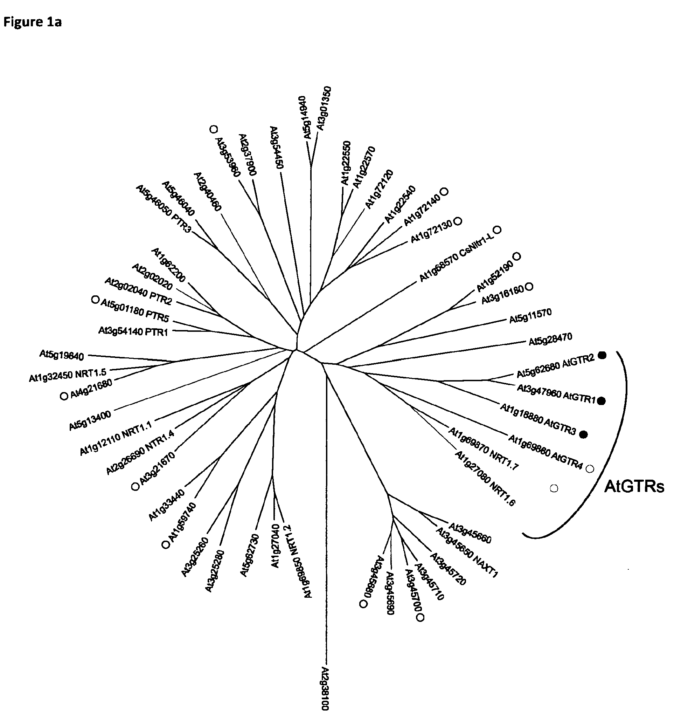

**Figure 1a**: Unrooted phylogenetic tree of protein sequences in the *Arabidopsis* NRT1/PTR family. Protein sequences were retrieved from the ARAMEMNON plant membrane protein database (Schwacke et al., Plant Physiol. 131: 16-26). The At3g47960 (AtGTR1) protein subclade is marked with a bracket. Grey shading of circles reflects relative in vitro GSL uptake activity in *Xenopus* oocytes with white indicating zero uptake and black maximum uptake observed. Unmarked genes have not been tested. The phylogenetic relationship was inferred using the Neighbor-Joining method (Saitou and Nei, 1987, Mol Biol Evol. 4(4):406-25). The phylogenetic relationship was computed using the Poisson correction method (Zuckerkandl and Pauling, 1965, In Bryson V, Vogel HJ (eds), Academic Press, New York, pp 97-166). All positions containing gaps and missing data were eliminated from the dataset (Complete deletion option). There were a total of 300 positions in the final dataset. Phylogenetic analyses were conducted in MEGA4 (Tamura et al., 2007, Mol Biol Evol 24: 1596-1599).

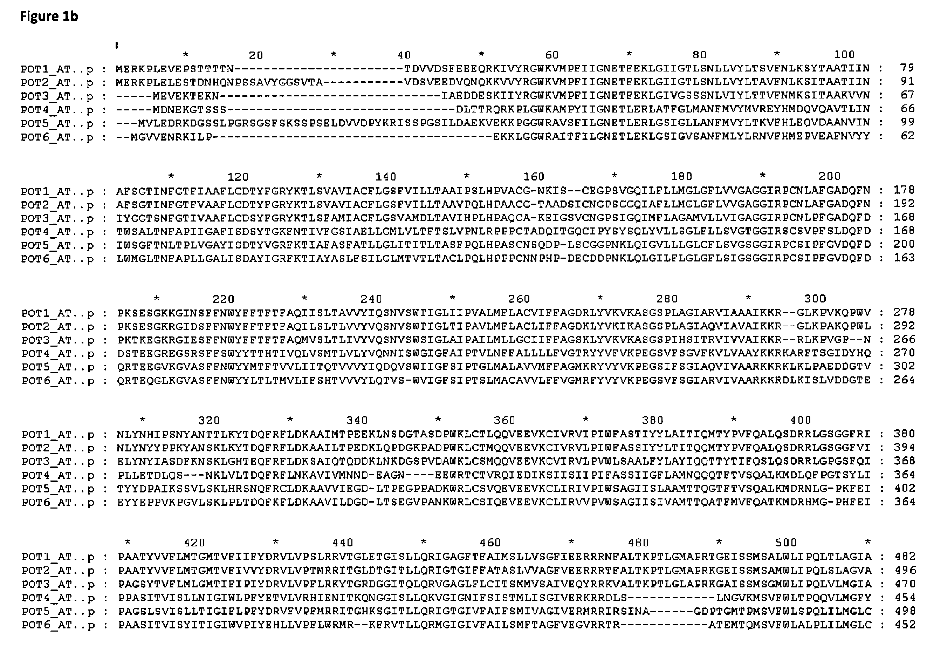

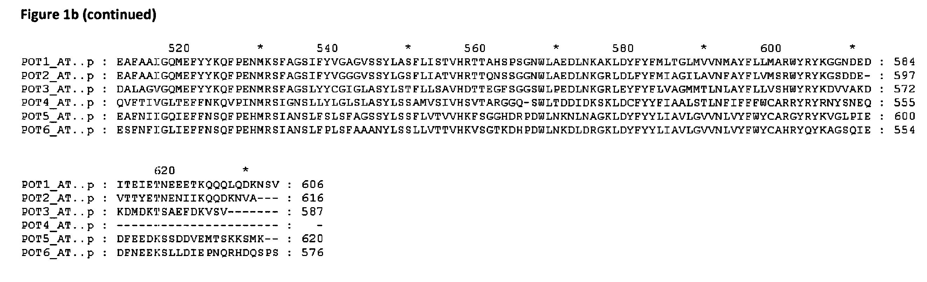

**Figure 1b****:** Alignment of *Arabidopsis* GTR amino acid sequences. POT1-6_AT..p: AtGTR1-6 (SEQ ID Nos 2, 4, 6, 8, 10 and 12)

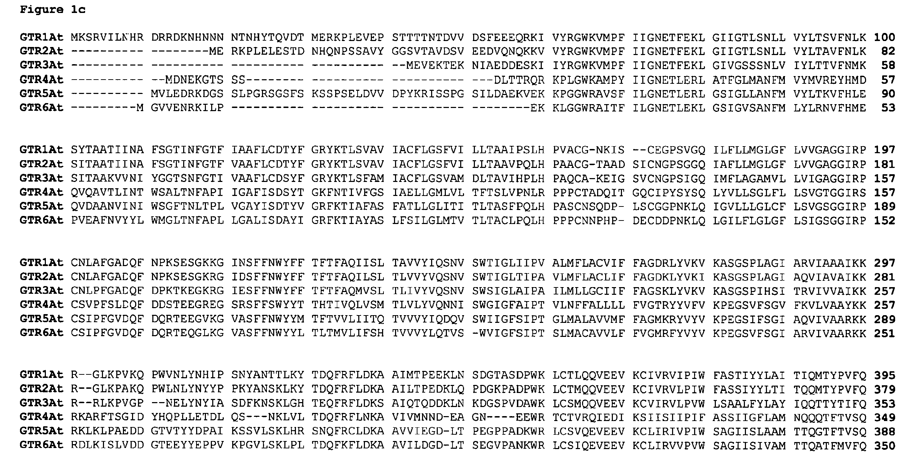

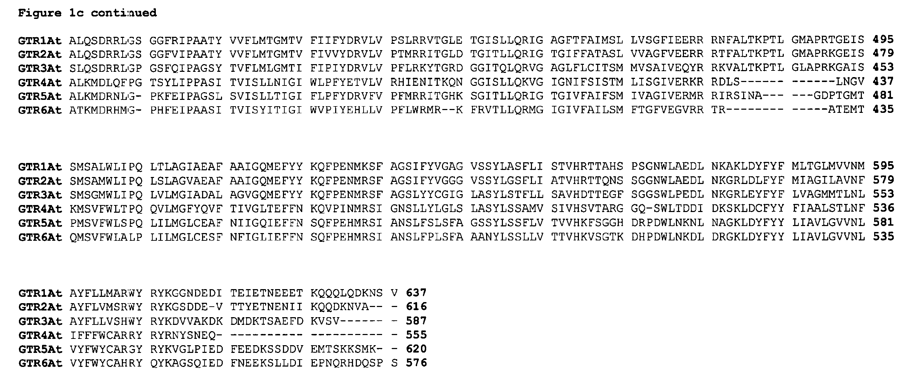

**Figure 1c****:** Alignment of *Arabidopsis* GTR amino acid sequences 1 to 6 including the N-terminal extension of 30 amino acids in GTR1 (SEQ ID Nos 142, 4, 6, 8, 10 and 12).

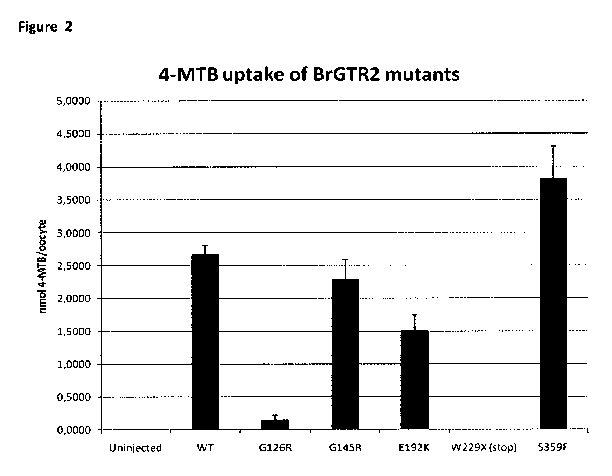

**Figure 2****:** 4-MTB uptake in *Xenopus* oocytes expressing BrGTR2-A2 proteins with substitutions of amino acids corresponding to the amino acid at position 126 (Gly to Arg), 145 (Gly to Arg), 192 (Glu to Lys), 229 (Trp to STOP) and 359 (Ser to Phe) of SEQ ID NO: 66 and wildtype BrGTR2-A2 protein. Assay conditions: 0.5mM 4-MTB for one hour. Each bar is the mean of 4 replicates, error is standard deviation.

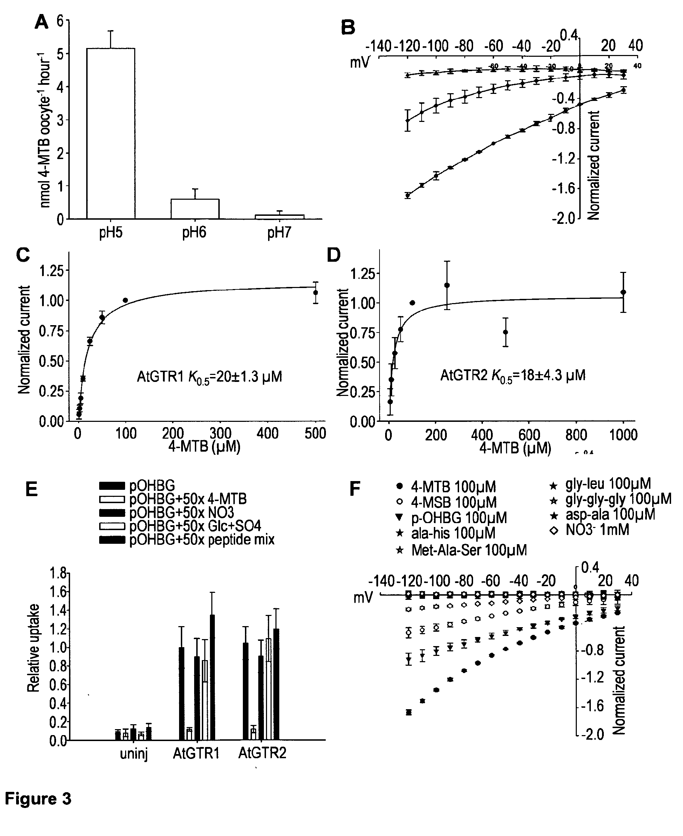

**Figure 3****:** Biochemical analysis of AtGTR1 and 2 mediated GSL transport in *Xenopus* oocytes. A and B, pH dependence of AtGTR-mediated GSL transport. A, AtGTR2 mediated 4-MTB uptake measured by LC-MS analysis of oocyte extracts, n=3 oocytes. B, AtGTR1 currents induced by 1 mM 4-MTB were measured at different membrane potentials at pH 5 (●), pH 6(◆), and pH 7 (▲). Currents were normalized to the current induced at pH 5 at -60mV, error bars are SD, n=4 oocytes. C and D, kinetic analysis of AtGTR-mediated glucosinolate transport. Normalized glucosinolate dependent currents at a membrane potential of -60mV are plotted against 4-MTB concentrations, line indicates a fit of the Michaelis-Menten equation to the data; error bars are SD. For AtGTR1 (n=6 oocytes); For AtGTR2 (n=5 oocytes). Each oocyte-dataset were normalized to current elicited at 100µM 4-MTB. E and F, substrate specificity of AtGTRs. E, AtGTR1 and AtGTR2 uptake of 0,04mM C-14 labled pOHBG at pH 5 in the presence of 2mM 4-MTB, 2 mM NO₃⁻, 2mM glucose + 2 mM SO₄⁻⁻, or a solution of 2 mM Ala-His + 2 mM Asp-Ala + 2 mM Gly-Leu + 2 mM Ala-Phe + 2 mM Gly-Glu + 2 mM Gly-Gly-Gly, error bars are SD, n=8 oocytes. F, AtGTR1 currents induced by endogenous and exogenous giucosinolates, representative positive, negative and neutral dipeptides and two tripeptides Currents were normalized to the current induced by 100µM 4-MTB at pH 5 clamped at -60mV, error bars are SD, n=4 oocytes.

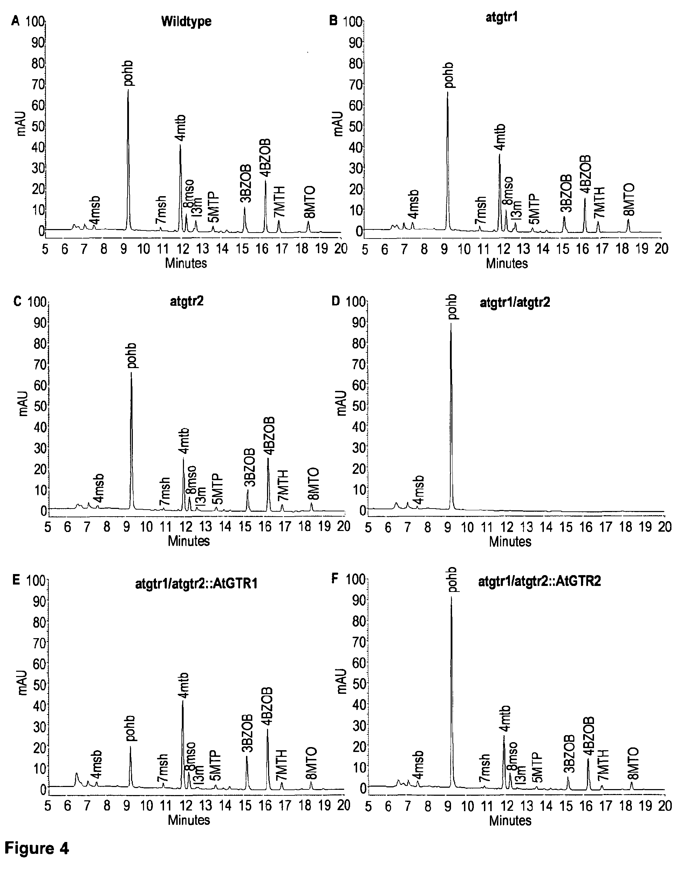

**Figure 4****:** Glucosinolate content in seeds from *Arabidopsis* wild type and GTR knockout plants. A-D, HPLC trace showing GSL content in total seeds from a representative silique from wildtype, atgtr1, atgtr2 and atgtr1/atgtr2 plants, respectively. A, 54 seeds from wildtype plants. B, 51 seeds from atgtr2. C, 53 seeds from atgtr1. D, 39 seeds from atgtr1/atgtr2 plants. E, 20 seeds from atgtr1/atgtr2 plant transformed with native AtGTR2 complementation construct. Abbreviations denote the prefix of detected glucosinolates. 4ohb: 4-hydroxybutyl-GSL, 4msb: 4-methylsulfinylbutyl-GSL, 5msp: 5-methyldsulfinylpentyl-GSL, 6msh: 6 methylsulfinylhexyl-GSL, 7msh: 7-methylsulfinylheptyl-GSL, 4mtb: 4-methylthiobutyl-GSL, 8mso: 8-methylsulfinyl-GSL, i3m: indol-3-yl-methyl-GSL, 5mtp: 5-methylthiobutyl-GSL, 3bzop: 3-benzoylpropyl-GSL, 4bzob: 4-benzoylbutyl-GSL, 7mth: 7-methylthioheptyl-GSL, 8-methylthiooctyl-GSL. 2-propenyl-GSL: internal standard GSL, normalizing for loss during purification.

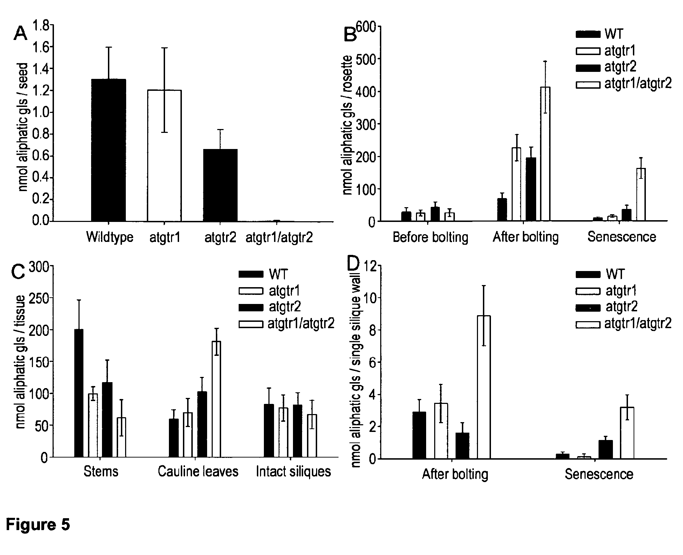

**Figure 5****:** Total aliphatic glucosinolate (abbreviated as "gls" on the Y-axis) content in different plant parts from soil grown *Arabidopsis* plants through development. Harvesting time points: before bolting : 3 week old plants, after bolting (and onset of silique development): 5 week old plants, senescence (and seed maturity): 8 week. For time points "after bolting" and "senescence" tissues are harvested from the same plants. A, seeds. B, rosette. C, Stems (including flowers), total cauline leaves, total intact siliques (including developing and mature seeds). D, Single silique walls. Bars represent mean ± SD. (Total aliphatic GSLs include all methionine-derived GSLs including benzoyloxy-GSLs).

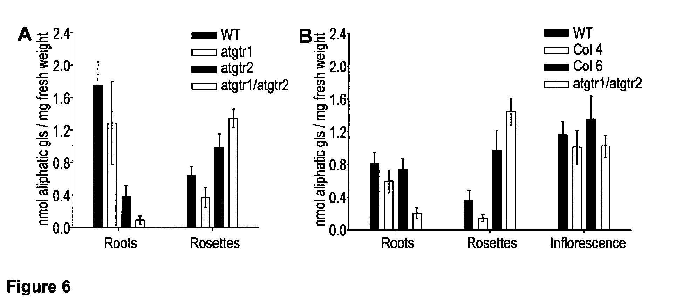

**Figure 6****:** Total aliphatic glucosinolate (abbreviated as "gls" on the Y-axis) content in different plant parts from hydroponically grown *Arabidopsis* plants through development. Harvesting time points as stated for soil grown plants in Figure 2. A, roots. B, rosette bolting. C, aliphatic glucosinolate concentration per mg plant.

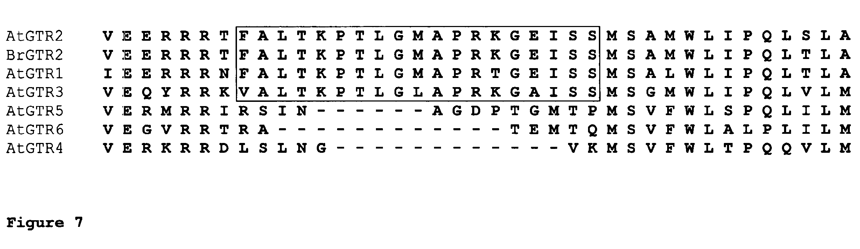

**Figure 7****:** Detail of alignment of part of Arabidopsis GTR amino acid sequences: AtGTR2: SEQ ID NO: 4 from amino acid position 454 to amino acid position 493; BrGTR2: SEQ ID NO: 26 from amino acid position 450 to amino acid position 489; AtGTR1: SEQ ID NO: 2 from amino acid position 440 to amino acid position 479 (or SEQ ID No 142 from amino acid position 470 to 509) ; AtGTR3: SEQ ID NO: 6 from amino acid position 438 to amino acid position 467; AtGTR5: SEQ ID NO: 10 from amino acid position 463 to amino acid position 495; AtGTR6: SEQ ID NO: 12 from amino acid position 423 to amino acid position 449; and AtGTR4: SEQ ID NO: 8 from amino acid position 425 to amino acid position 451.

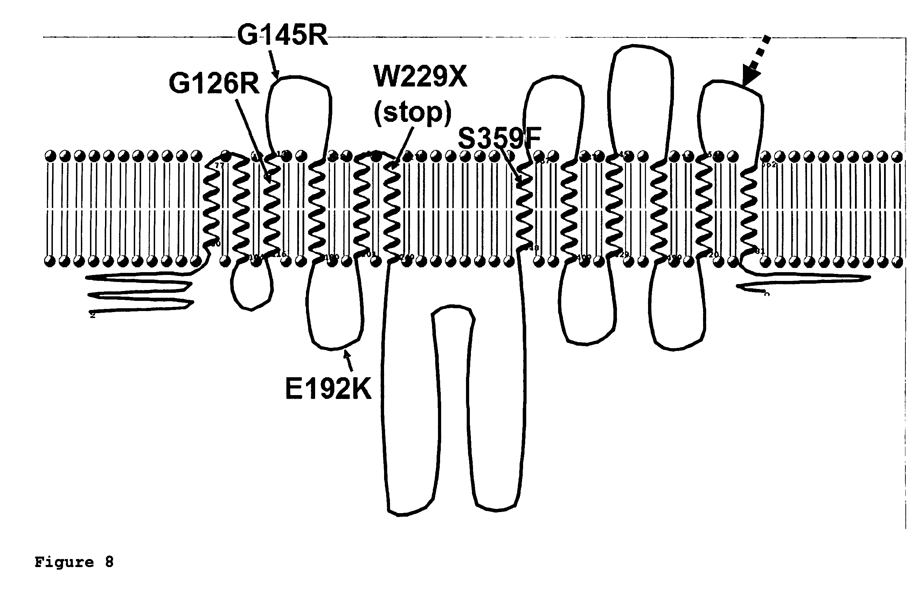

**Figure 8****:** Predicted protein structure for GTR2 protein with relative indication of the mutations in the mutant BrGTR2 protein. The dashed arrow indicates a loop characteristic for GTR1, GTR2 and GTR3 proteins (also indicated by the boxed sequences in Figure 7.

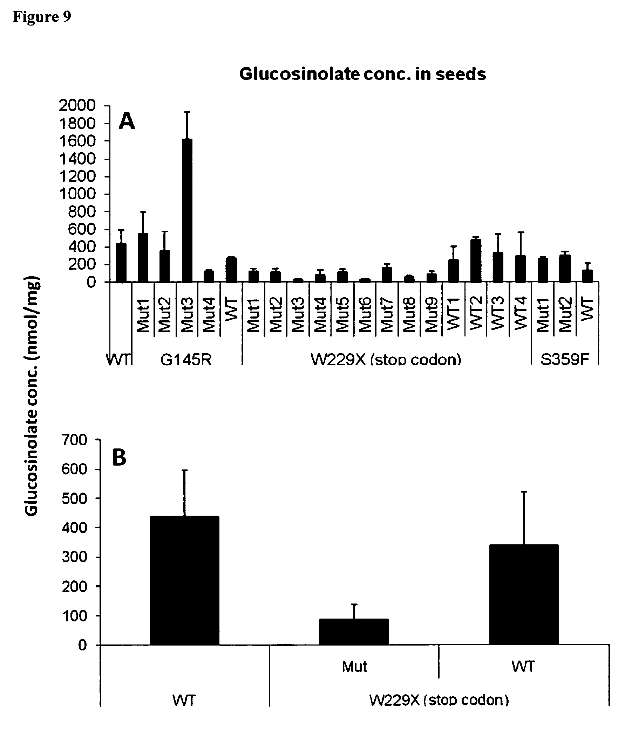

**Figure 9****:** 3-butenyl content in B. rapa seeds. The X-axis shows the BrGTR2-genotype of seeds; darker columns indicate seeds from homozygous mutants, whereas lighter columns indicate seeds from wild type segregants (WTS) for the respective mutations. WT represent unmutated reference wild type seeds. The Y-axis shows 3-butenyl concentration in nmol/mg. Panel A) Glucosinolate concentration of seeds from each plant; standard deviations are calculated from n=3 seeds. Panel B) Average glucosinolate concentration of all "W229X-" (stop codon) mutants, with standard deviations calculated from n=27 seeds, n=12 seeds and n=3seeds, respectively.

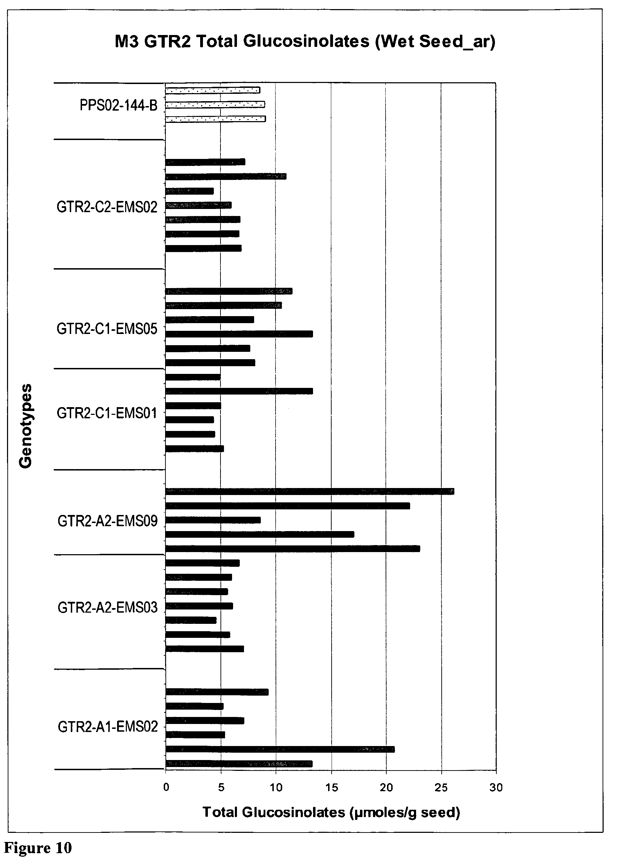

**Figure 10****:** Total glucosinolate content in mutant and reference B. napus seeds. The Y axis shows the genotype of the seeds. Reference are unmutated isogenic wild-type seeds.

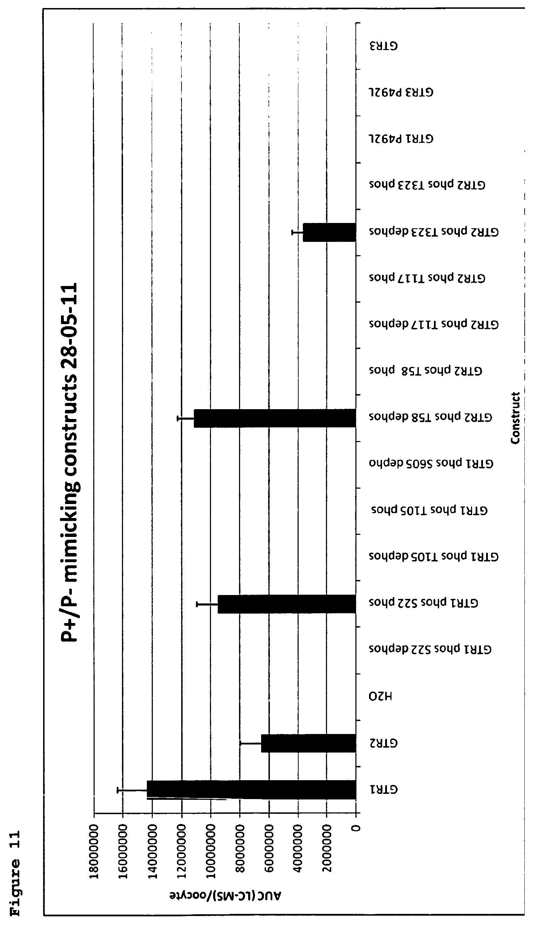

**Figure 11****:** 4-MTB uptake of phosphorylation/dephosphorylation mimicking constructs in comparison with wild type At GTR1 and GTR2; X-axis represents the genotypes of the different constructs.

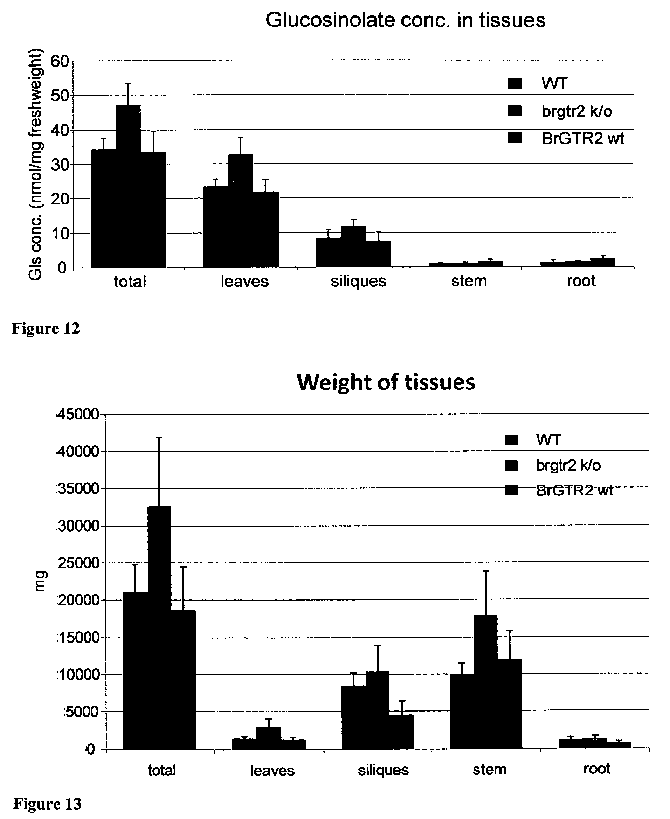

**Figure 12****:** Concentration of glucosinolates in various tissues in Brassica rapa. The X-axis shows plant part analyzed and the Y-axis shows nmol glucosinolate/mg freshweight. The analysis shows that glucosinolate concentration in entire *Brgtr2* plants is higher than in WTs and that this increased concentration are caused by the glucosinolate levels in leaves and siliques, not stem and root. Standard deviations are calculated from n=5.
**Figure 13****:** Freshweight of various tissues. The X-axis shows plant part weighed and the Y-axis shows mg freshweight. The analysis shows that *Brgtr2* plants are larger than the WTs, and that this increase is primarily caused by the weights of leaves and stem. It should be noted that the low weight of BrGTR2 WT siliques is partly caused by a low amount of seeds in these compared to true WT and *Brgtr2* siliques (data not shown). Standard deviations are calculated from n=5.

### General definitions

"Glucosinolates" (abbreviated herein as "GSLs" or "GLSs"), as used herein, refers to amino acid-derived thioglucosidic organic anions comprising a sulfonated aldoxime moiety. A variable side chain depending on the parent amino acid and further side chain modifications gives the distinct chemical and biological properties for GSLs. Approximately 120 different GSLs have been described in the literature and they are all derived from only 8 different amino acids. The parent amino acids are conveniently used as a classification criteria. GSLs derived from Ala, Leu, Ile, Val and Met are called "aliphatic GSLs", those derived from Tyr and Phe are called "aromatic GSLs" and those derived from Trp are called "indole GSLs". The great variety in GSL types is caused by a number of modifications on the side chain of the parent amino acid. Especially, methionine undergoes a wide range of transformations. The predominant aliphatic GSLs in the *Brassicaceae* possess side chains derived from chain elongated forms of Met, such as aliphatic thio-GSLs 3-methylthiopropyl (3-MTP)-, 4-methylthiobutyl (4-MTB)-, 5-methylthiopentyl (5-MTP)-, 6-methylthiohexyl (6-MTH)-, 7-methylthioheptyl (7-MTH)- and 8-methylthiooctyl (8-MTO)-GSL; aliphatic sulfinyl-GSLs 3-methylsulfinylpropyl (3-MSP)-, 4-methylsulfinylbutyl (4-MSB)-, 5-methylsulfinylpentyl (5-MSP)-, 6-methylsulfinylhexyl (6-MSH)-, 7-methylsulfinylheptyl (7-MSH)- and 8-methylsulfinyloctyl (8-MSO)-GSL; aliphatic hydroxy-GSLs 3-hydroxypropyl (3-OHP)- and 4-hydroxybutyl (4-OHB)-GSL; aliphatic benzoyloxy-GSLs 3-benzoyloxypropyl (3-BZOP)- and 4-benzoyloxybutyl (4-BZOB)-GSL, and aliphatic alkenyl-GSLs 2-propenyl (2-P)- and 3-butenyl (3-B)-GSL. The predominant aromatic GSLs in the *Brassicaceae* possess side chains derived from Phe, such as aromatic GSL 2-phenylethyl (2-PE)-GSL. Lower amounts of GSLs with indolylic side chains derived from Trp, such as indol-GSL indol-3-ylmethyl (i3M)-GSL, also occur. GSLs co-occur in plants with the GSL-specific thioglucosidase myrosinase. This enzyme is physically separated from GSLs in plants, but is brought into contact with its substrate upon tissue disruption. The resulting hydrolysis product consists of one free glucose and one aglycone molecule per GSL molecule. The agclycones are unstable and readily rearrange into isothiocyanates, nitriles, thiocyanates and other more or less toxic compounds. Depending on the side chain of the parent amino acid these hydrolysis products contribute the actual biological activity of GSLs, while intact GSLs are believed to be an inactive storage form.

As used herein, "glucosinolate content" of a plant or plant part refers to the total of GSLs, including aliphatic, aromatic and indole GSLs, without regard to the type of GSLs. Thus the "total GSL content" or "GSL content" of a plant or plant part means the content of total GSLs of that plant or plant part and is expressed on a molecular (nmol/g or µmol/g) basis (rather than on a weight (mg/kg) basis) as GSLs have significantly different molecular weights depending on the size of their side chain. GSL accumulation varies between tissues and developmental stages. Young leaves and reproductive tissues such as siliques and seeds contain the highest concentrations while senescing leaves contain the lowest concentrations of GSLs. Intermediate concentrations are found throughout the "large" organs such as the roots, leaves and stem. In addition, the composition of the GSL profile varies markedly between organs. In roots and vegetative tissues, the GSL content is composed of indole and aliphatic GSLs while the aromatic are absent. In siliques and seeds, small amounts of aromatic and indole GSLs are found while the rest of the GSL content is entirely composed of aliphatic GSLs.

"Canola", "double-zero rapeseed" or "double-low rapeseed" is an offspring of rapeseed (*Brassica napus and Brassica campestris* or *rapa*) which was bred through standard plant breeding techniques to have low levels of erucic acid (below 2%) in the oil portion and low levels of GSLs (below 30 µmol/g) in the meal portion. "Seed" of (double-zero) rapeseed is small and round, 1-2 mm in diameter. It contains approximately 42-43% oil, which is extracted for use as edible vegetable oil. The remaining "seed meal" is a widely used protein source in animal feeds. The GSLs in rapeseed were reduced because they are toxic and unpalatable to most animals, and therefore limit the inclusion level of rapeseed meal in animal feeds to very low levels. Canola and rapeseed meals are commonly used in animal feeds around the world and are sold in bulk form as a mash or in pellets.

A "decrease in total GSL content" or "increase in total GSL content" of a plant or plant part by the methods of the present invention is measured relative to the total GSL content of a reference plant or plant part with similar genetic background. Total GSL content can be measured by any appropriate method. Methods to quantify total GSL content and to determine GSL composition of plant material are well known in the art and include but are not limited to: HPLC-UV desulfo-method involving HPLC analysis of methanol extracts desulfated and eluted from sephadex anion exchange columns as described by, e.g., Hansen et al. (2007, Plant J. 50 (5): 902-910); analysis of intact GSLs by MALDI-TOF mass spectrometry as described by, e.g., Botting et al. (2002, J. Agric. Food Chem. 50 (5): 983-988); near-infrared reflectance spectroscopy as described by, e.g., Font et al. (2005, J. Agric. Sci. 143: 65-73); methods yielding spectrophotometrically active degradation products as summarized by, e.g., Clarke (2010, Anal. Methods 2: 310-325); HPLC mass spectrometry analysis of intact glucosinolates as described by, e.g., Rochfort et al. (2008, Phytochemistry 69: 1671).

"Biofumigation", as used herein, refers to the use of GSL-containing plants, such as *Brassicaceae* (e.g. cabbage, cauliflower, kale and mustard), *Capparidaceae* (e.g. cleome) and Moringaceae (e.g. horse-radish) species, as biologically-active rotation and green manure crop for controlling several soil-borne pathogens and diseases.

"Crop plant" refers to plant species cultivated as a crop, such as *Brassica napus* (AACC, 2n=38), *Brassica juncea* (AABB, 2n=36), *Brassica carinata* (BBCC, 2n=34), *Brassica rapa* (syn. *B. campestris*) (AA, 2n=20), *Brassica oleracea* (CC, 2n=18) or *Brassica nigra* (BB, 2n=16). The definition does not encompass weeds, such as *Arabidopsis thaliana.*

The term "nucleic acid" or "nucleic acid molecule" refers to a DNA or RNA molecule in single or double stranded form, particularly a DNA encoding a protein or protein fragment as described herein. An "endogenous nucleic acid" refers to a nucleic acid within a plant cell, e.g. an endogenous allele of a GTR gene present within the nuclear genome of a *Brassica* cell. An "isolated nucleic acid" is used to refer to a nucleic acid that is no longer in its natural environment, for example *in vitro* or in a recombinant bacterial or plant host cell.

The term "gene" means a DNA fragment comprising a DNA region (transcribed region), which is transcribed into an RNA molecule (e.g. into a pre-mRNA, comprising intron sequences, which is then spliced into a mature mRNA, or directly into a mRNA without intron sequences, or into a pre-miRNA) in a cell, operable linked to regulatory regions (e.g. a promoter). A gene may thus comprise several operably linked DNA fragments, such as a promoter, a 5' leader sequence comprising e.g. sequences involved in translation initiation, a (protein) coding region (cDNA or genomic DNA) and a 3' non-translated sequence comprising e.g. transcription termination sites. "Endogenous gene" is used to differentiate from a "foreign gene", "transgene" or "chimeric gene", and refers to a gene from a plant of a certain plant genus, species or variety, which has not been introduced into that plant by transformation (i.e. it is not a "transgene"), but which is normally present in plants of that genus, species or variety, or which is introduced in that plant from plants of another plant genus, species or variety, in which it is normally present, by normal breeding techniques or by somatic hybridization, e.g., by protoplast fusion. Similarly, an "endogenous allele" of a gene is not introduced into a plant or plant tissue by plant transformation, but is, for example, generated by plant mutagenesis and/or selection or obtained by screening natural populations of plants.

As used herein a "chimeric nucleic acid construct" refers to a nucleic acid construct which is not normally found in a plant species. A chimeric nucleic acid construct can be DNA or RNA. "Chimeric DNA construct" and "chimeric gene" are used interchangeably to denote a gene which is not normally found in a plant species or to refer to any gene in which the promoter or one or more other regulatory regions of the gene are not associated in nature with part or all of the transcribed DNA region.

"Expression of a gene" or "gene expression" refers to the process wherein a DNA region, which is operably linked to appropriate regulatory regions, particularly a promoter, is transcribed into an RNA molecule. The RNA molecule is then processed further (by post-transcriptional processes) within the cell, e.g. by RNA splicing and translation initiation and translation into an amino acid chain (polypeptide), and translation termination by translation stop codons. The term "functionally expressed" is used herein to indicate that a functional protein is produced; the term "not functionally expressed" to indicate that a protein with significantly reduced or no functionality (biological activity) is produced or that no protein is produced (see further below). An RNA molecule is biologically active when it is either capable of interaction with another nucleic acid or protein or which is capable of being translated into a biologically active polypeptide or protein. A gene is said to encode an RNA when the end product of the expression of the gene is biologically active RNA, such as e.g. an antisense RNA, a ribozyme, or a miRNA. A gene is said to encode a protein when the end product of the expression of the gene is a protein or polypeptide. A gene is said to encode a GTR-inhibitory RNA when the end product of the expression of the gene is capable of down-regulating GTR functional activity, i.e. capable of down-regulating *GTR* gene expression and/or GTR protein activity.

For the purpose of the invention, the term "plant-operative promoter" and "plant-expressible promoter" mean a promoter which is capable of driving transcription in a plant, plant tissue, plant organ, plant part, or plant cell. This includes any promoter of plant origin, but also any promoter of non-plant origin which is capable of directing transcription in a plant cell.

Promoters that may be used in this respect are constitutive promoters, such as the promoter of the cauliflower mosaic virus (CaMV) 35S transcript (Harpster et al., 1988, Mol. Gen. Genet. 212: 182-190), the CaMV 19S promoter (US 5,352,605; WO 84/02913; Benfey et al., 1989, EMBO J. 8:2195-2202), the subterranean clover virus promoter No 4 or No 7 (WO 96/06932), the Rubisco small subunit promoter (US 4,962,028), the ubiquitin promoter (Holtorf et al., 1995, Plant Mol. Biol. 29:637-649), T-DNA gene promoters such as the octopine synthase (OCS) and nopaline synthase (NOS) promoters from Agrobacterium, and further promoters of genes whose constitutive expression in plants is known to the person skilled in the art.

Further promoters that may be used in this respect are tissue-specific or organ-specific promoters, preferably seed-specific promoters, such as the 2S albumin promoter (Joseffson et al., 1987, J. Biol. Chem. 262:12196-12201), the phaseolin promoter (US5,504,200; Bustos et al., 1989, Plant Cell 1.(9):839-53), the legumine promoter (Shirsat et al., 1989, Mol. Gen. Genet. 215(2):326-331), the "unknown seed protein" (USP) promoter (Baumlein et al., 1991, Mol. Gen. Genet. 225(3):459-67), the napin promoter (US5,608,152; Stalberg et al., 1996, Planta 199:515-519), the Arabidopsis oleosin promoter (WO 98/45461), the Brassica Bce4 promoter (WO 91/13980), and further promoters of genes whose seed-specific expression in plants is known to the person skilled in the art.

Other promoters that can be used are tissue-specific or organ-specific promoters like organ primordia-specific promoters (An et al., 1996, Plant Cell 8: 15-30), stem-specific promoters (Keller et al., 1988, EMBO J. 7(12): 3625-3633), leaf-specific promoters (Hudspeth et al., 1989, Plant Mol. Biol. 12: 579-589), mesophyl-specific promoters (such as the light-inducible Rubisco promoters), root-specific promoters (Keller et al., 1989, Genes Dev. 3: 1639-1646), tuber-specific promoters (Keil et al., 1989, EMBO J. 8(5): 1323-1330), vascular tissue-specific promoters (Peleman et al., 1989, Gene 84: 359-369), stamen-selective promoters (WO 89/10396, WO 92/13956), dehiscence zone-specific promoters (WO 97/13865), and the like.

Chimeric RNA constructs as described herein may be delivered to plant cells using means and methods such as described in WO90/12107, WO03/052108 or WO2005/098004.

The terms "protein" or "polypeptide" are used interchangeably and refer to molecules consisting of a chain of amino acids, without reference to a specific mode of action, size, 3-dimensional structure or origin. A "fragment" or "portion" of a GTR protein may thus still be referred to as a "protein". An "isolated protein" is used to refer to a protein that is no longer in its natural environment, for example *in vitro* or in a recombinant bacterial or plant host cell.

The term "transporter protein" is used to refer to a transmembrane protein that helps a certain substance or class of closely related substances to cross the membrane. A "glucosinolate transporter protein" (abbreviated herein as "GTR") is a proton-dependent oligopeptide transporter (POT) protein involved in glucosinolate transport.

The term "GTR gene" refers herein to a nucleic acid sequence encoding a glucosinolate transporter (GTR) protein.

As used herein, the term "allele(s)" means any of one or more alternative forms of a gene at a particular locus. In a diploid (or amphidiploid) cell of an organism, alleles of a given gene are located at a specific location or locus (loci plural) on a chromosome. One allele is present on each chromosome of the pair of homologous chromosomes.

As used herein, the term "homologous chromosomes" means chromosomes that contain information for the same biological features and contain the same genes at the same loci but possibly different alleles of those genes. Homologous chromosomes are chromosomes that pair during meiosis. "Non-homologous chromosomes", representing all the biological features of an organism, form a set, and the number of sets in a cell is called ploidy. Diploid organisms contain two sets of non-homologous chromosomes, wherein each homologous chromosome is inherited from a different parent. In amphidiploid species, essentially two sets of diploid genomes exist, whereby the chromosomes of the two genomes are referred to as "homeologous chromosomes" (and similarly, the loci or genes of the two genomes are referred to as homeologous loci or genes). A diploid, or amphidiploid, plant species may comprise a large number of different alleles at a particular locus.

As used herein, the term "heterozygous" means a genetic condition existing when two different alleles reside at a specific locus, but are positioned individually on corresponding pairs of homologous chromosomes in the cell. Conversely, as used herein, the term "homozygous" means a genetic condition existing when two identical alleles reside at a specific locus, but are positioned individually on corresponding pairs of homologous chromosomes in the cell.

As used herein, the term "locus" (loci plural) means a specific place or places or a site on a chromosome where for example a gene or genetic marker is found. For example, the *"GTR-A1* locus" refers to the position on a chromosome of the A genome where the *GTR-A1* gene (and two *GTR-A1* alleles) may be found, while the*"GTR-C1* locus" refers to the position on a chromosome of the C genome where the *GTR-C1* gene (and two *GTR-C1* alleles) may be found.

Whenever reference to a "plant" or "plants" according to the invention is made, it is understood that also plant parts, progeny of the plants which retain the distinguishing characteristics of the parents (especially the glucosinolate content in particular plant parts), such as seed obtained by selfing or crossing, e.g. hybrid seed (obtained by crossing two inbred parental lines), hybrid plants and plant parts derived thereof are encompassed herein, unless otherwise indicated.

"Plant parts", as used herein, refers to any part of the plant, including plant cells, plant tissues, plant organs, siliques or seed pods, seeds, severed parts such as roots, leaves, flowers, pollen, etc.).

A "molecular assay" (or test) refers herein to an assay that indicates (directly or indirectly) the presence or absence of one or more particular *GTR* alleles at one or both *GTR* loci (e.g. at one or both of the GTR-A1 or GTR-C1 loci). In one embodiment it allows one to determine whether a particular (wild type or mutant) allele is homozygous or heterozygous at the locus in any individual plant.

"Wild type" (also written "wildtype" or "wild-type"), as used herein, refers to a typical form of a plant or a gene as it most commonly occurs in nature. A "wild type plant" refers to a plant with the most common phenotype of such plant in the natural population. A "wild type allele" refers to an allele of a gene required to produce the wild-type phenotype. By contrast, a "mutant plant" refers to a plant with a different rare phenotype of such plant in the natural population or produced by human intervention, e.g. by mutagenesis, and a "mutant allele" refers to an allele of a gene required to produce the mutant phenotype.

As used herein, the term "wild type *GTR*" (e.g. wild type *GTR-A1* or *GTR-C1*), means a naturally occurring *GTR* allele found within plants, in particular *Brassicaceae* plants, especially *Arabidopsis* and *Brassica* plants, which encodes a functional GTR protein (e.g. a functional GTR-A1 or GTR-C1, respectively). In contrast, the term "mutant *GTR*" (e.g. mutant *GTR-A1* or *GTR-C1*), as used herein, refers to an GTR allele, which does not encode a functional GTR protein, i.e. an GTR allele encoding a non-functional GTR protein (e.g. a non-functional *GTR-A1* or GTR-C1, respectively), which, as used herein, refers to an GTR protein having no biological activity or a significantly reduced biological activity as compared to the corresponding wild-type functional GTR protein, or encoding no GTR protein at all. Such a "mutant *GTR* allele" (also called "full knock-out" or "null" allele) is a wild-type *GTR* allele, which comprises one or more mutations in its nucleic acid sequence, whereby the mutation(s) preferably result in a significantly reduced (absolute or relative) amount of functional GTR protein in the cell *in vivo.* Mutant alleles of the GTR protein-encoding nucleic acid sequences are designated as *"gtr"* (e.g. *gtr-a1* or *gtr-c1,* respectively) herein. Mutant alleles can be either "natural mutant" alleles, which are mutant alleles found in nature (e.g. produced spontaneously without human application of mutagens) or "induced mutant" alleles, which are induced by human intervention, e.g. by mutagenesis.

A "significantly reduced amount of functional GTR protein" (e.g. functional GTR-A1 or GTR-C1 protein) refers to a reduction in the amount of a functional GTR protein produced by the cell comprising a mutant *GTR* allele by at least 30%, 40%, 50%, 60%, 70%, 80%, 90%, 95% or 100% (i.e. no functional GTR protein is produced by the cell) as compared to the amount of the functional GTR protein produced by the cell not comprising the mutant *GTR* allele. This definition encompasses the production of a "non-functional" GTR protein (e.g. truncated GTR protein) having no GSL transport activity *in vivo,* the reduction in the absolute amount of the functional GTR protein (e.g. no functional GTR protein being made due to the mutation in the *GTR* gene), and/or the production of an GTR protein with significantly reduced GSL transport activity compared to the activity of a functional wild type GTR protein (such as an GTR protein in which one or more amino acid residues that are crucial for the GSL transport activity of the encoded GTR protein, as exemplified below, are substituted with another amino acid residue). The term "mutant GTR protein", as used herein, refers to an GTR protein encoded by a mutant *GTR* nucleic acid sequence ("*gtr* allele") whereby the mutation results in a significantly reduced and/or no GTR activity *in vivo,* compared to the activity of the GTR protein encoded by a non-mutant, wild type *GTR* sequence ("*GTR* allele").

"Mutagenesis", as used herein, refers to the process in which plant cells (e.g., a plurality of *Brassica* seeds or other parts, such as pollen, etc.) are subjected to a technique which induces mutations in the DNA of the cells, such as contact with a mutagenic agent, such as a chemical substance (such as ethylmethylsulfonate (EMS), ethylnitrosourea (ENU), etc.) or ionizing radiation (neutrons (such as in fast neutron mutagenesis, etc.), alpha rays, gamma rays (such as that supplied by a Cobalt 60 source), X-rays, UV-radiation, etc.), or a combination of two or more of these. Thus, the desired mutagenesis of one or more *GTR* alleles may be accomplished by use of chemical means such as by contact of one or more plant tissues with ethylmethylsulfonate (EMS), ethylnitrosourea, etc., by the use of physical means such as x-ray, etc, or by gamma radiation, such as that supplied by a Cobalt 60 source. While mutations created by irradiation are often large deletions or other gross lesions such as translocations or complex rearrangements, mutations created by chemical mutagens are often more discrete lesions such as point mutations. For example, EMS alkylates guanine bases, which results in base mispairing: an alkylated guanine will pair with a thymine base, resulting primarily in G/C to A/T transitions.

As used herein, the term "non-naturally occurring" when used in reference to a plant, means a plant with a genome that has been modified by man. A transgenic plant, for example, is a non-naturally occurring plant that contains an exogenous nucleic acid molecule, e.g., a chimeric gene comprising a transcribed region which when transcribed yields a biologically active RNA molecule capable of reducing the expression of an endogenous gene, such as an GTR gene according to the invention, and, therefore, has been genetically modified by man. In addition, a plant that contains a mutation in an endogenous gene, for example, a mutation in an endogenous GTR gene, (e.g. in a regulatory element or in the coding sequence) as a result of an exposure to a mutagenic agent is also considered a non-naturally plant, since it has been genetically modified by man. Furthermore, a plant of a particular, species, such as *Brassica napus,* that contains a mutation in an endogenous gene, for example, in an endogenous GTR gene, that in nature does not occur in that particular plant species, as a result of, for example, directed breeding processes, such as marker-assisted breeding and selection or introgression, with a plant of the same or another species, such as *Brassica juncea* or *rapa,* of that plant is also considered a non-naturally occurring plant. In contrast, a plant containing only spontaneous or naturally occurring mutations, i.e. a plant that has not been genetically modified by man, is not a "non-naturally occurring plant" as defined herein and, therefore, is not encompassed within the invention. One skilled in the art understands that, while a non-naturally occurring plant typically has a nucleotide sequence that is altered as compared to a naturally occurring plant, a non-naturally occurring plant also can be genetically modified by man without altering its nucleotide sequence, for example, by modifying its methylation pattern.

The term "ortholog" of a gene or protein refers herein to the homologous gene or protein found in another species, which has the same function as the gene or protein, but is (usually) diverged in sequence from the time point on when the species harboring the genes diverged (i.e. the genes evolved from a common ancestor by speciation). Orthologs of, for example, the *Brassica napus GTR* genes may thus be identified in other plant species (e.g. *Brassica juncea,* etc.) based on both sequence comparisons (e.g. based on percentages sequence identity over the entire sequence or over specific domains) and/or functional analysis.

A "variety" is used herein in conformity with the UPOV convention and refers to a plant grouping within a single botanical taxon of the lowest known rank, which grouping can be defined by the expression of the characteristics resulting from a given genotype or combination of genotypes, can be distinguished from any other plant grouping by the expression of at least one of the said characteristics and is considered as a unit with regard to its suitability for being propagated unchanged (stable).

The term "comprising" is to be interpreted as specifying the presence of the stated parts, steps or components, but does not exclude the presence of one or more additional parts, steps or components. A plant comprising a certain trait may thus comprise additional traits. A nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined may comprise additional DNA regions etc.

It is understood that when referring to a word in the singular (e.g. plant or root), the plural is also included herein (e.g. a plurality of plants, a plurality of roots). Thus, reference to an element by the indefinite article "a" or "an" does not exclude the possibility that more than one of the element is present, unless the context clearly requires that there be one and only one of the elements. The indefinite article "a" or "an" thus usually means "at least one".

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e., a position in an alignment where a residue is present in one sequence but not in the other, is regarded as a position with non-identical residues. The "optimal alignment" of two sequences is found by aligning the two sequences over the entire length according to the Needleman and Wunsch global alignment algorithm (Needleman and Wunsch, 1970, J Mol Biol 48(3):443-53) in The European Molecular Biology Open Software Suite (EMBOSS, Rice et al., 2000, Trends in Genetics 16(6): 276-277; see e.g. http://www.ebi.ac.uk/emboss/align/index.html) using default settings (gap opening penalty = 10 (for nucleotides) / 10 (for proteins) and gap extension penalty = 0.5 (for nucleotides) / 0.5 (for proteins)). For nucleotides the default scoring matrix used is EDNAFULL and for proteins the default scoring matrix is EBLOSUM62.

It will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

"Substantially identical" or "essentially similar", as used herein, refers to sequences, which, when optimally aligned as defined above, share at least a certain minimal percentage of sequence identity (as defined further below).

"Stringent hybridization conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tₘ) for the specific sequences at a defined ionic strength and pH. The Tₘ is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridizations (Northern blots using a probe of e.g. 100nt) are for example those which include at least one wash in 0.2X SSC at 63°C for 20min, or equivalent conditions.

"High stringency conditions" can be provided, for example, by hybridization at 65°C in an aqueous solution containing 6x SSC (20x SSC contains 3.0 M NaCl, 0.3 M Na-citrate, pH 7.0), 5x Denhardt's (100X Denhardt's contains 2% Ficoll, 2% Polyvinyl pyrollidone, 2% Bovine Serum Albumin), 0.5% sodium dodecyl sulphate (SDS), and 20 µg/ml denaturated carrier DNA (single-stranded fish sperm DNA, with an average length of 120 - 3000 nucleotides) as non-specific competitor. Following hybridization, high stringency washing may be done in several steps, with a final wash (about 30 min) at the hybridization temperature in 0.2-0.1× SSC, 0.1% SDS.

"Moderate stringency conditions" refers to conditions equivalent to hybridization in the above described solution but at about 60-62°C. Moderate stringency washing may be done at the hybridization temperature in 1x SSC, 0.1% SDS.

"Low stringency" refers to conditions equivalent to hybridization in the above described solution at about 50-52°C. Low stringency washing may be done at the hybridization temperature in 2x SSC, 0.1% SDS. See also Sambrook *et al.* (1989) and Sambrook and Russell (2001).

### Detailed description of the invention

The present invention is based on the identification of GSL transporter (GTR) proteins and corresponding GTR genes in the model plant *Arabidopsis thaliana* (Nour-Eldin, 2007, *supra*). It was found by the inventors that the *Arabidopsis* GTR genes form a small subclade with six homologs (Figure 1a), named *GTR1* to 6. The inventors further found that seeds of *Arabidopsis* plants knocked out in either GTR1 or GTR2 transporters had no significant reduction and about 50% reduction in total aliphatic GSLs concentrations, respectively, compared to wildtype plants, and that seeds of *Arabidopsis* plants knocked out in both GTR1 and GTR2 transporters had a zero GSL seed phenotype. Surprisingly, GSLs levels in senescent leaves from gtr knockout plants were high whereas, in wildtype plants, leaves become depleted in GSLs upon aging. The inventors further found that *Brassica rapa* (genome AA, 2n=20) and *Brassica oleracea* (genome CC, 2n=18) each comprise three *GTR₁* genes and three *GTR2* genes in their genome, while *Brassica napus* (genome AACC, 2n=4x=38), which is an allotetraploid (amphidiploid) species containing essentially two diploid genomes (the A and the C genome) due to its origin from diploid ancestors, comprises six *GTR1* genes and six *GTR2* genes in its genome; three of the six *GTR1* and GTR2 genes are located on the A genome and three on the C genome. *Brassica juncea* (genome AABB, 2n=4x= 36), which is another allotetraploid species containing essentially two diploid genomes (the A and the B genome) also comprises six *GTR2* genes in its genome; three of the six *GTR2* are located on the A genome and three on the B genome The *GTR1* and *GTR2* genes that are located on the A genome are herein referred to as *"GTRx-Ay"* (wherein x is 1 or 2 and y is 1, 2 or 3) and the *GTR1* and *GTR2* genes that are located on the C genome are herein referred to as *"GTRx-Cy"* (wherein x is 1 or 2 and y is 1, 2 or 3). The *GTR1* and *GTR2* genes that are located on the B genome are herein referred to as "*GTRx*-*By*" (wherein x is 1 or 2 and y is 1, 2 or 3). The GTRx-Ay genes from *B. napus* are said to be "homeologous" to the corresponding GTRx-Cy gene from *B*. *napus,* i.e. the "A gene" is found on the A genome and originates from the diploid ancestor *B*. *rapa* (AA), while the "C gene" is found on the C genome of *B*. *napus* and originates from the diploid ancestor *B. oleracea* (CC). Similarly, the GTRx-Ay genes from *B. juncea* are said to be "homeologous" to the corresponding GTRx-By gene from *B. juncea,* i.e. the "A gene" is found on the A genome and originates from the diploid ancestor *B*. *rapa* (AA), while the "B gene" is found on the B genome of *B*. *napus* and originates from the diploid ancestor *B*. *nigra* (BB)

As in any diploid genome, two "alleles" can be present *in vivo* for each *GTR* gene at each *GTR* locus in the genome (one allele being the gene sequence found on one chromosome and the other on the homologous chromosome). The nucleotide sequence of these two alleles may be identical (homozygous plant) or different (heterozygous plant) in any given plant, although the number of different possible alleles existing for each *GTR* gene may be much larger than two in the species population as a whole.

Described herein are nucleic acid sequences of wild type and mutant GTR genes/alleles from *Brassicaceae* species, as well as the wild type and mutant GTR proteins. Provided are methods of generating mutant *GTR* alleles in *Brassicaceae* plants, as well as *Brassicaceae* plants and plant parts comprising specific combinations of wild type and mutant *GTR* alleles in their genome, whereby the GSL content is decreased in specific parts of these plants, such as seed and seed meal, or increased in green plant tissue. These plants can be used for transferring mutant *GTR* alleles to other plants. In addition kits and methods for marker assisted selection (MAS) for combining or detecting *GTR* genes and/or alleles are described. Different embodiments of the invention are described in detail herein below.

### Nucleic acid sequences

Described herein are both wild type *GTR* nucleic acid sequences encoding functional GTR proteins and mutant *gtr* nucleic acid sequences (comprising one or more mutations, preferably mutations which result in no or a significantly reduced GSL transport activity of the encoded GTR protein or in no GTR protein being produced) of *GTR* genes from *Brassicaceae,* particularly from *Brassica* species, especially from *Brassica napus, Brassica juncea, Brassica rapa* or *Brassica oleracea,* but also from other *Brassica* crop species. For example, *Brassica* species comprising an A and/or a C genome may comprise different alleles of *GTR-A* or *GTR-C* genes, which can be identified and combined in a single plant according to the invention. In addition, mutagenesis methods can be used to generate mutations in wild type *GTR* alleles, thereby generating mutant *gtr* alleles for use according to the invention. Because specific *GTR* alleles are preferably combined in a plant by crossing and selection, the *GTR* and/or *gtr* nucleic acid sequences can be present within a plant (i.e. endogenously), e.g. a *Brassica* plant, preferably a *Brassica* plant which can be crossed with *Brassica napus, Brassica juncea, Brassica rapa* or *Brassica oleracea* or which can be used to make a "synthetic" *Brassica napus* plant. Hybridization between different *Brassica* species is described in the art, e.g., as referred to in Snowdon (2007, Chromosome research 15: 85-95). Interspecific hybridization can, for example, be used to transfer genes from, e.g., the C genome in *B*. *napus* (AACC) to the C genome in *B*. *carinata* (BBCC), or even from, e.g., the C genome in *B. napus* (AACC) to the B genome in *B. juncea* (AABB) (by the sporadic event of illegitimate recombination between their C and B genomes). "Resynthesized" or "synthetic" *Brassica napus* lines can be produced by crossing the original ancestors, *B. oleracea* (CC) and *B*. *rapa* (AA). Interspecific, and also intergeneric, incompatibility barriers can be successfully overcome in crosses between *Brassica* crop species and their relatives, e.g., by embryo rescue techniques or protoplast fusion (see e.g. Snowdon, above).

However, isolated *GTR* and *gtr* nucleic acid sequences (e.g. isolated from the plant by cloning or made synthetically by DNA synthesis), as well as variants thereof and fragments of any of these are also described herein, as these can be used to determine which sequence is present endogenously in a plant or plant part, whether the sequence encodes a functional, a non-functional or no protein (e.g. by expression in a recombinant host cell as described below) and for selection and transfer of specific alleles from one plant into another, in order to generate a plant having the desired combination of functional and mutant alleles.

"GTR nucleic acid sequences" or "GTR variant nucleic acid sequences" as used herein are nucleic acid sequences encoding an amino acid sequence having at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 55%, at least 56%, at least 57%, at least 58%, at least 59%, at least 60%, at least 61%, at least 62%, at least 63%, at least 64%, at least 65%, at least 70%, at least 75%, at least 76%, at least 77%, at least 78%, at least 79%, at least 80%, at least 85%, at least 90%, at least 95%, 98%, 99% or 100% sequence identity with SEQ ID NO: 2, or nucleic acid sequences having at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 55%, at least 56%, at least 57%, at least 58%, at least 59%, at least 60%, at least 61%, at least 62%, at least 63%, at least 64%, at least 65%, at least 70%, at least 75%, at least 76%, at least 77%, at least 78%, at least 79%, at least 80%, at least 85%, at least 90%, at least 95%, 98%, 99% or 100% sequence identity with SEQ ID NO: 1. These nucleic acid sequences may also be referred to as being "essentially similar" or "essentially identical" to the GTR sequences provided in the sequence listing.

Nucleic acid sequences of *GTR1* to 6 have been isolated from *Arabidopsis,* of *GTRx-Ay* from *B*. *rapa, B. juncea* and from *B. napus,* of *GTRx-By* from *B. juncea* and of *GTRx-Cy* from *B*. *oleracea* and from *B. napus* as depicted in the sequence listing. The wild type *GTR* sequences are depicted, while the mutant *gtr* sequences of these sequences, and of sequences essentially similar to these, are described herein below and in the Examples, with reference to the wild type *GTR* sequences. The genomic GTR protein-encoding DNA, and corresponding pre-mRNA, comprises 4 exons (numbered exons 1-4 starting from the 5'end) interrupted by 3 introns (numbered introns 1-3, starting from the 5'end). In the cDNA and corresponding processed mRNA (i.e. the spliced RNA), introns are removed and exons are joined, as depicted in the sequence listing. Exon sequences are more conserved evolutionarily and are therefore less variable than intron sequences.

"GTR1, 2, 3, 4, 5 or 6 nucleic acid sequences" or "GTR1, 2, 3, 4, 5 or 6 variant nucleic acid sequences" as used herein are nucleic acid sequences encoding an amino acid sequence having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, 96%, 97%, 98%, 99% or 100% sequence identity with SEQ ID NO: 2, 4, 6, 8, 10 or 12, respectively, or nucleic acid sequences having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, 96%, 97%, 98%, 99% or 100% sequence identity with SEQ ID NO: 1, 3, 5, 7, 9 or 11. These nucleic acid sequences may also be referred to as being "essentially similar" or "essentially identical" to the GTR sequences provided in the sequence listing.

Thus described herein are both nucleic acid sequences encoding wild type, functional GTR1, 2, 3, 4, 5 or 6 proteins, including variants and fragments thereof (as defined further below), as well as mutant nucleic acid sequences of any of these, whereby the mutation in the nucleic acid sequence preferably results in one or more amino acids being inserted, deleted or substituted in comparison to the wild type GTR protein. Preferably the mutation(s) in the nucleic acid sequence result in one or more amino acid changes (i.e. in relation to the wild type amino acid sequence one or more amino acids are inserted, deleted and/or substituted) whereby the GSL transport activity of the GTR protein is significantly reduced or completely abolished. A significant reduction in or complete abolishment of the GSL transport activity of the GTR protein refers herein to a reduction in or abolishment of the GSL transport activity of the GTR protein, such that the GSL content is modified in specific parts of a plant expressing the mutant GTR protein as compared to a plant expressing the corresponding wild type GTR protein.

To determine the functionality of specific *GTR* nucleic acids or proteins, they can, for example, be functionally screened in *Xenopus* oocytes as described by Nour-Eldin et al. (2006, Plant Methods 2: 17) and in the Examples below.

To determine the functionality of specific *GTR* alleles or proteins in plants, particularly in *Brassicaceae* plants, GSL content can, for example, be compared between specific parts of plants comprising different forms of the specific *GTR* alleles or proteins, for example, mutated and wildtype forms of the specific *GTR* alleles or proteins, e.g., by performing HPLC-UV analysis of methanol extracts desulfated and eluted from sephadex anion exchange columns as described by, e.g., Hansen et al. (2007, Plant J. 50 (5): 902-910) or as described in the Examples below.

Both endogenous and isolated nucleic acid sequences are described herein. Also described are fragments of the *GTR* sequences and *GTR* variant nucleic acid sequences defined above, for use as primers or probes and as components of kits (see further below). A "fragment" of a *GTR* or *gtr* nucleic acid sequence or variant thereof (as defined) may be of various lengths, such as at least 10, 12, 15, 18, 20, 50, 100, 200, 500, 600 contiguous nucleotides of the *GTR* or *gtr* sequence (or of the variant sequence).

### Nucleic acid sequences encoding functional GTR proteins

The nucleic acid sequences depicted in the sequence listing encode wild type, functional GTR proteins from *Arabidopsis, B. rapa, B. oleracea, B. juncea* and *B*. *napus.*
Thus, these sequences are endogenous to the plants from which they were isolated. Other *Brassicaceae* plants, including *Brassica* crop species, varieties, breeding lines or wild accessions, may be screened for other *GTR* alleles, encoding the same GTR proteins or variants thereof. For example, nucleic acid hybridization techniques (e.g. Southern blot analysis, using for example stringent hybridization conditions) or PCR-based techniques may be used to identify *GTR* alleles endogenous to other *Brassicaceae* plants, such as various *Brassica napus, oleracea* and *rapa* varieties, lines or accessions, but also *Brassica juncea* (especially *GTR* alleles on the A-genome) and *Brassica carinata* (especially *GTR* alleles on the C-genome) plants, organs and tissues can be screened for other wild type *GTR* alleles. To screen such plants, plant organs or tissues for the presence of *GTR* alleles, the *GTR* nucleic acid sequences provided in the sequence listing, or variants or fragments of any of these, may be used. For example whole sequences or fragments may be used as probes or primers. For example specific or degenerate primers may be used to amplify nucleic acid sequences encoding GTR proteins from the genomic DNA of the plant, plant organ or tissue. These *GTR* nucleic acid sequences may be isolated and sequenced using standard molecular biology techniques. Bioinformatics analysis may then be used to characterize the allele(s), for example in order to determine which *GTR* allele the sequence corresponds to and which GTR protein or protein variant is encoded by the sequence.

Whether a nucleic acid sequence encodes a functional GTR protein can be analyzed by recombinant DNA techniques as known in the art, e.g., by a genetic complementation test using, e.g., an *Arabidopsis* plant, which is homozygous for one or more full knock-out *gtr* mutant alleles or a *Brassica* plant, which is homozygous for one or more full knock-out *gtr* mutant allele or by functionally screening the GTR protein in *Xenopus* oocytes as described by Nour-Eldin et al. (2006, Plant Methods 2, 17).

In addition, it is understood that *GTR* nucleic acid sequences and variants thereof (or fragments of any of these) may be identified *in silico,* by screening nucleic acid databases for essentially similar sequences. Likewise, a nucleic acid sequence may be synthesized chemically. Fragments of nucleic acid molecules are described further below. Fragments include nucleic acid sequences encoding only specific conserved and functional domains as described below.

### Nucleic acid sequences encoding mutant GTR proteins

Nucleic acid sequences comprising one or more nucleotide deletions, insertions or substitutions relative to the wild type nucleic acid sequences are described herein, as are fragments of such mutant nucleic acid molecules. Such mutant nucleic acid sequences (referred to as *gtr* sequences) can be generated and/or identified using various known methods, as described further below. Again, such nucleic acid molecules can be present both in endogenous form and in isolated form. Suitable are mutation(s) which result in one or more changes (deletions, insertions and/or substitutions) in the amino acid sequence of the encoded GTR protein (i.e. it is not a "silent mutation"). Also suitable are mutation(s) in the nucleic acid sequence which result in a significantly reduced or completely abolished GSL transport activity of the encoded GTR protein relative to the wild type protein.

The nucleic acid molecules may, thus, comprise one or more mutations, such as:
(a) a "missense mutation" or "substitution mutation", which is a change in the nucleic acid sequence that results in the substitution of an amino acid with another amino acid;
(b) a "nonsense mutation" or "STOP codon mutation", which is a change in the nucleic acid sequence that results in the introduction of a premature STOP codon and thus the termination of translation (resulting in a truncated protein); plant genes contain the translation stop codons "TGA" (UGA in RNA), "TAA" (UAA in RNA) and "TAG" (UAG in RNA); thus any nucleotide substitution, insertion, deletion which results in one of these codons to be in the mature mRNA being translated (in the reading frame) will terminate translation;
(c) an "insertion mutation" of one or more amino acids, due to one or more codons having been added in the coding sequence of the nucleic acid;
(d) a "deletion mutation" of one or more amino acids, due to one or more codons having been deleted in the coding sequence of the nucleic acid;
(e) a "frameshift mutation", resulting in the nucleic acid sequence being translated in a different frame downstream of the mutation. A frameshift mutation can have various causes, such as the insertion, deletion or duplication of one or more nucleotides, but also mutations which affect pre-mRNA splicing (splice site mutations) can result in frameshifts;
(f) a "splice site mutation", which alters or abolishes the correct splicing of the pre-mRNA sequence, resulting in a protein of different amino acid sequence than the wild type. For example, one or more exons may be skipped during RNA splicing, resulting in a protein lacking the amino acids encoded by the skipped exons. Alternatively, the reading frame may be altered through incorrect splicing, or one or more introns may be retained, or alternate splice donors or acceptors may be generated, or splicing may be initiated at an alternate position (e.g. within an intron), or alternate polyadenylation signals may be generated. Correct pre-mRNA splicing is a complex process, which can be affected by various mutations in the nucleotide sequence of the GTR-encoding gene. In higher eukaryotes, such as plants, the major spliceosome splices introns containing GU at the 5' splice site (donor site) and AG at the 3' splice site (acceptor site). This GU-AG rule (or GT-AG rule; see Lewin, Genes VI, Oxford University Press 1998, pp885-920, ISBN 0198577788) is followed in about 99% of splice sites of nuclear eukaryotic genes, while introns containing other dinucleotides at the 5' and 3' splice site, such as GC-AG and AU-AC account for only about 1% and 0.1% respectively. Examples of introns containing GU at the 5' splice site (donor site) and AG at the 3' splice site (acceptor site) are indicated in the sequence listing.

As already mentioned, it is desired that the mutation(s) in the nucleic acid sequence preferably result in a mutant protein comprising significantly reduced or no GSL transport activity *in vivo* or in the production of no protein. Any mutation which results in a protein comprising at least one amino acid insertion, deletion and/or substitution relative to the wild type protein can lead to significantly reduced or no GSL transport activity. It is, however, understood that mutations in certain parts of the protein are more likely to result in a reduced function of the mutant GTR protein, such as mutations leading to truncated proteins, whereby significant portions of conserved or functional domains are lacking.

Conserved and functional domains of the *Arabidopsis* GTR1, 2, 3, 4, 5 and 6 protein can be found in the *Arabidopsis* Information Resource (TAIR) database (http://www.arabidopsis.org/) under At3g47960 for AtGTR1 (SEQ ID NO: 2), At5g62680 for AtGTR2 (SEQ ID NO: 4), At1g18880 for AtGTR3 (SEQ ID NO: 6), At1g69860 for AtGTR4 (SEQ ID NO: 8), At1g69870 for AtGTR5 (SEQ ID NO: 10) and At1g27080 for AtGTR6 (SEQ ID NO: 12) and/or by optimally aligning the *Arabidopsis* GTR1, 2, 3, 4, 5 and 6 protein sequences and determining conserved domains (see Figure 1), such as amino acid sequence F/VALTKPTLGM/LAPRKGE/AISS (SEQ ID NO: 2 from the amino acid at position 448 to 466 or SEQ ID No. 142 from amino acid position 478 to 496) and sequences essentially similar thereto (such as SEQ ID NO: 4 from the amino acid at position 462 to 480 and in SEQ ID NO: 6 from the amino acid at position 436 to 454); amino acid sequence U/INxxxLDxY/FY/FxxxxxxxxxNxxY/F (SEQ ID NO: 2 from the amino acid at position 545 to 567 or SEQ ID No. 142 from position 575 to 597) and sequences essentially similar thereto.

Corresponding conserved and functional domains can be determined for the *Brassica* GTR proteins by optimally aligning the *Brassica* and *Arabidopsis* GTR proteins and based on the annotation information in the TAIR database.

It has been found that *Arabidopsis* GTR1, 2 and GTR3 contain the above mentioned sequence F/VALTKPTLGM/LAPRKGE/AISS, while *Arabidopsis* GTR4, 5 and GTR6 do not contain that sequence (see alignment of Figure 7). As elaborated below in the Examples, GTR1, GTR2 and GTR3 exhibited most uptake activity of glucosinolates in the *Xenopus* oocytes test. The protein structure of GTR2 protein as predicted contains 12 transmembrane domains (Figure 8). The conserved sequence forms a GTR1/2/3 specific loop near the carboxyterminus of the protein, as indicated by a dashed arrow in Figure 8.

A similar domain can be found in GTR1 and GTR2 proteins of Brassica species including *BnGTR1-A1* (SEQ ID NO: 14 amino acid positions 456-474); *BnGTR1-A2* (SEQ ID NO: 16 amino acid positions 459-477); *BnGTR1-A3* (SEQ ID NO: 18 amino acid positions 457-475); *BnGTR1-C1* (SEQ ID NO: 20 amino acid positions 456-474); *BnGTR1-C2* (SEQ ID NO: 22 amino acid positions 459-477); *BnGTR1-C3* (SEQ ID NO: 24 amino acid positions 457-475); *BnGTR2-A1* (SEQ ID NO: 26 amino acid positions 458-476); *BnGTR2-A2* (SEQ ID NO: 28 amino acid positions 458-476); *BnGTR2-A3* (SEQ ID NO: 30 amino acid positions 458-476); *BnGTR2-C1* (SEQ ID NO: 32 amino acid positions 458-476); *BnGTR2-C2* (SEQ ID NO: 34 amino acid positions 401-419); *BnGTR2-C3* (SEQ ID NO: 36 amino acid positions 457-475); *BrGTR1-A1* (SEQ ID NO: 38 amino acid positions 456-474); *BrGTR1-A2* (SEQ ID NO: 40 amino acid positions 459-477); *BrGTR1-A3* (SEQ ID NO: 42 amino acid positions 457-475); *BrGTR2-A1* (SEQ ID NO: 44 amino acid positions 458-476); *BrGTR2-A2* (SEQ ID NO: 46 amino acid positions 458-476); *BrGTR2-A3* (SEQ ID NO: 48 amino acid positions 458-476); *BoGTR1-C1* (SEQ ID NO: 50 amino acid positions 456-474); *BoGTR1-C2* (SEQ ID NO: 52 amino acid positions 459-477); *BoGTR1-C3* (SEQ ID NO: 54 amino acid positions 457-475); *BoGTR2-C1* (SEQ ID NO: 56 amino acid positions 458-476); *BoGTR2-C2* (SEQ ID NO: 58 amino acid positions 458-476); *BoGTR2-C3* (SEQ ID NO: 60 amino acid positions 458-476); *BjGTR2-A1* (SEQ ID NO: 120 amino acid positions *458-476);BjGTR2-A2* (SEQ ID NO: 122 amino acid positions 458-476); *BjGTR2-A3* (SEQ ID NO: 124 amino acid positions 458-476); *BjGTR2-B1* (SEQ ID NO: 126 amino acid positions 405-423); *BjGTR2-B2* (SEQ ID NO: 128 amino acid positions 458-476) and *BjGTR2-B3* (SEQ ID NO: 130 amino acid positions 452-470).

It has also been recently found, and confirmed by proteomic data, that the GTR1 protein of Arabidopsis thaliana may comprise an aminoterminal peptide extension with a length of 30 amino acids when compared with the GTR1 protein as represented in SEQ ID No 2. The variant with the N-terminal extension is provided in SEQ ID No. 142 and the nucleotide sequence encoding such a protein is provided in SEQ ID No. 141. A similar N-terminal peptide could be found in *B*. *rapa* GTR1_A1, GTR1_A2, GTR1_A3, B. napus GTR1_A1, GTR1_A2, GTR1_A3, in *B. oleracea* GTR_C1, GT1_C2, GTR1_C3 and in B. *napus* GTR_C1, GT1_C2, GTR1_C3 (SEQ ID NOS: 143-150). It can be expected that a similar extension can be found in the GTR1_B1, GTR1_B2 and GTR1_B3 such as present in *B. juncea.* Mutations altering this 30/23 amino acid sequence may result in mutant GTR1 protein being located at a different subcellular location in the plant cell, and thus in functionally less or not active GTR1 protein. The amino terminal peptide may also interact with proteins providing a regulatory mechanism impacting the GTR1 activity under certain conditions such as e.g. different kinds of stresses, and mutants in this region may impact functionality also in this manner.

Thus described are nucleic acid sequences comprising one or more of any of the types of mutations described above. Also described are *gtr* sequences comprising one or more stop codon (nonsense) mutations, one or more substitution (missense) mutations and/or one or more splice site (frameshift) mutations. Any of the above mutant nucleic acid sequences may be present *per se* (in isolated form), or in plants and plant parts comprising such sequences endogenously. In the tables herein below the most preferred *gtr* alleles are described.

A nonsense mutation in a *GTR* allele, as used herein, is a mutation in a *GTR* allele whereby one or more translation stop codons are introduced into the coding DNA and the corresponding mRNA sequence of the corresponding wild type *GTR* allele. Translation stop codons are TGA (UGA in the mRNA), TAA (UAA) and TAG (UAG). Thus, any mutation (deletion, insertion or substitution) that leads to the generation of an in-frame stop codon in the coding sequence will result in termination of translation and truncation of the amino acid chain. A mutant *GTR* allele comprising a nonsense mutation can be a *GTR* allele wherein an in-frame stop codon is introduced in the *GTR* codon sequence by a single nucleotide substitution, such as the mutation of CAG to TAG, TGG to TAG or TGA, CGA to TGA, CAA to TAA, etc. (see Tables below).A mutant *GTR* allele comprising a nonsense mutation can be a *GTR* allele comprising a STOP codon at a position corresponding to the codon of the amino acid at position 229 in SEQ ID NO: 66, such as the GTR allele indicated in Table 7 of Example 3 below. Further, a mutant *GTR* allele comprising a nonsense mutation can be a *GTR* allele wherein an in-frame stop codon is introduced in the *GTR* codon sequence by double nucleotide substitutions, such as the mutation of CAG to TAA, TGG to TAA, CGA to TAA, etc. (see Tables below). Further, a mutant *GTR* allele comprising a nonsense mutation can be a *GTR* allele wherein an in-frame stop codon is introduced in the *GTR* codon sequence by triple nucleotide substitutions, such as the mutation of CGG to TAA, etc. (see Tables below). The truncated protein lacks the amino acids encoded by the coding DNA downstream of the mutation (i.e. the C-terminal part of the GTR protein) and maintains the amino acids encoded by the coding DNA upstream of the mutation (i.e. the N-terminal part of the GTR protein). The more truncated the mutant GTR protein is in comparison to the wild type GTR protein, the more the truncation may result in a significantly reduced or no activity of the GTR protein.

The Tables herein below describe a range of possible nonsense mutations in *Arabidopsis* and *Brassica GTR* sequences provided herein:

**Table 1 Potential EMS-induced STOP codon mutations in exon (abbreviated as E) 1, 2, 3 and 4 of AtGTR1 and AtGTR2**

| | *AtGTR1* | | | | *AfGTR2* | | | |
|---|---|---|---|---|---|---|---|---|
| | Nucleotide position in SEQ ID NO: 61 | Amino acid position in SEQ ID NO: 62 | Wild-type codon | → Potential stop codons | Nucleotide position in SEQ ID NO: 63 | Amino acid position in SEQ ID NO: 64 | Wild-type codon | → Potential stop codons |
| E1 | 79-81 | 27 | CAG | TAG TAA | 43-45 | 15 | CAG | TAA TAG |
| | 103-105 | 35 | TGG | TAG TAA TGA | 109-111 | 37 | CAG | TAA TAG |
| | | | | | 115-117 | 39 | CAG | TAA TAG |
| | | | | | 139-141 | 47 | TGG | TAA TAG TGA |
| E2 | | | | | 708-710 | 114 | CGA | TGA TAA |
| E3 | 1085-1087 | 147 | CAG | TAG TAA | 877-879 | 140 | CAA | TAA |
| | 1172-1174 | 176 | CAG | TAG TAA | 940-942 | 161 | CAG | TAG TAA |
| | 1226-1228 | 194 | TGG | TGA TAG TAA | 1027-1029 | 190 | CAG | TAA TAG |
| | 1253-1255 | 203 | CAG | TAA TAG | 1081-1083 | 208 | TGG | TGA TAG TAA |
| | 1286-1288 | 214 | CAG | TAG TAA | 1108-1110 | 217 | CAG | TAG TAA |
| | 1301-1303 | 219 | TGG | TAG TAA TGA | 1141-1143 | 228 | CAG | TAG TAA |
| | 1448-1450 | 268 | CGA | TAA TGA | 1156-1158 | 233 | TGG | TAG TGA TAA |
| | 1469-1471 | 275 | CAG | TAA TAG | 1276-1278 | 273 | CAA | TAA |
| | 1475-1477 | 277 | TGG | TAG TGA TAA | 1324-1326 | 289 | CAG | TAA TAG |
| | 1538-1540 | 298 | CAG | TAG TAA | 1330-1332 | 291 | TGG | TAA TGA TAG |
| | | | | | 1393-1395 | 312 | CAA | TAA |
| | 1705-1707 | 324 | TGG | TGA TAG TAA | 1527-1529 | 329 | CAG | TAA TAG |
| E4 | | | | | | | | |
| | 1723-1725 | 330 | CAG | TAA TAG | 1554-1556 | 338 | TGG | TGA TAG TAA |
| | 1726-1728 | 331 | CAA | TAA | 1572-1574 | 344 | CAA | TAA |
| | 1768-1770 | 345 | TGG | TAG TGA TAA | 1575-1577 | 345 | CAA | TAA |
| | 1807-1809 | 358 | CAA | TAA | 1617-1619 | 359 | TGG | TGA TAA TAG |
| | 1828-1830 | 365 | CAA | TAA | 1653-1655 | 371 | CAA | TAA |
| | 1837-1839 | 368 | CAG | TAG TAA | 1656-1658 | 372 | CAA | TAA |
| | 1846-1848 | 371 | CGA | TAATGA | 1677-1679 | 379 | CAA | TAA |
| | 1849-1851 | 372 | CGA | TGA TAA | 1686-1688 | 382 | CAG | TAG TAA |
| | 1999-2001 | 422 | CAA | TAA | 1848-1850 | 436 | CAG | TAA TAG |
| | 2113-2115 | 460 | CGG | TGA TAA TAG | 1914-1916 | 458 | CGA | TGA TAA |
| | 2146-2148 | 471 | TGG | TAA TGA TAG | 1995-1997 | 485 | TGG | TGA TAG TAA |
| | 2158-2160 | 475 | CAG | TAG TAA | 2007-2009 | 489 | CAG | TAA TAG |
| | 2203-2205 | 490 | CAA | TAA | 2052-2054 | 504 | CAA | TAA |
| | 2224-2226 | 497 | CAG | TAA TAG | 2073-2075 | 511 | CAG | TAG TAA |
| | 2323-2325 | 530 | CGA | TGA TAA | 2181-2183 | 547 | CAG | TAA TAG |
| | 2353-2355 | 540 | TGG | TAA TAG TGA | 2202-2204 | 554 | TGG | TGA TAA TAG |
| | 2455-2457 | 574 | TGG | TAA TAG TGA | 2304-2306 | 588 | TGG | TAA TGA TAG |
| | 2524-2526 | 597 | CAA | TAA | 2370-2372 | 610 | CAG | TAA TAG |
| | 2527-2529 | 598 | CAG | TAG TAA | 2373-2375 | 611 | CAA | TAA |
| | 2530-2532 | 599 | CAG | TAA TAG | | | | |
| | 2536-2538 | 601 | CAA | TAA | | | | |

| | | | | | | | | |
|---|---|---|---|---|---|---|---|---|
| For AtGTR1 the numbers may be increased with 90 nt or 30 AA to make reference to the position taking into account the N-terminal extension of 30 AA. | | | | | | | | |

**Table 2a Potential EMS-induced STOP codon mutations in exon (abbreviated as E) 1, 2, 3 and 4 of BnGTR1-A1 and C1**

| | *BnGTR1-A1* | | | *BnGTR1-C1* | | |
|---|---|---|---|---|---|---|
| | Nucleotide position in SEQ ID NO: 13* | Wildtype codon | → Potential stop codons | Nucleotide position in SEQ ID NO: 19* | Wildtype codon | → Potential stop codons |
| E1 | 103-105 | CAG | TAG TAA | 103-105 | CAG | TAA TAG |
| | 127-129 | TGG | TAA TAG TGA | 127-129 | TGG | TAG TAA TGA |
| E3 | 991-993 | CAG | TAA TAG | 992-994 | CAG | TAG TAA |
| | 1003-1005 | CAA | TAA | 1004-1006 | CAA | TAA |
| | 1021-1023 | CAG | TAA TAG | 1022-1024 | CAG | TAG TAA |
| | 1108-1110 | CAG | TAA TAG | 1109-1111 | CAG | TAA TAG |
| | 1162-1164 | TGG | TAA TAG TGA | 1163-1165 | TGG | TGA TAG TAA |
| | 1189-1191 | CAG | TAA TAG | 1190-1192 | CAG | TAG TAA |
| | 1222-1224 | CAG | TAA TAG | 1223-1225 | CAG | TAA TAG |
| | 1237-1239 | TGG | TGA TAA TAG | 1238-1240 | TGG | TAG TGA TAA |
| | 1405-1407 | CAG | TAG TAA | 1406-1408 | CAG | TAG TAA |
| | 1411-1413 | TGG | TAA TGA TAG | 1412-1414 | TGG | TGA TAG TAA |
| | 1474-1476 | CAG | TAG TAA | 1475-1477 | CAG | TAA TAG |
| E4 | 1632-1634 | TGG | TAA TAG TGA | 1633-1635 | TGG | TAA TGA TAG |
| | 1650-1652 | CAG | TAA TAG | 1651-1653 | CAG | TAA TAG |
| | 1695-1697 | TGG | TAA TGA TAG | 1696-1698 | TGG | TAA TAG TGA |
| | 1734-1736 | CAA | TAA | 1735-1737 | CAA | TAA |
| | 1755-1757 | CAA | TAA | 1756-1758 | CAA | TAA |
| | 1764-1766 | CAG | TAA TAG | 1765-1767 | CAG | TAA TAG |
| | 1776-1778 | CGA | TGA TAA | 1777-1779 | CTA | |
| | 1926-1928 | CAA | TAA | 1927-1929 | CAA | TAA |
| | 2016-2018 | CGA | TAA TGA | 2017-2019 | CGA | TAA TGA |
| | 2040-2042 | CGA | TAA TGA | 2041-2043 | CGA | TGA TAA |
| | 2073-2075 | TGG | TGA TAA TAG | 2074-2076 | TGG | TAG TAA TGA |
| | 2085-2087 | CAG | TAA TAG | 2086-2088 | CAG | TAG TAA |
| | 2130-2132 | CAA | TAA | 2131-2133 | CAA | TAA |
| | 2151-2153 | CAG | TAA TAG | 2152-2154 | CAG | TAA TAG |
| | 2280-2282 | TGG | TAG TAA TGA | 2281-2283 | TGG | TAG TAA TGA |
| | 2382-2384 | TGG | TAG TAA TGA | 2383-2385 | TGG | TAA TAG TGA |
| | 2451-2453 | CAA | TAA | 2452-2454 | CAA | TAA |
| | 2454-2456 | CAA | TAA | 2455-2457 | CAA | TAA |

| | | | | | | |
|---|---|---|---|---|---|---|
| *The corresponding amino acid position in SEQ ID NO: 14 and 20 corresponds to the number of the amino acid encoded by the amino acid codon at the indicated nucleotide positions in SEQ ID NO: 13 and 19, respectively, in the sequence listing. | | | | | | |

For BnGTR1-C1 of SEQ ID No. 137 stopcodons can be induced by EMS mutagenesis at each of the following position: 103-105, 127-129, 1227-1229, 1239-1241, 1257-1259, 1344-1346, 1398-1400, 1425-1427, 1458-1460, 1473-1475, 1641-1643, 1647-1649, 1710-1712, 1868-1870, 1886-1888, 1931-1933, 1970-1972, 1991-1993, 2000-2002, 2162-2164, 2252-2254, 2276-2278, 2309-2311, 2321-2323, 2366-2368, 2387-2389, 2516-2518, 2618-2620, 2687-2689, 2690-2692

**Table 2b Potential EMS-induced STOP codon mutations in exon (abbreviated as E) 1, 2, 3 and 4 of BnGTR1-A2 and C2**

| | *BnGTR1-A2* | | | *BnGTR1-C2* | | |
|---|---|---|---|---|---|---|
| | Nucleotide position in SEQ ID NO: 15 | Wildtype codon | → Potential stop codons | Nucleotide position in SEQ ID NO: 21 | Wildtype codon | → Potential stop codons |
| E1 | 49-51 | CAG | TAG TAA | 49-51 | CAG | TAA TAG |
| | 112-114 | CAG | TAG TAA | 112-114 | CAG | TAA TAG |
| | 136-138 | TGG | TGA TAA TAG | 136-138 | TGG | TAA TGA TAG |
| E2 | 730-732 | CAA | TAA | 703-705 | CAA | TAA |
| E3 | 1068-1070 | CAG | TAG TAA | 1048-1050 | CAG | TAA TAG |
| | 1155-1157 | CAG | TAG TAA | 1135-1137 | CAG | TAG TAA |
| | 1209-1211 | TGG | TAA TAG TGA | 1189-1191 | TGG | TGA TAA TAG |
| | 1236-1238 | CAG | TAG TAA | 1216-1218 | CAG | TAA TAG |
| | 1269-1271 | CAG | TAG TAA | 1249-1251 | CAG | TAG TAA |
| | 1284-1286 | TGG | TAG TGA TAA | 1264-1266 | TGG | TGA TAG TAA |
| | 1431-1433 | CGA | TGA TAA | 1411-1413 | CGA | TAA TGA |
| | 1452-1454 | CAG | TAG TAA | 1432-1434 | CAG | TAG TAA |
| | 1458-1460 | TGG | TGA TAA TAG | 1438-1440 | TGG | TGA TAA TAG |
| | 1521-1523 | CAG | TAATAG | 1501-1503 | CAG | TAG TAA |
| E4 | 1823-1825 | TGG | TAA TAG TGA | 1778-1780 | TGG | TAA TAG TGA |
| | 1841-1843 | CAG | TAG TAA | 1796-1798 | CAG | TAG TAA |
| | 1844-1846 | CAA | TAA | 1799-1801 | CAA | TAA |
| | 1886-1888 | TGG | TAA TAG TGA | 1841-1843 | TGG | TGA TAA TAG |
| | 1925-1927 | CAA | TAA | 1880-1882 | CAA | TAA |
| | 1946-1948 | CAA | TAA | 1901-1903 | CAA | TAA |
| | 1952-1954 | CAA | TAA | 1907-1909 | CAA | TAA |
| | 1955-1957 | CAG | TAA TAG | 1910-1912 | CAG | TAA TAG |
| | 2117-2119 | CAA | TAA | 2072-2074 | CAA | TAA |
| | 2210-2212 | CAG | TAA TAG | 2165-2167 | CAG | TAG TAA |
| | 2231-2233 | CGA | TGA TAA | 2186-2188 | CGA | TGA TAA |
| | 2264-2266 | TGG | TAA TGA TAG | 2219-2221 | TGG | TAG TGA TAA |
| | 2276-2278 | CAG | TAA TAG | 2231-2233 | CAG | TAA TAG |
| | 2321-2323 | CAG | TAA TAG | 2276-2278 | CAG | TAA TAG |
| | 2342-2344 | CAG | TAA TAG | 2297-2299 | CAG | TAG TAA |
| | 2441-2443 | CGG | TAA TGA TAG | 2396-2398 | AGG | |
| | 2471-2473 | TGG | TGA TAG TAA | 2426-2428 | TGG | TAA TGA TAG |
| | 2642-2644 | CAG | TAA TAG | 2597-2599 | CAG | TAG TAA |
| | 2648-2650 | CAA | TAA | 2603-2605 | CAA | TAA |

| | | | | | | |
|---|---|---|---|---|---|---|
| *The corresponding amino acid position in SEQ ID NO: 16 and 22 corresponds to the number of the amino acid encoded by the amino acid codon at the indicated nucleotide positions in SEQ ID NO: 15 and 21, respectively, in the sequence listing. | | | | | | |

**Table 2c Potential EMS-induced STOP codon mutations in exon (abbreviated as E) 1, 2, 3 and 4 of BnGTR1-A3 and C3**

| | *BnGTR1-A3* | | | *BnGTR1-C3* | | |
|---|---|---|---|---|---|---|
| | Nucleotide position in SEQ ID NO: 17 | Wildtype codon | → Potential stop codons | Nucleotide position in SEQ ID NO: 23 | Wildtype codon | → Potential stop codons |
| E1 | 106-108 | CAG | TAA TAG | 106-108 | CAG | TAA TAG |
| | 130-132 | TGG | TAA TGA TAG | 130-132 | TGG | TGA TAG TAA |
| E2 | 579-581 | CAA | TAA | 581-583 | CAA | TAA |
| E3 | 886-888 | CAA | TAA | 887-889 | CAA | TAA |
| | 904-906 | CAG | TAG TAA | 905-907 | CAG | TAG TAA |
| | 991-993 | CAG | TAG TAA | 992-994 | CAG | TAG TAA |
| | 1045-1047 | TGG | TGA TAG TAA | 1046-1048 | TGG | TAA TGA TAG |
| | 1072-1074 | CAG | TAG TAA | 1073-1075 | CAG | TAG TAA |
| | 1105-1107 | CAG | TAA TAG | 1106-1108 | CAG | TAG TAA |
| | 1120-1122 | TGG | TAG TAA TGA | 1121-1123 | TGG | TGA TAA TAG |
| | 1267-1269 | CGA | TAA TGA | 1268-1270 | CGA | TGA TAA |
| | 1357-1359 | CAG | TAA TAG | 1358-1360 | CAG | TAG TAA |
| E4 | 1503-1505 | TGG | TAA TAG TGA | 1512-1514 | TGG | TAA TAG TGA |
| | 1521-1523 | CAG | TAA TAG | 1530-1532 | CAG | TAA TAG |
| | 1524-1526 | CAG | TAA TAG | 1533-1535 | CAG | TAA TAG |
| | 1566-1568 | TGG | TAG TGA TAA | 1575-1577 | TGG | TAA TGA TAG |
| | 1605-1607 | CAA | TAA | 1614-1616 | CAA | TAA |
| | 1626-1628 | CAA | TAA | 1635-1637 | CAA | TAA |
| | 1635-1637 | CAG | TAA TAG | 1644-1646 | CAG | TAA TAG |
| | 1644-1646 | CGG | TGA TAA TAG | 1653-1655 | CGG | TAA TGA TAG |
| | 1797-1799 | CAA | TAA | 1806-1808 | CAA | TAA |
| | 1863-1865 | CGG | TAG TGA TAA | 1872-1874 | CGG | TAA TGA TAG |
| | 1866-1868 | CGA | TGA TAA | 1875-1877 | CGA | TGA TAA |
| | 1911-1913 | CGA | TGA TAA | 1920-1922 | CGA | TAA TGA |
| | 1944-1946 | TGG | TAG TAA TGA | 1953-1955 | TGG | TAG TGA TAA |
| | 1956-1958 | CAG | TAA TAG | 1965-1967 | CAG | TAG TAA |
| | 2001-2003 | CAA | TAA | 2010-2012 | CAA | TAA |
| | 2022-2024 | CAG | TAG TAA | 2031-2033 | CAG | TAA TAG |
| | 2151-2153 | TGG | TGA TAA TAG | 2160-2162 | TGG | TAA TAG TGA |
| | 2328-2330 | CAG | TAG TAA | 2337-2339 | CAG | TAA TAG |
| | 2331-2333 | CAA | TAA | 2340-2342 | CAA | TAA |

| | | | | | | |
|---|---|---|---|---|---|---|
| *The corresponding amino acid position in SEQ ID NO: 18 and 24 corresponds to the number of the amino acid encoded by the amino acid codon at the indicated nucleotide positions in SEQ ID NO: 17 and 23, respectively, in the sequence listing. | | | | | | |

**Table 3a Potential EMS-induced STOP codon mutations in exon (abbreviated as E) 1, 2, 3 and 4 of BnGTR2-A1 and C1**

| | *BnGTR2-A 1* | | | *BnGTR2-C1* | | |
|---|---|---|---|---|---|---|
| | Nucleotide position in SEQ ID NO: 25 | Wildtype codon | → Potential stop codons | Nucleotide position in SEQ ID NO: 31 | Wildtype codon | → Potential stop codons |
| E1 | 40-42 | CAA | TAA | 40-42 | CAA | TAA |
| | 133-135 | TGG | TGA TAA TAG | 133-135 | TGG | TGA TAA TAG |
| E2 | 675-677 | CGA | TGA TAA | 702-704 | CGA | TAA TGA |
| E3 | 848-850 | CAA | TAA | 872-874 | CAA | TAA |
| | 905-907 | CAG | TAA TAG | 929-931^{b} | CAG | TAG TAA |
| | 992-994 | CAG | TAA TAG | 1016-1018 | CAG | TAA TAG |
| | 1022-1024 | CGA | TAA TGA | 1046-1048 | CGA | TAA TGA |
| | 1046-1048 | TGG | TGA TAG TAA | 1070-1072 | TGG | TGA TAA TAG |
| | 1073-1075 | CAG | TAA TAG | 1097-1099 | CAG | TAG TAA |
| | 1106-1108 | CAG | TAA TAG | 1130-1132 | CAG | TAG TAA |
| | 1121-1123 | TGG | TAA TGA TAG | 1145-1147^{c} | TGG | TAG TGA TAA |
| | 1241-1243^{a} | CAA | TAA | 1265-1267 | CAA | TAA |
| | 1295-1297 | TGG | TGA TAA TAG | 1319-1321 | TGG | TAG TAA TGA |
| | 1358-1360 | CAG | TAA TAG | 1382-1384 | CAG | TAG TAA |
| E4 | 1535-1537 | TGG | TAG TAA TGA | 1554-1556 | TGG | TAA TGA TAG |
| | 1553-1555 | CAA | TAA | 1572-1574 | CAA | TAA |
| | 1556-1558 | CAA | TAA | 1575-1577 | CAA | TAA |
| | 1598-1600 | TGG | TAA TGA TAG | 1617-1619 | TGG | TAA TGA TAG |
| | 1634-1636 | CAA | TAA | 1653-1655 | CAA | TAA |
| | 1637-1639 | CAA | TAA | 1656-1658 | CAA | TAA |
| | 1658-1660 | CAA | TAA | 1677-1679 | CAA | TAA |
| | 1667-1669 | CAG | TAG TAA | 1686-1688 | CAG | TAA TAG |
| | 1829-1831 | CAG | TAA TAG | 1848-1850 | CAG | TAG TAA |
| | 1895-1897 | CGG | TAG TAA TGA | 1914-1916 | CGG | TAA TGA TAG |
| | 1976-1978 | TGG | TGA TAA TAG | 1995-1997 | TGG | TAG TGA TAA |
| | 1988-1990 | CAA | TAA | 2007-2009 | CAA | TAA |
| | 2033-2035 | CAG | TAA TAG | 2052-2054 | CAG | TAG TAA |
| | 2054-2056 | CAG | TAA TAG | 2073-2075 | CAA | TAA |
| | 2162-2164 | CAG | TAA TAG | 2181-2183 | CAG | TAA TAG |
| | 2183-2185 | TGG | TGA TAA TAG | 2202-2204 | TGG | TAA TGA TAG |
| | 2285-2287 | TGG | TGA TAA TAG | 2304-2306 | TGG | TAA TGA TAG |
| | 2351-2353 | CAA | TAA | 2370-2372 | CAA | TAA |
| | 2354-2356 | CAA | TAA | 2373-2375 | CAA | TAA |

| | | | | | | |
|---|---|---|---|---|---|---|
| *The corresponding amino acid position in SEQ ID NO: 26 and 32 corresponds to the number of the amino acid encoded by the amino acid codon at the indicated nucleotide positions in SEQ ID NO: 25 and 31, respectively, in the sequence listing. ^{a} BnGTR2-A1-*ems02* allele of the examples ^{b} BnGTR2-C1-*ems01* allele of the examples ^{c} BnGTR2-C1-*ems05* allele of the examples | | | | | | |

**Table 3b Potential EMS-induced STOP codon mutations in exon (abbreviated as E) 1, 2, 3 and 4 of BnGTR2-A2 and C2**

| | *BnGTR2-A2* | | | *BnGTR2-C2* | | |
|---|---|---|---|---|---|---|
| | Nucleotide position in SEQ ID NO: 27 | Wildtype codon | → Potential stop codons | Nucleotide position in SEQ ID NO: 33 | Wildtype codon | → Potential stop codons |
| E1 | 40-42 | CAG | TAATAG | | | |
| | 106-108 | CAG | TAATAG | | | |
| | 133-135 | TGG | TGA TAG TAA | | | |
| E3 | 870-872^{d} | CAA | TAA | 333-335 | CAA | TAA |
| | 927-929 | CAG | TAA TAG | 390-392 | CAG | TAG TAA |
| | 1014-1016 | CAG | TAG TAA | 477-479 | CAG | TAG TAA |
| | 1068-1070 | TGG | TGA TAA TAG | 531-533 | TGG | TAG TAA TGA |
| | 1095-1097 | CAG | TAG TAA | 558-560 | CAG | TAA TAG |
| | 1128-1130 | CAG | TAG TAA | 591-593 | CAG | TAA TAG |
| | 1143-1145 | TGG | TGA TAA TAG | 606-608 | TGG | TAA TGA TAG |
| | 1263-1265 | CAA | TAA | 726-728 | CAA | TAA |
| | 1311-1313 | CAG | TAA TAG | 774-776 | CAG | TAATAG |
| | 1317-1319 | TGG | TAA TGA TAG | 780-782^{f} | TGG | TGA TAA TAG |
| | 1380-1382 ^{e} | CAG | TAG TAA | 843-845 | CAG | TAG TAA |
| E4 | 1532-1534 | TGG | TAG TGA TAA | 994-996 | TGG | TGA TAA TAG |
| | 1550-1552 | CAA | TAA | 1012-1014 | CAA | TAA |
| | 1553-1555 | CAA | TAA | 1015-1017 | CAA | TAA |
| | 1595-1597 | TGG | TGA TAA TAG | 1057-1059 | TGG | TAA TGA TAG |
| | 1631-1633 | CAA | TAA | 1093-1095 | CAA | TAA |
| | 1634-1636 | CAA | TAA | 1096-1098 | CAA | TAA |
| | 1655-1657 | CAA | TAA | 1117-1119 | CAA | TAA |
| | 1664-1666 | CAG | TAA TAG | 1126-1128 | CAG | TAG TAA |
| | 1826-1828 | CAA | TAA | 1288-1290 | CAA | TAA |
| | 1892-1894 | CGG | TAA TAG TGA | 1354-1356 | CGG | TGA TAA TAG |
| | 1940-1942 | CGG | TAG TGA TAA | 1402-1404 | CGG | TGA TAG TAA |
| | 1973-1975 | TGG | TGA TAG TAA | 1435-1437 | TGG | TAG TAA TGA |
| | 1985-1987 | CAG | TAG TAA | 1447-1449 | CAG | TAATAG |
| | 2030-2032 | CAG | TAG TAA | 1492-1494 | CAG | TAATAG |
| | 2051-2053 | CAG | TAA TAG | 1513-1515 | CAG | TAG TAA |
| | 2159-2161 | CAG | TAG TAA | 1621-1623 | CAG | TAG TAA |
| | 2180-2182 | TGG | TAG TAA TGA | 1642-1644 | TGG | TGA TAA TAG |
| | 2282-2284 | TGG | TGA TAA TAG | 1744-1746 | TGG | TAA TAG TGA |
| | 2348-2350 | CAA | TAA | 1810-1812 | CAA | TAA |
| | 2351-2353 | CAA | TAA | 1813-1815 | CAA | TAA |

| | | | | | | |
|---|---|---|---|---|---|---|
| *The corresponding amino acid position in SEQ ID NO: 28 and 34 corresponds to the number of the amino acid encoded by the amino acid codon at the indicated nucleotide positions in SEQ ID NO: 27 and 33, respectively, in the sequence listing. ^{d} BnGTR2-A2-*ems03* allele of the examples ^{e} BnGTR2-A2-*ems09* allele of the examples ^{f} BnGTR2-C2-*ems02* allele of the examples | | | | | | |

**Table 3c Potential EMS-induced STOP codon mutations in exon (abbreviated as E) 1, 2, 3 and 4 of BnGTR2-A3 and C3**

| | *BnGTR2-A3* | | | *BnGTR2-C3* | | |
|---|---|---|---|---|---|---|
| | Nucleotide position in SEQ ID NO: 29 | Wildtype codon | → Potential stop codons | Nucleotide position in SEQ ID NO: 35 | Wildtype codon | → Potential stop codons |
| E1 | 40-42 | CAA | TAA | 40-42 | CAA | TAA |
| | 133-135 | TGG | TAG TAA TGA | 130-132 | TGG | TGA TAA TAG |
| E2 | 512-514 | CGA | TGATAA | 508-510 | CGA | TAATGA |
| E3 | 682-684 | CAG | TAA TAG | 674-676 | CAA | TAA |
| | 739-741 | CAG | TAG TAA | 731-733 | CAG | TAA TAG |
| | 826-828 | CAG | TAA TAG | 818-820 | CAG | TAA TAG |
| | 880-882 | TGG | TAG TGA TAA | 872-874 | TGG | TAG TAA TGA |
| | 907-909 | CAG | TAG TAA | 899-901 | CAG | TAG TAA |
| | 940-942 | CAG | TAA TAG | 932-934 | CAG | TAA TAG |
| | 955-957 | TGG | TAG TAA TGA | 947-949 | TGG | TAA TAG TGA |
| | 1123-1125 | CAG | TAG TAA | 1115-1117 | CAG | TAG TAA |
| | 1129-1131 | TGG | TGA TAA TAG | 1121-1123 | TGG | TAG TAA TGA |
| | 1189-1191 | CAA | TAA | | | |
| | 1192-1194 | CAA | TAA | 1184-1186 | CAA | TAA |
| E4 | 1464-1466 | TGG | TAA TAG TGA | 1528-1530 | TGG | TAA TAG TGA |
| | 1482-1484 | CAA | TAA | 1546-1548 | CAA | TAA |
| | 1485-1487 | CAA | TAA | 1549-1551 | CAG | TAG TAA |
| | 1527-1529 | TGG | TAA TGA TAG | 1591-1593 | TGG | TGA TAG TAA |
| | 1563-1565 | CAA | TAA | 1627-1629 | CAA | TAA |
| | 1566-1568 | CAA | TAA | 1630-1632 | CAA | TAA |
| | 1587-1589 | CAA | TAA | 1651-1653 | CAA | TAA |
| | 1596-1598 | CAG | TAG TAA | 1660-1662 | CAG | TAG TAA |
| | 1758-1760 | CAA | TAA | 1822-1824 | CAA | TAA |
| | 1848-1850 | CAA | TAA | | | |
| | 1872-1874 | CGG | TAA TAG TGA | 1936-1938 | CGG | TAA TAG TGA |
| | 1905-1907 | TGG | TAA TAG TGA | 1969-1971 | TGG | TAG TAA TGA |
| | 1917-1919 | CAG | TAG TAA | 1981-1983 | CAG | TAA TAG |
| | 1962-1964 | CAG | TAG TAA | 2026-2028 | CAG | TAA TAG |
| | 1983-1985 | CAG | TAA TAG | 2047-2049 | CAG | TAG TAA |
| | 2091-2093 | CAG | TAA TAG | 2155-2157 | CAG | TAG TAA |
| | 2112-2114 | TGG | TAA TAG TGA | 2176-2178 | TGG | TAA TAG TGA |
| | 2214-2216 | TGG | TAG TGA TAA | 2278-2280 | TGG | TAG TGA TAA |
| | 2283-2285 | CAA | TAA | 2347-2349 | CAA | TAA |

| | | | | | | |
|---|---|---|---|---|---|---|
| *The corresponding amino acid position in SEQ ID NO: 30 and 36 corresponds to the number of the amino acid encoded by the amino acid codon at the indicated nucleotide positions in SEQ ID NO: 29 and 35, respectively, in the sequence listing. | | | | | | |

**Table 3d Potential EMS-induced STOP codon mutations in exon 1, 2, 3 and 4 of BjGTR2-A1 and B1**

| | *BjGTR2-A1* | | | *BnGTR2-B1* | | |
|---|---|---|---|---|---|---|
| | Nucleotide position in SEQ ID NO: 119 | Wildtype codon | → Potential stop codons | Nucleotide position in SEQ ID NO: 125 | Wildtype codon | → Potential stop codons |
| E1 | 662-664 | CAA | TAA | | | |
| | 755-757 | TGG | TAA TGA TAG | | | |
| E2 | 1294-1296 | CGA | TAA TGA | 177-179 | CGA | TGA TAA |
| E3 | 1523-1525 | CAG | TAG TAA | 353-355 | CAG | TAG TAA |
| | 1610-1612 | CAG | TAG TAA | 410-412 | CAG | TAG TAA |
| | 1640-1642 | CGA | TAA TGA | 497-499 | CAG | TAA TAG |
| | 1664-1666 | TGG | TAG TAA TGA | 551-553 | TGG | TAA TAG TGA |
| | 1691-1693 | CAG | TAA TAG | 578-580 | CAG | TAG TAA |
| | 1724-1726 | CAG | TAA TAG | 611-613 | CAG | TAA TAG |
| | 1739-1741 | TGG | TGA TAG TAA | 626-628 | TGG | TAA TAG TGA |
| | 1859-1861 | CAA | TAA | 746-748 | CAA | TAA |
| | 1913-1915 | TGG | TAA TAG TGA | 794-796 | CAG | TAG TAA |
| | 1976-1978 | CAG | TAA TAG | 800-802 | TGG | TAA TAG TGA |
| | | | | 863-865 | CAG | TAA TAG |
| E4 | 2153-2155 | TGG | TAG TAA TGA | 1036-1038 | TGG | TGA TAG TAA |
| | 2171-2173 | CAA | TAA | 1054-1056 | CAA | TAA |
| | | | | 1057-1059 | CAA | TAA |
| | 2174-2176 | CAA | TAA | 1099-1101 | TGG | TAA TGA TAG |
| | 2216-2218 | TGG | TGA TAG TAA | 1135-1137 | CAA | TAA |
| | 2252-2254 | CAA | TAA | 1138-1140 | CAG | TAA TAG |
| | 2255-2257 | CAA | TAA | 1159-1161 | CAA | TAA |
| | 2276-2278 | CAA | TAA | 1168-1170 | CAG | TAG TAA |
| | 2285-2287 | CAG | TAA TAG | 1330-1332 | CAG | TAA TAG |
| | 2447-2449 | CAG | TAA TAG | 1477-1479 | TGG | TGA TAA TAG |
| | 2513-2515 | CGG | TGA TAG TAA | 1489-1491 | CAA | TAA |
| | 2594-2596 | TGG | TAA TAG TGA | 1534-1536 | CAG | TAG TAA |
| | 2606-2608 | CAA | TAA | 1555-1557 | CAA | TAA |
| | 2651-2653 | CAG | TAG TAA | 1663-1665 | CAG | TAA TAG |
| | 2672-2674 | CAG | TAA TAG | 1684-1686 | TGG | TGA TAA TAG |
| | 2780-2782 | CAG | TAA TAG | 1786-1788 | TGG | TGA TAA TAG |
| | 2801-2803 | TGG | TAA TGA TAG | 1852-1854 | CAA | TAA |
| | 2903-2905 | TGG | TAA TGA TAG | 1855-1857 | CAA | TAA |
| | 2969-2971 | CAA | TAA | | | |
| | 2972-2974 | CAA | TAA | | | |

| | | | | | | |
|---|---|---|---|---|---|---|
| *The corresponding amino acid position in SEQ ID NO: 120 and 126 corresponds to the number of the amino acid encoded by the amino acid codon at the indicated nucleotide positions in SEQ ID NO: 119 and 125, respectively, in the sequence listing. | | | | | | |

**Table 3e Potential EMS-induced STOP codon mutations in exon 1, 2, 3 and 4 of BjGTR2-A2 and B2**

| | *BjGTR2-A2* | | | *BnGTR2-B2* | | |
|---|---|---|---|---|---|---|
| | Nucleotide position in SEQ ID NO: 121 | Wildtype codon | → Potential stop codons | Nucleotide position in SEQ ID NO: 127 | Wildtype codon | → Potential stop codons |
| E1 | 1069-1071 | CAG | TAA TAG | 413-415 | CAG | TAA TAG |
| | 1135-1137 | CAG | TAG TAA | 467-469 | CAA | TAA |
| | 1162-1164 | TGG | TAA TAG TGA | 479-481 | CAG | TAA TAG |
| | | | | 506-508 | TGG | TAA TAG TGA |
| E2 | | | | | | |
| E3 | 1892-1894 | CAA | TAA | 1334-1336 | CAA | TAA |
| | 1949-1951 | CAG | TAG TAA | 1391-1393 | CAG | TAA TAG |
| | 2036-2038 | CAG | TAG TAA | 1478-1480 | CAG | TAA TAG |
| | 2090-2092 | TGG | TAA TAG TGA | 1532-1534 | TGG | TAA TAG TGA |
| | 2117-2119 | CAG | TAA TAG | 1559-1561 | CAG | TAG TAA |
| | 2150-2152 | CAG | TAA TAG | 1592-1594 | CAG | TAA TAG |
| | 2165-2167 | TGG | TAA TGA TAG | 1607-1609 | TGG | TAA TAG TGA |
| | 2285-2287 | CAA | TAA | 1727-1729 | CAA | TAA |
| | 2333-2335 | CAG | TAA TAG | 1775-1777 | CAG | TAG TAA |
| | 2339-2341 | TGG | TAG TAA TGA | 1781-1783 | TGG | TAA TGA TAG |
| | 2402-2404 | CAG | TAA TAG | 1844-1846 | CAG | TAG TAA |
| E4 | 2555-2557 | TGG | TAG TAA TGA | 2011-2013 | TGG | TGA TAG TAA |
| | 2573-2575 | CAA | TAA | 2029-2031 | CAA | TAA |
| | 2576-2578 | CAA | TAA | 2032-2034 | CAA | TAA |
| | 2618-2620 | TGG | TGA TAG TAA | 2074-2076 | TGG | TGA TAG TAA |
| | 2654-2656 | CAA | TAA | 2110-2112 | CAA | TAA |
| | 2657-2659 | CAA | TAA | 2113-2115 | CAG | TAA TAG |
| | 2678-2680 | CAA | TAA | 2134-2136 | CAA | TAA |
| | 2687-2689 | CAG | TAG TAA | 2143-2145 | CAG | TAA TAG |
| | 2849-2851 | CAA | TAA | 2305-2307 | CAG | TAG TAA |
| | 2915-2917 | CGG | TAG TGA TAA | 2371-2373 | CGG | TGA TAA TAG |
| | 2963-2965 | CGG | TGA TAA TAG | 2419-2421 | CGG | TAG TAA TGA |
| | 2996-2998 | TGG | TAA TGA TAG | 2452-2454 | TGG | TAG TGA TAA |
| | 3008-3010 | CAG | TAA TAG | 2464-2466 | CAG | TAA TAG |
| | 3053-3055 | CAG | TAA TAG | 2509-2511 | CAG | TAG TAA |
| | 3074-3076 | CAG | TAA TAGH | 2530-2532 | CAG | TAG TAA |
| | 3182-3184 | CAG | TAG TAA | 2638-2640 | CAG | TAG TAA |
| | 3203-3205 | TGG | TGA TAG TAA | 2659-2661 | TGG | TGA TAG TAA |
| | 3305-3307 | TGG | TGA TAA TAG | 2761-2763 | TGG | TAG TGA TAA |
| | 3371-3373 | CAA | TAA | 2827-2829 | CAA | TAA |
| | 3374-3376 | CAA | TAA | 2830-2832 | CAA | TAA |

| | | | | | | |
|---|---|---|---|---|---|---|
| *The corresponding amino acid position in SEQ ID NO: 122 and 128 corresponds to the number of the amino acid encoded by the amino acid codon at the indicated nucleotide positions in SEQ ID NO: 121 and 127, respectively, in the sequence listing. | | | | | | |

**Table 3f Potential EMS-induced STOP codon mutations in exon 1, 2, 3 and 4 of BjGTR2-A3 and B3**

| | *BjGTR2-A3* | | | *BnGTR2-B3* | | |
|---|---|---|---|---|---|---|
| | Nucleotide position in SEQ ID NO: 123 | Wildtype codon | → Potential stop codons | Nucleotide position in SEQ ID NO: 129 | Wildtype codon | → Potential stop codons |
| E1 | 72-74 | CAA | TAA | 829-831 | CAA | TAA |
| | 138-140 | CAG | TAG TAA | 877-879 | CAG | TAA TAG |
| | 165-167 | TGG | TAG TAA TGA | 904-906 | TGG | TAA TAG TGA |
| E2 | 543-545 | CGA | TGA TAA | 1357-1359 | CGA | TAA TGA |
| E3 | 711-713 | CAA | TAA | 1518-1520 | CAA | TAA |
| | 768-770 | CAG | TAG TAA | 1575-1577 | CAG | TAA TAG |
| | 855-857 | CAG | TAA TAG | 1662-1664 | CAG | TAA TAG |
| | 909-911 | TGG | TAA TGA TAG | 1716-1718 | TGG | TAA TAG TGA |
| | 936-938 | CAG | TAG TAA | 1743-1745 | CAG | TAG TAA |
| | 969-971 | CAG | TAA TAG | 1776-1778 | CAG | TAG TAA |
| | 984-986 | TGG | TAG TGA TAA | 1791-1793 | TGG | TAA TAG TGA |
| | 1152-1154 | CAG | TAA TAG | 1959-1961 | CAG | TAG TAA |
| | 1158-1160 | TGG | TAG TAA TGA | 1965-1967 | TGG | TAA TGA TAG |
| | 1221-1223 | CAA | TAA | 2028-2030 | CAA | TAA |
| | | | | | | |
| E4 | 1496-1498 | TGG | TAA TAG TGA | 2297-2299 | TGG | TGA TAA TAG |
| | 1514-1516 | CAA | TAA | 2315-2317 | CAA | TAA |
| | 1517-1519 | CAA | TAA | 2318-2320 | CAA | TAA |
| | 1559-1561 | TGG | TGA TAA TAG | 2396-2398 | CAA | TAA |
| | 1595-1597 | CAA | TAA | 2399-2401 | CAA | TAA |
| | 1598-1600 | CAA | TAA | 2420-2422 | CAA | TAA |
| | 1619-1621 | CAA | TAA | 2429-2431 | CAG | TAG TAA |
| | 1628-1630 | CAG | TAG TAA | 2591-2593 | CAA | TAA |
| | 1790-1792 | CAA | TAA | 2705-2707 | CGG | TGA TAA TAG |
| | 1904-1906 | CGG | TGA TAA TAG | 2738-2740 | TGG | TAA TAG TGA |
| | 1937-1939 | TGG | TAG TGA TAA | 2750-2752 | CAG | TAG TAA |
| | 1949-1951 | CAG | TAA TAG | 2795-2797 | CAG | TAG TAA |
| | 1994-1996 | CAG | TAA TAG | 2816-2818 | CAG | TAA TAG |
| | 2015-2017 | CAG | TAA TAG | 2924-2926 | CAG | TAG TAA |
| | 2123-2125 | CAG | TAA TAG | 2945-2947 | TGG | TAA TGA TAG |
| | 2144-2146 | TGG | TGA TAG TAA | 3047-3049 | TGG | TGA TAG TAA |
| | 2246-2248 | TGG | TAG TGA TAA | 3116-3118 | CAA | TAA |
| | 2315-2317 | CAA | TAA | | | |

| | | | | | | |
|---|---|---|---|---|---|---|
| *The corresponding amino acid position in SEQ ID NO: 124 and 130 corresponds to the number of the amino acid encoded by the amino acid codon at the indicated nucleotide positions in SEQ ID NO: 123 and 129, respectively, in the sequence listing. | | | | | | |

Obviously, mutations are not limited to the ones shown in the above tables and it is understood that analogous STOP codon mutations may be present in *gtr* alleles other than those depicted in the sequence listing and referred to in the tables above.

A missense mutation in a *GTR* allele, as used herein, is any mutation (deletion, insertion or substitution) in a *GTR* allele whereby one or more codons are changed into the coding DNA and the corresponding mRNA sequence of the corresponding wild type *GTR* allele, resulting in the substitution of one or more amino acids in the wild type GTR protein with one or more other amino acids in the mutant GTR protein. A mutant *GTR* allele comprising a missense mutation can be a *GTR* allele wherein one or more of the conserved amino acids indicated above is/are substituted. A mutant *GTR* allele comprising a missense mutation can also be a *GTR* allele encoding a GTR protein wherein an amino acid at a position corresponding to position 126, 145, 192 or 359 in SEQ ID NO: 66 is substituted, such as those indicated in Table 7 of Example 3 below.

A frameshift mutation in a *GTR* allele, as used herein, is a mutation (deletion, insertion, duplication, and the like) in a *GTR* allele that results in the nucleic acid sequence being translated in a different frame downstream of the mutation. As indicated above splice site mutations can result in frameshifts. Possible EMS-induced splice site mutations in *Arabidopsis* and *Brassica GTR* sequences are those which result in a mutation at the GU-donor site at the 5' splice site or at the AG-acceptor site at the 3' splice site indicated in the sequence listing, for example which result in a G/C to A/T transition in these sites.

### Amino acid sequences

Described herein are both wild type (functional) GTR amino acid sequences and mutant GTR amino acid sequences (comprising one or more mutations, preferably mutations which result in a significantly reduced or no GSL transport activity of the GTR protein) from *Brassicaceae,* particularly from *Brassica* species, especially from *Brassica napus,* but also from other *Brassica* crop species. For example, *Brassica* species comprising an A and/or a C or a B genome may encode different GTR-A, GTR-B or GTR-C amino acids. In addition, mutagenesis methods can be used to generate mutations in wild type *GTR* alleles, thereby generating mutant alleles which can encode further mutant GTR proteins. The wild type and/or mutant GTR amino acid sequences can be present within a *Brassica* plant (i.e. endogenously). However, isolated GTR amino acid sequences (e.g.. isolated from the plant or made synthetically), as well as variants thereof and fragments of any of these are also described herein.

"GTR amino acid sequence" or "GTR variant amino acid sequence" as used herein are amino acid sequences having at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 55%, at least 56%, at least 57%, at least 58%, at least 59%, at least 60%, at least 61%, at least 62%, at least 63%, at least 64%, at least 65%, at least 70%, at least 75%, at least 76%, at least 77%, at least 78%, at least 79%, at least 80%, at least 85%, at least 90%, at least 95%, 98%, 99% or 100% sequence identity with SEQ ID NO: 2. These amino acid sequences may also be referred to as being "essentially similar" or "essentially identical" to the *GTR* sequences provided in the sequence listing.

Amino acid sequences of GTR1 to 6 proteins have been isolated from *Arabidopsis,* of GTRx-Ay proteins from *B*. *rapa, B. juncea* and from *B. napus* and of GTRx-Cy proteins from *B. oleracea* and from *B*. *napus* and of GTRx-By proteins from *B. juncea* as depicted in the sequence listing. The wild type GTR amino acid sequences are depicted, while mutant sequences of these sequences, and of sequences essentially similar to these, are described herein below and in the Examples, with reference to the wild type GTR amino acid sequences.

"GTR1, 2, 3, 4, 5 or 6 amino acid sequences" or "GTR1, 2, 3, 4, 5 or 6 variant amino acid sequences" as used herein are amino acid sequences having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, 96%, 97%, 98%, 99% or 100% sequence identity with SEQ ID NO: 2, 4, 6, 8, 10 or 12, respectively. These amino acid sequences may also be referred to as being "essentially similar" or "essentially identical" to the GTR sequences provided in the sequence listing.

Thus, described herein are both amino acid sequences of wild type, functional GTR1, 2, 3, 4, 5 or 6 proteins, including variants and fragments thereof (as defined further below), as well as mutant amino acid sequences of any of these, whereby the mutation in the amino acid sequence preferably results in a significant reduction in or a complete abolishment of the GSL transport activity of the GTR protein as compared to the GSL transport activity of the corresponding wild type GTR protein. A significant reduction in or complete abolishment of the GSL transport activity of the GTR protein refers herein to a reduction in or abolishment of the GSL transport activity of the GTR protein, such that the GSL content is modified in specific parts of a plant expressing the mutant GTR protein as compared to a plant expressing the corresponding wild type GTR protein.

Both endogenous and isolated amino acid sequences are described herein. Also described are fragments of the GTR amino acid sequences and GTR variant amino acid sequences defined above. A "fragment" of a GTR amino acid sequence or variant thereof (as defined) may be of various lengths, such as at least 10, 12, 15, 18, 20, 50, 100, 150, 175, 180 contiguous amino acids of the GTR sequence (or of the variant sequence).

### Amino acid sequences of functional GTR proteins

The amino acid sequences depicted in the sequence listing encode wild type, functional GTR proteins from *Arabidopsis, B. rapa, B. oleracea, B. juncea* and *B*. *napus.* Thus, these sequences are endogenous to the plants from which they were isolated. Other *Brassicaceae* plants, including *Brassica* crop species, varieties, breeding lines or wild accessions, may be screened for other functional GTR proteins with the same amino acid sequences or variants thereof, as described above.

In addition, it is understood that GTR amino acid sequences and variants thereof (or fragments of any of these) may be identified *in silico,* by screening amino acid databases for essentially similar sequences. Fragments of amino acid molecules are also described. Fragments include amino acid sequences of conserved and functional domains as indicated above.

### Amino acid sequences of mutant GTR proteins

Amino acid sequences comprising one or more amino acid deletions, insertions or substitutions relative to the wild type amino acid sequences are described herein, as are fragments of such mutant amino acid molecules. Such mutant amino acid sequences can be generated and/or identified using various known methods, as described above. Again, such amino acid molecules can be present both in endogenous form and in isolated form.

The mutation(s) in the amino acid sequence can result in a significantly reduced or completely abolished GSL transport activity of the GTR protein relative to the wild type protein. As described above, basically, any mutation which results in a protein comprising at least one amino acid insertion, deletion and/or substitution relative to the wild type protein can lead to significantly reduced or no GSL transport activity. It is, however, understood that mutations in certain parts of the protein are more likely to result in a reduced function of the mutant GTR protein, such as mutations leading to truncated proteins, whereby significant portions of the functional domains, such as transmembrane domains or substrate binding domains, are lacking or are being substituted. A mutant GTR protein comprising a missense mutation in a transmembrane domain can be a GTR protein wherein an amino acid at a position corresponding to position 126 or to position 359 in SEQ ID NO: 66 is substituted, such as the mutant GTR protein indicated in Table 7 of Example 3 below.

Thus, mutant GTR proteins can comprise one or more deletion or insertion mutations, whereby the deletion(s) or insertion(s) result(s) in a mutant protein which has significantly reduced or no activity in vivo. Such mutant GTR proteins are GTR proteins wherein at least 1, at least 2, 3, 4, 5, 10, 20, 30, 50, 100, 100, 150, 175, 180 or more amino acids are deleted or inserted as compared to the wild type GTR protein, whereby the deletion(s) or insertion(s) result(s) in a mutant protein which has significantly reduced or no activity in vivo.

Mutant GTR proteins can also be truncated whereby the truncation results in a mutant protein that has significantly reduced or no activity in vivo. Such truncated GTR proteins are GTR proteins which lack functional domains in the C-terminal part of the corresponding wild type GTR protein and which maintain the N-terminal part of the corresponding wild type GTR protein. The more truncated the mutant protein is in comparison to the wild type protein, the more the truncation may result in a significantly reduced or no activity of the GTR protein.

Mutant GTR proteins may also comprise one or more substitution mutations, whereby the substitution(s) result(s) in a mutant protein that has significantly reduced or no activity in vivo. Such mutant GTR proteins are GTR proteins whereby conserved amino acid residues which have a specific function, such as a function in substrate or proton binding, are substituted.

Also disclosed are variant GTR proteins which are changed in their phosphorylation/dephosphorylation status. Examples of such proteins are GTR1 or GTR2 proteins as herein described wherein the Serine or Threonine residues of phosphorylation sites are substituted with Aspartic acid (mimicking constitutive phosphorylation at that site) or with Alanine (mimicking constitutive dephosphorylation at that site) such as the GTR1 protein comprising the amino acid sequence of SEQ ID NO: 2 with the following substitutions:
a. S at position 22 with A
b. S at position 22 with D
c. T at position 105 with A
d. T at position 105 with D
e. S at position 605 with A
f. S at position 605 with D
or the GTR1 protein comprising the amino acid sequence of SEQ ID NO: 142 with the following substitutions:
g. S at position 52 with A
h. S at position 52 with D
i. T at position 135 with A
j. T at position 135 with D
k. S at position 635 with A
I. S at position 635 with D
or a GTR2 protein comprising the amino acid sequence of SEQ ID No: 4 with the following substitutions:
m. T at position 58 with A
n. T at position 58 with D
o. T at position 117 with A
p. T at position 117 with D
q. T at position 323 with A
r. T at position 323 with D
Corresponding substitutions may be made in the GTR1 and GTR2 proteins from Brassica species, such as the ones described herein.

### Methods according to the invention

Mutant *gtr* alleles may be generated (for example induced by mutagenesis) and/or identified using a range of methods, which are conventional in the art, for example using PCR based methods to amplify part or all of the *gtr* genomic or cDNA.

Following mutagenesis, plants are grown from the treated seeds, or regenerated from the treated cells using known techniques. For instance, mutagenized seeds may be planted in accordance with conventional growing procedures and following self-pollination seed is formed on the plants. Alternatively, doubled haploid plantlets may be extracted from treated microspore or pollen cells to immediately form homozygous plants, for example as described by Coventry et al. (1988, Manual for Microspore Culture Technique for Brassica napus. Dep. Crop Sci. Techn. Bull. OAC Publication 0489. Univ. of Guelph, Guelph, Ontario, Canada). Additional seed which is formed as a result of such self-pollination in the present or a subsequent generation may be harvested and screened for the presence of mutant *GTR* alleles, using techniques which are conventional in the art, for example polymerase chain reaction (PCR) based techniques (amplification of the *gtr* alleles) or hybridization based techniques, e.g. Southern blot analysis, BAC library screening, and the like, and/or direct sequencing of *gtr* alleles. Several techniques are known to screen for specific mutant alleles, e.g., Deleteagene^{™} (Delete-a-gene; Li et al., 2001, Plant J 27: 235-242) uses polymerase chain reaction (PCR) assays to screen for deletion mutants generated by fast neutron mutagenesis, TILLING (targeted induced local lesions in genomes; McCallum et al., 2000, Nat Biotechnol 18:455-457) identifies EMS-induced point mutations, etc. To screen for the presence of point mutations (so called Single Nucleotide Polymorphisms or SNPs) in mutant *GTR* alleles, SNP detection methods conventional in the art can be used, for example oligoligation-based techniques, single base extension-based techniques or techniques based on differences in restriction sites, such as TILLING.

As described above, mutagenization (spontaneous as well as induced) of a specific wild-type *GTR* allele results in the presence of one or more deleted, inserted, or substituted nucleotides (hereinafter called "mutation region") in the resulting mutant *GTR* allele. The mutant *GTR* allele can thus be characterized by the location and the configuration of the one or more deleted, inserted, or substituted nucleotides in the wild type *GTR* allele. The site in the wild type *GTR* allele where the one or more nucleotides have been inserted, deleted, or substituted, respectively, is herein also referred to as the "mutation region or sequence". A "5' or 3' flanking region or sequence" as used herein refers to a DNA region or sequence in the mutant (or the corresponding wild type) *GTR* allele of at least 20 bp, preferably at least 50 bp, at least 750 bp, at least 1500 bp, and up to 5000 bp of DNA different from the DNA containing the one or more deleted, inserted, or substituted nucleotides, preferably DNA from the mutant (or the corresponding wild type) *GTR* allele which is located either immediately upstream of and contiguous with (5' flanking region or sequence") or immediately downstream of and contiguous with (3' flanking region or sequence") the mutation region in the mutant *GTR* allele (or in the corresponding wild type *GTR* allele). A "joining region" as used herein refers to a DNA region in the mutant (or the corresponding wild type) *GTR* allele where the mutation region and the 5' or 3' flanking region are linked to each other. A "sequence spanning the joining region between the mutation region and the 5' or 3' flanking region thus comprises a mutation sequence as well as the flanking sequence contiguous therewith.

The tools developed to identify a specific mutant *GTR* allele or the plant or plant material comprising a specific mutant *GTR* allele, or products which comprise plant material comprising a specific mutant *GTR* allele are based on the specific genomic characteristics of the specific mutant *GTR* allele as compared to the genomic characteristics of the corresponding wild type *GTR* allele, such as, a specific restriction map of the genomic region comprising the mutation region, molecular markers or the sequence of the flanking and/or mutation regions.

Once a specific mutant allele has been sequenced, primers and probes can be developed which specifically recognize a sequence within the 5' flanking, 3' flanking and/or mutation regions of the mutant *GTR* allele in the nucleic acid (DNA or RNA) of a sample by way of a molecular biological technique. For instance a PCR method can be developed to identify the mutant *GTR* allele in biological samples (such as samples of plants, plant material or products comprising plant material). Such a PCR is based on at least two specific "primers": one recognizing a sequence within the 5' or 3' flanking region of the mutant *GTR* allele and the other recognizing a sequence within the 3' or 5' flanking region of the mutant *GTR* allele, respectively; or one recognizing a sequence within the 5' or 3' flanking region of the mutant *GTR* allele and the other recognizing a sequence within the mutation region of the mutant *GTR* allele; or one recognizing a sequence within the 5' or 3' flanking region of the mutant *GTR* allele and the other recognizing a sequence spanning the joining region between the 3' or 5' flanking region and the mutation region of the specific mutant *GTR* allele (as described further below), respectively.

The primers preferably have a sequence of between 15 and 35 nucleotides which under optimized PCR conditions "specifically recognize" a sequence within the 5' or 3' flanking region, a sequence within the mutation region, or a sequence spanning the joining region between the 3' or 5' flanking and mutation regions of the specific mutant *GTR* allele, so that a specific fragment ("mutant *GTR* specific fragment" or discriminating amplicon) is amplified from a nucleic acid sample comprising the specific mutant *GTR* allele. This means that only the targeted mutant *GTR,* allele, and no other sequence in the plant genome, is amplified under optimized PCR conditions.

PCR primers suitable for the invention may be the following:
- oligonucleotides ranging in length from 17 nt to about 200 nt, comprising a nucleotide sequence of at least 17 consecutive nucleotides, preferably 20 consecutive nucleotides selected from the 5' or 3' flanking sequence of a specific mutant *GTR* allele or the complement thereof (i.e., for example, the sequence 5' or 3' flanking the one or more nucleotides deleted, inserted or substituted in the mutant *GTR* alleles, such as the sequence 5' or 3' flanking the non-sense, mis-sense or frameshift mutations described above or the sequence 5' or 3' flanking the STOP codon mutations indicated in the above Tables or the substitution mutations indicated above or the complement thereof) (primers recognizing 5' flanking sequences); or
- oligonucleotides ranging in length from 17 nt to about 200 nt, comprising a nucleotide sequence of at least 17 consecutive nucleotides, preferably 20 nucleotides selected from the sequence of the mutation region of a specific mutant *GTR* allele or the complement thereof (i.e., for example, the sequence of nucleotides inserted or substituted in the *GTR* genes as described herein or the complement thereof) (primers recognizing mutation sequences).

The primers may of course be longer than the mentioned 17 consecutive nucleotides, and may e.g. be 18, 19, 20, 21, 30, 35, 50, 75, 100, 150, 200 nt long or even longer. The primers may entirely consist of nucleotide sequence selected from the mentioned nucleotide sequences of flanking and mutation sequences. However, the nucleotide sequence of the primers at their 5' end (i.e. outside of the 3'-located 17 consecutive nucleotides) is less critical. Thus, the 5' sequence of the primers may consist of a nucleotide sequence selected from the flanking or mutation sequences, as appropriate, but may contain several (e.g. 1, 2, 5, 10) mismatches. The 5' sequence of the primers may even entirely consist of a nucleotide sequence unrelated to the flanking or mutation sequences, such as e.g. a nucleotide sequence representing restriction enzyme recognition sites. Such unrelated sequences or flanking DNA sequences with mismatches should preferably be not longer than 100, more preferably not longer than 50 or even 25 nucleotides.

Moreover, suitable primers may comprise or consist of a nucleotide sequence spanning the joining region between flanking and mutation sequences (i.e., for example, the joining region between a sequence 5' or 3' flanking one or more nucleotides deleted, inserted or substituted in the mutant *GTR* alleles and the sequence of the one or more nucleotides inserted or substituted or the sequence 3' or 5', respectively, flanking the one or more nucleotides deleted, such as the joining region between a sequence 5' or 3' flanking non-sense, missense or frameshift mutations in the *GTR* genes described above and the sequence of the non-sense, missense or frameshift mutations, or the joining region between a sequence 5' or 3' flanking a potential STOP codon mutation as indicated in the above Tables or the substitution mutations indicated above and the sequence of the potential STOP codon mutation or the substitution mutations, respectively), provided the nucleotide sequence is not derived exclusively from either the mutation region or flanking regions.

It will also be immediately clear to the skilled artisan that properly selected PCR primer pairs should also not comprise sequences complementary to each other.

For the purpose of the invention, the "complement of a nucleotide sequence represented in SEQ ID No: X" is the nucleotide sequence which can be derived from the represented nucleotide sequence by replacing the nucleotides through their complementary nucleotide according to Chargaff's rules (A↔T; G↔C) and reading the sequence in the 5' to 3' direction, i.e. in opposite direction of the represented nucleotide sequence.

Examples of primers suitable to identify specific mutant *GTR* alleles are described in the Examples.

As used herein, "the nucleotide sequence of SEQ ID No. Z from position X to position Y" indicates the nucleotide sequence including both nucleotide endpoints.

Preferably, the amplified fragment has a length of between 50 and 1000 nucleotides, such as a length between 50 and 500 nucleotides, or a length between 100 and 350 nucleotides. The specific primers may have a sequence which is between 80 and 100% identical to a sequence within the 5' or 3' flanking region, to a sequence within the mutation region, or to a sequence spanning the joining region between the 3' or 5' flanking and mutation regions of the specific mutant *GTR* allele, provided the mismatches still allow specific identification of the specific mutant GTR allele with these primers under optimized PCR conditions. The range of allowable mismatches however, can easily be determined experimentally and are known to a person skilled in the art.

Detection and/or identification of a "mutant *GTR* specific fragment" can occur in various ways, e.g., via size estimation after gel or capillary electrophoresis or via fluorescence-based detection methods. The mutant *GTR* specific fragments may also be directly sequenced. Other sequence specific methods for detection of amplified DNA fragments are also known in the art.

Standard PCR protocols are described in the art, such as in 'PCR Applications Manual" (Roche Molecular Biochemicals, 2nd Edition, 1999) and other references. The optimal conditions for the PCR, including the sequence of the specific primers, is specified in a "PCR identification protocol" for each specific mutant *GTR* allele. It is however understood that a number of parameters in the PCR identification protocol may need to be adjusted to specific laboratory conditions, and may be modified slightly to obtain similar results. For instance, use of a different method for preparation of DNA may require adjustment of, for instance, the amount of primers, polymerase, MgCl₂ concentration or annealing conditions used. Similarly, the selection of other primers may dictate other optimal conditions for the PCR identification protocol. These adjustments will however be apparent to a person skilled in the art, and are furthermore detailed in current PCR application manuals such as the one cited above.

Examples of PCR identification protocols to identify specific mutant GTR alleles are described in the Examples.

Alternatively, specific primers can be used to amplify a mutant *GTR* specific fragment that can be used as a "specific probe" for identifying a specific mutant *GTR* allele in biological samples. Contacting nucleic acid of a biological sample, with the probe, under conditions that allow hybridization of the probe with its corresponding fragment in the nucleic acid, results in the formation of a nucleic acid/probe hybrid. The formation of this hybrid can be detected (e.g. labeling of the nucleic acid or probe), whereby the formation of this hybrid indicates the presence of the specific mutant *GTR* allele. Such identification methods based on hybridization with a specific probe (either on a solid phase carrier or in solution) have been described in the art. The specific probe is preferably a sequence that, under optimized conditions, hybridizes specifically to a region within the 5' or 3' flanking region and/or within the mutation region of the specific mutant *GTR* allele (hereinafter referred to as "mutant *GTR* specific region"). Preferably, the specific probe comprises a sequence of between 10 and 1000 bp, 50 and 600 bp, between 100 to 500 bp, between 150 to 350bp, which is at least 80%, preferably between 80 and 85%, more preferably between 85 and 90%, especially preferably between 90 and 95%, most preferably between 95% and 100% identical (or complementary) to the nucleotide sequence of a specific region. Preferably, the specific probe will comprise a sequence of about 13 to about 100 contiguous nucleotides identical (or complementary) to a specific region of the specific mutant *GTR* allele.

Specific probes suitable for the invention may be the following:
- oligonucleotides ranging in length from 13 nt to about 1000 nt, comprising a nucleotide sequence of at least 13 consecutive nucleotides selected from the 5' or 3' flanking sequence of a specific mutant *GTR* allele or the complement thereof (i.e., for example, the sequence 5' or 3' flanking the one or more nucleotides deleted, inserted or substituted in the mutant *GTR* alleles as described herein, such as the sequence 5' or 3' flanking the non-sense, mis-sense or frameshift mutations described above or the sequence 5' or 3' flanking the potential STOP codon mutations indicated in the above Tables or the substitution mutations indicated above), or a sequence having at least 80% sequence identity therewith (probes recognizing 5' flanking sequences); or
- oligonucleotides ranging in length from 13 nt to about 1000 nt, comprising a nucleotide sequence of at least 13 consecutive nucleotides selected from the mutation sequence of a specific mutant *GTR* allele or the complement thereof (i.e., for example, the sequence of nucleotides inserted or substituted in the *GTR* genes as described herein, or the complement thereof), or a sequence having at least 80% sequence identity therewith (probes recognizing mutation sequences).

The probes may entirely consist of nucleotide sequence selected from the mentioned nucleotide sequences of flanking and mutation sequences. However, the nucleotide sequence of the probes at their 5' or 3' ends is less critical. Thus, the 5' or 3' sequences of the probes may consist of a nucleotide sequence selected from the flanking or mutation sequences, as appropriate, but may consist of a nucleotide sequence unrelated to the flanking or mutation sequences. Such unrelated sequences should preferably be not longer than 50, more preferably not longer than 25 or even not longer than 20 or 15 nucleotides.

Moreover, suitable probes may comprise or consist of a nucleotide sequence spanning the joining region between flanking and mutation sequences (i.e., for example, the joining region between a sequence 5' or 3' flanking one or more nucleotides deleted, inserted or substituted in the mutant *GTR* alleles as described herein and the sequence of the one or more nucleotides inserted or substituted or the sequence 3' or 5', respectively, flanking the one or more nucleotides deleted, such as the joining region between a sequence 5' or 3' flanking non-sense, mis-sense or frameshift mutations in the *GTR* genes as described herein described above and the sequence of the non-sense, mis-sense or frameshift mutations, or the joining region between a sequence 5' or 3' flanking a potential STOP codon mutation as indicated in the above Tables or the substitution mutations indicated above and the sequence of the potential STOP codon or substitution mutation, respectively), provided the mentioned nucleotide sequence is not derived exclusively from either the mutation region or flanking regions.

Examples of specific probes suitable to identify specific mutant *GTR* alleles are described in the Examples.

Detection and/or identification of a "mutant *GTR* specific region" hybridizing to a specific probe can occur in various ways, e.g., via size estimation after gel electrophoresis or via fluorescence-based detection methods. Other sequence specific methods for detection of a "mutant GTR specific region" hybridizing to a specific probe are also known in the art.

Alternatively, plants or plant parts comprising one or more mutant *gtr* alleles can be generated and identified using other methods, such as the "Delete-a-gene^{™}" method which uses PCR to screen for deletion mutants generated by fast neutron mutagenesis (reviewed by Li and Zhang, 2002, Funct Integr Genomics 2:254-258), by the TILLING (Targeting Induced Local Lesions IN Genomes) method which identifies EMS-induced point mutations using denaturing high-performance liquid chromatography (DHPLC) to detect base pair changes by heteroduplex analysis (McCallum et al., 2000, Nat Biotech 18:455, and McCallum et al. 2000, Plant Physiol. 123, 439-442), etc. As mentioned, TILLING uses high-throughput screening for mutations (e.g. using Cel 1 cleavage of mutant-wildtype DNA heteroduplexes and detection using a sequencing gel system). Thus, the use of TILLING to identify plants or plant parts comprising one or more mutant *gtr* alleles and methods for generating and identifying such plants, plant organs, tissues and seeds is encompassed herein. Thus in one embodiment, the method according to the invention comprises the steps of mutagenizing plant seeds (e.g. EMS mutagenesis), pooling of plant individuals or DNA, PCR amplification of a region of interest, heteroduplex formation and high-throughput detection, identification of the mutant plant, sequencing of the mutant PCR product. It is understood that other mutagenesis and selection methods may equally be used to generate such mutant plants.

Instead of inducing mutations in GTR alleles, natural (spontaneous) mutant alleles may be identified by methods known in the art. For example, ECOTILLING may be used (Henikoff et al. 2004, Plant Physiology 135(2):630-6) to screen a plurality of plants or plant parts for the presence of natural mutant *gtr* alleles. As for the mutagenesis techniques above, preferably *Brassica* species are screened which comprise an A and/or a C genome, so that the identified *gtr* allele can subsequently be introduced into other *Brassica* species, such as *Brassica napus,* by crossing (inter- or intraspecific crosses) and selection. In ECOTILLING natural polymorphisms in breeding lines or related species are screened for by the TILLING methodology described above, in which individual or pools of plants are used for PCR amplification of the *gtr* target, heteroduplex formation and high-throughput analysis. This can be followed by selecting individual plants having a required mutation that can be used subsequently in a breeding program to incorporate the desired mutant allele.

The identified mutant alleles can then be sequenced and the sequence can be compared to the wild type allele to identify the mutation(s). Optionally functionality can be tested as indicated above. Using this approach a plurality of mutant *gtr* alleles (and *Brassica* plants comprising one or more of these) can be identified. The desired mutant alleles can then be combined with the desired wild type alleles by crossing and selection methods as described further below. Finally a single plant comprising the desired number of mutant *gtr* and the desired number of wild type *GTR* alleles is generated.

Oligonucleotides suitable as PCR primers or specific probes for detection of a specific mutant *GTR* allele can also be used to develop methods to determine the zygosity status of the specific mutant *GTR* allele.

To determine the zygosity status of a specific mutant *GTR* allele, a PCR-based assay can be developed to determine the presence of a mutant and/or corresponding wild type GTR specific allele:

To determine the zygosity status of a specific mutant *GTR* allele, two primers specifically recognizing the wild-type *GTR* allele can be designed in such a way that they are directed towards each other and have the mutation region located in between the primers. These primers may be primers specifically recognizing the 5' and 3' flanking sequences, respectively. This set of primers allows simultaneous diagnostic PCR amplification of the mutant, as well as of the corresponding wild type *GTR* allele.

Alternatively, to determine the zygosity status of a specific mutant *GTR* allele, two primers specifically recognizing the wild-type *GTR* allele can be designed in such a way that they are directed towards each other and that one of them specifically recognizes the mutation region. These primers may be primers specifically recognizing the sequence of the 5' or 3' flanking region and the mutation region of the wild type *GTR* allele, respectively. This set of primers, together with a third primer which specifically recognizes the sequence of the mutation region in the mutant *GTR* allele, allow simultaneous diagnostic PCR amplification of the mutant *GTR* gene, as well as of the wild type *GTR* gene.

Alternatively, to determine the zygosity status of a specific mutant *GTR* allele, two primers specifically recognizing the wild-type *GTR* allele can be designed in such a way that they are directed towards each other and that one of them specifically recognizes the joining region between the 5' or 3' flanking region and the mutation region. These primers may be primers specifically recognizing the 5' or 3' flanking sequence and the joining region between the mutation region and the 3' or 5' flanking region of the wild type *GTR* allele, respectively. This set of primers, together with a third primer which specifically recognizes the joining region between the mutation region and the 3' or 5' flanking region of the mutant *GTR* allele, respectively, allow simultaneous diagnostic PCR amplification of the mutant *GTR* gene, as well as of the wild type *GTR* gene.

Alternatively, the zygosity status of a specific mutant *GTR* allele can be determined by using alternative primer sets that specifically recognize mutant and wild type *GTR* alleles.

If the plant is homozygous for the mutant *GTR* gene or the corresponding wild type GTR gene, the diagnostic PCR assays described above will give rise to a single PCR product typical, preferably typical in length, for either the mutant or wild type *GTR* allele. If the plant is heterozygous for the mutant *GTR* allele, two specific PCR products will appear, reflecting both the amplification of the mutant and the wild type *GTR* allele.

Identification of the wild type and mutant *GTR* specific PCR products can occur e.g. by size estimation after gel or capillary electrophoresis (e.g. for mutant *GTR* alleles comprising a number of inserted or deleted nucleotides which results in a size difference between the fragments amplified from the wild type and the mutant *GTR* allele, such that said fragments can be visibly separated on a gel); by evaluating the presence or absence of the two different fragments after gel or capillary electrophoresis, whereby the diagnostic PCR amplification of the mutant *GTR* allele can, optionally, be performed separately from the diagnostic PCR amplification of the wild type *GTR* allele; by direct sequencing of the amplified fragments; or by fluorescence-based detection methods.

Examples of primers suitable to determine the zygosity of specific mutant *GTR* alleles are described in the Examples.

Alternatively, to determine the zygosity status of a specific mutant *GTR* allele, a hybridization-based assay can be developed to determine the presence of a mutant and/or corresponding wild type *GTR* specific allele:

To determine the zygosity status of a specific mutant *GTR* allele, two specific probes recognizing the wild-type *GTR* allele can be designed in such a way that each probe specifically recognizes a sequence within the *GTR* wild type allele and that the mutation region is located in between the sequences recognized by the probes. These probes may be probes specifically recognizing the 5' and 3' flanking sequences, respectively. The use of one or, preferably, both of these probes allows simultaneous diagnostic hybridization of the mutant, as well as of the corresponding wild type *GTR* allele.

Alternatively, to determine the zygosity status of a specific mutant *GTR* allele, two specific probes recognizing the wild-type *GTR* allele can be designed in such a way that one of them specifically recognizes a sequence within the *GTR* wild type allele upstream or downstream of the mutation region, preferably upstream of the mutation region, and that one of them specifically recognizes the mutation region. These probes may be probes specifically recognizing the sequence of the 5' or 3' flanking region, preferably the 5' flanking region, and the mutation region of the wild type *GTR* allele, respectively. The use of one or, preferably, both of these probes, optionally, together with a third probe which specifically recognizes the sequence of the mutation region in the mutant *GTR* allele, allow diagnostic hybridization of the mutant and of the wild type *GTR* gene.

Alternatively, to determine the zygosity status of a specific mutant *GTR* allele, a specific probe recognizing the wild-type *GTR* allele can be designed in such a way that the probe specifically recognizes the joining region between the 5' or 3' flanking region, preferably the 5' flanking region, and the mutation region of the wild type *GTR* allele. This probe, optionally, together with a second probe that specifically recognizes the joining region between the 5' or 3' flanking region, preferably the 5' flanking region, and the mutation region of the mutant *GTR* allele, allows diagnostic hybridization of the mutant and of the wild type *GTR* gene.

Alternatively, the zygosity status of a specific mutant *GTR* allele can be determined by using alternative sets of probes that specifically recognize mutant and wild type *GTR* alleles.

If the plant is homozygous for the mutant *GTR* gene or the corresponding wild type *GTR* gene, the diagnostic hybridization assays described above will give rise to a single specific hybridization product, such as one or more hybridizing DNA (restriction) fragments, typical, preferably typical in length, for either the mutant or wild type *GTR* allele. If the plant is heterozygous for the mutant *GTR* allele, two specific hybridization products will appear, reflecting both the hybridization of the mutant and the wild type *GTR* allele.

Identification of the wild type and mutant *GTR* specific hybridization products can occur e.g. by size estimation after gel or capillary electrophoresis (e.g. for mutant *GTR* alleles comprising a number of inserted or deleted nucleotides which results in a size difference between the hybridizing DNA (restriction) fragments from the wild type and the mutant *GTR* allele, such that said fragments can be visibly separated on a gel); by evaluating the presence or absence of the two different specific hybridization products after gel or capillary electrophoresis, whereby the diagnostic hybridization of the mutant *GTR* allele can, optionally, be performed separately from the diagnostic hybridization of the wild type *GTR* allele; by direct sequencing of the hybridizing DNA (restriction) fragments; or by fluorescence-based detection methods.

Examples of probes suitable to determine the zygosity of specific mutant *GTR* alleles are described in the Examples.

Furthermore, detection methods specific for a specific mutant *GTR* allele that differ from PCR- or hybridization-based amplification methods can also be developed using the specific mutant *GTR* allele specific sequence information provided herein. Such alternative detection methods include linear signal amplification detection methods based on invasive cleavage of particular nucleic acid structures, also known as Invader™ technology, (as described e.g. in US patent 5,985,557 "Invasive Cleavage of Nucleic Acids", 6,001,567 "Detection of Nucleic Acid sequences by Invader Directed Cleavage), RT-PCR-based detection methods, such as Taqman, or other detection methods, such as SNPlex. Briefly, in the Invader™ technology, the target mutation sequence may e.g. be hybridized with a labeled first nucleic acid oligonucleotide comprising the nucleotide sequence of the mutation sequence or a sequence spanning the joining region between the 5' flanking region and the mutation region and with a second nucleic acid oligonucleotide comprising the 3' flanking sequence immediately downstream and adjacent to the mutation sequence, wherein the first and second oligonucleotide overlap by at least one nucleotide. The duplex or triplex structure that is produced by this hybridization allows selective probe cleavage with an enzyme (Cleavase®) leaving the target sequence intact. The cleaved labeled probe is subsequently detected, potentially via an intermediate step resulting in further signal amplification.

A "kit", as used herein, refers to a set of reagents for the purpose of performing the method as described herein, more particularly, the identification of a specific mutant *GTR* allele in biological samples or the determination of the zygosity status of plant material comprising a specific mutant *GTR* allele. More particularly, a suitable kit comprises at least two specific primers, as described above, for identification of a specific mutant *GTR* allele, or at least two or three specific primers for the determination of the zygosity status. Optionally, the kit can further comprise any other reagent described herein in the PCR identification protocol. Alternatively, the kit can comprise at least one specific probe, which specifically hybridizes with nucleic acid of biological samples to identify the presence of a specific mutant *GTR* allele therein, as described above, for identification of a specific mutant *GTR* allele, or at least two or three specific probes for the determination of the zygosity status. Optionally, the kit can further comprise any other reagent (such as but not limited to hybridizing buffer, label) for identification of a specific mutant *GTR* allele in biological samples, using the specific probe.

The kit as described herein can be used, and its components can be specifically adjusted, for purposes of quality control (e.g., purity of seed lots), detection of the presence or absence of a specific mutant *GTR* allele in plant material or material comprising or derived from plant material, such as but not limited to food or feed products.

The term "primer" as used herein encompasses any nucleic acid that is capable of priming the synthesis of a nascent nucleic acid in a template-dependent process, such as PCR. Typically, primers are oligonucleotides from 10 to 30 nucleotides, but longer sequences can be employed. Primers may be provided in double-stranded form, though the single-stranded form is preferred. Probes can be used as primers, but are designed to bind to the target DNA or RNA and need not be used in an amplification process.

The term "recognizing" as used herein when referring to specific primers, refers to the fact that the specific primers specifically hybridize to a nucleic acid sequence in a specific mutant *GTR* allele under the conditions set forth in the method (such as the conditions of the PCR identification protocol), whereby the specificity is determined by the presence of positive and negative controls.

The term "hybridizing", as used herein when referring to specific probes, refers to the fact that the probe binds to a specific region in the nucleic acid sequence of a specific mutant *GTR* allele under standard stringency conditions. Standard stringency conditions as used herein refers to the conditions for hybridization described herein or to the conventional hybridizing conditions as described by Sambrook et al., 1989 (Molecular Cloning: A Laboratory Manual, Second Edition, Cold Spring Harbour Laboratory Press, NY) which for instance can comprise the following steps: 1) immobilizing plant genomic DNA fragments or BAC library DNA on a filter, 2) prehybridizing the filter for 1 to 2 hours at 65°C in 6 X SSC, 5 X Denhardt's reagent, 0.5% SDS and 20 µg/ml denaturated carrier DNA, 3) adding the hybridization probe which has been labeled, 4) incubating for 16 to 24 hours, 5) washing the filter once for 30 min. at 68°C in 6X SSC, 0.1 %SDS, 6) washing the filter three times (two times for 30 min. in 30ml and once for 10 min in 500ml) at 68°C in 2 X SSC, 0.1 %SDS, and 7) exposing the filter for 4 to 48 hours to X-ray film at -70°C.

As used in herein, a "biological sample" is a sample of a plant, plant material or product comprising plant material. The term "plant" is intended to encompass plant tissues, at any stage of maturity, as well as any cells, tissues, or organs taken from or derived from any such plant, including without limitation, any seeds, leaves, stems, flowers, roots, single cells, gametes, cell cultures, tissue cultures or protoplasts. "Plant material", as used herein refers to material that is obtained or derived from a plant. Products comprising plant material relate to food, feed or other products that are produced using plant material or can be contaminated by plant material. It is understood that, in the context of the present invention, such biological samples are tested for the presence of nucleic acids specific for a specific mutant *GTR* allele, implying the presence of nucleic acids in the samples. Thus the methods referred to herein for identifying a specific mutant *GTR* allele in biological samples, relate to the identification in biological samples of nucleic acids that comprise the specific mutant *GTR* allele.

Specific *GTR* alleles may also be combined in one plant, to the transfer of one or more specific mutant *GTR* allele(s) from one plant to another plant, to the plants comprising one or more specific mutant *GTR* allele(s), the progeny obtained from these plants and to plant cells, plant parts, and plant seeds derived from these plants.

Thus, suitable is a method for combining two or more selected mutant *GTR* alleles in one plant comprising the steps of:
(a) generating and/or identifying two or more plants each comprising one or more selected mutant *GTR* alleles, as described above,
(b) crossing a first plant comprising one or more selected mutant *GTR* alleles with a second plant comprising one or more other selected mutant *GTR* alleles, collecting F1 seeds from the cross, and, optionally, identifying an F1 plant comprising one or more selected mutant *GTR* alleles from the first plant with one or more selected mutant *GTR* alleles from the second plant, as described above,
(c) optionally, repeating step (b) until an F1 plant comprising all selected mutant *GTR* alleles is obtained,
(d) optionally,
   - identifying an F1 plant, which is homozygous or heterozygous for a selected mutant *GTR* allele by determining the zygosity status of the mutant *GTR* alleles, as described above, or
   - generating plants which are homozygous for one or more of the selected mutant *GTR* alleles by performing one of the following steps:
      - extracting doubled haploid plants from treated microspore or pollen cells of F1 plants comprising the one or more selected mutant *GTR* alleles, as described above,
      - selfing the F1 plants comprising the one or more selected mutant *GTR* allele(s) for one or more generations (y), collecting F1 Sy seeds from the selfings, and identifying F1 Sy plants, which are homozygous for the one or more mutant *GTR* allele, as described above.

Also suitable is a method for transferring one or more mutant *GTR* alleles from one plant to another plant is provided comprising the steps of:
(a) generating and/or identifying a first plant comprising one or more selected mutant *GTR* alleles, as described above, or generating the first plant by combining the one or more selected mutant *GTR* alleles in one plant, as described above (wherein the first plant is homozygous or heterozygous for the one or more mutant *GTR* alleles),
(b) crossing the first plant comprising the one or more mutant *GTR* alleles with a second plant not comprising the one or more mutant *GTR* alleles, collecting F1 seeds from the cross (wherein the seeds are heterozygous for a mutant *GTR* allele if the first plant was homozygous for that mutant *GTR* allele, and wherein half of the seeds are heterozygous and half of the seeds are azygous for, i.e. do not comprise, a mutant *GTR* allele if the first plant was heterozygous for that mutant *GTR* allele), and, optionally, identifying F1 plants comprising one or more selected mutant *GTR* alleles, as described above,
(c) backcrossing F1 plants comprising one or more selected mutant *GTR* alleles with the second plant not comprising the one or more selected mutant *GTR* alleles for one or more generations (x), collecting BCx seeds from the crosses, and identifying in every generation BCx plants comprising the one or more selected mutant *GTR* alleles, as described above,
(d) optionally, generating BCx plants which are homozygous for the one or more selected mutant *GTR* alleles by performing one of the following steps:
   - extracting doubled haploid plants from treated microspore or pollen cells of BCx plants comprising the one or more desired mutant *GTR* allele(s), as described above,
   - selfing the BCx plants comprising the one or more desired mutant *GTR* allele(s) for one or more generations (y), collecting BCx Sy seeds from the selfings, and identifying BCx Sy plants, which are homozygous for the one or more desired mutant *GTR* allele, as described above.

The first and the second plant may be *Brassicaceae* plants, particularly *Brassica* plants, especially *Brassica napus* plants or plants from another *Brassica* crop species, such as *Brassica rapa, B. juncea* or *Brassica oleracea.* The first plant may also be a *Brassicaceae* plant, particularly a *Brassica* plant, especially a *Brassica napus* plant or a plant from another *Brassica* crop species, and the second plant is a plant from a *Brassicaceae* breeding line, particularly from a *Brassica* breeding line, especially from a *Brassica napus* breeding line or from a breeding line from another *Brassica* crop species. "Breeding line", as used herein, is a preferably homozygous plant line distinguishable from other plant lines by a preferred genotype and/or phenotype that is used to produce hybrid offspring.

The inventors further found that seeds of *Arabidopsis* plants knocked out in either GTR1 or GTR2 transporters had no significant reduction and about 50% reduction in total aliphatic GSLs concentrations, respectively, compared to wildtype plants, and that seeds of *Arabidopsis* plants knocked out in both GTR1 and GTR2 transporters had a zero GSL seed phenotype. In addition, GSLs levels were decreased in inflorescences and in root tissue of gtr knockout plants compared to GSLs levels in these tissues in wildtype plants. Surprisingly, GSLs levels in senescent leaves from gtr knockout plants were high whereas, in wildtype plants, leaves become depleted in GSLs upon aging. In addition, GSLs levels in silique walls of gtr knockout plants increased compared to wildtype plants. Similar observations were made in *B*. *rapa.*

Thus, the inventors found that *Brassicaceae* plants, wherein the GTR activity is reduced, in particular the GTR2 activity or the GTR1 and GTR2 activity, have a decreased to undetectable GSL content in seed, inflorescence tissue and root tissue, while the levels of GSLs in green tissues (such as rosette leaves, cauline leaves, silique walls) remain high. The observations indicate that the GTR proteins as disclosed herein, in particular the GTR1 and GRT2 proteins, are essential components of a transport pathway involved in the transport of GSLs from green tissues, such as rosette leaves, cauline leaves and silique walls (so-called "source" tissues), into seeds, flowers and, at a certain period in the plant's life cycle, into the roots (so-called "sink" tissues).

Suitable to the invention is a method to modify GSL transport in eukaryotic cells or organisms, such as plants, comprising modifying the functional GTR activity in said eukaryotic cells or organisms.

In one embodiment, the invention provides a method to modify the GSL content in *Brassicales* plants and plant parts comprising reducing the functional GTR activity in said plant or plant parts.

Modification of the GSL content in plants and plant parts enables modifications to be made, for example, to meal quality of oilseeds crucifers, cancer preventive activity and flavour of horticultural crucifers, and/or resistance to herbivores and pathogens and biofumigative activity.

In one aspect, the GSL content is decreased in *Brassicales* plant seed by reducing the functional GTR activity.

As used herein, "seed" comprises embryo, endosperm and/or seed coat.

The GSL content may also be increased or maintained in green plant tissue by reducing the functional GTR activity.

As used herein, "green plant tissue" refers to leaves, rosette leaves, cauline leaves and silique walls.

In yet another aspect, the GSL content is decreased in *Brassicales* plant seed and increased in green plant tissue by reducing the functional GTR activity.

In one embodiment of the invention, the plant is a plant from the *Brassicales* order having a high content of GSLs. Also suitable are plants from the *Capparales* order. Non-limiting examples of such plants are: plants of the *Akaniaceae* family, the *Bataceae* family, the *Brassicaceae* or *Cruciferae* family, the *Capparaceae* family, the *Caricaceae* family, the *Gyrostemonaceae* family, the *Koeberliniaceae* family, the *Limnanthaceae* family, the *Moringaceae* family, the *Pentadiplandraceae* family, the *Resedaceae* family, the *Salvadoraceae* family, the *Setchellanthaceae* family, the *Tovariaceae* family and the *Tropaeolaceae* family.

In a specific embodiment of the invention, the plant is a *Brassica* plant. The plant can be a *Brassica napus* plant (such as rapeseed, canola and rutabaga), a *Brassica rapa* plant (such as Chinese cabbage and turnip), a *Brassica oleracea* plant (such as kale, cabbage, broccoli, cauliflower, Brussels sprouts and kohlrabi), a *Brassica carinata* plant (Abyssinian mustard), a *Brassica juncea* plant (Indian mustard) or a *Brassica nigra* plant (black mustard).

The most important crops for modification of seed meal quality by, e.g., reducing the GSL content in seed, are oilseed forms of *Brassica* spp. (e.g. *B. napus, B. rapa* (syn *B*. *campestris), B. juncea* and *B. carinata).*

For enhancement of flavour and cancer preventive properties by, e.g., increasing the GSL content in green plant tissues, the most important species are *B. oleracea* (including e.g. broccoli and cauliflower), horticultural forms of *B. napus* (e.g. swedes [=rutabaga, spp. *napobrassica],* oil seed rape) and *B. rapa* (including both turnips and Chinese cabbage [= pakchois]), cruciferous salads (including e.g. *Eruca sativa* and *Diplotaxis tenuifolia*) and horticultural forms of *Raphanus* (e.g. radish *(Raphanus sativa*)).

GSL content can be modified by reducing functional GTR activity. As used herein, "functional GTR activity" in a plant or plant part refers to the GTR activity as present in said plant or plant part. Functional GTR activity is the result of *GTR* gene expression level and GTR activity. Accordingly, the functional GTR activity in a plant or plant part can be reduced by down-regulating *GTR* gene expression level or by down-regulating GTR activity, or both and, according to the invention, the modification of GSL content of a plant, plant tissue, plant organ, plant part, or plant cell can be achieved by down-regulation of *GTR* gene expression level, by down-regulation of GTR activity, or both.

Conveniently, *GTR* gene expression level or GTR activity is controlled genetically by introduction of chimeric genes altering the *GTR* gene expression level and/or by introduction of chimeric genes altering the GTR activity and/or by alteration of the endogenous GTR-encoding genes.

in accordance with the invention, it is preferred that in order to modify GSL content, the functional GTR activity is reduced significantly. Preferably, the functional GTR activity in the target cells should be decreased about 75%, preferably about 80%, particularly about 90%, more particularly about 95%, more preferably about 100% of the normal level and/or activity in the target cells. Methods to determine the content of a specific protein such as the GTR proteins are well known to the person skilled in the art and include, but are not limited to (histochemical) quantification of such proteins using specific antibodies. A method to quantify GTR activity is described in the Examples below.

Thus in one embodiment of the invention, a method for modifying the GSL content of a plant or plant part comprises the step of down-regulating *GTR* gene expression. In another embodiment of the invention, a method for modifying the GSL content of a plant, plant tissue, plant organ, plant part, or plant cell comprises down-regulating GTR activity.

In an embodiment of the invention, *GTR* gene expression is down-regulated by introducing a chimeric DNA construct in the plant or plant part comprising the following operably linked DNA regions:
a) a plant-expressible promoter which functions in the plant or plant part;
b) a DNA region which when transcribed yields a GTR-inhibitory RNA molecule capable of down-regulating *GTR* gene expression; and
c) a DNA region involved in transcription termination and polyadenylation, functional in plant cells.

The transcribed DNA region encodes a biologically active RNA which decreases the levels of GTR mRNAs available for translation. This can be achieved through well established techniques including co-suppression (sense RNA suppression), antisense RNA, double-stranded RNA (dsRNA), or microRNA (miRNA).

*GTR* gene expression may be down-regulated by introducing a chimeric DNA construct which yields a sense RNA molecule capable of down-regulating *GTR* gene expression by co-suppression. The transcribed DNA region will yield upon transcription a so-called sense RNA molecule capable of reducing the expression of a *GTR* gene in the target plant or plant cell in a transcriptional or post-transcriptional manner. The transcribed DNA region (and resulting RNA molecule) comprises at least 20 consecutive nucleotides having at least 95% sequence identity to the nucleotide sequence of a GTR-encoding gene present in the plant cell or plant.

*GTR* gene expression may also be down-regulated by introducing a chimeric DNA construct which yields an antisense RNA molecule capable of down-regulating *GTR* gene expression. The transcribed DNA region will yield upon transcription a so-called antisense RNA molecule capable of reducing the expression of a *GTR* gene in the target plant or plant cell in a transcriptional or post-transcriptional manner. The transcribed DNA region (and resulting RNA molecule) comprises at least 20 consecutive nucleotides having at least 95% sequence identity to the complement of the nucleotide sequence of a GTR-encoding gene present in the plant cell or plant.

However, the minimum nucleotide sequence of the antisense or sense RNA region of about 20 nt of the GTR-encoding region may be comprised within a larger RNA molecule, varying in size from 20 nt to a length equal to the size of the target gene. The mentioned antisense or sense nucleotide regions may thus be about from about 21 nt to about 5000 nt long, such as 21 nt, 40 nt, 50 nt, 100 nt, 200 nt, 300 nt, 500 nt, 1000 nt, 2000 nt or even about 5000 nt or larger in length. Moreover, it is not required for the purpose of the invention that the nucleotide sequence of the used inhibitory GTR RNA molecule or the encoding region of the transgene, is completely identical or complementary to the endogenous *GTR* gene the expression of which is targeted to be reduced in the plant cell. The longer the sequence, the less stringent the requirement for the overall sequence identity is. Thus, the sense or antisense regions may have an overall sequence identity of about 40 % or 50 % or 60 % or 70 % or 80 % or 90 % or 100 % to the nucleotide sequence of the endogenous *GTR* gene or the complement thereof. However, as mentioned, antisense or sense regions should comprise a nucleotide sequence of 20 consecutive nucleotides having about 95 to about 100% sequence identity to the nucleotide sequence of the endogenous GTR gene. The stretch of about 95 to about 100% sequence identity may be about 50, 75 or 100 nt.

The efficiency of the above mentioned chimeric genes for antisense RNA or sense RNA-mediated gene expression level down-regulation may be further enhanced by inclusion of DNA elements which result in the expression of aberrant, non-polyadenylated *GTR* inhibitory RNA molecules. One such DNA element suitable for that purpose is a DNA region encoding a self-splicing ribozyme, as described in WO 00/01133. The efficiency may also be enhanced by providing the generated RNA molecules with nuclear localization or retention signals as described in WO 03/076619.

*GTR* gene expression may also be down-regulated by introducing a chimeric DNA construct which yields a double-stranded RNA molecule capable of down-regulating *GTR* gene expression. Upon transcription of the DNA region the RNA is able to form dsRNA molecule through conventional base paring between a sense and antisense region, whereby the sense and antisense region are nucleotide sequences as hereinbefore described. dsRNA-encoding *GTR* expression-reducing chimeric genes according to the invention may further comprise an intron, such as a heterologous intron, located e.g. in the spacer sequence between the sense and antisense RNA regions in accordance with the disclosure of WO 99/53050. To achieve the construction of such a transgene, use can be made of the vectors described in WO 02/059294 A1.

*GTR* gene expression may also be down-regulated by introducing a chimeric DNA construct which yields a pre-miRNA RNA molecule which is processed into a miRNA capable of guiding the cleavage of GTR mRNA. miRNAs are small endogenous RNAs that regulate gene expression in plants, but also in other eukaryotes. In plants, these about 21 nucleotide long RNAs are processed from the stem-loop regions of long endogenous pre-miRNAs by the cleavage activity of DICERLIKE1 (DCL1). Plant miRNAs are highly complementary to conserved target mRNAs, and guide the cleavage of their targets. miRNAs appear to be key components in regulating the gene expression of complex networks of pathways involved inter alia in development.

As used herein, a "miRNA" is an RNA molecule of about 20 to 22 nucleotides in length which can be loaded into a RISC complex and direct the cleavage of a target RNA molecule, wherein the target RNA molecule comprises a nucleotide sequence essentially complementary to the nucleotide sequence of the miRNA molecule whereby one or more of the following mismatches may occur:
- A mismatch between the nucleotide at the 5' end of said miRNA and the corresponding nucleotide sequence in the target RNA molecule;
- A mismatch between any one of the nucleotides in position 1 to position 9 of said miRNA and the corresponding nucleotide sequence in the target RNA molecule;
- Three mismatches between any one of the nucleotides in position 12 to position 21 of said miRNA and the corresponding nucleotide sequence in the target RNA molecule provided that there are no more than two consecutive mismatches.
No mismatch is allowed at positions 10 and 11 of the miRNA (all miRNA positions are indicated starting from the 5' end of the miRNA molecule).

As used herein, a "pre-miRNA" molecule is an RNA molecule of about 100 to about 200 nucleotides, preferably about 100 to about 130 nucleotides which can adopt a secondary structure comprising a dsRNA stem and a single stranded RNA loop and further comprising the nucleotide sequence of the miRNA and its complement sequence of the miRNA* in the double-stranded RNA stem. Preferably, the miRNA and its complement are located about 10 to about 20 nucleotides from the free ends of the miRNA dsRNA stem. The length and sequence of the single stranded loop region are not critical and may vary considerably, e.g. between 30 and 50 nt in length. Preferably, the difference in free energy between unpaired and paired RNA structure is between -20 and -60 kcal/mole, particularly around -40 kcal/mole. The complementarity between the miRNA and the miRNA* do not need to be perfect and about 1 to 3 bulges of unpaired nucleotides can be tolerated. The secondary structure adopted by an RNA molecule can be predicted by computer algorithms conventional in the art such as mFold, UNAFold and RNAFold. The particular strand of the dsRNA stem from the pre-miRNA which is released by DCL activity and loaded onto the RISC complex is determined by the degree of complementarity at the 5' end, whereby the strand which at its 5' end is the least involved in hydrogen bounding between the nucleotides of the different strands of the cleaved dsRNA stem is loaded onto the RISC complex and will determine the sequence specificity of the target RNA molecule degradation. However, if empirically the miRNA molecule from a particular synthetic pre-miRNA molecule is not functional because the "wrong" strand is loaded on the RISC complex, it will be immediately evident that this problem can be solved by exchanging the position of the miRNA molecule and its complement on the respective strands of the dsRNA stem of the pre-miRNA molecule. As is known in the art, binding between A and U involving two hydrogen bounds, or G and U involving two hydrogen bounds is less strong that between G and C involving three hydrogen bounds.

miRNA molecules may be comprised within their naturally occurring pre-miRNA molecules but they can also be introduced into existing pre-miRNA molecule scaffolds by exchanging the nucleotide sequence of the miRNA molecule normally processed from such existing pre-miRNA molecule for the nucleotide sequence of another miRNA of interest. The scaffold of the pre-miRNA can also be completely synthetic. Likewise, synthetic miRNA molecules may be comprised within, and processed from, existing pre-miRNA molecule scaffolds or synthetic pre-miRNA scaffolds.

GTR protein activity may be down-regulated by introducing a chimeric DNA construct in the plant, plant tissue, plant organ, plant part, or plant cell, comprising the following operably linked DNA regions:
a) a promoter, operative in the plant, plant tissue, plant organ, plant part, or plant cell;
b) a DNA region which when transcribed yields a GTR-inhibitory RNA molecule capable of down-regulating GTR activity; and
c) a DNA region involved in transcription termination and polyadenylation.

The GTR-inhibitory RNA molecule capable of down-regulating endogenous GTR protein activity may be an RNA molecule which can be translated into a biologically active protein capable of decreasing the levels of GTR activity. This can be achieved, e.g., inactivating antibodies to GTR proteins. "Inactivating antibodies to GTR proteins" are antibodies or parts thereof which specifically bind at least to some epitopes of GTR proteins, such as the substrate/proton binding domain or the conserved domains described above, or which trap the transport protein in a conformation that does not allow transport (as described e.g. in JBC 274(22): 15420-15426, 1999) and which inhibit the activity of the target protein.

The chimeric DNA construct used to reduce the functional GTR activity by down-regulation of *GTR* gene expression level and/or by down-regulation of GTR protein activity can be stably inserted in a conventional manner into the nuclear genome of a single plant cell, and the so-transformed plant cell can be used in a conventional manner to produce a transformed plant with modified GSL content. In this regard, a T-DNA vector, containing the chimeric DNA construct used to reduce the functional GTR activity, in *Agrobacterium tumefaciens* can be used to transform the plant cell, and thereafter, a transformed plant can be regenerated from the transformed plant cell using the procedures described, for example, in EP0116718, EP0270822, WO84/02913 and published European Patent application EP 0 242 246 and in Gould et al. (1991). The construction of a T-DNA vector for *Agrobacterium* mediated plant transformation is well known in the art. The T-DNA vector may be either a binary vector as described in EP0120561 and EP0120515 or a cointegrate vector which can integrate into the *Agrobacterium* Ti-plasmid by homologous recombination, as described in EP0116718. Preferred T-DNA vectors each contain a promoter operably linked to the transcribed DNA region between T-DNA border sequences, or at least located to the left of the right border sequence. Border sequences are described in Gielen et al. (1984). Introduction of the T-DNA vector into *Agrobacterium* can be carried out using known methods, such as electroporation or triparental mating. Of course, other types of vectors can be used to transform the plant cell, using procedures such as direct gene transfer (as described, for example in EP0223247), pollen mediated transformation (as described, for example in EP0270356 and WO85/01856), protoplast transformation as, for example, described in US4,684,611, plant RNA virus-mediated transformation (as described, for example in EP0067553 and US4,407,956), liposome-mediated transformation (as described, for example in US4,536,475), and other methods The resulting transformed plant can be used in a conventional plant breeding scheme to produce more transformed plants with modifying total GSL content.

In another embodiment of the invention, the functional activity of GTR may be reduced by modification of the nucleotide sequence of the endogenous *GTR* genes by generating a mutant *gtr* allele comprising one or more mutations in its nucleic acid sequence, whereby the mutations result in a significantly reduced amount of functional GTR protein in the cell *in vivo.* The *GTR* gene expression-regulating sequences may be altered so that the *GTR* gene expression levels are down-regulated.

Methods to achieve such a modification of endogenous *GTR* genes include homologous recombination to exchange the endogenous *GTR* genes for mutant *GTR* genes e.g. by the methods described in US patent 5,527,695. In a preferred embodiment such site-directed modification of the nucleotide sequence of the endogenous *GTR* genes is achieved by introduction of chimeric DNA/RNA oligonucleotides as described in WO 96/22364 or US patent 5,565,350.

Methods to achieve such a modification of endogenous *GTR* genes also include mutagenesis. It will be immediately clear to the skilled artisan, that mutant plant cells and plant lines, wherein the functional GTR activity is reduced may be used to the same effect as the transgenic plant cells and plant lines described herein. Mutants in *GTR* gene of a plant cell or plant may be easily identified using screening methods known in the art, whereby chemical mutagenesis, such as e.g., EMS mutagenesis, is combined with sensitive detection methods (such as e.g., denaturing HPLC). An example of such a technique is the so-called "Targeted Induced Local Lesions in Genomes" method as described in McCallum et al, Plant Physiology 123 439-442 or WO 01/75167. However, other methods to detect mutations in particular genome regions or even alleles, are also available and include screening of libraries of existing or newly generated insertion mutant plant lines, whereby pools of genomic DNA of these mutant plant lines are subjected to PCR amplification using primers specific for the inserted DNA fragment and primers specific for the genomic region or allele, wherein the insertion is expected (see e.g. Maes et al., 1999, Trends in Plant Science, 4, pp 90-96). Thus, methods are available in the art to identify plant cells and plant lines comprising a mutation in the *GTR* gene. This population of mutant cells or plant lines can then be tested for functional GTR activity and GSL content and compared to non-mutated cells or plant lines with similar genetic background.

Further provided are plants obtainable by the above described methods and parts and products thereof, including seed, seed meal, seed oil, green plant tissue, such as rosette leaves, cauline leaves en silique walls, and root tissue, as well as uses thereof, for example, in animal feed, in pest management, such as biofumigation, and in cancer-prevention.

The transformed or mutated plant cells and plants obtained by the methods of the invention may contain, respectively, at least one other or at least one chimeric gene containing a nucleic acid encoding a protein of interest. Examples of such proteins of interest include an enzyme for resistance to a herbicide, such as the *bar* or *pat* enzyme for tolerance to glufosinate-based herbicides (EP 0 257 542, WO 87/05629 and EP 0 257 542, White et al. 1990), the EPSPS enzyme for tolerance to glyphosate-based herbicides such as a double-mutant corn EPSPS enzyme (US 6,566,587 and WO 97/04103), or the HPPD enzyme for tolerance to HPPD inhibitor herbicides such as isoxazoles (WO 96/38567).

The transformed or mutated plant cells and plants obtained by the methods of the invention may be further used in breeding procedures well known in the art, such as crossing, selfing, and backcrossing. Breeding programs may involve crossing to generate an F1 (first filial) generation, followed by several generations of selfing (generating F2, F3, etc.). The breeding program may also involve backcrossing (BC) steps, whereby the offspring is backcrossed to one of the parental lines, termed the recurrent parent.

The transformed or mutated plant cells and plants obtained by the methods of the invention may also be further used in subsequent transformation procedures.

The following non-limiting examples describe the characteristics of plants obtained in accordance with the invention. Unless otherwise stated, all recombinant DNA techniques are carried out according to standard protocols as described in Sambrook et al. (1989) Molecular Cloning: A Laboratory Manual, Second Edition, Cold Spring Harbour Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy published by BIOS Scientific Publications Ltd (UK) and Blackwell Scientific Publications, UK.

In the description and examples, reference is made to the following sequences:

| | |
|---|---|
| SEQ ID NO: 1: | Coding DNA (without introns) of the *GTR1* gene encoding a wild-type GTR1 protein from *Arabidopsis thaliana.* |
| SEQ ID NO: 2: | wild type GTR1 protein encoded by SEQ ID NO: 1. |
| SEQ ID NO: 3: | Coding DNA (without introns) of the *GTR2* gene encoding a wild-type GTR2 protein from *Arabidopsis thaliana.* |
| SEQ ID NO: 4: | wild type GTR2 protein encoded by SEQ ID NO: 3. |
| SEQ ID NO: 5: | Coding DNA (without introns) of the *GTR3* gene encoding a wild-type GTR3 protein from *Arabidopsis thaliana.* |
| SEQ ID NO: 6: | wild type GTR3 protein encoded by SEQ ID NO: 5. |
| SEQ ID NO: 7: | Coding DNA (without introns) of the *GTR4* gene encoding a wild-type GTR4 protein from *Arabidopsis thaliana.* |
| SEQ ID NO: 8: | wild type GTR4 protein encoded by SEQ ID NO: 7. |
| SEQ ID NO: 9: | Coding DNA (without introns) of the *GTR5* gene encoding a wild-type GTR5 protein from *Arabidopsis thaliana.* |
| SEQ ID NO: 10: | wild type GTR5 protein encoded by SEQ ID NO: 9. |
| SEQ ID NO: 11: | Coding DNA (without introns) of the *GTR6* gene encoding a wild-type GTR6 protein from *Arabidopsis thaliana.* |
| SEQ ID NO: 12: | wild type GTR6 protein encoded by SEQ ID NO: 11. |
| SEQ ID NO: 13: | Coding DNA (with introns) of the *GTR1-A1* gene encoding a wild-type GTR1-A1 protein from *Brassica napus.* |
| SEQ ID NO: 14: | wild type GTR1-A1 protein encoded by SEQ ID NO: 13. |
| SEQ ID NO: 15: | Coding DNA (with introns) of the *GTR1-A2* gene encoding a wild-type GTR1-A2 protein from *Brassica napus.* |
| SEQ ID NO: 16: | wild type GTR1-A2 protein encoded by SEQ ID NO: 15. |
| SEQ ID NO: 17: | Coding DNA (with introns) of the GTR1-A3 gene encoding a wild-type GTR1-A3 protein from *Brassica napus.* |
| SEQ ID NO: 18: | wild type GTR1-A3 protein encoded by SEQ ID NO: 17. |
| SEQ ID NO: 19: | Coding DNA (with introns) of the *GTR1-C1* gene encoding a wild-type GTR1-C1 protein from *Brassica napus.* |
| [SEQ ID NO: 20: | wild type GTR1-C1 protein encoded by SEQ ID NO: 19. |
| SEQ ID NO: 21: | Coding DNA (with introns) of the *GTR1-C2* gene encoding a wild-type GTR1-C2 protein from *Brassica napus.* |
| SEQ ID NO: 22: | wild type GTR1-C2 protein encoded by SEQ ID NO: 21. |
| SEQ ID NO: 23: | Coding DNA (with introns) of the *GTR1-C3* gene encoding a wild-type GTR1-C3 protein from *Brassica napus.* |
| SEQ ID NO: 24: | wild type GTR1-C3 protein encoded by SEQ ID NO: 23. |
| SEQ ID NO: 25: | Coding DNA (with introns) of the *GTR2-A1* gene encoding a wild-type GTR2-A1 protein from *Brassica napus.* |
| SEQ ID NO: 26: | wild type GTR2-A1 protein encoded by SEQ ID NO: 25. |
| SEQ ID NO: 27: | Coding DNA (with introns) of the *GTR2-A2* gene encoding a wild-type GTR2-A2 protein from *Brassica napus.* |
| SEQ ID NO: 28: | wild type GTR2-A2 protein encoded by SEQ ID NO: 27. |
| SEQ ID NO: 29: | Coding DNA (with introns) of the *GTR2-A3* gene encoding a wild-type GTR2-A3 protein from *Brassica napus.* |
| SEQ ID NO: 30: | wild type GTR2-A3 protein encoded by SEQ ID NO: 29. |
| SEQ ID NO: 31: | Coding DNA (with introns) of the *GTR2-C1* gene encoding a wild-type GTR2-C1 protein from *Brassica napus.* |
| SEQ ID NO: 32: | wild type GTR2-C1 protein encoded by SEQ ID NO: 31. |
| SEQ ID NO: 33: | Coding DNA (with introns) of the *GTR2-C2* gene encoding a wild-type GTR2-C2 protein from *Brassica napus* (partial sequence). |
| SEQ ID NO: 34: | wild type GTR2-C2 protein encoded by SEQ ID NO: 33 (partial sequence). |
| SEQ ID NO: 35: | Coding DNA (with introns) of the *GTR2-C3* gene encoding a wild-type GTR2-C3 protein from *Brassica napus.* |
| SEQ ID NO: 36: | wild type GTR2-C3 protein encoded by SEQ ID NO: 35. |
| SEQ ID NO: 37: | Coding DNA (with introns) of the *GTR1-A1* gene encoding a wild-type GTR1-A1 protein from *Brassica rapa.* |
| SEQ ID NO: 38: | wild type GTR1-A1 protein encoded by SEQ ID NO: 37. |
| SEQ ID NO: 39: | Coding DNA (with introns) of the *GTR1-A2* gene encoding a wild-type GTR1-A2 protein from *Brassica rapa.* |
| SEQ ID NO: 40: | wild type GTR1-A2 protein encoded by SEQ ID NO: 39. |
| SEQ ID NO: 41: | Coding DNA (with introns) of the *GTR1-A3* gene encoding a wild-type GTR1-A3 protein from *Brassica rapa.* |
| SEQ ID NO: 42: | wild type GTR1-A3 protein encoded by SEQ ID NO: 41. |
| SEQ ID NO: 43: | Coding DNA (with introns) of the *GTR2-A1* gene encoding a wild-type GTR2-A1 protein from *Brassica rapa.* |
| SEQ ID NO: 44: | wild type GTR2-A1 protein encoded by SEQ ID NO: 43. |
| SEQ ID NO: 45: | Coding DNA (with introns) of the *GTR2-A2* gene encoding a wild-type GTR2-A2 protein from *Brassica rapa.* |
| SEQ ID NO: 46: | wild type GTR2-A2 protein encoded by SEQ ID NO: 45. |
| SEQ ID NO: 47: | Coding DNA (with introns) of the *GTR2-A3* gene encoding a wild-type GTR2-A3 protein from *Brassica rapa.* |
| SEQ ID NO: 48: | wild type GTR2-A3 protein encoded by SEQ ID NO: 47. |
| SEQ ID NO: 49: | Coding DNA (with introns) of the *GTR1-C1* gene encoding a wild-type GTR1-C1 protein from *Brassica oleracea.* |
| SEQ ID NO: 50: | wild type GTR1-C1 protein encoded by SEQ ID NO: 49. |
| SEQ ID NO: 51: | Coding DNA (with introns) of the *GTR1-C2* gene encoding a wild-type GTR1-C2 protein from *Brassica oleracea.* |
| SEQ ID NO: 52: | Wild type GTR1-C2 protein encoded by SEQ ID NO: 51. |
| SEQ ID NO: 53: | Coding DNA (with introns) of the *GTR1-C3* gene encoding a wild-type GTR1-C3 protein from *Brassica oleracea.* |
| SEQ ID NO: 54: | wild type GTR1-C3 protein encoded by SEQ ID NO: 53. |
| SEQ ID NO: 55: | Coding DNA (with introns) of the *GTR2-C1* gene encoding a wild-type GTR2-C1 protein from *Brassica oleracea.* |
| SEQ ID NO: 56: | wild type GTR2-C1 protein encoded by SEQ ID NO: 55. |
| SEQ ID NO: 57: | Coding DNA (with introns) of the *GTR2-C2* gene encoding a wild-type GTR2-C2 protein from *Brassica oleracea.* |
| SEQ ID NO: 58: | wild type GTR2-C2 protein encoded by SEQ ID NO: 57. |
| SEQ ID NO: 59: | Coding DNA (with introns) of the *GTR2-C3* gene encoding a wild-type GTR2-C3 protein from *Brassica oleracea.* |
| SEQ ID NO: 60: | wild type GTR2-C3 protein encoded by SEQ ID NO: 59. |
| SEQ ID NO: 61: | Coding DNA (with introns) of the *GTR1* gene encoding a wild-type GTR1 protein from *Arabidopsis thaliana.* |
| SEQ ID NO: 62: | wild type GTR1 protein encoded by SEQ ID NO: 61. |
| SEQ ID NO: 63: | Coding DNA (with introns) of the *GTR2* gene encoding a wild-type GTR2 protein from *Arabidopsis thaliana.* |
| [SEQ ID NO: 64: | wild type GTR2 protein encoded by SEQ ID NO: 63. |
| SEQ ID NO: 65: | Coding DNA (with introns) of the *GTR2-A2* gene encoding a wild-type GTR2-A2 protein from *Brassica rapa* ecotype *pekinensis.* |
| SEQ ID NO: 66: | wild type GTR2-A2 protein encoded by SEQ ID NO: 65. |
| SEQ ID NO: 67: | Primer BrGTR2-A2-f (uracil at position 8) |
| SEQ ID NO: 68: | Primer BrGTR2-A2-r (uracil at position 8) |
| SEQ ID NO: 69: | Primer BrGTR2-A2-Till-f |
| SEQ ID NO: 70: | Primer BrGTR2-A2-Till-r |
| SEQ ID NO: 71: | Primer BrGTR2-A2-Inner-fw |
| SEQ ID NO: 72: | Primer BrGTR2-A2-Inner-fw2 |
| SEQ ID NO: 73: | Primer BrGTR2-A2-Inner-rv |
| SEQ ID NO: 74: | Primer BrGTR2-A2-126-f (uracil at position 10) |
| SEQ ID NO: 75: | Primer BrGTR2-A2-126-r (uracil at position 10) |
| SEQ ID NO: 76: | Primer BrGTR2-A2-145-f (uracil at position 9) |
| SEQ ID NO: 77: | Primer BrGTR2-A2-145-r (uracil at position 9) |
| SEQ ID NO: 78: | Primer BrGTR2-A2-192-f (uracil at position 9) |
| SEQ ID NO: 79: | Primer BrGTR2-A2-192-r (uracil at position 9) |
| SEQ ID NO: 80: | Primer BrGTR2-A2-229-f (uracil at position 11) |
| SEQ ID NO: 81: | Primer BrGTR2-A2-229-r (uracil at position 11) |
| SEQ ID NO: 82: | Primer BrGTR2-A2-359-f (uracil at position 10) |
| SEQ ID NO: 83: | Primer BrGTR2-A2-359-r (uracil at position 10) |
| SEQ ID NO: 84: | Primer AtGTR2f (uracil at position 8) |
| SEQ ID NO: 85: | Primer AtGTR2r (uracil at position 8) |
| SEQ ID NO: 86: | Primer AtGTR4e1f (uracil at position 8) |
| SEQ ID NO: 87: | Primer AtGTR4e1r (uracil at position 9) |
| SEQ ID NO: 88: | Primer AtGTR4e2f (uracil at position 9) |
| SEQ ID NO: 89: | Primer AtGTR4e2r (uracil at position 13) |
| SEQ ID NO: 90: | Primer AtGTR4e3f (uracil at position 13) |
| SEQ ID NO: 91: | Primer AtGTR4e3r (uracil at position 9) |
| SEQ ID NO: 92: | Primer AtGTR4e4f (uracil at position 9) |
| SEQ ID NO: 93: | Primer AtGTR4e4r (uracil at position 8) |
| SEQ ID NO: 94: | Primer AtGTR5f (uracil at position 8) |
| SEQ ID NO: 95: | Primer AtGTR5r (uracil at position 8) |
| SEQ ID NO: 96: | Primer T7 |
| SEQ ID NO: 97: | Primer pNB1rev |
| SEQ ID NO: 98: | Primer AtGTR1_N879742_RP |
| SEQ ID NO: 99: | Primer AtGTR1_N879742_RP |
| SEQ ID NO: 100: | Primer AtGTR1_N870210_RP |
| SEQ ID NO: 101: | Primer AtGTR1_N870210_RP |
| SEQ ID NO: 102: | Primer AtGTR1_N409421_RP |
| SEQ ID NO: 103: | Primer AtGTR1_N409421_RP |
| SEQ ID NO: 104: | Primer AtGTR1_RP_fw |
| SEQ ID NO: 105: | Primer AtGTR1_RP_rev |
| SEQ ID NO: 106: | Primer AtGTR2_RP_fw |
| SEQ ID NO: 107: | Primer AtGTR2_RP_rev |
| SEQ ID NO: 108: | Primer AtGTR3_RP_fw |
| SEQ ID NO: 109: | Primer AtGTR3_RP_rev |
| SEQ ID NO: 110: | Primer AtGTR1pf |
| SEQ ID NO: 111: | Primer AtGTR1pr |
| SEQ ID NO: 112: | Primer AtGTR2pf |
| SEQ ID NO: 113: | Primer AtGTR2pr |
| SEQ ID NO: 114: | Primer AtGTR1r-YFP |
| SEQ ID NO: 115: | Primer YFPf-AtGTR1-fusion |
| SEQ ID NO: 116: | Primer YFPr-AtGTR1-3'utr-fusion |
| SEQ ID NO: 117: | Primer AtGTR1 (3'utr)f-YFP fusion |
| SEQ ID NO: 118: | Primer AtGTR1 (3'utr)r |
| SEQ ID NO: 119: | Coding DNA (with introns) of the *GTR2-A1* gene encoding a wild-type GTR2-A1 protein from *Brassica juncea.* |
| SEQ ID NO: 120: | wild type GTR2-A1 protein encoded by SEQ ID NO: 119. |
| SEQ ID NO: 121: | Coding DNA (with introns) of the *GTR2-A2* gene encoding a wild-type GTR2-A2 protein from *Brassica juncea.* |
| SEQ ID NO: 122: | wild type GTR2-A2 protein encoded by SEQ ID NO: 121. |
| SEQ ID NO: 123: | Coding DNA (with introns) of the *GTR2-A3* gene encoding a wild-type GTR2-A3 protein from *Brassica juncea.* |
| SEQ ID NO: 124: | wild type GTR2-A3 protein encoded by SEQ ID NO: 123. |
| SEQ ID NO: 125: | Coding DNA (with introns) of the *GTR2*-*B1* gene encoding a wild-type GTR2-B1 protein from *Brassica juncea* (partial sequence from exon 2 on). |
| SEQ ID NO: 126: | wild type GTR2-B1 protein encoded by SEQ ID NO: 125 (partial sequence). |
| SEQ ID NO: 127: | Coding DNA (with introns) of the *GTR2-B2* gene encoding a wild-type GTR2-B2 protein from *Brassica juncea.* |
| SEQ ID NO: 128: | wild type GTR2-B2 protein encoded by SEQ ID NO: 127. |
| SEQ ID NO: 129: | Coding DNA (with introns) of the *GTR2-B3* gene encoding a wild-type GTR2-B3 protein from *Brassica juncea.* |
| SEQ ID NO: 130: | wild type GTR2-B3 protein encoded by SEQ ID NO: 129. |
| SEQ ID NO: 131: | Coding DNA (without introns) of the *GTR2-A1* gene encoding a wild-type GTR2-C1 protein from *Brassica juncea.* |
| SEQ ID NO: 132: | Coding DNA (without introns) of the *GTR2-A2* gene encoding a wild-type GTR2-A2 protein from *Brassica juncea.* |
| SEQ ID NO: 133: | Coding DNA (without introns) of the *GTR2-A3* gene encoding a wild-type GTR2-A3 protein from *Brassica juncea.* |
| SEQ ID NO: 134: | Coding DNA (without introns) of the *GTR2-B1* gene encoding a wild-type GTR2-B1 protein from *Brassica juncea.* |
| SEQ ID NO: 135: | Coding DNA (without introns) of the *GTR2-B2* gene encoding a wild-type GTR2-B2 protein from *Brassica juncea.* |
| SEQ ID NO: 136: | Coding DNA (without introns) of the *GTR2-B3* gene encoding a wild-type GTR2-B3 protein from *Brassica juncea.* |
| SEQ ID NO: 137: | Coding DNA (with introns) of the *GTR1-C1* gene encoding a wild-type GTR1-C1 protein from *Brassica napus (variant).* |
| SEQ ID NO: 138: | dsRNA construct for downregulation of the expression of GTR1 expression in Brassica napus. |
| SEQ ID NO: 139: | dsRNA construct for downregulation of the expression of GTR2 expression in Brassica napus |
| SEQ ID NO: 140: | dsRNA construct for downregulation of the expression of GTR1 and GTR2 expression in *Brassica napus.* |
| SEQ ID NO: 141: | Coding DNA (without introns) of the *GTR1* gene encoding a wild-type GTR1 protein from *Arabidopsis thaliana* including the 30 amino acids NH2-terminal extension |
| SEQ ID NO: 142: | wild type GTR1 protein including 30 amino acids N-terminal extension encoded by SEQ ID NO: 141 |
| SEQ ID NO: 143: | Coding DNA (without introns) of the *GTR1-A1* gene encoding a wild-type GTR1_A1 protein from *Brassica napus* including the 23 amino acids NH2-terminal extension |
| SEQ ID NO: 144: | wild type GTR1-A1 protein including 23 amino acids N-terminal extension encoded by SEQ ID NO: 143 |
| SEQ ID NO: 145: | Coding DNA (without introns) of the *GTR1-C1* gene encoding a wild-type GTR1 protein from *Brassica napus* including the 23 amino acids NH2-terminal extension |
| SEQ ID NO: 146: | wild type GTR1-C1 protein including 23 amino acids N-terminal extension encoded by SEQ ID NO: 145 |
| SEQ ID NO: 147: | Coding DNA (without introns) of the *GTR1-* A1 gene encoding a wild-type GTR1-A1 protein from *Brassica rapa* including the 23 amino acids NH2-terminal extension |
| SEQ ID NO: 148: | wild type GTR1-A1 protein including 23 amino acids N-terminal extension encoded by SEQ ID NO: 147 |
| SEQ ID NO: 149: | Coding DNA (without introns) of the *GTR1-C1* gene encoding a wild-type GTR1-C1 protein from *Brassica oleracea* including the 23 amino acids NH2-terminal extension. |
| SEQ ID NO: 150: | wild type GTR1-C1 protein including 23 amino acids N-terminal extension encoded by SEQ ID NO: 149. |

Unless stated otherwise in the Examples, all recombinant DNA techniques are carried out according to standard molecular biological techniques as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second Edition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

### Examples

Any methods of the invention not specifically described below may be performed by one of ordinary skill in the art without undue burden in the light of the disclosure herein.

### Example 1: Identification and characterization of GTR proteins

### Identification and characterization of Arabidopsis GTR sequences

A library of *Arabidopsis* transporters available as full length cDNAs from the RIKEN bioresource center (Ibaraki, Japan) was functionally screened in pools of ten genes for uptake of the most abundant *Arabidopsis* GSL 4-methylthiobutyl glucosinolate (4-MTB) into *Xenopus* oocytes. The constructed library consists of 239 transporters categorized as "organic solute transporters" or as "unknown function" with 10-14 transmembrane segments in the *Arabidopsis* membrane protein library database (Nour-Eldin et al., 2006, Plant Methods 2 : 17). As intended transport protein target in this study were potential importers from the apoplast, *Xenopus* oocytes uptake assays were performed in an acidic buffer (pH 5). Following this procedure At3g47960 (named herein AtGTR1; SEQ ID NO: 1, 2, 61 and 62) and At1g18880 (named herein AtGTR3; SEQ ID NO: 5 and 6) were identified as GSL transporters (Nour-Eldin, 2007, *supra*).

At3g47960 and At1g18880 belong to the NRT/PTR transporter family (Steiner et al., 1995, Molecular Microbiology 16: 825-834; Tsay et al., 2007, FEBS letters 581: 2290-2300), which has been shown to consist of nitrate, nitrite and peptide transporters (Tsay et al., 2007, FEBS letters 581: 2290-2300; Segonzac et al., 2007, Plant Cell 19 : 3760-3777; Komarova et al., 2008, Plant Physiol 148: 856-869). Phylogenetic analysis showed that these two genes form a small subclade with six homologs (see Figure 1a): At3g47960 (named herein AtGTR1; SEQ ID NO: 1, 2, 61 and 62), At5g62680 (named herein AtGTR2; SEQ ID NO: 3, 4, 63 and 64), At1g18880 (named herein AtGTR3; SEQ ID NO: 5 and 6), At1g69860 (named herein AtGTR4; SEQ ID NO: 7 and 8), At1g69870 (named herein AtGTR5 or NRT1.7; SEQ ID NO: 9 and 10), At1g27080 (named herein AtGTR6 or NRT1.6; SEQ ID NO: 11 and 12). Figure 1b and Table 4 indicate the sequence identity between the different AtGTR sequences.

**Table 4 Sequence identity (in %) between Arabidopsis GTR1 (without the 30 N-terminal AA i.e. as represented in SEQ ID No 2), 2, 3, 4, 5 and 6 amino acid sequences / nucleic acid (coding sequences without introns) sequences (the respective SEQ ID NO: numbers are indicated between brackets)**

| | AtGTR1 (SEQ ID 2/1) | AtGTR2 (SEQ ID 4/ 3) | AtGTR3 (SEQ ID 6/ 5) | AtGTR4 (SEQ ID 8/ 7) | AtGTR5 (SEQ ID 10/ 9) | AtGTR6 (SEQ ID 12/ 11) |
|---|---|---|---|---|---|---|
| AtGTR1 (2/1) | 100/100 | 78/75 | 60/62 | 34/46 | 42/50 | 38/49 |
| AtGTR2 (4/3) | | 100/100 | 60/63 | 33/46 | 43/50 | 37/49 |
| AtGTR3 (6/ 5) | | | 100/100 | 35/48 | 39/48 | 37/50 |
| AtGTR4 (8/7) | | | | 100/100 | 41/53 | 41/54 |
| AtGTR5 (10/9) | | | | | 100/100 | 63/67 |
| AtGTR6 (12/11) | | | | | | 100/100 |

### Identification and characterization of Brassica spp. GTR sequences

*Brassica napus, Brassica rapa,* and *Brassica oleracea* GTR1 and GTR2 nucleic and amino acid sequences were identified *in silico* by screening genomic databases for sequences essentially similar to the *Arabidopsis* GTR1 and GTR2 sequences identified above. The *Brassica* GTR1 sequences were named GTR1-(A/C)1, 2 and 3 (see sequence listing SEQ ID NO: 13-24, SEQ ID NO: 37-42, SEQ ID NO: 49-54 and SEQ ID NO: 61-62) according to their decreasing similarity to the *Arabidopsis* GTR1 homolog (Table 5a and b). Similarly, the *Brassica* GTR2 sequences were named GTR2-(A/C)1, 2 and 3 (see sequence listing SEQ ID NO: 25-36, SEQ ID NO: 43-48, SEQ ID NO: 55-60 and SEQ ID NO: 63-66) according to their decreasing similarity to the *Arabidopsis* GTR2 homolog (Table 6a and b). B. juncea nucleotide and amino acid sequences are provided in SEQ ID NOs: 119-136.

**Table 5a Sequence identity (in %) between Arabidopsis (At), Brassica rapa (Br), Brassica oleracea (Bo), Brassica napus (Bn) GTR1 amino acid sequences (as determined by ClustalW - the SEQ ID NO: numbers are indicated between brackets) (using the short GTR1 of Arabidopsis thaliana as represented in SEQ ID No. 2)**

| | At (2) | Br (38) | Br (40) | Br (42) | Bo (50) | Bo (52) | Bo (54) | Bn (14) | Bn (16) | Bn (18) | Bn (20) | Bn (22) | Bn (24) |
|---|---|---|---|---|---|---|---|---|---|---|---|---|---|
| At (2) | 100 | 84 | 83 | 81 | 83 | 83 | 81 | 83 | 83 | 81 | 83 | 83 | 81 |
| Br (38) | | 100 | 89 | 83 | 99 | 88 | 83 | 98 | 89 | 84 | 98 | 88 | 83 |
| Br (40) | | | 100 | 84 | 88 | 99 | 84 | 88 | 100 | 85 | 88 | 99 | 84 |
| Br (42) | | | | 100 | 83 | 84 | 97 | 83 | 84 | 99 | 83 | 84 | 97 |
| Bo (50) | | | | | 100 | 88 | 83 | 98 | 88 | 83 | 99 | 88 | 83 |
| Bo (52) | | | | | | 100 | 83 | 88 | 99 | 84 | 88 | 100 | 84 |
| Bo (54) | | | | | | | 100 | 83 | 84 | 97 | 83 | 83 | 99 |
| Bn (14) | | | | | | | | 100 | 88 | 83 | 98 | 88 | 83 |
| Bn (16) | | | | | | | | | 100 | 85 | 88 | 99 | 84 |
| Bn (18) | | | | | | | | | | 100 | 83 | 84 | 98 |
| Bn (20) | | | | | | | | | | | 100 | 88 | 83 |
| Bn (22) | | | | | | | | | | | | 100 | 84 |
| Bn (24) | | | | | | | | | | | | | 100 |

**Table 5b Sequence identity (in %) between Arabidopsis (At), Brassica rapa (Br), Brassica oleracea (Bo), Brassica napus (Bn) GTR1 nucleic acid (coding -without introns) sequences (as determined by ClustalW - the SEQ ID NO: numbers are indicated between brackets) (using the short GTR1 of Arabidopsis thaliana as represented in SEQ ID No. 1)**

| | At (1) | Br (37) | Br (39) | Br (41) | Bo (49) | Bo (51) | Bo (53) | Bn (13) | Bn (15) | Bn (17) | Bn (19) | Bn (21) | Bn (23) |
|---|---|---|---|---|---|---|---|---|---|---|---|---|---|
| At (1) | 100 | 84 | 83 | 83 | 84 | 83 | 83 | 84 | 84 | 83 | 84 | 83 | 83 |
| Br (37) | | 100 | 88 | 87 | 98 | 89 | 87 | 98 | 88 | 87 | 98 | 89 | 87 |
| Br (39) | | | 100 | 86 | 88 | 98 | 86 | 88 | 99 | 86 | 88 | 98 | 86 |
| Br (41) | | | | 100 | 87 | 86 | 97 | 86 | 86 | 99 | 86 | 86 | 98 |
| Bo (49) | | | | | 100 | 88 | 87 | 98 | 88 | 87 | 99 | 88 | 87 |
| Bo (51) | | | | | | 100 | 86 | 88 | 98 | 86 | 88 | 100 | 86 |
| Bo (53) | | | | | | | 100 | 86 | 86 | 98 | 87 | 86 | 99 |
| Bn (13) | | | | | | | | 100 | 88 | 86 | 98 | 88 | 86 |
| Bn (15) | | | | | | | | | 100 | 86 | 88 | 98 | 86 |
| Bn (17) | | | | | | | | | | 100 | 87 | 86 | 98 |
| Bn (19) | | | | | | | | | | | 100 | 88 | 86 |
| Bn (21) | | | | | | | | | | | | 100 | 86 |
| Bn (23) | | | | | | | | | | | | | 100 |

**Table 6a Sequence identity (in %) between Arabidopsis (At), Brassica rapa (Br), Brassica oleracea (Bo), Brassica napus (Bn) GTR2 amino acid sequences (as determined by ClustalW - the SEQ ID NO: numbers are indicated between brackets)**

| | At (4) | Br (44) | Br (46) | Br (48) | Bo (56) | Bo (58) | Bo (60) | Bn (26) | Bn (28) | Bn (30) | Bn (32) | Bn (36) |
|---|---|---|---|---|---|---|---|---|---|---|---|---|
| At (4) | 100 | 91 | 91 | 83 | 91 | 91 | 82 | 91 | 91 | 84 | 91 | 84 |
| Br (44) | | 100 | 94 | 85 | 99 | 94 | 84 | 99 | 94 | 87 | 99 | 86 |
| Br (46) | | | 100 | 86 | 94 | 99 | 85 | 94 | 99 | 88 | 94 | 87 |
| Br (48) | | | | 100 | 86 | 86 | 95 | 86 | 86 | 97 | 86 | 95 |
| Bo (56) | | | | | 100 | 94 | 85 | 99 | 94 | 87 | 100 | 87 |
| Bo (58) | | | | | | 100 | 85 | 94 | 99 | 88 | 94 | 87 |
| Bo (60) | | | | | | | 100 | 85 | 85 | 94 | 85 | 95 |
| Bn (26) | | | | | | | | 100 | 94 | 87 | 99 | 87 |
| Bn (28) | | | | | | | | | 100 | 88 | 94 | 87 |
| Bn (30) | | | | | | | | | | 100 | 87 | 97 |
| Bn (32) | | | | | | | | | | | 100 | 87 |
| Bn (36) | | | | | | | | | | | | 100 |

**Table 6b Sequence identity (in %) between Arabidopsis (At), Brassica rapa (Br), Brassica oleracea (Bo), Brassica napus (Bn) GTR2 nucleic acid (coding -without introns) sequences (as determined by ClustalW - the SEQ ID NO: numbers are indicated between brackets)**

| | At (3) | Br (43) | Br (45) | Br (47) | Bo (55) | Bo (57) | Bo (59) | Bn (25) | Bn (27) | Bn (29) | Bn (31) | Bn (35) |
|---|---|---|---|---|---|---|---|---|---|---|---|---|
| At (3) | 100 | 87 | 87 | 83 | 87 | 86 | 82 | 87 | 87 | 84 | 87 | 85 |
| Br (43) | | 100 | 89 | 86 | 98 | 89 | 85 | 98 | 89 | 88 | 97 | 88 |
| Br (45) | | | 100 | 86 | 89 | 97 | 86 | 89 | 98 | 87 | 89 | 88 |
| Br (47) | | | | 100 | 86 | 86 | 96 | 86 | 86 | 97 | 86 | 96 |
| Bo (55) | | | | | 100 | 89 | 85 | 98 | 89 | 88 | 98 | 88 |
| Bo (57) | | | | | | 100 | 86 | 89 | 97 | 87 | 90 | 88 |
| Bo (59) | | | | | | | 100 | 85 | 85 | 95 | 85 | 96 |
| Bn (25) | | | | | | | | 100 | 89 | 88 | 97 | 88 |
| Bn (27) | | | | | | | | | 100 | 87 | 89 | 88 |
| Bn (29) | | | | | | | | | | 100 | 88 | 97 |
| Bn (31) | | | | | | | | | | | 100 | 88 |
| Bn (35) | | | | | | | | | | | | 100 |

In addition, type 1 and 2 sequences of the GTR1 and GTR2 sequences (i.e. GTR1-A1, GTR1-C1, GTR1-A2, GTR1-C2, GTR2-A1, GTR2-C1, GTR2-A2 and GTR2-C2 sequences) were identified in a *B. napus* embryo transcriptome database indicating that these types of GTR1 and 2 sequences are expressed in *B. napus* embryo's.

Further, *Brassica rapa* GTR2-A2 nucleic and amino acid sequences (SEQ ID NO: 65 and 66 in the sequence listing) were identified by cloning cDNA isolated from *B*. *rapa* ecotype *pekinensis* leaves using primers BrGTR2-A2-f (SEQ ID NO: 67) and BrGTR2-A2-r (SEQ ID NO: 68).

### Example 2: Functional characterization of GTR proteins

### Functional characterization of Arabidopsis and Brassica GTR proteins

AtGTR1 to 5, BrGTR2-A2 and 12 other members from the *Arabidopsis* NRT/PTR family were tested individually for uptake activity of 4-MTB in *Xenopus* oocytes.

AtGTR1 and 2 exhibited 5-fold higher uptake activity to AtGTR3 whereas AtGTR4 and AtGTR5 showed 10% 4-MTB uptake activity compared to AtGTR1. Uptake activity of all other tested NRT/PTRs was indistinguishable from uninjected oocytes.

Oocytes expressing BrGTR2-A2 imported about 2.5 nmol 4-MTB per hour per oocyte (Figure 2) while non-injected oocytes showed no detectable uptake of 4-MTB. In comparison, oocytes expressing AtGTR2 imported about 5 nmol 4-MTB/hour (Figure 3A).

### Biochemical characterization of Arabidopsis glucosinolate transporter proteins

AtGTR1 and AtGTR2, which showed the highest GSL uptake activity, were biochemically characterized in *Xenopus* oocytes. For both genes, lowering pH from 6 to 5 in the uptake medium resulted in an about 8-fold higher 4-MTB accumulation inside oocytes while uptake at pH 7 was indistinguishable from uninjected oocytes at pH 5 (Figure 3A). This indicated that AtGTRs are proton:GSL symporters.

When current recordings were performed on oocytes expressing, respectively, AtGTR1 and AtGTR2 exposed to 100µM 4-MTB at pH 5 and voltage clamped at -50mV elicited inward currents in the 30 nA range. This indicates a net influx of positive ions through the AtGTRs upon exposure to GSLs. GSL-induced inward currents were voltage dependent increasing with hyperpolarizing membrane potential within the range of +30 to -120 mV (Figure 3B and F). As GSLs carry one negative charge per molecule the stochiometry of protons to GSLs must be at least 2:1, which appears to be a typical characteristic of proton-dependent oligopeptide transporter proteins (POTs) in general when transporting negatively charged substrates (Daniel et al., 2006, Physiology 21 : 93-102). At pH 5 clamped at -60mV, import of 4-MTB by AtGTR1 and 2, respectively, followed Michaelis-Menten saturation kinetics displaying high affinity of AtGTR1 and ATGTR2 towards 4-MTB with Kₘ = 20 ± 1.2 µM and Kₘ = 18,7 ±4,2 µM (Figure 3C-D). This suggests that GSLs are likely to be physiological substrates of AtGTRs *in planta.* Remarkably, substrate dependent transport rates for AtGTR2 at 500µM 4-MTB were 25% lower than at 250 µM and 1 mM. This observation was also noticeable in substrate dependent transport rates measured by direct LC-MS detection of GSL uptake.

The substrate specificity of the AtGTRs was investigated towards GSLs with varying sidechains. When AtGTR1 expressing oocytes clamped at -50 mV at pH 5 were perfused with 100 µM of the endogenous short chain methionine derived GSLs 4-MTB, 4-methylsulfinylbutyl-GSL and the exogenous phenylalanine derived p-hydroxybenzyl-GSL (pOHBG), an inward current in the magnitude of 30, 10 and nA for AtGTR1 was observed (Figure 3F). Similarly AtGTR2 took up all three glucosinolates measured by LC-MS analysis of oocyte extracts (data not shown).This indicated that the transporters have broad specificity with respect to the GSL side-chain.

The substrate specificity investigations were extended to include substrates previously identified for the NRT/PTR transporter family namely di- and tripeptides and nitrate. Perfusing oocytes expressing AtGTR1 with 133 µM of the dipeptides, ala-his, gly-leu, asp-ala and the tripeptides gly-gly-gly and met-ala-ser at pH 5 did not result in any detectable currents. However, perfusing with 1 mM NO₃ did result in currents but these amounted only to 1/10^{th} of currents measured when perfusing with 100 µM 4-MTB (Figure 3F). This indicated that the AtGTRs possibly possess dual substrate specificities. To investigate this further, AtGTR1 and AtGTR2 expressing oocytes were exposed to 40 µM C¹⁴-labled pOHBG in the presence of 50x excess unlabeled 4-MTB, NO₃⁻, and a mixture of di/tri peptides. Only 50x excess of 4-MTB reduced the uptake of pOHBG to background levels, while the other compounds did not have significant inhibitory effects (Figure 3E).

Collectively, our biochemical analyses showed that AtGTR1 and AtGTR2 are high affinity, specific, electrogenic proton-driven GSL transporters with broad specificity towards a wide range of GSLs.

### Materials and methods - Example 2:

Functional screening of transporter cDNA library in *Xenopus* oocytes: Preparation and functional screening of a library of 239 Arabidopsis transporters in *Xenopus* oocytes was performed essentially as described in Nour-Eldin et al. (2006, Plant Methods 2 : 17) with two modifications. Firstly, DNA templates were not pooled prior to *in vitro* transcription. Instead, each PCR product was individually *in vitro* transcribed and then pooled into ten transcripts per pool. Individual transcription was performed to avoid unbalanced gene pools due to possible varying transcription efficiencies of pooled PCR fragments. Secondly, the library was divided into 23 pools containing 10 transcripts each, and a 24th pool containing 9 transcripts. Import assays were performed at room temperature in Kulori pH 5 containing 1 mM 4-MTB (for Arabidopsis sequences) or 0.5 mM 4-MTB (for Brassica sequences). Transcript pools which mediated GSL uptake into injected oocytes were subsequently injected as individual transcripts to identify GSL transporter.

Constructs for expression in *Xenopus* oocytes: Coding sequences for AtGTR2 and AtGTR5 were cloned from cDNA from leaf tissue using primer pair AtGTR2f (SEQ ID NO: 84) and AtGTR2r (SEQ ID NO: 85) and primer pair AtGTR5f (SEQ ID NO: 94) and AtGTR5r (SEQ ID NO: 95), respectively (Nour-Eldin et al., 2006, Nucl Acids Res 34 : e122). AtGTR4 was cloned by PCR amplification using primer pair AtGTR4e1f and AtGTR4e1 r (SEQ ID NO: 86 and 87) for the first exon, primer pair AtGTR4e2f and AtGTR4e2r (SEQ ID NO: 88 and 89) for the second exon, primer pair AtGTR4e3f and AtGTR4e3r (SEQ ID NO: 90 and 91) for the third exon and primer pair AtGTR4e4f and AtGTR4e4r (SEQ ID NO: 92 and 93) for the fourth exon, and subsequent fusion of its four exons from genomic DNA. USER fusion and cloning into pNB1 u were performed as described previously (Geu-Flores et al., 2007, Nucl Acids Res 35 :e55).

Coding sequence for BrGTR2-A2 (SEQ ID NO: 65) was cloned from cDNA isolated from *B. rapa* ecotype *pekinensis* leaves. The CDS was cloned using primers BrGTR2-A2-f (SEQ ID NO: 67) and BrGTR2-A2-r (SEQ ID NO: 68) and USER cloned into the Xenopus expression vector pNB1u (Nour-Eldin et al., 2006, Nucl Acids Res 34 : e122).

Linear templates for *in vitro* transcription were generated by PCR using primers T7 (SEQ ID NO: 96) and pNB1rev (SEQ ID NO: 97). cRNA was *In vitro* transcribed as follows in 50µl reaction volumes: 1-5 µg linear template was incubated at 37°C in T7 RNA polymerase transcription buffer containing 80 U T7 RNA polymerase (Fermentas); 0,01 M DTT; 0,1 µg/µl BSA; 60µM 3'-0-Me-m⁷G(5')ppp(5')G RNA Cap Structure Analog (New England Biolabs); 20 U Ribolock™ RNase inhibitor (Fermentas); 0,01 U pyrophosphatase (Fermentas); 1 mM rATP, rUTP, rCTP and 0,05 mM rGTP (LAROVA) for 30 min. rGTP was then added to a final concentration of 1 mM, and the reaction was incubated for 3 hours. RNA was purified by lithium chloride precipitation and dissolved in nuclease free H₂O to a final concentration of 0,5 µg/µl.

### GSL transport assays in Xenopus oocytes:

*Oocyte treatment and injection:* Oocytes were prepared as described previously (1998, Methods Enzymol. 296: 17-52), and injected with 50 ng cRNA. Oocytes were incubated for 3-4 days at 17 °C, before assaying for transport activity.

*Assay conditions:* Assays were performed in saline buffer kulori (90 mM NaCl, 1 mM KCI, 1 mM CaCl₂, 1 mM MgCl₂, 5 mM MES) adjusted to pH 5 with TRIS. Oocytes were preincubated in kulori buffer pH 5 for 5 min to ensure intracellular steady state pH, and subsequently transferred to 500 µl kulori pH 5 buffer containing indicated concentrations of substrates and incubated for 1 hour at room temperature. Oocytes were washed four times in ice cold kulori pH 5 buffer.

Transport quantification: For assays with radiolabeled substrates, single oocytes were ruptured in 100 µl 10% SDS in 4 ml scintillation vials by shaking for 20-30 minutes. 2.5 ml EcoScint™ scintillation fluid (National Diagnostics) was added, and imported compounds quantified by scintillation counting. For assays with unlabeled substrates, oocyte extracts were analysed by LC-MS. Oocytes were analyzed in batches of 5 oocytes per repetition. Washed oocytes were homogenized in 100 µl kulori pH 5 containing 1 mg/ml sulphatase (Sigma A-25-120) and left for 12 hours at room temperature. An equal volume of 100% methanol was then added and samples incubated at -20 °C for one hour and centrifuged for 15 min at 20000 g. Three µl supernatant containing the desulfated-glucosinolate was analyzed by analytical LC-MS using an Agilent 1100 Series LC (Agilent Technologies, Germany) coupled to a Bruker HCT-Ultra ion trap mass spectrometer (Bruker Daltonics, Bremen, Germany). A Zorbax SB-C18 column (Agilent; 1,8 mm, 2,1 mm X 50 mm) was used at a flow rate of 0,2 ml/min, and the oven temperature was maintained at 35 °C. The mobile phases were: A, water with 0,1 % (v/v) HCOOH and 50 µM NaCl; B, acetonitrile with 0,1 % (v/v) HCOOH. The gradient program was: 0 to 0,5 min , isocratic 2 % B; 0,5 to 7,5 min, linear gradient 2 to 40 % B; 7,5 to 8,5 min, linear gradient 40 % to 90 % B; 8,5 to 11,5 min isocratic 90 % B; 11,6 to 15 min, isocratic 2 % B. The flow rate was increased to 0,3 ml/ min in the interval 11,2 to 13,5 min. The mass spectrometer was run in positive electrospray mode.

### Example 3: Generation and characterization of plants and plant parts with mutant GTR genes

### Generation and characterization of plants with mutant GTR genes

*Arabidopsis* T-DNA mutants of AtGTR1 (*atgtr1-1*), AtGTR2 (*atgtr2-1*) and AtGTR3 *(atgtr3-1),* respectively, were identified from the SALK T-DNA collection (Alonso et al., 2003, Science 301: 653-657). *In silico* genomic sequence analysis, showed that *atgtr1-1* contains a T-DNA insertion in the first exon while *atgtr2-1* contains a T-DNA insertion in the fourth exon of the coding regions and *atgtr3-1* contains the T-DNA insertion in the third exon. Genotyping [using primer pair AtGTR1_N879742_RP (SEQ ID NO: 98) and AtGTR1_N879742_LP (SEQ ID NO: 99) for *atgtr1,* AtGTR2_N870210_RP (SEQ ID NO: 100) and AtGTR2_N870210_LP (SEQ ID NO: 101) for *atgtr2;* and AtGTR3_N409421_RP (SEQ ID NO: 102) and AtGTR3_N409421_LP (SEQ ID NO: 103) for *atgtr3*] and RT-PCR analysis [using primer pair AtGTR1_RT_fw (SEQ ID NO: 104) and AtGTR1_RT_rev (SEQ ID NO: 105) for AtGTR1, AtGTR2_RT_fw (SEQ ID NO: 106) and AtGTR2_RT_rev (SEQ ID NO: 107) for AtGTR2 and AtGTR3_RT_fw (SEQ ID NO: 108) and AtGTR3_RT_rev (SEQ ID NO: 109) for AtGTR3] showed that *atgtr1, atgtr2* and *atgtr3* homozygous mutants were null mutants for their respective AtGTR. A double knockout mutant was generated by crossing *atgtr1* mutant plants to *atgtr2* mutant plants. From the segregating F2 population, 3 homozygous lines were identified for each of the wild type, *atgtr1, atgtr2* and *atgtr1*/*atgtr2* genotypes.

*Brassica rapa* substitution mutants of BrGTR2-A2 were identified in a mutated *B. rapa* ecotype R-0-18 plant population (RevGenUK) by TILLING using the BrGTR2-A2 sequence of SEQ ID NO: 65 and tilling primers BrGTR2-A2-Till-f (SEQ ID NO: 69), BrGTR2-A2-Till-r (SEQ ID NO: 70), BrGTR2-A2-Inner-fw (SEQ ID NO: 71), BrGTR2-A2-Inner-fw2 (SEQ ID NO: 72), and BrGTR2-A2-Inner-rv (SEQ ID NO: 73). Twenty substitution mutations were found: 7 silent mutations (nucleic acid mutation resulting in codon encoding the same amino acid), 8 non-severe mutations (nucleic acid mutation resulting in codon encoding an amino acid belonging to the same functional class), 4 severe mutations (nucleic acid mutation resulting in codon encoding an amino acid belonging to a different functional class, e.g. small for large, nonpolar for polar, aliphatic for nonaliphatic, aromatic for nonaromatic, hydrophobic for polar, acidic side chain for nonacidic side chain) and 1 stop codon mutation. The nucleotide position of severe and STOP mutant codons found in the genomic DNA sequence of *BrGTR2-A2* from *B. rapa* ecotype R-0-18 are indicated in Table 7 as well as the corresponding amino acid position in SEQ ID NO: 66. Codons encoding the indicated amino acids in *BrGTR2-A2* from *B. rapa* ecotype *pekinensis* can be found at the indicated amino acid positions in SEQ ID NO: 65. Homozygous wildtype and mutants *B. rapa* plants were identified for each mutation using primers BrGTR2-A2-Inner-fw (SEQ ID NO: 71), BrGTR2-A2-Inner-fw2 (SEQ ID NO: 72), and BrGTR2-A2-Inner-rv (SEQ ID NO: 73) and sequencing of the obtained amplicons to determine the presence of a wildtype or mutant codon at the positions indicated in Table 7. Mutant plants are grown and GSL content is determined in different plant parts.

Mutant plants are grown and GSL content is determined in different plant parts. The results are summarized in Figure 9. Seeds from *Brgtr2* knockout mutants (BrGTR2 (W229X Mut1-9)) have reduced glucosinolate content, both compared to "true" WT *B. rapa* seeds as well as to BrGTR2 S(W229X) WTS. The reduction is ∼80% compared to BrGTR2 (W229X-WT) plants, when pooling seeds of each genotype. *B. rapa* plants containing BrGTR2 mutated in amino acid G145R (G145R Mut1-4) show highly variable glucosinolate content, which is probably caused by segregation of additional EMS-induced point mutations in genes affecting seed glucosinolate content.

**Table 7 Substitution and STOP codon mutations in BrGTR2-A2 from B. rapa ecotype R-0-18**

| SEQ ID NO: of used tilling primers: | Nucleotide position | WT->mutant codon | WT->mutant amino acid | Amino acid position in SEQ ID NO: 66 | uptake activity in % of WT |
|---|---|---|---|---|---|
| 69 + 73 | 833 | AGG->AGA | Gly->Arg | 126 | 5% |
| 69 + 73 | 890 | GGA->AGA | Gly->Arg | 145 | 85% |
| 71 + 73 | 1031 | GAG->AAG | Glu->Lys | 192 | 56% |
| 71 + 73 | 1143 | TTG->TAG | Trp->STOP | 229 | 0% |
| 72 + 73 | 1608 | TCC->TTC | Ser->Phe | 359 | 143% |

### Functional characterization of mutant Brassica rapa GTR2-A2 proteins

The identified mutant BrGTR2-A2 proteins were tested individually for uptake activity of 4-MTB in *Xenopus* oocytes. Point mutations were made in BrGTR2-A2 by USER fusion as decribed previously (Geu-Flores et al., 2007, Nucl Acids Res 35 :e55). Briefly, the coding sequence is PCR amplified as two fragments flanking the desired mutation location. Each fragment has complementary tails with the base substitution embedded. In addition each tail contains a uracil residue which upon cleavage with the uracil specific excision reagent (USER) yields a long single stranded overhang which readily and firmly anneals with the complementary tail from the other fragment. At the distal terminal of each fragment the BrGTR2-A2-f primer was used as the forward primer for the fragment lying upstream of the mutation and the BrGTR2-A2-r as the reverse primer for the fragment lying downstream of the mutation. Primers used at the junction are given in SEQ ID NO: 74 and 75 (forward and reverse primer for amino acid 126 substitution as indicated in Table 7), in SEQ ID NO: 76 and 77 (forward and reverse primer for amino acid 145 substitution as indicated in Table 7), in SEQ ID NO: 78 and 79 (forward and reverse primer for amino acid 192 substitution as indicated in Table 7), in SEQ ID NO: 80 and 81 (forward and reverse primer for amino acid 229 substitution as indicated in Table 7) and in SEQ ID NO: 82 and 83 (forward and reverse primer for amino acid 359 substitution as indicated in Table 7). Each clone was verified by sequencing after insertion into the pNB1 u vector, RNA was made and injected into oocytes as described above. Uptake activity of each clone is given in Figure 2 and Table 7.

In conclusion, BrGTR2 containing a stop codon was non-functional, indicating that the truncated form of the protein is nonfunctional *in planta,* whereas remaining mutations exhibited varying transport activity. These varying transport activities indicate that amino acid positions that were subject to substitution are important for BrGTR2 transport activity. According to the proposed model for POT proteins, transmembrane helices no. 1, 2, 4, 5, 7, 8, 10 and 11 line the central pore of the transporter and are involved in the transport mechanism. Remaining helices (no. 3, 6, 9 and 12) are found in the periphery and may confer structural stability. Mutations BrGTR2 (G126R) and BrGTR2 (S359F) are in transmembrane helix 3 and 7, respectively. Helix 3 should not be involved in the transport mechanism; therefore, it can be hypothesized that BrGTR2 (G126R) may have severely disrupted the protein tertiary structure, leading to almost no uptake activity. Helix 7 lines the central pore, where transport occurs; thus, the increased uptake activity seen for this BrGTR2 (S359F) could be due to the transition from a small side chain (serine) to a large side chain (phenylalanine), leading to an enlargement of the pore. The change in polarity of the S359F-transition may further contribute to the increased activity. Mutation BrGTR2 (G145R) causes no significant change in uptake of 4-MTB. Mutation BrGTR2 (E192K) in the cytoplasmic loop between helices 4 and 5 seems to cause a reduction in glucosinolate uptake. An amino acid shift from a negatively charged glutamic acid to a positively charged lysine is quite drastic and possibly may have disrupted protein structure. Furthermore, a phosphorylation site has been suggested on the peptide sequence "ser-glu-ser-gly-lys-arg" of AtGTR2. An alignment of AtGTR2 and BrGTR2 shows that this sequence is conserved and that the peptide corresponds to the residues 191-196 in BrGTR2. Thus, if one of the serines (191 and 193) surrounding the mutated glutamic acid (E192K) is normally phosphorylated (or dephosphorylated), this could be prevented by the transition to lysine; which could in turn reduce uptake activity.

### Characterization of GSL content in seed of mutant GTR Arabidopsis plants

GSL analysis of segregating progeny from heterozygous *atgtr1, atgtr2* and *atgtr3* mutants showed that seeds from homozygous *atgtr3* plants contain wild type levels of total aliphatic and indole GSLs. Similarly, *atgtr2* had no significant reduction in total aliphatic and indole glucosinolate content per seed whereas *atgtr2* seeds about 50% reduction in total aliphatic GSLs concentrations (Figure 4A-C and 5A). In addition, indole GSL content was not detectable in *atgtr2* seeds. When analyzing 20 seeds per plant, GSL levels in seeds from each homozygous *atgtr1*/*atgtr2* line were below detection limits, indicating a critical role for the combined activities of AtGTR1 and AtGTR2 in transporting GSLs into seeds. When analyzing 10 mg seeds from wildtype and atgtr1/atgtr2 plants, it was possible to detect trace amounts of glucosinolates in seeds from atgtr1/atgtr2 plants at 250 fold lower levels compared to wildtype.

For molecular complementation, respectively, a AtGTR1 construct including 2kb promoter, the genomic sequence fused to YFP and 3'UTR and a AtGTR2 construct consisting of the 2kb promoter followed by the genomic sequence were introduced into the atgtr1/atgtr2 double mutant and were shown to restore both aliphatic and indole GSL content in seeds (Figure 4E-F). This demonstrates that the GSL-free seed phenotype in the atgtr1/atgtr2 mutant is due to the absence of AtGTR1 and AtGTR2 transport proteins.

### Characterization of GSL content in leaves of mutant GTR Arabidopsis plants

GSL levels were measured in entire rosettes before bolting, after bolting and at senescence. In rosettes from wildtype plants, aliphatic GSL content was measured to, respectively, 28±13 and 68±19 nmol/rosette before and after bolting and to 8±3 nmol in senescent rosettes. In contrast, in rosettes from atgtr1/atgtr2 plants, aliphatic GSL content increased dramatically from 27±11 before bolting to 410±78 nmol/rosette after bolting and remained at 161±31 nmol/rosette at senescence. atgtr1 and atgtr2 single knockout line rosettes contained intermediate levels compared to the atgtr1/atgtr2 rosettes (Figure 4B). In comparison to aliphatic GSLs, indole GSL content displayed a larger variation in rosette tissues. However, when comparing wildtype to atgtr1/atgtr2 rosettes after bolting and at senescence, atgtr1/atgtr2 rosettes contained in average 2 fold higher indole GSLs. Collectively, these observations indicate that rosette tissues continuously synthesize and export both aliphatic and indole GSLs and demonstrate that both AtGTR1 and AtGTR2 are critical for this activity in wildtype plants.

### Characterization of GSL content in stems, flowers, leaves, siliques and silique walls of mutant GTR Arabidopsis plants

From plants after bolting, GSL content was determined separately in inflorescences (without siliques and cauline leaves), cauline leaves, total intact siliques (including seeds) and silique walls from single dissected siliques (lowest and most mature). In addition, single silique walls were analysed at senescence.

In wildtype plants after bolting, inflorescences minus siliques and cauline leaves contained 200±46 nmol aliphatic GSLs per inflorescence whereas inflorescences from atgtr1/atgtr2 plants contained 61±28 nmol and inflorescences atgtr1 and atgtr2 plants contained intermediate levels (Figure 5C). In cauline leaves from wildtype plants after bolting, aliphatic GSL content amounted to 59±15 nmol per total cauline leaves while atgtr1/atgtr2 mutants contained 181±20 nmol in their cauline leaves (Figure 5C). Increased contents were also observed for aliphatic GSLs in silique walls where concentrations in atgtr1/atgtr2 plants after bolting were 8.8±3 nmol per silique wall in contrast to 2.9±0,8 nmol/silique wall from wildtype plants. At senescence, GSL levels in silique walls from wildtype decreased to 0.3±0,13 nmol per silique wall whereas atgtr1/atgtr2 silique walls retained 3±0,78 nmols (Figure 5D).

Collectively, these observations indicate that at this developmental stage, cauline leaves and silique walls in addition to rosette tissues function as GSL sources, whereas the entire inflorescence or parts thereof appears to constitute sinks for AtGTR1 and AtGTR2 mediated GSL transport. Two additional observations are noteworthy when analyzing siliques. Firstly, no significant differences were observed in GSL content when analyzing intact siliques from wildtype or atgrt1/atgtr2 mutants after bolting (Figure 5C) which indicates that at this developmental stage GSL content of the intact siliques does not exclusively depend on the activity of AtGTR1 and AtGTR2 (Figure 5D). Secondly, in contrast to the other tissues atgtr1 and atgtr2 single mutants after bolting did not exhibit an intermediate phenotype compared to atgtr1/atgtr2, on the contrary both atgtr1 and atgtr2 resembled wildtype possibly indicating an intriguing synergistic effect of mutating both transporters (Figure 5D).

When calculating the balance of total aliphatic GSL content in the various tissues of the plants after bolting, atgtr1/atgtr2 plants is seen to contain an excess of about 460 nmol in rosettes and cauline leaves compared to wildtype. As this increase is only met by an about 160 nmol decrease in stems and intact siliques in atgtr1/atgtr2 plants, this results in an about 300 nmol increased content of total aliphatic GSLs per total aerial parts in atgtr1/atgtr2 plants compared to wildtype (Table 8).

**Table 8. Aliphatic glucosinolate content per tissue (nmol/g) in soil grown plants after bolting**

| | Wildtype | atgtr1/atgtr2 |
|---|---|---|
| Rosette | 68±19 | 410±78 |
| Cauline leaves | 59±15 | 181±20 |
| Intact siliques | 83±25 | 67±22 |
| Stems | 200±46 | 61±28 |
| Balance | 398±51 | 703±75 |

### Characterization of GSL content in roots of mutant GTR Arabidopsis plants

GSL concentrations were measured in roots, rosettes and inflorescence from hydroponically grown plants before and after bolting. Before bolting, aliphatic glucosinolate content in wildtype roots was 1,75±0.28 nmol/mg fresh weight, whereas atgtr1/atgtr2 roots contained 20 fold less (0,08±0,06 nmol/mg). After bolting, roots from wildtype plants contained 0,8±0,14 nmol/mg aliphatic glucosinolates whereas roots from atgtr1/atgtr2 plants contained 0.2±0.06 nmol/mg i.e. 4 fold less (Figure 6A). In contrast to soil grown plants, aliphatic glucosinolate content in the corresponding rosettes from hydronically grown plants before bolting was 2-fold higher in atgtr1/atgtr2 plants (1,33±0,11 nm/mg) compared to wildtype (0,64±0,1 nmol/mg) (Figure 6B). After bolting the fold difference between rosettes from wildtype and atgtr1/atgtr2 plants became more pronounced with wildtype plants containing 0.35±0,13 nmol/mg whereas atgtr1/atgtr2 rosettes contained 1.44±0,16 nmol/mg i.e.4 fold higher concentrations (Figure 6B). Lastly, no significant differences was seen in aliphatic glucosinolate concentration between inflorescences from wildtype and atgtr1/atgtr2 plants (Figure 6B). Taken together these observations show that the increased glucosinolate accumulation in atgtr1/atgtr2 aerial tissues, to a large extent, can be accounted for by amounts otherwise transported to the roots. Roots thus constitute a strong and yet unidentified sink for glucosinolate transport.

### Characterization of tissue, cellular and subcellular localization of AtGTR1&2 in Arabidopsis plants

2kb of the respective promoters were used to drive the expression of a beta-D-glucoronidase (GUS). Before bolting pGTR1::GUS activity was confined to distinct spots surrounding the hydathodes. In contrast, after bolting pGTR1::GUS activity was detected throughout the leaf including the vascular tissue, mesophyl and epidermis of rosette leaves. In developing flowers, pGTR1::GUS activity was found in individual cells in the epidermis of sepals, while developing siliques showed activity in the epidermis as well as in the funiculus. This expression pattern indicated that AtGTR1 may play a general role in transporting glucosinolates between cells throughout the leaf after bolting. The localization to the epidermis indicated that glucosinolates might be transported to the outer perimeter of leaves.

In pGTR2::NLS-GFP-GUS transgenic plants GUS activity was strongly and predominantly detected in the vascular tissue throughout development with some activity also detected in the epidermis of leaves. GUS activity was furthermore detected in the vasculature of sepals, petals and in the filaments of developing flowers. Expression was also detected in the vascular tissue of silique walls and the funiculus of developing seeds. In comparison, GUS activity could not be detected in any tissue of nontransformed plants at any developmental stage. The apparent confined and constitutive expression of AtGTR2 in the vascular tissue indicated a major role in transporting glucosinolates across barriers in the vascular tissue to enable long distance transport to sinks

### Materials and methods - Example 3:

Plant Material and Growth Conditions: For GSL analysis and expression localization studies in above ground tissues, Arabidopsis plants (ecotype Columbia-0) were cultivated on soil in 4 cm pots in growth chambers at 20°C, at 16 hour light (700 µE*m*⁻²*s⁻¹). For GSL analysis in roots, plants were grown in a hydroponic system as described in (Lehmann et al. 2009, Mol Plant 2: 390-406). Briefly, seeds were germinated on 0.5% plant agar / 50% Gibeaut's nutrient solution (Gibeaut et al., 1997, Plant Physiol 115: 317-319) in 0.5ml microfuge tubes held in an empty pipette-tip box. Approximately one week after germination, the bottoms of the tubes were cut off and the boxes filled with 100% nutrient solution. The plants were transferred to larger containers approximately two weeks later and were grown under a 16h:8h photoperiod, 20 °C. Nutrient solution was changed weekly, and the genotypes were grown in separate containers. Harvesting of individual tissues for glucosinolate analysis was performed as described for soil-grown plants.

GSL extraction and analysis from plant tissues: GSL levels were analyzed in a representative line (of three identified for each) for each genotype originating from the atgtr1-1xatgtr2-1 cross at three time points, 1) Before bolting: 3-week old plants (growth stage 1.10-1.12) characterized by a fully formed rosette with 10-14 leaves before emergence of inflorescence, 2) After bolting: 5 week old plants (growth stage 6.10-6.30) characterized by the lowest silique being at full length and the presence of at least 7-10 developing siliques , 3) Senescence: 8-9 week old plants (growth stage 9.7) characterized by senescent dry green tissues and all siliques developed containing mature seeds. Growth stages definitions are as described previously (Boyes et al., 2001, Plant cell 13: 1499-1510). GSL levels were also analyzed in the representative line for each genotype in rosettes, roots and inflorescence in hydroponically grown plants before and after bolting.

For analyses performed on plants before and after bolting grown in soil or hydroponically fresh tissue was harvested and homogenized. Specifically for analyses of silique walls after bolting intact siliques were freeze dried prior to dissection into silique walls and immature seeds. For analyses of senescent plants dry tissue were harvested and homogenized. For mature seed analysis 20 seeds were analyzed. GSLs were extracted and analyzed as desulfo GSLs as described previously (Hansen et al. 2007, Plant J 50 : 902-910) using an HP1200 Series HPLC from Agilent equipped with a C-18 reversed phase column (Supelcosil LC-18-DB, 25 cm × 4.6 mm, 5 µm particle size, Supelco, Bellefonte, PA, USA) by using a water (solvent A)-acetonitrile (solvent B) gradient at a flow rate of 1 ml min⁻¹ (injection volume 45 µl). The gradient was as follows: 1.5 -7% B (5 min), 7-25% (6 min), 25-80% (4 min), 80% B (3 min), 80-35% B (2 min), 35 -1.5% B (2 min), and 1.5% B (3 min). The eluent was monitored by diode array detection between 200 and 400 nm (2-nm interval). Desulfo-GSLs were identified based on comparison of retention times and UV absorption spectra with those of known standards (Reichelt et al., 2002). Results are given as nmols calculated relative to response factors (Brown et al., 2003; Fiebig and Arens, 1992).

Construction of reporter and complementation constructs: The E.coli strain DH10B was used for all cloning. For AtGTR1 and AtGTR2 promoter:reporter constructs, promoters were amplified from Arabidopsis (Col-0) genomic DNA, using primers AtGTR1 pf (SEQ ID NO: 110) and AtGTR1pr (SEQ ID NO: 111) for the AtGTR1 promoter and primers AtGTR2pf (SEQ ID NO: 112) and AtGTR2pr (SEQ ID NO: 113) for the AtGTR2 promoter. Promoter fragments were USER cloned into pCambia3300u-NLS-GPF-GUS, upstream of a nuclear localization signal followed by GFP fused to beta-D-glucoronidase (Chytilova et al., 1999, Ann. Bot. 83: 645-654). For complementation construct AtGTR2 promoter-ATGTR2 genomic gene fragments weas PCR amplified using primers primers AtGTR2pf (SEQ ID NO: 112) and AtGTR2pr (SEQ ID NO: 113) and USER cloned into pCambia1300u. For complementation construct AGTR1 promoter-AtGTR1 genomic gene-YFP-3'UTRs each fragment was PCR amplified using primers AtGTR1pf (SEQ ID NO: 110) and AtGTR1r-YFP (SEQ ID NO: 114) for promoter-genomic gene fragment, primers YFPf-AtGTR1-fusion (SEQ ID NO: 115) and YFPr-pot13'UTRfusion (SEQ ID NO: 116) for YFP fragment and primer AtGTR1(3'UTR)f-YFP fusion (SEQ ID NO: 117) and AtGTR1(3'UTR)r (SEQ ID NO: 118) for 3'UTR fragment. Fragments were USER fused into the pCambia1300u vector, Plant expression plasmids were used to transform Agrobacterium (GV3101) for stable Arabidopsis transformations.

Staining of transgenic *Arabidopsis* plants for GUS activity: X week old *Arabidopsis* GTR1 prom-GFP-GUS and GTR2 prom-GFP-GUS plants were submerged in a solution containing 1 mM X-Glc, 0.5% Triton X-100, 20% (v/v) methanol, 100 mM Tris-HCl (pH 7.5) and 10 mM ascorbate, and incubated at 37'C for 16 hours. Chlorophyll were cleared after staining by washing and incubation in 70% ethanol.

### Example 4: Generation and characterization of mutant Brassica GTR genes and plants and plant parts comprising them

Seeds from *Brassica* plants (M0 seeds) are exposed to EMS. M1 plants are grown from mutagenized seeds (M1 seeds) and selfed to generate M2 seeds. M2 plants are grown and DNA samples are prepared from plant samples. The DNA samples are screened for the presence of point mutations in the *GTR* genes causing the introduction of STOP codons in the protein-encoding regions of the *GTR* genes, the substitution of amino acids in the GTR proteins or splice site mutations by direct sequencing with GTR-specific primers as described above and analyzing the sequences for the presence of the point mutations. For each mutant *GTR* gene identified in the DNA sample of an M2 plant, M2 plants derived from the same M1 plant as the M2 plant comprising the *GTR* mutation are grown and DNA samples are prepared from plant samples of each individual M2 plant. The DNA samples are screened for the presence of the identified *GTR* point mutation.

The following mutant alleles in B. napus have been isolated and the total glucosinolate content was tested in M3 wet seeds:
*a. GTR2-C2-ems02*
b. GTR2-*C1*-ems05
*c. GTR2-C1-ems01*
*d. GTR2-A2-ems09*
*e. GTR2-A2-ems03*
*f. GTR2-A1-ems01*

For more details concerning the mutant alleles see Tables 3a and 3b. The results are summarized in Figure 10. The maximal reduction in total glucosinolates observed was 51% with single gene knock-outs. Each gene copy tested resulted in similar effects. The high level of variation may be related to the high number of unrelated background mutations in non-backcrossed material.

Mutant *GTR* alleles are thus generated and isolated. Also, plants comprising such mutant alleles can be used to combine selected mutant and/or wild type alleles in a plant, for example a *Brassica* breeding line, as follows: A *Brassica* plant containing a mutant *GTR* gene (donor plant line) is crossed with a *Brassica* plant lacking the mutant *GTR* gene (acceptor plant line). The following introgression scheme is used (the mutant *GTR* gene is abbreviated to *gtr* while the wild type is depicted as *GTR*):
Initial cross: *gtr*/*gtr* (donor plant) X *GTR l GTR* (acceptor plant, e.g. breeding line)
F1 plant: *GTR* / *gtr*
BC1 cross: *GTR* / *gtr* X *GTR* / *GTR* (acceptor plant)
BC1 plants: 50% *GTR* / *gtr* and 50% *GTR* / *GTR*
The 50% *GTR* / *gtr* are selected using molecular markers (e.g. AFLP, PCR, Invader^{™}, and the iike) for the mutant *GTR,* allele (*gtr*).
BC2 cross: *GTR*/*gtr*(BC1 plant) X *GTR* / *GTR* (acceptor plant)
BC2 plants: 50% *GTR* / *gtr* and 50% *GTR* / *GTR*
The 50% *GTR* / *gtr* are selected using molecular markers for the mutant *GTR* allele (*gtr*)*.*
Backcrossing is repeated until BC3 to BC6
BC3-6 plants: 50% *GTR* / *gtr* and 50% *GTR*/*GTR*
The 50% *GTR* / *gtr* are selected using molecular markers for the mutant *GTR* allele (*gtr*)*.* To reduce the number of backcrossings (e.g. until BC3 in stead of BC6), molecular markers can be used specific for the genetic background of the acceptor plant line.
BC3-6 S1 cross: *GTR* / *gtr* X *GTR* / *gtr*
BC3-6 S1 plants: 25% *GTR* / *GTR* and 50% *GTR* / *gtr* and 25% *gtr*/*gfr*

Plants containing *gtr* are selected using molecular markers for the mutant *GTR* allele (*gtr*)*.* Individual BC3-6 S1 plants that are homozygous for the mutant *GTR* allele (*gtr*/*gtr*) are selected using molecular markers for the mutant and the wild-type *GTR* alleles. These plants are then used for seed production.

### Characterization of GSL content in parts of mutant GTR Brassica plants

The GSL content of seed, stems, flowers, leaves, roots, siliques and silique walls is analysed in mutant GTR *Brassica* plants as described above for the mutant GTR *Arabidopsis* plants. *Brassica* plants comprising specific combinations of mutant GTR genes resulting in specific GSL contents in specific plant parts are selected for further breeding, for seed production or for crop cultivation.

WT and mutated (by Targeting Induced Local Lesions IN Genomes, TILLING) Brassica rapa seeds of the ecotype "R-0-18" (inbred line of the Brassica rapa subsp. trilocularis) were purchased from Reverse Genetics UK (RevGenUK). Seeds were sown on plant substrate and watered with Bactimos (B. thuringiensis israelensis; after sowing, the seeds were cold-stratified at 4 °C for two days to obtain a high and uniform germination rate. Finally, germinating seeds were transferred to a growth chamber with 16 hours day length and a temperature of 20 °C. B. rapa plants used for analyses were 2.5 months old; they had green siliques containing immature green seeds. The number of replicate plants used in the experiment was n=5 plants.

B. rapa leaves and other tissues were weighted and homogenized in liquid N2 using either a mortar (leaves and roots) or a blender (stem and siliques). Glucosinolates from the ice-cold homogenized plant material were extracted in 85% methanol containing p-hydroxybenzyl glucosinolate (pOHB) as internal standard. Part of the supernatant was transferred to a 96-well filter plate loaded with 45µl DEAE sephadexTM A-25 column material. Glucosinolates were bound to the column material while samples were sucked through the filter plate by applying brief vacuum. Afterwards, columns were washed with 70% methanol and water. A sulfatase solution (2 mg/ml) was added to the columns and allowed to incubate at room temperature overnight. 100 µl water were applied to the columns and a short spin eluted the desulfo-glucosinolates into a 96-well format plate. The samples were analyzed on an Agilent technologies 1200 series HPLC-DAD system and separated on a Zorbax SB-AQ column. Figure 12 summarizes the glucosinolate concentration in the different tissues of wt and knock-out B. rapa, while Figure 13 summarizes the weight of these tissues

### Example 5: Generation and characterization of mutant GTR1 and GTR2 alleles with altered phosphorylation/dephosphorylation status

Arabidopsis thaliana GTR1 and GTR2 alleles which mimick constitutive phophorylation or dephosphorylation were constructed based on on experimentally verified phosphorylation sites fsearchable in the http://phosphat.mpimp-golm.mpg.de/ - a database collecting phosphoproteomics data. Phosphorylation was mimicked by substituting the possible phosphorylation site amino acid (S or T) with aspartic acid. Dephosphorylation was mimicked by substituting with alanine. The following constructs were made:

Constructs encoding a GTR1 protein with the amino acid sequence of SEQ ID NO: 2 with the following substitutions:
a. S at position 22 with A
b. S at position 22 with D
c. T at position 105 with A
d. T at position 105 with D
e. S at position 605 with A
f. S at position 605 with D
(which corresponds to the amino acid sequence of SEQ ID No: 142 with the following substitution:
g. S at position 52 with A
h. S at position 52 with D
i. T at position 135 with A
j. T at position 135 with D
k. S at position 635 with A
I. S at position 635 with D
or constructs encoding a a GTR2 protein with the amino acid sequence of SEQ ID No: 4 with the following substitutions:
m. T at position 58 with A
n. T at position 58 with D
o. T at position 117 with A
p. T at position 117 with D
q. T at position 323 with A
r. T at position 323 with D
Also tested were GTR1 or GTR2 encoding constructs wherein the Proline at position 492 (or 522) was substituted by Leucine.

Phosphorylation/dephosphorylation constructs were assayed for proton dependent import of the glucosinolate 4-MTB (4-methylsulfinylbutyl *glucosinolate*) at a concentration of 100 µM.. 5 Oocytes were injected with cRNA (times 4 replications) of one of the constructs (at a 100 ng/µl) and kept at 17 degrees for 5 days. On day 5 the oocytes were preincubated for 5 minutes in Kulori pH 5 and then transferred to the assay media (Kulori pH5 with 4-MTB at a concentration of 100 µM). After one hour oocytes were removed from the media containing 4-MTB and washed 4 times before being extracted in 100% methanol. LC-MS analysis to detect the uptake of 4-MTB was carried out and the results presented in Figure 11.

It can be observed that constitutive dephosphorylation of GTR1 at position S22 (S52*) turns of f transport completely. Likewise it can be observed that phosphorylation of the same residue keeps the import intact. Furthermore, it can be observed that dephosphorylation of GTR2 T58 keeps the transport activity intact whereas phosphorylation of the same residue inactivates the transport. Both constitutive phosphorylation and dephosphorylation of T105 (T135*) in GTR1 and T117 in GTR2 block the transport activity. It could also be observed that mutating GTR1 P492 (P522*) and GTR3 P492 to a leucine inactivates transport activity. In summary it can be concluded that phosphorylation/dephosphorylation at specific residues regulates GTR1 and GTR2 mediated glucosinolate transport. (amino acid position indicated by * are with reference to the long version of GTR1 including the N-terminal extension of 30 AA)

### Example 6: Downregulation of GTR1 and/or GTR2 expression in Brassica spp. using dsRNA constructs

Using standard recombinant DNA techniques dsRNA encoding recombinant genes are constructed comprising the following operably linked DNA fragments
a) GTR1 downregulating recombinant gene
   i. a plant expressible promoter such as CaMV35S
   ii.a DNA region comprising the nucleotide sequence of SEQ ID 138
   iii. a transcription termination and polyadenylation region, such as the 3' nos region.
b) GTR2 downregulating recombinant gene
   i. a plant expressible promoter such as CaMV35S
   ii. a DNA region comprising the nucleotide sequence of SEQ ID 139
   iii. a transcription termination and polyadenylation region, such as the 3' nos region.
c) GTR1/GTR2 downregulating recombinant gene
   i. a plant expressible promoter such as CaMV35S
   ii. a DNA region comprising the nucleotide sequence of SEQ ID 140
   iii. a transcription termination and polyadenylation region, such as the 3' nos region.

These dsRNA encoding recombinant genes are introduced (separately) between the borders of a T-DNA vector together with a selectable marker gene such as the phosphinotricin acetyltransferase encoding selectable marker gene._The T-DNA vector is introduced into an Agrobacterium strain comprising a helper Ti-plasmid using conventional methods. Hypocotyl explants of Brassica napus are obtained, cultured and transformed essentially as described by De Block et al. (1989), Plant Physiol. 91: 694) to transfer the chimeric genes into Brassica napus plants.

Trangenic Brassica napus plant are identified and analyzed for glucosinolate content in leaves and seeds.

## Claims

1. A method for decreasing the glucosinolate (GSL) content in seed and seed meal, or for increasing the GSL content in green plant tissue of a *Brassicales* plant, such as a *Brassica* plant, or part thereof comprising reducing the functional activity of at least one glucosinolate transport (GTR) protein comprising an amino acid sequence having at least 33% sequence identity with SEQ ID NO: 2 or SEQ ID NO: 142 in cells of said plant or said plant part.

2. The method of claim 1, wherein the GTR protein is selected from the group of:
a. a GTR protein comprising an amino acid sequence having at least 80% sequence identity with SEQ ID No. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 or 150; and
b. a GTR protein encoded by a nucleic acid having at least 80% sequence identity with any one of SEQ ID No. 13, 15, 17, 19, 21, 23, 25, 27, 29, 31, 33, 35, 37, 39, 41, 43, 45, 47, 49, 51, 53, 55, 57, 59, 119, 121, 123, 125, 127, 129, 131, 132, 133, 134, 135, 136 or 137 or 143, 145, 147 or 149.

3. The method of claim 1 or 2, comprising modifying the functional activity of at least two GTR proteins.

4. The method of claim 3, wherein a first GTR protein comprises an amino acid sequence having at least 80% sequence identity with SEQ ID NO: 2 or SEQ ID NO: 142 and a second GTR protein comprises an amino acid sequence having at least 80% sequence identity with SEQ ID NO: 4.

5. The method of claim 3, wherein said first GTR protein comprises an amino acid sequence having at least 80% sequence identity with any one of SEQ ID No. 14, 16, 18, 20, 22, 24, 38, 40, 42, 50, 52 or 54 or 144, 146, 148 or 150 and wherein said second GTR protein comprises an amino acid sequence having at least 80% sequence identity with any one of SEQ ID No. 26, 28, 30, 32, 34, 36, 44, 46, 48, 56, 58, 60, 120, 122, 124, 126, 128, or 130.

6. The method of claim 5, wherein said first GTR protein is encoded by a nucleic acid having at least 80% sequence identity with any one of SEQ ID No. 13, 15, 17, 19, 21, 23, 37, 39, 41, 49, 51, 53 or 137 or 143, 145, 147 or 149 and wherein said second GTR protein is encoded by a nucleotide sequence having at least 80% sequence identity with any one of SEQ ID No. 25, 27, 29, 31, 33, 35, 43, 45, 47, 55, 57, 59, 119, 121, 123, 125, 127, 129, 131, 132, 133, 134, 135, 136.

7. A method to reduce GTR activity in a cell of a plant, such as a *Brassica* plant, comprising introducing an RNA molecule in said plant or plant part, wherein said RNA molecule comprises a GTR-inhibitory RNA molecule capable of down-regulating the expression of a *GTR* gene, said GTR gene comprising a nucleic acid sequence encoding a glucosinolate transporter (GTR) protein, said GTR protein having an amino acid sequence having at least 80% sequence identity with any one of SEQ ID No. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 or 150.

8. The method of claim 7, comprising introducing a chimeric DNA construct in said plant or plant part, wherein said chimeric DNA construct comprises the following operably linked DNA regions:
a) a promoter, operative in said plant or plant part;
b) a transcribed DNA region, which when transcribed yields a GTR-inhibitory RNA molecule, said GTR-inhibitory RNA molecule being capable of down-regulating the expression of said *GTR* gene;
c) a DNA region involved in transcription termination and polyadenylation.

9. A method to reduce GTR activity in a cell of a plant, comprising altering the nucleotide sequence of the endogenous *GTR* gene by generating a mutant *gtr* allele comprising one or more mutations in its nucleic acid sequence, whereby the mutations result in a significantly reduced amount of functional GTR protein in the cell *in vivo,* said endogenous GTR gene comprising a nucleic acid sequence encoding a GTR protein, said GTR protein comprising an amino acid sequence having at least 80% sequence identity with any one of SEQ ID No. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 or 150.

10. The method according to claim 9 wherein the GTR protein is a protein comprising the amino acid sequence of SEQ ID NO: 2 or SEQ ID NO: 142 with any of the following substitutions:
a. S at position 22 with A (position 52 for SEQ ID No 142)
b. S at position 22 with D (position 52 for SEQ ID No 142)
c. T at position 105 with A (position 135 for SEQ ID No 142)
d. T at position 105 with D (position 135 for SEQ ID No 142)
e. S at position 605 with A (position 635 for SEQ ID No 142)
f. S at position 605 with D (position 635 for SEQ ID No 142) or the GTR protein is a protein with the amino acid sequence of SEQ ID No: 4 with the following substitutions:
a) T at position 58 with A
b) T at position 58 with D
c) T at position 117 with A
d) T at position 117 with D
e) T at position 323 with A
f) T at position 323 with D
or the GTR protein is encoded by a nucleic acid of SEQ ID No 25
g) comprising a stop codon at position 1241 to 1243
or GTR protein is encoded by a nucleic acid of SEQ ID No 31
h) comprising a stop codon at position 929 to 931
i) comprising a stop codon at position 1145 to 1147
or GTR protein is encoded by a nucleic acid of SEQ ID No 27
j) comprising a stop codon at position 870 to 872
k) comprising a stop codon at position 1380 to 1382
or the GTR protein is encoded by a nucleic acid of SEQ ID No 33
l) comprising a stop codon at position 780 to 782
or the GTR protein is a protein with the amino acid sequence of SEQ ID No. 66 comprising a mutation at any of the following positions:
m) Gly 126
n) Gly 145
o) Glu 192
p) Trp 229
q) Ser 359

11. A plant or plant part obtainable by the method of any one of claims 7 to 10.

12. Seed from the plant of claim 11, said seed comprising
a) said RNA molecule which comprises a GTR-inhibitory RNA molecule capable of down-regulating the expression of a *GTR* gene, or
b) said mutant *gtr* allele comprising one or more mutations in its nucleic acid sequence, whereby the mutations result in a significantly reduced amount of functional GTR protein in the cell *in vivo.*

13. Use of a GTR-encoding nucleic acid sequence, said GTR-encoding nucleic acid sequence encoding a GTR protein, said GTR protein comprising an amino acid sequence having at least 80% sequence identity with any one of SEQ ID No. 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 144, 146, 148 or 150, to obtain a modified GSL content in a plant or plant part.

## Patentansprüche

1. Verfahren zur Verringerung des Glucosinolat(GSL)-Gehalts in Samen und Samenmehl oder zur Erhöhung des GSL-Gehalts in grünem Pflanzengewebe einer *Brassicales-Pflanze,* wie etwa einer *Brassica-*Pflanze, oder eines Teils davon, umfassend Reduzieren der funktionalen Aktivität wenigstens eines Glucosinolat-Transport(GTR)-Proteins, das eine Aminosäuresequenz mit wenigstens 33% Sequenzidentität mit SEQ ID NO: 2 oder SEQ ID NO: 142 umfasst, in Zellen der Pflanze oder des Pflanzenteils.

2. Verfahren nach Anspruch 1, wobei das GTR-Protein aus der folgenden Gruppe ausgewählt ist:
a. einem GTR-Protein, das eine Aminosäuresequenz mit wenigstens 80% Sequenzidentität mit SEQ ID Nr. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 oder 150 umfasst; und
b. einem GTR-Protein, das von einer Nukleinsäure mit wenigstens 80% Sequenzidentität mit einer der SEQ ID Nr. 13, 15, 17, 19, 21, 23, 25, 27, 29, 31, 33, 35, 37, 39, 41, 43, 45, 47, 49, 51, 53, 55, 57, 59, 119, 121, 123, 125, 127, 129, 131, 132, 133, 134, 135, 136 oder 137 oder 143, 145, 147 oder 149 codiert wird.

3. Verfahren nach Anspruch 1 oder 2, umfassend Modifizieren der funktionalen Aktivität von wenigstens zwei GTR-Proteinen.

4. Verfahren nach Anspruch 3, wobei ein erstes GTR-Protein eine Aminosäuresequenz mit wenigstens 80% Sequenzidentität mit SEQ ID NO: 2 oder SEQ ID NO: 142 und ein zweites GTR-Protein eine Aminosäuresequenz mit wenigstens 80% Sequenzidentität mit SEQ ID NO: 4 umfasst.

5. Verfahren nach Anspruch 3, wobei das erste GTR-Protein eine Aminosäuresequenz mit wenigstens 80% Sequenzidentität mit einer der SEQ ID Nr. 14, 16, 18, 20, 22, 24, 38, 40, 42, 50, 52 oder 54 oder 144, 146, 148 oder 150 umfasst und wobei das zweite GTR-Protein eine Aminosäuresequenz mit wenigstens 80% Sequenzidentität mit einer der SEQ ID Nr. 26, 28, 30, 32, 34, 36, 44, 46, 48, 56, 58, 60, 120, 122, 124, 126, 128 oder 130 umfasst.

6. Verfahren nach Anspruch 5, wobei das erste GTR-Protein von einer Nukleinsäure mit wenigstens 80% Sequenzidentität mit einer der SEQ ID Nr. 13, 15, 17, 19, 21, 23, 37, 39, 41, 49, 51, 53 oder 137 oder 143, 145, 147 oder 149 codiert wird und wobei das zweite GTR-Protein von einer Nukleotidsequenz mit wenigstens 80% Sequenzidentität mit einer der SEQ ID Nr. 25, 27, 29, 31, 33, 35, 43, 45, 47, 55, 57, 59, 119, 121, 123, 125, 127, 129, 131, 132, 133, 134, 135, 136 codiert wird.

7. Verfahren zur Reduzierung von GTR-Aktivität in einer Zelle einer Pflanze, wie etwa einer Brassica-Pflanze, umfassend Einführen eines RNA-Moleküls in die Pflanze oder den Pflanzenteil, wobei das RNA-Molekül ein GTR hemmendes RNA-Molekül mit der Fähigkeit zur Herunterregulierung der Expression eines GTR-Gens umfasst, wobei das GTR-Gen eine ein Glucosinolat-Transporter(GTR)-Protein codierende Nukleinsäuresequenz umfasst, wobei das GTR-Protein eine Aminosäuresequenz mit wenigstens 80% Sequenzidentität mit einer der SEQ ID Nr. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 oder 150 aufweist.

8. Verfahren nach Anspruch 7, umfassend Einführen eines chimären DNA-Konstrukts in die Pflanze oder den Pflanzenteil, wobei das chimäre DNA-Konstrukt die folgenden in operativer Verknüpfung stehenden DNA-Bereiche umfasst:
a) einen Promotor, operativ in der Pflanze oder dem Pflanzenteil;
b) einen transkribierten DNA-Bereich, dessen Transkription ein GTR hemmendes RNA-Molekül ergibt, wobei das GTR hemmende RNA-Molekül zur Herunterregulierung der Expression des GTR-Gens fähig ist;
c) einen an Transkriptionstermination und Polyadenylierung beteiligten DNA-Bereich.

9. Verfahren zur Reduzierung von GTR-Aktivität in einer Zelle einer Pflanze, umfassend Verändern der Nukleotidsequenz des endogenen GTR-Gens durch Erzeugen eines mutierten gtr-Allels, dessen Nukleinsäuresequenz eine oder mehrere Mutationen umfasst, wobei die Mutationen zu einer signifikant reduzierten Menge an funktionellem GTR-Protein in der Zelle in vivo führen, wobei das endogene GTR-Gen eine ein GTR-Protein codierende Nukleinsäuresequenz umfasst, wobei das GTR-Protein eine Aminosäuresequenz mit wenigstens 80% Sequenzidentität mit einer der SEQ ID Nr. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 oder 150 umfasst.

10. Verfahren nach Anspruch 9, wobei es sich bei dem GTR-Protein um ein Protein handelt, das die Aminosäuresequenz von SEQ ID NO: 2 oder SEQ ID NO: 142 mit einer der folgenden Substitutionen umfasst:
a. S in Position 22 gegen A (Position 52 für SEQ ID Nr. 142)
b. S in Position 22 gegen D (Position 52 für SEQ ID Nr. 142)
c. T in Position 105 gegen A (Position 135 für SEQ ID Nr. 142)
d. T in Position 105 gegen D (Position 135 für SEQ ID Nr. 142)
e. S in Position 605 gegen A (Position 635 für SEQ ID Nr. 142)
f. S in Position 605 gegen D (Position 635 für SEQ ID Nr. 142)
oder es sich bei dem GTR-Protein um ein Protein mit der Aminosäuresequenz von SEQ ID NO: 4 mit den folgenden Substitutionen handelt:
a) T in Position 58 gegen A
b) T in Position 58 gegen D
c) T in Position 117 gegen A
d) T in Position 117 gegen D
e) T in Position 323 gegen A
f) T in Position 323 gegen D
oder das GTR-Protein von einer Nukleinsäure von SEQ ID Nr. 25 codiert wird, die
g) ein Stoppcodon in Position 1241 bis 1243 umfasst,
oder GTR-Protein von einer Nukleinsäure von SEQ ID Nr. 31 codiert wird, die
h) ein Stoppcodon in Position 929 bis 931 umfasst,
i) ein Stoppcodon in Position 1145 bis 1147 umfasst,
oder GTR-Protein von einer Nukleinsäure von SEQ ID Nr. 27 codiert wird, die
j) ein Stoppcodon in Position 870 bis 872 umfasst,
k) ein Stoppcodon in Position 1380 bis 1382 umfasst,
oder das GTR-Protein von einer Nukleinsäure von SEQ ID Nr. 33 codiert wird, die
l) ein Stoppcodon in Position 780 bis 782 umfasst,
oder es sich bei dem GTR-Protein um ein Protein mit der Aminosäuresequenz von SEQ ID Nr. 66 handelt, die eine Mutation an einer der folgenden Positionen umfasst:
m) Gly 126
n) Gly 145
o) Glu 192
p) Trp 229
q) Ser 359.

11. Pflanze oder Pflanzenteil, erhältlich mit dem Verfahren nach einem der Ansprüche 7 bis 10.

12. Samen aus der Pflanze nach Anspruch 11, wobei der Samen
a) das RNA-Molekül, das ein GTR hemmendes RNA-Molekül mit der Fähigkeit zur Herunterregulierung der Expression eines GTR-Gens umfasst, oder
b) das mutierte gtr-Allel, dessen Nukleinsäuresequenz eine oder mehrere Mutationen umfasst, wobei die Mutationen zu einer signifikant reduzierten Menge an funktionellem GTR-Protein in der Zelle in vivo führen,
umfasst.

13. Verwendung einer GTR codierenden Nukleinsäuresequenz, wobei die GTR codierende Nukleinsäuresequenz ein GTR-Protein codiert, wobei das GTR-Protein eine Aminosäuresequenz mit wenigstens 80% Sequenzidentität mit einer der SEQ ID Nr. 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 144, 146, 148 oder 150 umfasst, so dass ein modifizierter GSL-Gehalt in einer Pflanze oder einem Pflanzenteil erhalten wird.

## Revendications

1. Procédé pour diminuer la teneur en glucosinolate (GSL) d'une graine et d'une farine de graine, ou pour augmenter la teneur en GSL d'un tissu de plante verte d'une plante de l'ordre des *Brassicales,* telle qu'une plante *Brassica,* ou une partie de cette dernière, comprenant la réduction de l'activité fonctionnelle d'au moins une protéine de transport du glucosinolate (GTR) comprenant une séquence d'acides aminés ayant une identité de séquence d'au moins 33 % avec SEQ ID NO:2 ou SEQ ID NO:142 dans les cellules de ladite plante ou de ladite partie de plante.

2. Procédé selon la revendication 1, dans lequel la protéine GTR est choisie dans le groupe consistant en :
a. une protéine GTR comprenant une séquence d'acides aminés ayant une identité de séquence d'au moins 80 % avec SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 ou 150 ; et
b. une protéine GTR codée par un acide nucléique ayant une identité de séquence d'au moins 80 % avec l'une quelconque des séquences SEQ ID NO:13, 15, 17, 19, 21, 23, 25, 27, 29, 31, 33, 35, 37, 39, 41, 43, 45, 47, 49, 51, 53, 55, 57, 59, 119, 121, 123, 125, 127, 129, 131, 132, 133, 134, 135, 136 ou 137 ou 143, 145, 147 ou 149.

3. Procédé selon la revendication 1 ou 2, comprenant la modification de l'activité fonctionnelle d'au moins deux protéines GTR.

4. Procédé selon la revendication 3, dans lequel une première protéine GTR comprend une séquence d'acides aminés ayant une identité de séquence d'au moins 80 % avec SEQ ID NO:2 ou SEQ ID NO:142 et une deuxième protéine GTR comprend une séquence d'acides aminés ayant une identité de séquence d'au moins 80 % avec SEQ ID NO:4.

5. Procédé selon la revendication 3, dans lequel ladite première protéine GTR comprend une séquence d'acides aminés ayant une identité de séquence d'au moins 80 % avec l'une quelconque des séquences SEQ ID NO:14, 16, 18, 20, 22, 24, 38, 40, 42, 50, 52 ou 54 ou 144, 146, 148 ou 150, et dans lequel ladite deuxième protéine GTR comprend une séquence d'acides aminés ayant une identité de séquence d'au moins 80 % avec l'une quelconque des séquences SEQ ID NO:26, 28, 30, 32, 34, 36, 44, 46, 48, 56, 58, 60, 120, 122, 124, 126, 128 ou 130.

6. Procédé selon la revendication 5, dans lequel ladite première protéine GTR est codée par un acide nucléique ayant une identité de séquence d'au moins 80 % avec l'une quelconque des séquences SEQ ID NO:13, 15, 17, 19, 21, 23, 37, 39, 41, 49, 51, 53 ou 137 ou 143, 145, 147 ou 149, et dans lequel ladite deuxième protéine GTR est codée par une séquence nucléotidique ayant une identité de séquence d'au moins 80 % avec l'une quelconque des séquences SEQ ID NO:25, 27, 29, 31, 33, 35, 43, 45, 47, 55, 57, 59, 119, 121, 123, 125, 127, 129, 131, 132, 133, 134, 135, 136.

7. Procédé de réduction de l'activité de GTR dans une cellule d'une plante, telle qu'une plante *Brassica,* comprenant l'introduction d'une molécule d'ARN dans ladite plante ou partie de plante, ladite molécule d'ARN comprenant une molécule d'ARN inhibitrice de GTR, capable de réguler négativement l'expression d'un gène *GTR,* ledit gène GTR comprenant une séquence d'acide nucléique codant pour une protéine de transport du glucosinolate (GTR), ladite protéine GTR ayant une séquence d'acides aminés ayant une identité de séquence d'au moins 80 % avec l'une quelconque des séquences SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 ou 150.

8. Procédé selon la revendication 7, comprenant l'introduction d'une construction d'ADN chimère dans ladite plante ou partie de plante, ladite construction d'ADN chimère comprenant les régions d'ADN suivantes, fonctionnellement liées :
a) un promoteur, fonctionnel dans ladite plante ou partie de plante ;
b) une région d'ADN transcrite qui, quand elle est transcrite, donne une molécule d'ARN inhibitrice de GTR, ladite molécule d'ARN inhibitrice de GTR étant capable de réguler négativement l'expression dudit gène GTR ;
c) une région d'ADN impliquée dans la terminaison de la transcription et dans la polyadénylation.

9. Procédé de réduction de l'activité de GTR dans une cellule d'une plante, comprenant l'altération de la séquence nucléotidique du gène GTR endogène par génération d'un allèle GTR mutant comprenant une ou plusieurs mutations dans sa séquence d'acide nucléique, les mutations conduisant ainsi à une quantité significativement réduite de protéine GTR fonctionnelle dans la cellule *in vivo,* ledit gène GTR endogène comprenant une séquence d'acide nucléique codant pour une protéine GTR, ladite protéine GTR comprenant une séquence d'acides aminés ayant une identité de séquence d'au moins 80 % avec l'une quelconque des séquences SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 142, 144, 146, 148 ou 150.

10. Procédé selon la revendication 9, dans lequel la protéine GTR est une protéine comprenant la séquence d'acides aminés SEQ ID NO:2 ou SEQ ID NO:142, avec l'une quelconque des substitutions suivantes .
a. S en position 22 remplacé par A (position 52 pour SEQ ID NO:142)
b. S en position 22 remplacé par D (position 52 pour SEQ ID NO:142)
c. T en position 105 remplacé par A (position 135 pour SEQ ID NO:142)
d. T en position 105 remplacé par D (position 135 pour SEQ ID NO:142)
e. S en position 605 remplacé par A (position 635 pour SEQ ID NO:142)
f. S en position 605 remplacé par D (position 635 pour SEQ ID NO:142)
ou la protéine GTR est une protéine ayant la séquence d'acides aminés SEQ ID NO:4 avec les substitutions suivantes :
a) T en position 58 remplacé par A
b) T en position 58 remplacé par D
c) T en position 117 remplacé par A
d) T en position 117 remplacé par D
e) T en position 323 remplacé par A
f) T en position 323 remplacé par D
ou la protéine GTR est codée par un acide nucléique SEQ ID NO:25
g) comprenant un codon d'arrêt sur les positions 1241 à 1243,
ou la protéine GTR est codée par un acide nucléique SEQ ID NO:31
h) comprenant un codon d'arrêt sur les positions 929 à 931,
i) comprenant un codon d'arrêt sur les positions 1145 à 1147,
ou la protéine GTR est codée par un acide nucléique SEQ ID NO:27
j) comprenant un codon d'arrêt sur les positions 870 à 872,
k) comprenant un codon d'arrêt sur les positions 1380 à 1382,
ou la protéine GTR est codée par un acide nucléique SEQ ID NO:33
1) comprenant un codon d'arrêt sur les positions 780 à 782,
ou la protéine GTR est une protéine ayant la séquence d'acides aminés SEQ ID NO:66 comprenant une mutation sur l'une quelconque des positions suivantes :
m) Gly 126
n) Gly 145
o) Glu 192
p) Trp 229
q) Ser 359.

11. Plante ou partie de plante pouvant être obtenue par le procédé selon l'une quelconque des revendications 7 à 10.

12. Graine de la plante selon la revendication 11, ladite graine comprenant
a) ladite molécule d'ARN qui comprend une molécule d'ARN inhibitrice de GTR capable de réguler négativement l'expression d'un gène GTR, ou
b) ledit allèle GTR mutant comprenant une ou plusieurs mutations dans sa séquence d'acide nucléique, les mutations conduisant ainsi à une quantité significativement réduite de protéine GTR fonctionnelle dans la cellule *in vivo.*

13. Utilisation d'une séquence d'acide nucléique codant pour une GTR, ladite séquence d'acide nucléique codant pour une GTR codant pour une protéine GTR, ladite protéine GTR comprenant une séquence d'acides aminés ayant une identité de séquence d'au moins 80 ° % avec l'une quelconque des séquences SEQ ID NO:14, 16, 18, 20, 22, 24, 26, 28, 30, 32, 34, 36, 38, 40, 42, 44, 46, 48, 50, 52, 54, 56, 58, 60, 120, 122, 124, 126, 128, 130, 144, 146, 148 ou 150, pour obtenir une teneur en GSL modifiée d'une plante ou d'une partie de plante.


### SEQUENCE LISTING

<110> University of Copenhagen Bayer BioScience N.V.
<120> Glucosinolate transporter protein and uses thereof
<130> BCS 10-2012
<160> 150
<170> PatentIn version 3.5
<210> 1
   <211> 1821
   <212> DNA
   <213> Arabidopsis thaliana
<220>
   <221> CDS
   <222> (1)..(1821)
<400> 1
   atg gag aga aag cct ctt gag gtt gag cca tct act acg acg act aac      48
   Met Glu Arg Lys Pro Leu Glu Val Glu Pro Ser Thr Thr Thr Thr Asn
   1               5                   10                  15
   acc gat gtg gtc gat tct ttc gag gaa gag cag aga aaa atc gtt tat      96
   Thr Asp Val Val Asp Ser Phe Glu Glu Glu Gln Arg Lys Ile Val Tyr
               20                  25                  30
   aga gga tgg aaa gtc atg cct ttt atc att ggt aat gag aca ttt gag     144
   Arg Gly Trp Lys Val Met Pro Phe Ile Ile Gly Asn Glu Thr Phe Glu
           35                  40                  45
   aag ctt ggg atc ata ggg aca tta tca aac ctt ctt gtg tac cta act     192
   Lys Leu Gly Ile Ile Gly Thr Leu Ser Asn Leu Leu Val Tyr Leu Thr
       50                  55                  60
   tct gta ttc aac ctt aag agc tac aca gct gca act atc atc aat gcc     240
   Ser Val Phe Asn Leu Lys Ser Tyr Thr Ala Ala Thr Ile Ile Asn Ala
   65                  70                  75                  80
   ttt agt ggt aca atc aat ttc ggg act ttt att gct gcc ttt ctt tgc     288
   Phe Ser Gly Thr Ile Asn Phe Gly Thr Phe Ile Ala Ala Phe Leu Cys
                   85                  90                  95
   gac act tac ttt ggt cgc tac aag act ctc agt gtc gct gtc atc gct     336
   Asp Thr Tyr Phe Gly Arg Tyr Lys Thr Leu Ser Val Ala Val Ile Ala
               100                 105                 110
   tgt ttt ctg gga tcg ttt gtg ata cta ctt act gct gca att ccg tcg     384
   Cys Phe Leu Gly Ser Phe Val Ile Leu Leu Thr Ala Ala Ile Pro Ser
           115                 120                 125
   ttg cac ccc gtt gct tgc gga aac aaa atc tcg tgc gaa ggg cca agt     432
   Leu His Pro Val Ala Cys Gly Asn Lys Ile Ser Cys Glu Gly Pro Ser
       130                 135                 140
   gtg gga cag ata ctg ttt ctg ctc atg ggt tta ggc ttt ctt gtg gta     480
   Val Gly Gln Ile Leu Phe Leu Leu Met Gly Leu Gly Phe Leu Val Val
   145                 150                 155                 160
   ggc gct ggt ggt atc aga ccg tgt aat tta gcg ttt ggt gct gat cag     528
   Gly Ala Gly Gly Ile Arg Pro Cys Asn Leu Ala Phe Gly Ala Asp Gln
                   165                 170                 175
   ttc aac ccc aaa tcc gaa tca gga aag aaa ggg atc aac agt ttc ttc     576
   Phe Asn Pro Lys Ser Glu Ser Gly Lys Lys Gly Ile Asn Ser Phe Phe
               180                 185                 190
   aac tgg tat ttc ttc act ttc acg ttc gcg cag atc atc tcg ctc aca     624
   Asn Trp Tyr Phe Phe Thr Phe Thr Phe Ala Gln Ile Ile Ser Leu Thr
           195                 200                 205
   gca gtt gtg tat atc cag tca aat gtg agt tgg aca atc ggt ttg atc      672
   Ala Val Val Tyr Ile Gln Ser Asn Val Ser Trp Thr Ile Gly Leu Ile
       210                 215                 220
   att ccg gtg gct ctt atg ttc ttg gcg tgt gtg att ttc ttt gct ggc      720
   Ile Pro Val Ala Leu Met Phe Leu Ala Cys Val Ile Phe Phe Ala Gly
   225                 230                 235                 240
   gat aga ttg tat gta aaa gtg aaa gcc tcg ggt agt cca ttg gct ggt      768
   Asp Arg Leu Tyr Val Lys Val Lys Ala Ser Gly Ser Pro Leu Ala Gly
                   245                 250                 255
   att gct cgt gtt ata gca gcc gcg atc aag aaa cga ggt ttg aag ccg      816
   Ile Ala Arg Val Ile Ala Ala Ala Ile Lys Lys Arg Gly Leu Lys Pro
               260                 265                 270
   gtt aag cag cct tgg gtc aat ctt tac aac cat atc ccg tct aac tat      864
   Val Lys Gln Pro Trp Val Asn Leu Tyr Asn His Ile Pro Ser Asn Tyr
           275                 280                 285
   gca aat act act cta aaa tac acc gac cag ttc aga ttt cta gac aaa      912
   Ala Asn Thr Thr Leu Lys Tyr Thr Asp Gln Phe Arg Phe Leu Asp Lys
       290                 295                 300
   gca gca att atg acc ccc gag gag aag ttg aac tcc gat gga acg gct      960
   Ala Ala Ile Met Thr Pro Glu Glu Lys Leu Asn Ser Asp Gly Thr Ala
   305                 310                 315                 320
   tcc gat cca tgg aaa cta tgc acg ttg cag caa gtg gaa gaa gtg aaa      1008
   Ser Asp Pro Trp Lys Leu Cys Thr Leu Gln Gln Val Glu Glu Val Lys
                   325                 330                 335
   tgc att gta aga gtg att cca atc tgg ttt gca tcc acg ata tac tac      1056
   Cys Ile Val Arg Val Ile Pro Ile Trp Phe Ala Ser Thr Ile Tyr Tyr
               340                 345                 350
   ctt gca ata act ata caa atg act tat ccg gtc ttc caa gcg ctc cag      1104
   Leu Ala Ile Thr Ile Gln Met Thr Tyr Pro Val Phe Gln Ala Leu Gln
           355                 360                 365
   agc gac cga cga tta ggt tct ggt ggc ttc agg att cct gcg gcc acc      1152
   Ser Asp Arg Arg Leu Gly Ser Gly Gly Phe Arg Ile Pro Ala Ala Thr
       370                 375                 380
   tat gta gtg ttc ttg atg act ggc atg aca gtt ttc atc ata ttc tac      1200
   Tyr Val Val Phe Leu Met Thr Gly Met Thr Val Phe Ile Ile Phe Tyr
   385                 390                 395                 400
   gac cgt gta ctt gtc ccg tcg ctc aga aga gtg acc ggg tta gaa acc      1248
   Asp Arg Val Leu Val Pro Ser Leu Arg Arg Val Thr Gly Leu Glu Thr
                   405                 410                 415
   ggt ata tca ctc tta caa aga atc gga gct ggg ttt aca ttt gcg ata      1296
   Gly Ile Ser Leu Leu Gln Arg Ile Gly Ala Gly Phe Thr Phe Ala Ile
               420                 425                 430
   atg agt tta tta gtt tcc ggg ttt ata gag gaa agg aga aga aac ttt      1344
   Met Ser Leu Leu Val Ser Gly Phe Ile Glu Glu Arg Arg Arg Asn Phe
           435                 440                 445
   gca ttg acc aaa ccg aca ctc ggg atg gcg cct cgg aca gga gaa atc      1392
   Ala Leu Thr Lys Pro Thr Leu Gly Met Ala Pro Arg Thr Gly Glu Ile
       450                 455                 460
   tca tca atg tca gcg ttg tgg ctg att ccg cag ctt aca ctt gca ggc      1440
   Ser Ser Met Ser Ala Leu Trp Leu Ile Pro Gln Leu Thr Leu Ala Gly
   465                 470                 475                 480
   ata gca gaa gct ttt gct gcc att gga caa atg gag ttt tac tac aag      1488
   Ile Ala Glu Ala Phe Ala Ala Ile Gly Gln Met Glu Phe Tyr Tyr Lys
                   485                 490                 495
   cag ttt cct gaa aac atg aag agc ttc gcg ggt tct atc ttt tat gtc      1536
   Gln Phe Pro Glu Asn Met Lys Ser Phe Ala Gly Ser Ile Phe Tyr Val
               500                 505                 510
   ggt gca ggg gtt tcg agc tac ctc gct agc ttc ttg ata tca act gtt          1584
   Gly Ala Gly Val Ser Ser Tyr Leu Ala Ser Phe Leu Ile Ser Thr Val
           515                     520                     525
   cat cga aca acc gcg cat tca cct tca ggg aat tgg tta gct gag gat          1632
   His Arg Thr Thr Ala His Ser Pro Ser Gly Asn Trp Leu Ala Glu Asp
       530                     535                     540
   ctg aac aaa gcg aaa ttg gat tac ttc tac ttc atg ctc aca gga ctc          1680
   Leu Asn Lys Ala Lys Leu Asp Tyr Phe Tyr Phe Met Leu Thr Gly Leu
   545                 550                     555                 560
   atg gtt gtt aac atg gct tac ttc ttg ttg atg gct aga tgg tat aga          1728
   Met Val Val Asn Met Ala Tyr Phe Leu Leu Met Ala Arg Trp Tyr Arg
                   565                     570                 575
   tac aaa gga ggt aat gat gaa gac atc act gag att gag acc aat gaa          1776
   Tyr Lys Gly Gly Asn Asp Glu Asp Ile Thr Glu Ile Glu Thr Asn Glu
               580                     585                     590
   gaa gaa acc aag caa cag cag cta caa gac aag aac tct gtc tga              1821
   Glu Glu Thr Lys Gln Gln Gln Leu Gln Asp Lys Asn Ser Val
           595                     600                     605
<210> 2
   <211> 606
   <212> PRT
   <213> Arabidopsis thaliana
<400> 2
   Met Glu Arg Lys Pro Leu Glu Val Glu Pro Ser Thr Thr Thr Thr Asn
   1               5                   10                  15
   Thr Asp Val Val Asp Ser Phe Glu Glu Glu Gln Arg Lys Ile Val Tyr
               20                  25                  30
   Arg Gly Trp Lys Val Met Pro Phe Ile Ile Gly Asn Glu Thr Phe Glu
           35                  40                  45
   Lys Leu Gly Ile Ile Gly Thr Leu Ser Asn Leu Leu Val Tyr Leu Thr
       50                  55                  60
   Ser Val Phe Asn Leu Lys Ser Tyr Thr Ala Ala Thr Ile Ile Asn Ala
   65                  70                  75                  80
   Phe Ser Gly Thr Ile Asn Phe Gly Thr Phe Ile Ala Ala Phe Leu Cys
                   85                  90                  95
   Asp Thr Tyr Phe Gly Arg Tyr Lys Thr Leu Ser Val Ala Val Ile Ala
               100                 105                 110
   Cys Phe Leu Gly Ser Phe Val Ile Leu Leu Thr Ala Ala Ile Pro Ser
           115                 120                 125
   Leu His Pro Val Ala Cys Gly Asn Lys Ile Ser Cys Glu Gly Pro Ser
       130                 135                 140
   Val Gly Gln Ile Leu Phe Leu Leu Met Gly Leu Gly Phe Leu Val Val
   145                 150                 155                 160
   Gly Ala Gly Gly Ile Arg Pro Cys Asn Leu Ala Phe Gly Ala Asp Gln
                   165                 170                 175
   Phe Asn Pro Lys Ser Glu Ser Gly Lys Lys Gly Ile Asn Ser Phe Phe
   180 185 190
   Asn Trp Tyr Phe Phe Thr Phe Thr Phe Ala Gln Ile Ile Ser Leu Thr
   195 200 205
   Ala Val Val Tyr Ile Gln Ser Asn Val Ser Trp Thr Ile Gly Leu Ile
   210 215 220
   Ile Pro Val Ala Leu Met Phe Leu Ala Cys Val Ile Phe Phe Ala Gly
   225 230 235 240
   Asp Arg Leu Tyr Val Lys Val Lys Ala Ser Gly Ser Pro Leu Ala Gly
   245 250 255
   Ile Ala Arg Val Ile Ala Ala Ala Ile Lys Lys Arg Gly Leu Lys Pro
   260 265 270
   Val Lys Gln Pro Trp Val Asn Leu Tyr Asn His Ile Pro Ser Asn Tyr
   275 280 285
   Ala Asn Thr Thr Leu Lys Tyr Thr Asp Gln Phe Arg Phe Leu Asp Lys
   290 295 300
   Ala Ala Ile Met Thr Pro Glu Glu Lys Leu Asn Ser Asp Gly Thr Ala
   305 310 315 320
   Ser Asp Pro Trp Lys Leu Cys Thr Leu Gln Gln Val Glu Glu Val Lys
   325 330 335
   Cys Ile Val Arg Val Ile Pro Ile Trp Phe Ala Ser Thr Ile Tyr Tyr
   340 345 350
   Leu Ala Ile Thr Ile Gln Met Thr Tyr Pro Val Phe Gln Ala Leu Gln
   355 360 365
   Ser Asp Arg Arg Leu Gly Ser Gly Gly Phe Arg Ile Pro Ala Ala Thr
   370 375 380
   Tyr Val Val Phe Leu Met Thr Gly Met Thr Val Phe Ile Ile Phe Tyr
   385 390 395 400
   Asp Arg Val Leu Val Pro Ser Leu Arg Arg Val Thr Gly Leu Glu Thr
   405 410 415
   Gly Ile Ser Leu Leu Gln Arg Ile Gly Ala Gly Phe Thr Phe Ala Ile
   420 425 430
   Met Ser Leu Leu Val Ser Gly Phe Ile Glu Glu Arg Arg Arg Asn Phe
   435 440 445
   Ala Leu Thr Lys Pro Thr Leu Gly Met Ala Pro Arg Thr Gly Glu Ile
   450 455 460
   Ser Ser Met Ser Ala Leu Trp Leu Ile Pro Gln Leu Thr Leu Ala Gly
   465 470 475 480
   Ile Ala Glu Ala Phe Ala Ala Ile Gly Gln Met Glu Phe Tyr Tyr Lys
                   485                 490                 495
   Gln Phe Pro Glu Asn Met Lys Ser Phe Ala Gly Ser Ile Phe Tyr Val
               500                 505                 510
   Gly Ala Gly Val Ser Ser Tyr Leu Ala Ser Phe Leu Ile Ser Thr Val
           515                 520                 525
   His Arg Thr Thr Ala His Ser Pro Ser Gly Asn Trp Leu Ala Glu Asp
       530                 535                 540
   Leu Asn Lys Ala Lys Leu Asp Tyr Phe Tyr Phe Met Leu Thr Gly Leu
   545                 550                 555                 560
   Met Val Val Asn Met Ala Tyr Phe Leu Leu Met Ala Arg Trp Tyr Arg
                   565                 570                 575
   Tyr Lys Gly Gly Asn Asp Glu Asp Ile Thr Glu Ile Glu Thr Asn Glu
               580                 585                 590
   Glu Glu Thr Lys Gln Gln Gln Leu Gln Asp Lys Asn Ser Val
           595                 600                 605
<210> 3
   <211> 1851
   <212> DNA
   <213> Arabidopsis thaliana
<220>
   <221> CDS
   <222> (1)..(1851)
<400> 3
   atg gag aga aag cct ctt gaa ctt gag tcg acg gat aac cac cag aat        48
   Met Glu Arg Lys Pro Leu Glu Leu Glu Ser Thr Asp Asn His Gln Asn
   1               5                   10                  15
   cct tcc tcc gcc gta tac ggt ggc tct gtt acg gcg gtt gac tct gtt        96
   Pro Ser Ser Ala Val Tyr Gly Gly Ser Val Thr Ala Val Asp Ser Val
               20                  25                  30
   gaa gaa gat gtt cag aat cag aag aaa gtc gtt tat aga ggc tgg aaa       144
   Glu Glu Asp Val Gln Asn Gln Lys Lys Val Val Tyr Arg Gly Trp Lys
           35                  40                  45
   gtc atg ccc ttt att att gga aat gag aca ttt gag aag ctt ggg atc       192
   Val Met Pro Phe Ile Ile Gly Asn Glu Thr Phe Glu Lys Leu Gly Ile
       50                  55                  60
   att gga aca cta tca aac ctt ctg gtt tat tta act gca gtc ttc aac       240
   Ile Gly Thr Leu Ser Asn Leu Leu Val Tyr Leu Thr Ala Val Phe Asn
   65                  70                  75                  80
   ctg aag agt att aca gct gca acc atc att aac gcc ttt agt ggc aca       288
   Leu Lys Ser Ile Thr Ala Ala Thr Ile Ile Asn Ala Phe Ser Gly Thr
                   85                  90                  95
   att aat ttc gga act ttc gtt gct gct ttt ctc tgt gac act tac ttc       336
   Ile Asn Phe Gly Thr Phe Val Ala Ala Phe Leu Cys Asp Thr Tyr Phe
               100                 105                 110
   ggt cga tac aag act cta agt gtc gcg gtc atc gca tgt ttt ctt gga       384
   Gly Arg Tyr Lys Thr Leu Ser Val Ala Val Ile Ala Cys Phe Leu Gly
           115                 120                 125
   tca ttt gtg ata cta ttg act gca gca gtg cca caa ctg cat cca gct       432
   Ser Phe Val Ile Leu Leu Thr Ala Ala Val Pro Gln Leu His Pro Ala
   130 135 140
   gca tgt gga aca gcg gcg gat agc ata tgt aac ggg cca agt gga ggc 480
   Ala Cys Gly Thr Ala Ala Asp Ser Ile Cys Asn Gly Pro Ser Gly Gly
   145 150 155 160
   cag ata gcg ttt cta ctg atg ggt ctc ggg ttc ctt gta gtg gga gcg 528
   Gln Ile Ala Phe Leu Leu Met Gly Leu Gly Phe Leu Val Val Gly Ala
   165 170 175
   ggc ggt att aga ccg tgt aac tta gct ttt ggg gct gat cag ttt aac 576
   Gly Gly Ile Arg Pro Cys Asn Leu Ala Phe Gly Ala Asp Gln Phe Asn
   180 185 190
   ccg aaa agc gaa tca ggg aaa aga ggg att gac agt ttc ttt aat tgg 624
   Pro Lys Ser Glu Ser Gly Lys Arg Gly Ile Asp Ser Phe Phe Asn Trp
   195 200 205
   tat ttc ttt acc ttc act ttc gcg cag atc ctg tcg ctg act cta gtc 672
   Tyr Phe Phe Thr Phe Thr Phe Ala Gln Ile Leu Ser Leu Thr Leu Val
   210 215 220
   gtc tac gtt cag tct aat gtc agt tgg acg atc ggt cta acc atc cct 720
   Val Tyr Val Gln Ser Asn Val Ser Trp Thr Ile Gly Leu Thr Ile Pro
   225 230 235 240
   gcc gtt cta atg ttt ttg gcc tgc ttg att ttc ttt gcg ggc gat aag 768
   Ala Val Leu Met Phe Leu Ala Cys Leu Ile Phe Phe Ala Gly Asp Lys
   245 250 255
   ttg tat gtg aag atc aaa gcc tcg ggt agt cca ttg gcc ggt ata gct 816
   Leu Tyr Val Lys Ile Lys Ala Ser Gly Ser Pro Leu Ala Gly Ile Ala
   260 265 270
   caa gtt ata gcg gtt gcg atc aag aaa cgc gga tta aag ccc gcg aaa 864
   Gln Val Ile Ala Val Ala Ile Lys Lys Arg Gly Leu Lys Pro Ala Lys
   275 280 285
   cag cct tgg ctt aac ctt tac aat tac tac cca cca aaa tac gca aac 912
   Gln Pro Trp Leu Asn Leu Tyr Asn Tyr Tyr Pro Pro Lys Tyr Ala Asn
   290 295 300
   tcc aag ctc aaa tac aca gac caa ttc aga ttt ctt gat aaa gcc gct 960
   Ser Lys Leu Lys Tyr Thr Asp Gln Phe Arg Phe Leu Asp Lys Ala Ala
   305 310 315 320
   atc ttg act cct gaa gac aag ttg cag cca gat ggt aag cct gct gat 1008
   Ile Leu Thr Pro Glu Asp Lys Leu Gln Pro Asp Gly Lys Pro Ala Asp
   325 330 335
   cct tgg aag ctc tgt aca atg caa caa gtt gaa gaa gtg aag tgc atc 1056
   Pro Trp Lys Leu Cys Thr Met Gln Gln Val Glu Glu Val Lys Cys Ile
   340 345 350
   gtg aga gtg ctt cct ata tgg ttt gca tca tca att tac tac tta acc 1104
   Val Arg Val Leu Pro Ile Trp Phe Ala Ser Ser Ile Tyr Tyr Leu Thr
   355 360 365
   ata act caa caa atg act tat ccc gtc ttc caa gcc ctt cag agc gac 1152
   Ile Thr Gln Gln Met Thr Tyr Pro Val Phe Gln Ala Leu Gln Ser Asp
   370 375 380
   cgt cgc tta ggc tca gga ggt ttt gtg att ccc gca gcc acc tac gtg 1200
   Arg Arg Leu Gly Ser Gly Gly Phe Val Ile Pro Ala Ala Thr Tyr Val
   385 390 395 400
   gtt ttc ttg atg acg gga atg aca gtt ttc atc gta gtc tac gac cgt 1248
   Val Phe Leu Met Thr Gly Met Thr Val Phe Ile Val Val Tyr Asp Arg
   405 410 415
   gta ctt gtg cca acc atg aga aga att act ggt cta gac acc ggg ata 1296
   Val Leu Val Pro Thr Met Arg Arg Ile Thr Gly Leu Asp Thr Gly Ile
   420 425 430
   acg ctc ttg cag aga att gga act ggc att ttc ttc gct acc gcg agt 1344
   Thr Leu Leu Gln Arg Ile Gly Thr Gly Ile Phe Phe Ala Thr Ala Ser
           435                 440                 445
   tta gta gtc gcg gga ttt gtc gag gaa cga agg aga aca ttc gct ctg      1392
   Leu Val Val Ala Gly Phe Val Glu Glu Arg Arg Arg Thr Phe Ala Leu
       450                 455                 460
   act aaa cca aca ctc ggt atg gcg cca aga aag gga gaa atc tcg tcg      1440
   Thr Lys Pro Thr Leu Gly Met Ala Pro Arg Lys Gly Glu Ile Ser Ser
   465                 470                 475                 480
   atg tca gct atg tgg ctg att ccg cag ctc tca ctg gcg ggt gta gca      1488
   Met Ser Ala Met Trp Leu Ile Pro Gln Leu Ser Leu Ala Gly Val Ala
                   485                 490                 495
   gag gca ttt gca gcc att gga caa atg gag ttt tac tac aag cag ttt      1536
   Glu Ala Phe Ala Ala Ile Gly Gln Met Glu Phe Tyr Tyr Lys Gln Phe
               500                 505                 510
   ccc gag aac atg agg agt ttc gct gga tcc atc ttt tac gtt gga gga      1584
   Pro Glu Asn Met Arg Ser Phe Ala Gly Ser Ile Phe Tyr Val Gly Gly
           515                 520                 525
   gga gtt tcg agt tac ctt ggt agc ttc ttg att gca aca gtt cac agg      1632
   Gly Val Ser Ser Tyr Leu Gly Ser Phe Leu Ile Ala Thr Val His Arg
       530                 535                 540
   aca aca cag aac tcg tca ggg ggt aac tgg tta gct gag gat ttg aac      1680
   Thr Thr Gln Asn Ser Ser Gly Gly Asn Trp Leu Ala Glu Asp Leu Asn
   545                 550                 555                 560
   aaa gga aga ttg gat ctc ttc tat ttc atg atc gct gga atc ctg gca      1728
   Lys Gly Arg Leu Asp Leu Phe Tyr Phe Met Ile Ala Gly Ile Leu Ala
                   565                 570                 575
   gtt aat ttt gct tat ttc ttg gtg atg tca aga tgg tat agg tac aaa      1776
   Val Asn Phe Ala Tyr Phe Leu Val Met Ser Arg Trp Tyr Arg Tyr Lys
               580                 585                 590
   gga agt gat gac gaa gtg aca aca tat gaa acc aat gaa aat atc atc      1824
   Gly Ser Asp Asp Glu Val Thr Thr Tyr Glu Thr Asn Glu Asn Ile Ile
           595                 600                 605
   aaa cag caa gac aag aac gtt gcc tga                                  1851
   Lys Gln Gln Asp Lys Asn Val Ala
       610                 615
<210> 4
   <211> 616
   <212> PRT
   <213> Arabidopsis thaliana
<400> 4
   Met Glu Arg Lys Pro Leu Glu Leu Glu Ser Thr Asp Asn His Gln Asn
   1               5                   10                  15
   Pro Ser Ser Ala Val Tyr Gly Gly Ser Val Thr Ala Val Asp Ser Val
               20                  25                  30
   Glu Glu Asp Val Gln Asn Gln Lys Lys Val Val Tyr Arg Gly Trp Lys
           35                  40                  45
   Val Met Pro Phe Ile Ile Gly Asn Glu Thr Phe Glu Lys Leu Gly Ile
       50                  55                  60
   Ile Gly Thr Leu Ser Asn Leu Leu Val Tyr Leu Thr Ala Val Phe Asn
   65                  70                  75                  80
   Leu Lys Ser Ile Thr Ala Ala Thr Ile Ile Asn Ala Phe Ser Gly Thr
   85 90 95
   Ile Asn Phe Gly Thr Phe Val Ala Ala Phe Leu Cys Asp Thr Tyr Phe
   100 105 110
   Gly Arg Tyr Lys Thr Leu Ser Val Ala Val Ile Ala Cys Phe Leu Gly
   115 120 125
   Ser Phe Val Ile Leu Leu Thr Ala Ala Val Pro Gln Leu His Pro Ala
   130 135 140
   Ala Cys Gly Thr Ala Ala Asp Ser Ile Cys Asn Gly Pro Ser Gly Gly
   145 150 155 160
   Gln Ile Ala Phe Leu Leu Met Gly Leu Gly Phe Leu Val Val Gly Ala
   165 170 175
   Gly Gly Ile Arg Pro Cys Asn Leu Ala Phe Gly Ala Asp Gln Phe Asn
   180 185 190
   Pro Lys Ser Glu Ser Gly Lys Arg Gly Ile Asp Ser Phe Phe Asn Trp
   195 200 205
   Tyr Phe Phe Thr Phe Thr Phe Ala Gln Ile Leu Ser Leu Thr Leu Val
   210 215 220
   Val Tyr Val Gln Ser Asn Val Ser Trp Thr Ile Gly Leu Thr Ile Pro
   225 230 235 240
   Ala Val Leu Met Phe Leu Ala Cys Leu Ile Phe Phe Ala Gly Asp Lys
   245 250 255
   Leu Tyr Val Lys Ile Lys Ala Ser Gly Ser Pro Leu Ala Gly Ile Ala
   260 265 270
   Gln Val Ile Ala Val Ala Ile Lys Lys Arg Gly Leu Lys Pro Ala Lys
   275 280 285
   Gln Pro Trp Leu Asn Leu Tyr Asn Tyr Tyr Pro Pro Lys Tyr Ala Asn
   290 295 300
   Ser Lys Leu Lys Tyr Thr Asp Gln Phe Arg Phe Leu Asp Lys Ala Ala
   305 310 315 320
   Ile Leu Thr Pro Glu Asp Lys Leu Gln Pro Asp Gly Lys Pro Ala Asp
   325 330 335
   Pro Trp Lys Leu Cys Thr Met Gln Gln Val Glu Glu Val Lys Cys Ile
   340 345 350
   Val Arg Val Leu Pro Ile Trp Phe Ala Ser Ser Ile Tyr Tyr Leu Thr
   355 360 365
   Ile Thr Gln Gln Met Thr Tyr Pro Val Phe Gln Ala Leu Gln Ser Asp
   370 375 380
   Arg Arg Leu Gly Ser Gly Gly Phe Val Ile Pro Ala Ala Thr Tyr Val
   385                 390                 395                 400
   Val Phe Leu Met Thr Gly Met Thr Val Phe Ile Val Val Tyr Asp Arg
                   405                 410                 415
   Val Leu Val Pro Thr Met Arg Arg Ile Thr Gly Leu Asp Thr Gly Ile
               420                 425                 430
   Thr Leu Leu Gln Arg Ile Gly Thr Gly Ile Phe Phe Ala Thr Ala Ser
           435                 440                 445
   Leu Val Val Ala Gly Phe Val Glu Glu Arg Arg Arg Thr Phe Ala Leu
       450                 455                 460
   Thr Lys Pro Thr Leu Gly Met Ala Pro Arg Lys Gly Glu Ile Ser Ser
   465                 470                 475                 480
   Met Ser Ala Met Trp Leu Ile Pro Gln Leu Ser Leu Ala Gly Val Ala
                   485                 490                 495
   Glu Ala Phe Ala Ala Ile Gly Gln Met Glu Phe Tyr Tyr Lys Gln Phe
               500                 505                 510
   Pro Glu Asn Met Arg Ser Phe Ala Gly Ser Ile Phe Tyr Val Gly Gly
           515                 520                 525
   Gly Val Ser Ser Tyr Leu Gly Ser Phe Leu Ile Ala Thr Val His Arg
       530                 535                 540
   Thr Thr Gln Asn Ser Ser Gly Gly Asn Trp Leu Ala Glu Asp Leu Asn
   545                 550                 555                 560
   Lys Gly Arg Leu Asp Leu Phe Tyr Phe Met Ile Ala Gly Ile Leu Ala
                   565                 570                 575
   Val Asn Phe Ala Tyr Phe Leu Val Met Ser Arg Trp Tyr Arg Tyr Lys
               580                 585                 590
   Gly Ser Asp Asp Glu Val Thr Thr Tyr Glu Thr Asn Glu Asn Ile Ile
           595                 600                 605
   Lys Gln Gln Asp Lys Asn Val Ala
       610                 615
<210> 5
   <211> 1764
   <212> DNA
   <213> Arabidopsis thaliana
<220>
   <221> CDS
   <222> (1)..(1764)
<400> 5
   atg gag gtt gag aag aca gag aag aac att gca gag gat gat gag tct      48
   Met Glu Val Glu Lys Thr Glu Lys Asn Ile Ala Glu Asp Asp Glu Ser
   1               5                   10                  15
   aag atc atc tac aga gga tgg aaa gtc atg ccc ttc atc att gga aat      96
   Lys Ile Ile Tyr Arg Gly Trp Lys Val Met Pro Phe Ile Ile Gly Asn
                   20                      25                      30
   gag aca ttt gaa aaa ttg ggg att gtt gga agt tca tcg aat ctg gtt        144
   Glu Thr Phe Glu Lys Leu Gly Ile Val Gly Ser Ser Ser Asn Leu Val
               35                  40                  45
   ata tac tta acg acg gtt ttc aac atg aag agc atc acg gct gcg aaa        192
   Ile Tyr Leu Thr Thr Val Phe Asn Met Lys Ser Ile Thr Ala Ala Lys
       50                  55                  60
   gtc gtc aac atc tac ggt ggc aca agc aac ttt ggc acc atc gtc gct        240
   Val Val Asn Ile Tyr Gly Gly Thr Ser Asn Phe Gly Thr Ile Val Ala
   65                  70                  75                  80
   gct ttc ctc tgt gac tcc tac ttt ggc cgc tac aag acc cta tct ttc        288
   Ala Phe Leu Cys Asp Ser Tyr Phe Gly Arg Tyr Lys Thr Leu Ser Phe
                   85                  90                  95
   gcc atg atc gct tgc ttt ctc ggt tcg gtg gca atg gat cta acg gct        336
   Ala Met Ile Ala Cys Phe Leu Gly Ser Val Ala Met Asp Leu Thr Ala
               100                 105                 110
   gtg att cat ccc ctg cat cca gct caa tgc gct aag gaa ata gga agc        384
   Val Ile His Pro Leu His Pro Ala Gln Cys Ala Lys Glu Ile Gly Ser
           115                 120                 125
   gtg tgc aat ggg cca tcc atc gga caa atc atg ttt ctc gcc gga gca        432
   Val Cys Asn Gly Pro Ser Ile Gly Gln Ile Met Phe Leu Ala Gly Ala
       130                 135                 140
   atg gtt ttg ctg gtg atc gga gcc ggt ggg att agg cca tgc aat ctt        480
   Met Val Leu Leu Val Ile Gly Ala Gly Gly Ile Arg Pro Cys Asn Leu
   145                 150                 155                 160
   cca ttt ggt gct gat cag ttc gat ccg aag aca aaa gaa ggg aaa cga        528
   Pro Phe Gly Ala Asp Gln Phe Asp Pro Lys Thr Lys Glu Gly Lys Arg
                   165                 170                 175
   ggg att gag agt ttc ttc aat tgg tat ttc ttc acc ttc acg ttt gct        576
   Gly Ile Glu Ser Phe Phe Asn Trp Tyr Phe Phe Thr Phe Thr Phe Ala
               180                 185                 190
   cag atg gtg tcg tta acc ctc atc gtc tat gtt cag tca aac gtg agc        624
   Gln Met Val Ser Leu Thr Leu Ile Val Tyr Val Gln Ser Asn Val Ser
           195                 200                 205
   tgg agc att ggt tta gcc atc cca gct att cta atg cta ctt ggt tgc        672
   Trp Ser Ile Gly Leu Ala Ile Pro Ala Ile Leu Met Leu Leu Gly Cys
       210                 215                 220
   atc ata ttc ttc gct ggt tct aag ctc tat gtc aag gtt aaa gct agt        720
   Ile Ile Phe Phe Ala Gly Ser Lys Leu Tyr Val Lys Val Lys Ala Ser
   225                 230                 235                 240
   ggc agt ccc att cac agc ata act cgt gtc att gtt gtc gcg atc aag        768
   Gly Ser Pro Ile His Ser Ile Thr Arg Val Ile Val Val Ala Ile Lys
                   245                 250                 255
   aag agg cgg tta aag ccc gtt ggg cct aac gag ctt tac aac tac atc        816
   Lys Arg Arg Leu Lys Pro Val Gly Pro Asn Glu Leu Tyr Asn Tyr Ile
               260                 265                 270
   gcc agt gat ttc aag aac tcg aaa ttg ggc cat aca gag cag ttc agg        864
   Ala Ser Asp Phe Lys Asn Ser Lys Leu Gly His Thr Glu Gln Phe Arg
           275                 280                 285
   ttt ctt gac aaa tcg gca atc caa aca caa gac gac aag ctg aac aaa        912
   Phe Leu Asp Lys Ser Ala Ile Gln Thr Gln Asp Asp Lys Leu Asn Lys
       290                 295                 300
   gat ggg tcg ccg gtg gat gca tgg aaa ctc tgc agt atg caa caa gtg        960
   Asp Gly Ser Pro Val Asp Ala Trp Lys Leu Cys Ser Met Gln Gln Val
   305                 310                 315                 320
   gag gaa gtt aaa tgc gtg atc cgg gtg ctt ccg gtt tgg ctc tcg gcc       1008
   Glu Glu Val Lys Cys Val Ile Arg Val Leu Pro Val Trp Leu Ser Ala
                   325                             330                           335
   gcc tta ttc tac cta gct tac att caa caa aca acc tac aca atc ttt      1056
   Ala Leu Phe Tyr Leu Ala Tyr Ile Gln Gln Thr Thr Tyr Thr Ile Phe
               340                     345                     350
   cag tct ctt caa tcc gat aga cgc ctt ggt cca gga agt ttc cag atc      1104
   Gln Ser Leu Gln Ser Asp Arg Arg Leu Gly Pro Gly Ser Phe Gln Ile
           355                     360                     365
   ccg gcc ggt tct tat acc gtc ttc cta atg ctt gga atg acg ata ttc      1152
   Pro Ala Gly Ser Tyr Thr Val Phe Leu Met Leu Gly Met Thr Ile Phe
       370                     375                     380
   ata cct atc tat gac cgc gtc ctt gta cct ttc ctt aga aag tat aca      1200
   Ile Pro Ile Tyr Asp Arg Val Leu Val Pro Phe Leu Arg Lys Tyr Thr
   385                     390                     395                     400
   ggc aga gac ggc ggt atc aca caa ctg cag aga gtt gga gca gga ctg      1248
   Gly Arg Asp Gly Gly Ile Thr Gln Leu Gln Arg Val Gly Ala Gly Leu
                   405                     410                     415
   ttt cta tgc atc aca agt atg atg gtg tct gca att gta gaa caa tac      1296
   Phe Leu Cys Ile Thr Ser Met Met Val Ser Ala Ile Val Glu Gln Tyr
               420                     425                     430
   aga aga aag gta gct ctc aca aaa ccg acg cta ggg ttg gcc cca aga      1344
   Arg Arg Lys Val Ala Leu Thr Lys Pro Thr Leu Gly Leu Ala Pro Arg
           435                     440                     445
   aaa ggc gca atc tct tcg atg tcc ggt atg tgg ttg att cct cag cta      1392
   Lys Gly Ala Ile Ser Ser Met Ser Gly Met Trp Leu Ile Pro Gln Leu
       450                     455                     460
   gtg cta atg ggt atc gcg gac gcc ctc gct gga gtt gga caa atg gag      1440
   Val Leu Met Gly Ile Ala Asp Ala Leu Ala Gly Val Gly Gln Met Glu
   465                     470                     475                     480
   ttt tac tac aaa cag ttt cca gag aac atg cga agt ttt gct ggt tct      1488
   Phe Tyr Tyr Lys Gln Phe Pro Glu Asn Met Arg Ser Phe Ala Gly Ser
                   485                     490                     495
   ctg tat tat tgt gga atc gga ttg gcg agt tac ctc agc acc ttt ttg      1536
   Leu Tyr Tyr Cys Gly Ile Gly Leu Ala Ser Tyr Leu Ser Thr Phe Leu
               500                     505                     510
   tta tcg gcg gta cac gat acc aca gag gga ttt tca gga gga agt tgg      1584
   Leu Ser Ala Val His Asp Thr Thr Glu Gly Phe Ser Gly Gly Ser Trp
           515                     520                     525
   ctt ccg gaa gat tta aac aag gga aga ttg gag tat ttc tac ttc ttg      1632
   Leu Pro Glu Asp Leu Asn Lys Gly Arg Leu Glu Tyr Phe Tyr Phe Leu
       530                     535                     540
   gtt gct ggt atg atg act cta aac ttg gcc tat ttc ttg ttg gtc tca      1680
   Val Ala Gly Met Met Thr Leu Asn Leu Ala Tyr Phe Leu Leu Val Ser
   545                     550                     555                     560
   cat tgg tac cgt tac aaa gat gtt gtg gcc aag gac aag gac atg gac      1728
   His Trp Tyr Arg Tyr Lys Asp Val Val Ala Lys Asp Lys Asp Met Asp
                   565                     570                     575
   aag act tct gct gag ttt gat aag gtg tca gtg taa                      1764
   Lys Thr Ser Ala Glu Phe Asp Lys Val Ser Val
               580                     585
<210> 6
   <211> 587
   <212> PRT
   <213> Arabidopsis thaliana
<400> 6
   Met Glu Val Glu Lys Thr Glu Lys Asn Ile Ala Glu Asp Asp Glu Ser
   1               5                   10                  15
   Lys Ile Ile Tyr Arg Gly Trp Lys Val Met Pro Phe Ile Ile Gly Asn
               20                  25                  30
   Glu Thr Phe Glu Lys Leu Gly Ile Val Gly Ser Ser Ser Asn Leu Val
           35                  40                  45
   Ile Tyr Leu Thr Thr Val Phe Asn Met Lys Ser Ile Thr Ala Ala Lys
       50                  55                  60
   Val Val Asn Ile Tyr Gly Gly Thr Ser Asn Phe Gly Thr Ile Val Ala
   65                  70                  75                  80
   Ala Phe Leu Cys Asp Ser Tyr Phe Gly Arg Tyr Lys Thr Leu Ser Phe
                   85                  90                  95
   Ala Met Ile Ala Cys Phe Leu Gly Ser Val Ala Met Asp Leu Thr Ala
               100                 105                 110
   Val Ile His Pro Leu His Pro Ala Gln Cys Ala Lys Glu Ile Gly Ser
           115                 120                 125
   Val Cys Asn Gly Pro Ser Ile Gly Gln Ile Met Phe Leu Ala Gly Ala
       130                 135                 140
   Met Val Leu Leu Val Ile Gly Ala Gly Gly Ile Arg Pro Cys Asn Leu
   145                 150                 155                 160
   Pro Phe Gly Ala Asp Gln Phe Asp Pro Lys Thr Lys Glu Gly Lys Arg
                   165                 170                 175
   Gly Ile Glu Ser Phe Phe Asn Trp Tyr Phe Phe Thr Phe Thr Phe Ala
               180                 185                 190
   Gln Met Val Ser Leu Thr Leu Ile Val Tyr Val Gln Ser Asn Val Ser
           195                 200                 205
   Trp Ser Ile Gly Leu Ala Ile Pro Ala Ile Leu Met Leu Leu Gly Cys
       210                 215                 220
   Ile Ile Phe Phe Ala Gly Ser Lys Leu Tyr Val Lys Val Lys Ala Ser
   225                 230                 235                 240
   Gly Ser Pro Ile His Ser Ile Thr Arg Val Ile Val Val Ala Ile Lys
                   245                 250                 255
   Lys Arg Arg Leu Lys Pro Val Gly Pro Asn Glu Leu Tyr Asn Tyr Ile
               260                 265                 270
   Ala Ser Asp Phe Lys Asn Ser Lys Leu Gly His Thr Glu Gln Phe Arg
           275                 280                 285
   Phe Leu Asp Lys Ser Ala Ile Gln Thr Gln Asp Asp Lys Leu Asn Lys
       290                 295                 300
   Asp Gly Ser Pro Val Asp Ala Trp Lys Leu Cys Ser Met Gln Gln Val
   305                 310                 315                 320
   Glu Glu Val Lys Cys Val Ile Arg Val Leu Pro Val Trp Leu Ser Ala
   325 330 335
   Ala Leu Phe Tyr Leu Ala Tyr Ile Gln Gln Thr Thr Tyr Thr Ile Phe
   340 345 350
   Gln Ser Leu Gln Ser Asp Arg Arg Leu Gly Pro Gly Ser Phe Gln Ile
   355 360 365
   Pro Ala Gly Ser Tyr Thr Val Phe Leu Met Leu Gly Met Thr Ile Phe
   370 375 380
   Ile Pro Ile Tyr Asp Arg Val Leu Val Pro Phe Leu Arg Lys Tyr Thr
   385 390 395 400
   Gly Arg Asp Gly Gly Ile Thr Gln Leu Gln Arg Val Gly Ala Gly Leu
   405 410 415
   Phe Leu Cys Ile Thr Ser Met Met Val Ser Ala Ile Val Glu Gln Tyr
   420 425 430
   Arg Arg Lys Val Ala Leu Thr Lys Pro Thr Leu Gly Leu Ala Pro Arg
   435 440 445
   Lys Gly Ala Ile Ser Ser Met Ser Gly Met Trp Leu Ile Pro Gln Leu
   450 455 460
   Val Leu Met Gly Ile Ala Asp Ala Leu Ala Gly Val Gly Gln Met Glu
   465 470 475 480
   Phe Tyr Tyr Lys Gln Phe Pro Glu Asn Met Arg Ser Phe Ala Gly Ser
   485 490 495
   Leu Tyr Tyr Cys Gly Ile Gly Leu Ala Ser Tyr Leu Ser Thr Phe Leu
   500 505 510
   Leu Ser Ala Val His Asp Thr Thr Glu Gly Phe Ser Gly Gly Ser Trp
   515 520 525
   Leu Pro Glu Asp Leu Asn Lys Gly Arg Leu Glu Tyr Phe Tyr Phe Leu
   530 535 540
   Val Ala Gly Met Met Thr Leu Asn Leu Ala Tyr Phe Leu Leu Val Ser
   545 550 555 560
   His Trp Tyr Arg Tyr Lys Asp Val Val Ala Lys Asp Lys Asp Met Asp
   565 570 575
   Lys Thr Ser Ala Glu Phe Asp Lys Val Ser Val
   580 585
<210> 7
   <211> 1668
   <212> DNA
   <213> Arabidopsis thaliana
<220>
   <221> CDS
   <222> (1)..(1668)
<400> 7
   atg gac aat gag aaa ggg aca agt tcc tcg gat tta acg acg aga caa      48
   Met Asp Asn Glu Lys Gly Thr Ser Ser Ser Asp Leu Thr Thr Arg Gln
   1               5                   10                  15
   agg aag cct ctt ggt tgg aaa gcg atg cct tat atc ata ggg aat gaa      96
   Arg Lys Pro Leu Gly Trp Lys Ala Met Pro Tyr Ile Ile Gly Asn Glu
               20                  25                  30
   aca ttg gag agg ctt gcc aca ttc ggt ttg atg gcg aat ttc atg gtg     144
   Thr Leu Glu Arg Leu Ala Thr Phe Gly Leu Met Ala Asn Phe Met Val
           35                  40                  45
   tat atg gta aga gag tat cat atg gac caa gtt caa gct gtc act ctt     192
   Tyr Met Val Arg Glu Tyr His Met Asp Gln Val Gln Ala Val Thr Leu
       50                  55                  60
   atc aac act tgg tct gct ctc acc aac ttt gct cct atc atc gga gct     240
   Ile Asn Thr Trp Ser Ala Leu Thr Asn Phe Ala Pro Ile Ile Gly Ala
   65                  70                  75                  80
   ttc atc tcc gac tcc tac acc gga aaa ttt aat acc att gtc ttc ggt     288
   Phe Ile Ser Asp Ser Tyr Thr Gly Lys Phe Asn Thr Ile Val Phe Gly
                   85                  90                  95
   tcc atc gct gag ttg ttg ggc atg ttg gtg ttg act ttc acg tct ctg     336
   Ser Ile Ala Glu Leu Leu Gly Met Leu Val Leu Thr Phe Thr Ser Leu
               100                 105                 110
   gtc cct aat cta cga cca cca cct tgc act gcc gac caa atc act gga     384
   Val Pro Asn Leu Arg Pro Pro Pro Cys Thr Ala Asp Gln Ile Thr Gly
           115                 120                 125
   caa tgt att ccc tat agc tat tcg cag tta tac gtt tta ctc tca gga     432
   Gln Cys Ile Pro Tyr Ser Tyr Ser Gln Leu Tyr Val Leu Leu Ser Gly
       130                 135                 140
   ctt ttc ttg tta tca gtt gga aca ggt gga atc cgg tca tgc agt gtt     480
   Leu Phe Leu Leu Ser Val Gly Thr Gly Gly Ile Arg Ser Cys Ser Val
   145                 150                 155                 160
   cct ttc agt ctc gat cag ttc gat gat tcg act gaa gaa gga aga gaa     528
   Pro Phe Ser Leu Asp Gln Phe Asp Asp Ser Thr Glu Glu Gly Arg Glu
                   165                 170                 175
   gga agt aga agc ttc ttt agc tgg tac tat acc act cat aca atc gtc     576
   Gly Ser Arg Ser Phe Phe Ser Trp Tyr Tyr Thr Thr His Thr Ile Val
               180                 185                 190
   cag tta gtt tca atg aca cta gtg ttg tac gta cag aac aac atc agt     624
   Gln Leu Val Ser Met Thr Leu Val Leu Tyr Val Gln Asn Asn Ile Ser
           195                 200                 205
   tgg ggc att gga ttc gca atc ccg acc gtt ctc aat ttt ttt gcg ctt     672
   Trp Gly Ile Gly Phe Ala Ile Pro Thr Val Leu Asn Phe Phe Ala Leu
       210                 215                 220
   ctt ctg ctt ttt gtg ggt act cgg tat tat gtc ttt gtc aaa ccc gaa     720
   Leu Leu Leu Phe Val Gly Thr Arg Tyr Tyr Val Phe Val Lys Pro Glu
   225                 230                 235                 240
   gga agt gtc ttc tct gga gtt ttc aag gtt ctt gtg gct gct tat aag     768
   Gly Ser Val Phe Ser Gly Val Phe Lys Val Leu Val Ala Ala Tyr Lys
                   245                 250                 255
   aaa cgc aag gca cgg ttt act tct gga att gac tac cat caa ccc tta     816
   Lys Arg Lys Ala Arg Phe Thr Ser Gly Ile Asp Tyr His Gln Pro Leu
               260                 265                 270
   cta gag aca gat tta caa tca aat aaa ctc gta ctc act gat caa ttc     864
   Leu Glu Thr Asp Leu Gln Ser Asn Lys Leu Val Leu Thr Asp Gln Phe
           275                 280                 285
   aga ttc ttg aac aaa gct gtg ata gtg atg aac aat gat gaa gct gga      912
   Arg Phe Leu Asn Lys Ala Val Ile Val Met Asn Asn Asp Glu Ala Gly
       290                 295                 300
   aat gag gag tgg aga acc tgc aca gtg aga cag ata gag gac atc aag      960
   Asn Glu Glu Trp Arg Thr Cys Thr Val Arg Gln Ile Glu Asp Ile Lys
   305                 310                 315                 320
   tca ata atc agt att att cca ata ttt gct tcg agc att att gga ttt     1008
   Ser Ile Ile Ser Ile Ile Pro Ile Phe Ala Ser Ser Ile Ile Gly Phe
                   325                 330                 335
   ttg gct atg aac caa caa cag acg ttt aca gtc tca caa gca cta aaa     1056
   Leu Ala Met Asn Gln Gln Gln Thr Phe Thr Val Ser Gln Ala Leu Lys
               340                 345                 350
   atg gac ctc caa ttt cca ggc act tct tac cta atc cct cct gct tct     1104
   Met Asp Leu Gln Phe Pro Gly Thr Ser Tyr Leu Ile Pro Pro Ala Ser
           355                 360                 365
   ata acc gtc ata tca tta tta aac ata ggt ata tgg ctt ccc ttc tat     1152
   Ile Thr Val Ile Ser Leu Leu Asn Ile Gly Ile Trp Leu Pro Phe Tyr
       370                 375                 380
   gaa acc gtt ttg gtc cga cac atc gaa aac att aca aaa caa aac gga     1200
   Glu Thr Val Leu Val Arg His Ile Glu Asn Ile Thr Lys Gln Asn Gly
   385                 390                 395                 400
   ggt atc tcg tta cta caa aaa gtc ggc att ggg aat att ttc tcc atc     1248
   Gly Ile Ser Leu Leu Gln Lys Val Gly Ile Gly Asn Ile Phe Ser Ile
                   405                 410                 415
   tcg acg atg ctc ata tcc ggt att gtg gag cga aag aga aga gat tta     1296
   Ser Thr Met Leu Ile Ser Gly Ile Val Glu Arg Lys Arg Arg Asp Leu
               420                 425                 430
   tcg ctt aat gga gta aaa atg tcg gtt ttc tgg cta acc ccg caa caa     1344
   Ser Leu Asn Gly Val Lys Met Ser Val Phe Trp Leu Thr Pro Gln Gln
           435                 440                 445
   gta cta atg gga ttt tat caa gtc ttc acg att gtt ggt ctc acc gag     1392
   Val Leu Met Gly Phe Tyr Gln Val Phe Thr Ile Val Gly Leu Thr Glu
       450                 455                 460
   ttc ttc aac aaa caa gtt cca atc aac atg aga agc att ggg aac tcg     1440
   Phe Phe Asn Lys Gln Val Pro Ile Asn Met Arg Ser Ile Gly Asn Ser
   465                 470                 475                 480
   ttg ctt tac tta ggt ttg tct cta gcc agt tat cta agc agt gcc atg     1488
   Leu Leu Tyr Leu Gly Leu Ser Leu Ala Ser Tyr Leu Ser Ser Ala Met
                   485                 490                 495
   gtg agc att gtt cat agt gtg act gct cgt gga gga cag agt tgg cta     1536
   Val Ser Ile Val His Ser Val Thr Ala Arg Gly Gly Gln Ser Trp Leu
               500                 505                 510
   acg gat gat att gac aaa agc aag ttg gat tgt ttc tat tac ttt att     1584
   Thr Asp Asp Ile Asp Lys Ser Lys Leu Asp Cys Phe Tyr Tyr Phe Ile
           515                 520                 525
   gcc gct tta agc act ctt aat ttc atc ttc ttc ttc tgg tgt gct cga     1632
   Ala Ala Leu Ser Thr Leu Asn Phe Ile Phe Phe Phe Trp Cys Ala Arg
       530                 535                 540
   agg tat cgt tac aga aac tat agt aat gaa caa taa                     1668
   Arg Tyr Arg Tyr Arg Asn Tyr Ser Asn Glu Gln
   545                 550                 555
<210> 8
   <211> 555
   <212> PRT
   <213> Arabidopsis thaliana
<400> 8
   Met Asp Asn Glu Lys Gly Thr Ser Ser Ser Asp Leu Thr Thr Arg Gln
   1               5                   10                  15
   Arg Lys Pro Leu Gly Trp Lys Ala Met Pro Tyr Ile Ile Gly Asn Glu
               20                  25                  30
   Thr Leu Glu Arg Leu Ala Thr Phe Gly Leu Met Ala Asn Phe Met Val
           35                  40                  45
   Tyr Met Val Arg Glu Tyr His Met Asp Gln Val Gln Ala Val Thr Leu
       50                  55                  60
   Ile Asn Thr Trp Ser Ala Leu Thr Asn Phe Ala Pro Ile Ile Gly Ala
   65                  70                  75                  80
   Phe Ile Ser Asp Ser Tyr Thr Gly Lys Phe Asn Thr Ile Val Phe Gly
                   85                  90                  95
   Ser Ile Ala Glu Leu Leu Gly Met Leu Val Leu Thr Phe Thr Ser Leu
               100                 105                 110
   Val Pro Asn Leu Arg Pro Pro Pro Cys Thr Ala Asp Gln Ile Thr Gly
           115                 120                 125
   Gln Cys Ile Pro Tyr Ser Tyr Ser Gln Leu Tyr Val Leu Leu Ser Gly
       130                 135                 140
   Leu Phe Leu Leu Ser Val Gly Thr Gly Gly Ile Arg Ser Cys Ser Val
   145                 150                 155                 160
   Pro Phe Ser Leu Asp Gln Phe Asp Asp Ser Thr Glu Glu Gly Arg Glu
                   165                 170                 175
   Gly Ser Arg Ser Phe Phe Ser Trp Tyr Tyr Thr Thr His Thr Ile Val
               180                 185                 190
   Gln Leu Val Ser Met Thr Leu Val Leu Tyr Val Gln Asn Asn Ile Ser
           195                 200                 205
   Trp Gly Ile Gly Phe Ala Ile Pro Thr Val Leu Asn Phe Phe Ala Leu
       210                 215                 220
   Leu Leu Leu Phe Val Gly Thr Arg Tyr Tyr Val Phe Val Lys Pro Glu
   225                 230                 235                 240
   Gly Ser Val Phe Ser Gly Val Phe Lys Val Leu Val Ala Ala Tyr Lys
                   245                 250                 255
   Lys Arg Lys Ala Arg Phe Thr Ser Gly Ile Asp Tyr His Gln Pro Leu
               260                 265                 270
   Leu Glu Thr Asp Leu Gln Ser Asn Lys Leu Val Leu Thr Asp Gln Phe
           275                 280                 285
   Arg Phe Leu Asn Lys Ala Val Ile Val Met Asn Asn Asp Glu Ala Gly
       290                 295                 300
   Asn Glu Glu Trp Arg Thr Cys Thr Val Arg Gln Ile Glu Asp Ile Lys
   305                 310                 315                 320
   Ser Ile Ile Ser Ile Ile Pro Ile Phe Ala Ser Ser Ile Ile Gly Phe
                   325                 330                 335
   Leu Ala Met Asn Gln Gln Gln Thr Phe Thr Val Ser Gln Ala Leu Lys
               340                 345                 350
   Met Asp Leu Gln Phe Pro Gly Thr Ser Tyr Leu Ile Pro Pro Ala Ser
           355                 360                 365
   Ile Thr Val Ile Ser Leu Leu Asn Ile Gly Ile Trp Leu Pro Phe Tyr
       370                 375                 380
   Glu Thr Val Leu Val Arg His Ile Glu Asn Ile Thr Lys Gln Asn Gly
   385                 390                 395                 400
   Gly Ile Ser Leu Leu Gln Lys Val Gly Ile Gly Asn Ile Phe Ser Ile
                   405                 410                 415
   Ser Thr Met Leu Ile Ser Gly Ile Val Glu Arg Lys Arg Arg Asp Leu
               420                 425                 430
   Ser Leu Asn Gly Val Lys Met Ser Val Phe Trp Leu Thr Pro Gln Gln
           435                 440                 445
   Val Leu Met Gly Phe Tyr Gln Val Phe Thr Ile Val Gly Leu Thr Glu
       450                 455                 460
   Phe Phe Asn Lys Gln Val Pro Ile Asn Met Arg Ser Ile Gly Asn Ser
   465                 470                 475                 480
   Leu Leu Tyr Leu Gly Leu Ser Leu Ala Ser Tyr Leu Ser Ser Ala Met
                   485                 490                 495
   Val Ser Ile Val His Ser Val Thr Ala Arg Gly Gly Gln Ser Trp Leu
               500                 505                 510
   Thr Asp Asp Ile Asp Lys Ser Lys Leu Asp Cys Phe Tyr Tyr Phe Ile
           515                 520                 525
   Ala Ala Leu Ser Thr Leu Asn Phe Ile Phe Phe Phe Trp Cys Ala Arg
       530                 535                 540
   Arg Tyr Arg Tyr Arg Asn Tyr Ser Asn Glu Gln
   545                 550                 555
<210> 9
   <211> 1863
   <212> DNA
   <213> Arabidopsis thaliana
<220>
   <221> CDS
   <222> (1)..(1863)
<400> 9
   atg gtt ttg gag gat aga aag gac ggt tct tct ttg ccg gga cga tcc      48
   Met Val Leu Glu Asp Arg Lys Asp Gly Ser Ser Leu Pro Gly Arg Ser
   1               5                   10                  15
   ggt agt ttc tct aaa tcg tca ccg tcg gag ttg gat gtt gtt gat ccc      96
   Gly Ser Phe Ser Lys Ser Ser Pro Ser Glu Leu Asp Val Val Asp Pro
               20                  25                  30
   tac aag cgg ata agt tcg ccg gga tct ata ttg gat gct gag aag gta     144
   Tyr Lys Arg Ile Ser Ser Pro Gly Ser Ile Leu Asp Ala Glu Lys Val
           35                  40                  45
   gag aaa aag cct gga gga tgg aga gcc gtc tcg ttc att tta gga aat     192
   Glu Lys Lys Pro Gly Gly Trp Arg Ala Val Ser Phe Ile Leu Gly Asn
       50                  55                  60
   gag acg ctg gag aga ctg gga tcg ata gga ttg ttg gca aac ttc atg     240
   Glu Thr Leu Glu Arg Leu Gly Ser Ile Gly Leu Leu Ala Asn Phe Met
   65                  70                  75                  80
   gtt tat cta acc aaa gtg ttt cac tta gaa caa gtc gac gct gca aat     288
   Val Tyr Leu Thr Lys Val Phe His Leu Glu Gln Val Asp Ala Ala Asn
                   85                  90                  95
   gtc atc aac att tgg tca ggt ttc acc aat ctc act cct ctc gtc gga     336
   Val Ile Asn Ile Trp Ser Gly Phe Thr Asn Leu Thr Pro Leu Val Gly
               100                 105                 110
   gcg tat atc tca gac act tat gtt ggc cgc ttc aag acc atc gct ttc     384
   Ala Tyr Ile Ser Asp Thr Tyr Val Gly Arg Phe Lys Thr Ile Ala Phe
           115                 120                 125
   gcc tca ttc gcc act ctc ctc gga cta ata aca att aca ctc aca gca     432
   Ala Ser Phe Ala Thr Leu Leu Gly Leu Ile Thr Ile Thr Leu Thr Ala
       130                 135                 140
   tcg ttt cct caa ctc cac cca gca tca tgc aac agc cag gac cca ctc     480
   Ser Phe Pro Gln Leu His Pro Ala Ser Cys Asn Ser Gln Asp Pro Leu
   145                 150                 155                 160
   agt tgc ggc ggt ccg aat aag ctc cag atc gga gtt ttg cta ttg gga     528
   Ser Cys Gly Gly Pro Asn Lys Leu Gln Ile Gly Val Leu Leu Leu Gly
                   165                 170                 175
   ctc tgt ttc ctc tcc gta ggg agt gga gga ata cga cct tgt agc atc     576
   Leu Cys Phe Leu Ser Val Gly Ser Gly Gly Ile Arg Pro Cys Ser Ile
               180                 185                 190
   cct ttt ggg gtt gat cag ttt gac caa cga act gag gaa ggg gtt aaa     624
   Pro Phe Gly Val Asp Gln Phe Asp Gln Arg Thr Glu Glu Gly Val Lys
           195                 200                 205
   gga gtg gcc agt ttc ttc aac tgg tat tac atg act ttc act gtg gtt     672
   Gly Val Ala Ser Phe Phe Asn Trp Tyr Tyr Met Thr Phe Thr Val Val
       210                 215                 220
   ctg atc att aca cag acc gta gtt gtg tat atc cag gat caa gtc agt     720
   Leu Ile Ile Thr Gln Thr Val Val Val Tyr Ile Gln Asp Gln Val Ser
   225                 230                 235                 240
   tgg att atc ggt ttt agt atc cct acc gga ctc atg gct ctt gcg gtt     768
   Trp Ile Ile Gly Phe Ser Ile Pro Thr Gly Leu Met Ala Leu Ala Val
                   245                 250                 255
   gtt atg ttt ttt gcc gga atg aag cgt tat gtc tac gtt aaa cca gaa     816
   Val Met Phe Phe Ala Gly Met Lys Arg Tyr Val Tyr Val Lys Pro Glu
               260                 265                 270
   gga agt ata ttc tct ggg atc gct caa gtt atc gtg gca gct cgt aag     864
   Gly Ser Ile Phe Ser Gly Ile Ala Gln Val Ile Val Ala Ala Arg Lys
           275                 280                 285
   aag cga aag ctg aaa ctt ccg gcg gaa gat gac ggc act gtc acc tat     912
   Lys Arg Lys Leu Lys Leu Pro Ala Glu Asp Asp Gly Thr Val Thr Tyr
       290                 295                 300
   tac gac cca gcc atc aag tct agc gtg tta tcc aag tta cac cgc agt       960
   Tyr Asp Pro Ala Ile Lys Ser Ser Val Leu Ser Lys Leu His Arg Ser
   305                 310                 315                 320
   aac caa ttc agg tgt ctt gac aaa gcc gcg gtg gtt ata gaa ggt gac      1008
   Asn Gln Phe Arg Cys Leu Asp Lys Ala Ala Val Val Ile Glu Gly Asp
                   325                 330                 335
   cta aca ccc gag gga cct ccc gca gac aag tgg cgg tta tgc agc gtc      1056
   Leu Thr Pro Glu Gly Pro Pro Ala Asp Lys Trp Arg Leu Cys Ser Val
               340                 345                 350
   caa gaa gtg gaa gaa gtg aag tgt ttg atc cga att gtt cct atc tgg      1104
   Gln Glu Val Glu Glu Val Lys Cys Leu Ile Arg Ile Val Pro Ile Trp
           355                 360                 365
   tcg gcc gga ata atc tca ctc gcg gcc atg aca aca caa ggc act ttc      1152
   Ser Ala Gly Ile Ile Ser Leu Ala Ala Met Thr Thr Gln Gly Thr Phe
       370                 375                 380
   acg gtc tct caa gct ttg aaa atg gat cga aac tta ggt cct aaa ttc      1200
   Thr Val Ser Gln Ala Leu Lys Met Asp Arg Asn Leu Gly Pro Lys Phe
   385                 390                 395                 400
   gag att ccg gct ggt tca ctc tcc gtc atc tct ctc ctc aca atc ggc      1248
   Glu Ile Pro Ala Gly Ser Leu Ser Val Ile Ser Leu Leu Thr Ile Gly
                   405                 410                 415
   atc ttt ctt ccc ttc tac gac cgc gtt ttt gta cca ttc atg cgg cga      1296
   Ile Phe Leu Pro Phe Tyr Asp Arg Val Phe Val Pro Phe Met Arg Arg
               420                 425                 430
   atc acc ggc cat aaa tcc gga atc aca ctc ctc caa agg ata gga aca      1344
   Ile Thr Gly His Lys Ser Gly Ile Thr Leu Leu Gln Arg Ile Gly Thr
           435                 440                 445
   ggg atc gtt ttc gcg atc ttt tct atg atc gtt gcg ggc att gtg gag      1392
   Gly Ile Val Phe Ala Ile Phe Ser Met Ile Val Ala Gly Ile Val Glu
       450                 455                 460
   cgt atg aga cgc ata cgc tcc atc aat gcc gga gat cca acg gga atg      1440
   Arg Met Arg Arg Ile Arg Ser Ile Asn Ala Gly Asp Pro Thr Gly Met
   465                 470                 475                 480
   act cca atg tcg gtg ttt tgg ctt tcg ccg cag cta att ctc atg gga      1488
   Thr Pro Met Ser Val Phe Trp Leu Ser Pro Gln Leu Ile Leu Met Gly
                   485                 490                 495
   cta tgt gaa gca ttc aat atc atc gga caa att gag ttc ttc aac agt      1536
   Leu Cys Glu Ala Phe Asn Ile Ile Gly Gln Ile Glu Phe Phe Asn Ser
               500                 505                 510
   cag ttt cca gag cac atg aga agt atc gct aac tct ctc ttc tct tta      1584
   Gln Phe Pro Glu His Met Arg Ser Ile Ala Asn Ser Leu Phe Ser Leu
           515                 520                 525
   tcg ttc gcc ggt tcg agc tac ctt agt agt ttc ctt gtg act gtc gtt      1632
   Ser Phe Ala Gly Ser Ser Tyr Leu Ser Ser Phe Leu Val Thr Val Val
       530                 535                 540
   cat aaa ttc tcc ggt ggg cat gat cgt ccg gat tgg cta aac aag aat      1680
   His Lys Phe Ser Gly Gly His Asp Arg Pro Asp Trp Leu Asn Lys Asn
   545                 550                 555                 560
   ctc aac gcg gga aaa ttg gat tac ttc tat tat ctg att gcg gtt ttg      1728
   Leu Asn Ala Gly Lys Leu Asp Tyr Phe Tyr Tyr Leu Ile Ala Val Leu
                   565                 570                 575
   ggt gtg gtt aat ctg gtt tac ttt tgg tat tgt gct cgg gga tac cgg      1776
   Gly Val Val Asn Leu Val Tyr Phe Trp Tyr Cys Ala Arg Gly Tyr Arg
               580                 585                 590
   tac aag gtc ggt tta ccg att gaa gac ttt gag gag gac aag tcc tcc      1824
   Tyr Lys Val Gly Leu Pro Ile Glu Asp Phe Glu Glu Asp Lys Ser Ser
           595                 600                 605
   gat gat gtt gag atg act tcg aag aaa tcg atg aaa tga                1863
   Asp Asp Val Glu Met Thr Ser Lys Lys Ser Met Lys
       610                 615                 620
<210> 10
   <211> 620
   <212> PRT
   <213> Arabidopsis thaliana
<400> 10
   Met Val Leu Glu Asp Arg Lys Asp Gly Ser Ser Leu Pro Gly Arg Ser
   1               5                   10                  15
   Gly Ser Phe Ser Lys Ser Ser Pro Ser Glu Leu Asp Val Val Asp Pro
               20                  25                  30
   Tyr Lys Arg Ile Ser Ser Pro Gly Ser Ile Leu Asp Ala Glu Lys Val
           35                  40                  45
   Glu Lys Lys Pro Gly Gly Trp Arg Ala Val Ser Phe Ile Leu Gly Asn
       50                  55                  60
   Glu Thr Leu Glu Arg Leu Gly Ser Ile Gly Leu Leu Ala Asn Phe Met
   65                  70                  75                  80
   Val Tyr Leu Thr Lys Val Phe His Leu Glu Gln Val Asp Ala Ala Asn
                   85                  90                  95
   Val Ile Asn Ile Trp Ser Gly Phe Thr Asn Leu Thr Pro Leu Val Gly
               100                 105                 110
   Ala Tyr Ile Ser Asp Thr Tyr Val Gly Arg Phe Lys Thr Ile Ala Phe
           115                 120                 125
   Ala Ser Phe Ala Thr Leu Leu Gly Leu Ile Thr Ile Thr Leu Thr Ala
       130                 135                 140
   Ser Phe Pro Gln Leu His Pro Ala Ser Cys Asn Ser Gln Asp Pro Leu
   145                 150                 155                 160
   Ser Cys Gly Gly Pro Asn Lys Leu Gln Ile Gly Val Leu Leu Leu Gly
                   165                 170                 175
   Leu Cys Phe Leu Ser Val Gly Ser Gly Gly Ile Arg Pro Cys Ser Ile
               180                 185                 190
   Pro Phe Gly Val Asp Gln Phe Asp Gln Arg Thr Glu Glu Gly Val Lys
           195                 200                 205
   Gly Val Ala Ser Phe Phe Asn Trp Tyr Tyr Met Thr Phe Thr Val Val
       210                 215                 220
   Leu Ile Ile Thr Gln Thr Val Val Val Tyr Ile Gln Asp Gln Val Ser
   225                 230                 235                 240
   Trp Ile Ile Gly Phe Ser Ile Pro Thr Gly Leu Met Ala Leu Ala Val
                   245                 250                 255
   Val Met Phe Phe Ala Gly Met Lys Arg Tyr Val Tyr Val Lys Pro Glu
   260 265 270
   Gly Ser Ile Phe Ser Gly Ile Ala Gln Val Ile Val Ala Ala Arg Lys
   275 280 285
   Lys Arg Lys Leu Lys Leu Pro Ala Glu Asp Asp Gly Thr Val Thr Tyr
   290 295 300
   Tyr Asp Pro Ala Ile Lys Ser Ser Val Leu Ser Lys Leu His Arg Ser
   305 310 315 320
   Asn Gln Phe Arg Cys Leu Asp Lys Ala Ala Val Val Ile Glu Gly Asp
   325 330 335
   Leu Thr Pro Glu Gly Pro Pro Ala Asp Lys Trp Arg Leu Cys Ser Val
   340 345 350
   Gln Glu Val Glu Glu Val Lys Cys Leu Ile Arg Ile Val Pro Ile Trp
   355 360 365
   Ser Ala Gly Ile Ile Ser Leu Ala Ala Met Thr Thr Gln Gly Thr Phe
   370 375 380
   Thr Val Ser Gln Ala Leu Lys Met Asp Arg Asn Leu Gly Pro Lys Phe
   385 390 395 400
   Glu Ile Pro Ala Gly Ser Leu Ser Val Ile Ser Leu Leu Thr Ile Gly
   405 410 415
   Ile Phe Leu Pro Phe Tyr Asp Arg Val Phe Val Pro Phe Met Arg Arg
   420 425 430
   Ile Thr Gly His Lys Ser Gly Ile Thr Leu Leu Gln Arg Ile Gly Thr
   435 440 445
   Gly Ile Val Phe Ala Ile Phe Ser Met Ile Val Ala Gly Ile Val Glu
   450 455 460
   Arg Met Arg Arg Ile Arg Ser Ile Asn Ala Gly Asp Pro Thr Gly Met
   465 470 475 480
   Thr Pro Met Ser Val Phe Trp Leu Ser Pro Gln Leu Ile Leu Met Gly
   485 490 495
   Leu Cys Glu Ala Phe Asn Ile Ile Gly Gln Ile Glu Phe Phe Asn Ser
   500 505 510
   Gln Phe Pro Glu His Met Arg Ser Ile Ala Asn Ser Leu Phe Ser Leu
   515 520 525
   Ser Phe Ala Gly Ser Ser Tyr Leu Ser Ser Phe Leu Val Thr Val Val
   530 535 540
   His Lys Phe Ser Gly Gly His Asp Arg Pro Asp Trp Leu Asn Lys Asn
   545 550 555 560
   Leu Asn Ala Gly Lys Leu Asp Tyr Phe Tyr Tyr Leu Ile Ala Val Leu
                   565                 570                 575
   Gly Val Val Asn Leu Val Tyr Phe Trp Tyr Cys Ala Arg Gly Tyr Arg
               580                 585                 590
   Tyr Lys Val Gly Leu Pro Ile Glu Asp Phe Glu Glu Asp Lys Ser Ser
           595                 600                 605
   Asp Asp Val Glu Met Thr Ser Lys Lys Ser Met Lys
       610                 615                 620
<210> 11
   <211> 1731
   <212> DNA
   <213> Arabidopsis thaliana
<220>
   <221> CDS
   <222> (1)..(1731)
<400> 11
   atg gga gtt gtt gag aat cgg aaa att ttg cca gag aaa aaa ctt gga      48
   Met Gly Val Val Glu Asn Arg Lys Ile Leu Pro Glu Lys Lys Leu Gly
   1               5                   10                  15
   ggc tgg aga gcc att acg ttc atc cta ggt aat gag acg ttg gag aag      96
   Gly Trp Arg Ala Ile Thr Phe Ile Leu Gly Asn Glu Thr Leu Glu Lys
               20                  25                  30
   tta gga tcg att gga gta tca gca aat ttt atg ttg tat ttg agg aat     144
   Leu Gly Ser Ile Gly Val Ser Ala Asn Phe Met Leu Tyr Leu Arg Asn
           35                  40                  45
   gtg ttt cac atg gag cca gta gaa gct ttt aac gta tac tac ttg tgg     192
   Val Phe His Met Glu Pro Val Glu Ala Phe Asn Val Tyr Tyr Leu Trp
       50                  55                  60
   atg ggt ctc act aac ttt gct cca ctc ctc ggc gca tta atc tcc gac     240
   Met Gly Leu Thr Asn Phe Ala Pro Leu Leu Gly Ala Leu Ile Ser Asp
   65                  70                  75                  80
   gcc tac att ggc cgt ttc aag acc atc gct tac gca tcc ctc ttc tca     288
   Ala Tyr Ile Gly Arg Phe Lys Thr Ile Ala Tyr Ala Ser Leu Phe Ser
                   85                  90                  95
   att cta gga cta atg acg gtg aca ctc acc gcc tgc ctg cct caa ctc     336
   Ile Leu Gly Leu Met Thr Val Thr Leu Thr Ala Cys Leu Pro Gln Leu
               100                 105                 110
   cac cca cca ccg tgc aac aac cct cat cca gac gaa tgc gac gat ccg     384
   His Pro Pro Pro Cys Asn Asn Pro His Pro Asp Glu Cys Asp Asp Pro
           115                 120                 125
   aac aag ctc cag ctc ggg att cta ttc ctc ggt ctc ggc ttt ctc tcc     432
   Asn Lys Leu Gln Leu Gly Ile Leu Phe Leu Gly Leu Gly Phe Leu Ser
       130                 135                 140
   atc ggt agc ggc gga atc cgg cct tgc agc att cca ttc gga gtt gat     480
   Ile Gly Ser Gly Gly Ile Arg Pro Cys Ser Ile Pro Phe Gly Val Asp
   145                 150                 155                 160
   cag ttt gac caa cga aca gag caa ggc ctt aaa gga gtg gcc agt ttc     528
   Gln Phe Asp Gln Arg Thr Glu Gln Gly Leu Lys Gly Val Ala Ser Phe
                   165                 170                 175
   ttt aat tgg tac tac ttg acc tta act atg gtt ctc atc ttt tcg cat     576
   Phe Asn Trp Tyr Tyr Leu Thr Leu Thr Met Val Leu Ile Phe Ser His
               180                 185                 190
   acc gtt gtg gtc tac tta cag act gtc agt tgg gtc atc ggg ttt agc     624
   Thr Val Val Val Tyr Leu Gln Thr Val Ser Trp Val Ile Gly Phe Ser
           195                 200                 205
   atc ccg act agt tta atg gct tgt gca gtg gtt ctg ttc ttt gtc ggt      672
   Ile Pro Thr Ser Leu Met Ala Cys Ala Val Val Leu Phe Phe Val Gly
       210                 215                 220
   atg cgg ttt tac gta tat gtc aaa ccg gaa ggt agt gta ttc tct ggt      720
   Met Arg Phe Tyr Val Tyr Val Lys Pro Glu Gly Ser Val Phe Ser Gly
   225                 230                 235                 240
   att gct cgg gtc atc gtg gca gct cgt aag aaa cgg gac ctc aaa att      768
   Ile Ala Arg Val Ile Val Ala Ala Arg Lys Lys Arg Asp Leu Lys Ile
                   245                 250                 255
   tcg ctt gtg gac gat ggg act gag gag tat tat gag ccg ccg gtg aaa      816
   Ser Leu Val Asp Asp Gly Thr Glu Glu Tyr Tyr Glu Pro Pro Val Lys
               260                 265                 270
   cct ggc gtg tta tcc aag tta cct ctt act gac caa ttc aag ttt ctg      864
   Pro Gly Val Leu Ser Lys Leu Pro Leu Thr Asp Gln Phe Lys Phe Leu
           275                 280                 285
   gac aaa gct gcg gtg ata cta gac ggt gat ctt aca tct gaa gga gtt      912
   Asp Lys Ala Ala Val Ile Leu Asp Gly Asp Leu Thr Ser Glu Gly Val
       290                 295                 300
   ccg gca aat aag tgg cgg cta tgt agc atc caa gaa gtt gaa gag gtc      960
   Pro Ala Asn Lys Trp Arg Leu Cys Ser Ile Gln Glu Val Glu Glu Val
   305                 310                 315                 320
   aag tgc ctg atc aga gtt gtt ccg gtt tgg tct gct gga ata att tca     1008
   Lys Cys Leu Ile Arg Val Val Pro Val Trp Ser Ala Gly Ile Ile Ser
                   325                 330                 335
   atc gtt gca atg acc acg caa gca act ttc atg gtc ttt caa gct aca     1056
   Ile Val Ala Met Thr Thr Gln Ala Thr Phe Met Val Phe Gln Ala Thr
               340                 345                 350
   aaa atg gat cga cac atg ggt cca cac ttt gag atc ccg gcc gcc tcc     1104
   Lys Met Asp Arg His Met Gly Pro His Phe Glu Ile Pro Ala Ala Ser
           355                 360                 365
   ata acc gtc att tct tac atc act ata ggc att tgg gtg cct atc tac     1152
   Ile Thr Val Ile Ser Tyr Ile Thr Ile Gly Ile Trp Val Pro Ile Tyr
       370                 375                 380
   gaa cac ctc tta gtc ccg ttc ctt tgg cga atg aga aag ttc agg gtc     1200
   Glu His Leu Leu Val Pro Phe Leu Trp Arg Met Arg Lys Phe Arg Val
   385                 390                 395                 400
   act ctc ctt caa cgg atg ggg ata ggg atc gtc ttc gcc att ctc tcc     1248
   Thr Leu Leu Gln Arg Met Gly Ile Gly Ile Val Phe Ala Ile Leu Ser
                   405                 410                 415
   atg ttc act gca ggg ttt gta gag gga gtg agg cgg aca cgg gct act     1296
   Met Phe Thr Ala Gly Phe Val Glu Gly Val Arg Arg Thr Arg Ala Thr
               420                 425                 430
   gaa atg act caa atg tcg gtg ttt tgg ctg gct ttg cca ctg att ctg     1344
   Glu Met Thr Gln Met Ser Val Phe Trp Leu Ala Leu Pro Leu Ile Leu
           435                 440                 445
   atg ggg ctc tgc gag tca ttt aat ttc att gga ctg atc gag ttc ttc     1392
   Met Gly Leu Cys Glu Ser Phe Asn Phe Ile Gly Leu Ile Glu Phe Phe
       450                 455                 460
   aac agt cag ttt cca gag cac atg aga agc ata gcg aat tcg cta ttt     1440
   Asn Ser Gln Phe Pro Glu His Met Arg Ser Ile Ala Asn Ser Leu Phe
   465                 470                 475                 480
   cca ctc tca ttt gct gca gct aat tat ctg agc agt ctg ctg gtg acc     1488
   Pro Leu Ser Phe Ala Ala Ala Asn Tyr Leu Ser Ser Leu Leu Val Thr
                   485                 490                 495
   acc gtg cat aaa gtc tca ggc aca aaa gac cac cct gac tgg ctg aac     1536
   Thr Val His Lys Val Ser Gly Thr Lys Asp His Pro Asp Trp Leu Asn
               500                 505                 510
   aag gac ctt gat aga ggg aag ttg gac tac ttc tac tat ctg att gcc      1584
   Lys Asp Leu Asp Arg Gly Lys Leu Asp Tyr Phe Tyr Tyr Leu Ile Ala
           515                 520                 525
   gtt ttg ggt gtg gtt aac ctt gtg tat ttc tgg tac tgt gcc cat aga      1632
   Val Leu Gly Val Val Asn Leu Val Tyr Phe Trp Tyr Cys Ala His Arg
       530                 535                 540
   tac cag tat aaa gcc ggt tcg caa att gaa gat ttc aat gag gag aag      1680
   Tyr Gln Tyr Lys Ala Gly Ser Gln Ile Glu Asp Phe Asn Glu Glu Lys
   545                 550                 555                 560
   tct ctt cta gac atc gaa cca aac cag aga cac gat caa tct cca agt      1728
   Ser Leu Leu Asp Ile Glu Pro Asn Gln Arg His Asp Gln Ser Pro Ser
                   565                 570                 575
   tag                                                                  1731
<210> 12
   <211> 576
   <212> PRT
   <213> Arabidopsis thaliana
<400> 12
   Met Gly Val Val Glu Asn Arg Lys Ile Leu Pro Glu Lys Lys Leu Gly
   1               5                   10                  15
   Gly Trp Arg Ala Ile Thr Phe Ile Leu Gly Asn Glu Thr Leu Glu Lys
               20                  25                  30
   Leu Gly Ser Ile Gly Val Ser Ala Asn Phe Met Leu Tyr Leu Arg Asn
           35                  40                  45
   Val Phe His Met Glu Pro Val Glu Ala Phe Asn Val Tyr Tyr Leu Trp
       50                  55                  60
   Met Gly Leu Thr Asn Phe Ala Pro Leu Leu Gly Ala Leu Ile Ser Asp
   65                  70                  75                  80
   Ala Tyr Ile Gly Arg Phe Lys Thr Ile Ala Tyr Ala Ser Leu Phe Ser
                   85                  90                  95
   Ile Leu Gly Leu Met Thr Val Thr Leu Thr Ala Cys Leu Pro Gln Leu
               100                 105                 110
   His Pro Pro Pro Cys Asn Asn Pro His Pro Asp Glu Cys Asp Asp Pro
           115                 120                 125
   Asn Lys Leu Gln Leu Gly Ile Leu Phe Leu Gly Leu Gly Phe Leu Ser
       130                 135                 140
   Ile Gly Ser Gly Gly Ile Arg Pro Cys Ser Ile Pro Phe Gly Val Asp
   145                 150                 155                 160
   Gln Phe Asp Gln Arg Thr Glu Gln Gly Leu Lys Gly Val Ala Ser Phe
                   165                 170                 175
   Phe Asn Trp Tyr Tyr Leu Thr Leu Thr Met Val Leu Ile Phe Ser His
               180                 185                 190
   Thr Val Val Val Tyr Leu Gln Thr Val Ser Trp Val Ile Gly Phe Ser
           195                 200                 205
   Ile Pro Thr Ser Leu Met Ala Cys Ala Val Val Leu Phe Phe Val Gly
       210                 215                 220
   Met Arg Phe Tyr Val Tyr Val Lys Pro Glu Gly Ser Val Phe Ser Gly
   225                 230                 235                 240
   Ile Ala Arg Val Ile Val Ala Ala Arg Lys Lys Arg Asp Leu Lys Ile
                   245                 250                 255
   Ser Leu Val Asp Asp Gly Thr Glu Glu Tyr Tyr Glu Pro Pro Val Lys
               260                 265                 270
   Pro Gly Val Leu Ser Lys Leu Pro Leu Thr Asp Gln Phe Lys Phe Leu
           275                 280                 285
   Asp Lys Ala Ala Val Ile Leu Asp Gly Asp Leu Thr Ser Glu Gly Val
       290                 295                 300
   Pro Ala Asn Lys Trp Arg Leu Cys Ser Ile Gln Glu Val Glu Glu Val
   305                 310                 315                 320
   Lys Cys Leu Ile Arg Val Val Pro Val Trp Ser Ala Gly Ile Ile Ser
                   325                 330                 335
   Ile Val Ala Met Thr Thr Gln Ala Thr Phe Met Val Phe Gln Ala Thr
               340                 345                 350
   Lys Met Asp Arg His Met Gly Pro His Phe Glu Ile Pro Ala Ala Ser
           355                 360                 365
   Ile Thr Val Ile Ser Tyr Ile Thr Ile Gly Ile Trp Val Pro Ile Tyr
       370                 375                 380
   Glu His Leu Leu Val Pro Phe Leu Trp Arg Met Arg Lys Phe Arg Val
   385                 390                 395                 400
   Thr Leu Leu Gln Arg Met Gly Ile Gly Ile Val Phe Ala Ile Leu Ser
                   405                 410                 415
   Met Phe Thr Ala Gly Phe Val Glu Gly Val Arg Arg Thr Arg Ala Thr
               420                 425                 430
   Glu Met Thr Gln Met Ser Val Phe Trp Leu Ala Leu Pro Leu Ile Leu
           435                 440                 445
   Met Gly Leu Cys Glu Ser Phe Asn Phe Ile Gly Leu Ile Glu Phe Phe
       450                 455                 460
   Asn Ser Gln Phe Pro Glu His Met Arg Ser Ile Ala Asn Ser Leu Phe
   465                 470                 475                 480
   Pro Leu Ser Phe Ala Ala Ala Asn Tyr Leu Ser Ser Leu Leu Val Thr
                   485                 490                 495
   Thr Val His Lys Val Ser Gly Thr Lys Asp His Pro Asp Trp Leu Asn
               500                 505                 510
   Lys Asp Leu Asp Arg Gly Lys Leu Asp Tyr Phe Tyr Tyr Leu Ile Ala
           515                 520                 525
   Val Leu Gly Val Val Asn Leu Val Tyr Phe Trp Tyr Cys Ala His Arg
       530                 535                 540
   Tyr Gln Tyr Lys Ala Gly Ser Gln Ile Glu Asp Phe Asn Glu Glu Lys
   545                 550                 555                 560
   Ser Leu Leu Asp Ile Glu Pro Asn Gln Arg His Asp Gln Ser Pro Ser
                   565                 570                 575
<210> 13
   <211> 2474
   <212> DNA
   <213> Brassica napus
<220>
   <221> CDS
   <222> (1)..(151)
<220>
   <221> CDS
   <222> (619)..(836)
<220>
   <221> CDS
   <222> (928)..(1481)
<220>
   <221> CDS
   <222> (1562)..(2471)
<400> 13
   atg gag aga aag ccc ttt gat gtt gag aca act gag gat cac aaa ctc        48
   Met Glu Arg Lys Pro Phe Asp Val Glu Thr Thr Glu Asp His Lys Leu
   1               5                   10                  15
   tac ccc tcc gtc gat ggc ggt ggt tct gat tcg gtc gat tca ttt gaa        96
   Tyr Pro Ser Val Asp Gly Gly Gly Ser Asp Ser Val Asp Ser Phe Glu
               20                  25                  30
   gac gag cag aaa aag ctc gtt tat aga ggc tgg aaa gtc atg ccc ttt       144
   Asp Glu Gln Lys Lys Leu Val Tyr Arg Gly Trp Lys Val Met Pro Phe
           35                  40                  45
   atc att g gtaacgcttt cttcttctct tcagacaact ttttttttag tttattctgt       201
   Ile Ile
       50
   tttgttttgt tgtgtctctt ttgtcggatt atagaacgtt ttgcccgtga tgtttttatt    261
   ttaatttcgt ttcttttgtc ttcttagact tgactgttga aaatcttgat ttggagttct    321
   tgtcttgacg tttcgagatt attgaaacac agatcaacca aaccagttga gtttcctaga    381
   taaatctacc atggaaagtg agagactttg aatcatgcaa gttttaagtt tctgaaataa    441
   gattttagtt agtttctggg agttataaca gttaaagaat gaatgaatga ataaagtgtc    501
   cagtccacca ctgaaagtcc aattctttga tttcaacatt gtgaattaaa tgtaaaaagg    561
   ttgttacttt atggtttgac taattattta tgttgaaaaa aatatattgt gtcacag       618
   gt  aat gag aca ttt gag aag ata ggg atc att ggg aca cta tca aac       665
   Gly Asn Glu Thr Phe Glu Lys Ile Gly Ile Ile Gly Thr Leu Ser Asn
   55 60 65
   ctt ctg gtg tac cta act tca gta ttc aac ctt aag agt gtt aca gct 713
   Leu Leu Val Tyr Leu Thr Ser Val Phe Asn Leu Lys Ser Val Thr Ala
   70 75 80
   gca acc atc atc aac gcc ttc agt ggc act atc aac ttc ggc act ttc 761
   Ala Thr Ile Ile Asn Ala Phe Ser Gly Thr Ile Asn Phe Gly Thr Phe
   85 90 95
   ctt gct gct ttc ctc tgc gac act tac ttt ggt cgc tac aag act ctc 809
   Leu Ala Ala Phe Leu Cys Asp Thr Tyr Phe Gly Arg Tyr Lys Thr Leu
   100 105 110
   tct gtt gct gtc atc gcc tgt ttt ctg gtactctaat caaatgacca 856
   Ser Val Ala Val Ile Ala Cys Phe Leu
   115 120
   tcttgtttta ttttggccaa gattttgtct tttttttttt attactaaac catatttcat 916
   atttcttgta g gga tcg ctt gtg ata cta ctg acg gct gca gtt cca gga 966
   Gly Ser Leu Val Ile Leu Leu Thr Ala Ala Val Pro Gly
   125 130 135
   ttg cac ccc att cct tgt gga aca cag agt tct tgc caa ggc cca agc 1014
   Leu His Pro Ile Pro Cys Gly Thr Gln Ser Ser Cys Gln Gly Pro Ser
   140 145 150
   gag ggc cag att gcg ttt ctt ctg atg ggt tta gcg ctt ctt gtg gtg 1062
   Glu Gly Gln Ile Ala Phe Leu Leu Met Gly Leu Ala Leu Leu Val Val
   155 160 165
   ggt gct ggt gga att agg ccg tgt aac ttg gcc ttt ggt gct gac cag 1110
   Gly Ala Gly Gly Ile Arg Pro Cys Asn Leu Ala Phe Gly Ala Asp Gln
   170 175 180
   ttc aac ccc aag tca gaa tct gga aag aaa gga atc aac agt ttc ttc 1158
   Phe Asn Pro Lys Ser Glu Ser Gly Lys Lys Gly Ile Asn Ser Phe Phe
   185 190 195 200
   aac tgg tac ttc ttc act ttc act ttc gcg cag atc atc tct ctc acg 1206
   Asn Trp Tyr Phe Phe Thr Phe Thr Phe Ala Gln Ile Ile Ser Leu Thr
   205 210 215
   cta gtt gtg tat atc cag tca aat gtg agc tgg acc atc ggt ttg agt 1254
   Leu Val Val Tyr Ile Gln Ser Asn Val Ser Trp Thr Ile Gly Leu Ser
   220 225 230
   atc cct gtg ggt ctt atg ttc ttg gcc tgc gtc atc ttc ttt gct ggt 1302
   Ile Pro Val Gly Leu Met Phe Leu Ala Cys Val Ile Phe Phe Ala Gly
   235 240 245
   cat aaa ctg tat gtg aaa gtg aaa gcc tct ggt agt cca ttg gct ggt 1350
   His Lys Leu Tyr Val Lys Val Lys Ala Ser Gly Ser Pro Leu Ala Gly
   250 255 260
   ata ggt cat gtt ata gcc gca gcg atc aag aaa cgt ggc ttg aag cca 1398
   Ile Gly His Val Ile Ala Ala Ala Ile Lys Lys Arg Gly Leu Lys Pro
   265 270 275 280
   gtt aag cag cct tgg atc gat ctt tac aac cac att cct cat aac tat 1446
   Val Lys Gln Pro Trp Ile Asp Leu Tyr Asn His Ile Pro His Asn Tyr
   285 290 295
   gca aac tct act ctt aag tac acc gac cag cta ag gtaaagtcgg 1491
   Ala Asn Ser Thr Leu Lys Tyr Thr Asp Gln Leu Arg
   300 305
   aatagttctg tctctgtctt tctttgttca tttgctttat gctttgtgtg aacaaatgtt 1551
   gtattgccag g ttt cta gac aaa gca gcg att atg acc gct gag gac aag 1601
   Phe Leu Asp Lys Ala Ala Ile Met Thr Ala Glu Asp Lys
   310 315 320
   ttg aac tcg gat gga acg gct tcg gat cca tgg aaa cta tgt acg atg 1649
   Leu Asn Ser Asp Gly Thr Ala Ser Asp Pro Trp Lys Leu Cys Thr Met
                   325                          330                          335
   cag aaa gtg gaa gaa gtg aaa tgc att tta aga gtg att cca atc tgg         1697
   Gln Lys Val Glu Glu Val Lys Cys Ile Leu Arg Val Ile Pro Ile Trp
               340                          345                          350
   ttc gca tgc gcc att tac tac ctt gca ata agt atg caa atg act tat         1745
   Phe Ala Cys Ala Ile Tyr Tyr Leu Ala Ile Ser Met Gln Met Thr Tyr
       355                          360                          365
   cca gtc ttc caa gcg ctt cag agc gac agg cga tta ggt tca ggt ggc         1793
   Pro Val Phe Gln Ala Leu Gln Ser Asp Arg Arg Leu Gly Ser Gly Gly
   370                          375                          380                          385
   ttc aag att cca gcc gcc acc tat gtc gtg ttc ttg atg tct ggt atg         1841
   Phe Lys Ile Pro Ala Ala Thr Tyr Val Val Phe Leu Met Ser Gly Met
                   390                          395                          400
   acg gtt ttc atc ata atc tac gac cgt gtc ctc gtc ccc tcg ctc aaa         1889
   Thr Val Phe Ile Ile Ile Tyr Asp Arg Val Leu Val Pro Ser Leu Lys
               405                          410                          415
   aga gtg acc ggt cta gac acg ggg ata aca ctc tta caa aga ata gga         1937
   Arg Val Thr Gly Leu Asp Thr Gly Ile Thr Leu Leu Gln Arg Ile Gly
           420                          425                          430
   gct ggc att ttc ttt gcg gtg ttg agt tta ttg gtc tcc ggg ttc ata         1985
   Ala Gly Ile Phe Phe Ala Val Leu Ser Leu Leu Val Ser Gly Phe Ile
       435                          440                          445
   gag gaa cgt aga aga acc ttt gcg ctg aca cga cct acg ctg ggg tcg         2033
   Glu Glu Arg Arg Arg Thr Phe Ala Leu Thr Arg Pro Thr Leu Gly Ser
   450                          455                          460                          465
   gag cct cga aca gga gag atc tcc tca atg tca ggg atg tgg ctg att         2081
   Glu Pro Arg Thr Gly Glu Ile Ser Ser Met Ser Gly Met Trp Leu Ile
                   470                          475                          480
   ccg cag ctg gtg ctt gca ggg gta gca gaa gct ttt gca gcc att ggg         2129
   Pro Gln Leu Val Leu Ala Gly Val Ala Glu Ala Phe Ala Ala Ile Gly
               485                          490                          495
   caa atg gag ttt tac tac aag cag ttt cct gaa aac atg aag agc ttt         2177
   Gln Met Glu Phe Tyr Tyr Lys Gln Phe Pro Glu Asn Met Lys Ser Phe
           500                          505                          510
   gcg ggt tct atc ttc tat gtc ggt gga gga gtt tct agc tac ctc gct         2225
   Ala Gly Ser Ile Phe Tyr Val Gly Gly Gly Val Ser Ser Tyr Leu Ala
       515                          520                          525
   agc ttc ttg atc tcg act gtt cat aga aca act gag cat tca cct acg         2273
   Ser Phe Leu Ile Ser Thr Val His Arg Thr Thr Glu His Ser Pro Thr
   530                          535                          540                          545
   ggg aat tgg tta gct gag gat ctg aac aaa gcg aag ctg gac tac ttc         2321
   Gly Asn Trp Leu Ala Glu Asp Leu Asn Lys Ala Lys Leu Asp Tyr Phe
                   550                          555                          560
   tat ttc atg ctc aca gga ctc atg ttt gtt aac ttt ctt tac ttc ttg         2369
   Tyr Phe Met Leu Thr Gly Leu Met Phe Val Asn Phe Leu Tyr Phe Leu
               565                          570                          575
   tta atg gct aga tgg tat aga tac aaa ggc act cat gat gaa gtc agt         2417
   Leu Met Ala Arg Trp Tyr Arg Tyr Lys Gly Thr His Asp Glu Val Ser
           580                          585                          590
   tat gtg gtt gac act gtt gac gaa gag aca aag caa caa gac aag aac         2465
   Tyr Val Val Asp Thr Val Asp Glu Glu Thr Lys Gln Gln Asp Lys Asn
       595                          600                          605
   tct gtc tga                                                             2474
   Ser Val
   610
<210> 14
   <211> 611
   <212> PRT
   <213> Brassica napus
<400> 14
   Met Glu Arg Lys Pro Phe Asp Val Glu Thr Thr Glu Asp His Lys Leu
   1               5                   10                  15
   Tyr Pro Ser Val Asp Gly Gly Gly Ser Asp Ser Val Asp Ser Phe Glu
               20                  25                  30
   Asp Glu Gln Lys Lys Leu Val Tyr Arg Gly Trp Lys Val Met Pro Phe
           35                  40                  45
   Ile Ile Gly Asn Glu Thr Phe Glu Lys Ile Gly Ile Ile Gly Thr Leu
       50                  55                  60
   Ser Asn Leu Leu Val Tyr Leu Thr Ser Val Phe Asn Leu Lys Ser Val
   65                  70                  75                  80
   Thr Ala Ala Thr Ile Ile Asn Ala Phe Ser Gly Thr Ile Asn Phe Gly
                   85                  90                  95
   Thr Phe Leu Ala Ala Phe Leu Cys Asp Thr Tyr Phe Gly Arg Tyr Lys
               100                 105                 110
   Thr Leu Ser Val Ala Val Ile Ala Cys Phe Leu Gly Ser Leu Val Ile
           115                 120                 125
   Leu Leu Thr Ala Ala Val Pro Gly Leu His Pro Ile Pro Cys Gly Thr
       130                 135                 140
   Gln Ser Ser Cys Gln Gly Pro Ser Glu Gly Gln Ile Ala Phe Leu Leu
   145                 150                 155                 160
   Met Gly Leu Ala Leu Leu Val Val Gly Ala Gly Gly Ile Arg Pro Cys
                   165                 170                 175
   Asn Leu Ala Phe Gly Ala Asp Gln Phe Asn Pro Lys Ser Glu Ser Gly
               180                 185                 190
   Lys Lys Gly Ile Asn Ser Phe Phe Asn Trp Tyr Phe Phe Thr Phe Thr
           195                 200                 205
   Phe Ala Gln Ile Ile Ser Leu Thr Leu Val Val Tyr Ile Gln Ser Asn
       210                 215                 220
   Val Ser Trp Thr Ile Gly Leu Ser Ile Pro Val Gly Leu Met Phe Leu
   225                 230                 235                 240
   Ala Cys Val Ile Phe Phe Ala Gly His Lys Leu Tyr Val Lys Val Lys
                   245                 250                 255
   Ala Ser Gly Ser Pro Leu Ala Gly Ile Gly His Val Ile Ala Ala Ala
               260                 265                 270
   Ile Lys Lys Arg Gly Leu Lys Pro Val Lys Gln Pro Trp Ile Asp Leu
           275                 280                 285
   Tyr Asn His Ile Pro His Asn Tyr Ala Asn Ser Thr Leu Lys Tyr Thr
   290 295 300
   Asp Gln Leu Arg Phe Leu Asp Lys Ala Ala Ile Met Thr Ala Glu Asp
   305 310 315 320
   Lys Leu Asn Ser Asp Gly Thr Ala Ser Asp Pro Trp Lys Leu Cys Thr
   325 330 335
   Met Gln Lys Val Glu Glu Val Lys Cys Ile Leu Arg Val Ile Pro Ile
   340 345 350
   Trp Phe Ala Cys Ala Ile Tyr Tyr Leu Ala Ile Ser Met Gln Met Thr
   355 360 365
   Tyr Pro Val Phe Gln Ala Leu Gln Ser Asp Arg Arg Leu Gly Ser Gly
   370 375 380
   Gly Phe Lys Ile Pro Ala Ala Thr Tyr Val Val Phe Leu Met Ser Gly
   385 390 395 400
   Met Thr Val Phe Ile Ile Ile Tyr Asp Arg Val Leu Val Pro Ser Leu
   405 410 415
   Lys Arg Val Thr Gly Leu Asp Thr Gly Ile Thr Leu Leu Gln Arg Ile
   420 425 430
   Gly Ala Gly Ile Phe Phe Ala Val Leu Ser Leu Leu Val Ser Gly Phe
   435 440 445
   Ile Glu Glu Arg Arg Arg Thr Phe Ala Leu Thr Arg Pro Thr Leu Gly
   450 455 460
   Ser Glu Pro Arg Thr Gly Glu Ile Ser Ser Met Ser Gly Met Trp Leu
   465 470 475 480
   Ile Pro Gln Leu Val Leu Ala Gly Val Ala Glu Ala Phe Ala Ala Ile
   485 490 495
   Gly Gln Met Glu Phe Tyr Tyr Lys Gln Phe Pro Glu Asn Met Lys Ser
   500 505 510
   Phe Ala Gly Ser Ile Phe Tyr Val Gly Gly Gly Val Ser Ser Tyr Leu
   515 520 525
   Ala Ser Phe Leu Ile Ser Thr Val His Arg Thr Thr Glu His Ser Pro
   530 535 540
   Thr Gly Asn Trp Leu Ala Glu Asp Leu Asn Lys Ala Lys Leu Asp Tyr
   545 550 555 560
   Phe Tyr Phe Met Leu Thr Gly Leu Met Phe Val Asn Phe Leu Tyr Phe
   565 570 575
   Leu Leu Met Ala Arg Trp Tyr Arg Tyr Lys Gly Thr His Asp Glu Val
   580 585 590
   Ser Tyr Val Val Asp Thr Val Asp Glu Glu Thr Lys Gln Gln Asp Lys
   595 600 605
   Asn Ser Val
   610
<210> 15
   <211> 2668
   <212> DNA
   <213> Brassica napus
<220>
   <221> CDS
   <222> (1)..(160)
<220>
   <221> CDS
   <222> (665)..(882)
<220>
   <221> CDS
   <222> (975)..(1528)
<220>
   <221> CDS
   <222> (1753)..(2665)
<400> 15
   atg gaa aga aag cct ttt gag gtt gag aca aca aag acg gat gag gat      48
   Met Glu Arg Lys Pro Phe Glu Val Glu Thr Thr Lys Thr Asp Glu Asp
   1               5                   10                  15
   cag aaa ccc tac tcc gct gtc gat ggc ggt ggt tct gat tcg gtc gat      96
   Gln Lys Pro Tyr Ser Ala Val Asp Gly Gly Gly Ser Asp Ser Val Asp
               20                  25                  30
   tca ttt gat gaa gat cag aaa aag ctc gtg tat aga gga tgg aaa gtc     144
   Ser Phe Asp Glu Asp Gln Lys Lys Leu Val Tyr Arg Gly Trp Lys Val
           35                  40                  45
   atg cct ttt atc att g gtaacgctta ttttcttctc tattcagaca ctcctttttt      200
   Met Pro Phe Ile Ile
       50
   attttattgt gtgtctgctt ctctcaacac ggtccagaaa ctaaataaag atgtggaaaa   260
   taaactagaa catgttgtcc gtgaaattta aaaagtactt ttcttttctc ttcttctctt   320
   tgactgaaaa cgattgaaac caggatcaac caaatctacc agatctagag aaaggtgtta   380
   ttacaacttt ttttcattaa taatgctttt taatttttag ttttatacgc ttaaagacaa   440
   agttatttag tttctgggag ttacagcagt taaaagttaa aacaaaagga ataaagtttc   500
   aagttaaaga agcactgaaa gtacaacttc gattcgtgtg tgtttcggtt taaaagttgt   560
   gaattaaaaa gtttgttact gtatgggaaa cccgtttttc cctttgacta ctgattcatt   620
   tattgtgaaa aagaaattaa taatgattct tgttcttttt acag gt  aat gaa aca   675
                                                   Gly Asn Glu Thr
                                                       55
   ttt gag aag att ggg atc att ggg aca tta tca aac ctt ctt gtg tac     723
   Phe Glu Lys Ile Gly Ile Ile Gly Thr Leu Ser Asn Leu Leu Val Tyr
           60                  65                  70
   cta act caa gta ttc aac ctc aag aaa tac aca gct gca acc atc atc     771
   Leu Thr Gln Val Phe Asn Leu Lys Lys Tyr Thr Ala Ala Thr Ile Ile
       75                  80                  85
   aat gcc ttt agt ggc acc atc aac ttc ggc act ttc ctc gct gct ttc     819
   Asn Ala Phe Ser Gly Thr Ile Asn Phe Gly Thr Phe Leu Ala Ala Phe
   90                  95                  100                 105
   ctc tgc gac act tac ttt ggt cgc tac aag act ctc agt gtt gct gtc      867
   Leu Cys Asp Thr Tyr Phe Gly Arg Tyr Lys Thr Leu Ser Val Ala Val
                   110                 115                 120
   atc gct tgt ttt ctg gtacctacta atgctgttct taacggtttt gtttttcgtc     922
   Ile Ala Cys Phe Leu
               125
   atgactctta ttcttttttt ctttttgcta aaccgtgttt tatcttttgt ag gga tcg    980
                                                             Gly Ser
   ctt gcg ata cta ctc acg gca gca gtt ccg gga ctg cac ccg act cct     1028
   Leu Ala Ile Leu Leu Thr Ala Ala Val Pro Gly Leu His Pro Thr Pro
       130                 135                 140
   tgt gga aca aag agt tca tgc gaa ggg cca ggt gcg ggg cag att ttg     1076
   Cys Gly Thr Lys Ser Ser Cys Glu Gly Pro Gly Ala Gly Gln Ile Leu
   145                 150                 155                 160
   ttt ctg atg ttg ggt tta gcg ctt ctt gtg gtt ggt gct ggt ggt atc     1124
   Phe Leu Met Leu Gly Leu Ala Leu Leu Val Val Gly Ala Gly Gly Ile
                   165                 170                 175
   agg ccg tgt aac ttg gcg ttt ggt gcc gat cag ttc aac ccc aag tcc     1172
   Arg Pro Cys Asn Leu Ala Phe Gly Ala Asp Gln Phe Asn Pro Lys Ser
               180                 185                 190
   gaa tca gga aag aaa gga atc aac agt ttc ttc aac tgg tac ttc ttc     1220
   Glu Ser Gly Lys Lys Gly Ile Asn Ser Phe Phe Asn Trp Tyr Phe Phe
           195                 200                 205
   act ttc acg ttc gcg cag atc atc tcg ctc acg cta gtt gtg tat atc     1268
   Thr Phe Thr Phe Ala Gln Ile Ile Ser Leu Thr Leu Val Val Tyr Ile
       210                 215                 220
   cag tcg aat gtg agc tgg acc atc ggt ttg agt atc ccc gtg ggt ctc     1316
   Gln Ser Asn Val Ser Trp Thr Ile Gly Leu Ser Ile Pro Val Gly Leu
   225                 230                 235                 240
   atg ttc ttg gct tgc gtc att ttc ttc gct ggt cat aaa ctt tat gta     1364
   Met Phe Leu Ala Cys Val Ile Phe Phe Ala Gly His Lys Leu Tyr Val
                   245                 250                 255
   aaa gtg aaa gcc tcg ggt agt ccc ttg gct ggt ata gct cgt gtt ata     1412
   Lys Val Lys Ala Ser Gly Ser Pro Leu Ala Gly Ile Ala Arg Val Ile
               260                 265                 270
   gca gcc gcg atc tcg aaa cga gga ttg aag ccg gtt aag cag cct tgg     1460
   Ala Ala Ala Ile Ser Lys Arg Gly Leu Lys Pro Val Lys Gln Pro Trp
           275                 280                 285
   gtc gat ctt tac aat cac att cct cgt aac tat gca aac tta acc cta     1508
   Val Asp Leu Tyr Asn His Ile Pro Arg Asn Tyr Ala Asn Leu Thr Leu
       290                 295                 300
   aaa tac acc gac cag ttc ag  gtatactcaa tagttctgtc tctattgctt        1558
   Lys Tyr Thr Asp Gln Phe Arg
   305                 310
   tatatcttgg cacagccgga tggatgcctt gacttctaaa tatttttaaa atgggtcgta    1618
   tagacccacc ccaggttgtc aaattttgtt taccgaaata tatatttttt atttaccaaa    1678
   atatttttaa aatgggcccg gttggaaaca aaatgctgaa aactaaatgt tggttttgtt    1738
   tgttatattg ttag g ttt cta gac aaa gca gcg ata atg aca cct gag gac    1789
                     Phe Leu Asp Lys Ala Ala Ile Met Thr Pro Glu Asp
                                 315                 320
   aag ttg aac tct gat gga aca gct tcg gat cca tgg aaa cta tgt acg     1837
   Lys Leu Asn Ser Asp Gly Thr Ala Ser Asp Pro Trp Lys Leu Cys Thr
       325                 330                 335
   atg cag caa gtg gaa gaa gtg aaa tgc att ata aga gtg att ccg atc     1885
   Met Gln Gln Val Glu Glu Val Lys Cys Ile Ile Arg Val Ile Pro Ile
   340                 345                 350                 355
   tgg ttt gct tgc gct atc tac tac ctt gca ata agc att caa atg act     1933
   Trp Phe Ala Cys Ala Ile Tyr Tyr Leu Ala Ile Ser Ile Gln Met Thr
                   360                 365                 370
   tat ccc gtc ttc caa gcg caa cag agc gac agg aga tta ggt acc ggt     1981
   Tyr Pro Val Phe Gln Ala Gln Gln Ser Asp Arg Arg Leu Gly Thr Gly
               375                 380                 385
   ggc ttc aag atc ccc gca gcc acc tac gta gtt ttc ttg atg acc ggt     2029
   Gly Phe Lys Ile Pro Ala Ala Thr Tyr Val Val Phe Leu Met Thr Gly
           390                 395                 400
   atg acg gtt ttc atc ata atc tac gac cgt gtc ctt gtc ccg tcg ctc     2077
   Met Thr Val Phe Ile Ile Ile Tyr Asp Arg Val Leu Val Pro Ser Leu
       405                 410                 415
   aaa aga gtg acc ggt cta gac act ggt ata aca ctc ttg caa agg atc     2125
   Lys Arg Val Thr Gly Leu Asp Thr Gly Ile Thr Leu Leu Gln Arg Ile
   420                 425                 430                 435
   gga gcg ggg atc ttc ttt gcg gtg ttg agt tta ttg gtc tcc ggg ttc     2173
   Gly Ala Gly Ile Phe Phe Ala Val Leu Ser Leu Leu Val Ser Gly Phe
                   440                 445                 450
   ata gag gaa cgt aga aga agc att gcg ctg aca aaa cag act cta ggg     2221
   Ile Glu Glu Arg Arg Arg Ser Ile Ala Leu Thr Lys Gln Thr Leu Gly
               455                 460                 465
   atg gag cct cga aca gga gag atc tca tca atg tcg gcg atg tgg ctg     2269
   Met Glu Pro Arg Thr Gly Glu Ile Ser Ser Met Ser Ala Met Trp Leu
           470                 475                 480
   att ccg cag ctg gtt ctt gca ggg ata gct gaa gcg ttt aca gcc att     2317
   Ile Pro Gln Leu Val Leu Ala Gly Ile Ala Glu Ala Phe Thr Ala Ile
       485                 490                 495
   gga cag atg gag ttt tac tac aag cag ttt cct gag aac atg agg agc     2365
   Gly Gln Met Glu Phe Tyr Tyr Lys Gln Phe Pro Glu Asn Met Arg Ser
   500                 505                 510                 515
   ttt gct ggt tct atc ttc tat gtc ggt gcg ggg gtt tct agc tac ctc     2413
   Phe Ala Gly Ser Ile Phe Tyr Val Gly Ala Gly Val Ser Ser Tyr Leu
                   520                 525                 530
   gct agc ttc ttg atc tcc act gtt cat cgg aca act gaa cat tca cct     2461
   Ala Ser Phe Leu Ile Ser Thr Val His Arg Thr Thr Glu His Ser Pro
               535                 540                 545
   tcg ggg aac tgg tta gct gag gat ctg aac aag gcc aag ttg gat tac     2509
   Ser Gly Asn Trp Leu Ala Glu Asp Leu Asn Lys Ala Lys Leu Asp Tyr
           550                 555                 560
   ttc tat ttc atg ctc acg ggg ctc atg ttc gtt aac atg gtt tac ttc     2557
   Phe Tyr Phe Met Leu Thr Gly Leu Met Phe Val Asn Met Val Tyr Phe
       565                 570                 575
   ttg ata atg gct aag ttt tat aga tac aaa ggc agt gat gat aaa gtc     2605
   Leu Ile Met Ala Lys Phe Tyr Arg Tyr Lys Gly Ser Asp Asp Lys Val
   580                 585                 590                 595
   atc tct gct att gag acc agt gaa gac gag acc aag cag aag caa gcg     2653
   Ile Ser Ala Ile Glu Thr Ser Glu Asp Glu Thr Lys Gln Lys Gln Ala
                   600                 605                 610
   aag aac tcg gtt taa                                                 2668
   Lys Asn Ser Val
               615
<210> 16
   <211> 615
   <212> PRT
   <213> Brassica napus
<400> 16
   Met Glu Arg Lys Pro Phe Glu Val Glu Thr Thr Lys Thr Asp Glu Asp
   1               5                   10                  15
   Gln Lys Pro Tyr Ser Ala Val Asp Gly Gly Gly Ser Asp Ser Val Asp
               20                  25                  30
   Ser Phe Asp Glu Asp Gln Lys Lys Leu Val Tyr Arg Gly Trp Lys Val
           35                  40                  45
   Met Pro Phe Ile Ile Gly Asn Glu Thr Phe Glu Lys Ile Gly Ile Ile
       50                  55                  60
   Gly Thr Leu Ser Asn Leu Leu Val Tyr Leu Thr Gln Val Phe Asn Leu
   65                  70                  75                  80
   Lys Lys Tyr Thr Ala Ala Thr Ile Ile Asn Ala Phe Ser Gly Thr Ile
                   85                  90                  95
   Asn Phe Gly Thr Phe Leu Ala Ala Phe Leu Cys Asp Thr Tyr Phe Gly
               100                 105                 110
   Arg Tyr Lys Thr Leu Ser Val Ala Val Ile Ala Cys Phe Leu Gly Ser
           115                 120                 125
   Leu Ala Ile Leu Leu Thr Ala Ala Val Pro Gly Leu His Pro Thr Pro
       130                 135                 140
   Cys Gly Thr Lys Ser Ser Cys Glu Gly Pro Gly Ala Gly Gln Ile Leu
   145                 150                 155                 160
   Phe Leu Met Leu Gly Leu Ala Leu Leu Val Val Gly Ala Gly Gly Ile
                   165                 170                 175
   Arg Pro Cys Asn Leu Ala Phe Gly Ala Asp Gln Phe Asn Pro Lys Ser
               180                 185                 190
   Glu Ser Gly Lys Lys Gly Ile Asn Ser Phe Phe Asn Trp Tyr Phe Phe
           195                 200                 205
   Thr Phe Thr Phe Ala Gln Ile Ile Ser Leu Thr Leu Val Val Tyr Ile
       210                 215                 220
   Gln Ser Asn Val Ser Trp Thr Ile Gly Leu Ser Ile Pro Val Gly Leu
   225                 230                 235                 240
   Met Phe Leu Ala Cys Val Ile Phe Phe Ala Gly His Lys Leu Tyr Val
                   245                 250                 255
   Lys Val Lys Ala Ser Gly Ser Pro Leu Ala Gly Ile Ala Arg Val Ile
               260                 265                 270
   Ala Ala Ala Ile Ser Lys Arg Gly Leu Lys Pro Val Lys Gln Pro Trp
           275                 280                 285
   Val Asp Leu Tyr Asn His Ile Pro Arg Asn Tyr Ala Asn Leu Thr Leu
   290 295 300
   Lys Tyr Thr Asp Gln Phe Arg Phe Leu Asp Lys Ala Ala Ile Met Thr
   305 310 315 320
   Pro Glu Asp Lys Leu Asn Ser Asp Gly Thr Ala Ser Asp Pro Trp Lys
   325 330 335
   Leu Cys Thr Met Gln Gln Val Glu Glu Val Lys Cys Ile Ile Arg Val
   340 345 350
   Ile Pro Ile Trp Phe Ala Cys Ala Ile Tyr Tyr Leu Ala Ile Ser Ile
   355 360 365
   Gln Met Thr Tyr Pro Val Phe Gln Ala Gln Gln Ser Asp Arg Arg Leu
   370 375 380
   Gly Thr Gly Gly Phe Lys Ile Pro Ala Ala Thr Tyr Val Val Phe Leu
   385 390 395 400
   Met Thr Gly Met Thr Val Phe Ile Ile Ile Tyr Asp Arg Val Leu Val
   405 410 415
   Pro Ser Leu Lys Arg Val Thr Gly Leu Asp Thr Gly Ile Thr Leu Leu
   420 425 430
   Gln Arg Ile Gly Ala Gly Ile Phe Phe Ala Val Leu Ser Leu Leu Val
   435 440 445
   Ser Gly Phe Ile Glu Glu Arg Arg Arg Ser Ile Ala Leu Thr Lys Gln
   450 455 460
   Thr Leu Gly Met Glu Pro Arg Thr Gly Glu Ile Ser Ser Met Ser Ala
   465 470 475 480
   Met Trp Leu Ile Pro Gln Leu Val Leu Ala Gly Ile Ala Glu Ala Phe
   485 490 495
   Thr Ala Ile Gly Gln Met Glu Phe Tyr Tyr Lys Gln Phe Pro Glu Asn
   500 505 510
   Met Arg Ser Phe Ala Gly Ser Ile Phe Tyr Val Gly Ala Gly Val Ser
   515 520 525
   Ser Tyr Leu Ala Ser Phe Leu Ile Ser Thr Val His Arg Thr Thr Glu
   530 535 540
   His Ser Pro Ser Gly Asn Trp Leu Ala Glu Asp Leu Asn Lys Ala Lys
   545 550 555 560
   Leu Asp Tyr Phe Tyr Phe Met Leu Thr Gly Leu Met Phe Val Asn Met
   565 570 575
   Val Tyr Phe Leu Ile Met Ala Lys Phe Tyr Arg Tyr Lys Gly Ser Asp
   580 585 590
   Asp Lys Val Ile Ser Ala Ile Glu Thr Ser Glu Asp Glu Thr Lys Gln
   595 600 605
   Lys Gln Ala Lys Asn Ser Val
   610 615
<210> 17
   <211> 2354
   <212> DNA
   <213> Brassica napus
<220>
   <221> CDS
   <222> (1)..(154)
<220>
   <221> CDS
   <222> (514)..(731)
<220>
   <221> CDS
   <222> (811)..(1364)
<220>
   <221> CDS
   <222> (1433)..(2351)
<400> 17
   atg gag aga aag ccc ttt gag gtt gag acg acg gag gat cac aaa ccc         48
   Met Glu Arg Lys Pro Phe Glu Val Glu Thr Thr Glu Asp His Lys Pro
   1               5                   10                  15
   tac tcc gat ggc ggt ggt tct gat tta acg tcg acg gtt gat tca tct         96
   Tyr Ser Asp Gly Gly Gly Ser Asp Leu Thr Ser Thr Val Asp Ser Ser
               20                  25                  30
   ggc gac gag cag aaa aag ctc gtt tat aga ggc tgg aaa gtc atg cct        144
   Gly Asp Glu Gln Lys Lys Leu Val Tyr Arg Gly Trp Lys Val Met Pro
           35                  40                  45
   tat atc att g gtaacgcttt tctctctctc tctagaccgt tatttttaaa              194
   Tyr Ile Ile
       50
   ctctgttttc tttttgtcaa ctaactctgt tttttcgtgt aacagaacac ttttccccgt      254
   gatggttttt ttgtcattta ataatttgtt tatttttatc ttgtcacgtt ggcttgactg      314
   tgtcagtatt ctagataatc taccaggtgt agtgtataga aagaaatgtt acgttgtgtt      374
   ctttagtaac gtttttaaat gtccgtgaaa aggactttga atcactggaa gtccaatctt      434
   ggttccaaac tgtgttactt tatgagaaac ccatgttggt ttgactattc attaacgaaa      494
   agaatttgta tcttgatag gt  aat gag aca ttt gag aag att ggg atc ata       545
                        Gly Asn Glu Thr Phe Glu Lys Ile Gly Ile Ile
                                    55                  60
   ggg aca tta tca aac ctt cta gtg tac cta act caa gta ttc aac ctg        593
   Gly Thr Leu Ser Asn Leu Leu Val Tyr Leu Thr Gln Val Phe Asn Leu
           65                  70                  75
   aag aaa tac aca gct gca act att atc agt gcc ttt agt ggc act atc        641
   Lys Lys Tyr Thr Ala Ala Thr Ile Ile Ser Ala Phe Ser Gly Thr Ile
       80                  85                  90
   aac ttc ggc act ttc ttc gct gct ttt ctg tgt gac act tac ttt ggt        689
   Asn Phe Gly Thr Phe Phe Ala Ala Phe Leu Cys Asp Thr Tyr Phe Gly
   95                  100                 105                 110
   cgc tac aag act ctc agt gtg gct gtc atc gct tgt ttt ctg                731
   Arg Tyr Lys Thr Leu Ser Val Ala Val Ile Ala Cys Phe Leu
                        115                 120
   gtactaatgc tgctgatctt aatgggtttt ttttcttacg ccaagaactc ttaatgctga      791
   tcatttttgg tctgtgtag gga tcg ctt ttt ata cta atg acg gct gca gtt       843
                        Gly Ser Leu Phe Ile Leu Met Thr Ala Ala Val
                        125                 130                 135
   ccg gga ttg cac ccg act cct tgt gga act aaa agt tcg tgc caa ggg        891
   Pro Gly Leu His Pro Thr Pro Cys Gly Thr Lys Ser Ser Cys Gln Gly
                   140                 145                 150
   cca aat ggg ggg cag gtt ttg ttt ctg ctg ttg ggt tta gcg ctt ctc        939
   Pro Asn Gly Gly Gln Val Leu Phe Leu Leu Leu Gly Leu Ala Leu Leu
               155                 160                 165
   gta gtc ggt gct ggt ggt atc agg ccg tgt aat ttg gcc ttt gga gct        987
   Val Val Gly Ala Gly Gly Ile Arg Pro Cys Asn Leu Ala Phe Gly Ala
           170                 175                 180
   gat cag ttc aac ccc aag tca gaa tct gga agg aaa gga atc aac agt       1035
   Asp Gln Phe Asn Pro Lys Ser Glu Ser Gly Arg Lys Gly Ile Asn Ser
       185                 190                 195
   ttc ttc aac tgg tac ttc ttc act ttc acg ttt gcg cag atc atc tcg       1083
   Phe Phe Asn Trp Tyr Phe Phe Thr Phe Thr Phe Ala Gln Ile Ile Ser
   200                 205                 210                 215
   ctc acg cta gtt gtg tat atc cag tca aac gtg agc tgg acc atc ggt       1131
   Leu Thr Leu Val Val Tyr Ile Gln Ser Asn Val Ser Trp Thr Ile Gly
                   220                 225                 230
   ttg agt atc cct gtg ggt cta atg ttc ttg gcc tgc gtc att tac ttt       1179
   Leu Ser Ile Pro Val Gly Leu Met Phe Leu Ala Cys Val Ile Tyr Phe
               235                 240                 245
   gct ggc cat aat ctc tac gta aaa gtg aaa gcc tcg ggt agt ccc ttg       1227
   Ala Gly His Asn Leu Tyr Val Lys Val Lys Ala Ser Gly Ser Pro Leu
           250                 255                 260
   gct ggc atc gct cgt gtt ata gcc gca gcg atc aag aaa cga ggg ttg       1275
   Ala Gly Ile Ala Arg Val Ile Ala Ala Ala Ile Lys Lys Arg Gly Leu
       265                 270                 275
   aag cct gtt aag aag cct tgc tcc gat ctt tac aat cac atc ccg cct       1323
   Lys Pro Val Lys Lys Pro Cys Ser Asp Leu Tyr Asn His Ile Pro Pro
   280                 285                 290                 295
   aac tat gca aac tct aca ctc aaa tac tcg gac cag ttt ag                1364
   Asn Tyr Ala Asn Ser Thr Leu Lys Tyr Ser Asp Gln Phe Arg
                   300                 305
   gtaaaactct aagagtttct ctctctgttg ctttatgttt ctgtctttta acatgtttat     1424
   gtttctag a ttt ctc gac aag gca gcg gtg atg acc cct gag gac aag        1472
              Phe Leu Asp Lys Ala Ala Val Met Thr Pro Glu Asp Lys
              310                 315                 320
   ttg aaa ccc gat gga acg gct tca gat cca tgg aat cta tgt aca atg       1520
   Leu Lys Pro Asp Gly Thr Ala Ser Asp Pro Trp Asn Leu Cys Thr Met
           325                 330                 335
   cag cag gtg gaa gaa gtg aaa tgc att gta aga gtg att cca atc tgg       1568
   Gln Gln Val Glu Glu Val Lys Cys Ile Val Arg Val Ile Pro Ile Trp
       340                 345                 350
   tta gct tgc gca gtt tac tac ctg gca ata aca ttg caa atg act tat       1616
   Leu Ala Cys Ala Val Tyr Tyr Leu Ala Ile Thr Leu Gln Met Thr Tyr
   355                 360                 365                 370
   ccg gtc ttc caa gcg ctc cag agc gac cgg aga ttg ggt tca ggt ggc       1664
   Pro Val Phe Gln Ala Leu Gln Ser Asp Arg Arg Leu Gly Ser Gly Gly
                   375                 380                 385
   ttc aag att ccc ggc gcc acc tat gta gtg ttc ttg atg tcc ggt atg       1712
   Phe Lys Ile Pro Gly Ala Thr Tyr Val Val Phe Leu Met Ser Gly Met
                   390                          395                          400
   acg gtt ttc atc ata ttc tac gac cgt gtc ctt gtc ccg tcg ctc aga      1760
   Thr Val Phe Ile Ile Phe Tyr Asp Arg Val Leu Val Pro Ser Leu Arg
               405                     410                     415
   aga gtg aca ggg cta gac aat ggt ata act ctc tta caa aga atc gga      1808
   Arg Val Thr Gly Leu Asp Asn Gly Ile Thr Leu Leu Gln Arg Ile Gly
           420                     425                     430
   tca ggc att ttc ttc gcg ctt ttg agt ttg ttg gtt tct ggg ttc gtt      1856
   Ser Gly Ile Phe Phe Ala Leu Leu Ser Leu Leu Val Ser Gly Phe Val
   435                     440                     445                     450
   gag gaa cgg cga aga gcc att gca ctg aca aaa cct act ctc ggg att      1904
   Glu Glu Arg Arg Arg Ala Ile Ala Leu Thr Lys Pro Thr Leu Gly Ile
                   455                     460                     465
   gag cct cga gct gga gaa atc tca tca atg tca gcg atg tgg ctg ata      1952
   Glu Pro Arg Ala Gly Glu Ile Ser Ser Met Ser Ala Met Trp Leu Ile
               470                     475                     480
   ccg cag ctc gtg ctt gca ggg ata gct gaa gct ttt gca gcc gtt gga      2000
   Pro Gln Leu Val Leu Ala Gly Ile Ala Glu Ala Phe Ala Ala Val Gly
           485                     490                     495
   caa atg gag ttt tac tac aag cag ttt cct gaa aac atg aag agc ttt      2048
   Gln Met Glu Phe Tyr Tyr Lys Gln Phe Pro Glu Asn Met Lys Ser Phe
       500                     505                     510
   gcg ggt tct atc ttc tat gtt ggt gca gga gtt tct agc tac ctc gct      2096
   Ala Gly Ser Ile Phe Tyr Val Gly Ala Gly Val Ser Ser Tyr Leu Ala
   515                     520                     525                     530
   agc ttc ttg atc tcg act gtt cat aaa aca act gag cat tca cgc tcc      2144
   Ser Phe Leu Ile Ser Thr Val His Lys Thr Thr Glu His Ser Arg Ser
                   535                     540                     545
   ggg aat tgg cta gct gag gat ttg aac aaa ggg aag ctg gat tac ttt      2192
   Gly Asn Trp Leu Ala Glu Asp Leu Asn Lys Gly Lys Leu Asp Tyr Phe
               550                     555                     560
   tat ttc atg ctc act ggc ctc atg ttt gtt aac atg gtt tac ttc ttg      2240
   Tyr Phe Met Leu Thr Gly Leu Met Phe Val Asn Met Val Tyr Phe Leu
           565                     570                     575
   tta atg gct aaa ttt tat aga tac aaa gtc act aac gat gaa gca aat      2288
   Leu Met Ala Lys Phe Tyr Arg Tyr Lys Val Thr Asn Asp Glu Ala Asn
       580                     585                     590
   tct gtg att aag acc aat gaa gaa gag acc aag gag aat cag caa cac      2336
   Ser Val Ile Lys Thr Asn Glu Glu Glu Thr Lys Glu Asn Gln Gln His
   595                     600                     605                     610
   gac aag agc tat gtc tga                                              2354
   Asp Lys Ser Tyr Val
                   615
<210> 18
   <211> 615
   <212> PRT
   <213> Brassica napus
<400> 18
   Met Glu Arg Lys Pro Phe Glu Val Glu Thr Thr Glu Asp His Lys Pro
   1               5                   10                  15
   Tyr Ser Asp Gly Gly Gly Ser Asp Leu Thr Ser Thr Val Asp Ser Ser
               20                  25                  30
   Gly Asp Glu Gln Lys Lys Leu Val Tyr Arg Gly Trp Lys Val Met Pro
           35                  40                  45
   Tyr Ile Ile Gly Asn Glu Thr Phe Glu Lys Ile Gly Ile Ile Gly Thr
   50 55 60
   Leu Ser Asn Leu Leu Val Tyr Leu Thr Gln Val Phe Asn Leu Lys Lys
   65 70 75 80
   Tyr Thr Ala Ala Thr Ile Ile Ser Ala Phe Ser Gly Thr Ile Asn Phe
   85 90 95
   Gly Thr Phe Phe Ala Ala Phe Leu Cys Asp Thr Tyr Phe Gly Arg Tyr
   100 105 110
   Lys Thr Leu Ser Val Ala Val Ile Ala Cys Phe Leu Gly Ser Leu Phe
   115 120 125
   Ile Leu Met Thr Ala Ala Val Pro Gly Leu His Pro Thr Pro Cys Gly
   130 135 140
   Thr Lys Ser Ser Cys Gln Gly Pro Asn Gly Gly Gln Val Leu Phe Leu
   145 150 155 160
   Leu Leu Gly Leu Ala Leu Leu Val Val Gly Ala Gly Gly Ile Arg Pro
   165 170 175
   Cys Asn Leu Ala Phe Gly Ala Asp Gln Phe Asn Pro Lys Ser Glu Ser
   180 185 190
   Gly Arg Lys Gly Ile Asn Ser Phe Phe Asn Trp Tyr Phe Phe Thr Phe
   195 200 205
   Thr Phe Ala Gln Ile Ile Ser Leu Thr Leu Val Val Tyr Ile Gln Ser
   210 215 220
   Asn Val Ser Trp Thr Ile Gly Leu Ser Ile Pro Val Gly Leu Met Phe
   225 230 235 240
   Leu Ala Cys Val Ile Tyr Phe Ala Gly His Asn Leu Tyr Val Lys Val
   245 250 255
   Lys Ala Ser Gly Ser Pro Leu Ala Gly Ile Ala Arg Val Ile Ala Ala
   260 265 270
   Ala Ile Lys Lys Arg Gly Leu Lys Pro Val Lys Lys Pro Cys Ser Asp
   275 280 285
   Leu Tyr Asn His Ile Pro Pro Asn Tyr Ala Asn Ser Thr Leu Lys Tyr
   290 295 300
   Ser Asp Gln Phe Arg Phe Leu Asp Lys Ala Ala Val Met Thr Pro Glu
   305 310 315 320
   Asp Lys Leu Lys Pro Asp Gly Thr Ala Ser Asp Pro Trp Asn Leu Cys
   325 330 335
   Thr Met Gln Gln Val Glu Glu Val Lys Cys Ile Val Arg Val Ile Pro
   340 345 350
   Ile Trp Leu Ala Cys Ala Val Tyr Tyr Leu Ala Ile Thr Leu Gln Met
           355                 360                 365
   Thr Tyr Pro Val Phe Gln Ala Leu Gln Ser Asp Arg Arg Leu Gly Ser
       370                 375                 380
   Gly Gly Phe Lys Ile Pro Gly Ala Thr Tyr Val Val Phe Leu Met Ser
   385                 390                 395                 400
   Gly Met Thr Val Phe Ile Ile Phe Tyr Asp Arg Val Leu Val Pro Ser
                   405                 410                 415
   Leu Arg Arg Val Thr Gly Leu Asp Asn Gly Ile Thr Leu Leu Gln Arg
               420                 425                 430
   Ile Gly Ser Gly Ile Phe Phe Ala Leu Leu Ser Leu Leu Val Ser Gly
           435                 440                 445
   Phe Val Glu Glu Arg Arg Arg Ala Ile Ala Leu Thr Lys Pro Thr Leu
       450                 455                 460
   Gly Ile Glu Pro Arg Ala Gly Glu Ile Ser Ser Met Ser Ala Met Trp
   465                 470                 475                 480
   Leu Ile Pro Gln Leu Val Leu Ala Gly Ile Ala Glu Ala Phe Ala Ala
                   485                 490                 495
   Val Gly Gln Met Glu Phe Tyr Tyr Lys Gln Phe Pro Glu Asn Met Lys
               500                 505                 510
   Ser Phe Ala Gly Ser Ile Phe Tyr Val Gly Ala Gly Val Ser Ser Tyr
           515                 520                 525
   Leu Ala Ser Phe Leu Ile Ser Thr Val His Lys Thr Thr Glu His Ser
       530                 535                 540
   Arg Ser Gly Asn Trp Leu Ala Glu Asp Leu Asn Lys Gly Lys Leu Asp
   545                 550                 555                 560
   Tyr Phe Tyr Phe Met Leu Thr Gly Leu Met Phe Val Asn Met Val Tyr
                   565                 570                 575
   Phe Leu Leu Met Ala Lys Phe Tyr Arg Tyr Lys Val Thr Asn Asp Glu
               580                 585                 590
   Ala Asn Ser Val Ile Lys Thr Asn Glu Glu Glu Thr Lys Glu Asn Gln
           595                 600                 605
   Gln His Asp Lys Ser Tyr Val
       610                 615
<210> 19
   <211> 2475
   <212> DNA
   <213> Brassica napus
<220>
   <221> CDS
   <222> (1)..(151)
<220>
   <221> CDS
   <222> (620)..(837)
<220>
   <221> CDS
   <222> (929)..(1482)
<220>
   <221> CDS
   <222> (1563)..(2472)
<400> 19
   atg gag aga aag ccc ttt gat gtt gag aca act gag gat cac aaa gcc       48
   Met Glu Arg Lys Pro Phe Asp Val Glu Thr Thr Glu Asp His Lys Ala
   1               5                   10                  15
   tac tcc tcc gtc gat ggc ggt ggt tct gat tcg gtc gat tca ttt gaa       96
   Tyr Ser Ser Val Asp Gly Gly Gly Ser Asp Ser Val Asp Ser Phe Glu
               20                  25                  30
   gac gag cag aaa aag ctc gtt tat aga ggc tgg aaa gtc atg ccc ttt      144
   Asp Glu Gln Lys Lys Leu Val Tyr Arg Gly Trp Lys Val Met Pro Phe
           35                  40                  45
   atc att g gtaacgcttt cttcttctct tcagacaact tttttttttt agtttattct       201
   Ile Ile
       50
   gttttgtttt gttgtgtccc ttttgtcgga ttatagaacg ttttgcccgt gatgttttta    261
   ttttaatttc gtttcttttg tcttcttagt cttgactgtt gaaaatcttg atttggagtt    321
   cttgtcttga cgtttcgaga tgaatgaaac acagatcaaa caaatcaacc aaaccagttg    381
   agtttcctag ataaatctac catggaaagt gagagacttt gaatcatgca agttttcagt    441
   ttcagatata agattttagt tagtttctgg gagttataac aaagaatgaa taataaagtg    501
   tccagtccaa cactgaaagt ccaatctttg atttcaacat tgtgaattaa atgtaaaaag    561
   gttgttactt tatggtttga ctaattattt atgttggaaa aaatatattg tgtcacag      619
   gt  aat gag aca ttt gag aag ata ggg atc att ggg aca cta tca aac      666
   Gly Asn Glu Thr Phe Glu Lys Ile Gly Ile Ile Gly Thr Leu Ser Asn
                   55                  60                  65
   ctt ctg gtg tac cta act tca gta ttc aac ctt aag agt gtt aca gct      714
   Leu Leu Val Tyr Leu Thr Ser Val Phe Asn Leu Lys Ser Val Thr Ala
               70                  75                  80
   gca acc atc atc aac gcc ttc agt ggc act atc aac ttc ggc act ttc      762
   Ala Thr Ile Ile Asn Ala Phe Ser Gly Thr Ile Asn Phe Gly Thr Phe
           85                  90                  95
   ctt gct gct ttc ctc tgc gac act tac ttt ggt cgc tac aag act ctc      810
   Leu Ala Ala Phe Leu Cys Asp Thr Tyr Phe Gly Arg Tyr Lys Thr Leu
       100                 105                 110
   tct gtt gct gtc atc gcc tgt ttt ctg gtactctaat caaatgacca            857
   Ser Val Ala Val Ile Ala Cys Phe Leu
   115                 120
   tcttgtttta ttttggccaa gattttgtct tttttttttt attactaaac catatttcat    917
   atttcttgta g gga tcg ctt gtg ata cta ctg acg gct gca gtt cca gga     967
                Gly Ser Leu Val Ile Leu Leu Thr Ala Ala Val Pro Gly
                    125                 130                 135
   ttg cac ccc att cct tgt gga aca cag agt tct tgc caa ggc cca agc     1015
   Leu His Pro Ile Pro Cys Gly Thr Gln Ser Ser Cys Gln Gly Pro Ser
               140                 145                 150
   gag ggc cag att gcg ttt ctt ctg atg ggt tta gcg ctt ctt gtg gtg        1063
   Glu Gly Gln Ile Ala Phe Leu Leu Met Gly Leu Ala Leu Leu Val Val
               155                 160                 165
   ggt gct ggt gga att agg ccg tgt aac ttg gcc ttt ggt gct gac cag        1111
   Gly Ala Gly Gly Ile Arg Pro Cys Asn Leu Ala Phe Gly Ala Asp Gln
           170                 175                 180
   ttc aac ccc aag tca gaa tct gga aag aaa gga atc aac agt ttc ttc        1159
   Phe Asn Pro Lys Ser Glu Ser Gly Lys Lys Gly Ile Asn Ser Phe Phe
   185                 190                 195                 200
   aac tgg tac ttc ttc act ttc acg ttc gcg cag atc atc tcc ctc aca        1207
   Asn Trp Tyr Phe Phe Thr Phe Thr Phe Ala Gln Ile Ile Ser Leu Thr
                   205                 210                 215
   cta gtt gtg tat atc cag tca aat gtg agc tgg acc atc ggt ttg agt        1255
   Leu Val Val Tyr Ile Gln Ser Asn Val Ser Trp Thr Ile Gly Leu Ser
               220                 225                 230
   atc cct gtg ggt ctt atg ttc ttg gcc tgc gtc att ttc ttt gct ggt        1303
   Ile Pro Val Gly Leu Met Phe Leu Ala Cys Val Ile Phe Phe Ala Gly
           235                 240                 245
   cac aaa ctg tat gtg aaa gtg aaa gcc tct ggt agt cca ttg gct ggt        1351
   His Lys Leu Tyr Val Lys Val Lys Ala Ser Gly Ser Pro Leu Ala Gly
       250                 255                 260
   ata ggt cat gtt ata gcc gca gct atc aag aaa cgt ggc ttg aag cca        1399
   Ile Gly His Val Ile Ala Ala Ala Ile Lys Lys Arg Gly Leu Lys Pro
   265                 270                 275                 280
   gtt aag cag cct tgg atc gat ctt tac aac cac att cct cct aac tat        1447
   Val Lys Gln Pro Trp Ile Asp Leu Tyr Asn His Ile Pro Pro Asn Tyr
                   285                 290                 295
   gca aac tct act ctt aaa tac acc gac cag ctt ag  gtaaagtcgg             1492
   Ala Asn Ser Thr Leu Lys Tyr Thr Asp Gln Leu Arg
               300                 305
   aatagttctg tctctgtctt tctttgttca tttgctttat gctttgtctg aacaaatgtt      1552
   gtattgccag g ttt cta gac aaa gca gcg att atg acc cct gag gac aag       1602
                Phe Leu Asp Lys Ala Ala Ile Met Thr Pro Glu Asp Lys
                    310                 315                 320
   ttg aac tcg gat gga acg gct tcg gat cca tgg aaa cta tgt acg atg        1650
   Leu Asn Ser Asp Gly Thr Ala Ser Asp Pro Trp Lys Leu Cys Thr Met
               325                 330                 335
   cag aaa gtg gaa gaa gtg aaa tgc att tta aga gtg att cca atc tgg        1698
   Gln Lys Val Glu Glu Val Lys Cys Ile Leu Arg Val Ile Pro Ile Trp
           340                 345                 350
   ttc gca tgc gcc att tac tac ctt gca ata agt atg caa atg act tat        1746
   Phe Ala Cys Ala Ile Tyr Tyr Leu Ala Ile Ser Met Gln Met Thr Tyr
       355                 360                 365
   cca gtc ttc caa gct ctc cag agc gac agg cta tta ggt tca ggt ggc        1794
   Pro Val Phe Gln Ala Leu Gln Ser Asp Arg Leu Leu Gly Ser Gly Gly
   370                 375                 380                 385
   ttc aag att cca gcc gcc acc tat gtc gtg ttc ttg atg tct ggt atg        1842
   Phe Lys Ile Pro Ala Ala Thr Tyr Val Val Phe Leu Met Ser Gly Met
                   390                 395                 400
   acg gtt ttc atc ata atc tac gac cgt gtc ctt gtc ccc tcg ctc aaa        1890
   Thr Val Phe Ile Ile Ile Tyr Asp Arg Val Leu Val Pro Ser Leu Lys
               405                 410                 415
   aga gtg acc ggt cta gac acg ggg ata aca ctc tta caa aga ata gga        1938
   Arg Val Thr Gly Leu Asp Thr Gly Ile Thr Leu Leu Gln Arg Ile Gly
           420                 425                 430
   gct ggc att ttc ttt gcc gtg ttg agt tta ttg gtc tcc ggg ttt ata        1986
   Ala Gly Ile Phe Phe Ala Val Leu Ser Leu Leu Val Ser Gly Phe Ile
       435                 440                 445
   gaa gaa cgt aga aga acc ttt gcg ctg aca cga cct acg ctc ggg tcg     2034
   Glu Glu Arg Arg Arg Thr Phe Ala Leu Thr Arg Pro Thr Leu Gly Ser
   450                 455                 460                 465
   gag cct cga aca gga gag atc tcc tca atg tca gcg atg tgg ctg att     2082
   Glu Pro Arg Thr Gly Glu Ile Ser Ser Met Ser Ala Met Trp Leu Ile
                   470                 475                 480
   ccg cag ctg gtg ctt gca ggg gta gca gaa gct ttt gta gcc att ggg     2130
   Pro Gln Leu Val Leu Ala Gly Val Ala Glu Ala Phe Val Ala Ile Gly
               485                 490                 495
   caa atg gag ttt tac tac aag cag ttt cct gaa aac atg aag agc ttt     2178
   Gln Met Glu Phe Tyr Tyr Lys Gln Phe Pro Glu Asn Met Lys Ser Phe
           500                 505                 510
   gcg ggt tct atc ttc tat gtc ggt gga gga gtt tct agc tac ctc gct     2226
   Ala Gly Ser Ile Phe Tyr Val Gly Gly Gly Val Ser Ser Tyr Leu Ala
       515                 520                 525
   agc ttc ttg atc tcg act gtt cat aga aca act gag cat tca cct acg     2274
   Ser Phe Leu Ile Ser Thr Val His Arg Thr Thr Glu His Ser Pro Thr
   530                 535                 540                 545
   ggg aat tgg tta gct gag gat ctg aac aaa gcg aag ctg gac tac ttc     2322
   Gly Asn Trp Leu Ala Glu Asp Leu Asn Lys Ala Lys Leu Asp Tyr Phe
                   550                 555                 560
   tat ttc atg ctc aca gga ctc atg ttt gtc aac ttt ctt tac ttc ttg     2370
   Tyr Phe Met Leu Thr Gly Leu Met Phe Val Asn Phe Leu Tyr Phe Leu
               565                 570                 575
   tta atg gct aga tgg tat aga tac aaa ggc act cat gat gaa gtc agt     2418
   Leu Met Ala Arg Trp Tyr Arg Tyr Lys Gly Thr His Asp Glu Val Ser
           580                 585                 590
   tat gtg gtt gac act gtt gac gaa gag acg aag caa caa gac aag aac     2466
   Tyr Val Val Asp Thr Val Asp Glu Glu Thr Lys Gln Gln Asp Lys Asn
       595                 600                 605
   tct gtc tga                                                         2475
   Ser Val
   610
<210> 20
   <211> 611
   <212> PRT
   <213> Brassica napus
<400> 20
   Met Glu Arg Lys Pro Phe Asp Val Glu Thr Thr Glu Asp His Lys Ala
   1 5 10 15
   Tyr Ser Ser Val Asp Gly Gly Gly Ser Asp Ser Val Asp Ser Phe Glu
   20 25 30
   Asp Glu Gln Lys Lys Leu Val Tyr Arg Gly Trp Lys Val Met Pro Phe
   35 40 45
   Ile Ile Gly Asn Glu Thr Phe Glu Lys Ile Gly Ile Ile Gly Thr Leu
   50 55 60
   Ser Asn Leu Leu Val Tyr Leu Thr Ser Val Phe Asn Leu Lys Ser Val
   65 70 75 80
   Thr Ala Ala Thr Ile Ile Asn Ala Phe Ser Gly Thr Ile Asn Phe Gly
   85 90 95
   Thr Phe Leu Ala Ala Phe Leu Cys Asp Thr Tyr Phe Gly Arg Tyr Lys
   100 105 110
   Thr Leu Ser Val Ala Val Ile Ala Cys Phe Leu Gly Ser Leu Val Ile
   115 120 125
   Leu Leu Thr Ala Ala Val Pro Gly Leu His Pro Ile Pro Cys Gly Thr
   130 135 140
   Gln Ser Ser Cys Gln Gly Pro Ser Glu Gly Gln Ile Ala Phe Leu Leu
   145 150 155 160
   Met Gly Leu Ala Leu Leu Val Val Gly Ala Gly Gly Ile Arg Pro Cys
   165 170 175
   Asn Leu Ala Phe Gly Ala Asp Gln Phe Asn Pro Lys Ser Glu Ser Gly
   180 185 190
   Lys Lys Gly Ile Asn Ser Phe Phe Asn Trp Tyr Phe Phe Thr Phe Thr
   195 200 205
   Phe Ala Gln Ile Ile Ser Leu Thr Leu Val Val Tyr Ile Gln Ser Asn
   210 215 220
   Val Ser Trp Thr Ile Gly Leu Ser Ile Pro Val Gly Leu Met Phe Leu
   225 230 235 240
   Ala Cys Val Ile Phe Phe Ala Gly His Lys Leu Tyr Val Lys Val Lys
   245 250 255
   Ala Ser Gly Ser Pro Leu Ala Gly Ile Gly His Val Ile Ala Ala Ala
   260 265 270
   Ile Lys Lys Arg Gly Leu Lys Pro Val Lys Gln Pro Trp Ile Asp Leu
   275 280 285
   Tyr Asn His Ile Pro Pro Asn Tyr Ala Asn Ser Thr Leu Lys Tyr Thr
   290 295 300
   Asp Gln Leu Arg Phe Leu Asp Lys Ala Ala Ile Met Thr Pro Glu Asp
   305 310 315 320
   Lys Leu Asn Ser Asp Gly Thr Ala Ser Asp Pro Trp Lys Leu Cys Thr
   325 330 335
   Met Gln Lys Val Glu Glu Val Lys Cys Ile Leu Arg Val Ile Pro Ile
   340 345 350
   Trp Phe Ala Cys Ala Ile Tyr Tyr Leu Ala Ile Ser Met Gln Met Thr
   355 360 365
   Tyr Pro Val Phe Gln Ala Leu Gln Ser Asp Arg Leu Leu Gly Ser Gly
   370 375 380
   Gly Phe Lys Ile Pro Ala Ala Thr Tyr Val Val Phe Leu Met Ser Gly
   385 390 395 400
   Met Thr Val Phe Ile Ile Ile Tyr Asp Arg Val Leu Val Pro Ser Leu
   405 410 415
   Lys Arg Val Thr Gly Leu Asp Thr Gly Ile Thr Leu Leu Gln Arg Ile
   420 425 430
   Gly Ala Gly Ile Phe Phe Ala Val Leu Ser Leu Leu Val Ser Gly Phe
   435 440 445
   Ile Glu Glu Arg Arg Arg Thr Phe Ala Leu Thr Arg Pro Thr Leu Gly
   450 455 460
   Ser Glu Pro Arg Thr Gly Glu Ile Ser Ser Met Ser Ala Met Trp Leu
   465 470 475 480
   Ile Pro Gln Leu Val Leu Ala Gly Val Ala Glu Ala Phe Val Ala Ile
   485 490 495
   Gly Gln Met Glu Phe Tyr Tyr Lys Gln Phe Pro Glu Asn Met Lys Ser
   500 505 510
   Phe Ala Gly Ser Ile Phe Tyr Val Gly Gly Gly Val Ser Ser Tyr Leu
   515 520 525
   Ala Ser Phe Leu Ile Ser Thr Val His Arg Thr Thr Glu His Ser Pro
   530 535 540
   Thr Gly Asn Trp Leu Ala Glu Asp Leu Asn Lys Ala Lys Leu Asp Tyr
   545 550 555 560
   Phe Tyr Phe Met Leu Thr Gly Leu Met Phe Val Asn Phe Leu Tyr Phe
   565 570 575
   Leu Leu Met Ala Arg Trp Tyr Arg Tyr Lys Gly Thr His Asp Glu Val
   580 585 590
   Ser Tyr Val Val Asp Thr Val Asp Glu Glu Thr Lys Gln Gln Asp Lys
   595 600 605
   Asn Ser Val
   610
<210> 21
   <211> 2623
   <212> DNA
   <213> Brassica napus
<220>
   <221> CDS
   <222> (1)..(160)
<220>
   <221> CDS
   <222> (638)..(855)
<220>
   <221> CDS
   <222> (955)..(1508)
<220>
   <221> CDS
   <222> (1708)..(2620)
<400> 21
   atg gaa aga aag cct ttt gag gtt gag aca aca aag acg gat gag gat      48
   Met Glu Arg Lys Pro Phe Glu Val Glu Thr Thr Lys Thr Asp Glu Asp
   1               5                   10                  15
   cag aaa ccc tac tcc gcc gtc gat ggc ggt ggt tct gat tcg gtc gat      96
   Gln Lys Pro Tyr Ser Ala Val Asp Gly Gly Gly Ser Asp Ser Val Asp
               20                  25                  30
   tca ttt gat gaa gat cag aaa aag ctc gtg tat aga gga tgg aaa gtc     144
   Ser Phe Asp Glu Asp Gln Lys Lys Leu Val Tyr Arg Gly Trp Lys Val
           35                  40                  45
   atg cct ttt atc att g gtaacgcttt ttttttttct ctattcagac actccgtttt   200
   Met Pro Phe Ile Ile
       50
   tttttttatt gtgcgtctgc ttttctcaac acggtccaga aactaaataa agatgtgaaa   260
   ataaactaga acatgtcgtc cgtgaatttt aaaagtactt ttcttttctc ttcttctctt   320
   tgactgaaaa cgaatgaaac caggatcaac caaatctacc agatctagag aaagagatat   380
   tacaactttt ttcattaata atgcttttta atttttagtt ttatacgctt aaagacaaag   440
   ttatttagtt tctgggagtt acagtaaagt ttcaagttaa agaagcactg aaagtacaat   500
   cttcgattcg tgtgtgtttc ggtttaaaag ttgtgaatta aaaagtttga tactttatgg   560
   gaaacccgtt ttttctttga caactgattc atttattgtg aaaaagaaat taataatgat   620
   tcttgttttt tttatag gt  aat gaa aca ttt gag aag att ggg atc att      669
                      Gly Asn Glu Thr Phe Glu Lys Ile Gly Ile Ile
                          55                  60
   ggg aca tta tca aac ctt ctc gtc tac cta act caa gta ttc aac ctc     717
   Gly Thr Leu Ser Asn Leu Leu Val Tyr Leu Thr Gln Val Phe Asn Leu
   65                  70                  75                  80
   aag aaa tac aca gct gca acc atc atc aat gcc ttt agt ggc acc atc     765
   Lys Lys Tyr Thr Ala Ala Thr Ile Ile Asn Ala Phe Ser Gly Thr Ile
                   85                  90                  95
   aac ttc ggc act ttc ctc gct gct ttc ctc tgc gac act tac ttt ggt     813
   Asn Phe Gly Thr Phe Leu Ala Ala Phe Leu Cys Asp Thr Tyr Phe Gly
               100                 105                 110
   cgc tac aag act ctc agt gtt gct gtc gtc gct tgt ttt ctg             855
   Arg Tyr Lys Thr Leu Ser Val Ala Val Val Ala Cys Phe Leu
           115                 120                 125
   gtaactacta atgctgttct taacggttta gttttcgtca agactctatt cttttttgtt    915
   tttttttgtt tttgctgaac cgtgttttat ctcttgtag gga tcg ctt gcg ata      969
                                             Gly Ser Leu Ala Ile
                                                         130
   cta ctc acg gca gca gtt ccg gga ctg cac ccg act cct tgt gga aca    1017
   Leu Leu Thr Ala Ala Val Pro Gly Leu His Pro Thr Pro Cys Gly Thr
               135                 140                 145
   aaa agt tca tgc gaa ggg cca ggt gcg ggc cag att ttg ttt ctg atg    1065
   Lys Ser Ser Cys Glu Gly Pro Gly Ala Gly Gln Ile Leu Phe Leu Met
           150                 155                 160
   ttg ggt tta gcg ctt ctt gtg gtt ggt gct ggt ggt atc agg ccg tgt    1113
   Leu Gly Leu Ala Leu Leu Val Val Gly Ala Gly Gly Ile Arg Pro Cys
       165                 170                 175
   aac ttg gcg ttc ggt gcc gat cag ttc aac ccc aag tcc gaa tca gga    1161
   Asn Leu Ala Phe Gly Ala Asp Gln Phe Asn Pro Lys Ser Glu Ser Gly
   180                 185                 190                 195
   aag aaa gga atc aac agt ttc ttc aac tgg tac ttc ttc act ttc acg      1209
   Lys Lys Gly Ile Asn Ser Phe Phe Asn Trp Tyr Phe Phe Thr Phe Thr
                   200                 205                 210
   ttc gcg cag atc atc tcg ctc acg cta gtt gtg tat atc cag tcg aat      1257
   Phe Ala Gln Ile Ile Ser Leu Thr Leu Val Val Tyr Ile Gln Ser Asn
               215                 220                 225
   gtg agc tgg acc atc ggt ttg agt atc ccc gtg ggt ctc atg ttc ttg      1305
   Val Ser Trp Thr Ile Gly Leu Ser Ile Pro Val Gly Leu Met Phe Leu
           230                 235                 240
   gct tgc gtc att ttc ttc gct ggt cat aaa ctt tat gtt aaa gtg aaa      1353
   Ala Cys Val Ile Phe Phe Ala Gly His Lys Leu Tyr Val Lys Val Lys
       245                 250                 255
   gcc tcg ggc agt ccc ttg gct ggt ata gct cgt gtt ata gca gca gcg      1401
   Ala Ser Gly Ser Pro Leu Ala Gly Ile Ala Arg Val Ile Ala Ala Ala
   260                 265                 270                 275
   atc tcg aaa cga gga ttg aag ccg gtc aag cag cct tgg gtc gat ctt      1449
   Ile Ser Lys Arg Gly Leu Lys Pro Val Lys Gln Pro Trp Val Asp Leu
                   280                 285                 290
   tac aat cac att cct cgt aac tat gca aac ttg acc cta aaa tac acc      1497
   Tyr Asn His Ile Pro Arg Asn Tyr Ala Asn Leu Thr Leu Lys Tyr Thr
               295                 300                 305
   gac cag ttc ag  gtatactcaa tagttctgtc tctattgctt tatatccgga          1548
   Asp Gln Phe Arg
           310
   tgaaacattc gcctttactt ctaaatattt ttaaaatggg tcgtatagac cccaagttgt    1608
   caaatttgtt taccaaaata tatatttttt atttaccaaa atatttttaa aatgggtccg      1668
   gttggaaact aaatgttggt ttgttcgtta tattgttag g ttt cta gac aaa gca     1723
                                                Phe Leu Asp Lys Ala
                                                            315
   gcg ata atg aca cct gag gac aag ttg aac tct gat gga aca gct tcg      1771
   Ala Ile Met Thr Pro Glu Asp Lys Leu Asn Ser Asp Gly Thr Ala Ser
               320                 325                 330
   gat cca tgg aaa cta tgt acg atg cag caa gtg gaa gaa gtg aaa tgc      1819
   Asp Pro Trp Lys Leu Cys Thr Met Gln Gln Val Glu Glu Val Lys Cys
           335                 340                 345
   att ata aga gtg att cca atc tgg ttt gct tgc gct atc tac tac ctt      1867
   Ile Ile Arg Val Ile Pro Ile Trp Phe Ala Cys Ala Ile Tyr Tyr Leu
       350                 355                 360
   gca ata agc att caa atg aca tat ccc gtc ttc caa gcg caa cag agc      1915
   Ala Ile Ser Ile Gln Met Thr Tyr Pro Val Phe Gln Ala Gln Gln Ser
   365                 370                 375                 380
   gac agg aga tta ggt acc ggt ggc ttc aag atg ccc gca gcc acc tac      1963
   Asp Arg Arg Leu Gly Thr Gly Gly Phe Lys Met Pro Ala Ala Thr Tyr
                   385                 390                 395
   gta gtg ttc ttg atg acc ggt atg acg gtt ttc atc ata atc tac gac      2011
   Val Val Phe Leu Met Thr Gly Met Thr Val Phe Ile Ile Ile Tyr Asp
               400                 405                 410
   cgt gtc ctt gtc cca tcg ctc aaa aga gtg acc ggt cta gac act ggt      2059
   Arg Val Leu Val Pro Ser Leu Lys Arg Val Thr Gly Leu Asp Thr Gly
           415                 420                 425
   ata aca ctc ttg caa agg atc gga gcg gga atc ttc ttt gcg gtg ctg      2107
   Ile Thr Leu Leu Gln Arg Ile Gly Ala Gly Ile Phe Phe Ala Val Leu
       430                 435                 440
   agt tta ttg gtc tcc ggg ttc ata gag gaa cgt aga aga agc att gcg      2155
   Ser Leu Leu Val Ser Gly Phe Ile Glu Glu Arg Arg Arg Ser Ile Ala
   445 450 455 460
   ctg aca aaa cag act cta ggg atg gag cct cga aca gga gag atc tca 2203
   Leu Thr Lys Gln Thr Leu Gly Met Glu Pro Arg Thr Gly Glu Ile Ser
   465 470 475
   tca atg tcg gcc atg tgg ctg att ccg cag ctg gtg ctt gca ggt ata 2251
   Ser Met Ser Ala Met Trp Leu Ile Pro Gln Leu Val Leu Ala Gly Ile
   480 485 490
   gct gaa gca ttt aca gcc att gga cag atg gag ttt tac tac aag cag 2299
   Ala Glu Ala Phe Thr Ala Ile Gly Gln Met Glu Phe Tyr Tyr Lys Gln
   495 500 505
   ttt cct gag aac atg agg agc ttt gct ggc tct atc ttc tat gtc ggt 2347
   Phe Pro Glu Asn Met Arg Ser Phe Ala Gly Ser Ile Phe Tyr Val Gly
   510 515 520
   gcg ggg gtt tct agc tac ctc gct agc ttc ttg atc tcc act gtt cat 2395
   Ala Gly Val Ser Ser Tyr Leu Ala Ser Phe Leu Ile Ser Thr Val His
   525 530 535 540
   agg aca act gaa cat tca cct tcg ggg aac tgg tta gct gag gat ctg 2443
   Arg Thr Thr Glu His Ser Pro Ser Gly Asn Trp Leu Ala Glu Asp Leu
   545 550 555
   aac aag gcg aag ttg gat tac ttc tat ttc atg ctc acg gga ctc atg 2491
   Asn Lys Ala Lys Leu Asp Tyr Phe Tyr Phe Met Leu Thr Gly Leu Met
   560 565 570
   ttc gtt aac atg gtt tac ttc ttg ata atg gct aag ttt tat aga tac 2539
   Phe Val Asn Met Val Tyr Phe Leu Ile Met Ala Lys Phe Tyr Arg Tyr
   575 580 585
   aaa ggc agt gat gat aaa gtc atc tct gct att gag acc aat gaa gac 2587
   Lys Gly Ser Asp Asp Lys Val Ile Ser Ala Ile Glu Thr Asn Glu Asp
   590 595 600
   gag acc aag cag aag caa gag aag aac tcg gtg taa 2623
   Glu Thr Lys Gln Lys Gln Glu Lys Asn Ser Val
   605 610 615
<210> 22
   <211> 615
   <212> PRT
   <213> Brassica napus
<400> 22
   Met Glu Arg Lys Pro Phe Glu Val Glu Thr Thr Lys Thr Asp Glu Asp
   1               5                   10                  15
   Gln Lys Pro Tyr Ser Ala Val Asp Gly Gly Gly Ser Asp Ser Val Asp
               20                  25                  30
   Ser Phe Asp Glu Asp Gln Lys Lys Leu Val Tyr Arg Gly Trp Lys Val
           35                  40                  45
   Met Pro Phe Ile Ile Gly Asn Glu Thr Phe Glu Lys Ile Gly Ile Ile
       50                  55                  60
   Gly Thr Leu Ser Asn Leu Leu Val Tyr Leu Thr Gln Val Phe Asn Leu
   65                  70                  75                  80
   Lys Lys Tyr Thr Ala Ala Thr Ile Ile Asn Ala Phe Ser Gly Thr Ile
                   85                  90                  95
   Asn Phe Gly Thr Phe Leu Ala Ala Phe Leu Cys Asp Thr Tyr Phe Gly
               100                 105                 110
   Arg Tyr Lys Thr Leu Ser Val Ala Val Val Ala Cys Phe Leu Gly Ser
           115                 120                 125
   Leu Ala Ile Leu Leu Thr Ala Ala Val Pro Gly Leu His Pro Thr Pro
       130                 135                 140
   Cys Gly Thr Lys Ser Ser Cys Glu Gly Pro Gly Ala Gly Gln Ile Leu
   145                 150                 155                 160
   Phe Leu Met Leu Gly Leu Ala Leu Leu Val Val Gly Ala Gly Gly Ile
                   165                 170                 175
   Arg Pro Cys Asn Leu Ala Phe Gly Ala Asp Gln Phe Asn Pro Lys Ser
               180                 185                 190
   Glu Ser Gly Lys Lys Gly Ile Asn Ser Phe Phe Asn Trp Tyr Phe Phe
           195                 200                 205
   Thr Phe Thr Phe Ala Gln Ile Ile Ser Leu Thr Leu Val Val Tyr Ile
       210                 215                 220
   Gln Ser Asn Val Ser Trp Thr Ile Gly Leu Ser Ile Pro Val Gly Leu
   225                 230                 235                 240
   Met Phe Leu Ala Cys Val Ile Phe Phe Ala Gly His Lys Leu Tyr Val
                   245                 250                 255
   Lys Val Lys Ala Ser Gly Ser Pro Leu Ala Gly Ile Ala Arg Val Ile
               260                 265                 270
   Ala Ala Ala Ile Ser Lys Arg Gly Leu Lys Pro Val Lys Gln Pro Trp
           275                 280                 285
   Val Asp Leu Tyr Asn His Ile Pro Arg Asn Tyr Ala Asn Leu Thr Leu
       290                 295                 300
   Lys Tyr Thr Asp Gln Phe Arg Phe Leu Asp Lys Ala Ala Ile Met Thr
   305                 310                 315                 320
   Pro Glu Asp Lys Leu Asn Ser Asp Gly Thr Ala Ser Asp Pro Trp Lys
                   325                 330                 335
   Leu Cys Thr Met Gln Gln Val Glu Glu Val Lys Cys Ile Ile Arg Val
               340                 345                 350
   Ile Pro Ile Trp Phe Ala Cys Ala Ile Tyr Tyr Leu Ala Ile Ser Ile
           355                 360                 365
   Gln Met Thr Tyr Pro Val Phe Gln Ala Gln Gln Ser Asp Arg Arg Leu
       370                 375                 380
   Gly Thr Gly Gly Phe Lys Met Pro Ala Ala Thr Tyr Val Val Phe Leu
   385                 390                 395                 400
   Met Thr Gly Met Thr Val Phe Ile Ile Ile Tyr Asp Arg Val Leu Val
                   405                 410                 415
   Pro Ser Leu Lys Arg Val Thr Gly Leu Asp Thr Gly Ile Thr Leu Leu
               420                 425                 430
   Gln Arg Ile Gly Ala Gly Ile Phe Phe Ala Val Leu Ser Leu Leu Val
           435                 440                 445
   Ser Gly Phe Ile Glu Glu Arg Arg Arg Ser Ile Ala Leu Thr Lys Gln
       450                 455                 460
   Thr Leu Gly Met Glu Pro Arg Thr Gly Glu Ile Ser Ser Met Ser Ala
   465                 470                 475                 480
   Met Trp Leu Ile Pro Gln Leu Val Leu Ala Gly Ile Ala Glu Ala Phe
                   485                 490                 495
   Thr Ala Ile Gly Gln Met Glu Phe Tyr Tyr Lys Gln Phe Pro Glu Asn
               500                 505                 510
   Met Arg Ser Phe Ala Gly Ser Ile Phe Tyr Val Gly Ala Gly Val Ser
           515                 520                 525
   Ser Tyr Leu Ala Ser Phe Leu Ile Ser Thr Val His Arg Thr Thr Glu
       530                 535                 540
   His Ser Pro Ser Gly Asn Trp Leu Ala Glu Asp Leu Asn Lys Ala Lys
   545                 550                 555                 560
   Leu Asp Tyr Phe Tyr Phe Met Leu Thr Gly Leu Met Phe Val Asn Met
                   565                 570                 575
   Val Tyr Phe Leu Ile Met Ala Lys Phe Tyr Arg Tyr Lys Gly Ser Asp
               580                 585                 590
   Asp Lys Val Ile Ser Ala Ile Glu Thr Asn Glu Asp Glu Thr Lys Gln
           595                 600                 605
   Lys Gln Glu Lys Asn Ser Val
       610                 615
<210> 23
   <211> 2363
   <212> DNA
   <213> Brassica napus
<220>
   <221> CDS
   <222> (1)..(154)
<220>
   <221> CDS
   <222> (516)..(733)
<220>
   <221> CDS
   <222> (812)..(1365)
<220>
   <221> CDS
   <222> (1442)..(2360)
<400> 23
   atg gag aga aag ccc ttt gag gtt gag acg acg gag gat cac aaa ccc        48
   Met Glu Arg Lys Pro Phe Glu Val Glu Thr Thr Glu Asp His Lys Pro
   1               5                   10                  15
   tac tcc gat gga ggt ggt tct gat tta acg tcg acg gtt gat tca tct        96
   Tyr Ser Asp Gly Gly Gly Ser Asp Leu Thr Ser Thr Val Asp Ser Ser
               20                  25                  30
   ggc gac gag cag aaa aag ctc gtt tat aga ggc tgg aaa gtc atg cct       144
   Gly Asp Glu Gln Lys Lys Leu Val Tyr Arg Gly Trp Lys Val Met Pro
           35                  40                  45
   tat atc att g gtaacgcttt tctctctctc tctagaccgt tatttttaaa             194
   Tyr Ile Ile
       50
   ctctgttttc tttttgtcaa ctaactctgt tttttcgtgt aacagaacac ttttccccgt      254
   gatgggtttt tttgtcattt aataattttg tttattttta tcttgtcacg ttggcttgac      314
   tgtgtcagtg ttctagataa tctaccaggt gtagtgtaca gaaagaaatg ttaccttgtg      374
   ttctttagta acgtttttaa atgtctgtgg gaaggacttt gaatcactgg aagtccaatc      434
   ttggttccaa actgtgttac tttatgagaa acccgtgttg gtttgactat tcattaacga      494
   aaagaatttg tatcttgata g gt  aat gag aca ttt gag aag att ggg atc       544
                           Gly Asn Glu Thr Phe Glu Lys Ile Gly Ile
                                       55                  60
   ata ggg aca tta tca aac ctt cta gtg tac cta act caa gta ttc aac       592
   Ile Gly Thr Leu Ser Asn Leu Leu Val Tyr Leu Thr Gln Val Phe Asn
               65                  70                  75
   ctt aag aaa tac aca gct gca act att atc aat gcc ttt agt ggc act       640
   Leu Lys Lys Tyr Thr Ala Ala Thr Ile Ile Asn Ala Phe Ser Gly Thr
           80                  85                  90
   atc aac ttc ggc act ttc ttc gct gct ttt ctg tgt gac act tac ttt       688
   Ile Asn Phe Gly Thr Phe Phe Ala Ala Phe Leu Cys Asp Thr Tyr Phe
       95                  100                 105
   ggc cgc tac aag act ctc agt gtg gct gtc atc gct tgt ttt ctg           733
   Gly Arg Tyr Lys Thr Leu Ser Val Ala Val Ile Ala Cys Phe Leu
   110                 115                 120
   gtactaatgc tgctgatctt aatgggtttt ttttcttacg ccaagactct taatgctgat      793
   catttttcgt ctgtgtag gga tcg gtt ttt ata cta atg acg gct gca gtt       844
                       Gly Ser Val Phe Ile Leu Met Thr Ala Ala Val
                       125                 130                 135
   ccg gga ttg cac ccg act cct tgt gga act aaa agt tcg tgc caa ggg       892
   Pro Gly Leu His Pro Thr Pro Cys Gly Thr Lys Ser Ser Cys Gln Gly
                   140                 145                 150
   cca aat ggg ggg cag gtt ttg ttt ctg ctg ttg ggt tta gcg ctt ctc       940
   Pro Asn Gly Gly Gln Val Leu Phe Leu Leu Leu Gly Leu Ala Leu Leu
               155                 160                 165
   gta gtc ggt gct ggt ggt atc agg ccg tgt aat ttg gcc ttt gga gct       988
   Val Val Gly Ala Gly Gly Ile Arg Pro Cys Asn Leu Ala Phe Gly Ala
           170                 175                 180
   gat cag ttc aac ccc aag tca gaa tct gga aag aaa gga atc aac agt      1036
   Asp Gln Phe Asn Pro Lys Ser Glu Ser Gly Lys Lys Gly Ile Asn Ser
       185                 190                 195
   ttc ttc aac tgg tac ttc ttc act ttc acg ttt gcg cag atc atc tcg      1084
   Phe Phe Asn Trp Tyr Phe Phe Thr Phe Thr Phe Ala Gln Ile Ile Ser
   200                 205                 210                 215
   ctc acg cta gtt gtg tat atc cag tca aac gtg agc tgg acc att ggt      1132
   Leu Thr Leu Val Val Tyr Ile Gln Ser Asn Val Ser Trp Thr Ile Gly
                   220                 225                 230
   ttg agt atc cct gtg ggt cta atg ttc ttg gcc tgc gtc att tac ttt      1180
   Leu Ser Ile Pro Val Gly Leu Met Phe Leu Ala Cys Val Ile Tyr Phe
               235                 240                 245
   gct ggc cat aat ctc tac gta aaa gtg aaa gcc tcg ggt agt ccc ttg      1228
   Ala Gly His Asn Leu Tyr Val Lys Val Lys Ala Ser Gly Ser Pro Leu
           250                 255                 260
   gct ggc atc gct cgt gtt ata gct gca gcg atc aag aaa cga ggg ttg      1276
   Ala Gly Ile Ala Arg Val Ile Ala Ala Ala Ile Lys Lys Arg Gly Leu
       265                 270                 275
   aag cct gtt aag aag cct tgc tcc gat ctt tac aat cac atc ccg cct      1324
   Lys Pro Val Lys Lys Pro Cys Ser Asp Leu Tyr Asn His Ile Pro Pro
   280                 285                 290                 295
   aac tat gca aac tct aca ctc aaa tac tcc gac cag ttt ag               1365
   Asn Tyr Ala Asn Ser Thr Leu Lys Tyr Ser Asp Gln Phe Arg
                   300                 305
   gtaaaattca aaaagtttct ctctctctct ctgttgcttt atgttttgtt ttgatgaaaa    1425
   catgttatgt tgctag a ttt ctc gac aaa gca gca gtc acg acc cct gat      1475
                       Phe Leu Asp Lys Ala Ala Val Thr Thr Pro Asp
                       310                 315                 320
   gac aag ttg aaa ccc gat gga acg gct tca gat cca tgg aat cta tgt      1523
   Asp Lys Leu Lys Pro Asp Gly Thr Ala Ser Asp Pro Trp Asn Leu Cys
                   325                 330                 335
   acg atg cag cag gtg gaa gaa gtg aaa tgc att gta aga gtg att cca      1571
   Thr Met Gln Gln Val Glu Glu Val Lys Cys Ile Val Arg Val Ile Pro
               340                 345                 350
   atc tgg ttt gct tgc gcg gtt tac tac ctc gca ata aca ttg caa atg      1619
   Ile Trp Phe Ala Cys Ala Val Tyr Tyr Leu Ala Ile Thr Leu Gln Met
           355                 360                 365
   act tat ccg gtc ttc caa gcg ctc cag agc gac cgg aga ttg ggt tca      1667
   Thr Tyr Pro Val Phe Gln Ala Leu Gln Ser Asp Arg Arg Leu Gly Ser
       370                 375                 380
   ggt ggc ttc aag att cca ggc ggc acc tat gta gtg ttc ttg atg tcc      1715
   Gly Gly Phe Lys Ile Pro Gly Gly Thr Tyr Val Val Phe Leu Met Ser
   385                 390                 395                 400
   ggt atg acg gtt ttc atc ata ttc tac gac cgt gtc ctt gtc ccg tcg      1763
   Gly Met Thr Val Phe Ile Ile Phe Tyr Asp Arg Val Leu Val Pro Ser
                   405                 410                 415
   ctc aga aga gtg aca ggg cta gac aat ggt ata act ctc tta caa aga      1811
   Leu Arg Arg Val Thr Gly Leu Asp Asn Gly Ile Thr Leu Leu Gln Arg
               420                 425                 430
   atc gga tca ggc att ttc ttt gcg ctt ttg agt atg ttg att tct ggg      1859
   Ile Gly Ser Gly Ile Phe Phe Ala Leu Leu Ser Met Leu Ile Ser Gly
           435                 440                 445
   ttc gtt gag gaa cgg cga aga gcc att gca ctg aca aaa cct act ctc      1907
   Phe Val Glu Glu Arg Arg Arg Ala Ile Ala Leu Thr Lys Pro Thr Leu
       450                 455                 460
   ggg atc gag cct cga gct gga gaa atc tca tca atg tca gcg atg tgg      1955
   Gly Ile Glu Pro Arg Ala Gly Glu Ile Ser Ser Met Ser Ala Met Trp
   465                 470                 475                 480
   ctg ata ccg cag ctc gtg ctt gca ggg ata gct gaa gct ttt gca gct      2003
   Leu Ile Pro Gln Leu Val Leu Ala Gly Ile Ala Glu Ala Phe Ala Ala
                   485                 490                 495
   gtt gga caa atg gag ttt tac tac aag cag ttt cct gaa aac atg aag      2051
   Val Gly Gln Met Glu Phe Tyr Tyr Lys Gln Phe Pro Glu Asn Met Lys
               500                 505                 510
   agc ttt gcg ggt tct atc ttc tat gtc ggt gca gga gtt tct agc tac      2099
   Ser Phe Ala Gly Ser Ile Phe Tyr Val Gly Ala Gly Val Ser Ser Tyr
           515                 520                 525
   ctc gct agc ttc ttg atc tcg act gtt cat aaa aca act gag cat tca     2147
   Leu Ala Ser Phe Leu Ile Ser Thr Val His Lys Thr Thr Glu His Ser
       530                 535                 540
   cgc tcc ggg aat tgg cta gct gag gat ctg aac aaa ggg aag ctg gat     2195
   Arg Ser Gly Asn Trp Leu Ala Glu Asp Leu Asn Lys Gly Lys Leu Asp
   545                 550                 555                 560
   tac ttt tat ttc atg atc act ggc ctc atg ttt gtc aac atg ggt tac     2243
   Tyr Phe Tyr Phe Met Ile Thr Gly Leu Met Phe Val Asn Met Gly Tyr
                   565                 570                 575
   ttc ttg tta atg gct aaa ttt tat aga tac aaa gtc act aac gat gaa     2291
   Phe Leu Leu Met Ala Lys Phe Tyr Arg Tyr Lys Val Thr Asn Asp Glu
               580                 585                 590
   gca aat tct gtg att aag acc aat gaa gaa gag acc aag gag aat cag     2339
   Ala Asn Ser Val Ile Lys Thr Asn Glu Glu Glu Thr Lys Glu Asn Gln
           595                 600                 605
   caa caa gac aag agc tat gtc tga                                     2363
   Gln Gln Asp Lys Ser Tyr Val
       610                 615
<210> 24
   <211> 615
   <212> PRT
   <213> Brassica napus
<400> 24
   Met Glu Arg Lys Pro Phe Glu Val Glu Thr Thr Glu Asp His Lys Pro
   1               5                   10                  15
   Tyr Ser Asp Gly Gly Gly Ser Asp Leu Thr Ser Thr Val Asp Ser Ser
               20                  25                  30
   Gly Asp Glu Gln Lys Lys Leu Val Tyr Arg Gly Trp Lys Val Met Pro
           35                  40                  45
   Tyr Ile Ile Gly Asn Glu Thr Phe Glu Lys Ile Gly Ile Ile Gly Thr
       50                  55                  60
   Leu Ser Asn Leu Leu Val Tyr Leu Thr Gln Val Phe Asn Leu Lys Lys
   65                  70                  75                  80
   Tyr Thr Ala Ala Thr Ile Ile Asn Ala Phe Ser Gly Thr Ile Asn Phe
                   85                  90                  95
   Gly Thr Phe Phe Ala Ala Phe Leu Cys Asp Thr Tyr Phe Gly Arg Tyr
               100                 105                 110
   Lys Thr Leu Ser Val Ala Val Ile Ala Cys Phe Leu Gly Ser Val Phe
           115                 120                 125
   Ile Leu Met Thr Ala Ala Val Pro Gly Leu His Pro Thr Pro Cys Gly
       130                 135                 140
   Thr Lys Ser Ser Cys Gln Gly Pro Asn Gly Gly Gln Val Leu Phe Leu
   145                 150                 155                 160
   Leu Leu Gly Leu Ala Leu Leu Val Val Gly Ala Gly Gly Ile Arg Pro
   165 170 175
   Cys Asn Leu Ala Phe Gly Ala Asp Gln Phe Asn Pro Lys Ser Glu Ser
   180 185 190
   Gly Lys Lys Gly Ile Asn Ser Phe Phe Asn Trp Tyr Phe Phe Thr Phe
   195 200 205
   Thr Phe Ala Gln Ile Ile Ser Leu Thr Leu Val Val Tyr Ile Gln Ser
   210 215 220
   Asn Val Ser Trp Thr Ile Gly Leu Ser Ile Pro Val Gly Leu Met Phe
   225 230 235 240
   Leu Ala Cys Val Ile Tyr Phe Ala Gly His Asn Leu Tyr Val Lys Val
   245 250 255
   Lys Ala Ser Gly Ser Pro Leu Ala Gly Ile Ala Arg Val Ile Ala Ala
   260 265 270
   Ala Ile Lys Lys Arg Gly Leu Lys Pro Val Lys Lys Pro Cys Ser Asp
   275 280 285
   Leu Tyr Asn His Ile Pro Pro Asn Tyr Ala Asn Ser Thr Leu Lys Tyr
   290 295 300
   Ser Asp Gln Phe Arg Phe Leu Asp Lys Ala Ala Val Thr Thr Pro Asp
   305 310 315 320
   Asp Lys Leu Lys Pro Asp Gly Thr Ala Ser Asp Pro Trp Asn Leu Cys
   325 330 335
   Thr Met Gln Gln Val Glu Glu Val Lys Cys Ile Val Arg Val Ile Pro
   340 345 350
   Ile Trp Phe Ala Cys Ala Val Tyr Tyr Leu Ala Ile Thr Leu Gln Met
   355 360 365
   Thr Tyr Pro Val Phe Gln Ala Leu Gln Ser Asp Arg Arg Leu Gly Ser
   370 375 380
   Gly Gly Phe Lys Ile Pro Gly Gly Thr Tyr Val Val Phe Leu Met Ser
   385 390 395 400
   Gly Met Thr Val Phe Ile Ile Phe Tyr Asp Arg Val Leu Val Pro Ser
   405 410 415
   Leu Arg Arg Val Thr Gly Leu Asp Asn Gly Ile Thr Leu Leu Gln Arg
   420 425 430
   Ile Gly Ser Gly Ile Phe Phe Ala Leu Leu Ser Met Leu Ile Ser Gly
   435 440 445
   Phe Val Glu Glu Arg Arg Arg Ala Ile Ala Leu Thr Lys Pro Thr Leu
   450 455 460
   Gly Ile Glu Pro Arg Ala Gly Glu Ile Ser Ser Met Ser Ala Met Trp
   465 470 475 480
   Leu Ile Pro Gln Leu Val Leu Ala Gly Ile Ala Glu Ala Phe Ala Ala
   485 490 495
   Val Gly Gln Met Glu Phe Tyr Tyr Lys Gln Phe Pro Glu Asn Met Lys
   500 505 510
   Ser Phe Ala Gly Ser Ile Phe Tyr Val Gly Ala Gly Val Ser Ser Tyr
   515 520 525
   Leu Ala Ser Phe Leu Ile Ser Thr Val His Lys Thr Thr Glu His Ser
   530 535 540
   Arg Ser Gly Asn Trp Leu Ala Glu Asp Leu Asn Lys Gly Lys Leu Asp
   545 550 555 560
   Tyr Phe Tyr Phe Met Ile Thr Gly Leu Met Phe Val Asn Met Gly Tyr
   565 570 575
   Phe Leu Leu Met Ala Lys Phe Tyr Arg Tyr Lys Val Thr Asn Asp Glu
   580 585 590
   Ala Asn Ser Val Ile Lys Thr Asn Glu Glu Glu Thr Lys Glu Asn Gln
   595 600 605
   Gln Gln Asp Lys Ser Tyr Val
   610 615
<210> 25
   <211> 2374
   <212> DNA
   <213> Brassica napus
<220>
   <221> CDS
   <222> (1)..(157)
<220>
   <221> CDS
   <222> (499)..(716)
<220>
   <221> CDS
   <222> (812)..(1365)
<220>
   <221> CDS
   <222> (1465)..(2371)
<400> 25
   atg gag aga aag cct cta gaa gtc gag tca acg gat cac caa aag cct      48
   Met Glu Arg Lys Pro Leu Glu Val Glu Ser Thr Asp His Gln Lys Pro
   1               5                   10                  15
   ccc tcc gcc gtg tac gat ggc tct gtt atg gcg gtt gat tct gtt gag      96
   Pro Ser Ala Val Tyr Asp Gly Ser Val Met Ala Val Asp Ser Val Glu
               20                  25                  30
   gaa gaa gtt gag gag acc aaa gtc gtt tac aga ggc tgg aaa gtc atg     144
   Glu Glu Val Glu Glu Thr Lys Val Val Tyr Arg Gly Trp Lys Val Met
           35                  40                  45
   ccc ttt atc att g gtaacacaaa cttttttttt ataaactttc ttcttttgtt       197
   Pro Phe Ile Ile
   50
   ccaagaactt ttttttttaa ttgcttttca tgtttttttc caaaataaat aatcaatagt      257
   tgaagaaatg aagaggaaaa catcagtctt gcttgaagac ttattggttt gatttaaaaa    317
   aaaaaaatcg tgagattctc tgtactgttg tcaaaagctt tgacttagtc aaccagtttt    377
   ttaagaaaat taaaaaaaag agagtgagat ctagaatttg agaaggtttc agaacataac    437
   tttgaggcat atgagatcaa agtcttatga tcatcttggt tttgtttgtt ggtattatca    497
   g ga  aat gag acg ttt gag aag ctt ggg atc att gga aca cta tca aac    545
     Gly Asn Glu Thr Phe Glu Lys Leu Gly Ile Ile Gly Thr Leu Ser Asn
             55                  60                  65
   ctt ctg gtt tat tta act gct gtc ttc aac atg aag agt atc aca gct      593
   Leu Leu Val Tyr Leu Thr Ala Val Phe Asn Met Lys Ser Ile Thr Ala
       70                  75                  80
   gca aca atc atc aat gca ttc agt ggc aca atc aac ttc gga acc ttc      641
   Ala Thr Ile Ile Asn Ala Phe Ser Gly Thr Ile Asn Phe Gly Thr Phe
   85                  90                  95                  100
   gtt gct gct ttc ctc tgc gac act tac ttc ggt cga tac aag act cta      689
   Val Ala Ala Phe Leu Cys Asp Thr Tyr Phe Gly Arg Tyr Lys Thr Leu
                   105                 110                 115
   act gtc gct gtc atc gcc tgt ttt ctt gtaatgatca ttcctttgca            736
   Thr Val Ala Val Ile Ala Cys Phe Leu
               120                 125
   atctttcttg ctccacaagc gttcttagtt taacaaccct aaaaatctga taattgttgt    796
   tctttttttt tgtag gga tca ctt gtg ata cta ttg aca gct gca gtg cca       847
                    Gly Ser Leu Val Ile Leu Leu Thr Ala Ala Val Pro
                                   130                 135
   caa tta cat cca gct cca tgc gga aca gct ctc tca tgt atc ggg cca      895
   Gln Leu His Pro Ala Pro Cys Gly Thr Ala Leu Ser Cys Ile Gly Pro
           140                 145                 150
   agt ggt ggc cag ata gcg ttt cta ctg ctg ggt ctc ggg ttt ctt gta      943
   Ser Gly Gly Gln Ile Ala Phe Leu Leu Leu Gly Leu Gly Phe Leu Val
       155                 160                 165
   gtt gga gca ggc ggg att aga cca tgt aat cta gct ttc ggg gct gat      991
   Val Gly Ala Gly Gly Ile Arg Pro Cys Asn Leu Ala Phe Gly Ala Asp
   170                 175                 180                 185
   cag ttt aac ccc aag agc gag tca gga aaa cga ggc atc gat agt ttc     1039
   Gln Phe Asn Pro Lys Ser Glu Ser Gly Lys Arg Gly Ile Asp Ser Phe
                   190                 195                 200
   ttc aat tgg tac ttc ttc acc ttc acg ttc gcg cag atc ctg tcg ctg     1087
   Phe Asn Trp Tyr Phe Phe Thr Phe Thr Phe Ala Gln Ile Leu Ser Leu
               205                 210                 215
   aca cta atc gtc tat atc cag tcc aat gtc agc tgg aca atc ggt tta     1135
   Thr Leu Ile Val Tyr Ile Gln Ser Asn Val Ser Trp Thr Ile Gly Leu
           220                 225                 230
   acc atc ccg gtc gtt ctc atg ttc ttg gcc tgc gtg att ttc ttt gct     1183
   Thr Ile Pro Val Val Leu Met Phe Leu Ala Cys Val Ile Phe Phe Ala
       235                 240                 245
   gga gat aag ttg tat gtg aag atc aaa gcc tca ggt agt cca ttg gct     1231
   Gly Asp Lys Leu Tyr Val Lys Ile Lys Ala Ser Gly Ser Pro Leu Ala
   250                 255                 260                 265
   ggt ata gct caa gtt ata gcg gtt gca atc aag aaa cgt ggg tta aag     1279
   Gly Ile Ala Gln Val Ile Ala Val Ala Ile Lys Lys Arg Gly Leu Lys
                   270                 275                 280
   cca acg aaa gag cct tgg ctt aac ctt tac aac tac tac cca cca aaa     1327
   Pro Thr Lys Glu Pro Trp Leu Asn Leu Tyr Asn Tyr Tyr Pro Pro Lys
                   285                     290                     295
   tac gca aac tcc aaa ctc aaa tac acc gac cag ttt ag  gtaacaaaac      1375
   Tyr Ala Asn Ser Lys Leu Lys Tyr Thr Asp Gln Phe Arg
           300                 305
   acacacacgc caagagttag ttttatattt aatatcatat ctttccactt ttgttttatg    1435
   gttactgagg aagtttcctg tttcggcag g ttc ctt gat aag gcg gcg att ttg    1489
                                     Phe Leu Asp Lys Ala Ala Ile Leu
                                                     315
   gct ccc gaa gac aag ttg gag gct gat ggt aag cct gcg gat ccc tgg      1537
   Ala Pro Glu Asp Lys Leu Glu Ala Asp Gly Lys Pro Ala Asp Pro Trp
       320                 325                 330
   aag ctt tgt aca atg caa caa gtt gaa gaa gtg aag tgc att gtg aga      1585
   Lys Leu Cys Thr Met Gln Gln Val Glu Glu Val Lys Cys Ile Val Arg
   335                 340                 345                 350
   gtg ctt cct ata tgg ttt gct gca tcg atc tat tac ttg acc atc acg      1633
   Val Leu Pro Ile Trp Phe Ala Ala Ser Ile Tyr Tyr Leu Thr Ile Thr
                   355                 360                 365
   caa caa atg act tac ccc gtc ttc caa gcc ctc cag agc gac cgt cgc      1681
   Gln Gln Met Thr Tyr Pro Val Phe Gln Ala Leu Gln Ser Asp Arg Arg
               370                 375                 380
   tta gga tcc aga gga ttt gtg atc ccg gca gcc acc tac gtt gtc ttt      1729
   Leu Gly Ser Arg Gly Phe Val Ile Pro Ala Ala Thr Tyr Val Val Phe
           385                 390                 395
   ttg atg aca gga atg acg gtt ttc atc ata ttc tac gac cgt gtc ctc      1777
   Leu Met Thr Gly Met Thr Val Phe Ile Ile Phe Tyr Asp Arg Val Leu
       400                 405                 410
   gtg cct acc tta aga aga ata acc ggt ata gac acg ggg ata acg cta      1825
   Val Pro Thr Leu Arg Arg Ile Thr Gly Ile Asp Thr Gly Ile Thr Leu
   415                 420                 425                 430
   ctg cag agg att gga acc gga att ttc ttc gcc ttt gtt agc ttg ata      1873
   Leu Gln Arg Ile Gly Thr Gly Ile Phe Phe Ala Phe Val Ser Leu Ile
                   435                 440                 445
   gtc tcc ggt ttc gtc gag gaa cgg agg aga acg ttc gcg ctg act aaa      1921
   Val Ser Gly Phe Val Glu Glu Arg Arg Arg Thr Phe Ala Leu Thr Lys
               450                 455                 460
   cca aca ctt ggt atg gct cca agg aag gga gaa atc tcc tca atg tca      1969
   Pro Thr Leu Gly Met Ala Pro Arg Lys Gly Glu Ile Ser Ser Met Ser
           465                 470                 475
   gct atg tgg ctg atc cca caa ctc gcc ctt gcg ggt ata gct gag gcg      2017
   Ala Met Trp Leu Ile Pro Gln Leu Ala Leu Ala Gly Ile Ala Glu Ala
       480                 485                 490
   ttt gga gct ata gga cag atg gag ttt tac tac aag cag ttc cct gag      2065
   Phe Gly Ala Ile Gly Gln Met Glu Phe Tyr Tyr Lys Gln Phe Pro Glu
   495                 500                 505                 510
   aac atg agg agt ttt gct ggt tcg atc ttc tat gta gga gca gga gtt      2113
   Asn Met Arg Ser Phe Ala Gly Ser Ile Phe Tyr Val Gly Ala Gly Val
                   515                 520                 525
   tcg agt tac ctc ggt agc ttc ttg att gca atg gtt cac agg acg acg      2161
   Ser Ser Tyr Leu Gly Ser Phe Leu Ile Ala Met Val His Arg Thr Thr
               530                 535                 540
   cag aac tca gct ggt ggt aat tgg ttg gct gag gat ttg aac aaa gga      2209
   Gln Asn Ser Ala Gly Gly Asn Trp Leu Ala Glu Asp Leu Asn Lys Gly
           545                 550                 555
   aga ttg gat tac ttc tat ttc atg atc gct gga atc ttg gca gtt aat      2257
   Arg Leu Asp Tyr Phe Tyr Phe Met Ile Ala Gly Ile Leu Ala Val Asn
       560                 565                 570
   ttt gct tac ttc ttg gtc atg tca aga tgg tat aga tac aaa gga agt        2305
   Phe Ala Tyr Phe Leu Val Met Ser Arg Trp Tyr Arg Tyr Lys Gly Ser
   575                 580                 585                 590
   gat ggt gaa gtg aca act tat gaa acc aat gga gat gcc atc aaa caa        2353
   Asp Gly Glu Val Thr Thr Tyr Glu Thr Asn Gly Asp Ala Ile Lys Gln
                   595                 600                 605
   caa gac aag aat act gtc tga                                            2374
   Gln Asp Lys Asn Thr Val
               610
<210> 26
   <211> 612
   <212> PRT
   <213> Brassica napus
<400> 26
   Met Glu Arg Lys Pro Leu Glu Val Glu Ser Thr Asp His Gln Lys Pro
   1               5                   10                  15
   Pro Ser Ala Val Tyr Asp Gly Ser Val Met Ala Val Asp Ser Val Glu
               20                  25                  30
   Glu Glu Val Glu Glu Thr Lys Val Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Thr Ala Leu Ser Cys Ile Gly Pro Ser Gly Gly Gln Ile Ala Phe
   145                 150                 155                 160
   Leu Leu Leu Gly Leu Gly Phe Leu Val Val Gly Ala Gly Gly Ile Arg
                   165                 170                 175
   Pro Cys Asn Leu Ala Phe Gly Ala Asp Gln Phe Asn Pro Lys Ser Glu
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Leu Ile Val Tyr Ile Gln
       210                 215                 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Gly Ile Ala Gln Val Ile Ala
   260 265 270
   Val Ala Ile Lys Lys Arg Gly Leu Lys Pro Thr Lys Glu Pro Trp Leu
   275 280 285
   Asn Leu Tyr Asn Tyr Tyr Pro Pro Lys Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Ala Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ala Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Arg Gly Phe Val Ile Pro Ala Ala Thr Tyr Val Val Phe Leu Met
   385 390 395 400
   Thr Gly Met Thr Val Phe Ile Ile Phe Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Val Ser Leu Ile Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465 470 475 480
   Trp Leu Ile Pro Gln Leu Ala Leu Ala Gly Ile Ala Glu Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Ala Gly Val Ser Ser
   515 520 525
   Tyr Leu Gly Ser Phe Leu Ile Ala Met Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Tyr Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Gly
               580                 585                 590
   Glu Val Thr Thr Tyr Glu Thr Asn Gly Asp Ala Ile Lys Gln Gln Asp
           595                 600                 605
   Lys Asn Thr Val
       610
<210> 27
   <211> 2371
   <212> DNA
   <213> Brassica napus
<220>
   <221> CDS
   <222> (1)..(157)
<220>
   <221> CDS
   <222> (525)..(742)
<220>
   <221> CDS
   <222> (834)..(1387)
<220>
   <221> CDS
   <222> (1462)..(2368)
<400> 27
   atg gag aga aag cct ctg gaa gtt gag tcc acc gat cat cag aaa cct      48
   Met Glu Arg Lys Pro Leu Glu Val Glu Ser Thr Asp His Gln Lys Pro
   1               5                   10                  15
   tcc tcc gcc gtg tac gac ggc tct gct acg gcg gtt gat tct gtt gaa      96
   Ser Ser Ala Val Tyr Asp Gly Ser Ala Thr Ala Val Asp Ser Val Glu
               20                  25                  30
   gaa gaa gtt cag gaa acc aaa ctc gtt tat aga ggc tgg aaa gtc atg     144
   Glu Glu Val Gln Glu Thr Lys Leu Val Tyr Arg Gly Trp Lys Val Met
           35                  40                  45
   cct ttc atc ata g gtaacgcata tgtttttaaa catattaaaa atataaattt       197
   Pro Phe Ile Ile
       50
   ttgataaaaa aataaatcat tttctttgat tggtatattt ccaaagtttt agaaactata   257
   tttctagaaa tttcctgtaa ttaaattttg acaattttct tctctgtttc tagaaactat   317
   attttgttgc tttgatttta tcattctttg tgttgttgtc ttaagctttg acttagtcaa   377
   acagtaattt aacccaaaag agtgagatct tgagtttgaa gatgtttcag aacacataaa   437
   tgatcaagaa tctcttgttt gattcttcgt ttggttgcta aggtcaaagt cttatgatct   497
   tttcttcttg catgttgtta ttaccag ga  aac gag aca ttt gag aag ctt ggg   550
                                 Gly Asn Glu Thr Phe Glu Lys Leu Gly
                                             55                  60
   atc att gga aca cta tca aac ctt ctg gtt tac tta act gct gtc ttc      598
   Ile Ile Gly Thr Leu Ser Asn Leu Leu Val Tyr Leu Thr Ala Val Phe
           65                  70                  75
   aac atg aag agt atc aca gcc gca aca atc atc aac gcc ttt agt ggc      646
   Asn Met Lys Ser Ile Thr Ala Ala Thr Ile Ile Asn Ala Phe Ser Gly
           80                  85                  90
   aca atc aac ttc gga acc ttc gtt gct gcc ttc ctt tgc gac act tac      694
   Thr Ile Asn Phe Gly Thr Phe Val Ala Ala Phe Leu Cys Asp Thr Tyr
       95                  100                 105
   ttc ggt cgt tac aag aca cta acc gtc gcg gtc atc gca tgt ttt ctt      742
   Phe Gly Arg Tyr Lys Thr Leu Thr Val Ala Val Ile Ala Cys Phe Leu
   110                 115                 120                 125
   gtaataatcc atcctctata accttttctt cctccacaag caatcttggt ttataaacct    802
   aaaaaaaaaa atctggttct tgttttggta g gga tca ctt gtg ata cta tta       854
                                      Gly Ser Leu Val Ile Leu Leu
                                                      130
   act gct gca gta cca caa ctg cat cca gct cca tgc gga aca gcc ctc      902
   Thr Ala Ala Val Pro Gln Leu His Pro Ala Pro Cys Gly Thr Ala Leu
           135                 140                 145
   acg tgt aca ggc cca agt ggc ggc cag ata gcc ttt cta cta atg ggt      950
   Thr Cys Thr Gly Pro Ser Gly Gly Gln Ile Ala Phe Leu Leu Met Gly
       150                 155                 160
   ctt gcc ttt ctt gtg gtg gga gca ggt ggg att aga ccg tgt aat ctt      998
   Leu Ala Phe Leu Val Val Gly Ala Gly Gly Ile Arg Pro Cys Asn Leu
   165                 170                 175                 180
   gct ttt gga gct gat cag ttt aac cct aaa agc gag tca ggg aag aga      1046
   Ala Phe Gly Ala Asp Gln Phe Asn Pro Lys Ser Glu Ser Gly Lys Arg
                   185                 190                 195
   ggg att gat agt ttc ttt aac tgg tac ttc ttc acc ttc acg ttc gcg      1094
   Gly Ile Asp Ser Phe Phe Asn Trp Tyr Phe Phe Thr Phe Thr Phe Ala
               200                 205                 210
   cag atc ttg tcg ctg aca cta gtt gtc tac atc cag tcc aac gtc agt      1142
   Gln Ile Leu Ser Leu Thr Leu Val Val Tyr Ile Gln Ser Asn Val Ser
           215                 220                 225
   tgg acc atc ggt tta acc ata cct gtc gtg tta atg ttc ttg gcc tgc      1190
   Trp Thr Ile Gly Leu Thr Ile Pro Val Val Leu Met Phe Leu Ala Cys
       230                 235                 240
   gtg atc ttc ttt gcg ggt gat aag ttg tat gtc aag atc aaa gcc tca      1238
   Val Ile Phe Phe Ala Gly Asp Lys Leu Tyr Val Lys Ile Lys Ala Ser
   245                 250                 255                 260
   ggt agt cca tta gct agt ata gct caa gtt ata acg gtt gct atc aag      1286
   Gly Ser Pro Leu Ala Ser Ile Ala Gln Val Ile Thr Val Ala Ile Lys
                   265                 270                 275
   aaa cgt ggg tta aag gct gtg aag cag cct tgg gtt aac ctt tac aat      1334
   Lys Arg Gly Leu Lys Ala Val Lys Gln Pro Trp Val Asn Leu Tyr Asn
               280                 285                 290
   tac tac cca cta aac tac gca aac tct aag ctc aag tac acc gac cag      1382
   Tyr Tyr Pro Leu Asn Tyr Ala Asn Ser Lys Leu Lys Tyr Thr Asp Gln
           295                 300                 305
   ttc ag  gtaaagacac acggttataa ataatatctt tccacttttg tttacttaga       1437
   Phe Arg
   aagcaagctt tctttttttg gtag g ttt ctt gat aag gcg gcg atc ttg gct      1489
                                 Phe Leu Asp Lys Ala Ala Ile Leu Ala
                                                     315
   cct gaa gac aag ttg gag gct gat ggt aag cct acg gat cca tgg aag      1537
   Pro Glu Asp Lys Leu Glu Ala Asp Gly Lys Pro Thr Asp Pro Trp Lys
   320                 325                 330                 335
   cta tgt aca atg caa caa gtt gaa gaa gtg aag tgc att gtg aga gtg      1585
   Leu Cys Thr Met Gln Gln Val Glu Glu Val Lys Cys Ile Val Arg Val
                   340                 345                 350
   ctt cct ata tgg ttc gct tca tca atc tat tac ttg acc atc aca caa      1633
   Leu Pro Ile Trp Phe Ala Ser Ser Ile Tyr Tyr Leu Thr Ile Thr Gln
               355                 360                 365
   caa atg acc tac cca gtc ttc caa gcc ctc cag agc gat cgt cgc tta      1681
   Gln Met Thr Tyr Pro Val Phe Gln Ala Leu Gln Ser Asp Arg Arg Leu
           370                 375                 380
   cgc tct gga ggg ttt gtg att cct gca gcc acc tat gtg gtc ttc ttg      1729
   Arg Ser Gly Gly Phe Val Ile Pro Ala Ala Thr Tyr Val Val Phe Leu
       385                 390                 395
   atg acg gga atg act gtt ttc atc ata atc tac gac cgc gta ctc gtg      1777
   Met Thr Gly Met Thr Val Phe Ile Ile Ile Tyr Asp Arg Val Leu Val
   400                 405                 410                 415
   cct acc ttg aga aga atc acc ggt ata gac acc ggg atc acg ctc ttg      1825
   Pro Thr Leu Arg Arg Ile Thr Gly Ile Asp Thr Gly Ile Thr Leu Leu
                   420                 425                 430
   caa agg ata gga acc ggg att ttc ttc gcc ttt ttg agc ttg gta gtg      1873
   Gln Arg Ile Gly Thr Gly Ile Phe Phe Ala Phe Leu Ser Leu Val Val
               435                 440                 445
   tct ggt ttc gtc gag gaa cgg agg aga acc ttc gca ttg act aaa cca      1921
   Ser Gly Phe Val Glu Glu Arg Arg Arg Thr Phe Ala Leu Thr Lys Pro
           450                 455                 460
   aca ctc ggt atg gcg cca cgg aag gga gaa atc tcc tca atg tca gct      1969
   Thr Leu Gly Met Ala Pro Arg Lys Gly Glu Ile Ser Ser Met Ser Ala
       465                 470                 475
   atg tgg ctg ata ccg cag ctc aca ctc gcg ggt gta gcc gag gca ttt      2017
   Met Trp Leu Ile Pro Gln Leu Thr Leu Ala Gly Val Ala Glu Ala Phe
   480                 485                 490                 495
   gga gct att gga cag atg gag ttt tac tac aag cag ttc ccc gag aac      2065
   Gly Ala Ile Gly Gln Met Glu Phe Tyr Tyr Lys Gln Phe Pro Glu Asn
                   500                 505                 510
   atg agg agt ttc gcc ggc tcc atc ttc tat gtc gga gga gga gtt tcg      2113
   Met Arg Ser Phe Ala Gly Ser Ile Phe Tyr Val Gly Gly Gly Val Ser
               515                 520                 525
   agt tac ctc ggc agc ttc ttg att gca acg gta cac agg acg acg cag      2161
   Ser Tyr Leu Gly Ser Phe Leu Ile Ala Thr Val His Arg Thr Thr Gln
           530                 535                 540
   aac tcg gct ggc ggt aac tgg ttg gct gaa gat ttg aac aaa gga aga      2209
   Asn Ser Ala Gly Gly Asn Trp Leu Ala Glu Asp Leu Asn Lys Gly Arg
       545                 550                 555
   ttg gat ttg ttt tac ttc atg atc gct gga atc ttg gcg gtt aat ttt      2257
   Leu Asp Leu Phe Tyr Phe Met Ile Ala Gly Ile Leu Ala Val Asn Phe
   560                 565                 570                 575
   gct tac ttc ttg gtg atg tca aga tgg tat agg tac aaa gga agt gat      2305
   Ala Tyr Phe Leu Val Met Ser Arg Trp Tyr Arg Tyr Lys Gly Ser Asp
                   580                 585                 590
   gat gaa gtg aca gct tat gaa aca aat gga gat gtg atc aaa caa caa      2353
   Asp Glu Val Thr Ala Tyr Glu Thr Asn Gly Asp Val Ile Lys Gln Gln
               595                 600                 605
   gac aag aac aaa gtc taa                                              2371
   Asp Lys Asn Lys Val
           610
<210> 28
   <211> 612
   <212> PRT
   <213> Brassica napus
<400> 28
   Met Glu Arg Lys Pro Leu Glu Val Glu Ser Thr Asp His Gln Lys Pro
   1               5                   10                  15
   Ser Ser Ala Val Tyr Asp Gly Ser Ala Thr Ala Val Asp Ser Val Glu
               20                  25                  30
   Glu Glu Val Gln Glu Thr Lys Leu Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Thr Ala Leu Thr Cys Thr Gly Pro Ser Gly Gly Gln Ile Ala Phe
   145                 150                 155                 160
   Leu Leu Met Gly Leu Ala Phe Leu Val Val Gly Ala Gly Gly Ile Arg
                   165                 170                 175
   Pro Cys Asn Leu Ala Phe Gly Ala Asp Gln Phe Asn Pro Lys Ser Glu
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Leu Val Val Tyr Ile Gln
       210                 215                 220
   Ser Asn Val Ser Trp Thr Ile Gly Leu Thr Ile Pro Val Val Leu Met
   225                 230                 235                 240
   Phe Leu Ala Cys Val Ile Phe Phe Ala Gly Asp Lys Leu Tyr Val Lys
                   245                 250                 255
   Ile Lys Ala Ser Gly Ser Pro Leu Ala Ser Ile Ala Gln Val Ile Thr
               260                 265                 270
   Val Ala Ile Lys Lys Arg Gly Leu Lys Ala Val Lys Gln Pro Trp Val
   275 280 285
   Asn Leu Tyr Asn Tyr Tyr Pro Leu Asn Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Thr Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ser Ser Ile Tyr Tyr Leu Thr Ile Thr Gln Gln
   355 360 365
   Met Thr Tyr Pro Val Phe Gln Ala Leu Gln Ser Asp Arg Arg Leu Arg
   370 375 380
   Ser Gly Gly Phe Val Ile Pro Ala Ala Thr Tyr Val Val Phe Leu Met
   385 390 395 400
   Thr Gly Met Thr Val Phe Ile Ile Ile Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Leu Ser Leu Val Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465 470 475 480
   Trp Leu Ile Pro Gln Leu Thr Leu Ala Gly Val Ala Glu Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Gly Gly Val Ser Ser
   515 520 525
   Tyr Leu Gly Ser Phe Leu Ile Ala Thr Val His Arg Thr Thr Gln Asn
   530 535 540
   Ser Ala Gly Gly Asn Trp Leu Ala Glu Asp Leu Asn Lys Gly Arg Leu
   545 550 555 560
   Asp Leu Phe Tyr Phe Met Ile Ala Gly Ile Leu Ala Val Asn Phe Ala
   565 570 575
   Tyr Phe Leu Val Met Ser Arg Trp Tyr Arg Tyr Lys Gly Ser Asp Asp
   580 585 590
   Glu Val Thr Ala Tyr Glu Thr Asn Gly Asp Val Ile Lys Gln Gln Asp
   595 600 605
   Lys Asn Lys Val
   610
<210> 29
   <211> 2303
   <212> DNA
   <213> Brassica napus
<220>
   <221> CDS
   <222> (1)..(157)
<220>
   <221> CDS
   <222> (336)..(553)
<220>
   <221> CDS
   <222> (646)..(1199)
<220>
   <221> CDS
   <222> (1394)..(2300)
<400> 29
   atg gag aga aag tct cta gaa gtc gag tct atg gat cac caa aaa ctt      48
   Met Glu Arg Lys Ser Leu Glu Val Glu Ser Met Asp His Gln Lys Leu
   1               5                   10                  15
   tcc tcc gcc gtg tac gat ggc tct gtt acg gcc gtt gat tct gtt aaa      96
   Ser Ser Ala Val Tyr Asp Gly Ser Val Thr Ala Val Asp Ser Val Lys
               20                  25                  30
   gaa gat gtt cag gag acc aaa gtc gtt tat aga ggc tgg aaa gtc atg     144
   Glu Asp Val Gln Glu Thr Lys Val Val Tyr Arg Gly Trp Lys Val Met
           35                  40                  45
   cct tac atc att g gtaacgcaat aatattatca tttaccgtgc tctataaact       197
   Pro Tyr Ile Ile
       50
   ttcttctttt taatgttaaa aaaaaacttt cttcttttgg aaactatttc ttttgtcgtt   257
   ttgttgtctt aagttttcac ttagtcataa tattgaagtc ttatgatctt ggttaatctt   317
   tgttgttgtt accaccag ga  aat gag aca ttt gag aag ctt ggg atc att     367
                       Gly Asn Glu Thr Phe Glu Lys Leu Gly Ile Ile
                               55                  60
   gga aca cta tca aac ctt ctg gtt tat tta act tct gtc ttc aac atg     415
   Gly Thr Leu Ser Asn Leu Leu Val Tyr Leu Thr Ser Val Phe Asn Met
       65                  70                  75
   aag agt gtc aca gct gca aca atc atc act gcc ttt gct ggc aca gtc     463
   Lys Ser Val Thr Ala Ala Thr Ile Ile Thr Ala Phe Ala Gly Thr Val
   80                  85                  90                  95
   aac ttc gga act ttc gtc gct gct ttc ctt tgc gac act tac ttc ggt     511
   Asn Phe Gly Thr Phe Val Ala Ala Phe Leu Cys Asp Thr Tyr Phe Gly
                   100                 105                 110
   cga tac aag act ctt acc gtt gcg gtc ctc gcc tgt ttt ctt             553
   Arg Tyr Lys Thr Leu Thr Val Ala Val Leu Ala Cys Phe Leu
               115                 120                 125
   gtaaagatcc atcccctcat aactctttct tcctctacag gcaatattgg tttataacct   613
   aaaaaaaaaa aaatctggtt cttgttttgt ag gga tca ctt gcg ata cta ttg       666
                                       Gly Ser Leu Ala Ile Leu Leu
                                                       130
   act gct gca gtc cca cag cta cat cca gcc cca tgc ggc aca gcg agc       714
   Thr Ala Ala Val Pro Gln Leu His Pro Ala Pro Cys Gly Thr Ala Ser
           135                 140                 145
   tcg tgt atc ggg cca agt ggt ggc cag ata gcg ttt cta ctg atg ggt       762
   Ser Cys Ile Gly Pro Ser Gly Gly Gln Ile Ala Phe Leu Leu Met Gly
       150                 155                 160
   ctt ggg att ctt gtc gtg gga gca gga ggg att aga ccg tgc aat cta       810
   Leu Gly Ile Leu Val Val Gly Ala Gly Gly Ile Arg Pro Cys Asn Leu
   165                 170                 175                 180
   gct ttc gga gct gat cag ttt aat ccg aag agc ggg tca ggg aag aga       858
   Ala Phe Gly Ala Asp Gln Phe Asn Pro Lys Ser Gly Ser Gly Lys Arg
                   185                 190                 195
   ggg att gat agt ttc ttc aac tgg tac ttc ttt acc ttc act ttc gcg       906
   Gly Ile Asp Ser Phe Phe Asn Trp Tyr Phe Phe Thr Phe Thr Phe Ala
               200                 205                 210
   cag atc ttg tct ctg aca gta gtt gtc tat gtc cag tct aat gtc agt       954
   Gln Ile Leu Ser Leu Thr Val Val Val Tyr Val Gln Ser Asn Val Ser
           215                 220                 225
   tgg acc atc ggt tta acc atc ccg gtt gtt cta atg ttc ttg gcc tcc      1002
   Trp Thr Ile Gly Leu Thr Ile Pro Val Val Leu Met Phe Leu Ala Ser
       230                 235                 240
   gtg att ttc ttt gcg ggt gct aag ttg tat gtt aag atc gaa gcc tca      1050
   Val Ile Phe Phe Ala Gly Ala Lys Leu Tyr Val Lys Ile Glu Ala Ser
   245                 250                 255                 260
   ggt agt cca ttg gct agc ata gct cat gtt ata acg gct gct atc aag      1098
   Gly Ser Pro Leu Ala Ser Ile Ala His Val Ile Thr Ala Ala Ile Lys
                   265                 270                 275
   aaa cac agg tta gag cca gtg gaa cag cct tgg ctg aac ctt tac aac      1146
   Lys His Arg Leu Glu Pro Val Glu Gln Pro Trp Leu Asn Leu Tyr Asn
               280                 285                 290
   tac tac cca tca aaa tac gca aac gcc aag ctc aaa tac acc caa caa      1194
   Tyr Tyr Pro Ser Lys Tyr Ala Asn Ala Lys Leu Lys Tyr Thr Gln Gln
           295                 300                 305
   ttc ag  gtaaacacac acttttaaac cggtaaaaaa acaccgttat atatatatat        1249
   Phe Arg
   atatattttt ttgagtgaat gaatgttaaa attattcata tcaaaacttt tttacaatga    1309
   ctgcatctat agttgatata tatatactta tacaatgtct tttttgatgc tttatgttta    1369
   ctgagagaga ttcttactcg gcag g ttt ctc gac aag gcg gcg atc ttg act     1421
                                Phe Leu Asp Lys Ala Ala Ile Leu Thr
                                                315
   cct gaa gac aag ttg gag gct gat ggt aag cct gtg aat ccg tgg aat      1469
   Pro Glu Asp Lys Leu Glu Ala Asp Gly Lys Pro Val Asn Pro Trp Asn
   320                 325                 330                 335
   cta tgt aca atg caa caa gtt gaa gaa gtg aag tgc atc atg aga gtg      1517
   Leu Cys Thr Met Gln Gln Val Glu Glu Val Lys Cys Ile Met Arg Val
                   340                 345                 350
   ctt cct ata tgg ttt gct tca tcg atc tac tac tta acc atg gcg caa      1565
   Leu Pro Ile Trp Phe Ala Ser Ser Ile Tyr Tyr Leu Thr Met Ala Gln
               355                 360                 365
   caa atg act tat cct gtc ttc caa gct ctc cag agt gat cgt cgc cta      1613
   Gln Met Thr Tyr Pro Val Phe Gln Ala Leu Gln Ser Asp Arg Arg Leu
           370                 375                 380
   ggc tcc gga ggt ttc atg atc ccg gca gcc acc tat gtt gtc ttc ttg      1661
   Gly Ser Gly Gly Phe Met Ile Pro Ala Ala Thr Tyr Val Val Phe Leu
       385                     390                     395
   atg act ggc atg acc ctc ttc atc ata ttt tat gac cgt gtc ttg gtt      1709
   Met Thr Gly Met Thr Leu Phe Ile Ile Phe Tyr Asp Arg Val Leu Val
   400                     405                     410                     415
   cct aca tta aaa aga ata acc ggt ata gag acg ggt ata acg ctc ttg      1757
   Pro Thr Leu Lys Arg Ile Thr Gly Ile Glu Thr Gly Ile Thr Leu Leu
                   420                     425                     430
   caa aga att gga gcc ggg att ttt ttc gcc ttt tta agc tta att gtc      1805
   Gln Arg Ile Gly Ala Gly Ile Phe Phe Ala Phe Leu Ser Leu Ile Val
               435                     440                     445
   tct ggt ttc gtc gag gaa cgt agg aga act tta gca cta act caa ccg      1853
   Ser Gly Phe Val Glu Glu Arg Arg Arg Thr Leu Ala Leu Thr Gln Pro
           450                     455                     460
   aca ctc ggt tta gcg cca cgg aag gga gaa atc tcc tca atg tcg gct      1901
   Thr Leu Gly Leu Ala Pro Arg Lys Gly Glu Ile Ser Ser Met Ser Ala
       465                     470                     475
   atg tgg ctg att ccg cag ctt gta ctt gct gga ata gcc gac gcg ttt      1949
   Met Trp Leu Ile Pro Gln Leu Val Leu Ala Gly Ile Ala Asp Ala Phe
   480                     485                     490                     495
   gga gct att gga cag atg gag ttt tac tac aag cag ttc cct gag aac      1997
   Gly Ala Ile Gly Gln Met Glu Phe Tyr Tyr Lys Gln Phe Pro Glu Asn
                   500                     505                     510
   atg agg agt ttc gct ggt tct atc ttt tac gta gga gga gga gtt tcg      2045
   Met Arg Ser Phe Ala Gly Ser Ile Phe Tyr Val Gly Gly Gly Val Ser
               515                     520                     525
   agt tac atc agt agc ttc ttg atc aca acg gtt cac agg atg acg cag      2093
   Ser Tyr Ile Ser Ser Phe Leu Ile Thr Thr Val His Arg Met Thr Gln
           530                     535                     540
   aac tcg gcg ggt ggt aat tgg tta gct gag gat ttg aac aaa ggg aga      2141
   Asn Ser Ala Gly Gly Asn Trp Leu Ala Glu Asp Leu Asn Lys Gly Arg
       545                     550                     555
   ttg gat tac ttc tat ttc ttg tta gct gga atc ttg gca gtt aat ttt      2189
   Leu Asp Tyr Phe Tyr Phe Leu Leu Ala Gly Ile Leu Ala Val Asn Phe
   560                     565                     570                     575
   gct tac ttc ttg gtg atg gca aga tgg tat agg tac aaa gga agt gat      2237
   Ala Tyr Phe Leu Val Met Ala Arg Trp Tyr Arg Tyr Lys Gly Ser Asp
                   580                     585                     590
   gat gaa gag aca agt tat gaa acc agt gga gat atc atc aaa gaa caa      2285
   Asp Glu Glu Thr Ser Tyr Glu Thr Ser Gly Asp Ile Ile Lys Glu Gln
               595                     600                     605
   gac aag aac aaa gtc tga                                              2303
   Asp Lys Asn Lys Val
           610
<210> 30
   <211> 612
   <212> PRT
   <213> Brassica napus
<400> 30
   Met Glu Arg Lys Ser Leu Glu Val Glu Ser Met Asp His Gln Lys Leu
   1               5                   10                  15
   Ser Ser Ala Val Tyr Asp Gly Ser Val Thr Ala Val Asp Ser Val Lys
               20                  25                  30
   Glu Asp Val Gln Glu Thr Lys Val Val Tyr Arg Gly Trp Lys Val Met
   35 40 45
   Pro Tyr Ile Ile Gly Asn Glu Thr Phe Glu Lys Leu Gly Ile Ile Gly
   50 55 60
   Thr Leu Ser Asn Leu Leu Val Tyr Leu Thr Ser Val Phe Asn Met Lys
   65 70 75 80
   Ser Val Thr Ala Ala Thr Ile Ile Thr Ala Phe Ala Gly Thr Val Asn
   85 90 95
   Phe Gly Thr Phe Val Ala Ala Phe Leu Cys Asp Thr Tyr Phe Gly Arg
   100 105 110
   Tyr Lys Thr Leu Thr Val Ala Val Leu Ala Cys Phe Leu Gly Ser Leu
   115 120 125
   Ala Ile Leu Leu Thr Ala Ala Val Pro Gln Leu His Pro Ala Pro Cys
   130 135 140
   Gly Thr Ala Ser Ser Cys Ile Gly Pro Ser Gly Gly Gln Ile Ala Phe
   145 150 155 160
   Leu Leu Met Gly Leu Gly Ile Leu Val Val Gly Ala Gly Gly Ile Arg
   165 170 175
   Pro Cys Asn Leu Ala Phe Gly Ala Asp Gln Phe Asn Pro Lys Ser Gly
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Val Val Val Tyr Val Gln
   210 215 220
   Ser Asn Val Ser Trp Thr Ile Gly Leu Thr Ile Pro Val Val Leu Met
   225 230 235 240
   Phe Leu Ala Ser Val Ile Phe Phe Ala Gly Ala Lys Leu Tyr Val Lys
   245 250 255
   Ile Glu Ala Ser Gly Ser Pro Leu Ala Ser Ile Ala His Val Ile Thr
   260 265 270
   Ala Ala Ile Lys Lys His Arg Leu Glu Pro Val Glu Gln Pro Trp Leu
   275 280 285
   Asn Leu Tyr Asn Tyr Tyr Pro Ser Lys Tyr Ala Asn Ala Lys Leu Lys
   290 295 300
   Tyr Thr Gln Gln Phe Arg Phe Leu Asp Lys Ala Ala Ile Leu Thr Pro
   305 310 315 320
   Glu Asp Lys Leu Glu Ala Asp Gly Lys Pro Val Asn Pro Trp Asn Leu
   325 330 335
   Cys Thr Met Gln Gln Val Glu Glu Val Lys Cys Ile Met Arg Val Leu
   340 345 350
   Pro Ile Trp Phe Ala Ser Ser Ile Tyr Tyr Leu Thr Met Ala Gln Gln
   355 360 365
   Met Thr Tyr Pro Val Phe Gln Ala Leu Gln Ser Asp Arg Arg Leu Gly
   370 375 380
   Ser Gly Gly Phe Met Ile Pro Ala Ala Thr Tyr Val Val Phe Leu Met
   385 390 395 400
   Thr Gly Met Thr Leu Phe Ile Ile Phe Tyr Asp Arg Val Leu Val Pro
   405 410 415
   Thr Leu Lys Arg Ile Thr Gly Ile Glu Thr Gly Ile Thr Leu Leu Gln
   420 425 430
   Arg Ile Gly Ala Gly Ile Phe Phe Ala Phe Leu Ser Leu Ile Val Ser
   435 440 445
   Gly Phe Val Glu Glu Arg Arg Arg Thr Leu Ala Leu Thr Gln Pro Thr
   450 455 460
   Leu Gly Leu Ala Pro Arg Lys Gly Glu Ile Ser Ser Met Ser Ala Met
   465 470 475 480
   Trp Leu Ile Pro Gln Leu Val Leu Ala Gly Ile Ala Asp Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Gly Gly Val Ser Ser
   515 520 525
   Tyr Ile Ser Ser Phe Leu Ile Thr Thr Val His Arg Met Thr Gln Asn
   530 535 540
   Ser Ala Gly Gly Asn Trp Leu Ala Glu Asp Leu Asn Lys Gly Arg Leu
   545 550 555 560
   Asp Tyr Phe Tyr Phe Leu Leu Ala Gly Ile Leu Ala Val Asn Phe Ala
   565 570 575
   Tyr Phe Leu Val Met Ala Arg Trp Tyr Arg Tyr Lys Gly Ser Asp Asp
   580 585 590
   Glu Glu Thr Ser Tyr Glu Thr Ser Gly Asp Ile Ile Lys Glu Gln Asp
   595 600 605
   Lys Asn Lys Val
   610
<210> 31
   <211> 2393
   <212> DNA
   <213> Brassica napus
<220>
   <221> CDS
   <222> (1)..(157)
<220>
   <221> CDS
   <222> (526)..(743)
<220>
   <221> CDS
   <222> (836)..(1389)
<220>
   <221> CDS
   <222> (1484)..(2390)
<400> 31
   atg gag aga aag cct cta gaa gtc gag tca acg gat cac caa aag cct          48
   Met Glu Arg Lys Pro Leu Glu Val Glu Ser Thr Asp His Gln Lys Pro
   1               5                   10                  15
   ccc tcc gcc gtg tac gat ggc tct gtt atg gcg gtt gat tct gtt gag          96
   Pro Ser Ala Val Tyr Asp Gly Ser Val Met Ala Val Asp Ser Val Glu
               20                  25                  30
   gaa gaa gtt ggg gag acc aaa gtc gtt tac aga ggc tgg aaa gtc atg         144
   Glu Glu Val Gly Glu Thr Lys Val Val Tyr Arg Gly Trp Lys Val Met
           35                  40                  45
   ccc ttt atc att g gtaacacaaa ctttttttta taaactttct tcttttgttc           197
   Pro Phe Ile Ile
       50
   caagaaaaaa aactattttt tattgctttt catgtttttt tacaaaataa ataatcaata       257
   gtcagaagaa atgaagagga aaacatcagt cttgctattg gtttgatttt ttcaaaaaaa       317
   aatcgtgaga ttctctgtac tgttgtcaaa agctttgact tagtcaacca gtttttcaag       377
   aaaattaaaa aaagagagtg agatctagaa tttgagaagg tttcagaaca taactgatca       437
   agaatcaact gtatgattca tgcaaagctt gaggcatatg agatcaaagt cttatgatca       497
   tcttggtttt gtttgttggt atcatcag ga  aat gag acg ttc gag aag ctt          548
                                  Gly Asn Glu Thr Phe Glu Lys Leu
                                              55                  60
   ggg atc att gga aca cta tca aac ctt ttg gtt tat tta act gcc gtc         596
   Gly Ile Ile Gly Thr Leu Ser Asn Leu Leu Val Tyr Leu Thr Ala Val
                   65                  70                  75
   ttc aac atg aag agt atc aca gct gca aca atc atc aat gca ttc agt         644
   Phe Asn Met Lys Ser Ile Thr Ala Ala Thr Ile Ile Asn Ala Phe Ser
               80                  85                  90
   ggc aca atc aac ttc gga acc ttc gtt gct gct ttc ctc tgc gac act         692
   Gly Thr Ile Asn Phe Gly Thr Phe Val Ala Ala Phe Leu Cys Asp Thr
           95                  100                 105
   tac ttc ggt cga tac aag act cta acc gtc gct gtc atc gcc tgt ttt         740
   Tyr Phe Gly Arg Tyr Lys Thr Leu Thr Val Ala Val Ile Ala Cys Phe
       110                 115                 120
   ctt gtaatgatca ttcctttgca atctttcttg ctccacaagc gttcttagtt              793
   Leu
   125
   taacaaccct aaaaatctga taattgctgt tcttttttgt ag ggg tca ctt gtg          847
                                                  Gly Ser Leu Val
   ata cta ttg aca gct gca gtg cca caa tta cat cca gct cca tgc gga         895
   Ile Leu Leu Thr Ala Ala Val Pro Gln Leu His Pro Ala Pro Cys Gly
   130                 135                 140                 145
   aca gct ctc tca tgt atc ggg cca agt ggc ggc cag ata gcg ttt cta        943
   Thr Ala Leu Ser Cys Ile Gly Pro Ser Gly Gly Gln Ile Ala Phe Leu
                   150                 155                 160
   ctg ttg ggt ctc ggg ttt ctt gta gtt gga gca ggc ggg att aga cca        991
   Leu Leu Gly Leu Gly Phe Leu Val Val Gly Ala Gly Gly Ile Arg Pro
               165                 170                 175
   tgt aat cta gct ttc ggg gct gat cag ttt aac ccc aag agc gag tca       1039
   Cys Asn Leu Ala Phe Gly Ala Asp Gln Phe Asn Pro Lys Ser Glu Ser
           180                 185                 190
   ggg aaa cga ggc atc gat agt ttc ttt aat tgg tac ttc ttc acc ttc       1087
   Gly Lys Arg Gly Ile Asp Ser Phe Phe Asn Trp Tyr Phe Phe Thr Phe
       195                 200                 205
   acg ttc gcg cag atc ttg tcg ctg aca ctg atc gtc tat atc cag tct       1135
   Thr Phe Ala Gln Ile Leu Ser Leu Thr Leu Ile Val Tyr Ile Gln Ser
   210                 215                 220                 225
   aat gtc agc tgg acc atc ggt tta acc atc ccg gtc gtt ctc atg ttc       1183
   Asn Val Ser Trp Thr Ile Gly Leu Thr Ile Pro Val Val Leu Met Phe
                   230                 235                 240
   ttg gct tgt gtg att ttc ttc gcg ggt gat aag ttg tat gtg aag atc       1231
   Leu Ala Cys Val Ile Phe Phe Ala Gly Asp Lys Leu Tyr Val Lys Ile
               245                 250                 255
   aag gcc tca ggt agt cca ttg gct ggt ata gct caa gtt ata gcg gtt       1279
   Lys Ala Ser Gly Ser Pro Leu Ala Gly Ile Ala Gln Val Ile Ala Val
           260                 265                 270
   gca atc aag aaa cgt ggg tta aag cca acg aaa gag cct tgg ctt aac       1327
   Ala Ile Lys Lys Arg Gly Leu Lys Pro Thr Lys Glu Pro Trp Leu Asn
       275                 280                 285
   ctt tac aat tac tac cca cca aaa tac gca aac tcc aaa ctc aaa tac       1375
   Leu Tyr Asn Tyr Tyr Pro Pro Lys Tyr Ala Asn Ser Lys Leu Lys Tyr
   290                 295                 300                 305
   acc gac cag ttt ag  gtaacaaaac acacacacgc caagagttag ttttatcttt       1429
   Thr Asp Gln Phe Arg
   aatatctttc cacttttgtt ttatggttac tgaggaagtt tcctgttttg gcag g ttt     1487
                                                                  Phe
   ctt gat aag gcg gcg att ttg gct ccc gaa gac aag ttg gag gct gat       1535
   Leu Asp Lys Ala Ala Ile Leu Ala Pro Glu Asp Lys Leu Glu Ala Asp
               315                 320                 325
   ggt aag cct gcg gat cca tgg aag ctt tgt aca atg caa caa gtt gaa       1583
   Gly Lys Pro Ala Asp Pro Trp Lys Leu Cys Thr Met Gln Gln Val Glu
           330                 335                 340
   gaa gtg aag tgc att gtg aga gtg ctt cct ata tgg ttt gct gca tcc       1631
   Glu Val Lys Cys Ile Val Arg Val Leu Pro Ile Trp Phe Ala Ala Ser
       345                 350                 355
   atc tat tac ttg acc atc acg caa caa atg act tac ccc gtc ttc caa       1679
   Ile Tyr Tyr Leu Thr Ile Thr Gln Gln Met Thr Tyr Pro Val Phe Gln
   360                 365                 370                 375
   gcc ctc cag agc gac cgt cgc tta gga tcc aga gga ttt gtg atc ccg       1727
   Ala Leu Gln Ser Asp Arg Arg Leu Gly Ser Arg Gly Phe Val Ile Pro
                   380                 385                 390
   gca gcc acc tac gtt gtc ttt ttg atg aca gga atg acg gtt ttc atc       1775
   Ala Ala Thr Tyr Val Val Phe Leu Met Thr Gly Met Thr Val Phe Ile
               395                 400                 405
   ata ttc tac gac cgt gtc ctc gtg cct acc tta aga aga ata acc ggt       1823
   Ile Phe Tyr Asp Arg Val Leu Val Pro Thr Leu Arg Arg Ile Thr Gly
           410                 415                 420
   ata gac acg ggg ata acg ctc ttg cag agg att gga acc ggt att ttc      1871
   Ile Asp Thr Gly Ile Thr Leu Leu Gln Arg Ile Gly Thr Gly Ile Phe
       425                 430                 435
   ttc gcc ttt gtt agc ttg ata gtc tcc ggt ttc gtc gag gaa cgg agg      1919
   Phe Ala Phe Val Ser Leu Ile Val Ser Gly Phe Val Glu Glu Arg Arg
   440                 445                 450                 455
   aga acg ttc gcg ctg act aaa cca aca ctt ggt atg gct cca agg aag      1967
   Arg Thr Phe Ala Leu Thr Lys Pro Thr Leu Gly Met Ala Pro Arg Lys
                   460                 465                 470
   gga gaa atc tcc tca atg tca gct atg tgg ctg atc cca caa ctc gcc      2015
   Gly Glu Ile Ser Ser Met Ser Ala Met Trp Leu Ile Pro Gln Leu Ala
               475                 480                 485
   ctt gcg ggt ata gcc gag gcg ttt gga gct att gga cag atg gag ttt      2063
   Leu Ala Gly Ile Ala Glu Ala Phe Gly Ala Ile Gly Gln Met Glu Phe
           490                 495                 500
   tac tac aag caa ttc cct gag aac atg agg agt ttt gct ggt tcg atc      2111
   Tyr Tyr Lys Gln Phe Pro Glu Asn Met Arg Ser Phe Ala Gly Ser Ile
       505                 510                 515
   ttc tat gta gga gca gga gtt tca agc tac ctc ggt agc ttc ttg att      2159
   Phe Tyr Val Gly Ala Gly Val Ser Ser Tyr Leu Gly Ser Phe Leu Ile
   520                 525                 530                 535
   gca atg gtt cac agg acg acg cag aac tcg gct ggt ggt aat tgg tta      2207
   Ala Met Val His Arg Thr Thr Gln Asn Ser Ala Gly Gly Asn Trp Leu
                   540                 545                 550
   gct gag gat ttg aac aaa gga aga ttg gat tac ttc tat ttc atg atc      2255
   Ala Glu Asp Leu Asn Lys Gly Arg Leu Asp Tyr Phe Tyr Phe Met Ile
               555                 560                 565
   gct gga atc ttg gcg gtt aat ttt gct tac ttc ttg gtc atg tca aga      2303
   Ala Gly Ile Leu Ala Val Asn Phe Ala Tyr Phe Leu Val Met Ser Arg
           570                 575                 580
   tgg tat aga tac aaa gga agt gat gat gaa gtg aca act tat gaa acc      2351
   Trp Tyr Arg Tyr Lys Gly Ser Asp Asp Glu Val Thr Thr Tyr Glu Thr
       585                 590                 595
   aat gga gat gct atc aaa caa caa gac aag aat act gtc tga              2393
   Asn Gly Asp Ala Ile Lys Gln Gln Asp Lys Asn Thr Val
   600                 605                 610
<210> 32
   <211> 612
   <212> PRT
   <213> Brassica napus
<400> 32
   Met Glu Arg Lys Pro Leu Glu Val Glu Ser Thr Asp His Gln Lys Pro
   1               5                   10                  15
   Pro Ser Ala Val Tyr Asp Gly Ser Val Met Ala Val Asp Ser Val Glu
               20                  25                  30
   Glu Glu Val Gly Glu Thr Lys Val Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
   85 90 95
   Phe Gly Thr Phe Val Ala Ala Phe Leu Cys Asp Thr Tyr Phe Gly Arg
   100 105 110
   Tyr Lys Thr Leu Thr Val Ala Val Ile Ala Cys Phe Leu Gly Ser Leu
   115 120 125
   Val Ile Leu Leu Thr Ala Ala Val Pro Gln Leu His Pro Ala Pro Cys
   130 135 140
   Gly Thr Ala Leu Ser Cys Ile Gly Pro Ser Gly Gly Gln Ile Ala Phe
   145 150 155 160
   Leu Leu Leu Gly Leu Gly Phe Leu Val Val Gly Ala Gly Gly Ile Arg
   165 170 175
   Pro Cys Asn Leu Ala Phe Gly Ala Asp Gln Phe Asn Pro Lys Ser Glu
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Leu Ile Val Tyr Ile Gln
   210 215 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Gly Ile Ala Gln Val Ile Ala
   260 265 270
   Val Ala Ile Lys Lys Arg Gly Leu Lys Pro Thr Lys Glu Pro Trp Leu
   275 280 285
   Asn Leu Tyr Asn Tyr Tyr Pro Pro Lys Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Ala Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ala Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Arg Gly Phe Val Ile Pro Ala Ala Thr Tyr Val Val Phe Leu Met
   385                 390                 395                 400
   Thr Gly Met Thr Val Phe Ile Ile Phe Tyr Asp Arg Val Leu Val Pro
                   405                 410                 415
   Thr Leu Arg Arg Ile Thr Gly Ile Asp Thr Gly Ile Thr Leu Leu Gln
               420                 425                 430
   Arg Ile Gly Thr Gly Ile Phe Phe Ala Phe Val Ser Leu Ile Val Ser
           435                 440                 445
   Gly Phe Val Glu Glu Arg Arg Arg Thr Phe Ala Leu Thr Lys Pro Thr
       450                 455                 460
   Leu Gly Met Ala Pro Arg Lys Gly Glu Ile Ser Ser Met Ser Ala Met
   465                 470                 475                 480
   Trp Leu Ile Pro Gln Leu Ala Leu Ala Gly Ile Ala Glu Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Ala Gly Val Ser Ser
           515                 520                 525
   Tyr Leu Gly Ser Phe Leu Ile Ala Met Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Tyr Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Glu Val Thr Thr Tyr Glu Thr Asn Gly Asp Ala Ile Lys Gln Gln Asp
           595                 600                 605
   Lys Asn Thr Val
       610
<210> 33
   <211> 1833
   <212> DNA
   <213> Brassica napus
<220>
   <221> CDS
   <222> (1)..(204)
<220>
   <221> CDS
   <222> (297)..(850)
<220>
   <221> CDS
   <222> (924)..(1830)
<400> 33
   gag aag ctt ggg atc att gga aca cta tca aac ctt ctg gtt tac tta      48
   Glu Lys Leu Gly Ile Ile Gly Thr Leu Ser Asn Leu Leu Val Tyr Leu
   1               5                   10                  15
   act gct gtc ttc aac atg aag agt atc aca gcc gca aca atc atc aac      96
   Thr Ala Val Phe Asn Met Lys Ser Ile Thr Ala Ala Thr Ile Ile Asn
               20                  25                  30
   gcc ttt agc ggc aca atc aac ttc gga acc ttc gtt gct gcc ttc ctt     144
   Ala Phe Ser Gly Thr Ile Asn Phe Gly Thr Phe Val Ala Ala Phe Leu
           35                  40                  45
   tgc gac act tac ttc ggc cgt tac aag aca cta acc gtc gcg gtc atc     192
   Cys Asp Thr Tyr Phe Gly Arg Tyr Lys Thr Leu Thr Val Ala Val Ile
       50                  55                  60
   gca tgt ttt ctt gtaataatcc atcctctata accttttctt cctccacaag         244
   Ala Cys Phe Leu
   65
   caatcttggt ttataaacct aaaaaaaaaa aatctgattc ttgttttggt ag gga tca   302
                                                             Gly Ser
                                                                 70
   ctt gtg ata cta tta act gct gca gta cca caa ctg cat cca gct cca     350
   Leu Val Ile Leu Leu Thr Ala Ala Val Pro Gln Leu His Pro Ala Pro
                   75                  80                  85
   tgc gga aca gcc ctc acg tgt aca ggc cca agt ggt ggc cag ata gcc     398
   Cys Gly Thr Ala Leu Thr Cys Thr Gly Pro Ser Gly Gly Gln Ile Ala
               90                  95                  100
   ttt cta cta atg ggt ctt gcc ttt ctt gtg gtg gga gca ggt ggg att     446
   Phe Leu Leu Met Gly Leu Ala Phe Leu Val Val Gly Ala Gly Gly Ile
           105                 110                 115
   aga ccg tgt aat ctt gct ttt gga gct gat cag ttt aac cct aaa agc     494
   Arg Pro Cys Asn Leu Ala Phe Gly Ala Asp Gln Phe Asn Pro Lys Ser
       120                 125                 130
   gag tca ggg aaa aga ggg att gat agt ttc ttt aac tgg tac ttc ttc     542
   Glu Ser Gly Lys Arg Gly Ile Asp Ser Phe Phe Asn Trp Tyr Phe Phe
   135                 140                 145                 150
   acc ttc acg ttc gcg cag atc ttg tct ctg aca cta gtt gtc tac atc     590
   Thr Phe Thr Phe Ala Gln Ile Leu Ser Leu Thr Leu Val Val Tyr Ile
                   155                 160                 165
   cag tct aac gtc agt tgg acc atc ggt tta acc atc ccg gtc gtg tta     638
   Gln Ser Asn Val Ser Trp Thr Ile Gly Leu Thr Ile Pro Val Val Leu
               170                 175                 180
   atg ttc ttg gcc tgc gtg atc ttc ttt gcg ggt gat aag ttg tat gtc     686
   Met Phe Leu Ala Cys Val Ile Phe Phe Ala Gly Asp Lys Leu Tyr Val
           185                 190                 195
   aag atc aaa gcc tca ggt agt cca ttg gct agt ata gct caa gtt ata     734
   Lys Ile Lys Ala Ser Gly Ser Pro Leu Ala Ser Ile Ala Gln Val Ile
       200                 205                 210
   acg gtt gct atc aag aaa cgt ggg tta aag gct gtg aag cag cct tgg     782
   Thr Val Ala Ile Lys Lys Arg Gly Leu Lys Ala Val Lys Gln Pro Trp
   215                 220                 225                 230
   gtt aac ctt tac aat tac tac cca cta aac tac gca aac tcc aag ctc     830
   Val Asn Leu Tyr Asn Tyr Tyr Pro Leu Asn Tyr Ala Asn Ser Lys Leu
                   235                 240                 245
   aag tac act gac cag ttc ag  gtaaaagaca cagttataaa taatatcttt        880
   Lys Tyr Thr Asp Gln Phe Arg
               250
   ccacttttgt ttacttagaa agcaagcttt ctatttttgg cag g ttt ctt gat aag       936
                                                     Phe Leu Asp Lys
                                                         255
   gcg gcg ata ttg gct cct gaa gac aag ttg gaa gct gat ggc aag cct         984
   Ala Ala Ile Leu Ala Pro Glu Asp Lys Leu Glu Ala Asp Gly Lys Pro
           260                 265                 270
   acg gat cca tgg aag cta tgt aca atg caa caa gtt gaa gaa gtg aag        1032
   Thr Asp Pro Trp Lys Leu Cys Thr Met Gln Gln Val Glu Glu Val Lys
       275                 280                 285
   tgc att gtg aga gtg ctt cct ata tgg ttt gct tca tcg atc tat tac        1080
   Cys Ile Val Arg Val Leu Pro Ile Trp Phe Ala Ser Ser Ile Tyr Tyr
   290                 295                 300                 305
   ttg acc atc acg caa caa atg act tat cca gtc ttc caa gcc ctc cag        1128
   Leu Thr Ile Thr Gln Gln Met Thr Tyr Pro Val Phe Gln Ala Leu Gln
                   310                 315                 320
   agc gat cgt cgc tta tgc tct gga ggg ttt gtg att cct gca gcc acc        1176
   Ser Asp Arg Arg Leu Cys Ser Gly Gly Phe Val Ile Pro Ala Ala Thr
               325                 330                 335
   tat gtg gtc ttc ttg atg acg gga atg act gtt ttc atc ata atc tac        1224
   Tyr Val Val Phe Leu Met Thr Gly Met Thr Val Phe Ile Ile Ile Tyr
           340                 345                 350
   gac cgc gtc ctc gtg cct acc ttg agg aga atc acc ggt ata gac acc        1272
   Asp Arg Val Leu Val Pro Thr Leu Arg Arg Ile Thr Gly Ile Asp Thr
       355                 360                 365
   ggg atc acg ctc ttg caa agg ata gga act ggg att ttc ttc gcc ttt        1320
   Gly Ile Thr Leu Leu Gln Arg Ile Gly Thr Gly Ile Phe Phe Ala Phe
   370                 375                 380                 385
   ttg agc ttg gta gtg tcc ggt ttc gtc gag gaa cgg agg agg acc ttc        1368
   Leu Ser Leu Val Val Ser Gly Phe Val Glu Glu Arg Arg Arg Thr Phe
                   390                 395                 400
   gca ttg act aaa cca aca ctc ggt atg gcg cca cgg aag gga gaa atc        1416
   Ala Leu Thr Lys Pro Thr Leu Gly Met Ala Pro Arg Lys Gly Glu Ile
               405                 410                 415
   tcc tca atg tca gct atg tgg ctg ata ccg cag ctc aca ctc gcg ggt        1464
   Ser Ser Met Ser Ala Met Trp Leu Ile Pro Gln Leu Thr Leu Ala Gly
           420                 425                 430
   gta gcc gag gcg ttt gga gcc att ggg cag atg gag ttt tac tac aag        1512
   Val Ala Glu Ala Phe Gly Ala Ile Gly Gln Met Glu Phe Tyr Tyr Lys
       435                 440                 445
   cag ttc ccc gag aac atg agg agt ttc gcc ggt tcc atc ttc tat gtg        1560
   Gln Phe Pro Glu Asn Met Arg Ser Phe Ala Gly Ser Ile Phe Tyr Val
   450                 455                 460                 465
   gga gga gga gtt tcg agt tac ctc ggt agc ttc ttg att gca acg gtt        1608
   Gly Gly Gly Val Ser Ser Tyr Leu Gly Ser Phe Leu Ile Ala Thr Val
                   470                 475                 480
   cac agg acg acg cag aac tcg gct ggc ggt aac tgg tta gct gaa gat        1656
   His Arg Thr Thr Gln Asn Ser Ala Gly Gly Asn Trp Leu Ala Glu Asp
               485                 490                 495
   ttg aac aaa gga aga ttg gat ttg ttt tac ttc atg atc gct gga atc        1704
   Leu Asn Lys Gly Arg Leu Asp Leu Phe Tyr Phe Met Ile Ala Gly Ile
           500                 505                 510
   ttg gcg gtt aat ttt gct tac ttc ttg gtg atg tca aga tgg tat agg        1752
   Leu Ala Val Asn Phe Ala Tyr Phe Leu Val Met Ser Arg Trp Tyr Arg
       515                 520                 525
   tac aaa gga agt gat gat gaa gtg aca gct tat gaa aca aat gga gat        1800
   Tyr Lys Gly Ser Asp Asp Glu Val Thr Ala Tyr Glu Thr Asn Gly Asp
   530                 535                 540                 545
   gtg atc aaa caa caa gac aag aac aaa gtc taa                    1833
   Val Ile Lys Gln Gln Asp Lys Asn Lys Val
                   550                 555
<210> 34
   <211> 555
   <212> PRT
   <213> Brassica napus
<400> 34
   Glu Lys Leu Gly Ile Ile Gly Thr Leu Ser Asn Leu Leu Val Tyr Leu
   1               5                   10                  15
   Thr Ala Val Phe Asn Met Lys Ser Ile Thr Ala Ala Thr Ile Ile Asn
               20                  25                  30
   Ala Phe Ser Gly Thr Ile Asn Phe Gly Thr Phe Val Ala Ala Phe Leu
           35                  40                  45
   Cys Asp Thr Tyr Phe Gly Arg Tyr Lys Thr Leu Thr Val Ala Val Ile
       50                  55                  60
   Ala Cys Phe Leu Gly Ser Leu Val Ile Leu Leu Thr Ala Ala Val Pro
   65                  70                  75                  80
   Gln Leu His Pro Ala Pro Cys Gly Thr Ala Leu Thr Cys Thr Gly Pro
                   85                  90                  95
   Ser Gly Gly Gln Ile Ala Phe Leu Leu Met Gly Leu Ala Phe Leu Val
               100                 105                 110
   Val Gly Ala Gly Gly Ile Arg Pro Cys Asn Leu Ala Phe Gly Ala Asp
           115                 120                 125
   Gln Phe Asn Pro Lys Ser Glu Ser Gly Lys Arg Gly Ile Asp Ser Phe
       130                 135                 140
   Phe Asn Trp Tyr Phe Phe Thr Phe Thr Phe Ala Gln Ile Leu Ser Leu
   145                 150                 155                 160
   Thr Leu Val Val Tyr Ile Gln Ser Asn Val Ser Trp Thr Ile Gly Leu
                   165                 170                 175
   Thr Ile Pro Val Val Leu Met Phe Leu Ala Cys Val Ile Phe Phe Ala
               180                 185                 190
   Gly Asp Lys Leu Tyr Val Lys Ile Lys Ala Ser Gly Ser Pro Leu Ala
           195                 200                 205
   Ser Ile Ala Gln Val Ile Thr Val Ala Ile Lys Lys Arg Gly Leu Lys
       210                 215                 220
   Ala Val Lys Gln Pro Trp Val Asn Leu Tyr Asn Tyr Tyr Pro Leu Asn
   225                 230                 235                 240
   Tyr Ala Asn Ser Lys Leu Lys Tyr Thr Asp Gln Phe Arg Phe Leu Asp
                   245                 250                 255
   Lys Ala Ala Ile Leu Ala Pro Glu Asp Lys Leu Glu Ala Asp Gly Lys
   260 265 270
   Pro Thr Asp Pro Trp Lys Leu Cys Thr Met Gln Gln Val Glu Glu Val
   275 280 285
   Lys Cys Ile Val Arg Val Leu Pro Ile Trp Phe Ala Ser Ser Ile Tyr
   290 295 300
   Tyr Leu Thr Ile Thr Gln Gln Met Thr Tyr Pro Val Phe Gln Ala Leu
   305 310 315 320
   Gln Ser Asp Arg Arg Leu Cys Ser Gly Gly Phe Val Ile Pro Ala Ala
   325 330 335
   Thr Tyr Val Val Phe Leu Met Thr Gly Met Thr Val Phe Ile Ile Ile
   340 345 350
   Tyr Asp Arg Val Leu Val Pro Thr Leu Arg Arg Ile Thr Gly Ile Asp
   355 360 365
   Thr Gly Ile Thr Leu Leu Gln Arg Ile Gly Thr Gly Ile Phe Phe Ala
   370 375 380
   Phe Leu Ser Leu Val Val Ser Gly Phe Val Glu Glu Arg Arg Arg Thr
   385 390 395 400
   Phe Ala Leu Thr Lys Pro Thr Leu Gly Met Ala Pro Arg Lys Gly Glu
   405 410 415
   Ile Ser Ser Met Ser Ala Met Trp Leu Ile Pro Gln Leu Thr Leu Ala
   420 425 430
   Gly Val Ala Glu Ala Phe Gly Ala Ile Gly Gln Met Glu Phe Tyr Tyr
   435 440 445
   Lys Gln Phe Pro Glu Asn Met Arg Ser Phe Ala Gly Ser Ile Phe Tyr
   450 455 460
   Val Gly Gly Gly Val Ser Ser Tyr Leu Gly Ser Phe Leu Ile Ala Thr
   465 470 475 480
   Val His Arg Thr Thr Gln Asn Ser Ala Gly Gly Asn Trp Leu Ala Glu
   485 490 495
   Asp Leu Asn Lys Gly Arg Leu Asp Leu Phe Tyr Phe Met Ile Ala Gly
   500 505 510
   Ile Leu Ala Val Asn Phe Ala Tyr Phe Leu Val Met Ser Arg Trp Tyr
   515 520 525
   Arg Tyr Lys Gly Ser Asp Asp Glu Val Thr Ala Tyr Glu Thr Asn Gly
   530 535 540
   Asp Val Ile Lys Gln Gln Asp Lys Asn Lys Val
   545 550 555
<210> 35
   <211> 2367
   <212> DNA
   <213> Brassica napus
<220>
   <221> CDS
   <222> (1)..(154)
<220>
   <221> CDS
   <222> (332)..(549)
<220>
   <221> CDS
   <222> (638)..(1191)
<220>
   <221> CDS
   <222> (1458)..(2364)
<400> 35
   atg gag aga aag tct ctc gaa gtc gag tct atg gat cac caa aaa ctt       48
   Met Glu Arg Lys Ser Leu Glu Val Glu Ser Met Asp His Gln Lys Leu
   1               5                   10                  15
   tcc tcc gcc tac gat ggc tct gtt acg gcc gtt gat tct gtt aaa gaa       96
   Ser Ser Ala Tyr Asp Gly Ser Val Thr Ala Val Asp Ser Val Lys Glu
               20                  25                  30
   gat gtt cag gag acc aaa gtc gtt tat aga ggc tgg aaa gtc atg cct      144
   Asp Val Gln Glu Thr Lys Val Val Tyr Arg Gly Trp Lys Val Met Pro
           35                  40                  45
   tac atc att g gtaacgcaat aatattatca tttaccgtgc tctgtaaact            194
   Tyr Ile Ile
       50
   ttcttctttt taatgttaaa aaaaaaactt tcttcttttg gaaactattt cttttgtcgt    254
   tttgttgtct taagttttca cttggtcatt attgaagtct tatgatcttg gttaattttt    314
   gttgttgtta ccaccag ga  aat gag act ttt gag aag ctt ggg atc att       363
                     Gly Asn Glu Thr Phe Glu Lys Leu Gly Ile Ile
                                 55                  60
   gga aca cta tca aac ctt ctg gtg tat tta act tct gtc ttc aac atg      411
   Gly Thr Leu Ser Asn Leu Leu Val Tyr Leu Thr Ser Val Phe Asn Met
           65                  70                  75
   aag agt gtc aca gct gca aca atc atc aat gcc ttt gct ggc aca gtc      459
   Lys Ser Val Thr Ala Ala Thr Ile Ile Asn Ala Phe Ala Gly Thr Val
       80                  85                  90
   aac ttc gga act ttc gtc gct gct ttc ctt tgc gac act tac ttc ggt      507
   Asn Phe Gly Thr Phe Val Ala Ala Phe Leu Cys Asp Thr Tyr Phe Gly
   95                  100                 105                 110
   cga tac aag act ctt acc gtc gcg gtc ctc gcc tgt ttt ctt              549
   Arg Tyr Lys Thr Leu Thr Val Ala Val Leu Ala Cys Phe Leu
                   115                 120
   gtaaagatcc accccgtcta attatttctt cctctacaag caatcttggt ttataaccta    609
   aaaataaaat ctggttcttg ttttgtag gga tca ctt gcg ata cta ttg act       661
                                  Gly Ser Leu Ala Ile Leu Leu Thr
                                  125                 130
   gct gca gtc cca caa cta cat cca gcc cca tgc ggc aca gcg agc tcg      709
   Ala Ala Val Pro Gln Leu His Pro Ala Pro Cys Gly Thr Ala Ser Ser
           135                 140                 145
   tgt agc ggg cca agt ggt ggc cag ata gcg ttt cta ctg atg ggt ctt      757
   Cys Ser Gly Pro Ser Gly Gly Gln Ile Ala Phe Leu Leu Met Gly Leu
       150                 155                 160
   ggg att ctt gtg gtg gga gca gga ggg att aga ccg tgc aat cta gct      805
   Gly Ile Leu Val Val Gly Ala Gly Gly Ile Arg Pro Cys Asn Leu Ala
   165                 170                 175                 180
   ttc gga gct gat cag ttt aat ccg aag agc ggg tca ggg aaa aga ggg      853
   Phe Gly Ala Asp Gln Phe Asn Pro Lys Ser Gly Ser Gly Lys Arg Gly
                   185                 190                 195
   att gat agt ttc ttc aac tgg tac ttc ttc acc ttc act ttc gcg cag      901
   Ile Asp Ser Phe Phe Asn Trp Tyr Phe Phe Thr Phe Thr Phe Ala Gln
               200                 205                 210
   atc ttg tct ctg aca gta gtt gtc tat gtc cag tct aat gtc agt tgg      949
   Ile Leu Ser Leu Thr Val Val Val Tyr Val Gln Ser Asn Val Ser Trp
           215                 220                 225
   acc atc ggt tta acc atc ccg gtt gtt tta atg ttc ttg gcc tcc gtg      997
   Thr Ile Gly Leu Thr Ile Pro Val Val Leu Met Phe Leu Ala Ser Val
       230                 235                 240
   att ttc ttt gcg ggt gct aag ttg tat gtt aag atc gaa gcc tca ggt      1045
   Ile Phe Phe Ala Gly Ala Lys Leu Tyr Val Lys Ile Glu Ala Ser Gly
   245                 250                 255                 260
   agt cca ttg gct agt ata gct cat gtt ata acg gct gct atc aag aaa      1093
   Ser Pro Leu Ala Ser Ile Ala His Val Ile Thr Ala Ala Ile Lys Lys
                   265                 270                 275
   cac ggg tta aag tca gtg gaa cag cct tgg ctg aac ctt tac aac tac      1141
   His Gly Leu Lys Ser Val Glu Gln Pro Trp Leu Asn Leu Tyr Asn Tyr
               280                 285                 290
   tac cca tca aaa tac gca aac gcc aag ctc aaa tac acc gaa caa ttc      1189
   Tyr Pro Ser Lys Tyr Ala Asn Ala Lys Leu Lys Tyr Thr Glu Gln Phe
           295                 300                 305
   ag  gtaaacacac ttttaaaccg gtaaaaacca ccgtttatac ttgatatata          1241
   Arg
   tatatatatt tatttttttt tttgaatgaa tgttaaaatt attcatatca aaacattttt   1301
   acaatgactg catctatagt tgatatatat ctatatataa tttgactcaa taaagaactc   1361
   aaaaacattc ggtaaaaaca ttgtcacacc taaaactagt cttggtttgt cttttctgat   1421
   gctttatttt tactgagaga gattcatact cggcag g ttt ctc gac aag gcc gcg   1476
                                             Phe Leu Asp Lys Ala Ala
                                             310                 315
   aac ttg act cct gaa gac aag ttg gat gct gat ggt aag cct gtg aat      1524
   Asn Leu Thr Pro Glu Asp Lys Leu Asp Ala Asp Gly Lys Pro Val Asn
                   320                 325                 330
   ccg tgg aat cta tgt aca atg caa cag gtt gaa gaa gtg aag tgc atc      1572
   Pro Trp Asn Leu Cys Thr Met Gln Gln Val Glu Glu Val Lys Cys Ile
               335                 340                 345
   atg aga gtg ctt cct ata tgg ttt gct tca tcg atc tac tac tta acc      1620
   Met Arg Val Leu Pro Ile Trp Phe Ala Ser Ser Ile Tyr Tyr Leu Thr
           350                 355                 360
   atc gcg caa caa atg act tat cct gtc ttc caa gct ctc cag agt gat      1668
   Ile Ala Gln Gln Met Thr Tyr Pro Val Phe Gln Ala Leu Gln Ser Asp
       365                 370                 375
   cgt cgc tta ggc tct gga ggt ttc atg atc ccg gca gcc acc tat gtt      1716
   Arg Arg Leu Gly Ser Gly Gly Phe Met Ile Pro Ala Ala Thr Tyr Val
   380                 385                 390                 395
   gtc ttc ttg atg act ggc atg acc ctc ttc atc ata ttt tat gac cgt      1764
   Val Phe Leu Met Thr Gly Met Thr Leu Phe Ile Ile Phe Tyr Asp Arg
                   400                 405                 410
   gta ttc gtt cct aca tta aaa aga ata acc ggt ata gag acg ggt ata      1812
   Val Phe Val Pro Thr Leu Lys Arg Ile Thr Gly Ile Glu Thr Gly Ile
               415                 420                 425
   acg ctc ttg caa aga att gga gcc ggg att ttc ttc gcc ttg tta agc      1860
   Thr Leu Leu Gln Arg Ile Gly Ala Gly Ile Phe Phe Ala Leu Leu Ser
           430                 435                 440
   tta att gtc tct ggt ttc gtc gag gaa cgt agg aga acc tta gca cta      1908
   Leu Ile Val Ser Gly Phe Val Glu Glu Arg Arg Arg Thr Leu Ala Leu
       445                 450                 455
   act aaa ccg aca ctc ggt tta gcg cca cgg aag gga gaa atc tcc tca      1956
   Thr Lys Pro Thr Leu Gly Leu Ala Pro Arg Lys Gly Glu Ile Ser Ser
   460                 465                 470                 475
   atg tcg gct atg tgg ctg att ccg cag ctt gta ctt gct ggt ata gcc      2004
   Met Ser Ala Met Trp Leu Ile Pro Gln Leu Val Leu Ala Gly Ile Ala
                   480                 485                 490
   gac gca ttt gga gct att gga cag atg gag ttt tac tac aag cag ttc      2052
   Asp Ala Phe Gly Ala Ile Gly Gln Met Glu Phe Tyr Tyr Lys Gln Phe
               495                 500                 505
   cct gag aac atg agg agt ttc gct ggt tct atc ttt tac gta gga gca      2100
   Pro Glu Asn Met Arg Ser Phe Ala Gly Ser Ile Phe Tyr Val Gly Ala
           510                 515                 520
   gga gtt tcg agt tac atc agt agc ttc ttg atc aca acg gtt cac agg      2148
   Gly Val Ser Ser Tyr Ile Ser Ser Phe Leu Ile Thr Thr Val His Arg
       525                 530                 535
   atg acg cag aac tcg gcg ggt ggt aat tgg tta gct gag gat ttg aac      2196
   Met Thr Gln Asn Ser Ala Gly Gly Asn Trp Leu Ala Glu Asp Leu Asn
   540                 545                 550                 555
   aaa gga aga ttg gat tac ttc tat ttc ttg tta gct gga atc ttg gca      2244
   Lys Gly Arg Leu Asp Tyr Phe Tyr Phe Leu Leu Ala Gly Ile Leu Ala
                   560                 565                 570
   gtt aat ttt gct tac ttc ttg gtg atg gca aga tgg tat aag tac aaa      2292
   Val Asn Phe Ala Tyr Phe Leu Val Met Ala Arg Trp Tyr Lys Tyr Lys
               575                 580                 585
   gga agt gat gat gaa gag aca agt tat gaa acc agt gga gat atc atc      2340
   Gly Ser Asp Asp Glu Glu Thr Ser Tyr Glu Thr Ser Gly Asp Ile Ile
           590                 595                 600
   aaa gaa caa gac aag aac aaa gtc tga                                  2367
   Lys Glu Gln Asp Lys Asn Lys Val
       605                 610
<210> 36
   <211> 611
   <212> PRT
   <213> Brassica napus
<400> 36
   Met Glu Arg Lys Ser Leu Glu Val Glu Ser Met Asp His Gln Lys Leu
   1               5                   10                  15
   Ser Ser Ala Tyr Asp Gly Ser Val Thr Ala Val Asp Ser Val Lys Glu
               20                  25                  30
   Asp Val Gln Glu Thr Lys Val Val Tyr Arg Gly Trp Lys Val Met Pro
           35                  40                  45
   Tyr Ile Ile Gly Asn Glu Thr Phe Glu Lys Leu Gly Ile Ile Gly Thr
       50                  55                  60
   Leu Ser Asn Leu Leu Val Tyr Leu Thr Ser Val Phe Asn Met Lys Ser
   65                  70                  75                  80
   Val Thr Ala Ala Thr Ile Ile Asn Ala Phe Ala Gly Thr Val Asn Phe
                   85                  90                  95
   Gly Thr Phe Val Ala Ala Phe Leu Cys Asp Thr Tyr Phe Gly Arg Tyr
               100                 105                 110
   Lys Thr Leu Thr Val Ala Val Leu Ala Cys Phe Leu Gly Ser Leu Ala
           115                 120                 125
   Ile Leu Leu Thr Ala Ala Val Pro Gln Leu His Pro Ala Pro Cys Gly
       130                 135                 140
   Thr Ala Ser Ser Cys Ser Gly Pro Ser Gly Gly Gln Ile Ala Phe Leu
   145                 150                 155                 160
   Leu Met Gly Leu Gly Ile Leu Val Val Gly Ala Gly Gly Ile Arg Pro
                   165                 170                 175
   Cys Asn Leu Ala Phe Gly Ala Asp Gln Phe Asn Pro Lys Ser Gly Ser
               180                 185                 190
   Gly Lys Arg Gly Ile Asp Ser Phe Phe Asn Trp Tyr Phe Phe Thr Phe
           195                 200                 205
   Thr Phe Ala Gln Ile Leu Ser Leu Thr Val Val Val Tyr Val Gln Ser
       210                 215                 220
   Asn Val Ser Trp Thr Ile Gly Leu Thr Ile Pro Val Val Leu Met Phe
   225                 230                 235                 240
   Leu Ala Ser Val Ile Phe Phe Ala Gly Ala Lys Leu Tyr Val Lys Ile
                   245                 250                 255
   Glu Ala Ser Gly Ser Pro Leu Ala Ser Ile Ala His Val Ile Thr Ala
               260                 265                 270
   Ala Ile Lys Lys His Gly Leu Lys Ser Val Glu Gln Pro Trp Leu Asn
           275                 280                 285
   Leu Tyr Asn Tyr Tyr Pro Ser Lys Tyr Ala Asn Ala Lys Leu Lys Tyr
       290                 295                 300
   Thr Glu Gln Phe Arg Phe Leu Asp Lys Ala Ala Asn Leu Thr Pro Glu
   305                 310                 315                 320
   Asp Lys Leu Asp Ala Asp Gly Lys Pro Val Asn Pro Trp Asn Leu Cys
                   325                 330                 335
   Thr Met Gln Gln Val Glu Glu Val Lys Cys Ile Met Arg Val Leu Pro
               340                 345                 350
   Ile Trp Phe Ala Ser Ser Ile Tyr Tyr Leu Thr Ile Ala Gln Gln Met
           355                 360                 365
   Thr Tyr Pro Val Phe Gln Ala Leu Gln Ser Asp Arg Arg Leu Gly Ser
   370 375 380
   Gly Gly Phe Met Ile Pro Ala Ala Thr Tyr Val Val Phe Leu Met Thr
   385 390 395 400
   Gly Met Thr Leu Phe Ile Ile Phe Tyr Asp Arg Val Phe Val Pro Thr
   405 410 415
   Leu Lys Arg Ile Thr Gly Ile Glu Thr Gly Ile Thr Leu Leu Gln Arg
   420 425 430
   Ile Gly Ala Gly Ile Phe Phe Ala Leu Leu Ser Leu Ile Val Ser Gly
   435 440 445
   Phe Val Glu Glu Arg Arg Arg Thr Leu Ala Leu Thr Lys Pro Thr Leu
   450 455 460
   Gly Leu Ala Pro Arg Lys Gly Glu Ile Ser Ser Met Ser Ala Met Trp
   465 470 475 480
   Leu Ile Pro Gln Leu Val Leu Ala Gly Ile Ala Asp Ala Phe Gly Ala
   485 490 495
   Ile Gly Gln Met Glu Phe Tyr Tyr Lys Gln Phe Pro Glu Asn Met Arg
   500 505 510
   Ser Phe Ala Gly Ser Ile Phe Tyr Val Gly Ala Gly Val Ser Ser Tyr
   515 520 525
   Ile Ser Ser Phe Leu Ile Thr Thr Val His Arg Met Thr Gln Asn Ser
   530 535 540
   Ala Gly Gly Asn Trp Leu Ala Glu Asp Leu Asn Lys Gly Arg Leu Asp
   545 550 555 560
   Tyr Phe Tyr Phe Leu Leu Ala Gly Ile Leu Ala Val Asn Phe Ala Tyr
   565 570 575
   Phe Leu Val Met Ala Arg Trp Tyr Lys Tyr Lys Gly Ser Asp Asp Glu
   580 585 590
   Glu Thr Ser Tyr Glu Thr Ser Gly Asp Ile Ile Lys Glu Gln Asp Lys
   595 600 605
   Asn Lys Val
   610
<210> 37
   <211> 2454
   <212> DNA
   <213> Brassica rapa
<220>
   <221> CDS
   <222> (1)..(151)
<220>
   <221> CDS
   <222> (598)..(815)
<220>
   <221> CDS
   <222> (908)..(1461)
<220>
   <221> CDS
   <222> (1542)..(2451)
<400> 37
   atg gag aga aag ccc ttt gat gtt gag aca act gag gat cac aaa ccc      48
   Met Glu Arg Lys Pro Phe Asp Val Glu Thr Thr Glu Asp His Lys Pro
   1               5                   10                  15
   tac tcc tcc gtc gat gac ggt ggt tct gat tcg gtc gat tca ttt gaa      96
   Tyr Ser Ser Val Asp Asp Gly Gly Ser Asp Ser Val Asp Ser Phe Glu
               20                  25                  30
   aac gag cag aaa aag ctc gtt tat aga ggc tgg aaa gtc atg ccc ttt      144
   Asn Glu Gln Lys Lys Leu Val Tyr Arg Gly Trp Lys Val Met Pro Phe
           35                  40                  45
   atc att g gtaacgcttc ttctcttcag accacttttt tttttagttt attctgtttt     201
   Ile Ile
       50
   gttttgttgt gtctcttttg tcggattata gaacgttttg cccgtgatgt ttttatttta     261
   atttcgtttc ttttgtcttc ttagacttga tttggagttc ttgtcttgac gtttcgagat     321
   tattgaaaca cagatcaacc aaaccagttg agtttcctag ataaatctac catggaaagt     381
   tagagacttt gaatcatgca agttttaagt ttctgaaata agattttagt tagtttctgg     441
   gagttataac agttaaagaa tgaatgaata ataaagtgtc cagtccacca ctgaaagtcc     501
   aatctttgat ttcaacattg tgaattaaat gtaaaaaggt tgttacttta tggtttgact     561
   aattatttat gttgaaaaaa atatattgtg tcacag gt  aat gag aca ttt gag      614
                                           Gly Asn Glu Thr Phe Glu
                                                           55
   aag ata ggg atc att ggg aca cta tca aac ctt ctg gtg tac cta act      662
   Lys Ile Gly Ile Ile Gly Thr Leu Ser Asn Leu Leu Val Tyr Leu Thr
               60                  65                  70
   tca gta ttc aac ctc aag agt gtt aca gct gca acc atc atc aac gcc      710
   Ser Val Phe Asn Leu Lys Ser Val Thr Ala Ala Thr Ile Ile Asn Ala
           75                  80                  85
   ttc agt ggc act atc aac ttc ggc act ttc ctc gct gct ttc ctc tgc      758
   Phe Ser Gly Thr Ile Asn Phe Gly Thr Phe Leu Ala Ala Phe Leu Cys
       90                  95                  100
   gac act tac ttt ggt cgc tac aag act ctc tct gtt gct gtc atc gcc      806
   Asp Thr Tyr Phe Gly Arg Tyr Lys Thr Leu Ser Val Ala Val Ile Ala
   105                 110                 115                 120
   tgt ttt ctg gtactctaat caaatgacca tcttgtttta ttttggccaa              855
   Cys Phe Leu
   gattttgtca ttttttttta aattactaaa ccatatttca tatttgttgt ag gga tcg     913
                                                             Gly Ser
                                                                 125
   ctt gtg ata cta ctg acg gct gca gtt cca gga ttg cac ccc att cct      961
   Leu Val Ile Leu Leu Thr Ala Ala Val Pro Gly Leu His Pro Ile Pro
                   130                 135                 140
   tgt gga aca caa agt tct tgc caa ggc cca agc gag ggc cag att gcg      1009
   Cys Gly Thr Gln Ser Ser Cys Gln Gly Pro Ser Glu Gly Gln Ile Ala
               145                 150                 155
   ttt ctt ctg atg ggt tta gcg ctt ctt gtg gtg ggt gct ggt gga atc      1057
   Phe Leu Leu Met Gly Leu Ala Leu Leu Val Val Gly Ala Gly Gly Ile
             160                 165                 170
   agg ccg tgt aac ttg gcc ttt ggt gct gac cag ttc aac ccc aag tca        1105
   Arg Pro Cys Asn Leu Ala Phe Gly Ala Asp Gln Phe Asn Pro Lys Ser
       175                 180                 185
   gaa tct gga aag aaa gga atc aac agt ttc ttc aac tgg tac ttc ttc        1153
   Glu Ser Gly Lys Lys Gly Ile Asn Ser Phe Phe Asn Trp Tyr Phe Phe
   190                 195                 200                 205
   act ttc acg ttc gcg cag atc atc tcc ctc acg cta gtt gtg tac atc        1201
   Thr Phe Thr Phe Ala Gln Ile Ile Ser Leu Thr Leu Val Val Tyr Ile
                   210                 215                 220
   cag tca aat gtg agc tgg acc atc ggt ttg agt atc cct gtg ggt ctt        1249
   Gln Ser Asn Val Ser Trp Thr Ile Gly Leu Ser Ile Pro Val Gly Leu
               225                 230                 235
   atg ttc ttg gcc tgt gtc att ttc ttt gct ggt cat aaa ctg tat gtg        1297
   Met Phe Leu Ala Cys Val Ile Phe Phe Ala Gly His Lys Leu Tyr Val
           240                 245                 250
   aaa gtg aaa gcc tct ggt agt cca ttg gct ggt ata ggt cat gtt ata        1345
   Lys Val Lys Ala Ser Gly Ser Pro Leu Ala Gly Ile Gly His Val Ile
       255                 260                 265
   gcc gca gcg atc aag aaa cgt ggc ttg aag cca gtt aag cag cct tgg        1393
   Ala Ala Ala Ile Lys Lys Arg Gly Leu Lys Pro Val Lys Gln Pro Trp
   270                 275                 280                 285
   atc gat ctt tac aac cac att cct cat aac tat gca aac tct act ctt        1441
   Ile Asp Leu Tyr Asn His Ile Pro His Asn Tyr Ala Asn Ser Thr Leu
                   290                 295                 300
   aaa tac acc gac cag ctt ag  gtaaagtcag aatagttctg tctctgtctt           1491
   Lys Tyr Thr Asp Gln Leu Arg
               305
   tctttgttca tttgctttat gctttgtgtg aacaaatgtt gtattgccag g ttt cta       1548
                                                            Phe Leu
                                                                310
   gac aaa gca gcg att atg acc cct gag gac aag ttg aac tcg gat gga        1596
   Asp Lys Ala Ala Ile Met Thr Pro Glu Asp Lys Leu Asn Ser Asp Gly
                   315                 320                 325
   acg gct tcg gat cca tgg aaa cta tgt acg atg cag aaa gtg gaa gaa        1644
   Thr Ala Ser Asp Pro Trp Lys Leu Cys Thr Met Gln Lys Val Glu Glu
               330                 335                 340
   gtg aaa tgc att tta aga gtg att cca atc tgg ttc gca tgc gcc att        1692
   Val Lys Cys Ile Leu Arg Val Ile Pro Ile Trp Phe Ala Cys Ala Ile
           345                 350                 355
   tac tac ctt gca ata agt atg caa atg act tat cca gtc ttc caa gcg        1740
   Tyr Tyr Leu Ala Ile Ser Met Gln Met Thr Tyr Pro Val Phe Gln Ala
       360                 365                 370
   ctc cag agc gac agg aga tta ggt tca ggt ggc ttc aag att cca gcc        1788
   Leu Gln Ser Asp Arg Arg Leu Gly Ser Gly Gly Phe Lys Ile Pro Ala
   375                 380                 385                 390
   gcc acc tat gtc gtg ttc ttg atg tct ggt atg acg gtt ttc atc ata        1836
   Ala Thr Tyr Val Val Phe Leu Met Ser Gly Met Thr Val Phe Ile Ile
                   395                 400                 405
   atc tac gac cgt gtc ctc gtc ccc tcg ctc aaa aga gtg acg ggt cta        1884
   Ile Tyr Asp Arg Val Leu Val Pro Ser Leu Lys Arg Val Thr Gly Leu
               410                 415                 420
   gac acg ggg ata aca ctc tta caa aga ata gga gct ggc att ttc ttt        1932
   Asp Thr Gly Ile Thr Leu Leu Gln Arg Ile Gly Ala Gly Ile Phe Phe
           425                 430                 435
   gcg gtg ttg agt tta ttg gtc tcc ggg ttc ata gag gaa cgt aga aga        1980
   Ala Val Leu Ser Leu Leu Val Ser Gly Phe Ile Glu Glu Arg Arg Arg
   440                   445                   450
   acc ttt gcg ctg aca cga cct acg ctg ggg tcg gag cct cga aca gga        2028
   Thr Phe Ala Leu Thr Arg Pro Thr Leu Gly Ser Glu Pro Arg Thr Gly
   455                 460                 465                 470
   gag atc tcc tca atg tca gcg atg tgg ctg att ccg cag ctg gtg ctt        2076
   Glu Ile Ser Ser Met Ser Ala Met Trp Leu Ile Pro Gln Leu Val Leu
                   475                 480                 485
   gca ggg gta gca gaa gct ttt gca gcc att ggg caa atg gag ttt tac        2124
   Ala Gly Val Ala Glu Ala Phe Ala Ala Ile Gly Gln Met Glu Phe Tyr
               490                 495                 500
   tac aag cag ttt cct gaa aac atg aag agc ttt gcg ggt tct atc ttc        2172
   Tyr Lys Gln Phe Pro Glu Asn Met Lys Ser Phe Ala Gly Ser Ile Phe
           505                 510                 515
   tat gtc ggt gca gga gtt tct agc tac ctc gct agc ttc ttg atc tcg        2220
   Tyr Val Gly Ala Gly Val Ser Ser Tyr Leu Ala Ser Phe Leu Ile Ser
       520                 525                 530
   act gtt cat aga aca act gag cat tca cct acg ggg aat tgg tta gct        2268
   Thr Val His Arg Thr Thr Glu His Ser Pro Thr Gly Asn Trp Leu Ala
   535                 540                 545                 550
   gag gat ctg aac aaa gcg aag ctg gac tac ttc tat ttc atg ctc aca        2316
   Glu Asp Leu Asn Lys Ala Lys Leu Asp Tyr Phe Tyr Phe Met Leu Thr
                   555                 560                 565
   gga ctc atg ttc gtt aac ttt ctt tac ttc ttg tta atg gct aga tgg        2364
   Gly Leu Met Phe Val Asn Phe Leu Tyr Phe Leu Leu Met Ala Arg Trp
               570                 575                 580
   tat aga tac aaa ggc act cat gat gaa gtc agt tat gtg gtt gac act        2412
   Tyr Arg Tyr Lys Gly Thr His Asp Glu Val Ser Tyr Val Val Asp Thr
           585                 590                 595
   gtt gac gaa gag aca aag caa caa gac aag aac tct gtc tga                2454
   Val Asp Glu Glu Thr Lys Gln Gln Asp Lys Asn Ser Val
       600                 605                 610
<210> 38
   <211> 611
   <212> PRT
   <213> Brassica rapa
<400> 38
   Met Glu Arg Lys Pro Phe Asp Val Glu Thr Thr Glu Asp His Lys Pro
   1               5                   10                  15
   Tyr Ser Ser Val Asp Asp Gly Gly Ser Asp Ser Val Asp Ser Phe Glu
               20                  25                  30
   Asn Glu Gln Lys Lys Leu Val Tyr Arg Gly Trp Lys Val Met Pro Phe
           35                  40                  45
   Ile Ile Gly Asn Glu Thr Phe Glu Lys Ile Gly Ile Ile Gly Thr Leu
       50                  55                  60
   Ser Asn Leu Leu Val Tyr Leu Thr Ser Val Phe Asn Leu Lys Ser Val
   65                  70                  75                  80
   Thr Ala Ala Thr Ile Ile Asn Ala Phe Ser Gly Thr Ile Asn Phe Gly
                   85                  90                  95
   Thr Phe Leu Ala Ala Phe Leu Cys Asp Thr Tyr Phe Gly Arg Tyr Lys
               100                 105                 110
   Thr Leu Ser Val Ala Val Ile Ala Cys Phe Leu Gly Ser Leu Val Ile
           115                 120                 125
   Leu Leu Thr Ala Ala Val Pro Gly Leu His Pro Ile Pro Cys Gly Thr
       130                 135                 140
   Gln Ser Ser Cys Gln Gly Pro Ser Glu Gly Gln Ile Ala Phe Leu Leu
   145                 150                 155                 160
   Met Gly Leu Ala Leu Leu Val Val Gly Ala Gly Gly Ile Arg Pro Cys
                   165                 170                 175
   Asn Leu Ala Phe Gly Ala Asp Gln Phe Asn Pro Lys Ser Glu Ser Gly
               180                 185                 190
   Lys Lys Gly Ile Asn Ser Phe Phe Asn Trp Tyr Phe Phe Thr Phe Thr
           195                 200                 205
   Phe Ala Gln Ile Ile Ser Leu Thr Leu Val Val Tyr Ile Gln Ser Asn
       210                 215                 220
   Val Ser Trp Thr Ile Gly Leu Ser Ile Pro Val Gly Leu Met Phe Leu
   225                 230                 235                 240
   Ala Cys Val Ile Phe Phe Ala Gly His Lys Leu Tyr Val Lys Val Lys
                   245                 250                 255
   Ala Ser Gly Ser Pro Leu Ala Gly Ile Gly His Val Ile Ala Ala Ala
               260                 265                 270
   Ile Lys Lys Arg Gly Leu Lys Pro Val Lys Gln Pro Trp Ile Asp Leu
           275                 280                 285
   Tyr Asn His Ile Pro His Asn Tyr Ala Asn Ser Thr Leu Lys Tyr Thr
       290                 295                 300
   Asp Gln Leu Arg Phe Leu Asp Lys Ala Ala Ile Met Thr Pro Glu Asp
   305                 310                 315                 320
   Lys Leu Asn Ser Asp Gly Thr Ala Ser Asp Pro Trp Lys Leu Cys Thr
                   325                 330                 335
   Met Gln Lys Val Glu Glu Val Lys Cys Ile Leu Arg Val Ile Pro Ile
               340                 345                 350
   Trp Phe Ala Cys Ala Ile Tyr Tyr Leu Ala Ile Ser Met Gln Met Thr
           355                 360                 365
   Tyr Pro Val Phe Gln Ala Leu Gln Ser Asp Arg Arg Leu Gly Ser Gly
       370                 375                 380
   Gly Phe Lys Ile Pro Ala Ala Thr Tyr Val Val Phe Leu Met Ser Gly
   385                 390                 395                 400
   Met Thr Val Phe Ile Ile Ile Tyr Asp Arg Val Leu Val Pro Ser Leu
                   405                 410                 415
   Lys Arg Val Thr Gly Leu Asp Thr Gly Ile Thr Leu Leu Gln Arg Ile
   420 425 430
   Gly Ala Gly Ile Phe Phe Ala Val Leu Ser Leu Leu Val Ser Gly Phe
   435 440 445
   Ile Glu Glu Arg Arg Arg Thr Phe Ala Leu Thr Arg Pro Thr Leu Gly
   450 455 460
   Ser Glu Pro Arg Thr Gly Glu Ile Ser Ser Met Ser Ala Met Trp Leu
   465 470 475 480
   Ile Pro Gln Leu Val Leu Ala Gly Val Ala Glu Ala Phe Ala Ala Ile
   485 490 495
   Gly Gln Met Glu Phe Tyr Tyr Lys Gln Phe Pro Glu Asn Met Lys Ser
   500 505 510
   Phe Ala Gly Ser Ile Phe Tyr Val Gly Ala Gly Val Ser Ser Tyr Leu
   515 520 525
   Ala Ser Phe Leu Ile Ser Thr Val His Arg Thr Thr Glu His Ser Pro
   530 535 540
   Thr Gly Asn Trp Leu Ala Glu Asp Leu Asn Lys Ala Lys Leu Asp Tyr
   545 550 555 560
   Phe Tyr Phe Met Leu Thr Gly Leu Met Phe Val Asn Phe Leu Tyr Phe
   565 570 575
   Leu Leu Met Ala Arg Trp Tyr Arg Tyr Lys Gly Thr His Asp Glu Val
   580 585 590
   Ser Tyr Val Val Asp Thr Val Asp Glu Glu Thr Lys Gln Gln Asp Lys
   595 600 605
   Asn Ser Val
   610
<210> 39
   <211> 2670
   <212> DNA
   <213> Brassica rapa
<220>
   <221> CDS
   <222> (1) .. (160)
<220>
   <221> CDS
   <222> (666)..(883)
<220>
   <221> CDS
   <222> (982)..(1535)
<220>
   <221> CDS
   <222> (1755) .. (2667)
<400> 39
   atg gaa aga aag cct ttt gag gtt gag aca aca aag acg gat gag gat        48
   Met Glu Arg Lys Pro Phe Glu Val Glu Thr Thr Lys Thr Asp Glu Asp
   1               5                   10                  15
   cag aaa ccc tac tcc gct gtc gat ggc ggt ggt tct gat tcg gtc gat        96
   Gln Lys Pro Tyr Ser Ala Val Asp Gly Gly Gly Ser Asp Ser Val Asp
               20                  25                  30
   tca ttt gat gaa gat cag aaa aag ctc gtg tac aga gga tgg aaa gtc       144
   Ser Phe Asp Glu Asp Gln Lys Lys Leu Val Tyr Arg Gly Trp Lys Val
           35                  40                  45
   atg cct ttt atc att g gtaacgctta ttttcttctc tattcagaca ctcctttttt     200
   Met Pro Phe Ile Ile
       50
   attttattgt gtgtctgctt ctctcaacac ggtccagaaa ctaaataaag atgtggaaaa     260
   taaactagaa catgttgtcc gtgaaattta aaaagtactt ttcttttctc ttcttctctt     320
   tgactgaaaa cgattgaaac caggatcaac caaatctacc agatctagag aaaggtgtta     380
   ctacaacttt ttttcattaa taatgctttt taatttttag ttttatacgc ttaaagacaa     440
   agttatttag tttctgggag ttacagcagt taaaagttaa aacaaaagga ataaagtttc     500
   aagttaaaga agcactgaaa gtacaacttc gattcgtgtg tgtttcggtt taaaagttgt     560
   gaattaaaaa gtttgttact gtataggaaa cccgtttttc cctttgacta ctgattcatt     620
   tattgtgaaa aagaaaatta ataatgattc ttgttctttt tacag gt  aat gaa aca     676
                                                     Gly Asn Glu Thr
                                                         55
   ttt gag aag att ggg atc att ggg aca tta tca aac ctt ctt gtg tac       724
   Phe Glu Lys Ile Gly Ile Ile Gly Thr Leu Ser Asn Leu Leu Val Tyr
           60                  65                  70
   cta act caa gta ttc aac ctc aag aaa tac aca gct gca acc atc atc       772
   Leu Thr Gln Val Phe Asn Leu Lys Lys Tyr Thr Ala Ala Thr Ile Ile
       75                  80                  85
   aat gcc ttt agt ggc acc atc aac ttc ggc act ttc ctc gct gct ttc       820
   Asn Ala Phe Ser Gly Thr Ile Asn Phe Gly Thr Phe Leu Ala Ala Phe
   90                  95                  100                 105
   ctc tgc gac act tac ttt ggt cgc tac aag act ctc agt gtt gct gtc       868
   Leu Cys Asp Thr Tyr Phe Gly Arg Tyr Lys Thr Leu Ser Val Ala Val
                   110                 115                 120
   atc gct tgt ttt ctg gtacctacta gtactaatgc tgtccttaac ggttttgttt       923
   Ile Ala Cys Phe Leu
               125
   tttcgtcaag gctctattct ttttttcttt ttgctaaacc gtgttttatc gtttgtag       981
   gga tcg ctt gcg ata cta ctc acg gca gca gtt ccg gga ctg cac ccg      1029
   Gly Ser Leu Ala Ile Leu Leu Thr Ala Ala Val Pro Gly Leu His Pro
               130                 135                 140
   act cct tgt gga aca aaa agt tca tgc gaa ggg cca ggt gcg ggg cag      1077
   Thr Pro Cys Gly Thr Lys Ser Ser Cys Glu Gly Pro Gly Ala Gly Gln
           145                 150                 155
   att ttg ttt ctg atg ttg ggt tta gcg ctt ctt gtg gtt ggt gct ggt      1125
   Ile Leu Phe Leu Met Leu Gly Leu Ala Leu Leu Val Val Gly Ala Gly
       160                 165                 170
   ggt atc agg ccg tgt aac ttg gcg ttt ggt gcc gat cag ttc aac ccc      1173
   Gly Ile Arg Pro Cys Asn Leu Ala Phe Gly Ala Asp Gln Phe Asn Pro
   175                 180                 185                 190
   aag tcc gaa tca gga aag aaa gga atc aac agt ttc ttc aac tgg tac      1221
   Lys Ser Glu Ser Gly Lys Lys Gly Ile Asn Ser Phe Phe Asn Trp Tyr
                   195                 200                 205
   ttc ttc act ttc acg ttc gcg cag atc atc tcg ctc acg cta gtt gtg         1269
   Phe Phe Thr Phe Thr Phe Ala Gln Ile Ile Ser Leu Thr Leu Val Val
               210                 215                 220
   tat atc cag tcg aat gtg agc tgg acc atc ggt ttg agt atc ccc gtg         1317
   Tyr Ile Gln Ser Asn Val Ser Trp Thr Ile Gly Leu Ser Ile Pro Val
           225                 230                 235
   ggt ctc atg ttc ttg gct tgc gtc att ttc ttc gct ggt cac aaa ctt         1365
   Gly Leu Met Phe Leu Ala Cys Val Ile Phe Phe Ala Gly His Lys Leu
       240                 245                 250
   tat gta aaa gtg aaa gcc tcg ggt agt ccc ttg gct ggt ata gct cgt         1413
   Tyr Val Lys Val Lys Ala Ser Gly Ser Pro Leu Ala Gly Ile Ala Arg
   255                 260                 265                 270
   gtt ata gca gcc gcg atc tcg aaa cga gga ttg aag ccg gtt aag cag         1461
   Val Ile Ala Ala Ala Ile Ser Lys Arg Gly Leu Lys Pro Val Lys Gln
                   275                 280                 285
   cct tgg gtc gat ctt tac aat cac att cct cgt aac tat gca aac tta         1509
   Pro Trp Val Asp Leu Tyr Asn His Ile Pro Arg Asn Tyr Ala Asn Leu
               290                 295                 300
   acc cta aaa tac acc gac cag ttc ag  gtaaactgaa tagttctgtc               1555
   Thr Leu Lys Tyr Thr Asp Gln Phe Arg
               305                 310
   tctattgctt tatatcttgg cacagccgga tggatgcctt gacttctaaa tatttttaaa       1615
   atgggtcgta tagactccaa gttgtcaaat ttgttgacca aaatatatat tttttattta       1675
   ccaaaatatt tttaaaatgg gcctggttgg aaacaaaatg ctgaaaacta aatgttggtt       1735
   ttgtttgtta tattgttag g ttt cta gac aaa gca gcg ata atg aca cct          1785
                          Phe Leu Asp Lys Ala Ala Ile Met Thr Pro
                                      315                 320
   gag gac aag ttg aac tct gat gga aca gct tcg gat cca tgg aaa cta         1833
   Glu Asp Lys Leu Asn Ser Asp Gly Thr Ala Ser Asp Pro Trp Lys Leu
               325                 330                 335
   tgt acg atg cag caa gtg gaa gaa gtg aaa tgc att ata aga gtg att         1881
   Cys Thr Met Gln Gln Val Glu Glu Val Lys Cys Ile Ile Arg Val Ile
           340                 345                 350
   ccg atc tgg ttt gct tgc gct atc tac tac ctt gca ata agc att caa         1929
   Pro Ile Trp Phe Ala Cys Ala Ile Tyr Tyr Leu Ala Ile Ser Ile Gln
       355                 360                 365
   atg act tat ccc gtc ttc caa gcg caa cag agc gac agg aga tta ggt         1977
   Met Thr Tyr Pro Val Phe Gln Ala Gln Gln Ser Asp Arg Arg Leu Gly
   370                 375                 380                 385
   acc ggt ggc ttc aag atc ccc gca gcc acc tac gta gtt ttc ttg atg         2025
   Thr Gly Gly Phe Lys Ile Pro Ala Ala Thr Tyr Val Val Phe Leu Met
                   390                 395                 400
   acc ggt atg acg gtt ttc atc ata atc tac gac cgt gtc ctt gtc ccg         2073
   Thr Gly Met Thr Val Phe Ile Ile Ile Tyr Asp Arg Val Leu Val Pro
               405                 410                 415
   tcg ctc aaa aga gtg acc ggt cta gac act ggt ata aca ctc ttg caa         2121
   Ser Leu Lys Arg Val Thr Gly Leu Asp Thr Gly Ile Thr Leu Leu Gln
           420                 425                 430
   agg atc gga gcg ggg atc ttc ttt gcc gtg ttg agt tta ttg gtc tcc         2169
   Arg Ile Gly Ala Gly Ile Phe Phe Ala Val Leu Ser Leu Leu Val Ser
       435                 440                 445
   ggg ttc ata gag gaa cgt aga aga agc att gcg ctg aca aaa cag act         2217
   Gly Phe Ile Glu Glu Arg Arg Arg Ser Ile Ala Leu Thr Lys Gln Thr
   450                 455                 460                 465
   cta ggg atg gag cct cga aca gga gag atc tca tca atg tcg gcg atg         2265
   Leu Gly Met Glu Pro Arg Thr Gly Glu Ile Ser Ser Met Ser Ala Met
                   470                 475                 480
   tgg ctg att ccg cag ctg gtt ctt gca ggg ata gct gaa gcg ttt aca      2313
   Trp Leu Ile Pro Gln Leu Val Leu Ala Gly Ile Ala Glu Ala Phe Thr
               485                 490                 495
   gcc att gga cag atg gag ttt tac tac aag cag ttt cct gag aac atg      2361
   Ala Ile Gly Gln Met Glu Phe Tyr Tyr Lys Gln Phe Pro Glu Asn Met
           500                 505                 510
   agg agc ttt gct ggt tct atc ttc tat gtc ggt gcg ggg gtt tct agc      2409
   Arg Ser Phe Ala Gly Ser Ile Phe Tyr Val Gly Ala Gly Val Ser Ser
       515                 520                 525
   tac ctc gct agc ttc ttg atc tcc act gtt cat cgg aca act gaa cat      2457
   Tyr Leu Ala Ser Phe Leu Ile Ser Thr Val His Arg Thr Thr Glu His
   530                 535                 540                 545
   tca cct tcg ggg aac tgg tta gct gag gat ctg aac aag gcc aag ttg      2505
   Ser Pro Ser Gly Asn Trp Leu Ala Glu Asp Leu Asn Lys Ala Lys Leu
                   550                 555                 560
   gat tac ttc tat ttc atg ctc acg ggg ctc atg ttc gtt aac atg gtt      2553
   Asp Tyr Phe Tyr Phe Met Leu Thr Gly Leu Met Phe Val Asn Met Val
               565                 570                 575
   tac ttc ttg ata atg gct aag ttt tat aga tac aaa ggc agt gat gat      2601
   Tyr Phe Leu Ile Met Ala Lys Phe Tyr Arg Tyr Lys Gly Ser Asp Asp
           580                 585                 590
   aaa gtc atc tct gct att gag acc agt gaa gac gag acc aag cag aag      2649
   Lys Val Ile Ser Ala Ile Glu Thr Ser Glu Asp Glu Thr Lys Gln Lys
       595                 600                 605
   caa gcg aag aac tcg gtt taa                                          2670
   Gln Ala Lys Asn Ser Val
   610                 615
<210> 40
   <211> 615
   <212> PRT
   <213> Brassica rapa
<400> 40
   Met Glu Arg Lys Pro Phe Glu Val Glu Thr Thr Lys Thr Asp Glu Asp
   1               5                   10                  15
   Gln Lys Pro Tyr Ser Ala Val Asp Gly Gly Gly Ser Asp Ser Val Asp
               20                  25                  30
   Ser Phe Asp Glu Asp Gln Lys Lys Leu Val Tyr Arg Gly Trp Lys Val
           35                  40                  45
   Met Pro Phe Ile Ile Gly Asn Glu Thr Phe Glu Lys Ile Gly Ile Ile
       50                  55                  60
   Gly Thr Leu Ser Asn Leu Leu Val Tyr Leu Thr Gln Val Phe Asn Leu
   65                  70                  75                  80
   Lys Lys Tyr Thr Ala Ala Thr Ile Ile Asn Ala Phe Ser Gly Thr Ile
                   85                  90                  95
   Asn Phe Gly Thr Phe Leu Ala Ala Phe Leu Cys Asp Thr Tyr Phe Gly
               100                 105                 110
   Arg Tyr Lys Thr Leu Ser Val Ala Val Ile Ala Cys Phe Leu Gly Ser
   115 120 125
   Leu Ala Ile Leu Leu Thr Ala Ala Val Pro Gly Leu His Pro Thr Pro
   130 135 140
   Cys Gly Thr Lys Ser Ser Cys Glu Gly Pro Gly Ala Gly Gln Ile Leu
   145 150 155 160
   Phe Leu Met Leu Gly Leu Ala Leu Leu Val Val Gly Ala Gly Gly Ile
   165 170 175
   Arg Pro Cys Asn Leu Ala Phe Gly Ala Asp Gln Phe Asn Pro Lys Ser
   180 185 190
   Glu Ser Gly Lys Lys Gly Ile Asn Ser Phe Phe Asn Trp Tyr Phe Phe
   195 200 205
   Thr Phe Thr Phe Ala Gln Ile Ile Ser Leu Thr Leu Val Val Tyr Ile
   210 215 220
   Gln Ser Asn Val Ser Trp Thr Ile Gly Leu Ser Ile Pro Val Gly Leu
   225 230 235 240
   Met Phe Leu Ala Cys Val Ile Phe Phe Ala Gly His Lys Leu Tyr Val
   245 250 255
   Lys Val Lys Ala Ser Gly Ser Pro Leu Ala Gly Ile Ala Arg Val Ile
   260 265 270
   Ala Ala Ala Ile Ser Lys Arg Gly Leu Lys Pro Val Lys Gln Pro Trp
   275 280 285
   Val Asp Leu Tyr Asn His Ile Pro Arg Asn Tyr Ala Asn Leu Thr Leu
   290 295 300
   Lys Tyr Thr Asp Gln Phe Arg Phe Leu Asp Lys Ala Ala Ile Met Thr
   305 310 315 320
   Pro Glu Asp Lys Leu Asn Ser Asp Gly Thr Ala Ser Asp Pro Trp Lys
   325 330 335
   Leu Cys Thr Met Gln Gln Val Glu Glu Val Lys Cys Ile Ile Arg Val
   340 345 350
   Ile Pro Ile Trp Phe Ala Cys Ala Ile Tyr Tyr Leu Ala Ile Ser Ile
   355 360 365
   Gln Met Thr Tyr Pro Val Phe Gln Ala Gln Gln Ser Asp Arg Arg Leu
   370 375 380
   Gly Thr Gly Gly Phe Lys Ile Pro Ala Ala Thr Tyr Val Val Phe Leu
   385 390 395 400
   Met Thr Gly Met Thr Val Phe Ile Ile Ile Tyr Asp Arg Val Leu Val
   405 410 415
   Pro Ser Leu Lys Arg Val Thr Gly Leu Asp Thr Gly Ile Thr Leu Leu
   420 425 430
   Gln Arg Ile Gly Ala Gly Ile Phe Phe Ala Val Leu Ser Leu Leu Val
   435 440 445
   Ser Gly Phe Ile Glu Glu Arg Arg Arg Ser Ile Ala Leu Thr Lys Gln
   450 455 460
   Thr Leu Gly Met Glu Pro Arg Thr Gly Glu Ile Ser Ser Met Ser Ala
   465 470 475 480
   Met Trp Leu Ile Pro Gln Leu Val Leu Ala Gly Ile Ala Glu Ala Phe
   485 490 495
   Thr Ala Ile Gly Gln Met Glu Phe Tyr Tyr Lys Gln Phe Pro Glu Asn
   500 505 510
   Met Arg Ser Phe Ala Gly Ser Ile Phe Tyr Val Gly Ala Gly Val Ser
   515 520 525
   Ser Tyr Leu Ala Ser Phe Leu Ile Ser Thr Val His Arg Thr Thr Glu
   530 535 540
   His Ser Pro Ser Gly Asn Trp Leu Ala Glu Asp Leu Asn Lys Ala Lys
   545 550 555 560
   Leu Asp Tyr Phe Tyr Phe Met Leu Thr Gly Leu Met Phe Val Asn Met
   565 570 575
   Val Tyr Phe Leu Ile Met Ala Lys Phe Tyr Arg Tyr Lys Gly Ser Asp
   580 585 590
   Asp Lys Val Ile Ser Ala Ile Glu Thr Ser Glu Asp Glu Thr Lys Gln
   595 600 605
   Lys Gln Ala Lys Asn Ser Val
   610 615
<210> 41
   <211> 2351
   <212> DNA
   <213> Brassica rapa
<220>
   <221> CDS
   <222> (1)..(154)
<220>
   <221> CDS
   <222> (512) .. (729)
<220>
   <221> CDS
   <222> (808)..(1361)
<220>
   <221> CDS
   <222> (1430)..(2348)
<400> 41
   atg gag aga aag ccc ttt gag ttt gag acg acg gag gat cac aaa ccc        48
   Met Glu Arg Lys Pro Phe Glu Phe Glu Thr Thr Glu Asp His Lys Pro
   1               5                   10                  15
   tac tcc gat ggc ggt ggt tct aat tta acg tcg acg gtt gat tca tct        96
   Tyr Ser Asp Gly Gly Gly Ser Asn Leu Thr Ser Thr Val Asp Ser Ser
               20                  25                  30
   ggc gac gag cag aaa aag ctc gtt tat aga ggc tgg aaa gtc atg cct       144
   Gly Asp Glu Gln Lys Lys Leu Val Tyr Arg Gly Trp Lys Val Met Pro
           35                  40                  45
   tat atc att g gtaacgcttt tctctctctc tagaccgtta tttttaaact             194
   Tyr Ile Ile
       50
   ctgttttctt tttgtcaact aactctgttt tttcgtgtaa cagaacactt ttccccgtga     254
   tggttttttt gtcatttaat aatttgttta tttttatttt gtcacgttgg cttgactgtg     314
   acagtgttct agataatcta ccaggtgtag tgtacagaaa gaaatgttac gttgtgttct     374
   ttagtaacgt ttttaaatgt ccgtgaaaag gactttgaat cactggaagt ccaatcttgg     434
   ttccaaactg tgttacttta tgagaaaccc atgttggttt gactattcat taacgaaaag     494
   aatttgtatc ttgatag gt  aat gag aca ttt gag aag att ggg atc ata       543
                      Gly Asn Glu Thr Phe Glu Lys Ile Gly Ile Ile
                                  55                  60
   ggg aca tta tca aac ctt cta gtg tac cta act caa gta ttc aac ctt       591
   Gly Thr Leu Ser Asn Leu Leu Val Tyr Leu Thr Gln Val Phe Asn Leu
           65                  70                  75
   aag aaa tac aca gct gca act att atc agt gcc ttt agt ggc act atc       639
   Lys Lys Tyr Thr Ala Ala Thr Ile Ile Ser Ala Phe Ser Gly Thr Ile
       80                  85                  90
   aac ttc ggc act ttc ttc gct gct ttt ctt tgt gac act tac ttt ggt       687
   Asn Phe Gly Thr Phe Phe Ala Ala Phe Leu Cys Asp Thr Tyr Phe Gly
   95                  100                 105                 110
   cgc tac aag act ctc agt gtg gct gtc atc gct tgt ttt ctg               729
   Arg Tyr Lys Thr Leu Ser Val Ala Val Ile Ala Cys Phe Leu
                   115                 120
   gtactaatgc agctgatctt aatgggtttt ttttcttacg ccaagactct taatgctgtt     789
   catttttcgt ctgtgtag gga tcg ctt ttt ata cta atg acg gct gca gtt         840
                       Gly Ser Leu Phe Ile Leu Met Thr Ala Ala Val
                       125                 130                 135
   ccg gga ttg cac ccg act cct tgt gga act aaa agt tcg tgc caa ggg       888
   Pro Gly Leu His Pro Thr Pro Cys Gly Thr Lys Ser Ser Cys Gln Gly
                   140                 145                 150
   cca aat ggg ggg cag gtt ttg ttt ctg ctg ttg ggt tta gcg ctt ctc       936
   Pro Asn Gly Gly Gln Val Leu Phe Leu Leu Leu Gly Leu Ala Leu Leu
               155                 160                 165
   gta gtc ggt gct ggt ggt atc agg ccg tgt aat ttg gcc ttt gga gct       984
   Val Val Gly Ala Gly Gly Ile Arg Pro Cys Asn Leu Ala Phe Gly Ala
           170                 175                 180
   gat cag ttc aac ccc aag tca gaa tct gga agg aaa gga atc aac agt      1032
   Asp Gln Phe Asn Pro Lys Ser Glu Ser Gly Arg Lys Gly Ile Asn Ser
       185                 190                 195
   ttc ttc aac tgg tac ttc ttc act ttc acg ttt gcg cag atc atc tcg      1080
   Phe Phe Asn Trp Tyr Phe Phe Thr Phe Thr Phe Ala Gln Ile Ile Ser
   200                 205                 210                 215
   ctc acg cta gtt gtg tat atc cag tca aac gtg agc tgg acc att ggc      1128
   Leu Thr Leu Val Val Tyr Ile Gln Ser Asn Val Ser Trp Thr Ile Gly
                   220                 225                 230
   ttg agt atc cct gtg ggt cta atg ttc ttg gcc tgc gtc att tac ttt      1176
   Leu Ser Ile Pro Val Gly Leu Met Phe Leu Ala Cys Val Ile Tyr Phe
                   235                 240                 245
   gct ggc cat aat ctc tac gta aaa gtg aaa gcc tcg ggt agt ccc ttg      1224
   Ala Gly His Asn Leu Tyr Val Lys Val Lys Ala Ser Gly Ser Pro Leu
           250                 255                 260
   gct ggc atc gct cgt gtt ata gct gca gcg atc aag aaa cga ggg ttg      1272
   Ala Gly Ile Ala Arg Val Ile Ala Ala Ala Ile Lys Lys Arg Gly Leu
       265                 270                 275
   aag cct gtt aag aag cct tgc tcc gat ctt tac aat cac atc ccg cct      1320
   Lys Pro Val Lys Lys Pro Cys Ser Asp Leu Tyr Asn His Ile Pro Pro
   280                 285                 290                 295
   aac tat gca aac tct aca ctc aaa tac tcg gac cag ttt ag               1361
   Asn Tyr Ala Asn Ser Thr Leu Lys Tyr Ser Asp Gln Phe Arg
                   300                 305
   gtaaaactct aagagtttct ctctctgttg ctttatgttt ctgtctttta acatgtttat    1421
   gtttctag a ttt ctc gac aag gca gcg gtg atg acc cct gag gac aag       1469
              Phe Leu Asp Lys Ala Ala Val Met Thr Pro Glu Asp Lys
              310                 315                 320
   ttg aaa ccc gat gga acg gct tca gat cca tgg aat cta tgt aca atg      1517
   Leu Lys Pro Asp Gly Thr Ala Ser Asp Pro Trp Asn Leu Cys Thr Met
           325                 330                 335
   cag cag gtg gaa gaa gtg aaa tgc att gta aga gtg att cca atc tgg      1565
   Gln Gln Val Glu Glu Val Lys Cys Ile Val Arg Val Ile Pro Ile Trp
       340                 345                 350
   tta gct tgc gca gtt tac tac ctg gca ata aca ttg caa atg act tat      1613
   Leu Ala Cys Ala Val Tyr Tyr Leu Ala Ile Thr Leu Gln Met Thr Tyr
   355                 360                 365                 370
   ccg gtc ttc caa gcg ctc cag agc gac cgg aga ttg ggt tca ggt ggc      1661
   Pro Val Phe Gln Ala Leu Gln Ser Asp Arg Arg Leu Gly Ser Gly Gly
                   375                 380                 385
   ttc aag att ccc ggc gcc acc tat gta gtg ttc ttg atg tcc ggt atg      1709
   Phe Lys Ile Pro Gly Ala Thr Tyr Val Val Phe Leu Met Ser Gly Met
               390                 395                 400
   acg gtt ttc atc ata ttc tac gac cgt gtc ctt gtc ccg tcg ctc aga      1757
   Thr Val Phe Ile Ile Phe Tyr Asp Arg Val Leu Val Pro Ser Leu Arg
           405                 410                 415
   aga gtg aca tgg cta gac aat ggt ata act ctc tta caa aga atc gga      1805
   Arg Val Thr Trp Leu Asp Asn Gly Ile Thr Leu Leu Gln Arg Ile Gly
       420                 425                 430
   tca ggc att ttc ttc gcg ctt ttg agt ttg ttg gtt tct ggg ttc gtt      1853
   Ser Gly Ile Phe Phe Ala Leu Leu Ser Leu Leu Val Ser Gly Phe Val
   435                 440                 445                 450
   gag gaa cgg cga aga gcc att gca ctg aca aaa cct act ctc ggg att      1901
   Glu Glu Arg Arg Arg Ala Ile Ala Leu Thr Lys Pro Thr Leu Gly Ile
                   455                 460                 465
   gag cct cga gca gga gaa atc tca gca atg tcg gcg atg tgg ctg att      1949
   Glu Pro Arg Ala Gly Glu Ile Ser Ala Met Ser Ala Met Trp Leu Ile
               470                 475                 480
   ccg cag ctc gtg ctt gca ggg ata gct gaa gct ttt gca gct gtt gga      1997
   Pro Gln Leu Val Leu Ala Gly Ile Ala Glu Ala Phe Ala Ala Val Gly
           485                 490                 495
   caa atg gag ttt tac tac aag cag ttt cct gaa aac atg aag agc ttt      2045
   Gln Met Glu Phe Tyr Tyr Lys Gln Phe Pro Glu Asn Met Lys Ser Phe
       500                 505                 510
   gcg ggt tct atc ttc tat gtc ggt gca gga gtt tct agc tac ctc gct      2093
   Ala Gly Ser Ile Phe Tyr Val Gly Ala Gly Val Ser Ser Tyr Leu Ala
   515                 520                 525                 530
   agc ttc ttg atc tcg act gtt cat aaa aca act gag cat tca cgc tcc      2141
   Ser Phe Leu Ile Ser Thr Val His Lys Thr Thr Glu His Ser Arg Ser
                   535                 540                 545
   ggg aat tgg cta gct gag gat ttg aac aaa ggg aag ctg gat tac ttt      2189
   Gly Asn Trp Leu Ala Glu Asp Leu Asn Lys Gly Lys Leu Asp Tyr Phe
               550                 555                 560
   tat ttc atg ctc act ggc ctc atg ttt gtt aac atg gtt tac ttc ttg      2237
   Tyr Phe Met Leu Thr Gly Leu Met Phe Val Asn Met Val Tyr Phe Leu
           565                 570                 575
   tta atg gct aaa ttt tat aga tac aaa gtc act aac gat gaa gca aat      2285
   Leu Met Ala Lys Phe Tyr Arg Tyr Lys Val Thr Asn Asp Glu Ala Asn
       580                 585                 590
   tct gtg att aag acc aat gaa gaa gag acc aag gag aat cag caa caa      2333
   Ser Val Ile Lys Thr Asn Glu Glu Glu Thr Lys Glu Asn Gln Gln Gln
   595                 600                 605                 610
   gac aag agc tat gtc tga                                              2351
   Asp Lys Ser Tyr Val
                   615
<210> 42
   <211> 615
   <212> PRT
   <213> Brassica rapa
<400> 42
   Met Glu Arg Lys Pro Phe Glu Phe Glu Thr Thr Glu Asp His Lys Pro
   1               5                   10                  15
   Tyr Ser Asp Gly Gly Gly Ser Asn Leu Thr Ser Thr Val Asp Ser Ser
               20                  25                  30
   Gly Asp Glu Gln Lys Lys Leu Val Tyr Arg Gly Trp Lys Val Met Pro
           35                  40                  45
   Tyr Ile Ile Gly Asn Glu Thr Phe Glu Lys Ile Gly Ile Ile Gly Thr
       50                  55                  60
   Leu Ser Asn Leu Leu Val Tyr Leu Thr Gln Val Phe Asn Leu Lys Lys
   65                  70                  75                  80
   Tyr Thr Ala Ala Thr Ile Ile Ser Ala Phe Ser Gly Thr Ile Asn Phe
                   85                  90                  95
   Gly Thr Phe Phe Ala Ala Phe Leu Cys Asp Thr Tyr Phe Gly Arg Tyr
               100                 105                 110
   Lys Thr Leu Ser Val Ala Val Ile Ala Cys Phe Leu Gly Ser Leu Phe
           115                 120                 125
   Ile Leu Met Thr Ala Ala Val Pro Gly Leu His Pro Thr Pro Cys Gly
       130                 135                 140
   Thr Lys Ser Ser Cys Gln Gly Pro Asn Gly Gly Gln Val Leu Phe Leu
   145                 150                 155                 160
   Leu Leu Gly Leu Ala Leu Leu Val Val Gly Ala Gly Gly Ile Arg Pro
                   165                 170                 175
   Cys Asn Leu Ala Phe Gly Ala Asp Gln Phe Asn Pro Lys Ser Glu Ser
               180                 185                 190
   Gly Arg Lys Gly Ile Asn Ser Phe Phe Asn Trp Tyr Phe Phe Thr Phe
           195                 200                 205
   Thr Phe Ala Gln Ile Ile Ser Leu Thr Leu Val Val Tyr Ile Gln Ser
       210                 215                 220
   Asn Val Ser Trp Thr Ile Gly Leu Ser Ile Pro Val Gly Leu Met Phe
   225                 230                 235                 240
   Leu Ala Cys Val Ile Tyr Phe Ala Gly His Asn Leu Tyr Val Lys Val
                   245                 250                 255
   Lys Ala Ser Gly Ser Pro Leu Ala Gly Ile Ala Arg Val Ile Ala Ala
               260                 265                 270
   Ala Ile Lys Lys Arg Gly Leu Lys Pro Val Lys Lys Pro Cys Ser Asp
           275                 280                 285
   Leu Tyr Asn His Ile Pro Pro Asn Tyr Ala Asn Ser Thr Leu Lys Tyr
       290                 295                 300
   Ser Asp Gln Phe Arg Phe Leu Asp Lys Ala Ala Val Met Thr Pro Glu
   305                 310                 315                 320
   Asp Lys Leu Lys Pro Asp Gly Thr Ala Ser Asp Pro Trp Asn Leu Cys
                   325                 330                 335
   Thr Met Gln Gln Val Glu Glu Val Lys Cys Ile Val Arg Val Ile Pro
               340                 345                 350
   Ile Trp Leu Ala Cys Ala Val Tyr Tyr Leu Ala Ile Thr Leu Gln Met
           355                 360                 365
   Thr Tyr Pro Val Phe Gln Ala Leu Gln Ser Asp Arg Arg Leu Gly Ser
       370                 375                 380
   Gly Gly Phe Lys Ile Pro Gly Ala Thr Tyr Val Val Phe Leu Met Ser
   385                 390                 395                 400
   Gly Met Thr Val Phe Ile Ile Phe Tyr Asp Arg Val Leu Val Pro Ser
                   405                 410                 415
   Leu Arg Arg Val Thr Trp Leu Asp Asn Gly Ile Thr Leu Leu Gln Arg
               420                 425                 430
   Ile Gly Ser Gly Ile Phe Phe Ala Leu Leu Ser Leu Leu Val Ser Gly
           435                 440                 445
   Phe Val Glu Glu Arg Arg Arg Ala Ile Ala Leu Thr Lys Pro Thr Leu
       450                 455                 460
   Gly Ile Glu Pro Arg Ala Gly Glu Ile Ser Ala Met Ser Ala Met Trp
   465                 470                 475                 480
   Leu Ile Pro Gln Leu Val Leu Ala Gly Ile Ala Glu Ala Phe Ala Ala
                   485                 490                 495
   Val Gly Gln Met Glu Phe Tyr Tyr Lys Gln Phe Pro Glu Asn Met Lys
               500                 505                 510
   Ser Phe Ala Gly Ser Ile Phe Tyr Val Gly Ala Gly Val Ser Ser Tyr
           515                 520                 525
   Leu Ala Ser Phe Leu Ile Ser Thr Val His Lys Thr Thr Glu His Ser
       530                 535                 540
   Arg Ser Gly Asn Trp Leu Ala Glu Asp Leu Asn Lys Gly Lys Leu Asp
   545                 550                 555                 560
   Tyr Phe Tyr Phe Met Leu Thr Gly Leu Met Phe Val Asn Met Val Tyr
                   565                 570                 575
   Phe Leu Leu Met Ala Lys Phe Tyr Arg Tyr Lys Val Thr Asn Asp Glu
               580                 585                 590
   Ala Asn Ser Val Ile Lys Thr Asn Glu Glu Glu Thr Lys Glu Asn Gln
           595                 600                 605
   Gln Gln Asp Lys Ser Tyr Val
       610                 615
<210> 43
   <211> 2369
   <212> DNA
   <213> Brassica rapa
<220>
   <221> CDS
   <222> (1)..(157)
<220>
   <221> CDS
   <222> (499)..(716)
<220>
   <221> CDS
   <222> (812)..(1365)
<220>
   <221> CDS
   <222> (1460)..(2366)
<400> 43
   atg gag aga aag cct cta gaa gtc gag tca acg gat cac caa aag cct      48
   Met Glu Arg Lys Pro Leu Glu Val Glu Ser Thr Asp His Gln Lys Pro
   1               5                   10                  15
   ccc tcc gcc gtg tac gat ggc tct gtt atg gcg gtt gat tct gtt gag      96
   Pro Ser Ala Val Tyr Asp Gly Ser Val Met Ala Val Asp Ser Val Glu
               20                  25                  30
   gaa gaa gtt gag gag acc aaa gtc gtt tac aga ggc tgg aaa gtc atg     144
   Glu Glu Val Glu Glu Thr Lys Val Val Tyr Arg Gly Trp Lys Val Met
           35                  40                  45
   ccc ttt atc att g gtaacacaaa cttttttttt ataaactttc ttcttttgtt           197
   Pro Phe Ile Ile
       50
   ccaagaactt ttttttttaa ttgcttttca tgtttttttt ccaaaataaa taatcaatag    257
   ttgaagaaat gaagaggaaa acatcagtct tgcttgaaga cttattggtt tgatttaaaa      317
   aaaaaaatcg tgagattctc tgtactgttg tcaaaagctt tgacttagtc aaccagtttt      377
   ttaagaaaat taaaaaaaag agagtgagat ctagaatttg agaaggtttc agaacataac      437
   tttgaggcat atgagatcaa agtcttatga tcatcttggt tttgtttgtt ggtattatca      497
   g ga  aat gag acg ttt gag aag ctt ggg atc att gga aca cta tca aac      545
     Gly Asn Glu Thr Phe Glu Lys Leu Gly Ile Ile Gly Thr Leu Ser Asn
             55                  60                  65
   ctt ctg gtt tat tta act gct gtc ttc aac atg aag agt atc aca gct        593
   Leu Leu Val Tyr Leu Thr Ala Val Phe Asn Met Lys Ser Ile Thr Ala
       70                  75                  80
   gca aca atc atc aat gca ttc agt ggc aca atc aac ttc gga acc ttc        641
   Ala Thr Ile Ile Asn Ala Phe Ser Gly Thr Ile Asn Phe Gly Thr Phe
   85                  90                  95                  100
   gtt gct gct ttc ctc tgc gac act tac ttc ggt cga tac aag act cta        689
   Val Ala Ala Phe Leu Cys Asp Thr Tyr Phe Gly Arg Tyr Lys Thr Leu
                   105                 110                 115
   act gtc gct gtc atc gcc tgt ttt ctt gtaatgatca ttcctttgca              736
   Thr Val Ala Val Ile Ala Cys Phe Leu
               120                 125
   atctttcttg ctccacaagc gttcttagtt taacaaccct aaaaatctga taattgttgt      796
   tctttttttt tgtag gga tca ctt gtg ata cta ttg aca gct gca gtg cca       847
                    Gly Ser Leu Val Ile Leu Leu Thr Ala Ala Val Pro
                                    130                 135
   caa tta cat cca gct cca tgc gga aca gct ctc tca tgt atc ggg cca        895
   Gln Leu His Pro Ala Pro Cys Gly Thr Ala Leu Ser Cys Ile Gly Pro
           140                 145                 150
   agt ggt ggc cag ata gcg ttt cta ctg ctg ggt ctc ggc ttt ctt gta        943
   Ser Gly Gly Gln Ile Ala Phe Leu Leu Leu Gly Leu Gly Phe Leu Val
       155                 160                 165
   gtt gga gca ggc ggg att aga cca tgt aat cta gct ttc ggg gct gat        991
   Val Gly Ala Gly Gly Ile Arg Pro Cys Asn Leu Ala Phe Gly Ala Asp
   170                 175                 180                 185
   cag ttt aac ccc aag agc gag tca gga aaa cga ggc atc gat agt ttc       1039
   Gln Phe Asn Pro Lys Ser Glu Ser Gly Lys Arg Gly Ile Asp Ser Phe
                   190                 195                 200
   ttc aat tgg tac ttc ttc acc ttc acg ttc gcg cag atc ctg tcg ctg       1087
   Phe Asn Trp Tyr Phe Phe Thr Phe Thr Phe Ala Gln Ile Leu Ser Leu
               205                 210                 215
   aca cta atc gtc tat atc cag tcc aat gtc agc tgg acc atc ggt tta       1135
   Thr Leu Ile Val Tyr Ile Gln Ser Asn Val Ser Trp Thr Ile Gly Leu
           220                 225                 230
   acc atc ccg gtc gtt ctc atg ttc ttg gcc tgc gtg att ttc ttt gct       1183
   Thr Ile Pro Val Val Leu Met Phe Leu Ala Cys Val Ile Phe Phe Ala
       235                 240                 245
   gga gat aag ttg tat gtg aag atc aaa gca tca ggt agt cca ttg gct       1231
   Gly Asp Lys Leu Tyr Val Lys Ile Lys Ala Ser Gly Ser Pro Leu Ala
   250                 255                 260                 265
   ggt ata gct caa gtt ata gcg gtt gca atc aag aaa cgt ggg tta aag       1279
   Gly Ile Ala Gln Val Ile Ala Val Ala Ile Lys Lys Arg Gly Leu Lys
                   270                 275                 280
   cca acg aaa gag cct tgg ctt aac ctt tac aat tac tac cca cca aaa       1327
   Pro Thr Lys Glu Pro Trp Leu Asn Leu Tyr Asn Tyr Tyr Pro Pro Lys
               285                 290                 295
   tac gcg aac tcc aaa ctc aaa tac acc gac cag ttt ag  gtaacaaaat        1375
   Tyr Ala Asn Ser Lys Leu Lys Tyr Thr Asp Gln Phe Arg
           300                 305
   acacacacgc caagagttag ttttatcttt aatatctttc cacttttgtt atatggttac      1435
   tgagaaagtt tcctgttttg gcag g ttt ctt gat aag gct gcg atc ttg gct      1487
                                Phe Leu Asp Lys Ala Ala Ile Leu Ala
                                                315
   ccc gaa gac aag ttg gag gct gat ggt aag cct gcg gat cca tgg aag      1535
   Pro Glu Asp Lys Leu Glu Ala Asp Gly Lys Pro Ala Asp Pro Trp Lys
   320                 325                 330                 335
   ctc cgt aca atg caa caa gtt gaa gaa gtg aag tgc att gtg aga gtg      1583
   Leu Arg Thr Met Gln Gln Val Glu Glu Val Lys Cys Ile Val Arg Val
                   340                 345                 350
   ctt cct ata tgg ttt gct gca tcg att tat tac ttg acc atc acg caa      1631
   Leu Pro Ile Trp Phe Ala Ala Ser Ile Tyr Tyr Leu Thr Ile Thr Gln
               355                 360                 365
   caa atg act tac ccc gtc ttc caa gcc ctc cag agc gac cgt cgc tta      1679
   Gln Met Thr Tyr Pro Val Phe Gln Ala Leu Gln Ser Asp Arg Arg Leu
           370                 375                 380
   gga tcc aga gga ttt gtg atc ccg gca gcc acc tac gtt gtc ttt ttg      1727
   Gly Ser Arg Gly Phe Val Ile Pro Ala Ala Thr Tyr Val Val Phe Leu
       385                 390                 395
   atg aca gga atg acg gtt ttc atc ata ttc tac gac cgt gtc ctc gtg      1775
   Met Thr Gly Met Thr Val Phe Ile Ile Phe Tyr Asp Arg Val Leu Val
   400                 405                 410                 415
   cct acc tta aga aga ata acc ggt ata gac acg ggg ata aca ctc ttg      1823
   Pro Thr Leu Arg Arg Ile Thr Gly Ile Asp Thr Gly Ile Thr Leu Leu
                   420                 425                 430
   cag agg att gga acc ggt att ttc ttc gcc ttt gtt agc ttg ata gtc      1871
   Gln Arg Ile Gly Thr Gly Ile Phe Phe Ala Phe Val Ser Leu Ile Val
               435                 440                 445
   tca ggt ttc gtc gag gaa cgg agg aga acg ttc gcg ctg act aaa cca      1919
   Ser Gly Phe Val Glu Glu Arg Arg Arg Thr Phe Ala Leu Thr Lys Pro
           450                 455                 460
   aca ctt ggt atg gct cca agg aag gga gaa atc tcc tca atg tca gct      1967
   Thr Leu Gly Met Ala Pro Arg Lys Gly Glu Ile Ser Ser Met Ser Ala
       465                 470                 475
   atg tgg cta atc cca caa ctc gcc ctt gcg ggt ata gcc gag gcg ttt      2015
   Met Trp Leu Ile Pro Gln Leu Ala Leu Ala Gly Ile Ala Glu Ala Phe
   480                 485                 490                 495
   gga gct att gga cag atg gag ttt tac tac aag caa ttc cct gag aac      2063
   Gly Ala Ile Gly Gln Met Glu Phe Tyr Tyr Lys Gln Phe Pro Glu Asn
                   500                 505                 510
   atg agg agt ttt gct ggt tcg atc ttc tat gta gga gca gga gtt tcg      2111
   Met Arg Ser Phe Ala Gly Ser Ile Phe Tyr Val Gly Ala Gly Val Ser
               515                 520                 525
   agc tac ctc ggt agc ttc ttg att gca atg gtt cac agg acg acg cag      2159
   Ser Tyr Leu Gly Ser Phe Leu Ile Ala Met Val His Arg Thr Thr Gln
           530                 535                 540
   aac tcg gct ggt ggt aat tgg ttg gct gag gat ttg aac aaa gga aga      2207
   Asn Ser Ala Gly Gly Asn Trp Leu Ala Glu Asp Leu Asn Lys Gly Arg
       545                 550                 555
   ttg gat tac ttc tat ttc atg atc gct gga atc ttg gca gtt aat ttt      2255
   Leu Asp Tyr Phe Tyr Phe Met Ile Ala Gly Ile Leu Ala Val Asn Phe
   560                 565                 570                 575
   gct tac ttc ttg gtc atg tca aga tgg tat aga tac aaa gga agt gat      2303
   Ala Tyr Phe Leu Val Met Ser Arg Trp Tyr Arg Tyr Lys Gly Ser Asp
                   580                 585                 590
   gat gaa gtg aca act tat gaa acc aat gta gat ccc atc aaa caa caa        2351
   Asp Glu Val Thr Thr Tyr Glu Thr Asn Val Asp Pro Ile Lys Gln Gln
               595                 600                 605
   gac aag aat act gcc tga                                                2369
   Asp Lys Asn Thr Ala
           610
<210> 44
   <211> 612
   <212> PRT
   <213> Brassica rapa
<400> 44
   Met Glu Arg Lys Pro Leu Glu Val Glu Ser Thr Asp His Gln Lys Pro
   1 5 10 15
   Pro Ser Ala Val Tyr Asp Gly Ser Val Met Ala Val Asp Ser Val Glu
   20 25 30
   Glu Glu Val Glu Glu Thr Lys Val Val Tyr Arg Gly Trp Lys Val Met
   35 40 45
   Pro Phe Ile Ile Gly Asn Glu Thr Phe Glu Lys Leu Gly Ile Ile Gly
   50 55 60
   Thr Leu Ser Asn Leu Leu Val Tyr Leu Thr Ala Val Phe Asn Met Lys
   65 70 75 80
   Ser Ile Thr Ala Ala Thr Ile Ile Asn Ala Phe Ser Gly Thr Ile Asn
   85 90 95
   Phe Gly Thr Phe Val Ala Ala Phe Leu Cys Asp Thr Tyr Phe Gly Arg
   100 105 110
   Tyr Lys Thr Leu Thr Val Ala Val Ile Ala Cys Phe Leu Gly Ser Leu
   115 120 125
   Val Ile Leu Leu Thr Ala Ala Val Pro Gln Leu His Pro Ala Pro Cys
   130 135 140
   Gly Thr Ala Leu Ser Cys Ile Gly Pro Ser Gly Gly Gln Ile Ala Phe
   145 150 155 160
   Leu Leu Leu Gly Leu Gly Phe Leu Val Val Gly Ala Gly Gly Ile Arg
   165 170 175
   Pro Cys Asn Leu Ala Phe Gly Ala Asp Gln Phe Asn Pro Lys Ser Glu
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Leu Ile Val Tyr Ile Gln
   210 215 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Gly Ile Ala Gln Val Ile Ala
   260 265 270
   Val Ala Ile Lys Lys Arg Gly Leu Lys Pro Thr Lys Glu Pro Trp Leu
   275 280 285
   Asn Leu Tyr Asn Tyr Tyr Pro Pro Lys Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Ala Asp Pro Trp Lys Leu
   325 330 335
   Arg Thr Met Gln Gln Val Glu Glu Val Lys Cys Ile Val Arg Val Leu
   340 345 350
   Pro Ile Trp Phe Ala Ala Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Arg Gly Phe Val Ile Pro Ala Ala Thr Tyr Val Val Phe Leu Met
   385 390 395 400
   Thr Gly Met Thr Val Phe Ile Ile Phe Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Val Ser Leu Ile Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465 470 475 480
   Trp Leu Ile Pro Gln Leu Ala Leu Ala Gly Ile Ala Glu Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Ala Gly Val Ser Ser
   515 520 525
   Tyr Leu Gly Ser Phe Leu Ile Ala Met Val His Arg Thr Thr Gln Asn
   530 535 540
   Ser Ala Gly Gly Asn Trp Leu Ala Glu Asp Leu Asn Lys Gly Arg Leu
   545                 550                 555                 560
   Asp Tyr Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Glu Val Thr Thr Tyr Glu Thr Asn Val Asp Pro Ile Lys Gln Gln Asp
           595                 600                 605
   Lys Asn Thr Ala
       610
<210> 45
   <211> 2383
   <212> DNA
   <213> Brassica rapa
<220>
   <221> CDS
   <222> (1)..(157)
<220>
   <221> CDS
   <222> (536)..(753)
<220>
   <221> CDS
   <222> (846)..(1399)
<220>
   <221> CDS
   <222> (1474)..(2380)
<400> 45
   atg gag aga aag cct ctg gaa gtt gag tcc acc gat cat cag aaa cct        48
   Met Glu Arg Lys Pro Leu Glu Val Glu Ser Thr Asp His Gln Lys Pro
   1               5                   10                  15
   tcc tcc gcc gtg tac gac ggc tct gct acg gcg gtt gat tct gtt gaa        96
   Ser Ser Ala Val Tyr Asp Gly Ser Ala Thr Ala Val Asp Ser Val Glu
               20                  25                  30
   gaa gaa gtt cag gaa acc aaa ctc gtt tat aga ggc tgg aaa gtc atg       144
   Glu Glu Val Gln Glu Thr Lys Leu Val Tyr Arg Gly Trp Lys Val Met
           35                  40                  45
   cct ttc atc ata g gtaacgcata tgttttaaac atataaaaaa tataaatttt         197
   Pro Phe Ile Ile
       50
   tgataaaaaa aatatatatc attttctttg attggtatat ttccaaagtt ttagaaacta     257
   tatttctaga aatttcctgt aattaaattt tgacaatttt cttctctgtt tctagaaact     317
   atattttgtt ggtttgattt tattattggt gagattcttt gtgttgttgt cttaagcttt     377
   gacttagtca aacagtaatt taacccaaaa gagtgagatc ttgagtttga agatgtttca     437
   gaacacataa atgatcaaga atctcttgtt tgattcttcg tttggttgct aaggtcaaag     497
   tcttatgatc ttttcttctt gcatgttgtt attaccag ga  aac gag aca ttt gag     552
                                            Gly Asn Glu Thr Phe Glu
                                                    55
   aag ctt ggg atc att gga aca cta tca aac ctt ctg gtt tac tta act       600
   Lys Leu Gly Ile Ile Gly Thr Leu Ser Asn Leu Leu Val Tyr Leu Thr
       60                  65                  70
   gct gtc ttc aac atg aag agt atc aca gct gct aca atc atc aac gcc       648
   Ala Val Phe Asn Met Lys Ser Ile Thr Ala Ala Thr Ile Ile Asn Ala
   75                  80                  85                  90
   ttt agt ggc aca atc aac ttc gga acc ttc gtt gcc gcc ttc ctt tgc       696
   Phe Ser Gly Thr Ile Asn Phe Gly Thr Phe Val Ala Ala Phe Leu Cys
                   95                  100                 105
   gac act tac ttc ggt cgt tac aag aca cta acc gtc gcg gtc atc gca       744
   Asp Thr Tyr Phe Gly Arg Tyr Lys Thr Leu Thr Val Ala Val Ile Ala
               110                 115                 120
   tgt ttt ctt gtaataatcc atcctctata accttttctt cctccacaag               793
   Cys Phe Leu
           125
   caatcttggt ttataaacct aaaaaaaaaa aatctggttc ttgttttggt ag gga tca     851
                                                             Gly Ser
   ctt gtg ata cta tta act gct gca gta cca caa ctg cat cca gct cca       899
   Leu Val Ile Leu Leu Thr Ala Ala Val Pro Gln Leu His Pro Ala Pro
           130                 135                 140
   tgc gga aca gcc ctc acg tgt aca ggc cca agt ggc ggc cag ata gcc       947
   Cys Gly Thr Ala Leu Thr Cys Thr Gly Pro Ser Gly Gly Gln Ile Ala
       145                 150                 155
   ttt cta cta atg ggt ctt gcc ttt ctt gtg gtg gga gca ggt ggg att       995
   Phe Leu Leu Met Gly Leu Ala Phe Leu Val Val Gly Ala Gly Gly Ile
   160                 165                 170                 175
   aga ccg tgt aat ctt gct ttt gga gct gat cag ttt aac cct aaa agc      1043
   Arg Pro Cys Asn Leu Ala Phe Gly Ala Asp Gln Phe Asn Pro Lys Ser
                   180                 185                 190
   gag tca ggg aag aga ggg att gat agt ttc ttt aac tgg tac ttc ttc      1091
   Glu Ser Gly Lys Arg Gly Ile Asp Ser Phe Phe Asn Trp Tyr Phe Phe
               195                 200                 205
   acc ttc acg ttc gcg cag atc ttg tcg ctg aca cta gtt gtc tac atc      1139
   Thr Phe Thr Phe Ala Gln Ile Leu Ser Leu Thr Leu Val Val Tyr Ile
           210                 215                 220
   cag tct aac gtc agt tgg acc att ggt tta acc ata cct gtc gtg tta      1187
   Gln Ser Asn Val Ser Trp Thr Ile Gly Leu Thr Ile Pro Val Val Leu
       225                 230                 235
   atg ttc ttg gcc tgc gtg atc ttc ttt gcg ggt gat aag ttg tat gtc      1235
   Met Phe Leu Ala Cys Val Ile Phe Phe Ala Gly Asp Lys Leu Tyr Val
   240                 245                 250                 255
   aag atc aaa gcc tca ggt agt cca ttg gct agt ata gct caa gtt ata      1283
   Lys Ile Lys Ala Ser Gly Ser Pro Leu Ala Ser Ile Ala Gln Val Ile
                   260                 265                 270
   acg gtt gct atc aag aaa cgt ggg tta aag gct gtg aag cag cct tgg      1331
   Thr Val Ala Ile Lys Lys Arg Gly Leu Lys Ala Val Lys Gln Pro Trp
               275                 280                 285
   gtt aac ctt tac aat tac tac cca cta aac tac gca aac tct aag ctc      1379
   Val Asn Leu Tyr Asn Tyr Tyr Pro Leu Asn Tyr Ala Asn Ser Lys Leu
           290                 295                 300
   aag tac acc gac cag ttc ag  gtaaagacac acggttataa ataatatctt         1429
   Lys Tyr Thr Asp Gln Phe Arg
       305
   tccacttttg tttacttaga aagcaagctt tctttttttg gtag g ttt ctt gat          1483
                                                      Phe Leu Asp
   aag gcg gcg atc ttg gct cct gaa gac aag ttg gag gct gat ggt aag      1531
   Lys Ala Ala Ile Leu Ala Pro Glu Asp Lys Leu Glu Ala Asp Gly Lys
                   315                 320                 325
   cct acg gat cca tgg aag cta tgt aca atg caa caa gtt gaa gaa gtg      1579
   Pro Thr Asp Pro Trp Lys Leu Cys Thr Met Gln Gln Val Glu Glu Val
   330                 335                 340                 345
   aag tgc att gtg aga gtg ctt cct ata tgg ttt gct tca tcc atc tat      1627
   Lys Cys Ile Val Arg Val Leu Pro Ile Trp Phe Ala Ser Ser Ile Tyr
                   350                 355                 360
   tac ttg acc atc acg caa caa atg act tat ccg gtc ttc caa gcc ctc      1675
   Tyr Leu Thr Ile Thr Gln Gln Met Thr Tyr Pro Val Phe Gln Ala Leu
               365                 370                 375
   cag agc gat cgt cgc ttg ggc tct gga ggg ttt gtg att cct gca gcc      1723
   Gln Ser Asp Arg Arg Leu Gly Ser Gly Gly Phe Val Ile Pro Ala Ala
           380                 385                 390
   acc tat gtg gtc ttc ttg atg acg gga atg act gtt ttc atc ata atc      1771
   Thr Tyr Val Val Phe Leu Met Thr Gly Met Thr Val Phe Ile Ile Ile
       395                 400                 405
   tac gac cgc gta ctc gtg cct acc ttg aga aga atc acc ggt ata gac      1819
   Tyr Asp Arg Val Leu Val Pro Thr Leu Arg Arg Ile Thr Gly Ile Asp
   410                 415                 420                 425
   acc ggg atc acg ctc ttg caa agg ata gga acc ggg att ttc ttc gcc      1867
   Thr Gly Ile Thr Leu Leu Gln Arg Ile Gly Thr Gly Ile Phe Phe Ala
                   430                 435                 440
   ttt ttg agc ttg gta gtg tct ggt ttc gtc gag gaa cgg agg agg acc      1915
   Phe Leu Ser Leu Val Val Ser Gly Phe Val Glu Glu Arg Arg Arg Thr
               445                 450                 455
   ttc gca ttg act aaa cca aca ctc ggt atg gcg cca cgg aag gga gaa      1963
   Phe Ala Leu Thr Lys Pro Thr Leu Gly Met Ala Pro Arg Lys Gly Glu
           460                 465                 470
   atc tcc tca atg tca gct atg tgg ctg ata ccg cag ctc aca ctc gcg      2011
   Ile Ser Ser Met Ser Ala Met Trp Leu Ile Pro Gln Leu Thr Leu Ala
       475                 480                 485
   ggt gta gcc gag gca ttt gga gct att gga cag atg gag ttt tac tac      2059
   Gly Val Ala Glu Ala Phe Gly Ala Ile Gly Gln Met Glu Phe Tyr Tyr
   490                 495                 500                 505
   aag cag ttc ccc gag aac atg agg agt ttc gcc ggc tcc atc ttc tat      2107
   Lys Gln Phe Pro Glu Asn Met Arg Ser Phe Ala Gly Ser Ile Phe Tyr
                   510                 515                 520
   gtg gga gga gga gtt tct agt tac ctc ggc agc ttc ttg att gca acg      2155
   Val Gly Gly Gly Val Ser Ser Tyr Leu Gly Ser Phe Leu Ile Ala Thr
               525                 530                 535
   gta cac agg acg acg cag aac tcg gct ggc ggt aac tgg ttg gct gaa      2203
   Val His Arg Thr Thr Gln Asn Ser Ala Gly Gly Asn Trp Leu Ala Glu
           540                 545                 550
   gat ttg aac aaa gga aga ttg gat ttg ttt tac ttc atg atc gct gga      2251
   Asp Leu Asn Lys Gly Arg Leu Asp Leu Phe Tyr Phe Met Ile Ala Gly
       555                 560                 565
   atc ttg gcg gtt aat ttt gct tac ttc ttg gtg atg tca aga tgg tat      2299
   Ile Leu Ala Val Asn Phe Ala Tyr Phe Leu Val Met Ser Arg Trp Tyr
   570                 575                 580                 585
   agg tac aaa gga agt gat gat gaa gtg aca agt tat gaa aca aat gga      2347
   Arg Tyr Lys Gly Ser Asp Asp Glu Val Thr Ser Tyr Glu Thr Asn Gly
                   590                 595                 600
   gat gtg atc aaa caa caa gac aag aac aaa gtc taa                      2383
   Asp Val Ile Lys Gln Gln Asp Lys Asn Lys Val
               605                 610
<210> 46
   <211> 612
   <212> PRT
   <213> Brassica rapa
<400> 46
   Met Glu Arg Lys Pro Leu Glu Val Glu Ser Thr Asp His Gln Lys Pro
   1               5                   10                  15
   Ser Ser Ala Val Tyr Asp Gly Ser Ala Thr Ala Val Asp Ser Val Glu
               20                  25                  30
   Glu Glu Val Gln Glu Thr Lys Leu Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Thr Ala Leu Thr Cys Thr Gly Pro Ser Gly Gly Gln Ile Ala Phe
   145                 150                 155                 160
   Leu Leu Met Gly Leu Ala Phe Leu Val Val Gly Ala Gly Gly Ile Arg
                   165                 170                 175
   Pro Cys Asn Leu Ala Phe Gly Ala Asp Gln Phe Asn Pro Lys Ser Glu
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Leu Val Val Tyr Ile Gln
       210                 215                 220
   Ser Asn Val Ser Trp Thr Ile Gly Leu Thr Ile Pro Val Val Leu Met
   225                 230                 235                 240
   Phe Leu Ala Cys Val Ile Phe Phe Ala Gly Asp Lys Leu Tyr Val Lys
                   245                 250                 255
   Ile Lys Ala Ser Gly Ser Pro Leu Ala Ser Ile Ala Gln Val Ile Thr
               260                 265                 270
   Val Ala Ile Lys Lys Arg Gly Leu Lys Ala Val Lys Gln Pro Trp Val
           275                 280                 285
   Asn Leu Tyr Asn Tyr Tyr Pro Leu Asn Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Thr Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ser Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Gly Gly Phe Val Ile Pro Ala Ala Thr Tyr Val Val Phe Leu Met
   385 390 395 400
   Thr Gly Met Thr Val Phe Ile Ile Ile Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Leu Ser Leu Val Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465 470 475 480
   Trp Leu Ile Pro Gln Leu Thr Leu Ala Gly Val Ala Glu Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Gly Gly Val Ser Ser
   515 520 525
   Tyr Leu Gly Ser Phe Leu Ile Ala Thr Val His Arg Thr Thr Gln Asn
   530 535 540
   Ser Ala Gly Gly Asn Trp Leu Ala Glu Asp Leu Asn Lys Gly Arg Leu
   545 550 555 560
   Asp Leu Phe Tyr Phe Met Ile Ala Gly Ile Leu Ala Val Asn Phe Ala
   565 570 575
   Tyr Phe Leu Val Met Ser Arg Trp Tyr Arg Tyr Lys Gly Ser Asp Asp
   580 585 590
   Glu Val Thr Ser Tyr Glu Thr Asn Gly Asp Val Ile Lys Gln Gln Asp
   595 600 605
   Lys Asn Lys Val
   610
<210> 47
   <211> 2304
   <212> DNA
   <213> Brassica rapa
<220>
   <221> CDS
   <222> (1)..(157)
<220>
   <221> CDS
   <222> (335)..(552)
<220>
   <221> CDS
   <222> (643)..(1196)
<220>
   <221> CDS
   <222> (1395)..(2301)
<400> 47
   atg gag aga aag tct cta gaa gtc gag tct atg gat cac caa aaa ctt       48
   Met Glu Arg Lys Ser Leu Glu Val Glu Ser Met Asp His Gln Lys Leu
   1               5                   10                  15
   tcc tcc gcc gtg tac gat ggc tct gtt acg gcc gtt gat tct gtt aaa       96
   Ser Ser Ala Val Tyr Asp Gly Ser Val Thr Ala Val Asp Ser Val Lys
               20                  25                  30
   gaa gat gtt cag gag acc aaa gtc gtt tat aga ggc tgg aaa gtc atg      144
   Glu Asp Val Gln Glu Thr Lys Val Val Tyr Arg Gly Trp Lys Val Met
           35                  40                  45
   cct tac atc att g gtaacgcaat aatattatca tttaccgtgc tctataaact        197
   Pro Tyr Ile Ile
       50
   ttcttctttt taatgttaaa aaaaactttc ttcttttgga aactatttct tttgtcgttt    257
   tgttgtctta agttttcact tagtcataat attgaagtct tatgatcttg gttaatcttt    317
   gttgttgtta ccaccag ga  aat gag aca ttt gag aag ctt ggg atc att       366
                      Gly Asn Glu Thr Phe Glu Lys Leu Gly Ile Ile
                              55                  60
   gga aca cta tca aac ctt ctg gtt tat tta act tct gtc ttc aac atg      414
   Gly Thr Leu Ser Asn Leu Leu Val Tyr Leu Thr Ser Val Phe Asn Met
       65                  70                  75
   aag agt gtc aca gct gca aca atc atc act gcc ttt gct ggc aca gtc      462
   Lys Ser Val Thr Ala Ala Thr Ile Ile Thr Ala Phe Ala Gly Thr Val
   80                  85                  90                  95
   aac ttc gga act ttc gtc gct gct ttc ctt tgc gac act tac ttc ggt      510
   Asn Phe Gly Thr Phe Val Ala Ala Phe Leu Cys Asp Thr Tyr Phe Gly
                   100                 105                 110
   cga tac aag act ctt acc gtt gcg gtc ctc gcc tgt ttt ctt              552
   Arg Tyr Lys Thr Leu Thr Val Ala Val Leu Ala Cys Phe Leu
               115                 120                 125
   gtaaagatcc atcccctcat aactctttct tcctctacag gcaatattgg tttataacct    612
   aaaaaaaaaa atctggttct tgttttgtag gga tca ctt gcg ata cta ttg act     666
                                           Gly Ser Leu Ala Ile Leu Leu Thr
                                                       130
   gct gca gtc cca caa cta cat cca gtc cca tgc ggc aca gcg agc tcg      714
   Ala Ala Val Pro Gln Leu His Pro Val Pro Cys Gly Thr Ala Ser Ser
       135                 140                 145
   tgt atc ggg cca agt ggt ggc cag ata gcg ttt cta ctg atg ggt ctt      762
   Cys Ile Gly Pro Ser Gly Gly Gln Ile Ala Phe Leu Leu Met Gly Leu
   150                 155                 160                 165
   gga att ctt gtc gtg gga gca gga ggg att aga ccg tgc aat cta gct      810
   Gly Ile Leu Val Val Gly Ala Gly Gly Ile Arg Pro Cys Asn Leu Ala
                   170                 175                 180
   ttc gga gct gat cag ttt aac ccg aag agc ggg tca ggg aag aga ggg      858
   Phe Gly Ala Asp Gln Phe Asn Pro Lys Ser Gly Ser Gly Lys Arg Gly
               185                 190                 195
   att gat agt ttc ttc aac tgg tac ttc ttc acc ttc act ttc gcc cag      906
   Ile Asp Ser Phe Phe Asn Trp Tyr Phe Phe Thr Phe Thr Phe Ala Gln
           200                 205                 210
   atc ttg tct ctg aca gta gtt gtc tat gtc cag tct aat gtc agt tgg      954
   Ile Leu Ser Leu Thr Val Val Val Tyr Val Gln Ser Asn Val Ser Trp
       215                 220                 225
   acc atc ggt tta acc atc ccg gtt gtt cta atg ttc ttg gcc tcc gtg     1002
   Thr Ile Gly Leu Thr Ile Pro Val Val Leu Met Phe Leu Ala Ser Val
   230                 235                 240                 245
   att ttc ttt gcg ggt gct aag ttg tat gtt aag atc gaa gcc tca ggt     1050
   Ile Phe Phe Ala Gly Ala Lys Leu Tyr Val Lys Ile Glu Ala Ser Gly
                   250                 255                 260
   agt cca ttg gct agc ata gct cat gtt ata acg gct tct atc aag aaa     1098
   Ser Pro Leu Ala Ser Ile Ala His Val Ile Thr Ala Ser Ile Lys Lys
               265                 270                 275
   cac agg tta gag cca gtg gaa cag cct tgg ctg aac ctt tac aac tac     1146
   His Arg Leu Glu Pro Val Glu Gln Pro Trp Leu Asn Leu Tyr Asn Tyr
           280                 285                 290
   tac cca tca aaa tac gct aac gcc aag ctc aaa tac acc caa caa ttc     1194
   Tyr Pro Ser Lys Tyr Ala Asn Ala Lys Leu Lys Tyr Thr Gln Gln Phe
       295                 300                 305
   ag  gtaaacacac acttttaaac cggtaaaaaa acaccgttat atatatatat           1246
   Arg
   attttttttt tttttgagtg aatgaatgtt aaaattattc atatcaaaac ttttttacaa    1306
   tgactgcatc tatagttgat atatatatac ttatacaatg tctttttttg atgctttatg   1366
   tttactgaga gagattctta ctcggcag g ttt ctc gac aag gcg gcg atc ttg     1419
                                    Phe Leu Asp Lys Ala Ala Ile Leu
                                                315
   act cct gaa gac aag ttg gag gct gat ggt aag cct gtg aat ccg tgg     1467
   Thr Pro Glu Asp Lys Leu Glu Ala Asp Gly Lys Pro Val Asn Pro Trp
       320                 325                 330
   aat cta tgt aca atg caa caa gtt gaa gaa gtg aag tgc atc atg aga     1515
   Asn Leu Cys Thr Met Gln Gln Val Glu Glu Val Lys Cys Ile Met Arg
   335                 340                 345                 350
   gtg ctt cct ata tgg ttt gct tca tcg atc tac tac tta acc atg gcg     1563
   Val Leu Pro Ile Trp Phe Ala Ser Ser Ile Tyr Tyr Leu Thr Met Ala
                   355                 360                 365
   caa caa atg act tat cct gtc ttc caa gct ctc cag agt gat cgt cgc     1611
   Gln Gln Met Thr Tyr Pro Val Phe Gln Ala Leu Gln Ser Asp Arg Arg
               370                 375                 380
   cta ggc tcc gga ggt ttc atg atc ccg gca gcc acc tat gtt gtc ttc     1659
   Leu Gly Ser Gly Gly Phe Met Ile Pro Ala Ala Thr Tyr Val Val Phe
           385                 390                 395
   ttg atg act ggc atg acc ctc ttc atc ata ttt tat gac cgt gtc ttg      1707
   Leu Met Thr Gly Met Thr Leu Phe Ile Ile Phe Tyr Asp Arg Val Leu
       400                 405                 410
   gtt cct aca tta aaa aga ata acc ggt ata gag acg ggt ata acg ctc      1755
   Val Pro Thr Leu Lys Arg Ile Thr Gly Ile Glu Thr Gly Ile Thr Leu
   415                 420                 425                 430
   ttg caa aga att gga gcc ggg att ttt ttc gcc ttt tta agc tta att      1803
   Leu Gln Arg Ile Gly Ala Gly Ile Phe Phe Ala Phe Leu Ser Leu Ile
                   435                 440                 445
   gtc tct ggt ttc gtc gag gaa cgt agg aga act tta gca cta act caa      1851
   Val Ser Gly Phe Val Glu Glu Arg Arg Arg Thr Leu Ala Leu Thr Gln
               450                 455                 460
   ccg aca ctc ggt tta gcg cca cgg aag gga gaa atc tcc tca atg tcg      1899
   Pro Thr Leu Gly Leu Ala Pro Arg Lys Gly Glu Ile Ser Ser Met Ser
           465                 470                 475
   gct atg tgg ctg att ccg cag ctt gta ctt gct ggt ata gcc gac gcg      1947
   Ala Met Trp Leu Ile Pro Gln Leu Val Leu Ala Gly Ile Ala Asp Ala
       480                 485                 490
   ttt gga gct att gga cag atg gag ttt tac tac aag cag ttc cct gag      1995
   Phe Gly Ala Ile Gly Gln Met Glu Phe Tyr Tyr Lys Gln Phe Pro Glu
   495                 500                 505                 510
   aac atg agg agt ttt gct ggt tct atc ttt tac gta gga gca gga gtt      2043
   Asn Met Arg Ser Phe Ala Gly Ser Ile Phe Tyr Val Gly Ala Gly Val
                   515                 520                 525
   tcg agt tac atc agt agc ttc ttg atc aca acg gtt cac agg atg acg      2091
   Ser Ser Tyr Ile Ser Ser Phe Leu Ile Thr Thr Val His Arg Met Thr
               530                 535                 540
   cag aac tcg gcg ggt ggt aat tgg tta gct gag gat ttg aac aaa ggg      2139
   Gln Asn Ser Ala Gly Gly Asn Trp Leu Ala Glu Asp Leu Asn Lys Gly
           545                 550                 555
   aga ttg gat tac ttc tat ttc ttg tta gct gga atc ttg gca gtt aat      2187
   Arg Leu Asp Tyr Phe Tyr Phe Leu Leu Ala Gly Ile Leu Ala Val Asn
       560                 565                 570
   ttt gct tac ttc ttg gtg atg gca aga tgg tat agg tac aaa gga agt      2235
   Phe Ala Tyr Phe Leu Val Met Ala Arg Trp Tyr Arg Tyr Lys Gly Ser
   575                 580                 585                 590
   gat gat gaa gag aca agt tat gaa acc agt gga gat atc atc aaa gaa      2283
   Asp Asp Glu Glu Thr Ser Tyr Glu Thr Ser Gly Asp Ile Ile Lys Glu
                   595                 600                 605
   caa gac aag aac aaa gtc tga                                          2304
   Gln Asp Lys Asn Lys Val
               610
<210> 48
   <211> 612
   <212> PRT
   <213> Brassica rapa
<400> 48
   Met Glu Arg Lys Ser Leu Glu Val Glu Ser Met Asp His Gln Lys Leu
   1               5                   10                  15
   Ser Ser Ala Val Tyr Asp Gly Ser Val Thr Ala Val Asp Ser Val Lys
               20                  25                  30
   Glu Asp Val Gln Glu Thr Lys Val Val Tyr Arg Gly Trp Lys Val Met
               35                  40                  45
   Pro Tyr Ile Ile Gly Asn Glu Thr Phe Glu Lys Leu Gly Ile Ile Gly
       50                  55                  60
   Thr Leu Ser Asn Leu Leu Val Tyr Leu Thr Ser Val Phe Asn Met Lys
   65                  70                  75                  80
   Ser Val Thr Ala Ala Thr Ile Ile Thr Ala Phe Ala Gly Thr Val Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Leu Ala Cys Phe Leu Gly Ser Leu
           115                 120                 125
   Ala Ile Leu Leu Thr Ala Ala Val Pro Gln Leu His Pro Val Pro Cys
       130                 135                 140
   Gly Thr Ala Ser Ser Cys Ile Gly Pro Ser Gly Gly Gln Ile Ala Phe
   145                 150                 155                 160
   Leu Leu Met Gly Leu Gly Ile Leu Val Val Gly Ala Gly Gly Ile Arg
                   165                 170                 175
   Pro Cys Asn Leu Ala Phe Gly Ala Asp Gln Phe Asn Pro Lys Ser Gly
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Val Val Val Tyr Val Gln
       210                 215                 220
   Ser Asn Val Ser Trp Thr Ile Gly Leu Thr Ile Pro Val Val Leu Met
   225                 230                 235                 240
   Phe Leu Ala Ser Val Ile Phe Phe Ala Gly Ala Lys Leu Tyr Val Lys
                   245                 250                 255
   Ile Glu Ala Ser Gly Ser Pro Leu Ala Ser Ile Ala His Val Ile Thr
               260                 265                 270
   Ala Ser Ile Lys Lys His Arg Leu Glu Pro Val Glu Gln Pro Trp Leu
           275                 280                 285
   Asn Leu Tyr Asn Tyr Tyr Pro Ser Lys Tyr Ala Asn Ala Lys Leu Lys
       290                 295                 300
   Tyr Thr Gln Gln Phe Arg Phe Leu Asp Lys Ala Ala Ile Leu Thr Pro
   305                 310                 315                 320
   Glu Asp Lys Leu Glu Ala Asp Gly Lys Pro Val Asn Pro Trp Asn Leu
                   325                 330                 335
   Cys Thr Met Gln Gln Val Glu Glu Val Lys Cys Ile Met Arg Val Leu
                   340                 345                 350
   Pro Ile Trp Phe Ala Ser Ser Ile Tyr Tyr Leu Thr Met Ala Gln Gln
           355                 360                 365
   Met Thr Tyr Pro Val Phe Gln Ala Leu Gln Ser Asp Arg Arg Leu Gly
       370                 375                 380
   Ser Gly Gly Phe Met Ile Pro Ala Ala Thr Tyr Val Val Phe Leu Met
   385                 390                 395                 400
   Thr Gly Met Thr Leu Phe Ile Ile Phe Tyr Asp Arg Val Leu Val Pro
                   405                 410                 415
   Thr Leu Lys Arg Ile Thr Gly Ile Glu Thr Gly Ile Thr Leu Leu Gln
               420                 425                 430
   Arg Ile Gly Ala Gly Ile Phe Phe Ala Phe Leu Ser Leu Ile Val Ser
           435                 440                 445
   Gly Phe Val Glu Glu Arg Arg Arg Thr Leu Ala Leu Thr Gln Pro Thr
       450                 455                 460
   Leu Gly Leu Ala Pro Arg Lys Gly Glu Ile Ser Ser Met Ser Ala Met
   465                 470                 475                 480
   Trp Leu Ile Pro Gln Leu Val Leu Ala Gly Ile Ala Asp Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Ala Gly Val Ser Ser
           515                 520                 525
   Tyr Ile Ser Ser Phe Leu Ile Thr Thr Val His Arg Met Thr Gln Asn
       530                 535                 540
   Ser Ala Gly Gly Asn Trp Leu Ala Glu Asp Leu Asn Lys Gly Arg Leu
   545                 550                 555                 560
   Asp Tyr Phe Tyr Phe Leu Leu Ala Gly Ile Leu Ala Val Asn Phe Ala
                   565                 570                 575
   Tyr Phe Leu Val Met Ala Arg Trp Tyr Arg Tyr Lys Gly Ser Asp Asp
               580                 585                 590
   Glu Glu Thr Ser Tyr Glu Thr Ser Gly Asp Ile Ile Lys Glu Gln Asp
           595                 600                 605
   Lys Asn Lys Val
       610
<210> 49
   <211> 2829
   <212> DNA
   <213> Brassica oleracea
<220>
   <221> CDS
   <222> (1)..(151)
<220>
   <221> CDS
   <222> (619)..(836)
<220>
   <221> CDS
   <222> (1283)..(1836)
<220>
   <221> CDS
   <222> (1917)..(2826)
<400> 49
   atg gag aga aag ccc ttt gat gtt gag aca act gag gat cac aaa ccc      48
   Met Glu Arg Lys Pro Phe Asp Val Glu Thr Thr Glu Asp His Lys Pro
   1               5                   10                  15
   tac tcc tcc gtc gat ggc ggt ggt tct gat tcg gtc gat tca ttt gaa      96
   Tyr Ser Ser Val Asp Gly Gly Gly Ser Asp Ser Val Asp Ser Phe Glu
               20                  25                  30
   gac gag cag aaa aag ctc gtt tat aga ggc tgg aaa gtc atg ccc ttt     144
   Asp Glu Gln Lys Lys Leu Val Tyr Arg Gly Trp Lys Val Met Pro Phe
           35                  40                  45
   atc att g gtaacgcttt cttcttctct tcagacaact ttttttttta gtttattctg    201
   Ile Ile
       50
   ttttgttttg ttgtgtctct tttgtcggat tatagaaagt tttgcccgtg atgtttttat   261
   tttaatttcg tttcttttgt cttcttagtc ttgactgttg aaaatcttga tttggagttc   321
   ttgtcttgac gtttcgagat gaatgaaaca cagatcaaac aaatcaacca aaccagttga   381
   gtttcctaga taaatctacc atggaaagtg agagacttcg aatcatgcaa gttttcagtt   441
   tcagatataa gattttagtt agtttctggg agttatagca aagaatgaat aataaagtgt   501
   ccagtccaac actgaaagtc caatctttga tttcaacatt gtgaattaaa tgtaaaaagg   561
   ttgttacttt atggtttgac taattattta tgttgaaaaa aatatattgt gtcacag      618
   gt  aat gag aca ttt gag aag ata ggg atc att ggg aca cta tca aac     665
   Gly Asn Glu Thr Phe Glu Lys Ile Gly Ile Ile Gly Thr Leu Ser Asn
                   55                  60                  65
   ctt ctg gtg tac cta act tca gta ttc aac ctt cag agt gtt aca gct     713
   Leu Leu Val Tyr Leu Thr Ser Val Phe Asn Leu Gln Ser Val Thr Ala
               70                  75                  80
   gca acc atc atc aac gcc ttc agt ggc act atc aat ttc ggc act ttc     761
   Ala Thr Ile Ile Asn Ala Phe Ser Gly Thr Ile Asn Phe Gly Thr Phe
           85                  90                  95
   ctc gct gct ttc ctc tgc gac act tac ttt ggt cgc tac aag act ctc     809
   Leu Ala Ala Phe Leu Cys Asp Thr Tyr Phe Gly Arg Tyr Lys Thr Leu
       100                 105                 110
   tct gtt gct gtc atc gcc tgt ttt ctg gtactctaat caaatgacca           856
   Ser Val Ala Val Ile Ala Cys Phe Leu
   115                 120
   tcttgtttta ttttggccaa gattttgtct ttttttctta ctaaaccata ctccctccgt   916
   ttcattttaa gtgtcgtttt agagattttt ttcgttacaa aataagtgtc gttttcgatt   976
   ttcaatgcaa aatttattga ttttatgcaa aatttattga ttttatgcaa aatttattga  1036
   ttttatgcaa aatttattga ttttatgcaa aatttattga ttttatgcaa aatttattga  1096
   ttttatgcaa aatttattga ttttatgcaa aatttatttt tctattggtt gaaatatggt     1156
   taggtgtata gttaatagtg tttttttata cgaaatgtac aaaattaagt gtttccttaa      1216
   tccgtgtgcc gaaacctaaa acgacactta taatgaaaca gagggagtat ttcatatttg     1276
   ttgtag gga tcg ctt gtg ata cta ctg acg gct gca gtt cca gga ttg       1324
          Gly Ser Leu Val Ile Leu Leu Thr Ala Ala Val Pro Gly Leu
              125                 130                 135
   cac cct att cct tgt gga aca caa agt tct tgc caa ggc cca agc gag      1372
   His Pro Ile Pro Cys Gly Thr Gln Ser Ser Cys Gln Gly Pro Ser Glu
           140                 145                 150
   ggc cag att gcg ttt ctg ctg atg ggt tta gcg ctt ctt gtg gtg ggt      1420
   Gly Gln Ile Ala Phe Leu Leu Met Gly Leu Ala Leu Leu Val Val Gly
       155                 160                 165
   gct ggt gga atc agg ccg tgt aac ttg gcc ttt ggt gct gac cag ttc      1468
   Ala Gly Gly Ile Arg Pro Cys Asn Leu Ala Phe Gly Ala Asp Gln Phe
   170                 175                 180                 185
   aac ccc aag tca gaa tct gga aag aaa gga atc aac agt ttc ttc aac      1516
   Asn Pro Lys Ser Glu Ser Gly Lys Lys Gly Ile Asn Ser Phe Phe Asn
                   190                 195                 200
   tgg tac ttc ttc act ttc acg ttc gcg cag atc atc tcc ctc acg cta      1564
   Trp Tyr Phe Phe Thr Phe Thr Phe Ala Gln Ile Ile Ser Leu Thr Leu
               205                 210                 215
   gtt gtg tat atc cag tca aat gtg agc tgg acc atc ggt ttg agt atc      1612
   Val Val Tyr Ile Gln Ser Asn Val Ser Trp Thr Ile Gly Leu Ser Ile
           220                 225                 230
   cct gtg ggt ctt atg ttc ttg gcc tgc gtc att ttc ttt gct ggt cac      1660
   Pro Val Gly Leu Met Phe Leu Ala Cys Val Ile Phe Phe Ala Gly His
       235                 240                 245
   aaa ctg tat gtg aaa gtg aaa gcc tct ggt agt cca ttg gct ggt ata      1708
   Lys Leu Tyr Val Lys Val Lys Ala Ser Gly Ser Pro Leu Ala Gly Ile
   250                 255                 260                 265
   ggt cat gtt ata gcc gca gct atc aag aaa cgt ggc ttg aag cca gtt      1756
   Gly His Val Ile Ala Ala Ala Ile Lys Lys Arg Gly Leu Lys Pro Val
                   270                 275                 280
   aag cag cct tgg atc gat ctt tac aac cac att cct cct aac tat gca      1804
   Lys Gln Pro Trp Ile Asp Leu Tyr Asn His Ile Pro Pro Asn Tyr Ala
               285                 290                 295
   aac tct act ctt aaa tac acc gac cag ctt ag  gtaaagtcgg aatagttctg    1856
   Asn Ser Thr Leu Lys Tyr Thr Asp Gln Leu Arg
           300                 305
   tctctgtctt tctttgttca tttgctttat gctttgtctg aacaaatgtt gtattgccag     1916
   g ttt cta gac aaa gca gcg att atg acc cct gag gac aag ttg aac tcg    1965
     Phe Leu Asp Lys Ala Ala Ile Met Thr Pro Glu Asp Lys Leu Asn Ser
         310                 315                 320
   gat gga acg gct tcg gat cca tgg aaa tta tgt acg atg cag aaa gtg      2013
   Asp Gly Thr Ala Ser Asp Pro Trp Lys Leu Cys Thr Met Gln Lys Val
   325                 330                 335                 340
   gaa gaa gtg aaa tgc att tta aga gtg att cca atc tgg ttc gca tgc      2061
   Glu Glu Val Lys Cys Ile Leu Arg Val Ile Pro Ile Trp Phe Ala Cys
                   345                 350                 355
   gcc att tac tac ctt gca ata agt atg caa atg act tat cca gtc ttc      2109
   Ala Ile Tyr Tyr Leu Ala Ile Ser Met Gln Met Thr Tyr Pro Val Phe
               360                 365                 370
   caa gct ctc cag agc gac agg cta tta ggt tca ggt ggc ttc aag att      2157
   Gln Ala Leu Gln Ser Asp Arg Leu Leu Gly Ser Gly Gly Phe Lys Ile
           375                 380                 385
   cca gcc gcc acc tat gtc gtg ttc ttg atg tct ggt atg acg gtt ttc      2205
   Pro Ala Ala Thr Tyr Val Val Phe Leu Met Ser Gly Met Thr Val Phe
       390                 395                 400
   atc ata atc tac gac cgt gtc ctt gtc ccc tcg ctc aaa aga gtg acc      2253
   Ile Ile Ile Tyr Asp Arg Val Leu Val Pro Ser Leu Lys Arg Val Thr
   405                 410                 415                 420
   ggt cta gac acg ggg ata aca ctc tta caa aga ata gga gct ggc att      2301
   Gly Leu Asp Thr Gly Ile Thr Leu Leu Gln Arg Ile Gly Ala Gly Ile
                   425                 430                 435
   ttc ttt gcc gtg ttg agt tta ttg gtc tcc ggg ttt ata gag gaa cgt      2349
   Phe Phe Ala Val Leu Ser Leu Leu Val Ser Gly Phe Ile Glu Glu Arg
               440                 445                 450
   aga aga acc ttt gcg ctg aca cga cct acg ctc ggg tcg gag cct cga      2397
   Arg Arg Thr Phe Ala Leu Thr Arg Pro Thr Leu Gly Ser Glu Pro Arg
           455                 460                 465
   aca gga gag atc tcc tca atg tca gcg atg tgg ctg att ccg cag ctg      2445
   Thr Gly Glu Ile Ser Ser Met Ser Ala Met Trp Leu Ile Pro Gln Leu
       470                 475                 480
   gtg ctt gca ggg gta gca gaa gct ttt gca gcc att ggg caa atg gag      2493
   Val Leu Ala Gly Val Ala Glu Ala Phe Ala Ala Ile Gly Gln Met Glu
   485                 490                 495                 500
   ttt tac tac aag cag ttt cct gaa aac atg aag agc ttt gcg ggt tct      2541
   Phe Tyr Tyr Lys Gln Phe Pro Glu Asn Met Lys Ser Phe Ala Gly Ser
                   505                 510                 515
   atc ttc tat gtc ggt gga gga gtt tct agc tac ctc gct agc ttc ttg      2589
   Ile Phe Tyr Val Gly Gly Gly Val Ser Ser Tyr Leu Ala Ser Phe Leu
               520                 525                 530
   atc tcg act gtt cat aga aca act gag cat tca cct acg ggg aat tgg      2637
   Ile Ser Thr Val His Arg Thr Thr Glu His Ser Pro Thr Gly Asn Trp
           535                 540                 545
   tta gct gag gat ctg aac aaa gcg aag ctg gac tac ttc tat ttc atg      2685
   Leu Ala Glu Asp Leu Asn Lys Ala Lys Leu Asp Tyr Phe Tyr Phe Met
       550                 555                 560
   ctc aca gga ctc atg ttt gtc aac ttt ctt tac ttc ttg tta atg gct      2733
   Leu Thr Gly Leu Met Phe Val Asn Phe Leu Tyr Phe Leu Leu Met Ala
   565                 570                 575                 580
   aga tgg tat aga tac aaa ggc act cat gat gaa gtc agt tat gtg gtt      2781
   Arg Trp Tyr Arg Tyr Lys Gly Thr His Asp Glu Val Ser Tyr Val Val
                   585                 590                 595
   gac act gtt gac gaa gag acg aag caa caa gac aag aac tct gtc tga      2829
   Asp Thr Val Asp Glu Glu Thr Lys Gln Gln Asp Lys Asn Ser Val
               600                 605                 610
<210> 50
   <211> 611
   <212> PRT
   <213> Brassica oleracea
<400> 50
   Met Glu Arg Lys Pro Phe Asp Val Glu Thr Thr Glu Asp His Lys Pro
   1               5                   10                  15
   Tyr Ser Ser Val Asp Gly Gly Gly Ser Asp Ser Val Asp Ser Phe Glu
               20                  25                  30
   Asp Glu Gln Lys Lys Leu Val Tyr Arg Gly Trp Lys Val Met Pro Phe
           35                  40                  45
   Ile Ile Gly Asn Glu Thr Phe Glu Lys Ile Gly Ile Ile Gly Thr Leu
   50 55 60
   Ser Asn Leu Leu Val Tyr Leu Thr Ser Val Phe Asn Leu Gln Ser Val
   65 70 75 80
   Thr Ala Ala Thr Ile Ile Asn Ala Phe Ser Gly Thr Ile Asn Phe Gly
   85 90 95
   Thr Phe Leu Ala Ala Phe Leu Cys Asp Thr Tyr Phe Gly Arg Tyr Lys
   100 105 110
   Thr Leu Ser Val Ala Val Ile Ala Cys Phe Leu Gly Ser Leu Val Ile
   115 120 125
   Leu Leu Thr Ala Ala Val Pro Gly Leu His Pro Ile Pro Cys Gly Thr
   130 135 140
   Gln Ser Ser Cys Gln Gly Pro Ser Glu Gly Gln Ile Ala Phe Leu Leu
   145 150 155 160
   Met Gly Leu Ala Leu Leu Val Val Gly Ala Gly Gly Ile Arg Pro Cys
   165 170 175
   Asn Leu Ala Phe Gly Ala Asp Gln Phe Asn Pro Lys Ser Glu Ser Gly
   180 185 190
   Lys Lys Gly Ile Asn Ser Phe Phe Asn Trp Tyr Phe Phe Thr Phe Thr
   195 200 205
   Phe Ala Gln Ile Ile Ser Leu Thr Leu Val Val Tyr Ile Gln Ser Asn
   210 215 220
   Val Ser Trp Thr Ile Gly Leu Ser Ile Pro Val Gly Leu Met Phe Leu
   225 230 235 240
   Ala Cys Val Ile Phe Phe Ala Gly His Lys Leu Tyr Val Lys Val Lys
   245 250 255
   Ala Ser Gly Ser Pro Leu Ala Gly Ile Gly His Val Ile Ala Ala Ala
   260 265 270
   Ile Lys Lys Arg Gly Leu Lys Pro Val Lys Gln Pro Trp Ile Asp Leu
   275 280 285
   Tyr Asn His Ile Pro Pro Asn Tyr Ala Asn Ser Thr Leu Lys Tyr Thr
   290 295 300
   Asp Gln Leu Arg Phe Leu Asp Lys Ala Ala Ile Met Thr Pro Glu Asp
   305 310 315 320
   Lys Leu Asn Ser Asp Gly Thr Ala Ser Asp Pro Trp Lys Leu Cys Thr
   325 330 335
   Met Gln Lys Val Glu Glu Val Lys Cys Ile Leu Arg Val Ile Pro Ile
   340 345 350
   Trp Phe Ala Cys Ala Ile Tyr Tyr Leu Ala Ile Ser Met Gln Met Thr
           355                 360                 365
   Tyr Pro Val Phe Gln Ala Leu Gln Ser Asp Arg Leu Leu Gly Ser Gly
       370                 375                 380
   Gly Phe Lys Ile Pro Ala Ala Thr Tyr Val Val Phe Leu Met Ser Gly
   385                 390                 395                 400
   Met Thr Val Phe Ile Ile Ile Tyr Asp Arg Val Leu Val Pro Ser Leu
                   405                 410                 415
   Lys Arg Val Thr Gly Leu Asp Thr Gly Ile Thr Leu Leu Gln Arg Ile
               420                 425                 430
   Gly Ala Gly Ile Phe Phe Ala Val Leu Ser Leu Leu Val Ser Gly Phe
           435                 440                 445
   Ile Glu Glu Arg Arg Arg Thr Phe Ala Leu Thr Arg Pro Thr Leu Gly
       450                 455                 460
   Ser Glu Pro Arg Thr Gly Glu Ile Ser Ser Met Ser Ala Met Trp Leu
   465                 470                 475                 480
   Ile Pro Gln Leu Val Leu Ala Gly Val Ala Glu Ala Phe Ala Ala Ile
                   485                 490                 495
   Gly Gln Met Glu Phe Tyr Tyr Lys Gln Phe Pro Glu Asn Met Lys Ser
               500                 505                 510
   Phe Ala Gly Ser Ile Phe Tyr Val Gly Gly Gly Val Ser Ser Tyr Leu
           515                 520                 525
   Ala Ser Phe Leu Ile Ser Thr Val His Arg Thr Thr Glu His Ser Pro
       530                 535                 540
   Thr Gly Asn Trp Leu Ala Glu Asp Leu Asn Lys Ala Lys Leu Asp Tyr
   545                 550                 555                 560
   Phe Tyr Phe Met Leu Thr Gly Leu Met Phe Val Asn Phe Leu Tyr Phe
                   565                 570                 575
   Leu Leu Met Ala Arg Trp Tyr Arg Tyr Lys Gly Thr His Asp Glu Val
               580                 585                 590
   Ser Tyr Val Val Asp Thr Val Asp Glu Glu Thr Lys Gln Gln Asp Lys
           595                 600                 605
   Asn Ser Val
       610
<210> 51
   <211> 2623
   <212> DNA
   <213> Brassica oleracea
<220>
   <221> CDS
   <222> (1)..(160)
<220>
   <221> CDS
   <222> (638)..(855)
<220>
   <221> CDS
   <222> (955)..(1508)
<220>
   <221> CDS
   <222> (1708)..(2620)
<400> 51
   atg gaa aga aag cct ttt gag gtt gag aca aca aag acg gat gag gat      48
   Met Glu Arg Lys Pro Phe Glu Val Glu Thr Thr Lys Thr Asp Glu Asp
   1               5                   10                  15
   cag aaa ccc tac tcc gcc gtc gat ggc ggt ggt tct gat tcg gtc gat      96
   Gln Lys Pro Tyr Ser Ala Val Asp Gly Gly Gly Ser Asp Ser Val Asp
               20                  25                  30
   tca ttt gat gaa gat cag aaa aag ctc gtg tat aga gga tgg aaa gtc      144
   Ser Phe Asp Glu Asp Gln Lys Lys Leu Val Tyr Arg Gly Trp Lys Val
           35                  40                  45
   atg cct ttt atc att g gtaacgcttt ttttttttct ctattcagac actccgtttt      200
   Met Pro Phe Ile Ile
       50
   tttttttatt gtgcgtctgc ttttctcaac acggtccaga aactaaataa agatgtgaaa    260
   ataaactaga acatgtcgtc cgtgaatttt aaaagtactt ttcttttctc ttcttctctt    320
   tgactgaaaa cgaatgaaac caggatcaac caaatctacc agatctagag aaagagatat    380
   tacaactttt ttcattaata atgcttttta atttttagtt ttatacgctt aaagacaaag    440
   ttatttagtt tctgggagtt acagtaaagt ttcaagttaa agaagcactg aaagtacaat    500
   cttcgattcg tgtgtgtttc ggtttaaaag ttgtgaatta aaaagtttga tactttatgg    560
   gaaacccgtt ttttctttga caactgattc atttattgtg aaaaagaaat taataatgat    620
   tcttgttttt tttatag gt  aat gaa aca ttt gag aag att ggg atc att       669
                      Gly Asn Glu Thr Phe Glu Lys Ile Gly Ile Ile
                          55                  60
   ggg aca tta tca aac ctt ctc gtc tac cta act caa gta ttc aac ctc      717
   Gly Thr Leu Ser Asn Leu Leu Val Tyr Leu Thr Gln Val Phe Asn Leu
   65                  70                  75                  80
   aag aaa tac aca gct gca acc atc atc aat gcc ttt agt ggc acc atc      765
   Lys Lys Tyr Thr Ala Ala Thr Ile Ile Asn Ala Phe Ser Gly Thr Ile
                   85                  90                  95
   aac ttc ggc act ttc ctc gct gct ttc ctc tgc gac act tac ttt ggt      813
   Asn Phe Gly Thr Phe Leu Ala Ala Phe Leu Cys Asp Thr Tyr Phe Gly
               100                 105                 110
   cgc tac aag act ctc agt gtt gct gtc gtc gct tgt ttt ctg              855
   Arg Tyr Lys Thr Leu Ser Val Ala Val Val Ala Cys Phe Leu
           115                 120                 125
   gtaactacta atgctgttct taacggttta gttttcgtca agactctatt cttttttgtt     915
   tttttttgtt tttgctgaac cgtgttttat ctcttgtag gga tcg ctt gcg ata       969
                                              Gly Ser Leu Ala Ile
                                                          130
   cta ctc acg gca gca gtt ccg gga ctg cac ccg act cct tgt gga aca      1017
   Leu Leu Thr Ala Ala Val Pro Gly Leu His Pro Thr Pro Cys Gly Thr
               135                 140                 145
   aaa agt tca tgc gaa ggg cca ggt gcg ggc cag att ttg ttt ctg atg      1065
   Lys Ser Ser Cys Glu Gly Pro Gly Ala Gly Gln Ile Leu Phe Leu Met
           150                 155                 160
   ttg ggt tta gcg ctt ctt gtg gtt ggt gct ggt ggt atc agg ccg tgt      1113
   Leu Gly Leu Ala Leu Leu Val Val Gly Ala Gly Gly Ile Arg Pro Cys
       165                 170                 175
   aac ttg gcg ttc ggt gcc gat cag ttc aac ccc aag tcc gaa tca gga      1161
   Asn Leu Ala Phe Gly Ala Asp Gln Phe Asn Pro Lys Ser Glu Ser Gly
   180                 185                 190                 195
   aag aaa gga atc aac agt ttc ttc aac tgg tac ttc ttc act ttc acg      1209
   Lys Lys Gly Ile Asn Ser Phe Phe Asn Trp Tyr Phe Phe Thr Phe Thr
                   200                 205                 210
   ttc gcg cag atc atc tcg ctc acg cta gtt gtg tat atc cag tcg aat      1257
   Phe Ala Gln Ile Ile Ser Leu Thr Leu Val Val Tyr Ile Gln Ser Asn
               215                 220                 225
   gtg agc tgg acc atc ggt ttg agt atc ccc gtg ggt ctc atg ttc ttg      1305
   Val Ser Trp Thr Ile Gly Leu Ser Ile Pro Val Gly Leu Met Phe Leu
           230                 235                 240
   gct tgc gtc att ttc ttc gct ggt cat aaa ctt tat gtt aaa gtg aaa      1353
   Ala Cys Val Ile Phe Phe Ala Gly His Lys Leu Tyr Val Lys Val Lys
       245                 250                 255
   gcc tcg ggc agt ccc ttg gct ggt ata gct cgt gtt ata gca gca gcg      1401
   Ala Ser Gly Ser Pro Leu Ala Gly Ile Ala Arg Val Ile Ala Ala Ala
   260                 265                 270                 275
   atc tcg aaa cga gga ttg aag ccg gtc aag cag cct tgg gtc gat ctt      1449
   Ile Ser Lys Arg Gly Leu Lys Pro Val Lys Gln Pro Trp Val Asp Leu
                   280                 285                 290
   tac aat cac att cct cgt aac tat gca aac ttg acc cta aaa tac acc      1497
   Tyr Asn His Ile Pro Arg Asn Tyr Ala Asn Leu Thr Leu Lys Tyr Thr
               295                 300                 305
   gac cag ttc ag  gtatactcaa tagttctgtc tctattgctt tatatccgga          1548
   Asp Gln Phe Arg
           310
   tgaaacattc gcctttactt ctaaatattt ttaaaatggg tcgtatagac cccaagttgt    1608
   caaatttgtt taccaaaata tatatttttt atttaccaaa atatttttaa aatgggtccg    1668
   gttggaaact aaatgttggt ttgttcgtta tattgttag g ttt cta gac aaa gca     1723
                                                Phe Leu Asp Lys Ala
                                                            315
   gcg ata atg aca cct gag gac aag ttg aac tct gat gga aca gct tcg      1771
   Ala Ile Met Thr Pro Glu Asp Lys Leu Asn Ser Asp Gly Thr Ala Ser
               320                 325                 330
   gat cca tgg aaa cta tgt acg atg cag caa gtg gaa gaa gtg aaa tgc      1819
   Asp Pro Trp Lys Leu Cys Thr Met Gln Gln Val Glu Glu Val Lys Cys
           335                 340                 345
   att ata aga gtg att cca atc tgg ttt gct tgc gct atc tac tac ctt      1867
   Ile Ile Arg Val Ile Pro Ile Trp Phe Ala Cys Ala Ile Tyr Tyr Leu
       350                 355                 360
   gca ata agc att caa atg aca tat ccc gtc ttc caa gcg caa cag agc      1915
   Ala Ile Ser Ile Gln Met Thr Tyr Pro Val Phe Gln Ala Gln Gln Ser
   365                 370                 375                 380
   gac agg aga tta ggt acc ggt ggc ttc aag atg ccc gca gcc acc tac      1963
   Asp Arg Arg Leu Gly Thr Gly Gly Phe Lys Met Pro Ala Ala Thr Tyr
                   385                 390                 395
   gta gtg ttc ttg atg acc ggt atg acg gtt ttc atc ata atc tac gac      2011
   Val Val Phe Leu Met Thr Gly Met Thr Val Phe Ile Ile Ile Tyr Asp
               400                 405                 410
   cgt gtc ctt gtc cca tcg ctc aaa aga gtg acc ggt cta gac act ggt      2059
   Arg Val Leu Val Pro Ser Leu Lys Arg Val Thr Gly Leu Asp Thr Gly
           415                 420                 425
   ata aca ctc ttg caa agg atc gga gcg gga atc ttc ttt gcg gtg ctg      2107
   Ile Thr Leu Leu Gln Arg Ile Gly Ala Gly Ile Phe Phe Ala Val Leu
       430                 435                 440
   agt tta ttg gtc tcc ggg ttc ata gag gaa cgt aga aga agc att gcg      2155
   Ser Leu Leu Val Ser Gly Phe Ile Glu Glu Arg Arg Arg Ser Ile Ala
   445                 450                 455                 460
   ctg aca aaa cag act cta ggg atg gag cct cga aca gga gag atc tca      2203
   Leu Thr Lys Gln Thr Leu Gly Met Glu Pro Arg Thr Gly Glu Ile Ser
                   465                 470                 475
   tca atg tcg gcc atg tgg ctg att ccg cag ctg gtg ctt gca ggt ata      2251
   Ser Met Ser Ala Met Trp Leu Ile Pro Gln Leu Val Leu Ala Gly Ile
               480                 485                 490
   gct gaa gca ttt aca gcc att gga cag atg gag ttt tac tac aag cag      2299
   Ala Glu Ala Phe Thr Ala Ile Gly Gln Met Glu Phe Tyr Tyr Lys Gln
           495                 500                 505
   ttt cct gag aac atg agg agc ttt gct ggc tct atc ttc tat gtc ggt      2347
   Phe Pro Glu Asn Met Arg Ser Phe Ala Gly Ser Ile Phe Tyr Val Gly
       510                 515                 520
   gcg ggg gtt tct agc tac ctc gct agc ttc ttg atc tcc act gtt cat      2395
   Ala Gly Val Ser Ser Tyr Leu Ala Ser Phe Leu Ile Ser Thr Val His
   525                 530                 535                 540
   agg aca act gaa cat tca cct tcg ggg aac tgg tta gct gag gat ctg      2443
   Arg Thr Thr Glu His Ser Pro Ser Gly Asn Trp Leu Ala Glu Asp Leu
                   545                 550                 555
   aac aag gcg aag ttg gat tac ttc tat ttc atg ctc acg gga ctc atg      2491
   Asn Lys Ala Lys Leu Asp Tyr Phe Tyr Phe Met Leu Thr Gly Leu Met
               560                 565                 570
   ttc gtt aac atg gtt tac ttc ttg ata atg gct aag ttt tat aga tac      2539
   Phe Val Asn Met Val Tyr Phe Leu Ile Met Ala Lys Phe Tyr Arg Tyr
           575                 580                 585
   aaa ggc agt gat gat aaa gtc atc tct gct att gag acc aat gaa gac      2587
   Lys Gly Ser Asp Asp Lys Val Ile Ser Ala Ile Glu Thr Asn Glu Asp
       590                 595                 600
   gag acc aag cag aag caa gag aag aac tcg gtg taa                      2623
   Glu Thr Lys Gln Lys Gln Glu Lys Asn Ser Val
   605                 610                 615
<210> 52
   <211> 615
   <212> PRT
   <213> Brassica oleracea
<400> 52
   Met Glu Arg Lys Pro Phe Glu Val Glu Thr Thr Lys Thr Asp Glu Asp
   1 5 10 15
   Gln Lys Pro Tyr Ser Ala Val Asp Gly Gly Gly Ser Asp Ser Val Asp
   20 25 30
   Ser Phe Asp Glu Asp Gln Lys Lys Leu Val Tyr Arg Gly Trp Lys Val
   35 40 45
   Met Pro Phe Ile Ile Gly Asn Glu Thr Phe Glu Lys Ile Gly Ile Ile
   50 55 60
   Gly Thr Leu Ser Asn Leu Leu Val Tyr Leu Thr Gln Val Phe Asn Leu
   65 70 75 80
   Lys Lys Tyr Thr Ala Ala Thr Ile Ile Asn Ala Phe Ser Gly Thr Ile
   85 90 95
   Asn Phe Gly Thr Phe Leu Ala Ala Phe Leu Cys Asp Thr Tyr Phe Gly
   100 105 110
   Arg Tyr Lys Thr Leu Ser Val Ala Val Val Ala Cys Phe Leu Gly Ser
   115 120 125
   Leu Ala Ile Leu Leu Thr Ala Ala Val Pro Gly Leu His Pro Thr Pro
   130 135 140
   Cys Gly Thr Lys Ser Ser Cys Glu Gly Pro Gly Ala Gly Gln Ile Leu
   145 150 155 160
   Phe Leu Met Leu Gly Leu Ala Leu Leu Val Val Gly Ala Gly Gly Ile
   165 170 175
   Arg Pro Cys Asn Leu Ala Phe Gly Ala Asp Gln Phe Asn Pro Lys Ser
   180 185 190
   Glu Ser Gly Lys Lys Gly Ile Asn Ser Phe Phe Asn Trp Tyr Phe Phe
   195 200 205
   Thr Phe Thr Phe Ala Gln Ile Ile Ser Leu Thr Leu Val Val Tyr Ile
   210 215 220
   Gln Ser Asn Val Ser Trp Thr Ile Gly Leu Ser Ile Pro Val Gly Leu
   225 230 235 240
   Met Phe Leu Ala Cys Val Ile Phe Phe Ala Gly His Lys Leu Tyr Val
   245 250 255
   Lys Val Lys Ala Ser Gly Ser Pro Leu Ala Gly Ile Ala Arg Val Ile
   260 265 270
   Ala Ala Ala Ile Ser Lys Arg Gly Leu Lys Pro Val Lys Gln Pro Trp
   275 280 285
   Val Asp Leu Tyr Asn His Ile Pro Arg Asn Tyr Ala Asn Leu Thr Leu
   290 295 300
   Lys Tyr Thr Asp Gln Phe Arg Phe Leu Asp Lys Ala Ala Ile Met Thr
   305 310 315 320
   Pro Glu Asp Lys Leu Asn Ser Asp Gly Thr Ala Ser Asp Pro Trp Lys
   325 330 335
   Leu Cys Thr Met Gln Gln Val Glu Glu Val Lys Cys Ile Ile Arg Val
   340 345 350
   Ile Pro Ile Trp Phe Ala Cys Ala Ile Tyr Tyr Leu Ala Ile Ser Ile
   355 360 365
   Gln Met Thr Tyr Pro Val Phe Gln Ala Gln Gln Ser Asp Arg Arg Leu
   370 375 380
   Gly Thr Gly Gly Phe Lys Met Pro Ala Ala Thr Tyr Val Val Phe Leu
   385 390 395 400
   Met Thr Gly Met Thr Val Phe Ile Ile Ile Tyr Asp Arg Val Leu Val
   405 410 415
   Pro Ser Leu Lys Arg Val Thr Gly Leu Asp Thr Gly Ile Thr Leu Leu
   420 425 430
   Gln Arg Ile Gly Ala Gly Ile Phe Phe Ala Val Leu Ser Leu Leu Val
   435 440 445
   Ser Gly Phe Ile Glu Glu Arg Arg Arg Ser Ile Ala Leu Thr Lys Gln
   450 455 460
   Thr Leu Gly Met Glu Pro Arg Thr Gly Glu Ile Ser Ser Met Ser Ala
   465 470 475 480
   Met Trp Leu Ile Pro Gln Leu Val Leu Ala Gly Ile Ala Glu Ala Phe
   485 490 495
   Thr Ala Ile Gly Gln Met Glu Phe Tyr Tyr Lys Gln Phe Pro Glu Asn
   500 505 510
   Met Arg Ser Phe Ala Gly Ser Ile Phe Tyr Val Gly Ala Gly Val Ser
   515 520 525
   Ser Tyr Leu Ala Ser Phe Leu Ile Ser Thr Val His Arg Thr Thr Glu
   530 535 540
   His Ser Pro Ser Gly Asn Trp Leu Ala Glu Asp Leu Asn Lys Ala Lys
   545 550 555 560
   Leu Asp Tyr Phe Tyr Phe Met Leu Thr Gly Leu Met Phe Val Asn Met
   565 570 575
   Val Tyr Phe Leu Ile Met Ala Lys Phe Tyr Arg Tyr Lys Gly Ser Asp
   580 585 590
   Asp Lys Val Ile Ser Ala Ile Glu Thr Asn Glu Asp Glu Thr Lys Gln
   595 600 605
   Lys Gln Glu Lys Asn Ser Val
   610 615
<210> 53
   <211> 2365
   <212> DNA
   <213> Brassica oleracea
<220>
   <221> CDS
   <222> (1)..(154)
<220>
   <221> CDS
   <222> (516)..(733)
<220>
   <221> CDS
   <222> (812)..(1365)
<220>
   <221> CDS
   <222> (1444)..(2362)
<400> 53
   atg gag aga aag ccc ttt gag gtt gag acg acg gag gat cac aaa ccc        48
   Met Glu Arg Lys Pro Phe Glu Val Glu Thr Thr Glu Asp His Lys Pro
   1               5                   10                  15
   tac tcc gat gga ggt ggt tct gat tta acg tcg acg gtt gat tca tct        96
   Tyr Ser Asp Gly Gly Gly Ser Asp Leu Thr Ser Thr Val Asp Ser Ser
               20                  25                  30
   ggc gac gag cag aaa aag ctc gtt tat aga ggc tgg aaa gtc atg cct       144
   Gly Asp Glu Gln Lys Lys Leu Val Tyr Arg Gly Trp Lys Val Met Pro
           35                  40                  45
   tat atc att g gtaacgcttt tctctctctc tctagaccgt tatttttaaa              194
   Tyr Ile Ile
       50
   ctctgttttc tttttgtcaa ctaactctgt tttttcgtgt aacagaacac ttttccccgt     254
   gttggttttt tttgtcattt aataattttg tttattttta tcttgtcacg ttggcttgac    314
   tgtgtcagtg ttctagataa tctaccaggt gtagtgtaca gaaagaaatg ttaccttgtg     374
   ttctttagta acgtttttaa atgtccgtgg aaaggacttt gaatcactgg aagtccaatc     434
   ttggttccaa actgtgttac tttatgagaa acccgtgttg gtttgactat tcattaacga     494
   aaagaatttg tatcttgata g gt  aat gag aca ttt gag aag att ggg atc       544
                           Gly Asn Glu Thr Phe Glu Lys Ile Gly Ile
                                       55                  60
   ata ggg aca tta tca aac ctt cta gtg tac cta act caa gta ttc aac       592
   Ile Gly Thr Leu Ser Asn Leu Leu Val Tyr Leu Thr Gln Val Phe Asn
               65                  70                  75
   ctt aag aaa tac aca gct gca act att atc aat gcc ttc agt ggc act       640
   Leu Lys Lys Tyr Thr Ala Ala Thr Ile Ile Asn Ala Phe Ser Gly Thr
           80                  85                  90
   atc aac ttc ggc act ttc ttc gct gct ttt ctg tgt gac act tac ttt       688
   Ile Asn Phe Gly Thr Phe Phe Ala Ala Phe Leu Cys Asp Thr Tyr Phe
       95                  100                 105
   ggc cgc tac aag act ctc agt gtg gct gtc atc gct tgt ttt ctg           733
   Gly Arg Tyr Lys Thr Leu Ser Val Ala Val Ile Ala Cys Phe Leu
   110                 115                 120
   gtactaatgc tgctgatctt aatgggtttt ttttcttacg ccaagactct taatgctgat     793
   catttttcgt ctgtgtag gga tcg gtt ttt ata cta atg acg gct gca gtt       844
                       Gly Ser Val Phe Ile Leu Met Thr Ala Ala Val
                       125                 130                 135
   ccg gga ttg cac ccg act cct tgt gga act aaa agt tcg tgc caa ggg       892
   Pro Gly Leu His Pro Thr Pro Cys Gly Thr Lys Ser Ser Cys Gln Gly
                   140                 145                 150
   cca aat ggg ggg cag gtt ttg ttt ctg ctg ttg ggt tta gcg ctt ctc       940
   Pro Asn Gly Gly Gln Val Leu Phe Leu Leu Leu Gly Leu Ala Leu Leu
               155                 160                 165
   gta gtc ggt gct ggt ggt atc agg ccg tgt aat ttg gcc ttt gga gct       988
   Val Val Gly Ala Gly Gly Ile Arg Pro Cys Asn Leu Ala Phe Gly Ala
           170                 175                 180
   gat cag ttc aac ccc aag tca gaa tct gga aag aaa gga atc aac agt      1036
   Asp Gln Phe Asn Pro Lys Ser Glu Ser Gly Lys Lys Gly Ile Asn Ser
           185                     190                     195
   ttc ttc aac tgg tac ttc ttc act ttc acg ttt gcg cag atc atc tcg      1084
   Phe Phe Asn Trp Tyr Phe Phe Thr Phe Thr Phe Ala Gln Ile Ile Ser
   200                 205                 210                 215
   ctc acg cta gtt gtg tat atc cag tca aac gtg agc tgg acc att ggt      1132
   Leu Thr Leu Val Val Tyr Ile Gln Ser Asn Val Ser Trp Thr Ile Gly
                   220                 225                 230
   ttg agt atc cct gtg ggt cta atg ttc ttg gcc tgc gtc att tac ttt      1180
   Leu Ser Ile Pro Val Gly Leu Met Phe Leu Ala Cys Val Ile Tyr Phe
               235                 240                 245
   gct ggc cat aat ctc tac gta aaa gtg aaa gcc tcg ggt agt ccc ttg      1228
   Ala Gly His Asn Leu Tyr Val Lys Val Lys Ala Ser Gly Ser Pro Leu
           250                 255                 260
   gct ggc atc gct cgt gtt ata gct gca gcg atc aag aaa cga ggg ttg      1276
   Ala Gly Ile Ala Arg Val Ile Ala Ala Ala Ile Lys Lys Arg Gly Leu
       265                 270                 275
   aag cct gtt aag aag cct tgc tcc gat ctt tac aat cac atc ccg cct      1324
   Lys Pro Val Lys Lys Pro Cys Ser Asp Leu Tyr Asn His Ile Pro Pro
   280                 285                 290                 295
   aac tat gca aac tct aca ctc aaa tac tcc gac cag ttt ag               1365
   Asn Tyr Ala Asn Ser Thr Leu Lys Tyr Ser Asp Gln Phe Arg
                   300                 305
   gtaaaattca aaaagtttct ctctctctct ctctgttgct ttatgttttg ttttgatgaa    1425
   aacatgttac gttgctag a ttt ctc gac aaa gca gca gtc atg acc cct gat    1477
                         Phe Leu Asp Lys Ala Ala Val Met Thr Pro Asp
                         310                 315                 320
   gac aag ttg aaa ccc gat gga acg gct tca gat cca tgg aat cta tgt      1525
   Asp Lys Leu Lys Pro Asp Gly Thr Ala Ser Asp Pro Trp Asn Leu Cys
                   325                 330                 335
   acg atg cag cag gtg gaa gaa gtg aaa tgc att gta aga gtg att cca      1573
   Thr Met Gln Gln Val Glu Glu Val Lys Cys Ile Val Arg Val Ile Pro
               340                 345                 350
   atc tgg ttt gct tgc gcg gtg tac tac ctc gca ata aca ttg caa atg      1621
   Ile Trp Phe Ala Cys Ala Val Tyr Tyr Leu Ala Ile Thr Leu Gln Met
           355                 360                 365
   act tat ccg gtc ttc caa gcg ctc cag agc gac cgg aga ttg ggt tca      1669
   Thr Tyr Pro Val Phe Gln Ala Leu Gln Ser Asp Arg Arg Leu Gly Ser
       370                 375                 380
   gat ggc ttc aag att cca ggc ggc acc tat gta gtg ttc ttg atg tcc      1717
   Asp Gly Phe Lys Ile Pro Gly Gly Thr Tyr Val Val Phe Leu Met Ser
   385                 390                 395                 400
   ggt atg acg gtt ttc atc ata ttc tac gac cgt gtc ctt gtc ccg tcg      1765
   Gly Met Thr Val Phe Ile Ile Phe Tyr Asp Arg Val Leu Val Pro Ser
                   405                 410                 415
   ctc aga aga gtg aca ggg cta gac aat ggt ata act ctc tta caa aga      1813
   Leu Arg Arg Val Thr Gly Leu Asp Asn Gly Ile Thr Leu Leu Gln Arg
               420                 425                 430
   atc gga tca ggc att ttc ttt gcg ctt ttg agt atg ttg att tct ggg      1861
   Ile Gly Ser Gly Ile Phe Phe Ala Leu Leu Ser Met Leu Ile Ser Gly
           435                 440                 445
   ttc gtt gag gaa cgg cga aga gcc att gca ctg aca aaa cct act ctc      1909
   Phe Val Glu Glu Arg Arg Arg Ala Ile Ala Leu Thr Lys Pro Thr Leu
       450                 455                 460
   ggg atc gag cct cga gct gga gaa atc tca tca atg tca gcg atg tgg      1957
   Gly Ile Glu Pro Arg Ala Gly Glu Ile Ser Ser Met Ser Ala Met Trp
   465                 470                 475                 480
   ctg ata ccg cag ctc gtg ctt gca ggg ata gct gaa gct ttt gca gct      2005
   Leu Ile Pro Gln Leu Val Leu Ala Gly Ile Ala Glu Ala Phe Ala Ala
                   485                 490                 495
   gtt gga caa atg gag ttt tac tac aag cag ttt cct gaa aac atg aag      2053
   Val Gly Gln Met Glu Phe Tyr Tyr Lys Gln Phe Pro Glu Asn Met Lys
               500                 505                 510
   agc ttt gcg ggt tct atc ttc tat gtc ggt gga gga gtt tct agc tac      2101
   Ser Phe Ala Gly Ser Ile Phe Tyr Val Gly Gly Gly Val Ser Ser Tyr
           515                 520                 525
   ctc gct agc ttc ttg atc tcg act gtt cat aaa aca act gag cat tca      2149
   Leu Ala Ser Phe Leu Ile Ser Thr Val His Lys Thr Thr Glu His Ser
       530                 535                 540
   cgc tcc ggg aat tgg cta gct gag gat ctg aac aaa ggg aag ctg gat      2197
   Arg Ser Gly Asn Trp Leu Ala Glu Asp Leu Asn Lys Gly Lys Leu Asp
   545                 550                 555                 560
   tac ttt tat ttc atg atc act ggc ctc atg ttt gtt aac atg ggt tac      2245
   Tyr Phe Tyr Phe Met Ile Thr Gly Leu Met Phe Val Asn Met Gly Tyr
                   565                 570                 575
   ttc ttg tta atg gct aaa ttt tat aga tac aaa gtc act aac gat gaa      2293
   Phe Leu Leu Met Ala Lys Phe Tyr Arg Tyr Lys Val Thr Asn Asp Glu
               580                 585                 590
   gca aat tct gtg att aag act tat gaa gaa gag acc aag gag aat caa      2341
   Ala Asn Ser Val Ile Lys Thr Tyr Glu Glu Glu Thr Lys Glu Asn Gln
           595                 600                 605
   caa caa gac aag agc tat gtc tga                                      2365
   Gln Gln Asp Lys Ser Tyr Val
       610                 615
<210> 54
   <211> 615
   <212> PRT
   <213> Brassica oleracea
<400> 54
   Met Glu Arg Lys Pro Phe Glu Val Glu Thr Thr Glu Asp His Lys Pro
   1               5                   10                  15
   Tyr Ser Asp Gly Gly Gly Ser Asp Leu Thr Ser Thr Val Asp Ser Ser
               20                  25                  30
   Gly Asp Glu Gln Lys Lys Leu Val Tyr Arg Gly Trp Lys Val Met Pro
           35                  40                  45
   Tyr Ile Ile Gly Asn Glu Thr Phe Glu Lys Ile Gly Ile Ile Gly Thr
       50                  55                  60
   Leu Ser Asn Leu Leu Val Tyr Leu Thr Gln Val Phe Asn Leu Lys Lys
   65                  70                  75                  80
   Tyr Thr Ala Ala Thr Ile Ile Asn Ala Phe Ser Gly Thr Ile Asn Phe
                   85                  90                  95
   Gly Thr Phe Phe Ala Ala Phe Leu Cys Asp Thr Tyr Phe Gly Arg Tyr
               100                 105                 110
   Lys Thr Leu Ser Val Ala Val Ile Ala Cys Phe Leu Gly Ser Val Phe
           115                 120                 125
   Ile Leu Met Thr Ala Ala Val Pro Gly Leu His Pro Thr Pro Cys Gly
       130                 135                 140
   Thr Lys Ser Ser Cys Gln Gly Pro Asn Gly Gly Gln Val Leu Phe Leu
   145                 150                 155                 160
   Leu Leu Gly Leu Ala Leu Leu Val Val Gly Ala Gly Gly Ile Arg Pro
                   165                 170                 175
   Cys Asn Leu Ala Phe Gly Ala Asp Gln Phe Asn Pro Lys Ser Glu Ser
               180                 185                 190
   Gly Lys Lys Gly Ile Asn Ser Phe Phe Asn Trp Tyr Phe Phe Thr Phe
           195                 200                 205
   Thr Phe Ala Gln Ile Ile Ser Leu Thr Leu Val Val Tyr Ile Gln Ser
       210                 215                 220
   Asn Val Ser Trp Thr Ile Gly Leu Ser Ile Pro Val Gly Leu Met Phe
   225                 230                 235                 240
   Leu Ala Cys Val Ile Tyr Phe Ala Gly His Asn Leu Tyr Val Lys Val
                   245                 250                 255
   Lys Ala Ser Gly Ser Pro Leu Ala Gly Ile Ala Arg Val Ile Ala Ala
               260                 265                 270
   Ala Ile Lys Lys Arg Gly Leu Lys Pro Val Lys Lys Pro Cys Ser Asp
           275                 280                 285
   Leu Tyr Asn His Ile Pro Pro Asn Tyr Ala Asn Ser Thr Leu Lys Tyr
       290                 295                 300
   Ser Asp Gln Phe Arg Phe Leu Asp Lys Ala Ala Val Met Thr Pro Asp
   305                 310                 315                 320
   Asp Lys Leu Lys Pro Asp Gly Thr Ala Ser Asp Pro Trp Asn Leu Cys
                   325                 330                 335
   Thr Met Gln Gln Val Glu Glu Val Lys Cys Ile Val Arg Val Ile Pro
               340                 345                 350
   Ile Trp Phe Ala Cys Ala Val Tyr Tyr Leu Ala Ile Thr Leu Gln Met
           355                 360                 365
   Thr Tyr Pro Val Phe Gln Ala Leu Gln Ser Asp Arg Arg Leu Gly Ser
       370                 375                 380
   Asp Gly Phe Lys Ile Pro Gly Gly Thr Tyr Val Val Phe Leu Met Ser
   385                 390                 395                 400
   Gly Met Thr Val Phe Ile Ile Phe Tyr Asp Arg Val Leu Val Pro Ser
                   405                 410                 415
   Leu Arg Arg Val Thr Gly Leu Asp Asn Gly Ile Thr Leu Leu Gln Arg
               420                 425                 430
   Ile Gly Ser Gly Ile Phe Phe Ala Leu Leu Ser Met Leu Ile Ser Gly
           435                 440                 445
   Phe Val Glu Glu Arg Arg Arg Ala Ile Ala Leu Thr Lys Pro Thr Leu
       450                 455                 460
   Gly Ile Glu Pro Arg Ala Gly Glu Ile Ser Ser Met Ser Ala Met Trp
   465                 470                 475                 480
   Leu Ile Pro Gln Leu Val Leu Ala Gly Ile Ala Glu Ala Phe Ala Ala
                   485                 490                 495
   Val Gly Gln Met Glu Phe Tyr Tyr Lys Gln Phe Pro Glu Asn Met Lys
               500                 505                 510
   Ser Phe Ala Gly Ser Ile Phe Tyr Val Gly Gly Gly Val Ser Ser Tyr
           515                 520                 525
   Leu Ala Ser Phe Leu Ile Ser Thr Val His Lys Thr Thr Glu His Ser
       530                 535                 540
   Arg Ser Gly Asn Trp Leu Ala Glu Asp Leu Asn Lys Gly Lys Leu Asp
   545                 550                 555                 560
   Tyr Phe Tyr Phe Met Ile Thr Gly Leu Met Phe Val Asn Met Gly Tyr
                   565                 570                 575
   Phe Leu Leu Met Ala Lys Phe Tyr Arg Tyr Lys Val Thr Asn Asp Glu
               580                 585                 590
   Ala Asn Ser Val Ile Lys Thr Tyr Glu Glu Glu Thr Lys Glu Asn Gln
           595                 600                 605
   Gln Gln Asp Lys Ser Tyr Val
       610                 615
<210> 55
   <211> 2393
   <212> DNA
   <213> Brassica oleracea
<220>
   <221> CDS
   <222> (1)..(157)
<220>
   <221> CDS
   <222> (526)..(743)
<220>
   <221> CDS
   <222> (836)..(1389)
<220>
   <221> CDS
   <222> (1484)..(2390)
<400> 55
   atg gag aga aag cct cta gaa gtc gag tca acg gat cac caa aag ccc        48
   Met Glu Arg Lys Pro Leu Glu Val Glu Ser Thr Asp His Gln Lys Pro
   1               5                   10                  15
   ccc tcc gcc gtg tac gat ggc tct gtt atg gcg gtt gat tct gtt gag        96
   Pro Ser Ala Val Tyr Asp Gly Ser Val Met Ala Val Asp Ser Val Glu
               20                  25                  30
   gaa gaa gtt ggg gag acc aaa gtc gtt tac aga ggc tgg aaa gtc atg      144
   Glu Glu Val Gly Glu Thr Lys Val Val Tyr Arg Gly Trp Lys Val Met
           35                  40                  45
   ccc ttt atc att g gtaacacaaa ctttttttta taaactttct tcttttgttc         197
   Pro Phe Ile Ile
       50
   caagaaaaaa agactatttt ttattgcttt tcatgttttt tccaaaataa ataatcaata     257
   gtcagaagaa atgaagagga aaacatcagt cttgctattg gtttgatttt taaaaaaaaa     317
   attcgtgaga ttctctgtac tgttgtcaaa agctttgact tagtcaacca gtttttcaag     377
   aaaattaaaa aaagagagtg agatctagaa tttgagaagg tttcagaaca taactgatca     437
   agaatcaact gtatgattca tgcaaagctt gaggcatatg agatcaaagt cttatgatca     497
   tcttggtttt gtttgttggt atcatcag ga aat gag acg ttc gag aag ctt         548
                                   Gly Asn Glu Thr Phe Glu Lys Leu
                                           55                  60
   ggg atc att gga aca cta tca aac ctt ttg gtt tat tta act gcc gtc      596
   Gly Ile Ile Gly Thr Leu Ser Asn Leu Leu Val Tyr Leu Thr Ala Val
                   65                  70                  75
   ttc aac atg aag agt atc aca gct gca aca atc atc aat gca ttc agt      644
   Phe Asn Met Lys Ser Ile Thr Ala Ala Thr Ile Ile Asn Ala Phe Ser
               80                  85                  90
   ggc aca atc aac ttc gga acc ttc gtt gct gct ttc ctc tgc gac act      692
   Gly Thr Ile Asn Phe Gly Thr Phe Val Ala Ala Phe Leu Cys Asp Thr
           95                  100                 105
   tac ttc ggt cga tac aag act cta acc gtc gct gtc atc gcc tgt ttt      740
   Tyr Phe Gly Arg Tyr Lys Thr Leu Thr Val Ala Val Ile Ala Cys Phe
       110                 115                 120
   ctt gtaatgatca ttcctttgca atctttcttg ctccacaagc gttcttagtt            793
   Leu
   125
   taacaaccgt aaaaatctga taattgttgt tcttttttgt ag gga tca ctt gtg        847
                                                  Gly Ser Leu Val
   ata cta ttg aca gct gca gtg cca caa tta cat cca gct cca tgc gga      895
   Ile Leu Leu Thr Ala Ala Val Pro Gln Leu His Pro Ala Pro Cys Gly
   130                 135                 140                 145
   aca gct ctc tca tgt atc ggg cca agt ggc ggc cag ata gcg ttt cta      943
   Thr Ala Leu Ser Cys Ile Gly Pro Ser Gly Gly Gln Ile Ala Phe Leu
                   150                 155                 160
   ctg ctg ggt ctc ggc ttt ctt gta gtt gga gca ggc ggg att aga cca      991
   Leu Leu Gly Leu Gly Phe Leu Val Val Gly Ala Gly Gly Ile Arg Pro
               165                 170                 175
   tgt aat cta gct ttc ggg gct gat cag ttt aac ccc aag agc gag tca     1039
   Cys Asn Leu Ala Phe Gly Ala Asp Gln Phe Asn Pro Lys Ser Glu Ser
           180                 185                 190
   gga aaa cga ggc atc gat agt ttc ttc aat tgg tac ttc ttc acc ttc     1087
   Gly Lys Arg Gly Ile Asp Ser Phe Phe Asn Trp Tyr Phe Phe Thr Phe
       195                 200                 205
   acg ttc gcg cag atc ctg tcg ctg aca cta atc gtc tat atc cag tcc     1135
   Thr Phe Ala Gln Ile Leu Ser Leu Thr Leu Ile Val Tyr Ile Gln Ser
   210                 215                 220                 225
   aat gtc agc tgg acc atc ggt tta acc atc ccg gtc gtt ctc atg ttc     1183
   Asn Val Ser Trp Thr Ile Gly Leu Thr Ile Pro Val Val Leu Met Phe
                   230                 235                 240
   ttg gcc tgc gtg att ttc ttt gct gga gat aag ttg tat gtg aag atc      1231
   Leu Ala Cys Val Ile Phe Phe Ala Gly Asp Lys Leu Tyr Val Lys Ile
               245                 250                 255
   aaa gcc tca ggt agt cca ttg gct ggt ata gct caa gtt ata gcg gtt      1279
   Lys Ala Ser Gly Ser Pro Leu Ala Gly Ile Ala Gln Val Ile Ala Val
           260                 265                 270
   gca atc aag aaa cgt ggg tta aag cca acg aaa gag cct tgg ctt aac      1327
   Ala Ile Lys Lys Arg Gly Leu Lys Pro Thr Lys Glu Pro Trp Leu Asn
       275                 280                 285
   ctt tac aac tac tac cca cca aaa tac gca aac tcc aaa ctc aaa tac      1375
   Leu Tyr Asn Tyr Tyr Pro Pro Lys Tyr Ala Asn Ser Lys Leu Lys Tyr
   290                 295                 300                 305
   acc gac cag ttt ag  gtaacaaaac acacacacgc caagagttag ttttatcttt      1429
   Thr Asp Gln Phe Arg
   aatatctttc cacttttgtt atatggttac tgagaaagtt tcctgttttg gcag g ttt    1487
                                                                 Phe
   ctt gat aag gct gcg atc ttg gct ccg gaa gac aag ttg gag gct gat      1535
   Leu Asp Lys Ala Ala Ile Leu Ala Pro Glu Asp Lys Leu Glu Ala Asp
               315                 320                 325
   ggt aag cct gcg gat cca tgg aag ctt tgt aca atg caa caa gtt gaa      1583
   Gly Lys Pro Ala Asp Pro Trp Lys Leu Cys Thr Met Gln Gln Val Glu
           330                 335                 340
   gaa gtg aag tgc att gtg aga gtg ctt cct ata tgg ttt gct gca tcc      1631
   Glu Val Lys Cys Ile Val Arg Val Leu Pro Ile Trp Phe Ala Ala Ser
       345                 350                 355
   atc tat tac tta acc atc acg caa caa atg act tac ccc gtt ttc caa      1679
   Ile Tyr Tyr Leu Thr Ile Thr Gln Gln Met Thr Tyr Pro Val Phe Gln
   360                 365                 370                 375
   gcc ctc cag agc gac cgt cgc tta gga tcc aga gga ttc gtg atc ccg      1727
   Ala Leu Gln Ser Asp Arg Arg Leu Gly Ser Arg Gly Phe Val Ile Pro
                   380                 385                 390
   gca gcc acc tac gtt gtc ttc ttg atg aca gga atg acg gtt ttc atc      1775
   Ala Ala Thr Tyr Val Val Phe Leu Met Thr Gly Met Thr Val Phe Ile
               395                 400                 405
   ata ttc tac gac cgt gtg ctc gtg cct acc tta aga aga ata acc ggt      1823
   Ile Phe Tyr Asp Arg Val Leu Val Pro Thr Leu Arg Arg Ile Thr Gly
           410                 415                 420
   ata gac acg ggg ata acg ctc ttg cag agg att gga acc ggt att ttc      1871
   Ile Asp Thr Gly Ile Thr Leu Leu Gln Arg Ile Gly Thr Gly Ile Phe
       425                 430                 435
   ttc gcc ttt gtt agc ttg ata gtc tca ggt ttc gtc gag gaa cgg agg      1919
   Phe Ala Phe Val Ser Leu Ile Val Ser Gly Phe Val Glu Glu Arg Arg
   440                 445                 450                 455
   aga acg ttc gcg ctg act aaa cca aca ctt ggt atg gct cca agg aag      1967
   Arg Thr Phe Ala Leu Thr Lys Pro Thr Leu Gly Met Ala Pro Arg Lys
                   460                 465                 470
   gga gaa atc tcc tca atg tca gct atg tgg ctg atc cca caa ctc gcc      2015
   Gly Glu Ile Ser Ser Met Ser Ala Met Trp Leu Ile Pro Gln Leu Ala
               475                 480                 485
   ctt gcg ggt ata gcc gag gcg ttt gga gct att gga cag atg gag ttt      2063
   Leu Ala Gly Ile Ala Glu Ala Phe Gly Ala Ile Gly Gln Met Glu Phe
           490                 495                 500
   tac tac aag caa ttc cct gag aac atg agg agt ttt gct ggt tcg atc      2111
   Tyr Tyr Lys Gln Phe Pro Glu Asn Met Arg Ser Phe Ala Gly Ser Ile
       505                 510                 515
   ttc tat gta gga gca gga gtt tca agc tac ctc ggt agc ttc ttg att        2159
   Phe Tyr Val Gly Ala Gly Val Ser Ser Tyr Leu Gly Ser Phe Leu Ile
   520                 525                 530                 535
   gca atg gtt cac agg acg acg cag aac tcg gct ggt ggt aat tgg tta        2207
   Ala Met Val His Arg Thr Thr Gln Asn Ser Ala Gly Gly Asn Trp Leu
                   540                 545                 550
   gct gag gat ttg aac aaa gga aga ttg gat tac ttc tat ttc atg atc        2255
   Ala Glu Asp Leu Asn Lys Gly Arg Leu Asp Tyr Phe Tyr Phe Met Ile
               555                 560                 565
   gct gga atc ttg gcg gtt aat ttt gct tac ttc ttg gtc atg tca aga        2303
   Ala Gly Ile Leu Ala Val Asn Phe Ala Tyr Phe Leu Val Met Ser Arg
           570                 575                 580
   tgg tat aga tac aaa gga agt gat gat gaa gtg aca act tat gaa acc        2351
   Trp Tyr Arg Tyr Lys Gly Ser Asp Asp Glu Val Thr Thr Tyr Glu Thr
       585                 590                 595
   aat gga gat gct atc aaa caa caa gac aag aat act gtc tga                2393
   Asn Gly Asp Ala Ile Lys Gln Gln Asp Lys Asn Thr Val
   600                 605                 610
<210> 56
   <211> 612
   <212> PRT
   <213> Brassica oleracea
<400> 56
   Met Glu Arg Lys Pro Leu Glu Val Glu Ser Thr Asp His Gln Lys Pro
   1               5                   10                  15
   Pro Ser Ala Val Tyr Asp Gly Ser Val Met Ala Val Asp Ser Val Glu
               20                  25                  30
   Glu Glu Val Gly Glu Thr Lys Val Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Thr Ala Leu Ser Cys Ile Gly Pro Ser Gly Gly Gln Ile Ala Phe
   145                 150                 155                 160
   Leu Leu Leu Gly Leu Gly Phe Leu Val Val Gly Ala Gly Gly Ile Arg
                   165                 170                 175
   Pro Cys Asn Leu Ala Phe Gly Ala Asp Gln Phe Asn Pro Lys Ser Glu
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Leu Ile Val Tyr Ile Gln
   210 215 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Gly Ile Ala Gln Val Ile Ala
   260 265 270
   Val Ala Ile Lys Lys Arg Gly Leu Lys Pro Thr Lys Glu Pro Trp Leu
   275 280 285
   Asn Leu Tyr Asn Tyr Tyr Pro Pro Lys Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Ala Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ala Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Arg Gly Phe Val Ile Pro Ala Ala Thr Tyr Val Val Phe Leu Met
   385 390 395 400
   Thr Gly Met Thr Val Phe Ile Ile Phe Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Val Ser Leu Ile Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465 470 475 480
   Trp Leu Ile Pro Gln Leu Ala Leu Ala Gly Ile Ala Glu Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Ala Gly Val Ser Ser
           515                 520                 525
   Tyr Leu Gly Ser Phe Leu Ile Ala Met Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Tyr Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Glu Val Thr Thr Tyr Glu Thr Asn Gly Asp Ala Ile Lys Gln Gln Asp
           595                 600                 605
   Lys Asn Thr Val
       610
<210> 57
   <211> 2396
   <212> DNA
   <213> Brassica oleracea
<220>
   <221> CDS
   <222> (1)..(157)
<220>
   <221> CDS
   <222> (550)..(767)
<220>
   <221> CDS
   <222> (858)..(1411)
<220>
   <221> CDS
   <222> (1487)..(2393)
<400> 57
<210> 58
   <211> 612
   <212> PRT
   <213> Brassica oleracea
<400> 58
   Met Glu Arg Asn Pro Leu Glu Val Glu Ser Thr Asp His Gln Lys Pro
   1               5                   10                  15
   Ser Ser Ala Val Tyr Asp Gly Ser Ala Thr Ala Val Asp Ser Val Glu
               20                  25                  30
   Glu Glu Val Gln Glu Thr Lys Leu Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Thr Ala Leu Thr Cys Thr Gly Pro Ser Gly Gly Gln Ile Ala Phe
   145                 150                 155                 160
   Leu Leu Met Gly Leu Ala Phe Leu Val Val Gly Ala Gly Gly Ile Arg
                   165                 170                 175
   Pro Cys Asn Leu Ala Phe Gly Ala Asp Gln Phe Asn Pro Lys Ser Glu
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Leu Val Val Tyr Ile Gln
       210                 215                 220
   Ser Asn Val Ser Trp Thr Ile Gly Leu Thr Ile Pro Val Val Leu Met
   225                 230                 235                 240
   Phe Leu Ala Cys Val Ile Phe Phe Ala Gly Asp Lys Leu Tyr Val Lys
   245 250 255
   Ile Lys Ala Ser Gly Ser Pro Leu Ala Ser Ile Ala Gln Val Ile Thr
   260 265 270
   Val Ala Ile Lys Lys Arg Gly Leu Lys Ala Val Lys Gln Pro Trp Val
   275 280 285
   Asn Leu Tyr Asn Tyr Tyr Pro Leu Asn Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Thr Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ser Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Gly Gly Phe Val Ile Pro Ala Ala Thr Tyr Val Val Phe Leu Met
   385 390 395 400
   Thr Gly Met Thr Val Phe Ile Ile Ile Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Leu Ser Leu Val Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465 470 475 480
   Trp Leu Ile Pro Gln Leu Thr Leu Ala Gly Val Ala Glu Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Gly Gly Val Ser Ser
   515 520 525
   Tyr Leu Gly Ser Phe Leu Ile Ala Thr Val His Arg Thr Thr Gln Asn
   530 535 540
   Ser Ala Gly Gly Asn Trp Leu Ala Glu Asp Leu Asn Lys Gly Arg Leu
   545                 550                 555                 560
   Asp Leu Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Glu Val Thr Ala Tyr Glu Thr Asn Gly Asp Val Ile Lys Gln Gln Asp
           595                 600                 605
   Lys Asn Lys Val
       610
<210> 59
   <211> 2379
   <212> DNA
   <213> Brassica oleracea
<220>
   <221> CDS
   <222> (1)..(157)
<220>
   <221> CDS
   <222> (332)..(549)
<220>
   <221> CDS
   <222> (638)..(1191)
<220>
   <221> CDS
   <222> (1470)..(2376)
<400> 59
   atg gag aga aag tct ctc gaa gtc gag tct atg aat cac caa aaa ctt        48
   Met Glu Arg Lys Ser Leu Glu Val Glu Ser Met Asn His Gln Lys Leu
   1               5                   10                  15
   tcc tcc gcc gtg tac gat ggc tct gtt acg gcc gtt gat tct gtt aaa        96
   Ser Ser Ala Val Tyr Asp Gly Ser Val Thr Ala Val Asp Ser Val Lys
               20                  25                  30
   gaa gat gtt cag gag acc aaa gtc gtt tat aga ggc tgg aaa gtc atg       144
   Glu Asp Val Gln Glu Thr Lys Val Val Tyr Arg Gly Trp Lys Val Met
           35                  40                  45
   cct ttc atc att g gtaacgcaat aatattatca tttacggtgc tcaactttct          197
   Pro Phe Ile Ile
       50
   tcttttgaat gctaaaagaa aactttcttc ttttggaaac tatttctttt gtcgttttgt      257
   tgtcttaagt tttcaattag tcagaatatt gaagtcttat gatcttggtt aatttttgtt      317
   gttgttacca ccag ga  aat gag aca ttt gag aag ctt ggg atc att gga        366
                   Gly Asn Glu Thr Phe Glu Lys Leu Gly Ile Ile Gly
                           55                  60
   aca cta tca aac ctt ctg gtt tac tta act tct gtc ttc aac atg aag       414
   Thr Leu Ser Asn Leu Leu Val Tyr Leu Thr Ser Val Phe Asn Met Lys
   65                  70                  75                  80
   agt gtt aca gct gca aca atc atc aat gcc ttt gct ggc aca gtc aac       462
   Ser Val Thr Ala Ala Thr Ile Ile Asn Ala Phe Ala Gly Thr Val Asn
                   85                  90                  95
   ttt gga act ttc gtt gct gct ttc ctt tgc gac act tac ttc ggt cga      510
   Phe Gly Thr Phe Val Ala Ala Phe Leu Cys Asp Thr Tyr Phe Gly Arg
               100                 105                 110
   tac aag act ctt acc gtc gcg gtc ctc gcc tgt ttt ctt gtaaaaattc       559
   Tyr Lys Thr Leu Thr Val Ala Val Leu Ala Cys Phe Leu
           115                 120                 125
   atcccctcta actatttctt actctacaag caatcttggt ttataaccta aaaataaaat    619
   ctggttcttg ttttgtag gga tca ctt gcg ata cta ttg act gct gca gtc      670
                       Gly Ser Leu Ala Ile Leu Leu Thr Ala Ala Val
                                   130                 135
   cca caa cta cat cca gcc cca tgc ggc aca gcg agc tcg tgt agc ggg      718
   Pro Gln Leu His Pro Ala Pro Cys Gly Thr Ala Ser Ser Cys Ser Gly
               140                 145                 150
   cca agt ggt ggc cag ata gcg ttt cta ctg atg ggt ctt ggg att ctt      766
   Pro Ser Gly Gly Gln Ile Ala Phe Leu Leu Met Gly Leu Gly Ile Leu
           155                 160                 165
   gtg gtg gga gca gga ggg att aga ccg tgc aat cta gct ttc gga gct      814
   Val Val Gly Ala Gly Gly Ile Arg Pro Cys Asn Leu Ala Phe Gly Ala
       170                 175                 180
   gat cag ttt aat ccg aag agc ggg tca ggg aaa aga ggg att gat agt      862
   Asp Gln Phe Asn Pro Lys Ser Gly Ser Gly Lys Arg Gly Ile Asp Ser
   185                 190                 195                 200
   ttc ttc aac tgg tac ttc ttc acc ttc act ttc gcg cag atc ttg tct      910
   Phe Phe Asn Trp Tyr Phe Phe Thr Phe Thr Phe Ala Gln Ile Leu Ser
                   205                 210                 215
   ctg aca gta gtt gtc tat gtc cag tct aat gtc agt tgg acc atc ggt      958
   Leu Thr Val Val Val Tyr Val Gln Ser Asn Val Ser Trp Thr Ile Gly
               220                 225                 230
   tta acc atc ccg gtt gtt cta atg ttc ttg gcc tcc gtg att ttc ttt     1006
   Leu Thr Ile Pro Val Val Leu Met Phe Leu Ala Ser Val Ile Phe Phe
           235                 240                 245
   gcg ggt gct aag ttg tat gtt aag atc gaa gcc tca ggt agt cca ttg     1054
   Ala Gly Ala Lys Leu Tyr Val Lys Ile Glu Ala Ser Gly Ser Pro Leu
       250                 255                 260
   gct agt ata gct cat gtt ata acg gct gct atc aag aaa cac ggg tta     1102
   Ala Ser Ile Ala His Val Ile Thr Ala Ala Ile Lys Lys His Gly Leu
   265                 270                 275                 280
   aag tca gtg gaa cag cct tgg ctg aac ctt tac aac tac tac cca tca     1150
   Lys Ser Val Glu Gln Pro Trp Leu Asn Leu Tyr Asn Tyr Tyr Pro Ser
                   285                 290                 295
   aaa cac gca aac gcc aag ctc aaa tac acc gaa caa ttc ag              1191
   Lys His Ala Asn Ala Lys Leu Lys Tyr Thr Glu Gln Phe Arg
               300                 305
   gtaaacacac ttttaaaccg gtaaaaacca ccgtttatac ttgatatata tatatatata    1251
   tatttttttt tttcttttga atgaatgtta aaattattca tatcaaaaca tttttacaat    1311
   gactgcatct atagttgata tatatctata tataatttga ctcaataaag aactcaaaaa    1371
   cattcggtaa aaacattgtt tatcaacaca cctaaaacta gtcttggttt gtcttttctg    1431
   atgctttatt tttactgaga gagattcata ctcggcag g ttt ctc gac aag gcg      1485
                                             Phe Leu Asp Lys Ala
                                                             315
   gcg atc ttg act cct gaa gac aag ttg gat gct gat ggt aag cct gtg     1533
   Ala Ile Leu Thr Pro Glu Asp Lys Leu Asp Ala Asp Gly Lys Pro Val
                   320                 325                 330
   aat ccg tgg aat cta tgt aca atg caa cag gtt gaa gaa gtg aag tgc     1581
   Asn Pro Trp Asn Leu Cys Thr Met Gln Gln Val Glu Glu Val Lys Cys
               335                 340                 345
   atc atg aga gtg ctt cct ata tgg ttt gct tca tcg atc tac tac tta        1629
   Ile Met Arg Val Leu Pro Ile Trp Phe Ala Ser Ser Ile Tyr Tyr Leu
           350                 355                 360
   acc atc gcg caa caa atg act tat cct gtc ttc caa gct ctc cag agt        1677
   Thr Ile Ala Gln Gln Met Thr Tyr Pro Val Phe Gln Ala Leu Gln Ser
       365                 370                 375
   gat cgt cgc tta ggc tct gga ggt ttc atg atc ccg gca gcc acc tat        1725
   Asp Arg Arg Leu Gly Ser Gly Gly Phe Met Ile Pro Ala Ala Thr Tyr
   380                 385                 390                 395
   gtt gtc ttc ttg atg act ggc atg acc ctc ttc atc ata ttt tat gac        1773
   Val Val Phe Leu Met Thr Gly Met Thr Leu Phe Ile Ile Phe Tyr Asp
                   400                 405                 410
   cgt gta ttg gtt cct aca tta aaa aga ata acc ggt ata gag acg ggt        1821
   Arg Val Leu Val Pro Thr Leu Lys Arg Ile Thr Gly Ile Glu Thr Gly
               415                 420                 425
   ata acg ctc ttg caa aga att gga gcc ggg att ttc ttc gcc ttg tta        1869
   Ile Thr Leu Leu Gln Arg Ile Gly Ala Gly Ile Phe Phe Ala Leu Leu
           430                 435                 440
   agc tta att atc tct ggt ttc gtc gag gaa cat agg aga acc tca gca        1917
   Ser Leu Ile Ile Ser Gly Phe Val Glu Glu His Arg Arg Thr Ser Ala
       445                 450                 455
   cta act aaa ccg aca ctc ggt tta gcg cca cgg aag gga gaa atc tcc        1965
   Leu Thr Lys Pro Thr Leu Gly Leu Ala Pro Arg Lys Gly Glu Ile Ser
   460                 465                 470                 475
   tca atg tcg gct atg tgg ctg att ccg cag ctt gta ctt gct ggt ata        2013
   Ser Met Ser Ala Met Trp Leu Ile Pro Gln Leu Val Leu Ala Gly Ile
                   480                 485                 490
   gcc gac gca ttt gga gct att gga cag atg gag ttt tac tac aag cag        2061
   Ala Asp Ala Phe Gly Ala Ile Gly Gln Met Glu Phe Tyr Tyr Lys Gln
               495                 500                 505
   ttc cct gag aac atg agg agt ttc gct ggt tct atc ttt tac gta gga        2109
   Phe Pro Glu Asn Met Arg Ser Phe Ala Gly Ser Ile Phe Tyr Val Gly
           510                 515                 520
   gca gga gtt tcg agt tac atc agt agc ttc ttg atc aca acg gtt cac        2157
   Ala Gly Val Ser Ser Tyr Ile Ser Ser Phe Leu Ile Thr Thr Val His
       525                 530                 535
   agg atg acg cag aac tcg gcg ggt ggt aat tgg tta gct gag gat ttg        2205
   Arg Met Thr Gln Asn Ser Ala Gly Gly Asn Trp Leu Ala Glu Asp Leu
   540                 545                 550                 555
   aac aaa gga aga ttg gat tac ttc tat ttc ttg tta gct gga atc ttg        2253
   Asn Lys Gly Arg Leu Asp Tyr Phe Tyr Phe Leu Leu Ala Gly Ile Leu
                   560                 565                 570
   gca gtt aat ttt gct tac ttc ttg gtg atg gca aga tgg tat agg tac        2301
   Ala Val Asn Phe Ala Tyr Phe Leu Val Met Ala Arg Trp Tyr Arg Tyr
               575                 580                 585
   aaa gga agt gat gat gaa gag aca agt tat gaa acc agt gga gat atc        2349
   Lys Gly Ser Asp Asp Glu Glu Thr Ser Tyr Glu Thr Ser Gly Asp Ile
           590                 595                 600
   atc aaa gaa caa gac aag aac aaa gtc tga                                2379
   Ile Lys Glu Gln Asp Lys Asn Lys Val
       605                 610
<210> 60
   <211> 612
   <212> PRT
   <213> Brassica oleracea
<400> 60
   Met Glu Arg Lys Ser Leu Glu Val Glu Ser Met Asn His Gln Lys Leu
   1               5                   10                  15
   Ser Ser Ala Val Tyr Asp Gly Ser Val Thr Ala Val Asp Ser Val Lys
               20                  25                  30
   Glu Asp Val Gln Glu Thr Lys Val Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ser Val Phe Asn Met Lys
   65                  70                  75                  80
   Ser Val Thr Ala Ala Thr Ile Ile Asn Ala Phe Ala Gly Thr Val Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Leu Ala Cys Phe Leu Gly Ser Leu
           115                 120                 125
   Ala Ile Leu Leu Thr Ala Ala Val Pro Gln Leu His Pro Ala Pro Cys
       130                 135                 140
   Gly Thr Ala Ser Ser Cys Ser Gly Pro Ser Gly Gly Gln Ile Ala Phe
   145                 150                 155                 160
   Leu Leu Met Gly Leu Gly Ile Leu Val Val Gly Ala Gly Gly Ile Arg
                   165                 170                 175
   Pro Cys Asn Leu Ala Phe Gly Ala Asp Gln Phe Asn Pro Lys Ser Gly
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Val Val Val Tyr Val Gln
       210                 215                 220
   Ser Asn Val Ser Trp Thr Ile Gly Leu Thr Ile Pro Val Val Leu Met
   225                 230                 235                 240
   Phe Leu Ala Ser Val Ile Phe Phe Ala Gly Ala Lys Leu Tyr Val Lys
                   245                 250                 255
   Ile Glu Ala Ser Gly Ser Pro Leu Ala Ser Ile Ala His Val Ile Thr
               260                 265                 270
   Ala Ala Ile Lys Lys His Gly Leu Lys Ser Val Glu Gln Pro Trp Leu
           275                 280                 285
   Asn Leu Tyr Asn Tyr Tyr Pro Ser Lys His Ala Asn Ala Lys Leu Lys
   290 295 300
   Tyr Thr Glu Gln Phe Arg Phe Leu Asp Lys Ala Ala Ile Leu Thr Pro
   305 310 315 320
   Glu Asp Lys Leu Asp Ala Asp Gly Lys Pro Val Asn Pro Trp Asn Leu
   325 330 335
   Cys Thr Met Gln Gln Val Glu Glu Val Lys Cys Ile Met Arg Val Leu
   340 345 350
   Pro Ile Trp Phe Ala Ser Ser Ile Tyr Tyr Leu Thr Ile Ala Gln Gln
   355 360 365
   Met Thr Tyr Pro Val Phe Gln Ala Leu Gln Ser Asp Arg Arg Leu Gly
   370 375 380
   Ser Gly Gly Phe Met Ile Pro Ala Ala Thr Tyr Val Val Phe Leu Met
   385 390 395 400
   Thr Gly Met Thr Leu Phe Ile Ile Phe Tyr Asp Arg Val Leu Val Pro
   405 410 415
   Thr Leu Lys Arg Ile Thr Gly Ile Glu Thr Gly Ile Thr Leu Leu Gln
   420 425 430
   Arg Ile Gly Ala Gly Ile Phe Phe Ala Leu Leu Ser Leu Ile Ile Ser
   435 440 445
   Gly Phe Val Glu Glu His Arg Arg Thr Ser Ala Leu Thr Lys Pro Thr
   450 455 460
   Leu Gly Leu Ala Pro Arg Lys Gly Glu Ile Ser Ser Met Ser Ala Met
   465 470 475 480
   Trp Leu Ile Pro Gln Leu Val Leu Ala Gly Ile Ala Asp Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Ala Gly Val Ser Ser
   515 520 525
   Tyr Ile Ser Ser Phe Leu Ile Thr Thr Val His Arg Met Thr Gln Asn
   530 535 540
   Ser Ala Gly Gly Asn Trp Leu Ala Glu Asp Leu Asn Lys Gly Arg Leu
   545 550 555 560
   Asp Tyr Phe Tyr Phe Leu Leu Ala Gly Ile Leu Ala Val Asn Phe Ala
   565 570 575
   Tyr Phe Leu Val Met Ala Arg Trp Tyr Arg Tyr Lys Gly Ser Asp Asp
   580 585 590
   Glu Glu Thr Ser Tyr Glu Thr Ser Gly Asp Ile Ile Lys Glu Gln Asp
   595 600 605
   Lys Asn Lys Val
   610
<210> 61
   <211> 2556
   <212> DNA
   <213> Arabidopsis thaliana
<220>
   <221> CDS
   <222> (1)..(127)
<220>
   <221> CDS
   <222> (694)..(911)
<220>
   <221> CDS
   <222> (992)..(1545)
<220>
   <221> CDS
   <222> (1635)..(2553)
<400> 61
   atg gag aga aag cct ctt gag gtt gag cca tct act acg acg act aac         48
   Met Glu Arg Lys Pro Leu Glu Val Glu Pro Ser Thr Thr Thr Thr Asn
   1               5                   10                  15
   acc gat gtg gtc gat tct ttc gag gaa gag cag aga aaa atc gtt tat         96
   Thr Asp Val Val Asp Ser Phe Glu Glu Glu Gln Arg Lys Ile Val Tyr
               20                  25                  30
   aga gga tgg aaa gtc atg cct ttt atc att g gtaacgctcc tttttacttg        147
   Arg Gly Trp Lys Val Met Pro Phe Ile Ile
           35                  40
   tttgtgtcta attcagacat catttttttc ttttggggtg ctttgtcgga ttttagggaa      207
   gtgtaacatt ttgtccgtga tgtttttaaa tttcgtttct tttctcttct ctttgacttg      267
   actctgtcaa aaatctcgaa tttggagttc tttctctgag atttcgaagt gaaattggtt      327
   ttaaaactga gatttcgttg aaacaatgaa acccagataa tcttacagat ctgtctaatg      387
   gaaattatat tttttacaat tgttttcatt ttattttcaa agagactttt gaaatcttta      447
   acacaaaaaa cagagacttt aaatcatgca gttaagtttc tgaaaaaagt agataagatt      507
   tttgtgaatt tctggataag acaaaattag tttatgggag ttacaaaaca gttataaagt      567
   ttcatgtgaa ccaaagtcag aacattggaa gtccaaactt tgatttgtgt gtttccttgt      627
   acattaaatg taaaaaaaaa actgtaactg tattcttaat ttcttatcat taatttgcta      687
   ttgtag gt  aat gag aca ttt gag aag ctt ggg atc ata ggg aca tta         734
          Gly Asn Glu Thr Phe Glu Lys Leu Gly Ile Ile Gly Thr Leu
                  45                  50                  55
   tca aac ctt ctt gtg tac cta act tct gta ttc aac ctt aag agc tac        782
   Ser Asn Leu Leu Val Tyr Leu Thr Ser Val Phe Asn Leu Lys Ser Tyr
               60                  65                  70
   aca gct gca act atc atc aat gcc ttt agt ggt aca atc aat ttc ggg        830
   Thr Ala Ala Thr Ile Ile Asn Ala Phe Ser Gly Thr Ile Asn Phe Gly
           75                  80                  85
   act ttt att gct gcc ttt ctt tgc gac act tac ttt ggt cgc tac aag        878
   Thr Phe Ile Ala Ala Phe Leu Cys Asp Thr Tyr Phe Gly Arg Tyr Lys
       90                  95                  100
   act ctc agt gtc gct gtc atc gct tgt ttt ctg gtactacttt tatatataat     931
   Thr Leu Ser Val Ala Val Ile Ala Cys Phe Leu
   105                 110                 115
   ctttcatgac tccggtttcg ttttcgccaa tgttttctaa accatttttc atctctgtag     991
   gga tcg ttt gtg ata cta ctt act gct gca att ccg tcg ttg cac ccc      1039
   Gly Ser Phe Val Ile Leu Leu Thr Ala Ala Ile Pro Ser Leu His Pro
                   120                 125                 130
   gtt gct tgc gga aac aaa atc tcg tgc gaa ggg cca agt gtg gga cag      1087
   Val Ala Cys Gly Asn Lys Ile Ser Cys Glu Gly Pro Ser Val Gly Gln
               135                 140                 145
   ata ctg ttt ctg ctc atg ggt tta ggc ttt ctt gtg gta ggc gct ggt      1135
   Ile Leu Phe Leu Leu Met Gly Leu Gly Phe Leu Val Val Gly Ala Gly
           150                 155                 160
   ggt atc aga ccg tgt aat tta gcg ttt ggt gct gat cag ttc aac ccc      1183
   Gly Ile Arg Pro Cys Asn Leu Ala Phe Gly Ala Asp Gln Phe Asn Pro
       165                 170                 175
   aaa tcc gaa tca gga aag aaa ggg atc aac agt ttc ttc aac tgg tat      1231
   Lys Ser Glu Ser Gly Lys Lys Gly Ile Asn Ser Phe Phe Asn Trp Tyr
   180                 185                 190                 195
   ttc ttc act ttc acg ttc gcg cag atc atc tcg ctc aca gca gtt gtg      1279
   Phe Phe Thr Phe Thr Phe Ala Gln Ile Ile Ser Leu Thr Ala Val Val
                   200                 205                 210
   tat atc cag tca aat gtg agt tgg aca atc ggt ttg atc att ccg gtg      1327
   Tyr Ile Gln Ser Asn Val Ser Trp Thr Ile Gly Leu Ile Ile Pro Val
               215                 220                 225
   gct ctt atg ttc ttg gcg tgt gtg att ttc ttt gct ggc gat aga ttg      1375
   Ala Leu Met Phe Leu Ala Cys Val Ile Phe Phe Ala Gly Asp Arg Leu
           230                 235                 240
   tat gta aaa gtg aaa gcc tcg ggt agt cca ttg gct ggt att gct cgt      1423
   Tyr Val Lys Val Lys Ala Ser Gly Ser Pro Leu Ala Gly Ile Ala Arg
       245                 250                 255
   gtt ata gca gcc gcg atc aag aaa cga ggt ttg aag ccg gtt aag cag      1471
   Val Ile Ala Ala Ala Ile Lys Lys Arg Gly Leu Lys Pro Val Lys Gln
   260                 265                 270                 275
   cct tgg gtc aat ctt tac aac cat atc ccg tct aac tat gca aat act      1519
   Pro Trp Val Asn Leu Tyr Asn His Ile Pro Ser Asn Tyr Ala Asn Thr
                   280                 285                 290
   act cta aaa tac acc gac cag ttc ag  gtaaattgat atctacttat            1565
   Thr Leu Lys Tyr Thr Asp Gln Phe Arg
               295
   tcttggttct cttgctttat gtttctggct tttgttttgc tgaaaaatgt ttgtttatta     1625
   tgttgccag a ttt cta gac aaa gca gca att atg acc ccc gag gag aag      1674
               Phe Leu Asp Lys Ala Ala Ile Met Thr Pro Glu Glu Lys
                           305                 310
   ttg aac tcc gat gga acg gct tcc gat cca tgg aaa cta tgc acg ttg      1722
   Leu Asn Ser Asp Gly Thr Ala Ser Asp Pro Trp Lys Leu Cys Thr Leu
       315                 320                 325
   cag caa gtg gaa gaa gtg aaa tgc att gta aga gtg att cca atc tgg      1770
   Gln Gln Val Glu Glu Val Lys Cys Ile Val Arg Val Ile Pro Ile Trp
   330                 335                 340                 345
   ttt gca tcc acg ata tac tac ctt gca ata act ata caa atg act tat      1818
   Phe Ala Ser Thr Ile Tyr Tyr Leu Ala Ile Thr Ile Gln Met Thr Tyr
                   350                 355                 360
   ccg gtc ttc caa gcg ctc cag agc gac cga cga tta ggt tct ggt ggc      1866
   Pro Val Phe Gln Ala Leu Gln Ser Asp Arg Arg Leu Gly Ser Gly Gly
               365                 370                 375
   ttc agg att cct gcg gcc acc tat gta gtg ttc ttg atg act ggc atg        1914
   Phe Arg Ile Pro Ala Ala Thr Tyr Val Val Phe Leu Met Thr Gly Met
           380                 385                 390
   aca gtt ttc atc ata ttc tac gac cgt gta ctt gtc ccg tcg ctc aga        1962
   Thr Val Phe Ile Ile Phe Tyr Asp Arg Val Leu Val Pro Ser Leu Arg
       395                 400                 405
   aga gtg acc ggg tta gaa acc ggt ata tca ctc tta caa aga atc gga        2010
   Arg Val Thr Gly Leu Glu Thr Gly Ile Ser Leu Leu Gln Arg Ile Gly
   410                 415                 420                 425
   gct ggg ttt aca ttt gcg ata atg agt tta tta gtt tcc ggg ttt ata        2058
   Ala Gly Phe Thr Phe Ala Ile Met Ser Leu Leu Val Ser Gly Phe Ile
                   430                 435                 440
   gag gaa agg aga aga aac ttt gca ttg acc aaa ccg aca ctc ggg atg        2106
   Glu Glu Arg Arg Arg Asn Phe Ala Leu Thr Lys Pro Thr Leu Gly Met
               445                 450                 455
   gcg cct cgg aca gga gaa atc tca tca atg tca gcg ttg tgg ctg att        2154
   Ala Pro Arg Thr Gly Glu Ile Ser Ser Met Ser Ala Leu Trp Leu Ile
           460                 465                 470
   ccg cag ctt aca ctt gca ggc ata gca gaa gct ttt gct gcc att gga        2202
   Pro Gln Leu Thr Leu Ala Gly Ile Ala Glu Ala Phe Ala Ala Ile Gly
       475                 480                 485
   caa atg gag ttt tac tac aag cag ttt cct gaa aac atg aag agc ttc        2250
   Gln Met Glu Phe Tyr Tyr Lys Gln Phe Pro Glu Asn Met Lys Ser Phe
   490                 495                 500                 505
   gcg ggt tct atc ttt tat gtc ggt gca ggg gtt tcg agc tac ctc gct        2298
   Ala Gly Ser Ile Phe Tyr Val Gly Ala Gly Val Ser Ser Tyr Leu Ala
                   510                 515                 520
   agc ttc ttg ata tca act gtt cat cga aca acc gcg cat tca cct tca        2346
   Ser Phe Leu Ile Ser Thr Val His Arg Thr Thr Ala His Ser Pro Ser
               525                 530                 535
   ggg aat tgg tta gct gag gat ctg aac aaa gcg aaa ttg gat tac ttc        2394
   Gly Asn Trp Leu Ala Glu Asp Leu Asn Lys Ala Lys Leu Asp Tyr Phe
           540                 545                 550
   tac ttc atg ctc aca gga ctc atg gtt gtt aac atg gct tac ttc ttg        2442
   Tyr Phe Met Leu Thr Gly Leu Met Val Val Asn Met Ala Tyr Phe Leu
       555                 560                 565
   ttg atg gct aga tgg tat aga tac aaa gga ggt aat gat gaa gac atc        2490
   Leu Met Ala Arg Trp Tyr Arg Tyr Lys Gly Gly Asn Asp Glu Asp Ile
   570                 575                 580                 585
   act gag att gag acc aat gaa gaa gaa acc aag caa cag cag cta caa        2538
   Thr Glu Ile Glu Thr Asn Glu Glu Glu Thr Lys Gln Gln Gln Leu Gln
                   590                 595                 600
   gac aag aac tct gtc tga                                                2556
   Asp Lys Asn Ser Val
               605
<210> 62
   <211> 606
   <212> PRT
   <213> Arabidopsis thaliana
<400> 62
   Met Glu Arg Lys Pro Leu Glu Val Glu Pro Ser Thr Thr Thr Thr Asn
   1               5                   10                  15
   Thr Asp Val Val Asp Ser Phe Glu Glu Glu Gln Arg Lys Ile Val Tyr
               20                  25                  30
   Arg Gly Trp Lys Val Met Pro Phe Ile Ile Gly Asn Glu Thr Phe Glu
           35                  40                  45
   Lys Leu Gly Ile Ile Gly Thr Leu Ser Asn Leu Leu Val Tyr Leu Thr
       50                  55                  60
   Ser Val Phe Asn Leu Lys Ser Tyr Thr Ala Ala Thr Ile Ile Asn Ala
   65                  70                  75                  80
   Phe Ser Gly Thr Ile Asn Phe Gly Thr Phe Ile Ala Ala Phe Leu Cys
                   85                  90                  95
   Asp Thr Tyr Phe Gly Arg Tyr Lys Thr Leu Ser Val Ala Val Ile Ala
               100                 105                 110
   Cys Phe Leu Gly Ser Phe Val Ile Leu Leu Thr Ala Ala Ile Pro Ser
           115                 120                 125
   Leu His Pro Val Ala Cys Gly Asn Lys Ile Ser Cys Glu Gly Pro Ser
       130                 135                 140
   Val Gly Gln Ile Leu Phe Leu Leu Met Gly Leu Gly Phe Leu Val Val
   145                 150                 155                 160
   Gly Ala Gly Gly Ile Arg Pro Cys Asn Leu Ala Phe Gly Ala Asp Gln
                   165                 170                 175
   Phe Asn Pro Lys Ser Glu Ser Gly Lys Lys Gly Ile Asn Ser Phe Phe
               180                 185                 190
   Asn Trp Tyr Phe Phe Thr Phe Thr Phe Ala Gln Ile Ile Ser Leu Thr
           195                 200                 205
   Ala Val Val Tyr Ile Gln Ser Asn Val Ser Trp Thr Ile Gly Leu Ile
       210                 215                 220
   Ile Pro Val Ala Leu Met Phe Leu Ala Cys Val Ile Phe Phe Ala Gly
   225                 230                 235                 240
   Asp Arg Leu Tyr Val Lys Val Lys Ala Ser Gly Ser Pro Leu Ala Gly
                   245                 250                 255
   Ile Ala Arg Val Ile Ala Ala Ala Ile Lys Lys Arg Gly Leu Lys Pro
               260                 265                 270
   Val Lys Gln Pro Trp Val Asn Leu Tyr Asn His Ile Pro Ser Asn Tyr
           275                 280                 285
   Ala Asn Thr Thr Leu Lys Tyr Thr Asp Gln Phe Arg Phe Leu Asp Lys
       290                 295                 300
   Ala Ala Ile Met Thr Pro Glu Glu Lys Leu Asn Ser Asp Gly Thr Ala
   305                 310                 315                 320
   Ser Asp Pro Trp Lys Leu Cys Thr Leu Gln Gln Val Glu Glu Val Lys
                   325                 330                 335
   Cys Ile Val Arg Val Ile Pro Ile Trp Phe Ala Ser Thr Ile Tyr Tyr
               340                 345                 350
   Leu Ala Ile Thr Ile Gln Met Thr Tyr Pro Val Phe Gln Ala Leu Gln
           355                 360                 365
   Ser Asp Arg Arg Leu Gly Ser Gly Gly Phe Arg Ile Pro Ala Ala Thr
       370                 375                 380
   Tyr Val Val Phe Leu Met Thr Gly Met Thr Val Phe Ile Ile Phe Tyr
   385                 390                 395                 400
   Asp Arg Val Leu Val Pro Ser Leu Arg Arg Val Thr Gly Leu Glu Thr
                   405                 410                 415
   Gly Ile Ser Leu Leu Gln Arg Ile Gly Ala Gly Phe Thr Phe Ala Ile
               420                 425                 430
   Met Ser Leu Leu Val Ser Gly Phe Ile Glu Glu Arg Arg Arg Asn Phe
           435                 440                 445
   Ala Leu Thr Lys Pro Thr Leu Gly Met Ala Pro Arg Thr Gly Glu Ile
       450                 455                 460
   Ser Ser Met Ser Ala Leu Trp Leu Ile Pro Gln Leu Thr Leu Ala Gly
   465                 470                 475                 480
   Ile Ala Glu Ala Phe Ala Ala Ile Gly Gln Met Glu Phe Tyr Tyr Lys
                   485                 490                 495
   Gln Phe Pro Glu Asn Met Lys Ser Phe Ala Gly Ser Ile Phe Tyr Val
               500                 505                 510
   Gly Ala Gly Val Ser Ser Tyr Leu Ala Ser Phe Leu Ile Ser Thr Val
           515                 520                 525
   His Arg Thr Thr Ala His Ser Pro Ser Gly Asn Trp Leu Ala Glu Asp
       530                 535                 540
   Leu Asn Lys Ala Lys Leu Asp Tyr Phe Tyr Phe Met Leu Thr Gly Leu
   545                 550                 555                 560
   Met Val Val Asn Met Ala Tyr Phe Leu Leu Met Ala Arg Trp Tyr Arg
                   565                 570                 575
   Tyr Lys Gly Gly Asn Asp Glu Asp Ile Thr Glu Ile Glu Thr Asn Glu
               580                 585                 590
   Glu Glu Thr Lys Gln Gln Gln Leu Gln Asp Lys Asn Ser Val
           595                 600                 605
<210> 63
   <211> 2393
   <212> DNA
   <213> Arabidopsis thaliana
<220>
   <221> CDS
   <222> (1) .. (163)
<220>
   <221> CDS
   <222> (532)..(749)
<220>
   <221> CDS
   <222> (841)..(1400)
<220>
   <221> CDS
   <222> (1484) .. (2390)
<400> 63
   atg gag aga aag cct ctt gaa ctt gag tcg acg gat aac cac cag aat          48
   Met Glu Arg Lys Pro Leu Glu Leu Glu Ser Thr Asp Asn His Gln Asn
   1               5                   10                  15
   cct tcc tcc gcc gta tac ggt ggc tct gtt acg gcg gtt gac tct gtt          96
   Pro Ser Ser Ala Val Tyr Gly Gly Ser Val Thr Ala Val Asp Ser Val
               20                  25                  30
   gaa gaa gat gtt cag aat cag aag aaa gtc gtt tat aga ggc tgg aaa         144
   Glu Glu Asp Val Gln Asn Gln Lys Lys Val Val Tyr Arg Gly Trp Lys
           35                  40                  45
   gtc atg ccc ttt att att g gtaacactaa tttgatcatt tgcttttctt              193
   Val Met Pro Phe Ile Ile
       50
   ttttgtgtcc aagaagctgt tttttctgtt ggtttcatga agtttgggaa tatccagaaa       253
   aactgtttaa aaaataaatc tataagaaaa acataagtct tgctcgaaga cttgtttctc       313
   tgagcaagga gagttttgtt agattctctg tgttgtctga aagtttgact tagtcaacgg       373
   taatttaaga caaaaaagag tgagatgtcg aattttgtct aagattttag aacatatttg       433
   gtcaacgatc aataagaaag tagaaacaca aatttcatct agttcttgat tcttggtatg       493
   gtgtctgatt atgatcttgt acatgttgtt gttatcag ga  aat gag aca ttt gag       548
                                             Gly Asn Glu Thr Phe Glu
                                                                 60
   aag ctt ggg atc att gga aca cta tca aac ctt ctg gtt tat tta act         596
   Lys Leu Gly Ile Ile Gly Thr Leu Ser Asn Leu Leu Val Tyr Leu Thr
                   65                  70                  75
   gca gtc ttc aac ctg aag agt att aca gct gca acc atc att aac gcc         644
   Ala Val Phe Asn Leu Lys Ser Ile Thr Ala Ala Thr Ile Ile Asn Ala
               80                  85                  90
   ttt agt ggc aca att aat ttc gga act ttc gtt gct gct ttt ctc tgt         692
   Phe Ser Gly Thr Ile Asn Phe Gly Thr Phe Val Ala Ala Phe Leu Cys
           95                  100                 105
   gac act tac ttc ggt cga tac aag act cta agt gtc gcg gtc atc gca         740
   Asp Thr Tyr Phe Gly Arg Tyr Lys Thr Leu Ser Val Ala Val Ile Ala
       110                 115                 120
   tgt ttt ctt gtaacgatcc ttcctctttt ttaatatttt ctttctctac                 789
   Cys Phe Leu
   125
   aacattcggt ttaaagtcaa acaaatctga tcagtaaatt cggttttgta g gga tca        846
                                                            Gly Ser
   ttt gtg ata cta ttg act gca gca gtg cca caa ctg cat cca gct gca         894
   Phe Val Ile Leu Leu Thr Ala Ala Val Pro Gln Leu His Pro Ala Ala
   130                 135                 140                 145
   tgt gga aca gcg gcg gat agc ata tgt aac ggg cca agt gga ggc cag         942
   Cys Gly Thr Ala Ala Asp Ser Ile Cys Asn Gly Pro Ser Gly Gly Gln
                   150                 155                 160
   ata gcg ttt cta ctg atg ggt ctc ggg ttc ctt gta gtg gga gcg ggc      990
   Ile Ala Phe Leu Leu Met Gly Leu Gly Phe Leu Val Val Gly Ala Gly
               165                 170                 175
   ggt att aga ccg tgt aac tta gct ttt ggg gct gat cag ttt aac ccg     1038
   Gly Ile Arg Pro Cys Asn Leu Ala Phe Gly Ala Asp Gln Phe Asn Pro
           180                 185                 190
   aaa agc gaa tca ggg aaa aga ggg att gac agt ttc ttt aat tgg tat     1086
   Lys Ser Glu Ser Gly Lys Arg Gly Ile Asp Ser Phe Phe Asn Trp Tyr
       195                 200                 205
   ttc ttt acc ttc act ttc gcg cag atc ctg tcg ctg act cta gtc gtc     1134
   Phe Phe Thr Phe Thr Phe Ala Gln Ile Leu Ser Leu Thr Leu Val Val
   210                 215                 220                 225
   tac gtt cag tct aat gtc agt tgg acg atc ggt cta acc atc cct gcc     1182
   Tyr Val Gln Ser Asn Val Ser Trp Thr Ile Gly Leu Thr Ile Pro Ala
                   230                 235                 240
   gtt cta atg ttt ttg gcc tgc ttg att ttc ttt gcg ggc gat aag ttg     1230
   Val Leu Met Phe Leu Ala Cys Leu Ile Phe Phe Ala Gly Asp Lys Leu
               245                 250                 255
   tat gtg aag atc aaa gcc tcg ggt agt cca ttg gcc ggt ata gct caa     1278
   Tyr Val Lys Ile Lys Ala Ser Gly Ser Pro Leu Ala Gly Ile Ala Gln
           260                 265                 270
   gtt ata gcg gtt gcg atc aag aaa cgc gga tta aag ccc gcg aaa cag     1326
   Val Ile Ala Val Ala Ile Lys Lys Arg Gly Leu Lys Pro Ala Lys Gln
       275                 280                 285
   cct tgg ctt aac ctt tac aat tac tac cca cca aaa tac gca aac tcc     1374
   Pro Trp Leu Asn Leu Tyr Asn Tyr Tyr Pro Pro Lys Tyr Ala Asn Ser
   290                 295                 300                 305
   aag ctc aaa tac aca gac caa ttc ag  gtaaaaaaaa cacaccccat             1420
   Lys Leu Lys Tyr Thr Asp Gln Phe Arg
                   310
   gaatagtatt ttcactttct ttatatgttt tatgttactg aaaaaaactt tcctatttgg   1480
   cag a ttt ctt gat aaa gcc gct atc ttg act cct gaa gac aag ttg cag   1529
         Phe Leu Asp Lys Ala Ala Ile Leu Thr Pro Glu Asp Lys Leu Gln
         315                 320                 325
   cca gat ggt aag cct gct gat cct tgg aag ctc tgt aca atg caa caa     1577
   Pro Asp Gly Lys Pro Ala Asp Pro Trp Lys Leu Cys Thr Met Gln Gln
   330                 335                 340                 345
   gtt gaa gaa gtg aag tgc atc gtg aga gtg ctt cct ata tgg ttt gca     1625
   Val Glu Glu Val Lys Cys Ile Val Arg Val Leu Pro Ile Trp Phe Ala
                   350                 355                 360
   tca tca att tac tac tta acc ata act caa caa atg act tat ccc gtc     1673
   Ser Ser Ile Tyr Tyr Leu Thr Ile Thr Gln Gln Met Thr Tyr Pro Val
               365                 370                 375
   ttc caa gcc ctt cag agc gac cgt cgc tta ggc tca gga ggt ttt gtg     1721
   Phe Gln Ala Leu Gln Ser Asp Arg Arg Leu Gly Ser Gly Gly Phe Val
           380                 385                 390
   att ccc gca gcc acc tac gtg gtt ttc ttg atg acg gga atg aca gtt     1769
   Ile Pro Ala Ala Thr Tyr Val Val Phe Leu Met Thr Gly Met Thr Val
       395                 400                 405
   ttc atc gta gtc tac gac cgt gta ctt gtg cca acc atg aga aga att     1817
   Phe Ile Val Val Tyr Asp Arg Val Leu Val Pro Thr Met Arg Arg Ile
   410                 415                 420                 425
   act ggt cta gac acc ggg ata acg ctc ttg cag aga att gga act ggc     1865
   Thr Gly Leu Asp Thr Gly Ile Thr Leu Leu Gln Arg Ile Gly Thr Gly
                   430                 435                 440
   att ttc ttc gct acc gcg agt tta gta gtc gcg gga ttt gtc gag gaa     1913
   Ile Phe Phe Ala Thr Ala Ser Leu Val Val Ala Gly Phe Val Glu Glu
               445                 450                 455
   cga agg aga aca ttc gct ctg act aaa cca aca ctc ggt atg gcg cca      1961
   Arg Arg Arg Thr Phe Ala Leu Thr Lys Pro Thr Leu Gly Met Ala Pro
           460                 465                 470
   aga aag gga gaa atc tcg tcg atg tca gct atg tgg ctg att ccg cag      2009
   Arg Lys Gly Glu Ile Ser Ser Met Ser Ala Met Trp Leu Ile Pro Gln
       475                 480                 485
   ctc tca ctg gcg ggt gta gca gag gca ttt gca gcc att gga caa atg      2057
   Leu Ser Leu Ala Gly Val Ala Glu Ala Phe Ala Ala Ile Gly Gln Met
   490                 495                 500                 505
   gag ttt tac tac aag cag ttt ccc gag aac atg agg agt ttc gct gga      2105
   Glu Phe Tyr Tyr Lys Gln Phe Pro Glu Asn Met Arg Ser Phe Ala Gly
                   510                 515                 520
   tcc atc ttt tac gtt gga gga gga gtt tcg agt tac ctt ggt agc ttc      2153
   Ser Ile Phe Tyr Val Gly Gly Gly Val Ser Ser Tyr Leu Gly Ser Phe
               525                 530                 535
   ttg att gca aca gtt cac agg aca aca cag aac tcg tca ggg ggt aac      2201
   Leu Ile Ala Thr Val His Arg Thr Thr Gln Asn Ser Ser Gly Gly Asn
           540                 545                 550
   tgg tta gct gag gat ttg aac aaa gga aga ttg gat ctc ttc tat ttc      2249
   Trp Leu Ala Glu Asp Leu Asn Lys Gly Arg Leu Asp Leu Phe Tyr Phe
       555                 560                 565
   atg atc gct gga atc ctg gca gtt aat ttt gct tat ttc ttg gtg atg      2297
   Met Ile Ala Gly Ile Leu Ala Val Asn Phe Ala Tyr Phe Leu Val Met
   570                 575                 580                 585
   tca aga tgg tat agg tac aaa gga agt gat gac gaa gtg aca aca tat      2345
   Ser Arg Trp Tyr Arg Tyr Lys Gly Ser Asp Asp Glu Val Thr Thr Tyr
                   590                 595                 600
   gaa acc aat gaa aat atc atc aaa cag caa gac aag aac gtt gcc tga      2393
   Glu Thr Asn Glu Asn Ile Ile Lys Gln Gln Asp Lys Asn Val Ala
               605                 610                 615
<210> 64
   <211> 616
   <212> PRT
   <213> Arabidopsis thaliana
<400> 64
   Met Glu Arg Lys Pro Leu Glu Leu Glu Ser Thr Asp Asn His Gln Asn
   1 5 10 15
   Pro Ser Ser Ala Val Tyr Gly Gly Ser Val Thr Ala Val Asp Ser Val
   20 25 30
   Glu Glu Asp Val Gln Asn Gln Lys Lys Val Val Tyr Arg Gly Trp Lys
   35 40 45
   Val Met Pro Phe Ile Ile Gly Asn Glu Thr Phe Glu Lys Leu Gly Ile
   50 55 60
   Ile Gly Thr Leu Ser Asn Leu Leu Val Tyr Leu Thr Ala Val Phe Asn
   65 70 75 80
   Leu Lys Ser Ile Thr Ala Ala Thr Ile Ile Asn Ala Phe Ser Gly Thr
   85 90 95
   Ile Asn Phe Gly Thr Phe Val Ala Ala Phe Leu Cys Asp Thr Tyr Phe
   100 105 110
   Gly Arg Tyr Lys Thr Leu Ser Val Ala Val Ile Ala Cys Phe Leu Gly
   115 120 125
   Ser Phe Val Ile Leu Leu Thr Ala Ala Val Pro Gln Leu His Pro Ala
   130 135 140
   Ala Cys Gly Thr Ala Ala Asp Ser Ile Cys Asn Gly Pro Ser Gly Gly
   145 150 155 160
   Gln Ile Ala Phe Leu Leu Met Gly Leu Gly Phe Leu Val Val Gly Ala
   165 170 175
   Gly Gly Ile Arg Pro Cys Asn Leu Ala Phe Gly Ala Asp Gln Phe Asn
   180 185 190
   Pro Lys Ser Glu Ser Gly Lys Arg Gly Ile Asp Ser Phe Phe Asn Trp
   195 200 205
   Tyr Phe Phe Thr Phe Thr Phe Ala Gln Ile Leu Ser Leu Thr Leu Val
   210 215 220
   Val Tyr Val Gln Ser Asn Val Ser Trp Thr Ile Gly Leu Thr Ile Pro
   225 230 235 240
   Ala Val Leu Met Phe Leu Ala Cys Leu Ile Phe Phe Ala Gly Asp Lys
   245 250 255
   Leu Tyr Val Lys Ile Lys Ala Ser Gly Ser Pro Leu Ala Gly Ile Ala
   260 265 270
   Gln Val Ile Ala Val Ala Ile Lys Lys Arg Gly Leu Lys Pro Ala Lys
   275 280 285
   Gln Pro Trp Leu Asn Leu Tyr Asn Tyr Tyr Pro Pro Lys Tyr Ala Asn
   290 295 300
   Ser Lys Leu Lys Tyr Thr Asp Gln Phe Arg Phe Leu Asp Lys Ala Ala
   305 310 315 320
   Ile Leu Thr Pro Glu Asp Lys Leu Gln Pro Asp Gly Lys Pro Ala Asp
   325 330 335
   Pro Trp Lys Leu Cys Thr Met Gln Gln Val Glu Glu Val Lys Cys Ile
   340 345 350
   Val Arg Val Leu Pro Ile Trp Phe Ala Ser Ser Ile Tyr Tyr Leu Thr
   355 360 365
   Ile Thr Gln Gln Met Thr Tyr Pro Val Phe Gln Ala Leu Gln Ser Asp
   370 375 380
   Arg Arg Leu Gly Ser Gly Gly Phe Val Ile Pro Ala Ala Thr Tyr Val
   385 390 395 400
   Val Phe Leu Met Thr Gly Met Thr Val Phe Ile Val Val Tyr Asp Arg
   405 410 415
   Val Leu Val Pro Thr Met Arg Arg Ile Thr Gly Leu Asp Thr Gly Ile
   420 425 430
   Thr Leu Leu Gln Arg Ile Gly Thr Gly Ile Phe Phe Ala Thr Ala Ser
   435 440 445
   Leu Val Val Ala Gly Phe Val Glu Glu Arg Arg Arg Thr Phe Ala Leu
   450 455 460
   Thr Lys Pro Thr Leu Gly Met Ala Pro Arg Lys Gly Glu Ile Ser Ser
   465 470 475 480
   Met Ser Ala Met Trp Leu Ile Pro Gln Leu Ser Leu Ala Gly Val Ala
   485 490 495
   Glu Ala Phe Ala Ala Ile Gly Gln Met Glu Phe Tyr Tyr Lys Gln Phe
   500 505 510
   Pro Glu Asn Met Arg Ser Phe Ala Gly Ser Ile Phe Tyr Val Gly Gly
   515 520 525
   Gly Val Ser Ser Tyr Leu Gly Ser Phe Leu Ile Ala Thr Val His Arg
   530 535 540
   Thr Thr Gln Asn Ser Ser Gly Gly Asn Trp Leu Ala Glu Asp Leu Asn
   545 550 555 560
   Lys Gly Arg Leu Asp Leu Phe Tyr Phe Met Ile Ala Gly Ile Leu Ala
   565 570 575
   Val Asn Phe Ala Tyr Phe Leu Val Met Ser Arg Trp Tyr Arg Tyr Lys
   580 585 590
   Gly Ser Asp Asp Glu Val Thr Thr Tyr Glu Thr Asn Glu Asn Ile Ile
   595 600 605
   Lys Gln Gln Asp Lys Asn Val Ala
   610 615
<210> 65
   <211> 2371
   <212> DNA
   <213> Brassica rapa
<220>
   <221> CDS
   <222> (1)..(157)
<220>
   <221> CDS
   <222> (525)..(742)
<220>
   <221> CDS
   <222> (833)..(1386)
<220>
   <221> CDS
   <222> (1462)..(2368)
<400> 65
   atg gag aga aag cct ctg gaa gtt gag tcc acc gat cat cag aaa cct        48
   Met Glu Arg Lys Pro Leu Glu Val Glu Ser Thr Asp His Gln Lys Pro
   1               5                   10                  15
   tcc tcc gcc gtg tac gac ggc tct gct acg gcg gtt gat tct gtt gaa        96
   Ser Ser Ala Val Tyr Asp Gly Ser Ala Thr Ala Val Asp Ser Val Glu
               20                  25                  30
   gaa gaa gtt cag gaa acc aaa ctc gtt tat aga ggc tgg aaa gtc atg       144
   Glu Glu Val Gln Glu Thr Lys Leu Val Tyr Arg Gly Trp Lys Val Met
           35                  40                  45
   cct ttc atc ata g gtaacgcata tgtttttaaa catattaaaa atataaattt        197
   Pro Phe Ile Ile
       50
   ttgataaaaa aataaatcat tttctttgat tggtatattt ccaaagtttt agaaactata     257
   tttctagaaa tttcctgtaa ttaaattttg acaattttct tctctgtttc tagaaactat     317
   attttgttgc tttgatttta tcattctttg tgttgttgtc ttaagctttg acttagtcaa     377
   acagtaattt aacccaaaag agtgagatct tgagtttgaa gatgtttcag aacacataaa     437
   tgatcaagaa tctcttgttt gattcttcgt ttggttgcta aggtcaaagt cttatgatct     497
   tttcttcttg catgttgtta ttaccag ga  aac gag aca ttt gag aag ctt ggg     550
                                 Gly Asn Glu Thr Phe Glu Lys Leu Gly
                                             55                  60
   atc att gga aca cta tca aac ctt ctg gtt tac tta act gct gtc ttc       598
   Ile Ile Gly Thr Leu Ser Asn Leu Leu Val Tyr Leu Thr Ala Val Phe
               65                  70                  75
   aac atg aag agt atc aca gcc gca aca atc atc aac gcc ttt agt ggc       646
   Asn Met Lys Ser Ile Thr Ala Ala Thr Ile Ile Asn Ala Phe Ser Gly
           80                  85                  90
   aca atc aac ttc gga acc ttc gtt gct gcc ttc ctt tgc gac act tac       694
   Thr Ile Asn Phe Gly Thr Phe Val Ala Ala Phe Leu Cys Asp Thr Tyr
       95                  100                 105
   ttc ggt cgt tac aag aca cta acc gtc gcg gtc atc gca tgt ttt ctt       742
   Phe Gly Arg Tyr Lys Thr Leu Thr Val Ala Val Ile Ala Cys Phe Leu
   110                 115                 120                 125
   gtaataatcc atcctctata accttttctt cctccacaag caatcttggt ttataaacct     802
   aaaaaaaaaa tctggttctt gttttggtag gga tca ctt gtg ata cta tta act      856
                                    Gly Ser Leu Val Ile Leu Leu Thr
                                                    130
   gct gca gta cca caa ctg cat cca gct cca tgc gga ata gcc ctc acg       904
   Ala Ala Val Pro Gln Leu His Pro Ala Pro Cys Gly Ile Ala Leu Thr
       135                 140                 145
   tgt aca ggc cca agt ggc ggc cag ata gcc ttt cta cta atg ggt ctt       952
   Cys Thr Gly Pro Ser Gly Gly Gln Ile Ala Phe Leu Leu Met Gly Leu
   150                 155                 160                 165
   gcc ttt ctt gtg gtg gga gca ggt ggg att aga ccg tgt aat ctt gct      1000
   Ala Phe Leu Val Val Gly Ala Gly Gly Ile Arg Pro Cys Asn Leu Ala
                   170                 175                 180
   ttt gga gct gat cag ttt aac cct aaa agc gag tca ggg aag aga ggg      1048
   Phe Gly Ala Asp Gln Phe Asn Pro Lys Ser Glu Ser Gly Lys Arg Gly
               185                 190                 195
   att gat agt ttc ttt aac tgg tac ttc ttc acc ttc acg ttc gcg cag      1096
   Ile Asp Ser Phe Phe Asn Trp Tyr Phe Phe Thr Phe Thr Phe Ala Gln
           200                 205                 210
   atc ttg tcg ctg aca cta gtt gtc tac atc cag tct aac gtc agt tgg      1144
   Ile Leu Ser Leu Thr Leu Val Val Tyr Ile Gln Ser Asn Val Ser Trp
       215                 220                 225
   acc atc ggt tta acc ata cct gtc gtg tta atg ttc ttg gcc tgc gtg      1192
   Thr Ile Gly Leu Thr Ile Pro Val Val Leu Met Phe Leu Ala Cys Val
   230                 235                 240                 245
   atc ttc ttt gcg ggt gat aag ttg tat gtc aag atc aaa gcc tca ggt      1240
   Ile Phe Phe Ala Gly Asp Lys Leu Tyr Val Lys Ile Lys Ala Ser Gly
                   250                 255                 260
   agt cca ttg gct agt ata gct caa gtt ata acg gtt gct atc aag aaa      1288
   Ser Pro Leu Ala Ser Ile Ala Gln Val Ile Thr Val Ala Ile Lys Lys
               265                 270                 275
   cgt ggg tta aag gct gtg aag cag cct tgg gtt aac ctt tac aat tac      1336
   Arg Gly Leu Lys Ala Val Lys Gln Pro Trp Val Asn Leu Tyr Asn Tyr
           280                 285                 290
   tac cca cta aac tac gca aac tct aag ctc aag tac acc gac cag ttc      1384
   Tyr Pro Leu Asn Tyr Ala Asn Ser Lys Leu Lys Tyr Thr Asp Gln Phe
       295                 300                 305
   ag  gtaaagacac acggttataa ataatatctt tccacttttg tttacttaga           1436
   Arg
   aagcaagctt tctttttttt ggtag g ttt ctt gat aag gcg gcg atc ttg gct    1489
                                 Phe Leu Asp Lys Ala Ala Ile Leu Ala
                                                 315
   cct gaa gac aag ttg gag gct gat ggt aag cct acg gat cca tgg aag      1537
   Pro Glu Asp Lys Leu Glu Ala Asp Gly Lys Pro Thr Asp Pro Trp Lys
   320                 325                 330                 335
   cta tgt aca atg caa caa gtt gaa gaa gtg aag tgc att gtg aga gtg      1585
   Leu Cys Thr Met Gln Gln Val Glu Glu Val Lys Cys Ile Val Arg Val
                   340                 345                 350
   ctt cct ata tgg ttc gct tca tca atc tat tac ttg acc atc aca caa      1633
   Leu Pro Ile Trp Phe Ala Ser Ser Ile Tyr Tyr Leu Thr Ile Thr Gln
               355                 360                 365
   caa atg acc tac cca gtc ttc caa gcc ctc cag agc gat cgt cgc tta      1681
   Gln Met Thr Tyr Pro Val Phe Gln Ala Leu Gln Ser Asp Arg Arg Leu
           370                 375                 380
   ggc tct gga ggg ttt gtg att cct gca gcc acc tat gtg gtc ttc ttg      1729
   Gly Ser Gly Gly Phe Val Ile Pro Ala Ala Thr Tyr Val Val Phe Leu
       385                 390                 395
   atg acg gga atg act gtt ttc atc ata atc tac gac cgc gta ctc gtg      1777
   Met Thr Gly Met Thr Val Phe Ile Ile Ile Tyr Asp Arg Val Leu Val
   400                 405                 410                 415
   cct acc ttg aga aga atc acc ggt ata gac acc ggg atc acg ctc ttg      1825
   Pro Thr Leu Arg Arg Ile Thr Gly Ile Asp Thr Gly Ile Thr Leu Leu
                   420                 425                 430
   caa agg ata gga acc ggg att ttc ttc gcc ttt ttg agc ttg gta gtg      1873
   Gln Arg Ile Gly Thr Gly Ile Phe Phe Ala Phe Leu Ser Leu Val Val
               435                 440                 445
   tct ggt ttc gtc gag gaa cgg agg agg act ttc gca ttg act aaa cca      1921
   Ser Gly Phe Val Glu Glu Arg Arg Arg Thr Phe Ala Leu Thr Lys Pro
           450                 455                 460
   aca ctc ggt atg gcg cca cgg aag gga gaa atc tcc tca atg tca gct      1969
   Thr Leu Gly Met Ala Pro Arg Lys Gly Glu Ile Ser Ser Met Ser Ala
       465                 470                 475
   atg tgg ctg ata ccg cag ctc aca ctc gcg ggt gta gcc gag gca ttt      2017
   Met Trp Leu Ile Pro Gln Leu Thr Leu Ala Gly Val Ala Glu Ala Phe
   480                 485                 490                 495
   gga gct att gga cag atg gag ttt tac tac aag cag ttc ccc gag aac      2065
   Gly Ala Ile Gly Gln Met Glu Phe Tyr Tyr Lys Gln Phe Pro Glu Asn
                       500                        505                        510
   atg agg agt ttc gcc ggc tcc atc ttc tat gtg gga gga gga gtt tct          2113
   Met Arg Ser Phe Ala Gly Ser Ile Phe Tyr Val Gly Gly Gly Val Ser
               515                     520                     525
   agt tac ctc ggc agc ttc ttg att gca acg gta cac agg acg acg cag          2161
   Ser Tyr Leu Gly Ser Phe Leu Ile Ala Thr Val His Arg Thr Thr Gln
           530                     535                     540
   aac tcg gct ggc ggt aac tgg ttg gct gaa gat ttg aac aaa gga aga          2209
   Asn Ser Ala Gly Gly Asn Trp Leu Ala Glu Asp Leu Asn Lys Gly Arg
       545                     550                     555
   ttg gat ttg ttt tac ttc atg atc gct gga atc ttg gcg gtt aat ttt          2257
   Leu Asp Leu Phe Tyr Phe Met Ile Ala Gly Ile Leu Ala Val Asn Phe
   560                     565                     570                     575
   gct tac ttc ttg gtg atg tca aga tgg tat agg tac aaa gga agt gat          2305
   Ala Tyr Phe Leu Val Met Ser Arg Trp Tyr Arg Tyr Lys Gly Ser Asp
                   580                     585                     590
   gat gaa gtg aca gct tat gaa aca aat gga gat gtg atc aaa caa caa          2353
   Asp Glu Val Thr Ala Tyr Glu Thr Asn Gly Asp Val Ile Lys Gln Gln
               595                     600                     605
   gac aag aac aaa gtc taa                                                  2371
   Asp Lys Asn Lys Val
           610
<210> 66
   <211> 612
   <212> PRT
   <213> Brassica rapa
<400> 66
   Met Glu Arg Lys Pro Leu Glu Val Glu Ser Thr Asp His Gln Lys Pro
   1               5                   10                  15
   Ser Ser Ala Val Tyr Asp Gly Ser Ala Thr Ala Val Asp Ser Val Glu
               20                  25                  30
   Glu Glu Val Gln Glu Thr Lys Leu Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Ile Ala Leu Thr Cys Thr Gly Pro Ser Gly Gly Gln Ile Ala Phe
   145                 150                 155                 160
   Leu Leu Met Gly Leu Ala Phe Leu Val Val Gly Ala Gly Gly Ile Arg
   165 170 175
   Pro Cys Asn Leu Ala Phe Gly Ala Asp Gln Phe Asn Pro Lys Ser Glu
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Leu Val Val Tyr Ile Gln
   210 215 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Ser Ile Ala Gln Val Ile Thr
   260 265 270
   Val Ala Ile Lys Lys Arg Gly Leu Lys Ala Val Lys Gln Pro Trp Val
   275 280 285
   Asn Leu Tyr Asn Tyr Tyr Pro Leu Asn Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Thr Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ser Ser Ile Tyr Tyr Leu Thr Ile Thr Gln Gln
   355 360 365
   Met Thr Tyr Pro Val Phe Gln Ala Leu Gln Ser Asp Arg Arg Leu Gly
   370 375 380
   Ser Gly Gly Phe Val Ile Pro Ala Ala Thr Tyr Val Val Phe Leu Met
   385 390 395 400
   Thr Gly Met Thr Val Phe Ile Ile Ile Tyr Asp Arg Val Leu Val Pro
   405 410 415
   Thr Leu Arg Arg Ile Thr Gly Ile Asp Thr Gly Ile Thr Leu Leu Gln
   420 425 430
   Arg Ile Gly Thr Gly Ile Phe Phe Ala Phe Leu Ser Leu Val Val Ser
   435 440 445
   Gly Phe Val Glu Glu Arg Arg Arg Thr Phe Ala Leu Thr Lys Pro Thr
   450 455 460
   Leu Gly Met Ala Pro Arg Lys Gly Glu Ile Ser Ser Met Ser Ala Met
   465                 470                 475                 480
   Trp Leu Ile Pro Gln Leu Thr Leu Ala Gly Val Ala Glu Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Gly Gly Val Ser Ser
           515                 520                 525
   Tyr Leu Gly Ser Phe Leu Ile Ala Thr Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Leu Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Glu Val Thr Ala Tyr Glu Thr Asn Gly Asp Val Ile Lys Gln Gln Asp
           595                 600                 605
   Lys Asn Lys Val
       610
<210> 67
   <211> 29
   <212> DNA
   <213> Artificial
<220>
   <223> oligonucleotide
<400> 67
   ggcttaatat ggagagaaag cctctggaa 29
<210> 68
   <211> 29
   <212> DNA
   <213> Artificial
<220>
   <223> oligonucleotide
<400> 68
   ggtttaattt agactttgtt cttgtcttg 29
<210> 69
   <211> 20
   <212> DNA
   <213> Artificial
<220>
   <223> oligonucleotide
<400> 69
   acgcctttag tggcacaatc 20
<210> 70
   <211> 20
   <212> DNA
   <213> Artificial
<220>
   <223> oligonucleotide
<400> 70
   cccgtcatca agaagaccac 20
<210> 71
   <211> 20
   <212> DNA
   <213> Artificial
<220>
   <223> oligonucleotide
<400> 71
   gcagtaccac aactgcatcc 20
<210> 72
   <211> 20
   <212> DNA
   <213> Artificial
<220>
   <223> oligonucleotide
<400> 72
   gtgaagcagc cttgggttaa 20
<210> 73
   <211> 20
   <212> DNA
   <213> Artificial
<220>
   <223> oligonucleotide
<400> 73
   ttaacccaag gctgcttcac 20
<210> 74
   <211> 31
   <212> DNA
   <213> Artificial
<220>
   <223> oligonucleotide
<400> 74
   atcgcatgtt ttcttagatc acttgtgata c 31
<210> 75
   <211> 32
   <212> DNA
   <213> Artificial
<220>
   <223> oligonucleotide
<400> 75
   aacatgcgat gaccgcgacg gttagtgtct tg 32
<210> 76
   <211> 33
   <212> DNA
   <213> Artificial
<220>
   <223> oligonucleotide
<400> 76
   agaacagctc tcacgtgtac aggcccaagt ggc 33
<210> 77
   <211> 32
   <212> DNA
   <213> Artificial
<220>
   <223> oligonucleotide
<400> 77
   agctgttctg catggagctg gatgcagttg tg 32
<210> 78
   <211> 31
   <212> DNA
   <213> Artificial
<220>
   <223> oligonucleotide
<400> 78
   aaagcaagtc agggaagaga gggattgata g 31
<210> 79
   <211> 30
   <212> DNA
   <213> Artificial
<220>
   <223> oligonucleotide
<400> 79
   acttgctttt agggttaaac tgatcagctc 30
<210> 80
   <211> 30
   <212> DNA
   <213> Artificial
<220>
   <223> oligonucleotide
<400> 80
   agttagacca tcggtttaac catacctgtc 30
<210> 81
   <211> 30
   <212> DNA
   <213> Artificial
<220>
   <223> oligonucleotide
<400> 81
   atggtctaac tgacgttaga ttggatgtag 30
<210> 82
   <211> 29
   <212> DNA
   <213> Artificial
<220>
   <223> oligonucleotide
<400> 82
   attcatctat tacttgacca tcacacaac 29
<210> 83
   <211> 30
   <212> DNA
   <213> Artificial
<220>
   <223> oligonucleotide
<400> 83
   atagatgaat gaagcgaacc atataggaag 30
<210> 84
   <211> 33
   <212> DNA
   <213> Artificial
<220>
   <223> oligonucleotide
<400> 84
   ggcttaatat ggagagaaag cctcttgaac ttg 33
<210> 85
   <211> 32
   <212> DNA
   <213> Artificial
<220>
   <223> oligonucleotide
<400> 85
   ggtttaattc aggcaacgtt cttgtcttgc tg 32
<210> 86
   <211> 34
   <212> DNA
   <213> Artificial
<220>
   <223> oligonucleotide
<400> 86
   ggcttaatat ggacaatgag aaagggacaa gttc 34
<210> 87
   <211> 28
   <212> DNA
   <213> Artificial
<220>
   <223> oligonucleotide
<400> 87
   attccctatg atataaggca tcgctttc 28
<210> 88
   <211> 29
   <212> DNA
   <213> Artificial
<220>
   <223> oligonucleotide
<400> 88
   atagggaatg aaacattgga gaggcttgc 29
<210> 89
   <211> 33
   <212> DNA
   <213> Artificial
<220>
   <223> oligonucleotide
<400> 89
   atgcccaaca actcagcgat ggaaccgaag aca 33
<210> 90
   <211> 34
   <212> DNA
   <213> Artificial
<220>
   <223> oligonucleotide
<400> 90
   agttgttggg catgttggtg ttgactttca cgtc 34
<210> 91
   <211> 29
   <212> DNA
   <213> Artificial
<220>
   <223> oligonucleotide
<400> 91
   atctgaattg atcagtgagt acgagttta 29
<210> 92
   <211> 30
   <212> DNA
   <213> Artificial
<220>
   <223> oligonucleotide
<400> 92
   aattcagatt cttgaacaaa gctgtgatag 30
<210> 93
   <211> 38
   <212> DNA
   <213> Artificial
<220>
   <223> oligonucleotide
<400> 93
   ggtttaattt attgttcatt actatagttt ctgtaacg 38
<210> 94
   <211> 33
   <212> DNA
   <213> Artificial
<220>
   <223> oligonucleotide
<400> 94
   ggcttaatat ggttttggag gatagaaagg acg 33
<210> 95
   <211> 33
   <212> DNA
   <213> Artificial
<220>
   <223> oligonucleotide
<400> 95
   ggtttaattc atttcatcga tttcttcgaa gtc 33
<210> 96
   <211> 43
   <212> DNA
   <213> Artificial
<220>
   <223> oligonucleotide
<400> 96
   aattaaccct cactaaaggg ttgtaatacg actcactata ggg 43
<210> 97
   <211> 49
   <212> DNA
   <213> Artificial
<220>
   <223> oligonucleotide
<400> 97
   tttttttttt tttttttttt ttttttttta tactcaagct agcctcgag 49
<210> 98
   <211> 21
   <212> DNA
   <213> Artificial
<220>
   <223> oligonucleotide
<400> 98
   tcccgaaatt gattgtacca c 21
<210> 99
   <211> 21
   <212> DNA
   <213> Artificial
<220>
   <223> oligonucleotide
<400> 99
   acagccaaat tcacaccatt c 21
<210> 100
   <211> 21
   <212> DNA
   <213> Artificial
<220>
   <223> oligonucleotide
<400> 100
   cgggagcttc acacacttaa g 21
<210> 101
   <211> 21
   <212> DNA
   <213> Artificial
<220>
   <223> oligonucleotide
<400> 101
   taaaccacca ctcggtatgg c 21
<210> 102
   <211> 21
   <212> DNA
   <213> Artificial
<220>
   <223> oligonucleotide
<400> 102
   ctagcgtcgg ttttgtgaga g 21
<210> 103
   <211> 21
   <212> DNA
   <213> Artificial
<220>
   <223> oligonucleotide
<400> 103
   aaataggaag cgtgtgcaat g 21
<210> 104
   <211> 25
   <212> DNA
   <213> Artificial
<220>
   <223> oligonucleotide
<400> 104
   gcagccttgg gtcaatcttt acaac 25
<210> 105
   <211> 24
   <212> DNA
   <213> Artificial
<220>
   <223> oligonucleotide
<400> 105
   gggggtcata attgctgctt tgtc 24
<210> 106
   <211> 23
   <212> DNA
   <213> Artificial
<220>
   <223> oligonucleotide
<400> 106
   taagtgtcgc ggtcatcgca tgt 23
<210> 107
   <211> 24
   <212> DNA
   <213> Artificial
<220>
   <223> oligonucleotide
<400> 107
   aggaacccga gacccatcag taga 24
<210> 108
   <211> 22
   <212> DNA
   <213> Artificial
<220>
   <223> oligonucleotide
<400> 108
   tctcggttcg gtggcaatgg at 22
<210> 109
   <211> 22
   <212> DNA
   <213> Artificial
<220>
   <223> oligonucleotide
<400> 109
   ctctgtatgg cccaatttcg ag 22
<210> 110
   <211> 32
   <212> DNA
   <213> Artificial
<220>
   <223> oligonucleotide
<400> 110
   ggcttaatgg ttcttagact ggcgagaaac tg 32
<210> 111
   <211> 32
   <212> DNA
   <213> Artificial
<220>
   <223> oligonucleotide
<400> 111
   ggtttaatgt caagcttctc cgctcaatta ta 32
<210> 112
   <211> 31
   <212> DNA
   <213> Artificial
<220>
   <223> oligonucleotide
<400> 112
   ggcttaatcc tcatagattg tcagtactat g 31
<210> 113
   <211> 31
   <212> DNA
   <213> Artificial
<220>
   <223> oligonucleotide
<400> 113
   ggtttaattt tgtttgtcta tatttttggc t 31
<210> 114
   <211> 41
   <212> DNA
   <213> Artificial
<220>
   <223> oligonucleotide
<400> 114
   aggctgaggt ttaatccgac agagttcttg tcttgtagct g 41
<210> 115
   <211> 48
   <212> DNA
   <213> Artificial
<220>
   <223> oligonucleotide
<400> 115
   acctcagcct gggtagcggt ggaatggtga gcaagggcga ggagctgt 48
<210> 116
   <211> 33
   <212> DNA
   <213> Artificial
<220>
   <223> oligonucleotide
<400> 116
   agtttccaga ttacttgtac agctcgtcca tgc 33
<210> 117
   <211> 29
   <212> DNA
   <213> Artificial
<220>
   <223> oligonucleotide
<400> 117
   atctggaaac ttatatattt ttggtattc 29
<210> 118
   <211> 32
   <212> DNA
   <213> Artificial
<220>
   <223> oligonucleotide
<400> 118
   ggtttaatgt tgggtcatga aggttgtgtc tg 32
<210> 119
   <211> 3466
   <212> DNA
   <213> Brassica juncea
<220>
   <221> CDS
   <222> (623)..(779)
   <223> exon 1
<220>
   <221> CDS
   <222> (1118)..(1335)
   <223> exon 2
<220>
   <221> CDS
   <222> (1430)..(1983)
   <223> exon 3
<220>
   <221> CDS
   <222> (2083)..(2989)
   <223> exon 4
<400> 119
   actaatggaa tatagttcct atgatttatc gacgtaaagt aaaaaaatat ctatataaat        60
   acataagatt tatggaataa aaaagaacga aatggataga aataaaatgt caaaataata       120
   aagaagaaga aggataaata aattaaaact ggacgatgga aaataagtgg acttaaaaat       180
   taaaatggac ggagagctta aaagaacgtg ccgttattaa gacagagtgt ttacgaaggg       240
   accacacaac acttgtaatg gaatctagat tttcatggat ttttagttct ttttaacttt       300
   ttttttggat ttttagtttt tacaaagttt aatctcggaa gaaaaagaga aaaaaaacct       360
   tcattaatta atgtttaatc aatattctaa tcactaacac agctctcttt cgtggccaca       420
   ttcacaaatg accattttgt ctagattcca acatctccac ccaccatcct ctttcttctt       480
   accactataa atagagacag gtctaacaat gaagaacaga gtcatcatta gccttttaga      540
   taacacacca tacatattat aaagaaagta caaattttaa ctactatttt agtagttgta       600
   actattatct cgagcagcaa aa atg gag aga aag cct cta gaa gtc gag tca        652
                            Met Glu Arg Lys Pro Leu Glu Val Glu Ser
                            1               5                   10
   acg gat cac caa aag cct ccc tcc gcc gtg tac gat ggc tct gtt atg         700
   Thr Asp His Gln Lys Pro Pro Ser Ala Val Tyr Asp Gly Ser Val Met
                   15                  20                  25
   gcg gtt gat tct gtt gag gaa gaa gtt gag gag acc aaa gtc gtt tac         748
   Ala Val Asp Ser Val Glu Glu Glu Val Glu Glu Thr Lys Val Val Tyr
               30                  35                  40
   aga ggc tgg aaa gtc atg ccc ttt atc att g gtaacacaaa cttttttttt         799
   Arg Gly Trp Lys Val Met Pro Phe Ile Ile
           45                  50
   ataaactttc ttcttttgtt ccaagaactt tttttttaat tgcttttaat gtttttttcc        859
   aaaataaata atcaatagtt gaagaaatga agaggaaaac atcagtcttg cttgaagact       919
   tattggtttg atttaaaaaa aaaatcgtga gattctctgt actgttgtca aaagctttga       979
   cttagtcaac cagtttttta agaaaattaa aaaaaagaga gtgagatcta gaatttgaga      1039
   aggtttcaga acataacttt gaggcatatg agatcaaagt cttatgatca tcttggtttt      1099
   gtttgttggt atcatcag ga  aat gag acg ttc gag aag ctt ggg atc att        1149
                       Gly Asn Glu Thr Phe Glu Lys Leu Gly Ile Ile
                               55                  60
   gga aca cta tca aac ctt ctg gtt tat tta act gct gtc ttc aac atg        1197
   Gly Thr Leu Ser Asn Leu Leu Val Tyr Leu Thr Ala Val Phe Asn Met
       65                  70                  75
   aag agt atc aca gct gca aca atc atc aat gca ttc agt ggc aca atc        1245
   Lys Ser Ile Thr Ala Ala Thr Ile Ile Asn Ala Phe Ser Gly Thr Ile
   80                  85                  90                  95
   aac ttc gga acc ttc gtt gct gct ttc ctc tgc gac act tac ttc ggt        1293
   Asn Phe Gly Thr Phe Val Ala Ala Phe Leu Cys Asp Thr Tyr Phe Gly
                   100                 105                 110
   cga tac aag act cta act gtc gct gtc atc gcc tgt ttt ctt                1335
   Arg Tyr Lys Thr Leu Thr Val Ala Val Ile Ala Cys Phe Leu
               115                 120                 125
   gtaatgatcc ttcctttgca atctttcttg ctccacaagc gttcttagtt taacaaccct      1395
   aaaaatctga taattgttgt tctttttttt gtag gga tca ctt gtg ata cta ttg      1450
                                        Gly Ser Leu Val Ile Leu Leu
                                                        130
   aca gct gca gtg cca caa tta cat cca gct cca tgc gga aca gct ctc         1498
   Thr Ala Ala Val Pro Gln Leu His Pro Ala Pro Cys Gly Thr Ala Leu
           135                 140                 145
   tca tgt atc ggg cca agt ggt ggc cag ata gcg ttt cta ctg ctg ggt         1546
   Ser Cys Ile Gly Pro Ser Gly Gly Gln Ile Ala Phe Leu Leu Leu Gly
       150                 155                 160
   ctc ggg ttt ctt gta gtt gga gca ggc ggg att aga cca tgt aat cta         1594
   Leu Gly Phe Leu Val Val Gly Ala Gly Gly Ile Arg Pro Cys Asn Leu
   165                 170                 175                 180
   gct ttc ggg gct gat cag ttt aac ccc aag agc gag tca gga aaa cga         1642
   Ala Phe Gly Ala Asp Gln Phe Asn Pro Lys Ser Glu Ser Gly Lys Arg
                   185                 190                 195
   ggc atc gat agt ttc ttc aat tgg tac ttc ttc acc ttc acg ttc gcg         1690
   Gly Ile Asp Ser Phe Phe Asn Trp Tyr Phe Phe Thr Phe Thr Phe Ala
               200                 205                 210
   cag atc ctg tcg ctg aca cta atc gtc tat atc cag tcc aat gtc agc         1738
   Gln Ile Leu Ser Leu Thr Leu Ile Val Tyr Ile Gln Ser Asn Val Ser
           215                 220                 225
   tgg aca atc ggt tta acc atc ccg gtc gtt ctc atg ttc ttg gcc tgc         1786
   Trp Thr Ile Gly Leu Thr Ile Pro Val Val Leu Met Phe Leu Ala Cys
       230                 235                 240
   gtg att ttc ttt gct gga gat aag ttg tat gtg aag atc aaa gcc tca         1834
   Val Ile Phe Phe Ala Gly Asp Lys Leu Tyr Val Lys Ile Lys Ala Ser
   245                 250                 255                 260
   ggt agt cca ttg gct ggt ata gct caa gtt ata gcg gtt gca atc aag         1882
   Gly Ser Pro Leu Ala Gly Ile Ala Gln Val Ile Ala Val Ala Ile Lys
                   265                 270                 275
   aaa cgt ggg tta aag cca acg aaa gag cct tgg ctt aac ctt tac aac         1930
   Lys Arg Gly Leu Lys Pro Thr Lys Glu Pro Trp Leu Asn Leu Tyr Asn
               280                 285                 290
   tac tac cca cca aaa tac gca aac tcc aaa ctc aaa tac acc gac cag         1978
   Tyr Tyr Pro Pro Lys Tyr Ala Asn Ser Lys Leu Lys Tyr Thr Asp Gln
           295                 300                 305
   ttt ag  gtaacaaaac acacacacgc caagagttag ttttatattt aatatcatat          2033
   Phe Arg
   ctttccactt ttgttttatg gttactgagg aagtttcctg tttcggcag g ttc ctt         2089
                                                           Phe Leu
   gat aag gcg gcg att ttg gct ccc gaa gac aag ttg gag gct gat ggt         2137
   Asp Lys Ala Ala Ile Leu Ala Pro Glu Asp Lys Leu Glu Ala Asp Gly
           315                 320                 325
   aag cct gcg gat ccc tgg aag ctt tgt aca atg caa caa gtt gaa gaa         2185
   Lys Pro Ala Asp Pro Trp Lys Leu Cys Thr Met Gln Gln Val Glu Glu
       330                 335                 340
   gtg aag tgc att gtg aga gtg ctt cct ata tgg ttt gct gca tcg atc         2233
   Val Lys Cys Ile Val Arg Val Leu Pro Ile Trp Phe Ala Ala Ser Ile
   345                 350                 355                 360
   tat tac ttg acc atc acg caa caa atg act tac ccc gtc ttc caa gcc         2281
   Tyr Tyr Leu Thr Ile Thr Gln Gln Met Thr Tyr Pro Val Phe Gln Ala
                   365                 370                 375
   ctc cag agc gac cgt cgc tta gga tcc aga gga ttt gtg atc ccg gca         2329
   Leu Gln Ser Asp Arg Arg Leu Gly Ser Arg Gly Phe Val Ile Pro Ala
               380                 385                 390
   gcc acc tac gtt gtc ttt ttg atg aca gga atg acg gtt ttc atc ata      2377
   Ala Thr Tyr Val Val Phe Leu Met Thr Gly Met Thr Val Phe Ile Ile
           395                 400                 405
   ttc tac gac cgt gtc ctc gtg cct acc tta aga aga ata acc ggt ata      2425
   Phe Tyr Asp Arg Val Leu Val Pro Thr Leu Arg Arg Ile Thr Gly Ile
       410                 415                 420
   gac acg ggg ata acg cta ctg cag agg att gga acc gga att ttc ttc      2473
   Asp Thr Gly Ile Thr Leu Leu Gln Arg Ile Gly Thr Gly Ile Phe Phe
   425                 430                 435                 440
   gcc ttt gtt agc ttg ata gtc tcc ggt ttc gtc gag gaa cgg agg aga      2521
   Ala Phe Val Ser Leu Ile Val Ser Gly Phe Val Glu Glu Arg Arg Arg
                   445                 450                 455
   acg ttc gcg ctg act aaa cca aca ctt ggt atg gct cca agg aag gga      2569
   Thr Phe Ala Leu Thr Lys Pro Thr Leu Gly Met Ala Pro Arg Lys Gly
               460                 465                 470
   gaa atc tcc tca atg tca gct atg tgg ctg atc cca caa ctc gcc ctt      2617
   Glu Ile Ser Ser Met Ser Ala Met Trp Leu Ile Pro Gln Leu Ala Leu
           475                 480                 485
   gcg ggt ata gct gag gcg ttt gga gct ata gga cag atg gag ttt tac      2665
   Ala Gly Ile Ala Glu Ala Phe Gly Ala Ile Gly Gln Met Glu Phe Tyr
       490                 495                 500
   tac aag cag ttc cct gag aac atg agg agt ttt gct ggt tcg atc ttc      2713
   Tyr Lys Gln Phe Pro Glu Asn Met Arg Ser Phe Ala Gly Ser Ile Phe
   505                 510                 515                 520
   tat gta gga gca gga gtt tcg agt tac ctc ggt agc ttc ttg att gca      2761
   Tyr Val Gly Ala Gly Val Ser Ser Tyr Leu Gly Ser Phe Leu Ile Ala
                   525                 530                 535
   atg gtt cac agg acg acg cag aac tca gct ggt ggt aat tgg ttg gct      2809
   Met Val His Arg Thr Thr Gln Asn Ser Ala Gly Gly Asn Trp Leu Ala
               540                 545                 550
   gag gat ttg aac aaa gga aga ttg gat tac ttc tat ttc atg atc gct      2857
   Glu Asp Leu Asn Lys Gly Arg Leu Asp Tyr Phe Tyr Phe Met Ile Ala
           555                 560                 565
   gga atc ttg gca gtt aat ttt gct tac ttc ttg gtc atg tca aga tgg      2905
   Gly Ile Leu Ala Val Asn Phe Ala Tyr Phe Leu Val Met Ser Arg Trp
       570                 575                 580
   tat aga tac aaa gga agt gat ggt gaa gtg aca act tat gaa acc aat      2953
   Tyr Arg Tyr Lys Gly Ser Asp Gly Glu Val Thr Thr Tyr Glu Thr Asn
   585                 590                 595                 600
   gga gat gcc atc aaa caa caa gac aag aat act gtc tgatgatcat           2999
   Gly Asp Ala Ile Lys Gln Gln Asp Lys Asn Thr Val
                   605                 610
   gggtttctca tcttctggaa tttataaggc atatatagtt tttgttcagc ttcaatcatc    3059
   ttacgccctt gaacttgttt gtagtaagcc aatgagaagt gcggtagtca tatacttgtc    3119
   atggctcttt attttgtttt tttactggtt actatgctgt gatcacatct ctttcgttct    3179
   accattgtgg agagaagaga gtttataaca tcaaatgtat tgcaaataaa aagttgtctt    3239
   gcatttcatg tttctatttc tgtcattcct cataatacaa caggaaaaag gaaacattac    3299
   ttagaaagat acttataaaa agcgatatgt tagagaaatg tagaatacaa tgttgtagag    3359
   ccaaatgacc ccggcccatc cccacccgaa cccttttgtc acaacaaagc tccaattcac    3419
   atagattaca agtagaatag ccacctgttt ggaagaaaat ggtcaag                  3466
<210> 120
   <211> 612
   <212> PRT
   <213> Brassica juncea
<400> 120
   Met Glu Arg Lys Pro Leu Glu Val Glu Ser Thr Asp His Gln Lys Pro
   1               5                   10                  15
   Pro Ser Ala Val Tyr Asp Gly Ser Val Met Ala Val Asp Ser Val Glu
               20                  25                  30
   Glu Glu Val Glu Glu Thr Lys Val Val Tyr Arg Gly Trp Lys Val Met
           35                  40                  45
   Pro Phe Ile Ile Gly Asn Glu Thr Phe Glu Lys Leu Gly Ile Ile Gly
       50                  55                  60
   Thr Leu Ser Asn Leu Leu Val Tyr Leu Thr Ala Val Phe Asn Met Lys
   65                  70                  75                  80
   Ser Ile Thr Ala Ala Thr Ile Ile Asn Ala Phe Ser Gly Thr Ile Asn
                   85                  90                  95
   Phe Gly Thr Phe Val Ala Ala Phe Leu Cys Asp Thr Tyr Phe Gly Arg
               100                 105                 110
   Tyr Lys Thr Leu Thr Val Ala Val Ile Ala Cys Phe Leu Gly Ser Leu
           115                 120                 125
   Val Ile Leu Leu Thr Ala Ala Val Pro Gln Leu His Pro Ala Pro Cys
       130                 135                 140
   Gly Thr Ala Leu Ser Cys Ile Gly Pro Ser Gly Gly Gln Ile Ala Phe
   145                 150                 155                 160
   Leu Leu Leu Gly Leu Gly Phe Leu Val Val Gly Ala Gly Gly Ile Arg
                   165                 170                 175
   Pro Cys Asn Leu Ala Phe Gly Ala Asp Gln Phe Asn Pro Lys Ser Glu
               180                 185                 190
   Ser Gly Lys Arg Gly Ile Asp Ser Phe Phe Asn Trp Tyr Phe Phe Thr
           195                 200                 205
   Phe Thr Phe Ala Gln Ile Leu Ser Leu Thr Leu Ile Val Tyr Ile Gln
       210                 215                 220
   Ser Asn Val Ser Trp Thr Ile Gly Leu Thr Ile Pro Val Val Leu Met
   225                 230                 235                 240
   Phe Leu Ala Cys Val Ile Phe Phe Ala Gly Asp Lys Leu Tyr Val Lys
                   245                 250                 255
   Ile Lys Ala Ser Gly Ser Pro Leu Ala Gly Ile Ala Gln Val Ile Ala
               260                 265                 270
   Val Ala Ile Lys Lys Arg Gly Leu Lys Pro Thr Lys Glu Pro Trp Leu
           275                 280                 285
   Asn Leu Tyr Asn Tyr Tyr Pro Pro Lys Tyr Ala Asn Ser Lys Leu Lys
       290                 295                 300
   Tyr Thr Asp Gln Phe Arg Phe Leu Asp Lys Ala Ala Ile Leu Ala Pro
   305                 310                 315                 320
   Glu Asp Lys Leu Glu Ala Asp Gly Lys Pro Ala Asp Pro Trp Lys Leu
                   325                 330                 335
   Cys Thr Met Gln Gln Val Glu Glu Val Lys Cys Ile Val Arg Val Leu
               340                 345                 350
   Pro Ile Trp Phe Ala Ala Ser Ile Tyr Tyr Leu Thr Ile Thr Gln Gln
           355                 360                 365
   Met Thr Tyr Pro Val Phe Gln Ala Leu Gln Ser Asp Arg Arg Leu Gly
       370                 375                 380
   Ser Arg Gly Phe Val Ile Pro Ala Ala Thr Tyr Val Val Phe Leu Met
   385                 390                 395                 400
   Thr Gly Met Thr Val Phe Ile Ile Phe Tyr Asp Arg Val Leu Val Pro
                   405                 410                 415
   Thr Leu Arg Arg Ile Thr Gly Ile Asp Thr Gly Ile Thr Leu Leu Gln
               420                 425                 430
   Arg Ile Gly Thr Gly Ile Phe Phe Ala Phe Val Ser Leu Ile Val Ser
           435                 440                 445
   Gly Phe Val Glu Glu Arg Arg Arg Thr Phe Ala Leu Thr Lys Pro Thr
       450                 455                 460
   Leu Gly Met Ala Pro Arg Lys Gly Glu Ile Ser Ser Met Ser Ala Met
   465                 470                 475                 480
   Trp Leu Ile Pro Gln Leu Ala Leu Ala Gly Ile Ala Glu Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Ala Gly Val Ser Ser
           515                 520                 525
   Tyr Leu Gly Ser Phe Leu Ile Ala Met Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Tyr Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Gly
               580                 585                 590
   Glu Val Thr Thr Tyr Glu Thr Asn Gly Asp Ala Ile Lys Gln Gln Asp
   595 600 605
   Lys Asn Thr Val
   610
<210> 121
   <211> 4477
   <212> DNA
   <213> Brassica juncea
<220>
   <221> CDS
   <222> (1030)..(1186)
   <223> exon 1
<220>
   <221> CDS
   <222> (1549)..(1766)
   <223> exon 2
<220>
   <221> CDS
   <222> (1856)..(2409)
   <223> exon 3
<220>
   <221> CDS
   <222> (2485)..(3391)
   <223> exon 4
<400> 121
   aataaatgat ttgagaaaaa aataaaatta aaataagaac caataaatcg gaataaaaaa      60
   attagtggat tttaaaagaa attttgtctg actttttctc ccgcaatcca ccaacattaa     120
   attttagcag atacaaattt tagtagatca taatctctta ccaactaatt tgactaaacc     180
   aatcatggtg gaccgatcaa atagtggaag attcgctatt gaatagaaat taagaacaga     240
   tttgtacgtg tgtttcaggt tagctcacta caaatttttt ttgttcttta ccatcattat     300
   ttcatatatt tgcattagct gcaaacgatg ttaaaaattt acattgctta actaaatgat     360
   tctgaaagtg ttgttgtttc atgatttaag ttttcataca tacaaaaata tctaagagtt     420
   gttttcaacc ttatttgtta gtatttagtt tcatttactt cagcattttt tcaaggacta     480
   acttaatata tataacatta tatcatatga aataaaaagg aaactaaaac agtcaatttt     540
   cataagatgg agcatgttct tgagatattt cttatattaa tgatatattt gttaaaatta     600
   aatgaagaca aataaaaagc ataaaaatag aagaagagga gataaatgaa atacgaaaat     660
   ggatgatgga aaacaaatag agagcttaaa agaacgtgcc gttattaaag agtgagtgat     720
   tatgatgggg ccacaacact tgtaatggaa tctacatttt cacctttttt taagtttaat     780
   tttgtaaaca aaaatacttt aattaactaa tttaaaacca aaattataat cactaacaca     840
   gttctctttc acggccatat tcacaaatta ccaatttgtc tatattccga cacatccacc     900
   atcctcttta ccactatata tagagacaac tccaagatga agaaaaaacg gagtcatcat     960
   tattagcctt ttgagacaac aatacaaaat aaagaaaata caactttaaa ctgcttcagt    1020
   agtagcaaa atg gag aga aag cct ctg gaa gtt gag tcc acc gat cat cag    1071
             Met Glu Arg Lys Pro Leu Glu Val Glu Ser Thr Asp His Gln
             1               5                   10
   aaa cct tcc tcc gcc gtc tac gac ggc tct gct acg gcg gtt gat tct      1119
   Lys Pro Ser Ser Ala Val Tyr Asp Gly Ser Ala Thr Ala Val Asp Ser
   15                  20                  25                  30
   gtt gaa gaa gaa gtt cag gaa acc aaa ctc gtt tat aga ggc tgg aaa      1167
   Val Glu Glu Glu Val Gln Glu Thr Lys Leu Val Tyr Arg Gly Trp Lys
                   35                  40                  45
   gtc atg cct ttc atc ata g gtaacgcata tgtttttaaa catattaaaa           1216
   Val Met Pro Phe Ile Ile
               50
   atataaattt tgataaaaaa tatatcattt tctttgattg gtatatttcc aaagttttag    1276
   aaactatatt tgtagaaatt tcctgtaatt aaattttgac aattttcttc tctgtttcta    1336
   gaaactatat tttgttggtt tgattttatt attggtgaga ttctttgtgt tgttgtctta    1396
   agctttgact tagtcaaaca gtaatttaac ccaaaagagt gagatcttga gtttgaagat    1456
   gtttcagaac acataaatga tcaagaatct cttgtttgat tgctaaggtc aaagtcttat    1516
   gatcttttct tcttgcatgt tgttattacc ag ga  aac gag aca ttt gag aag      1568
                                       Gly Asn Glu Thr Phe Glu Lys
                                               55
   ctt ggg atc att gga aca cta tca aac ctt ctg gtt tac tta act gct      1616
   Leu Gly Ile Ile Gly Thr Leu Ser Asn Leu Leu Val Tyr Leu Thr Ala
   60                  65                  70                  75
   gtc ttc aac atg aag agt atc aca gcc gca aca atc atc aac gcc ttt      1664
   Val Phe Asn Met Lys Ser Ile Thr Ala Ala Thr Ile Ile Asn Ala Phe
                   80                  85                  90
   agt ggc aca atc aac ttc gga acc ttc gtt gct gcc ttc ctt tgc gac      1712
   Ser Gly Thr Ile Asn Phe Gly Thr Phe Val Ala Ala Phe Leu Cys Asp
               95                  100                 105
   act tac ttc ggt cgt tac aag aca cta acc gtc gcg gtc atc gca tgt      1760
   Thr Tyr Phe Gly Arg Tyr Lys Thr Leu Thr Val Ala Val Ile Ala Cys
           110                 115                 120
   ttt ctt gtaataatcc atcctctata accttttctt cctccacaag caatcttggt       1816
   Phe Leu
       125
   ttataaacct aaaaaaaaat ctggttcttg ttttggtag gga tca ctt gtg ata       1870
                                              Gly Ser Leu Val Ile
                                                              130
   cta tta act gct gca gta cca caa ctg cat cca gct cca tgc gga ata      1918
   Leu Leu Thr Ala Ala Val Pro Gln Leu His Pro Ala Pro Cys Gly Ile
                   135                 140                 145
   gcc ctc acg tgt aca ggc cca agt ggc ggc cag ata gcc ttt cta cta      1966
   Ala Leu Thr Cys Thr Gly Pro Ser Gly Gly Gln Ile Ala Phe Leu Leu
               150                 155                 160
   atg ggt ctt gcc ttt ctt gtg gtg gga gca ggt ggg att aga ccg tgt      2014
   Met Gly Leu Ala Phe Leu Val Val Gly Ala Gly Gly Ile Arg Pro Cys
           165                 170                 175
   aat ctt gct ttt gga gct gat cag ttt aac cct aaa agc gag tca ggg      2062
   Asn Leu Ala Phe Gly Ala Asp Gln Phe Asn Pro Lys Ser Glu Ser Gly
       180                 185                 190
   aag aga ggg att gat agt ttc ttt aac tgg tac ttc ttc acc ttc acg      2110
   Lys Arg Gly Ile Asp Ser Phe Phe Asn Trp Tyr Phe Phe Thr Phe Thr
   195                 200                 205                 210
   ttc gcg cag atc ttg tcg ctg aca cta gtt gtc tac atc cag tct aac      2158
   Phe Ala Gln Ile Leu Ser Leu Thr Leu Val Val Tyr Ile Gln Ser Asn
               215                 220                 225
   gtc agt tgg acc atc ggt tta acc ata cct gtc gtg tta atg ttc ttg      2206
   Val Ser Trp Thr Ile Gly Leu Thr Ile Pro Val Val Leu Met Phe Leu
               230                 235                 240
   gcc tgc gtg atc ttc ttt gcg ggt gat aag ttg tat gtc aag atc aaa        2254
   Ala Cys Val Ile Phe Phe Ala Gly Asp Lys Leu Tyr Val Lys Ile Lys
           245                 250                 255
   gcc tca ggt agt cca ttg gct agt ata gct caa gtt ata acg gtt gct        2302
   Ala Ser Gly Ser Pro Leu Ala Ser Ile Ala Gln Val Ile Thr Val Ala
       260                 265                 270
   atc aag aaa cgt ggg tta aag gct gtg aag cag cct tgg gtt aac ctt        2350
   Ile Lys Lys Arg Gly Leu Lys Ala Val Lys Gln Pro Trp Val Asn Leu
   275                 280                 285                 290
   tac aat tac tac cca cta aac tac gca aac tct aag ctc aag tac acc        2398
   Tyr Asn Tyr Tyr Pro Leu Asn Tyr Ala Asn Ser Lys Leu Lys Tyr Thr
                   295                 300                 305
   gac cag ttc ag  gtaaagacac acggttataa ataatatctt tccacttttg            2449
   Asp Gln Phe Arg
   tttacttaga aagcaagctt tctttttttt ggtag g ttt ctt gat aag gcg gcg       2503
                                             Phe Leu Asp Lys Ala Ala
                                                             315
   atc ttg gct cct gaa gac aag ttg gag gct gat ggt aag cct acg gat        2551
   Ile Leu Ala Pro Glu Asp Lys Leu Glu Ala Asp Gly Lys Pro Thr Asp
               320                 325                 330
   cca tgg aag cta tgt aca atg caa caa gtt gaa gaa gtg aag tgc att        2599
   Pro Trp Lys Leu Cys Thr Met Gln Gln Val Glu Glu Val Lys Cys Ile
           335                 340                 345
   gtg aga gtg ctt cct ata tgg ttc gct tca tca atc tat tac ttg acc        2647
   Val Arg Val Leu Pro Ile Trp Phe Ala Ser Ser Ile Tyr Tyr Leu Thr
       350                 355                 360
   atc aca caa caa atg acc tac cca gtc ttc caa gcc ctc cag agc gat        2695
   Ile Thr Gln Gln Met Thr Tyr Pro Val Phe Gln Ala Leu Gln Ser Asp
   365                 370                 375                 380
   cgt cgc tta ggc tct gga ggg ttt gtg att cct gca gcc acc tat gtg        2743
   Arg Arg Leu Gly Ser Gly Gly Phe Val Ile Pro Ala Ala Thr Tyr Val
                   385                 390                 395
   gtc ttc ttg atg acg gga atg act gtt ttc atc ata atc tac gac cgc        2791
   Val Phe Leu Met Thr Gly Met Thr Val Phe Ile Ile Ile Tyr Asp Arg
               400                 405                 410
   gta ctc gtg cct acc ttg aga aga atc acc ggt ata gac acc ggg atc        2839
   Val Leu Val Pro Thr Leu Arg Arg Ile Thr Gly Ile Asp Thr Gly Ile
           415                 420                 425
   acg ctc ttg caa agg ata gga acc ggg att ttc ttc gcc ttt ttg agc        2887
   Thr Leu Leu Gln Arg Ile Gly Thr Gly Ile Phe Phe Ala Phe Leu Ser
       430                 435                 440
   ttg gta gtg tct ggt ttc gtc gag gaa cgg agg agg act ttc gca ttg        2935
   Leu Val Val Ser Gly Phe Val Glu Glu Arg Arg Arg Thr Phe Ala Leu
   445                 450                 455                 460
   act aaa cca aca ctc ggt atg gcg cca cgg aag gga gaa atc tcc tca        2983
   Thr Lys Pro Thr Leu Gly Met Ala Pro Arg Lys Gly Glu Ile Ser Ser
                   465                 470                 475
   atg tca gct atg tgg ctg ata ccg cag ctc aca ctc gcg ggt gta gcc        3031
   Met Ser Ala Met Trp Leu Ile Pro Gln Leu Thr Leu Ala Gly Val Ala
               480                 485                 490
   gag gca ttt gga gct att gga cag atg gag ttt tac tac aag cag ttc        3079
   Glu Ala Phe Gly Ala Ile Gly Gln Met Glu Phe Tyr Tyr Lys Gln Phe
           495                 500                 505
   ccc gag aac atg agg agt ttc gcc ggc tcc atc ttc tat gtg gga gga        3127
   Pro Glu Asn Met Arg Ser Phe Ala Gly Ser Ile Phe Tyr Val Gly Gly
       510                 515                 520
   gga gtt tct agt tac ctc ggc agc ttc ttg att gca acg gta cac agg      3175
   Gly Val Ser Ser Tyr Leu Gly Ser Phe Leu Ile Ala Thr Val His Arg
   525                 530                 535                 540
   acg acg cag aac tcg gct ggc ggt aac tgg ttg gct gaa gat ttg aac      3223
   Thr Thr Gln Asn Ser Ala Gly Gly Asn Trp Leu Ala Glu Asp Leu Asn
                   545                 550                 555
   aaa gga aga ttg gat ttg ttt tac ttc atg atc gct gga atc ttg gcg      3271
   Lys Gly Arg Leu Asp Leu Phe Tyr Phe Met Ile Ala Gly Ile Leu Ala
               560                 565                 570
   gtt aat ttt gct tac ttc ttg gtg atg tca aga tgg tat agg tac aaa      3319
   Val Asn Phe Ala Tyr Phe Leu Val Met Ser Arg Trp Tyr Arg Tyr Lys
           575                 580                 585
   gga agt gat gat gaa gtg aca gct tat gaa aca aat gga gat gtg atc      3367
   Gly Ser Asp Asp Glu Val Thr Ala Tyr Glu Thr Asn Gly Asp Val Ile
       590                 595                 600
   aaa caa caa gac aag aac aaa gtc taatgatcat gtggtttctc atcttctgga     3421
   Lys Gln Gln Asp Lys Asn Lys Val
   605                 610
   agcttttaga cttagttttt ggtcagcttc ataccattta caactctttg gactctttga    3481
   aataagctaa gtaatcatat acttgttata gctctttttc ttttttcctg atttgctata    3541
   ttacatcaca tctctttttt ttctatcact gtggaggaga aggagatcat aagttcagat    3601
   gtattacaga ataacaattt gttttgcatt ttaaggatta tgttttctac ttctgatcat    3661
   taccacaaca gaacaaactt aaaaaggaaa gcaaacacaa caaaacacta cttaggaaat    3721
   tgatacttat atgcataaca actttgtatc aaagtttcaa acagagacat cactttcttg    3781
   aatgacattg agaggctctc tctaggcttc tatcagactg tgtaggactg ttgaatggtt    3841
   tcaatgtcaa gacctttaag tctcaccaca gattcaacgt gacccttgag tgtgtgaagc    3901
   tccctcaacc tatcaaagtg taaaagacac tgagatatat aacaatgttc aggttttgag    3961
   tagagtagat gagtctttta cctagcgatc tctgctcttc tcttagcttc ttcagccatt    4021
   tgagacagct cgttgaaatg ggttcgctct gggaacatct tagcatctgg cgcttgcaac    4081
   ccatgcagcg tcctctgcgc atgtgcccat tgtaactccc tttgctcttt cccaaagtct    4141
   ttcttcctag tgaatgcaac ctgtggaaac attcacagag accattataa tcaaacgtgt    4201
   agaaaaagct atcatatatg gaaagaatct ttgatataca taccctttgt tcaataacaa    4261
   gatcccaagc tctgccacta agtgcatagc gtatcaagaa cttgatgatg tcaagaggaa    4321
   tgtagaagac aatgttgtaa agccaaatga ctccagccca tccccaccct atcccttcaa    4381
   ttgcagcaaa gctccaattt gcatagactg caattagagt agccacctgt ttgcaagaaa    4441
   atggtttcaa gaaaacacaa cagcagttta taaact                              4477
<210> 122
   <211> 612
   <212> PRT
   <213> Brassica juncea
<400> 122
   Met Glu Arg Lys Pro Leu Glu Val Glu Ser Thr Asp His Gln Lys Pro
   1               5                   10                  15
   Ser Ser Ala Val Tyr Asp Gly Ser Ala Thr Ala Val Asp Ser Val Glu
               20                  25                  30
   Glu Glu Val Gln Glu Thr Lys Leu Val Tyr Arg Gly Trp Lys Val Met
   35 40 45
   Pro Phe Ile Ile Gly Asn Glu Thr Phe Glu Lys Leu Gly Ile Ile Gly
   50 55 60
   Thr Leu Ser Asn Leu Leu Val Tyr Leu Thr Ala Val Phe Asn Met Lys
   65 70 75 80
   Ser Ile Thr Ala Ala Thr Ile Ile Asn Ala Phe Ser Gly Thr Ile Asn
   85 90 95
   Phe Gly Thr Phe Val Ala Ala Phe Leu Cys Asp Thr Tyr Phe Gly Arg
   100 105 110
   Tyr Lys Thr Leu Thr Val Ala Val Ile Ala Cys Phe Leu Gly Ser Leu
   115 120 125
   Val Ile Leu Leu Thr Ala Ala Val Pro Gln Leu His Pro Ala Pro Cys
   130 135 140
   Gly Ile Ala Leu Thr Cys Thr Gly Pro Ser Gly Gly Gln Ile Ala Phe
   145 150 155 160
   Leu Leu Met Gly Leu Ala Phe Leu Val Val Gly Ala Gly Gly Ile Arg
   165 170 175
   Pro Cys Asn Leu Ala Phe Gly Ala Asp Gln Phe Asn Pro Lys Ser Glu
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Leu Val Val Tyr Ile Gln
   210 215 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Ser Ile Ala Gln Val Ile Thr
   260 265 270
   Val Ala Ile Lys Lys Arg Gly Leu Lys Ala Val Lys Gln Pro Trp Val
   275 280 285
   Asn Leu Tyr Asn Tyr Tyr Pro Leu Asn Tyr Ala Asn Ser Lys Leu Lys
   290 295 300
   Tyr Thr Asp Gln Phe Arg Phe Leu Asp Lys Ala Ala Ile Leu Ala Pro
   305 310 315 320
   Glu Asp Lys Leu Glu Ala Asp Gly Lys Pro Thr Asp Pro Trp Lys Leu
   325 330 335
   Cys Thr Met Gln Gln Val Glu Glu Val Lys Cys Ile Val Arg Val Leu
               340                 345                 350
   Pro Ile Trp Phe Ala Ser Ser Ile Tyr Tyr Leu Thr Ile Thr Gln Gln
           355                 360                 365
   Met Thr Tyr Pro Val Phe Gln Ala Leu Gln Ser Asp Arg Arg Leu Gly
       370                 375                 380
   Ser Gly Gly Phe Val Ile Pro Ala Ala Thr Tyr Val Val Phe Leu Met
   385                 390                 395                 400
   Thr Gly Met Thr Val Phe Ile Ile Ile Tyr Asp Arg Val Leu Val Pro
                   405                 410                 415
   Thr Leu Arg Arg Ile Thr Gly Ile Asp Thr Gly Ile Thr Leu Leu Gln
               420                 425                 430
   Arg Ile Gly Thr Gly Ile Phe Phe Ala Phe Leu Ser Leu Val Val Ser
           435                 440                 445
   Gly Phe Val Glu Glu Arg Arg Arg Thr Phe Ala Leu Thr Lys Pro Thr
       450                 455                 460
   Leu Gly Met Ala Pro Arg Lys Gly Glu Ile Ser Ser Met Ser Ala Met
   465                 470                 475                 480
   Trp Leu Ile Pro Gln Leu Thr Leu Ala Gly Val Ala Glu Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Gly Gly Val Ser Ser
           515                 520                 525
   Tyr Leu Gly Ser Phe Leu Ile Ala Thr Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Leu Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Glu Val Thr Ala Tyr Glu Thr Asn Gly Asp Val Ile Lys Gln Gln Asp
           595                 600                 605
   Lys Asn Lys Val
       610
<210> 123
   <211> 2347
   <212> DNA
   <213> Brassica juncea
<220>
   <221> CDS
   <222> (33)..(189)
   <223> exon 1
<220>
   <221> CDS
   <222> (367)..(584)
   <223> exon 2
<220>
   <221> CDS
   <222> (675)..(1228)
   <223> exon 3
<220>
   <221> CDS
   <222> (1426)..(2332)
   <223> exon 4
<400> 123
<210> 124
   <211> 612
   <212> PRT
   <213> Brassica juncea
<400> 124
   Met Glu Arg Lys Ser Leu Glu Val Glu Ser Met Asp His Gln Lys Leu
   1               5                   10                  15
   Ser Ser Ala Val Tyr Asp Gly Ser Val Thr Ala Val Asp Ser Val Lys
               20                  25                  30
   Glu Asp Val Gln Glu Thr Lys Val Val Tyr Arg Gly Trp Lys Val Met
           35                  40                  45
   Pro Tyr Ile Ile Gly Asn Glu Thr Phe Glu Lys Leu Gly Ile Ile Gly
       50                  55                  60
   Thr Leu Ser Asn Leu Leu Val Tyr Leu Thr Ser Val Phe Asn Met Lys
   65                  70                  75                  80
   Ser Val Thr Ala Ala Thr Ile Ile Thr Ala Phe Ala Gly Thr Val Asn
   85 90 95
   Phe Gly Thr Phe Val Ala Ala Phe Leu Cys Asp Thr Tyr Phe Gly Arg
   100 105 110
   Tyr Lys Thr Leu Thr Val Ala Val Leu Ala Cys Phe Leu Gly Ser Leu
   115 120 125
   Ala Ile Leu Leu Thr Ala Ala Val Pro Gln Leu His Pro Val Pro Cys
   130 135 140
   Gly Thr Ala Ser Ser Cys Ile Gly Pro Ser Gly Gly Gln Ile Ala Phe
   145 150 155 160
   Leu Leu Met Gly Leu Gly Ile Leu Val Val Gly Ala Gly Gly Ile Arg
   165 170 175
   Pro Cys Asn Leu Ala Phe Gly Ala Asp Gln Phe Asn Pro Lys Ser Gly
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Val Val Val Tyr Val Gln
   210 215 220
   Ser Asn Val Ser Trp Thr Ile Gly Leu Thr Ile Pro Val Val Leu Met
   225 230 235 240
   Phe Leu Ala Ser Val Ile Phe Phe Ala Gly Ala Lys Leu Tyr Val Lys
   245 250 255
   Ile Glu Ala Ser Gly Ser Pro Leu Ala Ser Ala Ala His Val Ile Thr
   260 265 270
   Ala Ala Ile Lys Lys His Arg Leu Lys Ser Val Lys Gln Pro Trp Leu
   275 280 285
   Asn Leu Tyr Asn Tyr Tyr Pro Ser Lys Tyr Ala Asn Ala Lys Leu Lys
   290 295 300
   Tyr Thr Glu Gln Phe Arg Phe Leu Asp Lys Ala Ala Ile Leu Thr Pro
   305 310 315 320
   Glu Asp Lys Leu Glu Ala Asp Gly Lys Pro Val Asn Pro Trp Asn Leu
   325 330 335
   Cys Thr Met Gln Gln Val Glu Glu Val Lys Cys Ile Met Arg Val Leu
   340 345 350
   Pro Ile Trp Phe Ala Ser Ser Ile Tyr Tyr Leu Thr Met Ala Gln Gln
   355 360 365
   Met Thr Tyr Pro Val Phe Gln Ala Leu Gln Ser Asp Arg Arg Leu Gly
   370 375 380
   Ser Gly Gly Phe Met Ile Pro Ala Ala Thr Tyr Val Val Phe Leu Met
   385 390 395 400
   Thr Gly Met Thr Leu Phe Ile Ile Phe Tyr Asp Arg Val Leu Val Pro
   405 410 415
   Thr Leu Lys Ser Ile Thr Gly Ile Glu Thr Gly Ile Thr Leu Leu Gln
   420 425 430
   Arg Ile Gly Ala Gly Ile Leu Phe Ala Leu Leu Ser Leu Ile Val Ser
   435 440 445
   Gly Phe Val Glu Glu Arg Arg Arg Thr Leu Ala Leu Thr Gln Pro Thr
   450 455 460
   Leu Gly Leu Ala Pro Arg Lys Gly Glu Ile Ser Ser Met Ser Ala Met
   465 470 475 480
   Trp Leu Ile Pro Gln Leu Val Leu Ala Gly Ile Ala Asp Ala Phe Gly
   485 490 495
   Ala Ile Gly Gln Met Glu Phe Tyr Tyr Lys Gln Phe Pro Glu Asn Met
   500 505 510
   Arg Ser Phe Ala Gly Ser Ile Phe Tyr Val Gly Ala Gly Val Ser Ser
   515 520 525
   Tyr Ile Ser Ser Phe Leu Ile Thr Thr Val His Arg Met Thr Gln Asn
   530 535 540
   Ser Ala Gly Gly Asn Trp Leu Ala Glu Asp Leu Asn Lys Gly Arg Leu
   545 550 555 560
   Asp Tyr Phe Tyr Phe Leu Leu Ala Gly Ile Leu Ala Val Asn Phe Ala
   565 570 575
   Tyr Phe Leu Val Met Ala Arg Trp Tyr Arg Tyr Lys Gly Ser Asp Asp
   580 585 590
   Glu Glu Thr Ser Tyr Glu Thr Ser Gly Asp Ile Ile Lys Glu Gln Asp
   595 600 605
   Lys Asn Lys Val
   610
<210> 125
   <211> 2549
   <212> DNA
   <213> Brassica juncea
<220>
   <221> CDS
   <222> (3)..(218)
<220>
   <221> CDS
   <222> (317)..(870)
<220>
   <221> CDS
   <222> (966)..(1872)
<400> 125
   ga aac gag acg ttt gag aag ctt ggg atc att gga aca cta tca aac         47
      Asn Glu Thr Phe Glu Lys Leu Gly Ile Ile Gly Thr Leu Ser Asn
      1               5                   10                  15
   ctt ctg gtt tat cta act gct gtc ttc aac atg aag agt atc aca gct        95
   Leu Leu Val Tyr Leu Thr Ala Val Phe Asn Met Lys Ser Ile Thr Ala
                   20                  25                  30
   gca aca atc atc aat gca ttc agc ggc aca atc aac ttc gga acc ttc       143
   Ala Thr Ile Ile Asn Ala Phe Ser Gly Thr Ile Asn Phe Gly Thr Phe
               35                  40                  45
   ctt gct gct ttc ctc tgc gac act tac ttc ggt cga tac aag act cta       191
   Leu Ala Ala Phe Leu Cys Asp Thr Tyr Phe Gly Arg Tyr Lys Thr Leu
           50                  55                  60
   acc gtc gct gtc atc gcc tgt ttt ctt gtaatgatca ttccttttgc             238
   Thr Val Ala Val Ile Ala Cys Phe Leu
       65                  70
   aatctttctt gccccacaag ctttcttagt ttaacaaccc taaaaatctg ataaatgttt     298
   tttttttctt tttggtag gga tca ctc gtg ata cta tta aca gct gca gtg       349
                       Gly Ser Leu Val Ile Leu Leu Thr Ala Ala Val
                                   75                  80
   cca cag cta cat cca act ccg tgc gga aca gct atc tca tgt atc ggg       397
   Pro Gln Leu His Pro Thr Pro Cys Gly Thr Ala Ile Ser Cys Ile Gly
       85                  90                  95
   cca agc ggt ggc cag ata gcg ttt ctg ctg ctg ggt ctc ggg ttc ctt       445
   Pro Ser Gly Gly Gln Ile Ala Phe Leu Leu Leu Gly Leu Gly Phe Leu
   100                 105                 110                 115
   gta gtg gga gca ggc ggg att aga ccg tgt aat cta gct ttc gga gct       493
   Val Val Gly Ala Gly Gly Ile Arg Pro Cys Asn Leu Ala Phe Gly Ala
                   120                 125                 130
   gat cag ttt aat ccc aag agc gag tca ggg aaa aga ggg ata gat agt       541
   Asp Gln Phe Asn Pro Lys Ser Glu Ser Gly Lys Arg Gly Ile Asp Ser
               135                 140                 145
   ttc ttc aat tgg tac ttc ttc acc ttc act ttc gcg cag atc ttg tcg       589
   Phe Phe Asn Trp Tyr Phe Phe Thr Phe Thr Phe Ala Gln Ile Leu Ser
           150                 155                 160
   ctg aca ctg atc gtc tat gtc cag tct aac gtc agc tgg acc atc ggt       637
   Leu Thr Leu Ile Val Tyr Val Gln Ser Asn Val Ser Trp Thr Ile Gly
       165                 170                 175
   tta acc atc ccg gtc gtt ctc atg ttc ttg gcc tgt gtg att ttc ttt       685
   Leu Thr Ile Pro Val Val Leu Met Phe Leu Ala Cys Val Ile Phe Phe
   180                 185                 190                 195
   gcg ggt gat aag ttg tat gta aag atc aaa gcc tca ggt agt cct tta       733
   Ala Gly Asp Lys Leu Tyr Val Lys Ile Lys Ala Ser Gly Ser Pro Leu
                   200                 205                 210
   gcc agt ata gct caa gtt ata gcg gtt gca atc aag aaa cgt ggg tta       781
   Ala Ser Ile Ala Gln Val Ile Ala Val Ala Ile Lys Lys Arg Gly Leu
               215                 220                 225
   aag cca acg aaa cag cct tgg ctt aac ctc tac aat tat tac cca cca       829
   Lys Pro Thr Lys Gln Pro Trp Leu Asn Leu Tyr Asn Tyr Tyr Pro Pro
           230                 235                 240
   aag tac gca aac tcc aag ctc aaa tac acc gac cag ttt ag                870
   Lys Tyr Ala Asn Ser Lys Leu Lys Tyr Thr Asp Gln Phe Arg
       245                 250                 255
   gtaacaaaac acacaaatta ccaagaacta gttatatctt tactatcttt ccgtttttgt     930
   tttatggtta ctgagaaagt ttcctgtttt ggcag g ttt ctt gat aag gcg gcg      984
                                            Phe Leu Asp Lys Ala Ala
                                                    260
   atc ttg gct ccc gaa gac aag ttg gag gct gat ggt aag cct gcg gat      1032
   Ile Leu Ala Pro Glu Asp Lys Leu Glu Ala Asp Gly Lys Pro Ala Asp
       265                 270                 275
   cca tgg aag ctt tgt aca atg caa caa gtt gaa gaa gtg aag tgc att      1080
   Pro Trp Lys Leu Cys Thr Met Gln Gln Val Glu Glu Val Lys Cys Ile
   280                 285                 290                 295
   gtg aga gtg ctt cct ata tgg ttt gct gca tcc atc tat tac tta acc      1128
   Val Arg Val Leu Pro Ile Trp Phe Ala Ala Ser Ile Tyr Tyr Leu Thr
                   300                 305                 310
   atc acg caa cag atg acc tat ccc gtc ttt caa gcc ctc cag agc gac      1176
   Ile Thr Gln Gln Met Thr Tyr Pro Val Phe Gln Ala Leu Gln Ser Asp
               315                 320                 325
   cgt cgc tta ggc tcc aga gga ttt gtg atc ccc gca gcc acc tac gtt      1224
   Arg Arg Leu Gly Ser Arg Gly Phe Val Ile Pro Ala Ala Thr Tyr Val
           330                 335                 340
   gtc ttc ttg atg aca gga atg acg gtt ttc atc ata ttc tac gac cgt      1272
   Val Phe Leu Met Thr Gly Met Thr Val Phe Ile Ile Phe Tyr Asp Arg
       345                 350                 355
   gtc ctc gtg cct acc tta aga aga ata aca ggt ata gac acg ggg ata      1320
   Val Leu Val Pro Thr Leu Arg Arg Ile Thr Gly Ile Asp Thr Gly Ile
   360                 365                 370                 375
   acg ctc ttg cag agg att gga acc ggt att ttc ttc gcc ttt ctt agc      1368
   Thr Leu Leu Gln Arg Ile Gly Thr Gly Ile Phe Phe Ala Phe Leu Ser
                   380                 385                 390
   ttg ata gtc tcc ggt ttc gtc gag gaa cgt agg aga acg ttc gcc ctg      1416
   Leu Ile Val Ser Gly Phe Val Glu Glu Arg Arg Arg Thr Phe Ala Leu
               395                 400                 405
   act aaa cca aca ctc ggt atg gct cca agg aag gga gaa atc tct tca      1464
   Thr Lys Pro Thr Leu Gly Met Ala Pro Arg Lys Gly Glu Ile Ser Ser
           410                 415                 420
   atg tca gct atg tgg ctg atc cca caa ctc gcc ctt gcg ggt ata gcc      1512
   Met Ser Ala Met Trp Leu Ile Pro Gln Leu Ala Leu Ala Gly Ile Ala
       425                 430                 435
   gag gcg ttt gga gct att gga cag atg gag ttt tac tac aag caa ttc      1560
   Glu Ala Phe Gly Ala Ile Gly Gln Met Glu Phe Tyr Tyr Lys Gln Phe
   440                 445                 450                 455
   cct gag aac atg agg agt ttt gct ggt tcg atc ttt tat gta gga gca      1608
   Pro Glu Asn Met Arg Ser Phe Ala Gly Ser Ile Phe Tyr Val Gly Ala
                   460                 465                 470
   gga gtt tcg agt tac ctc ggt agc ttc ttg att gca atg gtt cac agg      1656
   Gly Val Ser Ser Tyr Leu Gly Ser Phe Leu Ile Ala Met Val His Arg
               475                 480                 485
   acg acg cag aac tcg gct ggt ggt aac tgg tta gct gag gat ttg aac      1704
   Thr Thr Gln Asn Ser Ala Gly Gly Asn Trp Leu Ala Glu Asp Leu Asn
           490                 495                 500
   aaa gga aga ttg gat ttg ttt tac ttc atg atc gct gga atc ttg gcg      1752
   Lys Gly Arg Leu Asp Leu Phe Tyr Phe Met Ile Ala Gly Ile Leu Ala
       505                 510                 515
   gtt aat ttt gct tat ttc ttg gtc atg tca aga tgg tat aga tac aaa      1800
   Val Asn Phe Ala Tyr Phe Leu Val Met Ser Arg Trp Tyr Arg Tyr Lys
   520                 525                 530                 535
   gga agt gat gat gaa gtg aaa act tat gaa acc aat gga gat gcc atc      1848
   Gly Ser Asp Asp Glu Val Lys Thr Tyr Glu Thr Asn Gly Asp Ala Ile
                   540                 545                 550
   aaa caa caa gac aag aac aat gtc tgatgaactg tagtttctca tcttctggaa      1902
   Lys Gln Gln Asp Lys Asn Asn Val
               555
   gttctaagac ttagtttttg gtcagcttca atcctcgtac actcttggac ttgcttgtag     1962
   taagccatag agaaatgcag tagtcatata cttgttctgg ctctttattt ttgttttttt     2022
   ttcttctggt tactgtgtta cgatcacatc tctttcgttc tatgattgtg gagaaaagag     2082
   agattataac atcaaatgta ttgcaaaata aaatgttgtc ttgcattttg aggtacatgt     2142
   ttgtcagtca tcatcataca acatgactaa ctcaaaagga aacaaactca ccaaaaacat     2202
   tacttaggca tttgatactt acatgatttg tatcaaactt taaaacggag atattgactt     2262
   tttggaagga aactgagacg gttcttgagc tctctccaca attctatgag actgtgtaag     2322
   attgttgaat ggtttgatgt caagaccttt tagtctcaag acagatccaa cgtgatccct     2382
   gagtgtgtga agctctcaaa accttacaaa gtgcaaaaga gaatgagacg aaatagccca     2442
   agtgttccag ttttgtgttc tttagcatga agaggagaga catatttgac ttctgttttt     2502
   tcttttcttt tcttttcttt tccacatgtt atgaaagttg aaactct                   2549
<210> 126
   <211> 559
   <212> PRT
   <213> Brassica juncea
<400> 126
   Asn Glu Thr Phe Glu Lys Leu Gly Ile Ile Gly Thr Leu Ser Asn Leu
   1 5 10 15
   Leu Val Tyr Leu Thr Ala Val Phe Asn Met Lys Ser Ile Thr Ala Ala
   20 25 30
   Thr Ile Ile Asn Ala Phe Ser Gly Thr Ile Asn Phe Gly Thr Phe Leu
   35 40 45
   Ala Ala Phe Leu Cys Asp Thr Tyr Phe Gly Arg Tyr Lys Thr Leu Thr
   50 55 60
   Val Ala Val Ile Ala Cys Phe Leu Gly Ser Leu Val Ile Leu Leu Thr
   65 70 75 80
   Ala Ala Val Pro Gln Leu His Pro Thr Pro Cys Gly Thr Ala Ile Ser
   85 90 95
   Cys Ile Gly Pro Ser Gly Gly Gln Ile Ala Phe Leu Leu Leu Gly Leu
   100 105 110
   Gly Phe Leu Val Val Gly Ala Gly Gly Ile Arg Pro Cys Asn Leu Ala
   115 120 125
   Phe Gly Ala Asp Gln Phe Asn Pro Lys Ser Glu Ser Gly Lys Arg Gly
   130 135 140
   Ile Asp Ser Phe Phe Asn Trp Tyr Phe Phe Thr Phe Thr Phe Ala Gln
   145 150 155 160
   Ile Leu Ser Leu Thr Leu Ile Val Tyr Val Gln Ser Asn Val Ser Trp
   165 170 175
   Thr Ile Gly Leu Thr Ile Pro Val Val Leu Met Phe Leu Ala Cys Val
   180 185 190
   Ile Phe Phe Ala Gly Asp Lys Leu Tyr Val Lys Ile Lys Ala Ser Gly
   195 200 205
   Ser Pro Leu Ala Ser Ile Ala Gln Val Ile Ala Val Ala Ile Lys Lys
   210 215 220
   Arg Gly Leu Lys Pro Thr Lys Gln Pro Trp Leu Asn Leu Tyr Asn Tyr
   225 230 235 240
   Tyr Pro Pro Lys Tyr Ala Asn Ser Lys Leu Lys Tyr Thr Asp Gln Phe
   245 250 255
   Arg Phe Leu Asp Lys Ala Ala Ile Leu Ala Pro Glu Asp Lys Leu Glu
   260 265 270
   Ala Asp Gly Lys Pro Ala Asp Pro Trp Lys Leu Cys Thr Met Gln Gln
   275 280 285
   Val Glu Glu Val Lys Cys Ile Val Arg Val Leu Pro Ile Trp Phe Ala
   290 295 300
   Ala Ser Ile Tyr Tyr Leu Thr Ile Thr Gln Gln Met Thr Tyr Pro Val
   305 310 315 320
   Phe Gln Ala Leu Gln Ser Asp Arg Arg Leu Gly Ser Arg Gly Phe Val
   325 330 335
   Ile Pro Ala Ala Thr Tyr Val Val Phe Leu Met Thr Gly Met Thr Val
   340 345 350
   Phe Ile Ile Phe Tyr Asp Arg Val Leu Val Pro Thr Leu Arg Arg Ile
   355 360 365
   Thr Gly Ile Asp Thr Gly Ile Thr Leu Leu Gln Arg Ile Gly Thr Gly
   370 375 380
   Ile Phe Phe Ala Phe Leu Ser Leu Ile Val Ser Gly Phe Val Glu Glu
   385 390 395 400
   Arg Arg Arg Thr Phe Ala Leu Thr Lys Pro Thr Leu Gly Met Ala Pro
   405 410 415
   Arg Lys Gly Glu Ile Ser Ser Met Ser Ala Met Trp Leu Ile Pro Gln
   420 425 430
   Leu Ala Leu Ala Gly Ile Ala Glu Ala Phe Gly Ala Ile Gly Gln Met
   435 440 445
   Glu Phe Tyr Tyr Lys Gln Phe Pro Glu Asn Met Arg Ser Phe Ala Gly
   450 455 460
   Ser Ile Phe Tyr Val Gly Ala Gly Val Ser Ser Tyr Leu Gly Ser Phe
   465 470 475 480
   Leu Ile Ala Met Val His Arg Thr Thr Gln Asn Ser Ala Gly Gly Asn
                   485                 490                 495
   Trp Leu Ala Glu Asp Leu Asn Lys Gly Arg Leu Asp Leu Phe Tyr Phe
               500                 505                 510
   Met Ile Ala Gly Ile Leu Ala Val Asn Phe Ala Tyr Phe Leu Val Met
           515                 520                 525
   Ser Arg Trp Tyr Arg Tyr Lys Gly Ser Asp Asp Glu Val Lys Thr Tyr
       530                 535                 540
   Glu Thr Asn Gly Asp Ala Ile Lys Gln Gln Asp Lys Asn Asn Val
   545                 550                 555
<210> 127
   <211> 3915
   <212> DNA
   <213> Brassica juncea
<220>
   <221> CDS
   <222> (374)..(530)
   <223> exon 1
<220>
   <221> CDS
   <222> (987)..(1204)
   <223> exon 2
<220>
   <221> CDS
   <222> (1298)..(1851)
   <223> exon 3
<220>
   <221> CDS
   <222> (1941)..(2847)
   <223> exon 4
<400> 127
   ttaaaagaac gtgccgttat taaagagtgg gtgttaataa tgggggccac agcaattgta        60
   atggaatcta cattttcatg aatttcagtt tttttttaaa gttaatttcg gtaagaaaac       120
   acttttgtta attaatttta attttagaac caatattata atcactaaca cagctccctt       180
   tcccggccat attcacaaat taccaatttg tctagattcc gacacatcca ccatcctctt       240
   tcttcttacc actatatata gagacaactc taagaggaag aaagcacaga gtcatcatta       300
   gccttttgag acaacaaaaa catatacaaa ataaagaaac cacaacttta gctactattt       360
   tagtagttgt aac atg gag aga aag cct cta gaa gtt gag tcc acg gac          409
                  Met Glu Arg Lys Pro Leu Glu Val Glu Ser Thr Asp
                  1               5                   10
   cac cag aaa cct tcc tcc gcc gtg tac gat ggc tct gct acg gcg gtt         457
   His Gln Lys Pro Ser Ser Ala Val Tyr Asp Gly Ser Ala Thr Ala Val
           15                  20                  25
   gat tct gtt caa gaa gaa gtt cag gag acg aaa gtc gtt tac aga ggc         505
   Asp Ser Val Gln Glu Glu Val Gln Glu Thr Lys Val Val Tyr Arg Gly
       30                  35                  40
   tgg aaa gtc atg cct ttc atc att g gtaacaacaa aacatattat                 550
   Trp Lys Val Met Pro Phe Ile Ile
   45                  50
   tcacatttaa catcagtctt gctcaaagag ttgttggttt aatttacatt tttaaaaata      610
   tattaatttt ttaaaaaaat atattaaata ttaataataa atataatatt ttctattatt      670
   actaattttt cataatttca gaaactatat ttctatagtt ttagaaattt tgataacttt      730
   cttctatgtt ttttttttct ctgtttcgtg aaacaatatt ttgttggttt catgagttgt      790
   taatttggtt ttattattga ttagattctc tgtgttgttg tcttaagctt tgacttagtc      850
   aaacggtaat ttattgcaaa aagatggaga tcttgaattt gaagatgttc cagaacataa      910
   atgatcaaga ataagtgttt ggtttctgat gtcaaagtct tatgatcatc ttgctcttgc      970
   atgttgttat taccag ga  aac gag aca ttt gag aag ctt ggg atc att gga     1021
                     Gly Asn Glu Thr Phe Glu Lys Leu Gly Ile Ile Gly
                                 55                  60
   aca cta tca aac ctc ctg gtt tac tta acc gct gtc ttc aac atg aag     1069
   Thr Leu Ser Asn Leu Leu Val Tyr Leu Thr Ala Val Phe Asn Met Lys
   65                  70                  75                  80
   agt atc aca gct gca aca atc atc aac gcc ttt agt ggc aca atc aac     1117
   Ser Ile Thr Ala Ala Thr Ile Ile Asn Ala Phe Ser Gly Thr Ile Asn
                   85                  90                  95
   ttc gga acc ttc gtt gct gct ttc ctc tgc gac act tac ttc ggt cgt     1165
   Phe Gly Thr Phe Val Ala Ala Phe Leu Cys Asp Thr Tyr Phe Gly Arg
               100                 105                 110
   tac aag act cta acc gtc gcg gtc atc gca tgt ttt ctt gtaatgatcc      1214
   Tyr Lys Thr Leu Thr Val Ala Val Ile Ala Cys Phe Leu
           115                 120                 125
   atccatcctc tataacacct ttcttcctcc acaagcaatc ttagtttata acctaaaaaa    1274
   aatatgattc atgtttttgg tag gga tca ctt gtg ata cta cta act gct gca    1327
                                 Gly Ser Leu Val Ile Leu Leu Thr Ala Ala
                                                 130                 135
   gta cca caa cta cat cca gct cca tgc gga aca gcc ctt acg tgt aca     1375
   Val Pro Gln Leu His Pro Ala Pro Cys Gly Thr Ala Leu Thr Cys Thr
                   140                 145                 150
   gga cca agt ggt ggc cag ata gcg ttc cta ctg atg ggt ctt ggc ttt     1423
   Gly Pro Ser Gly Gly Gln Ile Ala Phe Leu Leu Met Gly Leu Gly Phe
               155                 160                 165
   ctt gtg gtg gga gca ggt ggg ata aga ccg tgt aat ctt gcc ttc ggt     1471
   Leu Val Val Gly Ala Gly Gly Ile Arg Pro Cys Asn Leu Ala Phe Gly
           170                 175                 180
   gcg gat cag ttt aac ccc aag agc gag tca ggg aag aga ggg att gat     1519
   Ala Asp Gln Phe Asn Pro Lys Ser Glu Ser Gly Lys Arg Gly Ile Asp
       185                 190                 195
   agt ttc ttc aac tgg tac ttc ttc acc ttc acg ttc gcg cag atc ttg     1567
   Ser Phe Phe Asn Trp Tyr Phe Phe Thr Phe Thr Phe Ala Gln Ile Leu
   200                 205                 210                 215
   tcg ctg aca cta gtt gtc tac atc cag tct aac gtc agt tgg acc atc     1615
   Ser Leu Thr Leu Val Val Tyr Ile Gln Ser Asn Val Ser Trp Thr Ile
                   220                 225                 230
   ggt tta acc atc cct gtc gtg tta atg ttc ttg gcc tgc gtg atc ttc     1663
   Gly Leu Thr Ile Pro Val Val Leu Met Phe Leu Ala Cys Val Ile Phe
               235                 240                 245
   ttt gcg ggt gat aag ctg tat gta aag atc aaa gcc tca ggt agt cca     1711
   Phe Ala Gly Asp Lys Leu Tyr Val Lys Ile Lys Ala Ser Gly Ser Pro
           250                 255                 260
   ttg gct agt ata ggt caa gtt ata acg gtt gct atc aag aaa cgt ggg     1759
   Leu Ala Ser Ile Gly Gln Val Ile Thr Val Ala Ile Lys Lys Arg Gly
       265                 270                 275
   tta aag cct gtg aag cag cct tgg ctt aac ctt tac aat tac tac cca        1807
   Leu Lys Pro Val Lys Gln Pro Trp Leu Asn Leu Tyr Asn Tyr Tyr Pro
   280                 285                 290                 295
   cta aac tac gca aac tcc aag ctc aag tac act gac cag ttc ag             1851
   Leu Asn Tyr Ala Asn Ser Lys Leu Lys Tyr Thr Asp Gln Phe Arg
               300                 305
   gtaaagacac acagaccaaa aactagttta atgtcttttc tacttttgtt ttatggttac      1911
   ttagagagaa agctttctat ttttggcag g ttt ctt gat aag gca gcg atc ttt     1965
                                      Phe Leu Asp Lys Ala Ala Ile Phe
                                                      315
   gct cct gaa gac aag ctg gag gct gat gga aag cca gtg gat cca tgg        2013
   Ala Pro Glu Asp Lys Leu Glu Ala Asp Gly Lys Pro Val Asp Pro Trp
       320                 325                 330
   aag ctt tgt aca atg caa caa gtt gaa gaa gtg aag tgc att gtg aga        2061
   Lys Leu Cys Thr Met Gln Gln Val Glu Glu Val Lys Cys Ile Val Arg
   335                 340                 345                 350
   gtg ctt cct ata tgg ttt gct tcc tcg atc tac tac ttg acc atc acg        2109
   Val Leu Pro Ile Trp Phe Ala Ser Ser Ile Tyr Tyr Leu Thr Ile Thr
                   355                 360                 365
   caa cag atg acc tat cct gtc ttc caa gcc ctc cag agc gat cgt cgc        2157
   Gln Gln Met Thr Tyr Pro Val Phe Gln Ala Leu Gln Ser Asp Arg Arg
               370                 375                 380
   tta ggc tct gga gga ttt gtg att cct gca gcc acc tat gtt gtc ttc        2205
   Leu Gly Ser Gly Gly Phe Val Ile Pro Ala Ala Thr Tyr Val Val Phe
           385                 390                 395
   ttg atg aca ggc atg act gtt ttc atc att atc tac gac cgt gtc ctc        2253
   Leu Met Thr Gly Met Thr Val Phe Ile Ile Ile Tyr Asp Arg Val Leu
       400                 405                 410
   gtg cct acc ttg aga aga atc acc ggt cta gac acc ggg atc acg ctc        2301
   Val Pro Thr Leu Arg Arg Ile Thr Gly Leu Asp Thr Gly Ile Thr Leu
   415                 420                 425                 430
   ttg cag agg ata gga act ggg att ttc ttt gcc ttc ttg agc ttg gta        2349
   Leu Gln Arg Ile Gly Thr Gly Ile Phe Phe Ala Phe Leu Ser Leu Val
                   435                 440                 445
   gtc tcc ggt ttc gtc gag gaa cgg agg aga act ttc gcc ttg aca aaa        2397
   Val Ser Gly Phe Val Glu Glu Arg Arg Arg Thr Phe Ala Leu Thr Lys
               450                 455                 460
   cca acg ctc ggt atg gcg cca cgg aag gga gaa atc tcc tca atg tca        2445
   Pro Thr Leu Gly Met Ala Pro Arg Lys Gly Glu Ile Ser Ser Met Ser
           465                 470                 475
   gca atg tgg ctg ata ccg cag ctc aca ctc gcg ggt gta gcc gag gcg        2493
   Ala Met Trp Leu Ile Pro Gln Leu Thr Leu Ala Gly Val Ala Glu Ala
       480                 485                 490
   ttt gga gct att gga cag atg gag ttt tac tac aag cag ttc cct gag        2541
   Phe Gly Ala Ile Gly Gln Met Glu Phe Tyr Tyr Lys Gln Phe Pro Glu
   495                 500                 505                 510
   aac atg agg agt ttc gct ggt tcc atc ttt tac gta gga gga gga gtt        2589
   Asn Met Arg Ser Phe Ala Gly Ser Ile Phe Tyr Val Gly Gly Gly Val
                   515                 520                 525
   tcg agt tac ctc ggt agc ttc ttg att gca acg gtt cac agg acg acg        2637
   Ser Ser Tyr Leu Gly Ser Phe Leu Ile Ala Thr Val His Arg Thr Thr
               530                 535                 540
   cag aac tcg gct ggt ggt aac tgg tta gct gaa gat ttg aac aaa gga        2685
   Gln Asn Ser Ala Gly Gly Asn Trp Leu Ala Glu Asp Leu Asn Lys Gly
           545                 550                 555
   aga ttg gat ttg ttt tac ttc atg atc gct ggg atc ttg gcc gtt aat        2733
   Arg Leu Asp Leu Phe Tyr Phe Met Ile Ala Gly Ile Leu Ala Val Asn
       560                 565                 570
   ttt gct tat ttc ctg gtg atg tca aga tgg tat agg tac aaa gga agt      2781
   Phe Ala Tyr Phe Leu Val Met Ser Arg Trp Tyr Arg Tyr Lys Gly Ser
   575                 580                 585                 590
   gat gat gat gtg aca act tgt gaa acc aat ggt gat gtg atc aaa caa      2829
   Asp Asp Asp Val Thr Thr Cys Glu Thr Asn Gly Asp Val Ile Lys Gln
                   595                 600                 605
   caa gac aag aac aaa gtc taatgatcat gtggtttctc atcttctgga             2877
   Gln Asp Lys Asn Lys Val
               610
   agcttttaga cttagtttct ggtcagcttc aatccattca tactctttga actttcttgg    2937
   aataagctaa gtaatcatat actacttgaa gctctctttt attatttcct ggttactatg    2997
   ttacatcaca ttcacatctc tttctttcta tcattgtgga ggaaaaggag atcataactt    3057
   cagatgtaat acagaataac aatttgtttt gcatttaagg gtaatgtttc tacttcatga    3117
   tcattcctcg tcattcaaca gaacaagctt aaaaacgaaa gcaaacaagc aaaaccacta    3177
   cttaggaata tatgcataac ttgtatctaa gtttcaaaca gagacaccac tttcttgaag    3237
   gacattgaga tggttcctga gctctctctc tctctaggct tctatcagac tgtgtaagac    3297
   tgttgaatgg tttcaatgtc aagaccttta agtctcacga cagattcaac gtgacccttg    3357
   agtgtgtgaa gctccctcaa cctatcaaag tgtaaaagag aatgagacaa ataacaaatg    3417
   ttccagttaa tgtgacttca caggaaggaa actagtaaag aaagcagatg ggtcttttac    3477
   ctagcgatct ctgctcttct cttagcttct tcagccattt gggaaagctc gttgaaatgg    3537
   gttcgctctg ggaacatctt agcatccggt gcttgcaggc catgcagcgt cctctgtgca    3597
   tgtgcccatt gtaactccct ttgctctttc ccaaaatcct tcttcctagt gaatgcaatc    3657
   tatggaacaa catacacata caccattaca atcaaagatg tctccacaat acataaatgg    3717
   aaagaatctt gatatacata ccctttgttc aataacaaga tcccaagctc tgccactaag    3777
   ggcataccga atcaagaact tgatgatgtc aagaggaatg tagaacacaa tgttgtaaag    3837
   ccaaatgact ccagcccatc cccacccaat cccttcaata gcagcaaagc tccaatttgc    3897
   atagactgca attagagt                                                  3915
<210> 128
   <211> 612
   <212> PRT
   <213> Brassica juncea
<400> 128
   Met Glu Arg Lys Pro Leu Glu Val Glu Ser Thr Asp His Gln Lys Pro
   1 5 10 15
   Ser Ser Ala Val Tyr Asp Gly Ser Ala Thr Ala Val Asp Ser Val Gln
   20 25 30
   Glu Glu Val Gln Glu Thr Lys Val Val Tyr Arg Gly Trp Lys Val Met
   35 40 45
   Pro Phe Ile Ile Gly Asn Glu Thr Phe Glu Lys Leu Gly Ile Ile Gly
   50 55 60
   Thr Leu Ser Asn Leu Leu Val Tyr Leu Thr Ala Val Phe Asn Met Lys
   65 70 75 80
   Ser Ile Thr Ala Ala Thr Ile Ile Asn Ala Phe Ser Gly Thr Ile Asn
   85 90 95
   Phe Gly Thr Phe Val Ala Ala Phe Leu Cys Asp Thr Tyr Phe Gly Arg
   100 105 110
   Tyr Lys Thr Leu Thr Val Ala Val Ile Ala Cys Phe Leu Gly Ser Leu
   115 120 125
   Val Ile Leu Leu Thr Ala Ala Val Pro Gln Leu His Pro Ala Pro Cys
   130 135 140
   Gly Thr Ala Leu Thr Cys Thr Gly Pro Ser Gly Gly Gln Ile Ala Phe
   145 150 155 160
   Leu Leu Met Gly Leu Gly Phe Leu Val Val Gly Ala Gly Gly Ile Arg
   165 170 175
   Pro Cys Asn Leu Ala Phe Gly Ala Asp Gln Phe Asn Pro Lys Ser Glu
   180 185 190
   Ser Gly Lys Arg Gly Ile Asp Ser Phe Phe Asn Trp Tyr Phe Phe Thr
   195 200 205
   Phe Thr Phe Ala Gln Ile Leu Ser Leu Thr Leu Val Val Tyr Ile Gln
   210 215 220
   Ser Asn Val Ser Trp Thr Ile Gly Leu Thr Ile Pro Val Val Leu Met
   225 230 235 240
   Phe Leu Ala Cys Val Ile Phe Phe Ala Gly Asp Lys Leu Tyr Val Lys
   245 250 255
   Ile Lys Ala Ser Gly Ser Pro Leu Ala Ser Ile Gly Gln Val Ile Thr
   260 265 270
   Val Ala Ile Lys Lys Arg Gly Leu Lys Pro Val Lys Gln Pro Trp Leu
   275 280 285
   Asn Leu Tyr Asn Tyr Tyr Pro Leu Asn Tyr Ala Asn Ser Lys Leu Lys
   290 295 300
   Tyr Thr Asp Gln Phe Arg Phe Leu Asp Lys Ala Ala Ile Phe Ala Pro
   305 310 315 320
   Glu Asp Lys Leu Glu Ala Asp Gly Lys Pro Val Asp Pro Trp Lys Leu
   325 330 335
   Cys Thr Met Gln Gln Val Glu Glu Val Lys Cys Ile Val Arg Val Leu
   340 345 350
   Pro Ile Trp Phe Ala Ser Ser Ile Tyr Tyr Leu Thr Ile Thr Gln Gln
   355 360 365
   Met Thr Tyr Pro Val Phe Gln Ala Leu Gln Ser Asp Arg Arg Leu Gly
               370                 375                 380
   Ser Gly Gly Phe Val Ile Pro Ala Ala Thr Tyr Val Val Phe Leu Met
   385                 390                 395                 400
   Thr Gly Met Thr Val Phe Ile Ile Ile Tyr Asp Arg Val Leu Val Pro
                   405                 410                 415
   Thr Leu Arg Arg Ile Thr Gly Leu Asp Thr Gly Ile Thr Leu Leu Gln
               420                 425                 430
   Arg Ile Gly Thr Gly Ile Phe Phe Ala Phe Leu Ser Leu Val Val Ser
           435                 440                 445
   Gly Phe Val Glu Glu Arg Arg Arg Thr Phe Ala Leu Thr Lys Pro Thr
       450                 455                 460
   Leu Gly Met Ala Pro Arg Lys Gly Glu Ile Ser Ser Met Ser Ala Met
   465                 470                 475                 480
   Trp Leu Ile Pro Gln Leu Thr Leu Ala Gly Val Ala Glu Ala Phe Gly
                   485                 490                 495
   Ala Ile Gly Gln Met Glu Phe Tyr Tyr Lys Gln Phe Pro Glu Asn Met
               500                 505                 510
   Arg Ser Phe Ala Gly Ser Ile Phe Tyr Val Gly Gly Gly Val Ser Ser
           515                 520                 525
   Tyr Leu Gly Ser Phe Leu Ile Ala Thr Val His Arg Thr Thr Gln Asn
       530                 535                 540
   Ser Ala Gly Gly Asn Trp Leu Ala Glu Asp Leu Asn Lys Gly Arg Leu
   545                 550                 555                 560
   Asp Leu Phe Tyr Phe Met Ile Ala Gly Ile Leu Ala Val Asn Phe Ala
                   565                 570                 575
   Tyr Phe Leu Val Met Ser Arg Trp Tyr Arg Tyr Lys Gly Ser Asp Asp
               580                 585                 590
   Asp Val Thr Thr Cys Glu Thr Asn Gly Asp Val Ile Lys Gln Gln Asp
           595                 600                 605
   Lys Asn Lys Val
       610
<210> 129
   <211> 3545
   <212> DNA
   <213> Brassica juncea
<220>
   <221> CDS
   <222> (790)..(928)
   <223> exon 1
<220>
   <221> CDS
   <222> (1181)..(1398)
   <223> exon 2
<220>
   <221> CDS
   <222> (1482)..(2035)
   <223> exon 3
<220>
   <221> CDS
   <222> (2227)..(3133)
   <223> exon 4
<400> 129
   attaatttat ttatttttgt aattattact atttttaagt atttttatct ttttatttta        60
   aaacataata taatttgaaa aatctttatt atttttgttc cttttttaaa agatatcaaa       120
   tatcaaatag tcaaatacta ttagttggtg aatctaaaag ttcaccatag ggagtgaacc       180
   caataattac tcatttaaga ataagaagta gaaatatatc ataagaatgt agaaacaaaa       240
   ctgacaaaaa gcttaagaca attttttcgc atttttagaa gaaacctttt atttatatga       300
   aatttataca tataatgact tattacattt ctccatatat acataaaaca taagttaatg       360
   tacatctttt gaaaacataa gctaacattt ataggaacgt gctgttatta agagatagtt       420
   gcttgtgatg gggccatagc acttgtaatg gaatctagat ttccatggat tttcgttttt       480
   ttttctttaa agtttagttt tggaagaaaa aagaaaaata aaggtttgat taagtaattg       540
   taaaataaac attataatca ctaactaaca catgtctctt tcatggccat attcataaat       600
   taccgatttg tctagattcc gacagctcca cccaggactg tctccaccat cttctttatt       660
   ctgaacacta taaatagagt caggtccaag atgaagaaca tagtcattag cctttgagac       720
   aacaacaaca tacaaaaata aataaagtac caatttaacg gttactttag tagtaataat       780
   tactacaaa atg gag aga aag cct ctc gaa gtc gag tcg aca gat cac caa       831
             Met Glu Arg Lys Pro Leu Glu Val Glu Ser Thr Asp His Gln
             1               5                   10
   aaa ctt tcc tcc gcc gtg tac gat ggc cct gtt gaa gaa gat gtc cag        879
   Lys Leu Ser Ser Ala Val Tyr Asp Gly Pro Val Glu Glu Asp Val Gln
   15                  20                  25                  30
   gag acc aaa gtc gtt tat aga ggc tgg aaa gtc atg cct ttc atc att g      928
   Glu Thr Lys Val Val Tyr Arg Gly Trp Lys Val Met Pro Phe Ile Ile
                   35                  40                  45
   gtaacacaaa aatacaatca cttgccgtgc tctataaact ttcttcttgt ggaaactatt       988
   ggtttcacga agttaagaaa accccgaata gagtaattct tgatcaacaa gaaacaataa      1048
   cctagaatca actatgacaa aatctcattt ttaaaaactt tcttcttttg gaaactattt      1108
   cttttgtcgt gttgttgtct taagatcaaa gtcttaggtt cttggttatt tttttgttgt      1168
   tgttaccacc ag ga  aat gag aca ttt gag aag ctt ggg atc att gga aca      1218
                   Gly Asn Glu Thr Phe Glu Lys Leu Gly Ile Ile Gly Thr
                               50                  55
   cta tca aac ctt ctg gtt tat tta act gct gtc ttc aac atg aag agt        1266
   Leu Ser Asn Leu Leu Val Tyr Leu Thr Ala Val Phe Asn Met Lys Ser
   60                  65                  70                  75
   atc aca gct gca aca atc atc aat gcc ttt gct ggc aca gtc aac ttt        1314
   Ile Thr Ala Ala Thr Ile Ile Asn Ala Phe Ala Gly Thr Val Asn Phe
                   80                  85                  90
   gga act ttc gtt gct gct ttc ctc tgt gac act tac ttc ggt cga tac        1362
   Gly Thr Phe Val Ala Ala Phe Leu Cys Asp Thr Tyr Phe Gly Arg Tyr
               95                  100                 105
   aag act ctt acc gcc gcg gtc atc gcc tgt ttt ctt gtaaagatcc        1408
   Lys Thr Leu Thr Ala Ala Val Ile Ala Cys Phe Leu
           110                 115
   gtcctctcta actctttctt cctccacaag aaatcttggt ttataacctt taaatctaat     1468
   tcttgttttg tag gga tca ctt gcg ata cta ttg act gct gca gta cca      1517
                  Gly Ser Leu Ala Ile Leu Leu Thr Ala Ala Val Pro
                  120                 125                 130
   caa ctc cat cca gct cca tgc gga aca gcg agc tcg tgt atc ggg cca     1565
   Gln Leu His Pro Ala Pro Cys Gly Thr Ala Ser Ser Cys Ile Gly Pro
               135                 140                 145
   agt ggt ggc cag ata gcg ttt cta ctg atg ggt ctt ggg att ctt gta     1613
   Ser Gly Gly Gln Ile Ala Phe Leu Leu Met Gly Leu Gly Ile Leu Val
           150                 155                 160
   gtg gga gca gga ggg att aga ccg tgc aat cta gct ttc gga gct gat     1661
   Val Gly Ala Gly Gly Ile Arg Pro Cys Asn Leu Ala Phe Gly Ala Asp
       165                 170                 175
   cag ttt aat ccc aag agc ggg tca ggg aaa aga ggg att gat agt ttc     1709
   Gln Phe Asn Pro Lys Ser Gly Ser Gly Lys Arg Gly Ile Asp Ser Phe
   180                 185                 190                 195
   ttc aac tgg tat ttc ttc acc ttc act ttc gcg cag atc ttg tct ctg     1757
   Phe Asn Trp Tyr Phe Phe Thr Phe Thr Phe Ala Gln Ile Leu Ser Leu
                   200                 205                 210
   aca gta gtt gtc tat atc cag tct aat gtt agt tgg acc atc ggt tta     1805
   Thr Val Val Val Tyr Ile Gln Ser Asn Val Ser Trp Thr Ile Gly Leu
               215                 220                 225
   acc atc ccg gtc gtt cta atg ttc ttg gcc tcc gtg att ttc ttt gcg     1853
   Thr Ile Pro Val Val Leu Met Phe Leu Ala Ser Val Ile Phe Phe Ala
           230                 235                 240
   ggt gct aag ttg tat gtt aag atc gaa gcc tca ggt agt cca ttg gct     1901
   Gly Ala Lys Leu Tyr Val Lys Ile Glu Ala Ser Gly Ser Pro Leu Ala
       245                 250                 255
   agt ata gct cat gtt ata aca gct gca atc aag aaa cac ggg tta aag     1949
   Ser Ile Ala His Val Ile Thr Ala Ala Ile Lys Lys His Gly Leu Lys
   260                 265                 270                 275
   tca gtg gaa cag cct tgg ctg aac ctt tac aac tac tac cca gca aaa     1997
   Ser Val Glu Gln Pro Trp Leu Asn Leu Tyr Asn Tyr Tyr Pro Ala Lys
                   280                 285                 290
   tac gca aac gcc aag ctc aaa tac acc gac caa ttc ag  gtaaacacac      2045
   Tyr Ala Asn Ala Lys Leu Lys Tyr Thr Asp Gln Phe Arg
               295                 300
   acactttaaa aacctgtgaa aacaccgttt atactttata catatacttt gacgcagtaa     2105
   aaaaaactta aaacaaagta aaaacccgta aaaacactgt ttataaccac acagtaaaaa     2165
   ctagtctcgt catgtgtttt gatgttttat gtttactgag agaaagcatc atacttggca     2225
   g g ttt ctc gac aag gcg gcg atc ttg gct cct gaa gac aag ttg gag     2272
       Phe Leu Asp Lys Ala Ala Ile Leu Ala Pro Glu Asp Lys Leu Glu
       305                 310                 315
   gct gat ggt aag cct gtg aat cca tgg aag cta tgc aca atg caa caa     2320
   Ala Asp Gly Lys Pro Val Asn Pro Trp Lys Leu Cys Thr Met Gln Gln
   320                 325                 330                 335
   gtt gaa gaa gtg aag tgc atc atg aga gtg ctt cct ata tcg ttt gct     2368
   Val Glu Glu Val Lys Cys Ile Met Arg Val Leu Pro Ile Ser Phe Ala
                   340                 345                 350
   tcg tcg atc tat tac tta acc atc acg caa caa atg act tat ccg gtc     2416
   Ser Ser Ile Tyr Tyr Leu Thr Ile Thr Gln Gln Met Thr Tyr Pro Val
               355                 360                 365
   ttc caa gcc ctc cag agt gat cgt cgc tta ggc tcc gga ggt ttc gtg      2464
   Phe Gln Ala Leu Gln Ser Asp Arg Arg Leu Gly Ser Gly Gly Phe Val
           370                 375                 380
   atc ccg gca gcc acc tat gtt gtc ttc ttg atg act ggc atg aca ctc      2512
   Ile Pro Ala Ala Thr Tyr Val Val Phe Leu Met Thr Gly Met Thr Leu
       385                 390                 395
   ttc atc ata ctc tat gac cgt gtc ttg gtt cca acc tta aaa aga ata      2560
   Phe Ile Ile Leu Tyr Asp Arg Val Leu Val Pro Thr Leu Lys Arg Ile
   400                 405                 410                 415
   acc ggt ata gag acg ggt ata acg ctc ttg caa aga att gga acc ggg      2608
   Thr Gly Ile Glu Thr Gly Ile Thr Leu Leu Gln Arg Ile Gly Thr Gly
                   420                 425                 430
   att ttc ttc gcc ttg tta agc tta ata gtc tct ggt ttc gtg gag gaa      2656
   Ile Phe Phe Ala Leu Leu Ser Leu Ile Val Ser Gly Phe Val Glu Glu
               435                 440                 445
   cat agg aga aat tta gca cta act aaa cca aca ctc ggt tta gcg cca      2704
   His Arg Arg Asn Leu Ala Leu Thr Lys Pro Thr Leu Gly Leu Ala Pro
           450                 455                 460
   cgg aag gga gaa atc tcc tca atg tcg gct atg tgg ctg gtt cca cag      2752
   Arg Lys Gly Glu Ile Ser Ser Met Ser Ala Met Trp Leu Val Pro Gln
       465                 470                 475
   ctt gtg ctt gct ggt ata ggc gac gca ttt gga gct att gga cag atg      2800
   Leu Val Leu Ala Gly Ile Gly Asp Ala Phe Gly Ala Ile Gly Gln Met
   480                 485                 490                 495
   gag ttt tac tac aag cag ttc cct gag aac atg agg agt ttc gct ggt      2848
   Glu Phe Tyr Tyr Lys Gln Phe Pro Glu Asn Met Arg Ser Phe Ala Gly
                   500                 505                 510
   tca tta ttt tac gta gga gca gga gtt tcg agt tac atc agt agc ttc      2896
   Ser Leu Phe Tyr Val Gly Ala Gly Val Ser Ser Tyr Ile Ser Ser Phe
               515                 520                 525
   ttg atc aca acg gtt cac agg acg acg cag aac tcg gcg ggt ggt aac      2944
   Leu Ile Thr Thr Val His Arg Thr Thr Gln Asn Ser Ala Gly Gly Asn
           530                 535                 540
   tgg tta gct gag gat ttg aac aaa gga aga ttg gat tac ttc tat ttc      2992
   Trp Leu Ala Glu Asp Leu Asn Lys Gly Arg Leu Asp Tyr Phe Tyr Phe
       545                 550                 555
   ttg tta gct gga atc tcg gca gtt aat ttt gct tac ttc ttg gtg atg      3040
   Leu Leu Ala Gly Ile Ser Ala Val Asn Phe Ala Tyr Phe Leu Val Met
   560                 565                 570                 575
   gca aga tgg tat agg tac aaa gga agt gat gat gaa gag ata agt tat      3088
   Ala Arg Trp Tyr Arg Tyr Lys Gly Ser Asp Asp Glu Glu Ile Ser Tyr
                   580                 585                 590
   gaa acc aat gga gat atc atc aaa gaa caa gac aag aac aaa gtc          3133
   Glu Thr Asn Gly Asp Ile Ile Lys Glu Gln Asp Lys Asn Lys Val
               595                 600                 605
   tgatgatcat gtgtttctct tcttctggac ttttagtcta cactctttgg attttctttg    3193
   aataagccat ggagaaacga tgtaatgata tacttgctat ggctgctttc tttttttatt    3253
   ttctggttga tatgttagat cacatctctt tctttctatc aacgtggaga aaagagagat    3313
   tataacatca caagtatttg caaaataaaa aaattgtctt gtattttaag gattatgttt    3373
   ctgttactgt ctttcctcac cattcaacag aacaaactca aaaaatgaaa caaacccacc    3433
   caagaatact aatttgatag tttacttgtg cataatttgt atggatgttt caaaacagag    3493
   atatattgag acggttcatt ggttctctct atgcttctag tgtaagattt ta            3545
<210> 130
   <211> 606
   <212> PRT
   <213> Brassica juncea
<400> 130
   Met Glu Arg Lys Pro Leu Glu Val Glu Ser Thr Asp His Gln Lys Leu
   1               5                   10                  15
   Ser Ser Ala Val Tyr Asp Gly Pro Val Glu Glu Asp Val Gln Glu Thr
               20                  25                  30
   Lys Val Val Tyr Arg Gly Trp Lys Val Met Pro Phe Ile Ile Gly Asn
           35                  40                  45
   Glu Thr Phe Glu Lys Leu Gly Ile Ile Gly Thr Leu Ser Asn Leu Leu
       50                  55                  60
   Val Tyr Leu Thr Ala Val Phe Asn Met Lys Ser Ile Thr Ala Ala Thr
   65                  70                  75                  80
   Ile Ile Asn Ala Phe Ala Gly Thr Val Asn Phe Gly Thr Phe Val Ala
                   85                  90                  95
   Ala Phe Leu Cys Asp Thr Tyr Phe Gly Arg Tyr Lys Thr Leu Thr Ala
               100                 105                 110
   Ala Val Ile Ala Cys Phe Leu Gly Ser Leu Ala Ile Leu Leu Thr Ala
           115                 120                 125
   Ala Val Pro Gln Leu His Pro Ala Pro Cys Gly Thr Ala Ser Ser Cys
       130                 135                 140
   Ile Gly Pro Ser Gly Gly Gln Ile Ala Phe Leu Leu Met Gly Leu Gly
   145                 150                 155                 160
   Ile Leu Val Val Gly Ala Gly Gly Ile Arg Pro Cys Asn Leu Ala Phe
                   165                 170                 175
   Gly Ala Asp Gln Phe Asn Pro Lys Ser Gly Ser Gly Lys Arg Gly Ile
               180                 185                 190
   Asp Ser Phe Phe Asn Trp Tyr Phe Phe Thr Phe Thr Phe Ala Gln Ile
           195                 200                 205
   Leu Ser Leu Thr Val Val Val Tyr Ile Gln Ser Asn Val Ser Trp Thr
       210                 215                 220
   Ile Gly Leu Thr Ile Pro Val Val Leu Met Phe Leu Ala Ser Val Ile
   225                 230                 235                 240
   Phe Phe Ala Gly Ala Lys Leu Tyr Val Lys Ile Glu Ala Ser Gly Ser
                   245                 250                 255
   Pro Leu Ala Ser Ile Ala His Val Ile Thr Ala Ala Ile Lys Lys His
               260                 265                 270
   Gly Leu Lys Ser Val Glu Gln Pro Trp Leu Asn Leu Tyr Asn Tyr Tyr
           275                 280                 285
   Pro Ala Lys Tyr Ala Asn Ala Lys Leu Lys Tyr Thr Asp Gln Phe Arg
       290                 295                 300
   Phe Leu Asp Lys Ala Ala Ile Leu Ala Pro Glu Asp Lys Leu Glu Ala
   305                 310                 315                 320
   Asp Gly Lys Pro Val Asn Pro Trp Lys Leu Cys Thr Met Gln Gln Val
                   325                 330                 335
   Glu Glu Val Lys Cys Ile Met Arg Val Leu Pro Ile Ser Phe Ala Ser
               340                 345                 350
   Ser Ile Tyr Tyr Leu Thr Ile Thr Gln Gln Met Thr Tyr Pro Val Phe
           355                 360                 365
   Gln Ala Leu Gln Ser Asp Arg Arg Leu Gly Ser Gly Gly Phe Val Ile
       370                 375                 380
   Pro Ala Ala Thr Tyr Val Val Phe Leu Met Thr Gly Met Thr Leu Phe
   385                 390                 395                 400
   Ile Ile Leu Tyr Asp Arg Val Leu Val Pro Thr Leu Lys Arg Ile Thr
                   405                 410                 415
   Gly Ile Glu Thr Gly Ile Thr Leu Leu Gln Arg Ile Gly Thr Gly Ile
               420                 425                 430
   Phe Phe Ala Leu Leu Ser Leu Ile Val Ser Gly Phe Val Glu Glu His
           435                 440                 445
   Arg Arg Asn Leu Ala Leu Thr Lys Pro Thr Leu Gly Leu Ala Pro Arg
       450                 455                 460
   Lys Gly Glu Ile Ser Ser Met Ser Ala Met Trp Leu Val Pro Gln Leu
   465                 470                 475                 480
   Val Leu Ala Gly Ile Gly Asp Ala Phe Gly Ala Ile Gly Gln Met Glu
                   485                 490                 495
   Phe Tyr Tyr Lys Gln Phe Pro Glu Asn Met Arg Ser Phe Ala Gly Ser
               500                 505                 510
   Leu Phe Tyr Val Gly Ala Gly Val Ser Ser Tyr Ile Ser Ser Phe Leu
           515                 520                 525
   Ile Thr Thr Val His Arg Thr Thr Gln Asn Ser Ala Gly Gly Asn Trp
       530                 535                 540
   Leu Ala Glu Asp Leu Asn Lys Gly Arg Leu Asp Tyr Phe Tyr Phe Leu
   545                 550                 555                 560
   Leu Ala Gly Ile Ser Ala Val Asn Phe Ala Tyr Phe Leu Val Met Ala
                   565                 570                 575
   Arg Trp Tyr Arg Tyr Lys Gly Ser Asp Asp Glu Glu Ile Ser Tyr Glu
               580                 585                 590
   Thr Asn Gly Asp Ile Ile Lys Glu Gln Asp Lys Asn Lys Val
   595 600 605
<210> 131
   <211> 1839
   <212> DNA
   <213> Brassica juncea
<400> 131
   atggagagaa agcctctaga agtcgagtca acggatcacc aaaagcctcc ctccgccgtg 60
   tacgatggct ctgttatggc ggttgattct gttgaggaag aagttgagga gaccaaagtc 120
   gtttacagag gctggaaagt catgcccttt atcattggaa atgagacgtt cgagaagctt 180
   gggatcattg gaacactatc aaaccttctg gtttatttaa ctgctgtctt caacatgaag 240
   agtatcacag ctgcaacaat catcaatgca ttcagtggca caatcaactt cggaaccttc 300
   gttgctgctt tcctctgcga cacttacttc ggtcgataca agactctaac tgtcgctgtc 360
   atcgcctgtt ttcttggatc acttgtgata ctattgacag ctgcagtgcc acaattacat 420
   ccagctccat gcggaacagc tctctcatgt atcgggccaa gtggtggcca gatagcgttt 480
   ctactgctgg gtctcgggtt tcttgtagtt ggagcaggcg ggattagacc atgtaatcta 540
   gctttcgggg ctgatcagtt taaccccaag agcgagtcag gaaaacgagg catcgatagt 600
   ttcttcaatt ggtacttctt caccttcacg ttcgcgcaga tcctgtcgct gacactaatc 660
   gtctatatcc agtccaatgt cagctggaca atcggtttaa ccatcccggt cgttctcatg 720
   ttcttggcct gcgtgatttt ctttgctgga gataagttgt atgtgaagat caaagcctca 780
   ggtagtccat tggctggtat agctcaagtt atagcggttg caatcaagaa acgtgggtta 840
   aagccaacga aagagccttg gcttaacctt tacaactact acccaccaaa atacgcaaac 900
   tccaaactca aatacaccga ccagtttagg ttccttgata aggcggcgat tttggctccc 960
   gaagacaagt tggaggctga tggtaagcct gcggatccct ggaagctttg tacaatgcaa 1020
   caagttgaag aagtgaagtg cattgtgaga gtgcttccta tatggtttgc tgcatcgatc 1080
   tattacttga ccatcacgca acaaatgact taccccgtct tccaagccct ccagagcgac 1140
   cgtcgcttag gatccagagg atttgtgatc ccggcagcca cctacgttgt ctttttgatg 1200
   acaggaatga cggttttcat catattctac gaccgtgtcc tcgtgcctac cttaagaaga 1260
   ataaccggta tagacacggg gataacgcta ctgcagagga ttggaaccgg aattttcttc 1320
   gcctttgtta gcttgatagt ctccggtttc gtcgaggaac ggaggagaac gttcgcgctg 1380
   actaaaccaa cacttggtat ggctccaagg aagggagaaa tctcctcaat gtcagctatg 1440
   tggctgatcc cacaactcgc ccttgcgggt atagctgagg cgtttggagc tataggacag 1500
   atggagtttt actacaagca gttccctgag aacatgagga gttttgctgg ttcgatcttc 1560
   tatgtaggag caggagtttc gagttacctc ggtagcttct tgattgcaat ggttcacagg 1620
   acgacgcaga actcagctgg tggtaattgg ttggctgagg atttgaacaa aggaagattg 1680
   gattacttct atttcatgat cgctggaatc ttggcagtta attttgctta cttcttggtc 1740
   atgtcaagat ggtatagata caaaggaagt gatggtgaag tgacaactta tgaaaccaat 1800
   ggagatgcca tcaaacaaca agacaagaat actgtctga 1839
<210> 132
   <211> 1839
   <212> DNA
   <213> Brassica juncea
<400> 132
   atggagagaa agcctctgga agttgagtcc accgatcatc agaaaccttc ctccgccgtc      60
   tacgacggct ctgctacggc ggttgattct gttgaagaag aagttcagga aaccaaactc     120
   gtttatagag gctggaaagt catgcctttc atcataggaa acgagacatt tgagaagctt     180
   gggatcattg gaacactatc aaaccttctg gtttacttaa ctgctgtctt caacatgaag     240
   agtatcacag ccgcaacaat catcaacgcc tttagtggca caatcaactt cggaaccttc     300
   gttgctgcct tcctttgcga cacttacttc ggtcgttaca agacactaac cgtcgcggtc     360
   atcgcatgtt ttcttggatc acttgtgata ctattaactg ctgcagtacc acaactgcat     420
   ccagctccat gcggaatagc cctcacgtgt acaggcccaa gtggcggcca gatagccttt     480
   ctactaatgg gtcttgcctt tcttgtggtg ggagcaggtg ggattagacc gtgtaatctt     540
   gcttttggag ctgatcagtt taaccctaaa agcgagtcag ggaagagagg gattgatagt     600
   ttctttaact ggtacttctt caccttcacg ttcgcgcaga tcttgtcgct gacactagtt     660
   gtctacatcc agtctaacgt cagttggacc atcggtttaa ccatacctgt cgtgttaatg     720
   ttcttggcct gcgtgatctt ctttgcgggt gataagttgt atgtcaagat caaagcctca     780
   ggtagtccat tggctagtat agctcaagtt ataacggttg ctatcaagaa acgtgggtta     840
   aaggctgtga agcagccttg ggttaacctt tacaattact acccactaaa ctacgcaaac     900
   tctaagctca agtacaccga ccagttcagg tttcttgata aggcggcgat cttggctcct     960
   gaagacaagt tggaggctga tggtaagcct acggatccat ggaagctatg tacaatgcaa    1020
   caagttgaag aagtgaagtg cattgtgaga gtgcttccta tatggttcgc ttcatcaatc    1080
   tattacttga ccatcacaca acaaatgacc tacccagtct tccaagccct ccagagcgat    1140
   cgtcgcttag gctctggagg gtttgtgatt cctgcagcca cctatgtggt cttcttgatg    1200
   acgggaatga ctgttttcat cataatctac gaccgcgtac tcgtgcctac cttgagaaga    1260
   atcaccggta tagacaccgg gatcacgctc ttgcaaagga taggaaccgg gattttcttc    1320
   gcctttttga gcttggtagt gtctggtttc gtcgaggaac ggaggaggac tttcgcattg    1380
   actaaaccaa cactcggtat ggcgccacgg aagggagaaa tctcctcaat gtcagctatg    1440
   tggctgatac cgcagctcac actcgcgggt gtagccgagg catttggagc tattggacag    1500
   atggagtttt actacaagca gttccccgag aacatgagga gtttcgccgg ctccatcttc    1560
   tatgtgggag gaggagtttc tagttacctc ggcagcttct tgattgcaac ggtacacagg    1620
   acgacgcaga actcggctgg cggtaactgg ttggctgaag atttgaacaa aggaagattg    1680
   gatttgtttt acttcatgat cgctggaatc ttggcggtta attttgctta cttcttggtg    1740
   atgtcaagat ggtataggta caaaggaagt gatgatgaag tgacagctta tgaaacaaat    1800
   ggagatgtga tcaaacaaca agacaagaac aaagtctaa                           1839
<210> 133
   <211> 1839
   <212> DNA
   <213> Brassica juncea
<400> 133
   atggagagaa agtctctaga agtcgagtct atggatcacc aaaaactttc ctccgccgtg 60
   tacgatggct ctgttacggc cgttgattct gttaaagaag atgttcagga gaccaaagtc 120
   gtttatagag gctggaaagt catgccttac atcattggaa atgagacatt tgagaagctt 180
   gggatcattg gaacactatc aaaccttctg gtttatttaa cttctgtctt caacatgaag 240
   agtgtcacag ctgcaacaat catcactgcc tttgctggca cagtcaactt cggaactttc 300
   gtcgctgctt tcctttgcga cacttacttc ggtcgataca agactcttac cgttgcggtc 360
   ctcgcctgtt ttcttggatc acttgcgata ctattgactg ctgcagtccc acaactacat 420
   ccagtcccat gcggcacagc gagctcgtgt atcgggccaa gtggtggcca gatagcgttt 480
   ctactgatgg gtcttggaat tcttgtcgtg ggagcaggag ggattagacc gtgcaatcta 540
   gctttcggag ctgatcagtt taacccgaag agcgggtcag ggaagagagg gattgatagt 600
   ttcttcaact ggtacttctt caccttcact ttcgcccaga tcttgtctct gacagtagtt 660
   gtctatgtcc agtctaatgt cagttggacc atcggtttaa ccatcccggt tgttctaatg 720
   ttcttggcct ccgtgatttt ctttgcgggt gctaagttgt atgttaagat cgaagcctca 780
   ggtagtccat tggctagtgc agctcatgtt ataacggctg ctatcaagaa acacaggtta 840
   aagtcagtga aacagccttg gctgaacctt tacaactact acccatcaaa atacgcaaac 900
   gccaagctca aatacaccga acaattcagg tttctcgaca aggcggcgat cttgactcct 960
   gaagacaagt tggaggctga tggtaagcct gtgaatccgt ggaatctatg tacaatgcaa 1020
   caagttgaag aagtgaagtg catcatgaga gtgcttccta tatggtttgc ttcatcgatc 1080
   tactacttaa ccatggcgca acaaatgact tatcctgtct tccaagctct ccagagtgat 1140
   cgtcgcctag gctccggagg tttcatgatc ccggcagcca cctatgttgt cttcttgatg 1200
   actggcatga ccctcttcat catattttat gaccgtgtct tggttcctac attaaaaagt 1260
   ataaccggta tagagacggg tataacgctc ttgcaaagaa ttggagccgg gattttgttc 1320
   gccttgttaa gcttaattgt ctctggtttc gtcgaggaac gtaggagaac tttagcacta 1380
   actcaaccga cactcggttt agcgccacgg aagggagaaa tctcctcaat gtcggctatg 1440
   tggctgattc cgcagcttgt acttgctggt atagccgacg cgtttggagc tattggacag 1500
   atggagtttt actacaagca gttccctgag aacatgagga gttttgctgg ttctatcttt 1560
   tacgtaggag caggagtttc gagttacatc agtagcttct tgatcacaac ggttcacagg 1620
   atgacgcaga actcggcggg tggtaattgg ttagctgagg atttgaacaa agggagattg 1680
   gattacttct atttcttgtt agctggaatc ttggcagtta attttgctta cttcttggtg 1740
   atggcaagat ggtataggta caaaggaagt gatgatgaag agacaagtta tgaaaccagt 1800
   ggagatatca tcaaagaaca agacaagaac aaagtctga 1839
<210> 134
   <211> 1682
   <212> DNA
   <213> Brassica juncea
<400> 134
   gaaacgagac gtttgagaag cttgggatca ttggaacact atcaaacctt ctggtttatc      60
   taactgctgt cttcaacatg aagagtatca cagctgcaac aatcatcaat gcattcagcg     120
   gcacaatcaa cttcggaacc ttccttgctg ctttcctctg cgacacttac ttcggtcgat     180
   acaagactct aaccgtcgct gtcatcgcct gttttcttgg atcactcgtg atactattaa     240
   cagctgcagt gccacagcta catccaactc cgtgcggaac agctatctca tgtatcgggc     300
   caagcggtgg ccagatagcg tttctgctgc tgggtctcgg gttccttgta gtgggagcag     360
   gcgggattag accgtgtaat ctagctttcg gagctgatca gtttaatccc aagagcgagt     420
   cagggaaaag agggatagat agtttcttca attggtactt cttcaccttc actttcgcgc     480
   agatcttgtc gctgacactg atcgtctatg tccagtctaa cgtcagctgg accatcggtt     540
   taaccatccc ggtcgttctc atgttcttgg cctgtgtgat ttctttgcgg ggtgataagt     600
   tgtatgtaaa gatcaaagcc tcaggtagtc ctttagccag tatagctcaa gttatagcgg     660
   ttgcaatcaa gaaacgtggg ttaaagccaa cgaaacagcc ttggcttaac ctctacaatt     720
   attacccacc aaagtacgca aactccaagc tcaaatacac cgaccagttt aggtttcttg     780
   ataaggcggc gatcttggct cccgaagaca agttggaggc tgatggtaag cctgcggatc     840
   catggaagct ttgtacaatg caacaagttg aagaagtgaa gtgcattgtg agagtgcttc     900
   ctatatggtt tgctgcatcc atctattact taaccatcac gcaacagatg acctatcccg     960
   tctttcaagc cctccagagc gaccgtcgct taggctccag aggatttgtg atccccgcag    1020
   ccacctacgt tgtcttcttg atgacaggaa tgacggtttt catcatattc tacgaccgtg    1080
   tcctcgtgcc taccttaaga agaataacag gtatagacac ggggataacg ctcttgcaga    1140
   ggattggaac cggtattttc ttcgcctttc ttagcttgat agtctccggt ttcgtcgagg    1200
   aacgtaggag aacgttcgcc ctgactaaac caacactcgg tatggctcca aggaagggag    1260
   aaatctcttc aatgtcagct atgtggctga tcccacaact cgcccttgcg ggtatagccg    1320
   aggcgtttgg agctattgga cagatggagt tttactacaa gcaattccct gagaacatga    1380
   ggagttttgc tggttcgatc ttttatgtag gagcaggagt ttcgagttac ctcggtagct    1440
   tcttgattgc aatggttcac aggacgacgc agaactcggc tggtggtaac tggttagctg    1500
   aggatttgaa caaaggaaga ttggatttgt tttacttcat gatcgctgga atcttggcgg    1560
   ttaattttgc ttatttcttg gtcatgtcaa gatggtatag atacaaagga agtgatgatg    1620
   aagtgaaaac ttatgaaacc aatggagatg ccatcaaaca acaagacaag aacaatgtct    1680
   ga                                                                   1682
<210> 135
   <211> 1839
   <212> DNA
   <213> Brassica juncea
<400> 135
   atggagagaa agcctctaga agttgagtcc acggaccacc agaaaccttc ctccgccgtg 60
   tacgatggct ctgctacggc ggttgattct gttcaagaag aagttcagga gacgaaagtc 120
   gtttacagag gctggaaagt catgcctttc atcattggaa acgagacatt tgagaagctt 180
   gggatcattg gaacactatc aaacctcctg gtttacttaa ccgctgtctt caacatgaag 240
   agtatcacag ctgcaacaat catcaacgcc tttagtggca caatcaactt cggaaccttc 300
   gttgctgctt tcctctgcga cacttacttc ggtcgttaca agactctaac cgtcgcggtc 360
   atcgcatgtt ttcttggatc acttgtgata ctactaactg ctgcagtacc acaactacat 420
   ccagctccat gcggaacagc ccttacgtgt acaggaccaa gtggtggcca gatagcgttc 480
   ctactgatgg gtcttggctt tcttgtggtg ggagcaggtg ggataagacc gtgtaatctt 540
   gccttcggtg cggatcagtt taaccccaag agcgagtcag ggaagagagg gattgatagt 600
   ttcttcaact ggtacttctt caccttcacg ttcgcgcaga tcttgtcgct gacactagtt 660
   gtctacatcc agtctaacgt cagttggacc atcggtttaa ccatccctgt cgtgttaatg 720
   ttcttggcct gcgtgatctt ctttgcgggt gataagctgt atgtaaagat caaagcctca 780
   ggtagtccat tggctagtat aggtcaagtt ataacggttg ctatcaagaa acgtgggtta 840
   aagcctgtga agcagccttg gcttaacctt tacaattact acccactaaa ctacgcaaac 900
   tccaagctca agtacactga ccagttcagg tttcttgata aggcagcgat ctttgctcct 960
   gaagacaagc tggaggctga tggaaagcca gtggatccat ggaagctttg tacaatgcaa 1020
   caagttgaag aagtgaagtg cattgtgaga gtgcttccta tatggtttgc ttcctcgatc 1080
   tactacttga ccatcacgca acagatgacc tatcctgtct tccaagccct ccagagcgat 1140
   cgtcgcttag gctctggagg atttgtgatt cctgcagcca cctatgttgt cttcttgatg 1200
   acaggcatga ctgttttcat cattatctac gaccgtgtcc tcgtgcctac cttgagaaga 1260
   atcaccggtc tagacaccgg gatcacgctc ttgcagagga taggaactgg gattttcttt 1320
   gccttcttga gcttggtagt ctccggtttc gtcgaggaac ggaggagaac tttcgccttg 1380
   acaaaaccaa cgctcggtat ggcgccacgg aagggagaaa tctcctcaat gtcagcaatg 1440
   tggctgatac cgcagctcac actcgcgggt gtagccgagg cgtttggagc tattggacag 1500
   atggagtttt actacaagca gttccctgag aacatgagga gtttcgctgg ttccatcttt 1560
   tacgtaggag gaggagtttc gagttacctc ggtagcttct tgattgcaac ggttcacagg 1620
   acgacgcaga actcggctgg tggtaactgg ttagctgaag atttgaacaa aggaagattg 1680
   gatttgtttt acttcatgat cgctgggatc ttggccgtta attttgctta tttcctggtg 1740
   atgtcaagat ggtataggta caaaggaagt gatgatgatg tgacaacttg tgaaaccaat 1800
   ggtgatgtga tcaaacaaca agacaagaac aaagtctaa 1839
<210> 136
   <211> 1821
   <212> DNA
   <213> Brassica juncea
<400> 136
   atggagagaa agcctctcga agtcgagtcg acagatcacc aaaaactttc ctccgccgtg 60
   tacgatggcc ctgttgaaga agatgtccag gagaccaaag tcgtttatag aggctggaaa 120
   gtcatgcctt tcatcattgg aaatgagaca tttgagaagc ttgggatcat tggaacacta 180
   tcaaaccttc tggtttattt aactgctgtc ttcaacatga agagtatcac agctgcaaca 240
   atcatcaatg cctttgctgg cacagtcaac tttggaactt tcgttgctgc tttcctctgt 300
   gacacttact tcggtcgata caagactctt accgccgcgg tcatcgcctg ttttcttgga 360
   tcacttgcga tactattgac tgctgcagta ccacaactcc atccagctcc atgcggaaca 420
   gcgagctcgt gtatcgggcc aagtggtggc cagatagcgt ttctactgat gggtcttggg 480
   attcttgtag tgggagcagg agggattaga ccgtgcaatc tagctttcgg agctgatcag 540
   tttaatccca agagcgggtc agggaaaaga gggattgata gtttcttcaa ctggtatttc 600
   ttcaccttca ctttcgcgca gatcttgtct ctgacagtag ttgtctatat ccagtctaat 660
   gttagttgga ccatcggttt aaccatcccg gtcgttctaa tgttcttggc ctccgtgatt 720
   ttctttgcgg gtgctaagtt gtatgttaag atcgaagcct caggtagtcc attggctagt 780
   atagctcatg ttataacagc tgcaatcaag aaacacgggt taaagtcagt ggaacagcct 840
   tggctgaacc tttacaacta ctacccagca aaatacgcaa acgccaagct caaatacacc 900
   gaccaattca ggtttctcga caaggcggcg atcttggctc ctgaagacaa gttggaggct 960
   gatggtaagc ctgtgaatcc atggaagcta tgcacaatgc aacaagttga agaagtgaag 1020
   tgcatcatga gagtgcttcc tatatcgttt gcttcgtcga tctattactt aaccatcacg 1080
   caacaaatga cttatccggt cttccaagcc ctccagagtg atcgtcgctt aggctccgga 1140
   ggtttcgtga tcccggcagc cacctatgtt gtcttcttga tgactggcat gacactcttc 1200
   atcatactct atgaccgtgt cttggttcca accttaaaaa gaataaccgg tatagagacg 1260
   ggtataacgc tcttgcaaag aattggaacc gggattttct tcgccttgtt aagcttaata 1320
   gtctctggtt tcgtggagga acataggaga aatttagcac taactaaacc aacactcggt 1380
   ttagcgccac ggaagggaga aatctcctca atgtcggcta tgtggctggt tccacagctt 1440
   gtgcttgctg gtataggcga cgcatttgga gctattggac agatggagtt ttactacaag 1500
   cagttccctg agaacatgag gagtttcgct ggttcattat tttacgtagg agcaggagtt 1560
   tcgagttaca tcagtagctt cttgatcaca acggttcaca ggacgacgca gaactcggcg 1620
   ggtggtaact ggttagctga ggatttgaac aaaggaagat tggattactt ctatttcttg 1680
   ttagctggaa tctcggcagt taattttgct tacttcttgg tgatggcaag atggtatagg 1740
   tacaaaggaa gtgatgatga agagataagt tatgaaacca atggagatat catcaaagaa 1800
   caagacaaga acaaagtctg a 1821
<210> 137
   <211> 2710
   <212> DNA
   <213> Brassica napus
<220>
   <221> exon
   <222> (1)..(151)
   <223> exon 1
<220>
   <221> Intron
   <222> (152)..(619)
   <223> intron 1
<220>
   <221> exon
   <222> (620)..(837)
   <223> exon 2
<220>
   <221> Intron
   <222> (838)..(1163)
   <223> intron 2
<220>
   <221> exon
   <222> (1164)..(1717)
   <223> exon 3
<220>
   <221> Intron
   <222> (1718)..(1797)
   <223> intron 3
<220>
   <221> exon
   <222> (1798)..(2710)
   <223> exon 4
<400> 137
   atg gag aga aag ccc ttt gat gtt gag aca act gag gat cac aaa gcc          48
   Met Glu Arg Lys Pro Phe Asp Val Glu Thr Thr Glu Asp His Lys Ala
   1               5                   10                  15
   tac tcc tcc gtc gat ggc ggt ggt tct gat tcg gtc gat tca ttt gaa          96
   Tyr Ser Ser Val Asp Gly Gly Gly Ser Asp Ser Val Asp Ser Phe Glu
               20                  25                  30
   gac gag cag aaa aag ctc gtt tat aga ggc tgg aaa gtc atg ccc ttt         144
   Asp Glu Gln Lys Lys Leu Val Tyr Arg Gly Trp Lys Val Met Pro Phe
           35                  40                  45
   atc att g gtaacgcttt cttcttctct tcagacaact tttttttttt agtttattct     201
   Ile Ile
       50
   gttttgtttt gttgtgtccc ttttgtcgga ttatagaacg ttttgcccgt gatgttttta       261
   ttttaatttc gtttcttttg tcttcttagt cttgactgtt gaaaatcttg atttggagtt       321
   cttgtcttga cgtttcgaga tgaatgaaac acagatcaaa caaatcaacc aaaccagttg       381
   agtttcctag ataaatctac catggaaagt gagagacttt gaatcatgca agttttcagt       441
   ttcagatata agattttagt tagtttctgg gagttataac aaagaatgaa taataaagtg       501
   tccagtccaa cactgaaagt ccaatctttg atttcaacat tgtgaattaa atgtaaaaag       561
   gttgttactt tatggtttga ctaattattt atgttggaaa aaatatattg tgtcacag         619
   gt  aat gag aca ttt gag aag ata ggg atc att ggg aca cta tca aac         666
   Gly Asn Glu Thr Phe Glu Lys Ile Gly Ile Ile Gly Thr Leu Ser Asn
                   55                  60                  65
   ctt ctg gtg tac cta act tca gta ttc aac ctt aag agt gtt aca gct         714
   Leu Leu Val Tyr Leu Thr Ser Val Phe Asn Leu Lys Ser Val Thr Ala
               70                  75                  80
   gca acc atc atc aac gcc ttc agt ggc act atc aat ttc ggc act ttc         762
   Ala Thr Ile Ile Asn Ala Phe Ser Gly Thr Ile Asn Phe Gly Thr Phe
           85                  90                  95
   ctc gct gct ttc ctc tgc gac act tac ttt ggt cgc tac aag act ctc         810
   Leu Ala Ala Phe Leu Cys Asp Thr Tyr Phe Gly Arg Tyr Lys Thr Leu
       100                 105                 110
   tct gtt gct gtc atc gcc tgt ttt ctg gtactctaat caaatgacca               857
   Ser Val Ala Val Ile Ala Cys Phe Leu
   115                 120
   tcttgtttta ttttggccaa gattttgtct ttttttctta ctaaaccata ctccctccgt        917
   ttcattttaa gtgtcttttt agagattttt ttcgttacaa aataagtgtc gttttcgatt       977
   ttcaatgcaa aatttattga ttttatgcaa aatttatttt tctattagtt gaaatatggt      1037
   taggtgtata ggtaatagtg tttttttata cgaaatgtac aaaattaagt gtttccttaa       1097
   tccatgtgcc gaaacctaaa acgacactta taatgaaaca gagggagtat ttcatatttg      1157
   ttgtag gga tcg ctt gtg ata cta ctg acg gct gca gtt cca gga ttg         1205
          Gly Ser Leu Val Ile Leu Leu Thr Ala Ala Val Pro Gly Leu
              125                 130                 135
   cac cct att cct tgt gga aca caa agt tct tgc caa ggc cca agc gag        1253
   His Pro Ile Pro Cys Gly Thr Gln Ser Ser Cys Gln Gly Pro Ser Glu
           140                 145                 150
   ggc cag att gcg ttt ctg ctg atg ggt tta gcg ctt ctt gtg gtg ggt        1301
   Gly Gln Ile Ala Phe Leu Leu Met Gly Leu Ala Leu Leu Val Val Gly
       155                 160                 165
   gct ggt gga atc agg ccg tgt aac ttg gcc ttt ggt gct gac cag ttc        1349
   Ala Gly Gly Ile Arg Pro Cys Asn Leu Ala Phe Gly Ala Asp Gln Phe
   170                 175                 180                 185
   aac ccc aag tca gaa tct gga aag aaa gga atc aat agt ttc ttc aac      1397
   Asn Pro Lys Ser Glu Ser Gly Lys Lys Gly Ile Asn Ser Phe Phe Asn
                   190                 195                 200
   tgg tac ttc ttc act ttc acg ttc gcg cag atc atc tcc ctc aca cta      1445
   Trp Tyr Phe Phe Thr Phe Thr Phe Ala Gln Ile Ile Ser Leu Thr Leu
               205                 210                 215
   gtt gtg tat atc cag tca aat gtg agc tgg acc atc ggt ttg agt atc      1493
   Val Val Tyr Ile Gln Ser Asn Val Ser Trp Thr Ile Gly Leu Ser Ile
           220                 225                 230
   cct gtg ggt ctt atg ttc ttg gcc tgc gtc att ttc ttt gct ggt cac      1541
   Pro Val Gly Leu Met Phe Leu Ala Cys Val Ile Phe Phe Ala Gly His
       235                 240                 245
   aaa ctg tat gtg aaa gtg aaa gcc tct ggt agt cca ttg gct ggt ata      1589
   Lys Leu Tyr Val Lys Val Lys Ala Ser Gly Ser Pro Leu Ala Gly Ile
   250                 255                 260                 265
   ggt cat gtt ata gcc gca gct atc aag aaa cgt ggc ttg aag cca gtt      1637
   Gly His Val Ile Ala Ala Ala Ile Lys Lys Arg Gly Leu Lys Pro Val
                   270                 275                 280
   aag cag cct tgg atc gat ctt tac aac cac att cct cct aac tat gca      1685
   Lys Gln Pro Trp Ile Asp Leu Tyr Asn His Ile Pro Pro Asn Tyr Ala
               285                 290                 295
   aac tct act ctt aaa tac acc gac cag ctt ag  gtaaagtcgg aatagttctg    1737
   Asn Ser Thr Leu Lys Tyr Thr Asp Gln Leu Arg
           300                 305
   tctctgtctt tctttgttca tttgctttat gctttgtctg aacaaatgtt gtattgccag    1797
   g ttt cta gac aaa gca gcg att atg acc cct gag gac aag ttg aac tcg    1846
     Phe Leu Asp Lys Ala Ala Ile Met Thr Pro Glu Asp Lys Leu Asn Ser
         310                 315                 320
   gat gga acg gct tcg gat cca tgg aaa cta tgt acg atg cag aaa gtg      1894
   Asp Gly Thr Ala Ser Asp Pro Trp Lys Leu Cys Thr Met Gln Lys Val
   325                 330                 335                 340
   gaa gaa gtg aaa tgc att tta aga gtg att cca atc tgg ttc gca tgc      1942
   Glu Glu Val Lys Cys Ile Leu Arg Val Ile Pro Ile Trp Phe Ala Cys
                   345                 350                 355
   gcc att tac tac ctt gca ata agt atg caa atg act tat cca gtc ttc      1990
   Ala Ile Tyr Tyr Leu Ala Ile Ser Met Gln Met Thr Tyr Pro Val Phe
               360                 365                 370
   caa gct ctc cag agc gac agg cta tta ggt tca ggt ggc ttc aag att      2038
   Gln Ala Leu Gln Ser Asp Arg Leu Leu Gly Ser Gly Gly Phe Lys Ile
           375                 380                 385
   cca gcc gcc acc tat gtc gtg ttc ttg atg tct ggt atg acg gtt ttc      2086
   Pro Ala Ala Thr Tyr Val Val Phe Leu Met Ser Gly Met Thr Val Phe
       390                 395                 400
   atc ata atc tac gac cgt gtc ctt gtc ccc tcg ctc aaa aga gtg acc      2134
   Ile Ile Ile Tyr Asp Arg Val Leu Val Pro Ser Leu Lys Arg Val Thr
   405                 410                 415                 420
   ggt cta gac acg ggg ata aca ctc tta caa aga ata gga gct ggc att      2182
   Gly Leu Asp Thr Gly Ile Thr Leu Leu Gln Arg Ile Gly Ala Gly Ile
                   425                 430                 435
   ttc ttt gcc gtg ttg agt tta ttg gtc tcc ggg ttt ata gaa gaa cgt      2230
   Phe Phe Ala Val Leu Ser Leu Leu Val Ser Gly Phe Ile Glu Glu Arg
               440                 445                 450
   aga aga acc ttt gcg ctg aca cga cct acg ctc ggg tcg gag cct cga      2278
   Arg Arg Thr Phe Ala Leu Thr Arg Pro Thr Leu Gly Ser Glu Pro Arg
           455                 460                 465
   aca gga gag atc tcc tca atg tca gcg atg tgg ctg att ccg cag ctg      2326
   Thr Gly Glu Ile Ser Ser Met Ser Ala Met Trp Leu Ile Pro Gln Leu
       470                 475                 480
   gtg ctt gca ggg gta gca gaa gct ttt gta gcc att ggg caa atg gag      2374
   Val Leu Ala Gly Val Ala Glu Ala Phe Val Ala Ile Gly Gln Met Glu
   485                 490                 495                 500
   ttt tac tac aag cag ttt cct gaa aac atg aag agc ttt gcg ggt tct      2422
   Phe Tyr Tyr Lys Gln Phe Pro Glu Asn Met Lys Ser Phe Ala Gly Ser
                   505                 510                 515
   atc ttc tat gtc ggt gga gga gtt tct agc tac ctc gct agc ttc ttg      2470
   Ile Phe Tyr Val Gly Gly Gly Val Ser Ser Tyr Leu Ala Ser Phe Leu
               520                 525                 530
   atc tcg act gtt cat aga aca act gag cat tca cct acg ggg aat tgg      2518
   Ile Ser Thr Val His Arg Thr Thr Glu His Ser Pro Thr Gly Asn Trp
           535                 540                 545
   tta gct gag gat ctg aac aaa gcg aag ctg gac tac ttc tat ttc atg      2566
   Leu Ala Glu Asp Leu Asn Lys Ala Lys Leu Asp Tyr Phe Tyr Phe Met
       550                 555                 560
   ctc aca gga ctc atg ttt gtc aac ttt ctt tac ttc ttg tta atg gct      2614
   Leu Thr Gly Leu Met Phe Val Asn Phe Leu Tyr Phe Leu Leu Met Ala
   565                 570                 575                 580
   aga tgg tat aga tac aaa ggc act cat gat gaa gtc agt tat gtg gtt      2662
   Arg Trp Tyr Arg Tyr Lys Gly Thr His Asp Glu Val Ser Tyr Val Val
                   585                 590                 595
   gac act gtt gac gaa gag acg aag caa caa gac aag aac tct gtc tga      2710
   Asp Thr Val Asp Glu Glu Thr Lys Gln Gln Asp Lys Asn Ser Val
               600                 605                 610
<210> 138
   <211> 1238
   <212> DNA
   <213> Artificial Sequence
<220>
   <223> dsRNA construct for downregulation of GTR1 expression in Brassica napus
<220>
   <221> misc_feature
   <222> (1)..(215)
   <223> Fragment of BnGTR1_A2 (bp 384 to 598 from start codon in sense orientation
<220>
   <221> misc_feature
   <222> (248)..(991)
   <223> intron 2 from the Pdk gene of Flaveria
<220>
   <221> misc_feature
   <222> (1024)..(1238)
   <223> Fragment of BnGTR1_A2 (bp 384 to 598 from start codon in antisense orientation)
<400> 138
   gcttgcgata ctactcacgg cagcagttcc gggactgcac ccgactcctt gtggaacaaa 60
   gagttcatgc gaagggccag gtgcggggca gattttgttt ctgatgttgg gtttagcgct 120
   tcttgtggtt ggtgctggtg gtatcaggcc gtgtaacttg gcgtttggtg ccgatcagtt 180
   caaccccaag tccgaatcag gaaagaaagg aatcaggtac cccagcttgg taaggaaata 240
   attattttct tttttccttt tagtataaaa tagttaagtg atgttaatta gtatgattat 300
   aataatatag ttgttataat tgtgaaaaaa taatttataa atatattgtt tacataaaca 360
   acatagtaat gtaaaaaaat atgacaagtg atgtgtaaga cgaagaagat aaaagttgag 420
   agtaagtata ttatttttaa tgaatttgat cgaacatgta agatgatata ctagcattaa 480
   tatttgtttt aatcataata gtaattctag ctggtttgat gaattaaata tcaatgataa 540
   aatactatag taaaaataag aataaataaa ttaaaataat atttttttat gattaatagt 600
   ttattatata attaaatatc tataccatta ctaaatattt tagtttaaaa gttaataaat 660
   attttgttag aaattccaat ctgcttgtaa tttatcaata aacaaaatat taaataacaa 720
   gctaaagtaa caaataatat caaactaata gaaacagtaa tctaatgtaa caaaacataa 780
   tctaatgcta atataacaaa gcgcaagatc tatcaatttt atatagtatt atttttcaat 840
   caacattctt attaatttct aaataatact tgtagtttta ttaacttcta aatggattga 900
   ctattaatta aatgaattag tcgaacatga ataaacaagg taacatgata gatcatgtca 960
   ttgtgttatc attgatctta catttggatt gattacagtt gggaagctgg gttcgaaatc 1020
   gattgattcc tttctttcct gattcggact tggggttgaa ctgatcggca ccaaacgcca 1080
   agttacacgg cctgatacca ccagcaccaa ccacaagaag cgctaaaccc aacatcagaa 1140
   acaaaatctg ccccgcacct ggcccttcgc atgaactctt tgttccacaa ggagtcgggt 1200
   gcagtcccgg aactgctgcc gtgagtagta tcgcaagc 1238
<210> 139
   <211> 1146
   <212> DNA
   <213> Artificial Sequence
<220>
   <223> dsRNA for downregulation of GTR2 expression in Brassica napus
<220>
   <221> misc_feature
   <222> (1)..(169)
   <223> Fragment of BnGTR2_A1 (bp 993 to 1101 from start codon in sense orientation)
<220>
   <221> misc_feature
   <222> (202)..(945)
   <223> intron-2 from the Pdk gene of Flaveria
<220>
   <221> misc_feature
   <222> (978)..(1146)
   <223> Fragment of BnGTR2_A1 (bp 993 to 1101 from start codon in antisense orientation)
<400> 139
   ccttgataag gcggcgattt tggctcccga agacaagttg gaggctgatg gtaagcctgc      60
   ggatccctgg aagctttgta caatgcaaca agttgaagaa gtgaagtgca ttgtgagagt     120
   gcttcctata tggtttgctg catcgatcta ttacttgacc atcacgcaag gtaccccagc     180
   ttggtaagga aataattatt ttcttttttc cttttagtat aaaatagtta agtgatgtta     240
   attagtatga ttataataat atagttgtta taattgtgaa aaaataattt ataaatatat     300
   tgtttacata aacaacatag taatgtaaaa aaatatgaca agtgatgtgt aagacgaaga     360
   agataaaagt tgagagtaag tatattattt ttaatgaatt tgatcgaaca tgtaagatga     420
   tatactagca ttaatatttg ttttaatcat aatagtaatt ctagctggtt tgatgaatta     480
   aatatcaatg ataaaatact atagtaaaaa taagaataaa taaattaaaa taatattttt     540
   ttatgattaa tagtttatta tataattaaa tatctatacc attactaaat attttagttt     600
   aaaagttaat aaatattttg ttagaaattc caatctgctt gtaatttatc aataaacaaa     660
   atattaaata acaagctaaa gtaacaaata atatcaaact aatagaaaca gtaatctaat     720
   gtaacaaaac ataatctaat gctaatataa caaagcgcaa gatctatcaa ttttatatag     780
   tattattttt caatcaacat tcttattaat ttctaaataa tacttgtagt tttattaact     840
   tctaaatgga ttgactatta attaaatgaa ttagtcgaac atgaataaac aaggtaacat     900
   gatagatcat gtcattgtgt tatcattgat cttacatttg gattgattac agttgggaag     960
   ctgggttcga aatcgatttg cgtgatggtc aagtaataga tcgatgcagc aaaccatata    1020
   ggaagcactc tcacaatgca cttcacttct tcaacttgtt gcattgtaca aagcttccag    1080
   ggatccgcag gcttaccatc agcctccaac ttgtcttcgg gagccaaaat cgccgcctta    1140
   tcaagg                                                               1146
<210> 140
   <211> 1576
   <212> DNA
   <213> Artificial Sequence
<220>
   <223> dsRNA for downregulation of GTR1 and GTR2 expression in Brassica napus
<220>
   <221> misc_feature
   <222> (1)..(215)
   <223> Fragment of gtr1Bn_A2 (bp 384 to 598 from start codon
<220>
   <221> misc_feature
   <222> (216)..(384)
   <223> Fragment of gtr2Bn_A1 (bp 933 to 1101 from start codon in sense orientation)
<220>
   <221> misc_feature
   <222> (417)..(1160)
   <223> intron-2 from the Pdk gene of Flaveria
<220>
   <221> misc_feature
   <222> (1193)..(1361)
   <223> Fragment of gtr2Bn_A1 (bp 933 to 1101 from start codon in antisense orientation
<220>
   <221> misc_feature
   <222> (1362)..(1576)
   <223> Fragment of gtr1Bn_A2 (bp 384 to 598 from start codon in antisense orientation)
<400> 140
   gcttgcgata ctactcacgg cagcagttcc gggactgcac ccgactcctt gtggaacaaa      60
   gagttcatgc gaagggccag gtgcggggca gattttgttt ctgatgttgg gtttagcgct     120
   tcttgtggtt ggtgctggtg gtatcaggcc gtgtaacttg gcgtttggtg ccgatcagtt     180
   caaccccaag tccgaatcag gaaagaaagg aatcaccttg ataaggcggc gattttggct     240
   cccgaagaca agttggaggc tgatggtaag cctgcggatc cctggaagct ttgtacaatg     300
   caacaagttg aagaagtgaa gtgcattgtg agagtgcttc ctatatggtt tgctgcatcg     360
   atctattact tgaccatcac gcaaggtacc ccagcttggt aaggaaataa ttattttctt     420
   ttttcctttt agtataaaat agttaagtga tgttaattag tatgattata ataatatagt     480
   tgttataatt gtgaaaaaat aatttataaa tatattgttt acataaacaa catagtaatg     540
   taaaaaaata tgacaagtga tgtgtaagac gaagaagata aaagttgaga gtaagtatat     600
   tatttttaat gaatttgatc gaacatgtaa gatgatatac tagcattaat atttgtttta     660
   atcataatag taattctagc tggtttgatg aattaaatat caatgataaa atactatagt     720
   aaaaataaga ataaataaat taaaataata tttttttatg attaatagtt tattatataa     780
   ttaaatatct ataccattac taaatatttt agtttaaaag ttaataaata ttttgttaga     840
   aattccaatc tgcttgtaat ttatcaataa acaaaatatt aaataacaag ctaaagtaac     900
   aaataatatc aaactaatag aaacagtaat ctaatgtaac aaaacataat ctaatgctaa     960
   tataacaaag cgcaagatct atcaatttta tatagtatta tttttcaatc aacattctta    1020
   ttaatttcta aataatactt gtagttttat taacttctaa atggattgac tattaattaa    1080
   atgaattagt cgaacatgaa taaacaaggt aacatgatag atcatgtcat tgtgttatca    1140
   ttgatcttac atttggattg attacagttg ggaagctggg ttcgaaatcg atttgcgtga    1200
   tggtcaagta atagatcgat gcagcaaacc atataggaag cactctcaca atgcacttca    1260
   cttcttcaac ttgttgcatt gtacaaagct tccagggatc cgcaggctta ccatcagcct    1320
   ccaacttgtc ttcgggagcc aaaatcgccg ccttatcaag gtgattcctt tctttcctga    1380
   ttcggacttg gggttgaact gatcggcacc aaacgccaag ttacacggcc tgataccacc    1440
   agcaccaacc acaagaagcg ctaaacccaa catcagaaac aaaatctgcc ccgcacctgg    1500
   cccttcgcat gaactctttg ttccacaagg agtcgggtgc agtcccggaa ctgctgccgt    1560
   gagtagtatc gcaagc                                                    1576
<210> 141
   <211> 1911
   <212> DNA
   <213> Arabidopsis thaliana
<220>
   <221> CDS
   <222> (1)..(1908)
<400> 141
   atg aag agc aga gtc att ctt aac cat aga gac aga aga gat aag aat      48
   Met Lys Ser Arg Val Ile Leu Asn His Arg Asp Arg Arg Asp Lys Asn
   1               5                   10                  15
   cat aac aac aac aac acc aat cat tac aca cag gtt gat act atg gag      96
   His Asn Asn Asn Asn Thr Asn His Tyr Thr Gln Val Asp Thr Met Glu
               20                  25                  30
   aga aag cct ctt gag gtt gag cca tct act acg acg act aac acc gat     144
   Arg Lys Pro Leu Glu Val Glu Pro Ser Thr Thr Thr Thr Asn Thr Asp
           35                  40                  45
   gtg gtc gat tct ttc gag gaa gag cag aga aaa atc gtt tat aga gga     192
   Val Val Asp Ser Phe Glu Glu Glu Gln Arg Lys Ile Val Tyr Arg Gly
       50                  55                  60
   tgg aaa gtc atg cct ttt atc att ggt aat gag aca ttt gag aag ctt     240
   Trp Lys Val Met Pro Phe Ile Ile Gly Asn Glu Thr Phe Glu Lys Leu
   65                  70                  75                  80
   ggg atc ata ggg aca tta tca aac ctt ctt gtg tac cta act tct gta     288
   Gly Ile Ile Gly Thr Leu Ser Asn Leu Leu Val Tyr Leu Thr Ser Val
                   85                  90                  95
   ttc aac ctt aag agc tac aca gct gca act atc atc aat gcc ttt agt      336
   Phe Asn Leu Lys Ser Tyr Thr Ala Ala Thr Ile Ile Asn Ala Phe Ser
               100                 105                 110
   ggt aca atc aat ttc ggg act ttt att gct gcc ttt ctt tgc gac act      384
   Gly Thr Ile Asn Phe Gly Thr Phe Ile Ala Ala Phe Leu Cys Asp Thr
           115                 120                 125
   tac ttt ggt cgc tac aag act ctc agt gtc gct gtc atc gct tgt ttt      432
   Tyr Phe Gly Arg Tyr Lys Thr Leu Ser Val Ala Val Ile Ala Cys Phe
       130                 135                 140
   ctg gga tcg ttt gtg ata cta ctt act gct gca att ccg tcg ttg cac      480
   Leu Gly Ser Phe Val Ile Leu Leu Thr Ala Ala Ile Pro Ser Leu His
   145                 150                 155                 160
   ccc gtt gct tgc gga aac aaa atc tcg tgc gaa ggg cca agt gtg gga      528
   Pro Val Ala Cys Gly Asn Lys Ile Ser Cys Glu Gly Pro Ser Val Gly
                   165                 170                 175
   cag ata ctg ttt ctg ctc atg ggt tta ggc ttt ctt gtg gta ggc gct      576
   Gln Ile Leu Phe Leu Leu Met Gly Leu Gly Phe Leu Val Val Gly Ala
               180                 185                 190
   ggt ggt atc aga ccg tgt aat tta gcg ttt ggt gct gat cag ttc aac      624
   Gly Gly Ile Arg Pro Cys Asn Leu Ala Phe Gly Ala Asp Gln Phe Asn
           195                 200                 205
   ccc aaa tcc gaa tca gga aag aaa ggg atc aac agt ttc ttc aac tgg      672
   Pro Lys Ser Glu Ser Gly Lys Lys Gly Ile Asn Ser Phe Phe Asn Trp
       210                 215                 220
   tat ttc ttc act ttc acg ttc gcg cag atc atc tcg ctc aca gca gtt      720
   Tyr Phe Phe Thr Phe Thr Phe Ala Gln Ile Ile Ser Leu Thr Ala Val
   225                 230                 235                 240
   gtg tat atc cag tca aat gtg agt tgg aca atc ggt ttg atc att ccg      768
   Val Tyr Ile Gln Ser Asn Val Ser Trp Thr Ile Gly Leu Ile Ile Pro
                   245                 250                 255
   gtg gct ctt atg ttc ttg gcg tgt gtg att ttc ttt gct ggc gat aga      816
   Val Ala Leu Met Phe Leu Ala Cys Val Ile Phe Phe Ala Gly Asp Arg
               260                 265                 270
   ttg tat gta aaa gtg aaa gcc tcg ggt agt cca ttg gct ggt att gct      864
   Leu Tyr Val Lys Val Lys Ala Ser Gly Ser Pro Leu Ala Gly Ile Ala
           275                 280                 285
   cgt gtt ata gca gcc gcg atc aag aaa cga ggt ttg aag ccg gtt aag      912
   Arg Val Ile Ala Ala Ala Ile Lys Lys Arg Gly Leu Lys Pro Val Lys
       290                 295                 300
   cag cct tgg gtc aat ctt tac aac cat atc ccg tct aac tat gca aat      960
   Gln Pro Trp Val Asn Leu Tyr Asn His Ile Pro Ser Asn Tyr Ala Asn
   305                 310                 315                 320
   act act cta aaa tac acc gac cag ttc aga ttt cta gac aaa gca gca     1008
   Thr Thr Leu Lys Tyr Thr Asp Gln Phe Arg Phe Leu Asp Lys Ala Ala
                   325                 330                 335
   att atg acc ccc gag gag aag ttg aac tcc gat gga acg gct tcc gat     1056
   Ile Met Thr Pro Glu Glu Lys Leu Asn Ser Asp Gly Thr Ala Ser Asp
               340                 345                 350
   cca tgg aaa cta tgc acg ttg cag caa gtg gaa gaa gtg aaa tgc att     1104
   Pro Trp Lys Leu Cys Thr Leu Gln Gln Val Glu Glu Val Lys Cys Ile
           355                 360                 365
   gta aga gtg att cca atc tgg ttt gca tcc acg ata tac tac ctt gca     1152
   Val Arg Val Ile Pro Ile Trp Phe Ala Ser Thr Ile Tyr Tyr Leu Ala
       370                 375                 380
   ata act ata caa atg act tat ccg gtc ttc caa gcg ctc cag agc gac     1200
   Ile Thr Ile Gln Met Thr Tyr Pro Val Phe Gln Ala Leu Gln Ser Asp
   385                 390                 395                 400
   cga cga tta ggt tct ggt ggc ttc agg att cct gcg gcc acc tat gta       1248
   Arg Arg Leu Gly Ser Gly Gly Phe Arg Ile Pro Ala Ala Thr Tyr Val
                   405                 410                 415
   gtg ttc ttg atg act ggc atg aca gtt ttc atc ata ttc tac gac cgt       1296
   Val Phe Leu Met Thr Gly Met Thr Val Phe Ile Ile Phe Tyr Asp Arg
               420                 425                 430
   gta ctt gtc ccg tcg ctc aga aga gtg acc ggg tta gaa acc ggt ata       1344
   Val Leu Val Pro Ser Leu Arg Arg Val Thr Gly Leu Glu Thr Gly Ile
           435                 440                 445
   tca ctc tta caa aga atc gga gct ggg ttt aca ttt gcg ata atg agt       1392
   Ser Leu Leu Gln Arg Ile Gly Ala Gly Phe Thr Phe Ala Ile Met Ser
       450                 455                 460
   tta tta gtt tcc ggg ttt ata gag gaa agg aga aga aac ttt gca ttg       1440
   Leu Leu Val Ser Gly Phe Ile Glu Glu Arg Arg Arg Asn Phe Ala Leu
   465                 470                 475                 480
   acc aaa ccg aca ctc ggg atg gcg cct cgg aca gga gaa atc tca tca       1488
   Thr Lys Pro Thr Leu Gly Met Ala Pro Arg Thr Gly Glu Ile Ser Ser
                   485                 490                 495
   atg tca gcg ttg tgg ctg att ccg cag ctt aca ctt gca ggc ata gca       1536
   Met Ser Ala Leu Trp Leu Ile Pro Gln Leu Thr Leu Ala Gly Ile Ala
               500                 505                 510
   gaa gct ttt gct gcc att gga caa atg gag ttt tac tac aag cag ttt       1584
   Glu Ala Phe Ala Ala Ile Gly Gln Met Glu Phe Tyr Tyr Lys Gln Phe
           515                 520                 525
   cct gaa aac atg aag agc ttc gcg ggt tct atc ttt tat gtc ggt gca       1632
   Pro Glu Asn Met Lys Ser Phe Ala Gly Ser Ile Phe Tyr Val Gly Ala
       530                 535                 540
   ggg gtt tcg agc tac ctc gct agc ttc ttg ata tca act gtt cat cga       1680
   Gly Val Ser Ser Tyr Leu Ala Ser Phe Leu Ile Ser Thr Val His Arg
   545                 550                 555                 560
   aca acc gcg cat tca cct tca ggg aat tgg tta gct gag gat ctg aac       1728
   Thr Thr Ala His Ser Pro Ser Gly Asn Trp Leu Ala Glu Asp Leu Asn
                   565                 570                 575
   aaa gcg aaa ttg gat tac ttc tac ttc atg ctc aca gga ctc atg gtt       1776
   Lys Ala Lys Leu Asp Tyr Phe Tyr Phe Met Leu Thr Gly Leu Met Val
               580                 585                 590
   gtt aac atg gct tac ttc ttg ttg atg gct aga tgg tat aga tac aaa       1824
   Val Asn Met Ala Tyr Phe Leu Leu Met Ala Arg Trp Tyr Arg Tyr Lys
           595                 600                 605
   gga ggt aat gat gaa gac atc act gag att gag acc aat gaa gaa gaa       1872
   Gly Gly Asn Asp Glu Asp Ile Thr Glu Ile Glu Thr Asn Glu Glu Glu
       610                 615                 620
   acc aag caa cag cag cta caa gac aag aac tct gtc tga                   1911
   Thr Lys Gln Gln Gln Leu Gln Asp Lys Asn Ser Val
   625                 630                 635
<210> 142
   <211> 636
   <212> PRT
   <213> Arabidopsis thaliana
<400> 142
   Met Lys Ser Arg Val Ile Leu Asn His Arg Asp Arg Arg Asp Lys Asn
   1               5                   10                  15
   His Asn Asn Asn Asn Thr Asn His Tyr Thr Gln Val Asp Thr Met Glu
               20                  25                  30
   Arg Lys Pro Leu Glu Val Glu Pro Ser Thr Thr Thr Thr Asn Thr Asp
   35 40 45
   Val Val Asp Ser Phe Glu Glu Glu Gln Arg Lys Ile Val Tyr Arg Gly
   50 55 60
   Trp Lys Val Met Pro Phe Ile Ile Gly Asn Glu Thr Phe Glu Lys Leu
   65 70 75 80
   Gly Ile Ile Gly Thr Leu Ser Asn Leu Leu Val Tyr Leu Thr Ser Val
   85 90 95
   Phe Asn Leu Lys Ser Tyr Thr Ala Ala Thr Ile Ile Asn Ala Phe Ser
   100 105 110
   Gly Thr Ile Asn Phe Gly Thr Phe Ile Ala Ala Phe Leu Cys Asp Thr
   115 120 125
   Tyr Phe Gly Arg Tyr Lys Thr Leu Ser Val Ala Val Ile Ala Cys Phe
   130 135 140
   Leu Gly Ser Phe Val Ile Leu Leu Thr Ala Ala Ile Pro Ser Leu His
   145 150 155 160
   Pro Val Ala Cys Gly Asn Lys Ile Ser Cys Glu Gly Pro Ser Val Gly
   165 170 175
   Gln Ile Leu Phe Leu Leu Met Gly Leu Gly Phe Leu Val Val Gly Ala
   180 185 190
   Gly Gly Ile Arg Pro Cys Asn Leu Ala Phe Gly Ala Asp Gln Phe Asn
   195 200 205
   Pro Lys Ser Glu Ser Gly Lys Lys Gly Ile Asn Ser Phe Phe Asn Trp
   210 215 220
   Tyr Phe Phe Thr Phe Thr Phe Ala Gln Ile Ile Ser Leu Thr Ala Val
   225 230 235 240
   Val Tyr Ile Gln Ser Asn Val Ser Trp Thr Ile Gly Leu Ile Ile Pro
   245 250 255
   Val Ala Leu Met Phe Leu Ala Cys Val Ile Phe Phe Ala Gly Asp Arg
   260 265 270
   Leu Tyr Val Lys Val Lys Ala Ser Gly Ser Pro Leu Ala Gly Ile Ala
   275 280 285
   Arg Val Ile Ala Ala Ala Ile Lys Lys Arg Gly Leu Lys Pro Val Lys
   290 295 300
   Gln Pro Trp Val Asn Leu Tyr Asn His Ile Pro Ser Asn Tyr Ala Asn
   305 310 315 320
   Thr Thr Leu Lys Tyr Thr Asp Gln Phe Arg Phe Leu Asp Lys Ala Ala
   325 330 335
   Ile Met Thr Pro Glu Glu Lys Leu Asn Ser Asp Gly Thr Ala Ser Asp
   340 345 350
   Pro Trp Lys Leu Cys Thr Leu Gln Gln Val Glu Glu Val Lys Cys Ile
   355 360 365
   Val Arg Val Ile Pro Ile Trp Phe Ala Ser Thr Ile Tyr Tyr Leu Ala
   370 375 380
   Ile Thr Ile Gln Met Thr Tyr Pro Val Phe Gln Ala Leu Gln Ser Asp
   385 390 395 400
   Arg Arg Leu Gly Ser Gly Gly Phe Arg Ile Pro Ala Ala Thr Tyr Val
   405 410 415
   Val Phe Leu Met Thr Gly Met Thr Val Phe Ile Ile Phe Tyr Asp Arg
   420 425 430
   Val Leu Val Pro Ser Leu Arg Arg Val Thr Gly Leu Glu Thr Gly Ile
   435 440 445
   Ser Leu Leu Gln Arg Ile Gly Ala Gly Phe Thr Phe Ala Ile Met Ser
   450 455 460
   Leu Leu Val Ser Gly Phe Ile Glu Glu Arg Arg Arg Asn Phe Ala Leu
   465 470 475 480
   Thr Lys Pro Thr Leu Gly Met Ala Pro Arg Thr Gly Glu Ile Ser Ser
   485 490 495
   Met Ser Ala Leu Trp Leu Ile Pro Gln Leu Thr Leu Ala Gly Ile Ala
   500 505 510
   Glu Ala Phe Ala Ala Ile Gly Gln Met Glu Phe Tyr Tyr Lys Gln Phe
   515 520 525
   Pro Glu Asn Met Lys Ser Phe Ala Gly Ser Ile Phe Tyr Val Gly Ala
   530 535 540
   Gly Val Ser Ser Tyr Leu Ala Ser Phe Leu Ile Ser Thr Val His Arg
   545 550 555 560
   Thr Thr Ala His Ser Pro Ser Gly Asn Trp Leu Ala Glu Asp Leu Asn
   565 570 575
   Lys Ala Lys Leu Asp Tyr Phe Tyr Phe Met Leu Thr Gly Leu Met Val
   580 585 590
   Val Asn Met Ala Tyr Phe Leu Leu Met Ala Arg Trp Tyr Arg Tyr Lys
   595 600 605
   Gly Gly Asn Asp Glu Asp Ile Thr Glu Ile Glu Thr Asn Glu Glu Glu
   610 615 620
   Thr Lys Gln Gln Gln Leu Gln Asp Lys Asn Ser Val
   625 630 635
<210> 143
   <211> 1905
   <212> DNA
   <213> Brassica napus
<220>
   <221> CDS
   <222> (1)..(1905)
<400> 143
   atg aag agc aga gtc atc cac agc cat aga gag aga aga gat aag cat      48
   Met Lys Ser Arg Val Ile His Ser His Arg Glu Arg Arg Asp Lys His
   1               5                   10                  15
   aat tac aca cag att gaa aca atg gag aga aag ccc ttt gat gtt gag      96
   Asn Tyr Thr Gln Ile Glu Thr Met Glu Arg Lys Pro Phe Asp Val Glu
               20                  25                  30
   aca act gag gat cac aaa ctc tac ccc tcc gtc gat ggc ggt ggt tct     144
   Thr Thr Glu Asp His Lys Leu Tyr Pro Ser Val Asp Gly Gly Gly Ser
           35                  40                  45
   gat tcg gtc gat tca ttt gaa gac gag cag aaa aag ctc gtt tat aga     192
   Asp Ser Val Asp Ser Phe Glu Asp Glu Gln Lys Lys Leu Val Tyr Arg
       50                  55                  60
   ggc tgg aaa gtc atg ccc ttt atc att ggt aat gag aca ttt gag aag     240
   Gly Trp Lys Val Met Pro Phe Ile Ile Gly Asn Glu Thr Phe Glu Lys
   65                  70                  75                  80
   ata ggg atc att ggg aca cta tca aac ctt ctg gtg tac cta act tca     288
   Ile Gly Ile Ile Gly Thr Leu Ser Asn Leu Leu Val Tyr Leu Thr Ser
                   85                  90                  95
   gta ttc aac ctt aag agt gtt aca gct gca acc atc atc aac gcc ttc     336
   Val Phe Asn Leu Lys Ser Val Thr Ala Ala Thr Ile Ile Asn Ala Phe
               100                 105                 110
   agt ggc act atc aac ttc ggc act ttc ctt gct gct ttc ctc tgc gac     384
   Ser Gly Thr Ile Asn Phe Gly Thr Phe Leu Ala Ala Phe Leu Cys Asp
           115                 120                 125
   act tac ttt ggt cgc tac aag act ctc tct gtt gct gtc atc gcc tgt     432
   Thr Tyr Phe Gly Arg Tyr Lys Thr Leu Ser Val Ala Val Ile Ala Cys
       130                 135                 140
   ttt ctg gga tcg ctt gtg ata cta ctg acg gct gca gtt cca gga ttg     480
   Phe Leu Gly Ser Leu Val Ile Leu Leu Thr Ala Ala Val Pro Gly Leu
   145                 150                 155                 160
   cac ccc att cct tgt gga aca cag agt tct tgc caa ggc cca agc gag     528
   His Pro Ile Pro Cys Gly Thr Gln Ser Ser Cys Gln Gly Pro Ser Glu
                   165                 170                 175
   ggc cag att gcg ttt ctt ctg atg ggt tta gcg ctt ctt gtg gtg ggt     576
   Gly Gln Ile Ala Phe Leu Leu Met Gly Leu Ala Leu Leu Val Val Gly
               180                 185                 190
   gct ggt gga att agg ccg tgt aac ttg gcc ttt ggt gct gac cag ttc     624
   Ala Gly Gly Ile Arg Pro Cys Asn Leu Ala Phe Gly Ala Asp Gln Phe
           195                 200                 205
   aac ccc aag tca gaa tct gga aag aaa gga atc aac agt ttc ttc aac     672
   Asn Pro Lys Ser Glu Ser Gly Lys Lys Gly Ile Asn Ser Phe Phe Asn
       210                 215                 220
   tgg tac ttc ttc act ttc act ttc gcg cag atc atc tct ctc acg cta     720
   Trp Tyr Phe Phe Thr Phe Thr Phe Ala Gln Ile Ile Ser Leu Thr Leu
   225                 230                 235                 240
   gtt gtg tat atc cag tca aat gtg agc tgg acc atc ggt ttg agt atc     768
   Val Val Tyr Ile Gln Ser Asn Val Ser Trp Thr Ile Gly Leu Ser Ile
                   245                 250                 255
   cct gtg ggt ctt atg ttc ttg gcc tgc gtc atc ttc ttt gct ggt cat      816
   Pro Val Gly Leu Met Phe Leu Ala Cys Val Ile Phe Phe Ala Gly His
               260                 265                 270
   aaa ctg tat gtg aaa gtg aaa gcc tct ggt agt cca ttg gct ggt ata      864
   Lys Leu Tyr Val Lys Val Lys Ala Ser Gly Ser Pro Leu Ala Gly Ile
           275                 280                 285
   ggt cat gtt ata gcc gca gcg atc aag aaa cgt ggc ttg aag cca gtt      912
   Gly His Val Ile Ala Ala Ala Ile Lys Lys Arg Gly Leu Lys Pro Val
       290                 295                 300
   aag cag cct tgg atc gat ctt tac aac cac att cct cat aac tat gca      960
   Lys Gln Pro Trp Ile Asp Leu Tyr Asn His Ile Pro His Asn Tyr Ala
   305                 310                 315                 320
   aac tct act ctt aag tac acc gac cag cta agg ttt cta gac aaa gca     1008
   Asn Ser Thr Leu Lys Tyr Thr Asp Gln Leu Arg Phe Leu Asp Lys Ala
                   325                 330                 335
   gcg att atg acc gct gag gac aag ttg aac tcg gat gga acg gct tcg     1056
   Ala Ile Met Thr Ala Glu Asp Lys Leu Asn Ser Asp Gly Thr Ala Ser
               340                 345                 350
   gat cca tgg aaa cta tgt acg atg cag aaa gtg gaa gaa gtg aaa tgc     1104
   Asp Pro Trp Lys Leu Cys Thr Met Gln Lys Val Glu Glu Val Lys Cys
           355                 360                 365
   att tta aga gtg att cca atc tgg ttc gca tgc gcc att tac tac ctt     1152
   Ile Leu Arg Val Ile Pro Ile Trp Phe Ala Cys Ala Ile Tyr Tyr Leu
       370                 375                 380
   gca ata agt atg caa atg act tat cca gtc ttc caa gcg ctt cag agc     1200
   Ala Ile Ser Met Gln Met Thr Tyr Pro Val Phe Gln Ala Leu Gln Ser
   385                 390                 395                 400
   gac agg cga tta ggt tca ggt ggc ttc aag att cca gcc gcc acc tat     1248
   Asp Arg Arg Leu Gly Ser Gly Gly Phe Lys Ile Pro Ala Ala Thr Tyr
                   405                 410                 415
   gtc gtg ttc ttg atg tct ggt atg acg gtt ttc atc ata atc tac gac     1296
   Val Val Phe Leu Met Ser Gly Met Thr Val Phe Ile Ile Ile Tyr Asp
               420                 425                 430
   cgt gtc ctc gtc ccc tcg ctc aaa aga gtg acc ggt cta gac acg ggg     1344
   Arg Val Leu Val Pro Ser Leu Lys Arg Val Thr Gly Leu Asp Thr Gly
           435                 440                 445
   ata aca ctc tta caa aga ata gga gct ggc att ttc ttt gcg gtg ttg     1392
   Ile Thr Leu Leu Gln Arg Ile Gly Ala Gly Ile Phe Phe Ala Val Leu
       450                 455                 460
   agt tta ttg gtc tcc ggg ttc ata gag gaa cgt aga aga acc ttt gcg     1440
   Ser Leu Leu Val Ser Gly Phe Ile Glu Glu Arg Arg Arg Thr Phe Ala
   465                 470                 475                 480
   ctg aca cga cct acg ctg ggg tcg gag cct cga aca gga gag atc tcc     1488
   Leu Thr Arg Pro Thr Leu Gly Ser Glu Pro Arg Thr Gly Glu Ile Ser
                   485                 490                 495
   tca atg tca ggg atg tgg ctg att ccg cag ctg gtg ctt gca ggg gta     1536
   Ser Met Ser Gly Met Trp Leu Ile Pro Gln Leu Val Leu Ala Gly Val
               500                 505                 510
   gca gaa gct ttt gca gcc att ggg caa atg gag ttt tac tac aag cag     1584
   Ala Glu Ala Phe Ala Ala Ile Gly Gln Met Glu Phe Tyr Tyr Lys Gln
           515                 520                 525
   ttt cct gaa aac atg aag agc ttt gcg ggt tct atc ttc tat gtc ggt     1632
   Phe Pro Glu Asn Met Lys Ser Phe Ala Gly Ser Ile Phe Tyr Val Gly
       530                 535                 540
   gga gga gtt tct agc tac ctc gct agc ttc ttg atc tcg act gtt cat     1680
   Gly Gly Val Ser Ser Tyr Leu Ala Ser Phe Leu Ile Ser Thr Val His
   545                 550                 555                 560
   aga aca act gag cat tca cct acg ggg aat tgg tta gct gag gat ctg      1728
   Arg Thr Thr Glu His Ser Pro Thr Gly Asn Trp Leu Ala Glu Asp Leu
                   565                 570                 575
   aac aaa gcg aag ctg gac tac ttc tat ttc atg ctc aca gga ctc atg      1776
   Asn Lys Ala Lys Leu Asp Tyr Phe Tyr Phe Met Leu Thr Gly Leu Met
               580                 585                 590
   ttt gtt aac ttt ctt tac ttc ttg tta atg gct aga tgg tat aga tac      1824
   Phe Val Asn Phe Leu Tyr Phe Leu Leu Met Ala Arg Trp Tyr Arg Tyr
           595                 600                 605
   aaa ggc act cat gat gaa gtc agt tat gtg gtt gac act gtt gac gaa      1872
   Lys Gly Thr His Asp Glu Val Ser Tyr Val Val Asp Thr Val Asp Glu
       610                 615                 620
   gag aca aag caa caa gac aag aac tct gtc tga                          1905
   Glu Thr Lys Gln Gln Asp Lys Asn Ser Val
   625                 630
<210> 144
   <211> 634
   <212> PRT
   <213> Brassica napus
<400> 144
   Met Lys Ser Arg Val Ile His Ser His Arg Glu Arg Arg Asp Lys His
   1               5                   10                  15
   Asn Tyr Thr Gln Ile Glu Thr Met Glu Arg Lys Pro Phe Asp Val Glu
               20                  25                  30
   Thr Thr Glu Asp His Lys Leu Tyr Pro Ser Val Asp Gly Gly Gly Ser
           35                  40                  45
   Asp Ser Val Asp Ser Phe Glu Asp Glu Gln Lys Lys Leu Val Tyr Arg
       50                  55                  60
   Gly Trp Lys Val Met Pro Phe Ile Ile Gly Asn Glu Thr Phe Glu Lys
   65                  70                  75                  80
   Ile Gly Ile Ile Gly Thr Leu Ser Asn Leu Leu Val Tyr Leu Thr Ser
                   85                  90                  95
   Val Phe Asn Leu Lys Ser Val Thr Ala Ala Thr Ile Ile Asn Ala Phe
               100                 105                 110
   Ser Gly Thr Ile Asn Phe Gly Thr Phe Leu Ala Ala Phe Leu Cys Asp
           115                 120                 125
   Thr Tyr Phe Gly Arg Tyr Lys Thr Leu Ser Val Ala Val Ile Ala Cys
       130                 135                 140
   Phe Leu Gly Ser Leu Val Ile Leu Leu Thr Ala Ala Val Pro Gly Leu
   145                 150                 155                 160
   His Pro Ile Pro Cys Gly Thr Gln Ser Ser Cys Gln Gly Pro Ser Glu
                   165                 170                 175
   Gly Gln Ile Ala Phe Leu Leu Met Gly Leu Ala Leu Leu Val Val Gly
               180                 185                 190
   Ala Gly Gly Ile Arg Pro Cys Asn Leu Ala Phe Gly Ala Asp Gln Phe
           195                 200                 205
   Asn Pro Lys Ser Glu Ser Gly Lys Lys Gly Ile Asn Ser Phe Phe Asn
       210                 215                 220
   Trp Tyr Phe Phe Thr Phe Thr Phe Ala Gln Ile Ile Ser Leu Thr Leu
   225                 230                 235                 240
   Val Val Tyr Ile Gln Ser Asn Val Ser Trp Thr Ile Gly Leu Ser Ile
                   245                 250                 255
   Pro Val Gly Leu Met Phe Leu Ala Cys Val Ile Phe Phe Ala Gly His
               260                 265                 270
   Lys Leu Tyr Val Lys Val Lys Ala Ser Gly Ser Pro Leu Ala Gly Ile
           275                 280                 285
   Gly His Val Ile Ala Ala Ala Ile Lys Lys Arg Gly Leu Lys Pro Val
       290                 295                 300
   Lys Gln Pro Trp Ile Asp Leu Tyr Asn His Ile Pro His Asn Tyr Ala
   305                 310                 315                 320
   Asn Ser Thr Leu Lys Tyr Thr Asp Gln Leu Arg Phe Leu Asp Lys Ala
                   325                 330                 335
   Ala Ile Met Thr Ala Glu Asp Lys Leu Asn Ser Asp Gly Thr Ala Ser
               340                 345                 350
   Asp Pro Trp Lys Leu Cys Thr Met Gln Lys Val Glu Glu Val Lys Cys
           355                 360                 365
   Ile Leu Arg Val Ile Pro Ile Trp Phe Ala Cys Ala Ile Tyr Tyr Leu
       370                 375                 380
   Ala Ile Ser Met Gln Met Thr Tyr Pro Val Phe Gln Ala Leu Gln Ser
   385                 390                 395                 400
   Asp Arg Arg Leu Gly Ser Gly Gly Phe Lys Ile Pro Ala Ala Thr Tyr
                   405                 410                 415
   Val Val Phe Leu Met Ser Gly Met Thr Val Phe Ile Ile Ile Tyr Asp
               420                 425                 430
   Arg Val Leu Val Pro Ser Leu Lys Arg Val Thr Gly Leu Asp Thr Gly
           435                 440                 445
   Ile Thr Leu Leu Gln Arg Ile Gly Ala Gly Ile Phe Phe Ala Val Leu
       450                 455                 460
   Ser Leu Leu Val Ser Gly Phe Ile Glu Glu Arg Arg Arg Thr Phe Ala
   465                 470                 475                 480
   Leu Thr Arg Pro Thr Leu Gly Ser Glu Pro Arg Thr Gly Glu Ile Ser
                   485                 490                 495
   Ser Met Ser Gly Met Trp Leu Ile Pro Gln Leu Val Leu Ala Gly Val
   500 505 510
   Ala Glu Ala Phe Ala Ala Ile Gly Gln Met Glu Phe Tyr Tyr Lys Gln
   515 520 525
   Phe Pro Glu Asn Met Lys Ser Phe Ala Gly Ser Ile Phe Tyr Val Gly
   530 535 540
   Gly Gly Val Ser Ser Tyr Leu Ala Ser Phe Leu Ile Ser Thr Val His
   545 550 555 560
   Arg Thr Thr Glu His Ser Pro Thr Gly Asn Trp Leu Ala Glu Asp Leu
   565 570 575
   Asn Lys Ala Lys Leu Asp Tyr Phe Tyr Phe Met Leu Thr Gly Leu Met
   580 585 590
   Phe Val Asn Phe Leu Tyr Phe Leu Leu Met Ala Arg Trp Tyr Arg Tyr
   595 600 605
   Lys Gly Thr His Asp Glu Val Ser Tyr Val Val Asp Thr Val Asp Glu
   610 615 620
   Glu Thr Lys Gln Gln Asp Lys Asn Ser Val
   625 630
<210> 145
   <211> 1905
   <212> DNA
   <213> Brassica napus
<220>
   <221> CDS
   <222> (1)..(1905)
<400> 145
   atg aag agc aga gtc atc ctc agc cat aga gag aga aga gat aag cat         48
   Met Lys Ser Arg Val Ile Leu Ser His Arg Glu Arg Arg Asp Lys His
   1               5                   10                  15
   aat tat aca cag att gaa aca atg gag aga aag ccc ttt gat gtt gag         96
   Asn Tyr Thr Gln Ile Glu Thr Met Glu Arg Lys Pro Phe Asp Val Glu
               20                  25                  30
   aca act gag gat cac aaa gcc tac tcc tcc gtc gat ggc ggt ggt tct        144
   Thr Thr Glu Asp His Lys Ala Tyr Ser Ser Val Asp Gly Gly Gly Ser
           35                  40                  45
   gat tcg gtc gat tca ttt gaa gac gag cag aaa aag ctc gtt tat aga        192
   Asp Ser Val Asp Ser Phe Glu Asp Glu Gln Lys Lys Leu Val Tyr Arg
       50                  55                  60
   ggc tgg aaa gtc atg ccc ttt atc att ggt aat gag aca ttt gag aag        240
   Gly Trp Lys Val Met Pro Phe Ile Ile Gly Asn Glu Thr Phe Glu Lys
   65                  70                  75                  80
   ata ggg atc att ggg aca cta tca aac ctt ctg gtg tac cta act tca        288
   Ile Gly Ile Ile Gly Thr Leu Ser Asn Leu Leu Val Tyr Leu Thr Ser
                   85                  90                  95
   gta ttc aac ctt aag agt gtt aca gct gca acc atc atc aac gcc ttc        336
   Val Phe Asn Leu Lys Ser Val Thr Ala Ala Thr Ile Ile Asn Ala Phe
               100                 105                 110
   agt ggc act atc aat ttc ggc act ttc ctc gct gct ttc ctc tgc gac        384
   Ser Gly Thr Ile Asn Phe Gly Thr Phe Leu Ala Ala Phe Leu Cys Asp
           115                 120                 125
   act tac ttt ggt cgc tac aag act ctc tct gtt gct gtc atc gcc tgt      432
   Thr Tyr Phe Gly Arg Tyr Lys Thr Leu Ser Val Ala Val Ile Ala Cys
       130                 135                 140
   ttt ctg gga tcg ctt gtg ata cta ctg acg gct gca gtt cca gga ttg      480
   Phe Leu Gly Ser Leu Val Ile Leu Leu Thr Ala Ala Val Pro Gly Leu
   145                 150                 155                 160
   cac cct att cct tgt gga aca caa agt tct tgc caa ggc cca agc gag      528
   His Pro Ile Pro Cys Gly Thr Gln Ser Ser Cys Gln Gly Pro Ser Glu
                   165                 170                 175
   ggc cag att gcg ttt ctg ctg atg ggt tta gcg ctt ctt gtg gtg ggt      576
   Gly Gln Ile Ala Phe Leu Leu Met Gly Leu Ala Leu Leu Val Val Gly
               180                 185                 190
   gct ggt gga atc agg ccg tgt aac ttg gcc ttt ggt gct gac cag ttc      624
   Ala Gly Gly Ile Arg Pro Cys Asn Leu Ala Phe Gly Ala Asp Gln Phe
           195                 200                 205
   aac ccc aag tca gaa tct gga aag aaa gga atc aat agt ttc ttc aac      672
   Asn Pro Lys Ser Glu Ser Gly Lys Lys Gly Ile Asn Ser Phe Phe Asn
       210                 215                 220
   tgg tac ttc ttc act ttc acg ttc gcg cag atc atc tcc ctc aca cta      720
   Trp Tyr Phe Phe Thr Phe Thr Phe Ala Gln Ile Ile Ser Leu Thr Leu
   225                 230                 235                 240
   gtt gtg tat atc cag tca aat gtg agc tgg acc atc ggt ttg agt atc      768
   Val Val Tyr Ile Gln Ser Asn Val Ser Trp Thr Ile Gly Leu Ser Ile
                   245                 250                 255
   cct gtg ggt ctt atg ttc ttg gcc tgc gtc att ttc ttt gct ggt cac      816
   Pro Val Gly Leu Met Phe Leu Ala Cys Val Ile Phe Phe Ala Gly His
               260                 265                 270
   aaa ctg tat gtg aaa gtg aaa gcc tct ggt agt cca ttg gct ggt ata      864
   Lys Leu Tyr Val Lys Val Lys Ala Ser Gly Ser Pro Leu Ala Gly Ile
           275                 280                 285
   ggt cat gtt ata gcc gca gct atc aag aaa cgt ggc ttg aag cca gtt      912
   Gly His Val Ile Ala Ala Ala Ile Lys Lys Arg Gly Leu Lys Pro Val
       290                 295                 300
   aag cag cct tgg atc gat ctt tac aac cac att cct cct aac tat gca      960
   Lys Gln Pro Trp Ile Asp Leu Tyr Asn His Ile Pro Pro Asn Tyr Ala
   305                 310                 315                 320
   aac tct act ctt aaa tac acc gac cag ctt agg ttt cta gac aaa gca     1008
   Asn Ser Thr Leu Lys Tyr Thr Asp Gln Leu Arg Phe Leu Asp Lys Ala
                   325                 330                 335
   gcg att atg acc cct gag gac aag ttg aac tcg gat gga acg gct tcg     1056
   Ala Ile Met Thr Pro Glu Asp Lys Leu Asn Ser Asp Gly Thr Ala Ser
               340                 345                 350
   gat cca tgg aaa cta tgt acg atg cag aaa gtg gaa gaa gtg aaa tgc     1104
   Asp Pro Trp Lys Leu Cys Thr Met Gln Lys Val Glu Glu Val Lys Cys
           355                 360                 365
   att tta aga gtg att cca atc tgg ttc gca tgc gcc att tac tac ctt     1152
   Ile Leu Arg Val Ile Pro Ile Trp Phe Ala Cys Ala Ile Tyr Tyr Leu
       370                 375                 380
   gca ata agt atg caa atg act tat cca gtc ttc caa gct ctc cag agc     1200
   Ala Ile Ser Met Gln Met Thr Tyr Pro Val Phe Gln Ala Leu Gln Ser
   385                 390                 395                 400
   gac agg cta tta ggt tca ggt ggc ttc aag att cca gcc gcc acc tat     1248
   Asp Arg Leu Leu Gly Ser Gly Gly Phe Lys Ile Pro Ala Ala Thr Tyr
                   405                 410                 415
   gtc gtg ttc ttg atg tct ggt atg acg gtt ttc atc ata atc tac gac     1296
   Val Val Phe Leu Met Ser Gly Met Thr Val Phe Ile Ile Ile Tyr Asp
               420                 425                 430
   cgt gtc ctt gtc ccc tcg ctc aaa aga gtg acc ggt cta gac acg ggg      1344
   Arg Val Leu Val Pro Ser Leu Lys Arg Val Thr Gly Leu Asp Thr Gly
           435                 440                 445
   ata aca ctc tta caa aga ata gga gct ggc att ttc ttt gcc gtg ttg      1392
   Ile Thr Leu Leu Gln Arg Ile Gly Ala Gly Ile Phe Phe Ala Val Leu
       450                 455                 460
   agt tta ttg gtc tcc ggg ttt ata gaa gaa cgt aga aga acc ttt gcg      1440
   Ser Leu Leu Val Ser Gly Phe Ile Glu Glu Arg Arg Arg Thr Phe Ala
   465                 470                 475                 480
   ctg aca cga cct acg ctc ggg tcg gag cct cga aca gga gag atc tcc      1488
   Leu Thr Arg Pro Thr Leu Gly Ser Glu Pro Arg Thr Gly Glu Ile Ser
                   485                 490                 495
   tca atg tca gcg atg tgg ctg att ccg cag ctg gtg ctt gca ggg gta      1536
   Ser Met Ser Ala Met Trp Leu Ile Pro Gln Leu Val Leu Ala Gly Val
               500                 505                 510
   gca gaa gct ttt gta gcc att ggg caa atg gag ttt tac tac aag cag      1584
   Ala Glu Ala Phe Val Ala Ile Gly Gln Met Glu Phe Tyr Tyr Lys Gln
           515                 520                 525
   ttt cct gaa aac atg aag agc ttt gcg ggt tct atc ttc tat gtc ggt      1632
   Phe Pro Glu Asn Met Lys Ser Phe Ala Gly Ser Ile Phe Tyr Val Gly
       530                 535                 540
   gga gga gtt tct agc tac ctc gct agc ttc ttg atc tcg act gtt cat      1680
   Gly Gly Val Ser Ser Tyr Leu Ala Ser Phe Leu Ile Ser Thr Val His
   545                 550                 555                 560
   aga aca act gag cat tca cct acg ggg aat tgg tta gct gag gat ctg      1728
   Arg Thr Thr Glu His Ser Pro Thr Gly Asn Trp Leu Ala Glu Asp Leu
                   565                 570                 575
   aac aaa gcg aag ctg gac tac ttc tat ttc atg ctc aca gga ctc atg      1776
   Asn Lys Ala Lys Leu Asp Tyr Phe Tyr Phe Met Leu Thr Gly Leu Met
               580                 585                 590
   ttt gtc aac ttt ctt tac ttc ttg tta atg gct aga tgg tat aga tac      1824
   Phe Val Asn Phe Leu Tyr Phe Leu Leu Met Ala Arg Trp Tyr Arg Tyr
           595                 600                 605
   aaa ggc act cat gat gaa gtc agt tat gtg gtt gac act gtt gac gaa      1872
   Lys Gly Thr His Asp Glu Val Ser Tyr Val Val Asp Thr Val Asp Glu
       610                 615                 620
   gag acg aag caa caa gac aag aac tct gtc tga                          1905
   Glu Thr Lys Gln Gln Asp Lys Asn Ser Val
   625                 630
<210> 146
   <211> 634
   <212> PRT
   <213> Brassica napus
<400> 146
   Met Lys Ser Arg Val Ile Leu Ser His Arg Glu Arg Arg Asp Lys His
   1               5                   10                  15
   Asn Tyr Thr Gln Ile Glu Thr Met Glu Arg Lys Pro Phe Asp Val Glu
               20                  25                  30
   Thr Thr Glu Asp His Lys Ala Tyr Ser Ser Val Asp Gly Gly Gly Ser
           35                  40                  45
   Asp Ser Val Asp Ser Phe Glu Asp Glu Gln Lys Lys Leu Val Tyr Arg
       50                  55                  60
   Gly Trp Lys Val Met Pro Phe Ile Ile Gly Asn Glu Thr Phe Glu Lys
   65                  70                  75                  80
   Ile Gly Ile Ile Gly Thr Leu Ser Asn Leu Leu Val Tyr Leu Thr Ser
                   85                  90                  95
   Val Phe Asn Leu Lys Ser Val Thr Ala Ala Thr Ile Ile Asn Ala Phe
               100                 105                 110
   Ser Gly Thr Ile Asn Phe Gly Thr Phe Leu Ala Ala Phe Leu Cys Asp
           115                 120                 125
   Thr Tyr Phe Gly Arg Tyr Lys Thr Leu Ser Val Ala Val Ile Ala Cys
       130                 135                 140
   Phe Leu Gly Ser Leu Val Ile Leu Leu Thr Ala Ala Val Pro Gly Leu
   145                 150                 155                 160
   His Pro Ile Pro Cys Gly Thr Gln Ser Ser Cys Gln Gly Pro Ser Glu
                   165                 170                 175
   Gly Gln Ile Ala Phe Leu Leu Met Gly Leu Ala Leu Leu Val Val Gly
               180                 185                 190
   Ala Gly Gly Ile Arg Pro Cys Asn Leu Ala Phe Gly Ala Asp Gln Phe
           195                 200                 205
   Asn Pro Lys Ser Glu Ser Gly Lys Lys Gly Ile Asn Ser Phe Phe Asn
       210                 215                 220
   Trp Tyr Phe Phe Thr Phe Thr Phe Ala Gln Ile Ile Ser Leu Thr Leu
   225                 230                 235                 240
   Val Val Tyr Ile Gln Ser Asn Val Ser Trp Thr Ile Gly Leu Ser Ile
                   245                 250                 255
   Pro Val Gly Leu Met Phe Leu Ala Cys Val Ile Phe Phe Ala Gly His
               260                 265                 270
   Lys Leu Tyr Val Lys Val Lys Ala Ser Gly Ser Pro Leu Ala Gly Ile
           275                 280                 285
   Gly His Val Ile Ala Ala Ala Ile Lys Lys Arg Gly Leu Lys Pro Val
       290                 295                 300
   Lys Gln Pro Trp Ile Asp Leu Tyr Asn His Ile Pro Pro Asn Tyr Ala
   305                 310                 315                 320
   Asn Ser Thr Leu Lys Tyr Thr Asp Gln Leu Arg Phe Leu Asp Lys Ala
                   325                 330                 335
   Ala Ile Met Thr Pro Glu Asp Lys Leu Asn Ser Asp Gly Thr Ala Ser
               340                 345                 350
   Asp Pro Trp Lys Leu Cys Thr Met Gln Lys Val Glu Glu Val Lys Cys
   355 360 365
   Ile Leu Arg Val Ile Pro Ile Trp Phe Ala Cys Ala Ile Tyr Tyr Leu
   370 375 380
   Ala Ile Ser Met Gln Met Thr Tyr Pro Val Phe Gln Ala Leu Gln Ser
   385 390 395 400
   Asp Arg Leu Leu Gly Ser Gly Gly Phe Lys Ile Pro Ala Ala Thr Tyr
   405 410 415
   Val Val Phe Leu Met Ser Gly Met Thr Val Phe Ile Ile Ile Tyr Asp
   420 425 430
   Arg Val Leu Val Pro Ser Leu Lys Arg Val Thr Gly Leu Asp Thr Gly
   435 440 445
   Ile Thr Leu Leu Gln Arg Ile Gly Ala Gly Ile Phe Phe Ala Val Leu
   450 455 460
   Ser Leu Leu Val Ser Gly Phe Ile Glu Glu Arg Arg Arg Thr Phe Ala
   465 470 475 480
   Leu Thr Arg Pro Thr Leu Gly Ser Glu Pro Arg Thr Gly Glu Ile Ser
   485 490 495
   Ser Met Ser Ala Met Trp Leu Ile Pro Gln Leu Val Leu Ala Gly Val
   500 505 510
   Ala Glu Ala Phe Val Ala Ile Gly Gln Met Glu Phe Tyr Tyr Lys Gln
   515 520 525
   Phe Pro Glu Asn Met Lys Ser Phe Ala Gly Ser Ile Phe Tyr Val Gly
   530 535 540
   Gly Gly Val Ser Ser Tyr Leu Ala Ser Phe Leu Ile Ser Thr Val His
   545 550 555 560
   Arg Thr Thr Glu His Ser Pro Thr Gly Asn Trp Leu Ala Glu Asp Leu
   565 570 575
   Asn Lys Ala Lys Leu Asp Tyr Phe Tyr Phe Met Leu Thr Gly Leu Met
   580 585 590
   Phe Val Asn Phe Leu Tyr Phe Leu Leu Met Ala Arg Trp Tyr Arg Tyr
   595 600 605
   Lys Gly Thr His Asp Glu Val Ser Tyr Val Val Asp Thr Val Asp Glu
   610 615 620
   Glu Thr Lys Gln Gln Asp Lys Asn Ser Val
   625 630
<210> 147
   <211> 1905
   <212> DNA
   <213> Brassica rapa
<220>
   <221> CDS
   <222> (1)..(1905)
<400> 147
   atg aag agc aga gtc atc cac agc cat aga gag aga aga gat aag cat      48
   Met Lys Ser Arg Val Ile His Ser His Arg Glu Arg Arg Asp Lys His
   1               5                   10                  15
   aat tac aca cag att gga aca atg gag aga aag ccc ttt gat gtt gag      96
   Asn Tyr Thr Gln Ile Gly Thr Met Glu Arg Lys Pro Phe Asp Val Glu
               20                  25                  30
   aca act gag gat cac aaa ccc tac tcc tcc gtc gat gac ggt ggt tct      144
   Thr Thr Glu Asp His Lys Pro Tyr Ser Ser Val Asp Asp Gly Gly Ser
           35                  40                  45
   gat tcg gtc gat tca ttt gaa aac gag cag aaa aag ctc gtt tat aga      192
   Asp Ser Val Asp Ser Phe Glu Asn Glu Gln Lys Lys Leu Val Tyr Arg
       50                  55                  60
   ggc tgg aaa gtc atg ccc ttt atc att ggt aat gag aca ttt gag aag      240
   Gly Trp Lys Val Met Pro Phe Ile Ile Gly Asn Glu Thr Phe Glu Lys
   65                  70                  75                  80
   ata ggg atc att ggg aca cta tca aac ctt ctg gtg tac cta act tca      288
   Ile Gly Ile Ile Gly Thr Leu Ser Asn Leu Leu Val Tyr Leu Thr Ser
                   85                  90                  95
   gta ttc aac ctc aag agt gtt aca gct gca acc atc atc aac gcc ttc      336
   Val Phe Asn Leu Lys Ser Val Thr Ala Ala Thr Ile Ile Asn Ala Phe
               100                 105                 110
   agt ggc act atc aac ttc ggc act ttc ctc gct gct ttc ctc tgc gac      384
   Ser Gly Thr Ile Asn Phe Gly Thr Phe Leu Ala Ala Phe Leu Cys Asp
           115                 120                 125
   act tac ttt ggt cgc tac aag act ctc tct gtt gct gtc atc gcc tgt      432
   Thr Tyr Phe Gly Arg Tyr Lys Thr Leu Ser Val Ala Val Ile Ala Cys
       130                 135                 140
   ttt ctg gga tcg ctt gtg ata cta ctg acg gct gca gtt cca gga ttg      480
   Phe Leu Gly Ser Leu Val Ile Leu Leu Thr Ala Ala Val Pro Gly Leu
   145                 150                 155                 160
   cac ccc att cct tgt gga aca caa agt tct tgc caa ggc cca agc gag      528
   His Pro Ile Pro Cys Gly Thr Gln Ser Ser Cys Gln Gly Pro Ser Glu
                   165                 170                 175
   ggc cag att gcg ttt ctt ctg atg ggt tta gcg ctt ctt gtg gtg ggt      576
   Gly Gln Ile Ala Phe Leu Leu Met Gly Leu Ala Leu Leu Val Val Gly
               180                 185                 190
   gct ggt gga atc agg ccg tgt aac ttg gcc ttt ggt gct gac cag ttc      624
   Ala Gly Gly Ile Arg Pro Cys Asn Leu Ala Phe Gly Ala Asp Gln Phe
           195                 200                 205
   aac ccc aag tca gaa tct gga aag aaa gga atc aac agt ttc ttc aac      672
   Asn Pro Lys Ser Glu Ser Gly Lys Lys Gly Ile Asn Ser Phe Phe Asn
       210                 215                 220
   tgg tac ttc ttc act ttc acg ttc gcg cag atc atc tcc ctc acg cta      720
   Trp Tyr Phe Phe Thr Phe Thr Phe Ala Gln Ile Ile Ser Leu Thr Leu
   225                 230                 235                 240
   gtt gtg tac atc cag tca aat gtg agc tgg acc atc ggt ttg agt atc      768
   Val Val Tyr Ile Gln Ser Asn Val Ser Trp Thr Ile Gly Leu Ser Ile
                   245                 250                 255
   cct gtg ggt ctt atg ttc ttg gcc tgt gtc att ttc ttt gct ggt cat      816
   Pro Val Gly Leu Met Phe Leu Ala Cys Val Ile Phe Phe Ala Gly His
               260                 265                 270
   aaa ctg tat gtg aaa gtg aaa gcc tct ggt agt cca ttg gct ggt ata      864
   Lys Leu Tyr Val Lys Val Lys Ala Ser Gly Ser Pro Leu Ala Gly Ile
               275                 280                 285
   ggt cat gtt ata gcc gca gcg atc aag aaa cgt ggc ttg aag cca gtt          912
   Gly His Val Ile Ala Ala Ala Ile Lys Lys Arg Gly Leu Lys Pro Val
       290                 295                 300
   aag cag cct tgg atc gat ctt tac aac cac att cct cat aac tat gca          960
   Lys Gln Pro Trp Ile Asp Leu Tyr Asn His Ile Pro His Asn Tyr Ala
   305                 310                 315                 320
   aac tct act ctt aaa tac acc gac cag ctt agg ttt cta gac aaa gca         1008
   Asn Ser Thr Leu Lys Tyr Thr Asp Gln Leu Arg Phe Leu Asp Lys Ala
                   325                 330                 335
   gcg att atg acc cct gag gac aag ttg aac tcg gat gga acg gct tcg         1056
   Ala Ile Met Thr Pro Glu Asp Lys Leu Asn Ser Asp Gly Thr Ala Ser
               340                 345                 350
   gat cca tgg aaa cta tgt acg atg cag aaa gtg gaa gaa gtg aaa tgc         1104
   Asp Pro Trp Lys Leu Cys Thr Met Gln Lys Val Glu Glu Val Lys Cys
           355                 360                 365
   att tta aga gtg att cca atc tgg ttc gca tgc gcc att tac tac ctt         1152
   Ile Leu Arg Val Ile Pro Ile Trp Phe Ala Cys Ala Ile Tyr Tyr Leu
       370                 375                 380
   gca ata agt atg caa atg act tat cca gtc ttc caa gcg ctc cag agc         1200
   Ala Ile Ser Met Gln Met Thr Tyr Pro Val Phe Gln Ala Leu Gln Ser
   385                 390                 395                 400
   gac agg aga tta ggt tca ggt ggc ttc aag att cca gcc gcc acc tat         1248
   Asp Arg Arg Leu Gly Ser Gly Gly Phe Lys Ile Pro Ala Ala Thr Tyr
                   405                 410                 415
   gtc gtg ttc ttg atg tct ggt atg acg gtt ttc atc ata atc tac gac         1296
   Val Val Phe Leu Met Ser Gly Met Thr Val Phe Ile Ile Ile Tyr Asp
               420                 425                 430
   cgt gtc ctc gtc ccc tcg ctc aaa aga gtg acg ggt cta gac acg ggg         1344
   Arg Val Leu Val Pro Ser Leu Lys Arg Val Thr Gly Leu Asp Thr Gly
           435                 440                 445
   ata aca ctc tta caa aga ata gga gct ggc att ttc ttt gcg gtg ttg         1392
   Ile Thr Leu Leu Gln Arg Ile Gly Ala Gly Ile Phe Phe Ala Val Leu
       450                 455                 460
   agt tta ttg gtc tcc ggg ttc ata gag gaa cgt aga aga acc ttt gcg         1440
   Ser Leu Leu Val Ser Gly Phe Ile Glu Glu Arg Arg Arg Thr Phe Ala
   465                 470                 475                 480
   ctg aca cga cct acg ctg ggg tcg gag cct cga aca gga gag atc tcc         1488
   Leu Thr Arg Pro Thr Leu Gly Ser Glu Pro Arg Thr Gly Glu Ile Ser
                   485                 490                 495
   tca atg tca gcg atg tgg ctg att ccg cag ctg gtg ctt gca ggg gta         1536
   Ser Met Ser Ala Met Trp Leu Ile Pro Gln Leu Val Leu Ala Gly Val
               500                 505                 510
   gca gaa gct ttt gca gcc att ggg caa atg gag ttt tac tac aag cag         1584
   Ala Glu Ala Phe Ala Ala Ile Gly Gln Met Glu Phe Tyr Tyr Lys Gln
           515                 520                 525
   ttt cct gaa aac atg aag agc ttt gcg ggt tct atc ttc tat gtc ggt         1632
   Phe Pro Glu Asn Met Lys Ser Phe Ala Gly Ser Ile Phe Tyr Val Gly
       530                 535                 540
   gca gga gtt tct agc tac ctc gct agc ttc ttg atc tcg act gtt cat         1680
   Ala Gly Val Ser Ser Tyr Leu Ala Ser Phe Leu Ile Ser Thr Val His
   545                 550                 555                 560
   aga aca act gag cat tca cct acg ggg aat tgg tta gct gag gat ctg         1728
   Arg Thr Thr Glu His Ser Pro Thr Gly Asn Trp Leu Ala Glu Asp Leu
                   565                 570                 575
   aac aaa gcg aag ctg gac tac ttc tat ttc atg ctc aca gga ctc atg         1776
   Asn Lys Ala Lys Leu Asp Tyr Phe Tyr Phe Met Leu Thr Gly Leu Met
               580                     585                     590
   ttc gtt aac ttt ctt tac ttc ttg tta atg gct aga tgg tat aga tac      1824
   Phe Val Asn Phe Leu Tyr Phe Leu Leu Met Ala Arg Trp Tyr Arg Tyr
           595                 600                     605
   aaa ggc act cat gat gaa gtc agt tat gtg gtt gac act gtt gac gaa      1872
   Lys Gly Thr His Asp Glu Val Ser Tyr Val Val Asp Thr Val Asp Glu
       610                     615                     620
   gag aca aag caa caa gac aag aac tct gtc tga                          1905
   Glu Thr Lys Gln Gln Asp Lys Asn Ser Val
   625                 630
<210> 148
   <211> 634
   <212> PRT
   <213> Brassica rapa
<400> 148
   Met Lys Ser Arg Val Ile His Ser His Arg Glu Arg Arg Asp Lys His
   1               5                   10                  15
   Asn Tyr Thr Gln Ile Gly Thr Met Glu Arg Lys Pro Phe Asp Val Glu
               20                  25                  30
   Thr Thr Glu Asp His Lys Pro Tyr Ser Ser Val Asp Asp Gly Gly Ser
           35                  40                  45
   Asp Ser Val Asp Ser Phe Glu Asn Glu Gln Lys Lys Leu Val Tyr Arg
       50                  55                  60
   Gly Trp Lys Val Met Pro Phe Ile Ile Gly Asn Glu Thr Phe Glu Lys
   65                  70                  75                  80
   Ile Gly Ile Ile Gly Thr Leu Ser Asn Leu Leu Val Tyr Leu Thr Ser
                   85                  90                  95
   Val Phe Asn Leu Lys Ser Val Thr Ala Ala Thr Ile Ile Asn Ala Phe
               100                 105                 110
   Ser Gly Thr Ile Asn Phe Gly Thr Phe Leu Ala Ala Phe Leu Cys Asp
           115                 120                 125
   Thr Tyr Phe Gly Arg Tyr Lys Thr Leu Ser Val Ala Val Ile Ala Cys
       130                 135                 140
   Phe Leu Gly Ser Leu Val Ile Leu Leu Thr Ala Ala Val Pro Gly Leu
   145                 150                 155                 160
   His Pro Ile Pro Cys Gly Thr Gln Ser Ser Cys Gln Gly Pro Ser Glu
                   165                 170                 175
   Gly Gln Ile Ala Phe Leu Leu Met Gly Leu Ala Leu Leu Val Val Gly
               180                 185                 190
   Ala Gly Gly Ile Arg Pro Cys Asn Leu Ala Phe Gly Ala Asp Gln Phe
           195                 200                 205
   Asn Pro Lys Ser Glu Ser Gly Lys Lys Gly Ile Asn Ser Phe Phe Asn
       210                 215                 220
   Trp Tyr Phe Phe Thr Phe Thr Phe Ala Gln Ile Ile Ser Leu Thr Leu
   225 230 235 240
   Val Val Tyr Ile Gln Ser Asn Val Ser Trp Thr Ile Gly Leu Ser Ile
   245 250 255
   Pro Val Gly Leu Met Phe Leu Ala Cys Val Ile Phe Phe Ala Gly His
   260 265 270
   Lys Leu Tyr Val Lys Val Lys Ala Ser Gly Ser Pro Leu Ala Gly Ile
   275 280 285
   Gly His Val Ile Ala Ala Ala Ile Lys Lys Arg Gly Leu Lys Pro Val
   290 295 300
   Lys Gln Pro Trp Ile Asp Leu Tyr Asn His Ile Pro His Asn Tyr Ala
   305 310 315 320
   Asn Ser Thr Leu Lys Tyr Thr Asp Gln Leu Arg Phe Leu Asp Lys Ala
   325 330 335
   Ala Ile Met Thr Pro Glu Asp Lys Leu Asn Ser Asp Gly Thr Ala Ser
   340 345 350
   Asp Pro Trp Lys Leu Cys Thr Met Gln Lys Val Glu Glu Val Lys Cys
   355 360 365
   Ile Leu Arg Val Ile Pro Ile Trp Phe Ala Cys Ala Ile Tyr Tyr Leu
   370 375 380
   Ala Ile Ser Met Gln Met Thr Tyr Pro Val Phe Gln Ala Leu Gln Ser
   385 390 395 400
   Asp Arg Arg Leu Gly Ser Gly Gly Phe Lys Ile Pro Ala Ala Thr Tyr
   405 410 415
   Val Val Phe Leu Met Ser Gly Met Thr Val Phe Ile Ile Ile Tyr Asp
   420 425 430
   Arg Val Leu Val Pro Ser Leu Lys Arg Val Thr Gly Leu Asp Thr Gly
   435 440 445
   Ile Thr Leu Leu Gln Arg Ile Gly Ala Gly Ile Phe Phe Ala Val Leu
   450 455 460
   Ser Leu Leu Val Ser Gly Phe Ile Glu Glu Arg Arg Arg Thr Phe Ala
   465 470 475 480
   Leu Thr Arg Pro Thr Leu Gly Ser Glu Pro Arg Thr Gly Glu Ile Ser
   485 490 495
   Ser Met Ser Ala Met Trp Leu Ile Pro Gln Leu Val Leu Ala Gly Val
   500 505 510
   Ala Glu Ala Phe Ala Ala Ile Gly Gln Met Glu Phe Tyr Tyr Lys Gln
   515 520 525
   Phe Pro Glu Asn Met Lys Ser Phe Ala Gly Ser Ile Phe Tyr Val Gly
       530                 535                 540
   Ala Gly Val Ser Ser Tyr Leu Ala Ser Phe Leu Ile Ser Thr Val His
   545                 550                 555                 560
   Arg Thr Thr Glu His Ser Pro Thr Gly Asn Trp Leu Ala Glu Asp Leu
                   565                 570                 575
   Asn Lys Ala Lys Leu Asp Tyr Phe Tyr Phe Met Leu Thr Gly Leu Met
               580                 585                 590
   Phe Val Asn Phe Leu Tyr Phe Leu Leu Met Ala Arg Trp Tyr Arg Tyr
           595                 600                 605
   Lys Gly Thr His Asp Glu Val Ser Tyr Val Val Asp Thr Val Asp Glu
       610                 615                 620
   Glu Thr Lys Gln Gln Asp Lys Asn Ser Val
   625                 630
<210> 149
   <211> 1905
   <212> DNA
   <213> Brassica oleracea
<220>
   <221> CDS
   <222> (1)..(1905)
<400> 149
   atg aag agc aga gtc gtc ctc agc cat aga gag aga aga gat aag cat      48
   Met Lys Ser Arg Val Val Leu Ser His Arg Glu Arg Arg Asp Lys His
   1               5                   10                  15
   aat tat aca cag att gaa aca atg gag aga aag ccc ttt gat gtt gag      96
   Asn Tyr Thr Gln Ile Glu Thr Met Glu Arg Lys Pro Phe Asp Val Glu
               20                  25                  30
   aca act gag gat cac aaa ccc tac tcc tcc gtc gat ggc ggt ggt tct     144
   Thr Thr Glu Asp His Lys Pro Tyr Ser Ser Val Asp Gly Gly Gly Ser
           35                  40                  45
   gat tcg gtc gat tca ttt gaa gac gag cag aaa aag ctc gtt tat aga     192
   Asp Ser Val Asp Ser Phe Glu Asp Glu Gln Lys Lys Leu Val Tyr Arg
       50                  55                  60
   ggc tgg aaa gtc atg ccc ttt atc att ggt aat gag aca ttt gag aag     240
   Gly Trp Lys Val Met Pro Phe Ile Ile Gly Asn Glu Thr Phe Glu Lys
   65                  70                  75                  80
   ata ggg atc att ggg aca cta tca aac ctt ctg gtg tac cta act tca     288
   Ile Gly Ile Ile Gly Thr Leu Ser Asn Leu Leu Val Tyr Leu Thr Ser
                   85                  90                  95
   gta ttc aac ctt cag agt gtt aca gct gca acc atc atc aac gcc ttc     336
   Val Phe Asn Leu Gln Ser Val Thr Ala Ala Thr Ile Ile Asn Ala Phe
               100                 105                 110
   agt ggc act atc aat ttc ggc act ttc ctc gct gct ttc ctc tgc gac     384
   Ser Gly Thr Ile Asn Phe Gly Thr Phe Leu Ala Ala Phe Leu Cys Asp
           115                 120                 125
   act tac ttt ggt cgc tac aag act ctc tct gtt gct gtc atc gcc tgt     432
   Thr Tyr Phe Gly Arg Tyr Lys Thr Leu Ser Val Ala Val Ile Ala Cys
       130                 135                 140
   ttt ctg gga tcg ctt gtg ata cta ctg acg gct gca gtt cca gga ttg      480
   Phe Leu Gly Ser Leu Val Ile Leu Leu Thr Ala Ala Val Pro Gly Leu
   145                 150                 155                 160
   cac cct att cct tgt gga aca caa agt tct tgc caa ggc cca agc gag      528
   His Pro Ile Pro Cys Gly Thr Gln Ser Ser Cys Gln Gly Pro Ser Glu
                   165                 170                 175
   ggc cag att gcg ttt ctg ctg atg ggt tta gcg ctt ctt gtg gtg ggt      576
   Gly Gln Ile Ala Phe Leu Leu Met Gly Leu Ala Leu Leu Val Val Gly
               180                 185                 190
   gct ggt gga atc agg ccg tgt aac ttg gcc ttt ggt gct gac cag ttc      624
   Ala Gly Gly Ile Arg Pro Cys Asn Leu Ala Phe Gly Ala Asp Gln Phe
           195                 200                 205
   aac ccc aag tca gaa tct gga aag aaa gga atc aac agt ttc ttc aac      672
   Asn Pro Lys Ser Glu Ser Gly Lys Lys Gly Ile Asn Ser Phe Phe Asn
       210                 215                 220
   tgg tac ttc ttc act ttc acg ttc gcg cag atc atc tcc ctc acg cta      720
   Trp Tyr Phe Phe Thr Phe Thr Phe Ala Gln Ile Ile Ser Leu Thr Leu
   225                 230                 235                 240
   gtt gtg tat atc cag tca aat gtg agc tgg acc atc ggt ttg agt atc      768
   Val Val Tyr Ile Gln Ser Asn Val Ser Trp Thr Ile Gly Leu Ser Ile
                   245                 250                 255
   cct gtg ggt ctt atg ttc ttg gcc tgc gtc att ttc ttt gct ggt cac      816
   Pro Val Gly Leu Met Phe Leu Ala Cys Val Ile Phe Phe Ala Gly His
               260                 265                 270
   aaa ctg tat gtg aaa gtg aaa gcc tct ggt agt cca ttg gct ggt ata      864
   Lys Leu Tyr Val Lys Val Lys Ala Ser Gly Ser Pro Leu Ala Gly Ile
           275                 280                 285
   ggt cat gtt ata gcc gca gct atc aag aaa cgt ggc ttg aag cca gtt      912
   Gly His Val Ile Ala Ala Ala Ile Lys Lys Arg Gly Leu Lys Pro Val
       290                 295                 300
   aag cag cct tgg atc gat ctt tac aac cac att cct cct aac tat gca      960
   Lys Gln Pro Trp Ile Asp Leu Tyr Asn His Ile Pro Pro Asn Tyr Ala
   305                 310                 315                 320
   aac tct act ctt aaa tac acc gac cag ctt agg ttt cta gac aaa gca     1008
   Asn Ser Thr Leu Lys Tyr Thr Asp Gln Leu Arg Phe Leu Asp Lys Ala
                   325                 330                 335
   gcg att atg acc cct gag gac aag ttg aac tcg gat gga acg gct tcg     1056
   Ala Ile Met Thr Pro Glu Asp Lys Leu Asn Ser Asp Gly Thr Ala Ser
               340                 345                 350
   gat cca tgg aaa tta tgt acg atg cag aaa gtg gaa gaa gtg aaa tgc     1104
   Asp Pro Trp Lys Leu Cys Thr Met Gln Lys Val Glu Glu Val Lys Cys
           355                 360                 365
   att tta aga gtg att cca atc tgg ttc gca tgc gcc att tac tac ctt     1152
   Ile Leu Arg Val Ile Pro Ile Trp Phe Ala Cys Ala Ile Tyr Tyr Leu
       370                 375                 380
   gca ata agt atg caa atg act tat cca gtc ttc caa gct ctc cag agc     1200
   Ala Ile Ser Met Gln Met Thr Tyr Pro Val Phe Gln Ala Leu Gln Ser
   385                 390                 395                 400
   gac agg cta tta ggt tca ggt ggc ttc aag att cca gcc gcc acc tat     1248
   Asp Arg Leu Leu Gly Ser Gly Gly Phe Lys Ile Pro Ala Ala Thr Tyr
                   405                 410                 415
   gtc gtg ttc ttg atg tct ggt atg acg gtt ttc atc ata atc tac gac     1296
   Val Val Phe Leu Met Ser Gly Met Thr Val Phe Ile Ile Ile Tyr Asp
               420                 425                 430
   cgt gtc ctt gtc ccc tcg ctc aaa aga gtg acc ggt cta gac acg ggg     1344
   Arg Val Leu Val Pro Ser Leu Lys Arg Val Thr Gly Leu Asp Thr Gly
           435                 440                 445
   ata aca ctc tta caa aga ata gga gct ggc att ttc ttt gcc gtg ttg        1392
   Ile Thr Leu Leu Gln Arg Ile Gly Ala Gly Ile Phe Phe Ala Val Leu
       450                 455                 460
   agt tta ttg gtc tcc ggg ttt ata gag gaa cgt aga aga acc ttt gcg        1440
   Ser Leu Leu Val Ser Gly Phe Ile Glu Glu Arg Arg Arg Thr Phe Ala
   465                 470                 475                 480
   ctg aca cga cct acg ctc ggg tcg gag cct cga aca gga gag atc tcc        1488
   Leu Thr Arg Pro Thr Leu Gly Ser Glu Pro Arg Thr Gly Glu Ile Ser
                   485                 490                 495
   tca atg tca gcg atg tgg ctg att ccg cag ctg gtg ctt gca ggg gta        1536
   Ser Met Ser Ala Met Trp Leu Ile Pro Gln Leu Val Leu Ala Gly Val
               500                 505                 510
   gca gaa gct ttt gca gcc att ggg caa atg gag ttt tac tac aag cag        1584
   Ala Glu Ala Phe Ala Ala Ile Gly Gln Met Glu Phe Tyr Tyr Lys Gln
           515                 520                 525
   ttt cct gaa aac atg aag agc ttt gcg ggt tct atc ttc tat gtc ggt        1632
   Phe Pro Glu Asn Met Lys Ser Phe Ala Gly Ser Ile Phe Tyr Val Gly
       530                 535                 540
   gga gga gtt tct agc tac ctc gct agc ttc ttg atc tcg act gtt cat        1680
   Gly Gly Val Ser Ser Tyr Leu Ala Ser Phe Leu Ile Ser Thr Val His
   545                 550                 555                 560
   aga aca act gag cat tca cct acg ggg aat tgg tta gct gag gat ctg        1728
   Arg Thr Thr Glu His Ser Pro Thr Gly Asn Trp Leu Ala Glu Asp Leu
                   565                 570                 575
   aac aaa gcg aag ctg gac tac ttc tat ttc atg ctc aca gga ctc atg        1776
   Asn Lys Ala Lys Leu Asp Tyr Phe Tyr Phe Met Leu Thr Gly Leu Met
               580                 585                 590
   ttt gtc aac ttt ctt tac ttc ttg tta atg gct aga tgg tat aga tac        1824
   Phe Val Asn Phe Leu Tyr Phe Leu Leu Met Ala Arg Trp Tyr Arg Tyr
           595                 600                 605
   aaa ggc act cat gat gaa gtc agt tat gtg gtt gac act gtt gac gaa        1872
   Lys Gly Thr His Asp Glu Val Ser Tyr Val Val Asp Thr Val Asp Glu
       610                 615                 620
   gag acg aag caa caa gac aag aac tct gtc tga                            1905
   Glu Thr Lys Gln Gln Asp Lys Asn Ser Val
   625                 630
<210> 150
   <211> 634
   <212> PRT
   <213> Brassica oleracea
<400> 150
   Met Lys Ser Arg Val Val Leu Ser His Arg Glu Arg Arg Asp Lys His
   1               5                   10                  15
   Asn Tyr Thr Gln Ile Glu Thr Met Glu Arg Lys Pro Phe Asp Val Glu
               20                  25                  30
   Thr Thr Glu Asp His Lys Pro Tyr Ser Ser Val Asp Gly Gly Gly Ser
           35                  40                  45
   Asp Ser Val Asp Ser Phe Glu Asp Glu Gln Lys Lys Leu Val Tyr Arg
       50                  55                  60
   Gly Trp Lys Val Met Pro Phe Ile Ile Gly Asn Glu Thr Phe Glu Lys
   65                  70                  75                  80
   Ile Gly Ile Ile Gly Thr Leu Ser Asn Leu Leu Val Tyr Leu Thr Ser
   85 90 95
   Val Phe Asn Leu Gln Ser Val Thr Ala Ala Thr Ile Ile Asn Ala Phe
   100 105 110
   Ser Gly Thr Ile Asn Phe Gly Thr Phe Leu Ala Ala Phe Leu Cys Asp
   115 120 125
   Thr Tyr Phe Gly Arg Tyr Lys Thr Leu Ser Val Ala Val Ile Ala Cys
   130 135 140
   Phe Leu Gly Ser Leu Val Ile Leu Leu Thr Ala Ala Val Pro Gly Leu
   145 150 155 160
   His Pro Ile Pro Cys Gly Thr Gln Ser Ser Cys Gln Gly Pro Ser Glu
   165 170 175
   Gly Gln Ile Ala Phe Leu Leu Met Gly Leu Ala Leu Leu Val Val Gly
   180 185 190
   Ala Gly Gly Ile Arg Pro Cys Asn Leu Ala Phe Gly Ala Asp Gln Phe
   195 200 205
   Asn Pro Lys Ser Glu Ser Gly Lys Lys Gly Ile Asn Ser Phe Phe Asn
   210 215 220
   Trp Tyr Phe Phe Thr Phe Thr Phe Ala Gln Ile Ile Ser Leu Thr Leu
   225 230 235 240
   Val Val Tyr Ile Gln Ser Asn Val Ser Trp Thr Ile Gly Leu Ser Ile
   245 250 255
   Pro Val Gly Leu Met Phe Leu Ala Cys Val Ile Phe Phe Ala Gly His
   260 265 270
   Lys Leu Tyr Val Lys Val Lys Ala Ser Gly Ser Pro Leu Ala Gly Ile
   275 280 285
   Gly His Val Ile Ala Ala Ala Ile Lys Lys Arg Gly Leu Lys Pro Val
   290 295 300
   Lys Gln Pro Trp Ile Asp Leu Tyr Asn His Ile Pro Pro Asn Tyr Ala
   305 310 315 320
   Asn Ser Thr Leu Lys Tyr Thr Asp Gln Leu Arg Phe Leu Asp Lys Ala
   325 330 335
   Ala Ile Met Thr Pro Glu Asp Lys Leu Asn Ser Asp Gly Thr Ala Ser
   340 345 350
   Asp Pro Trp Lys Leu Cys Thr Met Gln Lys Val Glu Glu Val Lys Cys
   355 360 365
   Ile Leu Arg Val Ile Pro Ile Trp Phe Ala Cys Ala Ile Tyr Tyr Leu
   370 375 380
   Ala Ile Ser Met Gln Met Thr Tyr Pro Val Phe Gln Ala Leu Gln Ser
   385                 390                 395                 400
   Asp Arg Leu Leu Gly Ser Gly Gly Phe Lys Ile Pro Ala Ala Thr Tyr
                   405                 410                 415
   Val Val Phe Leu Met Ser Gly Met Thr Val Phe Ile Ile Ile Tyr Asp
               420                 425                 430
   Arg Val Leu Val Pro Ser Leu Lys Arg Val Thr Gly Leu Asp Thr Gly
           435                 440                 445
   Ile Thr Leu Leu Gln Arg Ile Gly Ala Gly Ile Phe Phe Ala Val Leu
       450                 455                 460
   Ser Leu Leu Val Ser Gly Phe Ile Glu Glu Arg Arg Arg Thr Phe Ala
   465                 470                 475                 480
   Leu Thr Arg Pro Thr Leu Gly Ser Glu Pro Arg Thr Gly Glu Ile Ser
                   485                 490                 495
   Ser Met Ser Ala Met Trp Leu Ile Pro Gln Leu Val Leu Ala Gly Val
               500                 505                 510
   Ala Glu Ala Phe Ala Ala Ile Gly Gln Met Glu Phe Tyr Tyr Lys Gln
           515                 520                 525
   Phe Pro Glu Asn Met Lys Ser Phe Ala Gly Ser Ile Phe Tyr Val Gly
       530                 535                 540
   Gly Gly Val Ser Ser Tyr Leu Ala Ser Phe Leu Ile Ser Thr Val His
   545                 550                 555                 560
   Arg Thr Thr Glu His Ser Pro Thr Gly Asn Trp Leu Ala Glu Asp Leu
                   565                 570                 575
   Asn Lys Ala Lys Leu Asp Tyr Phe Tyr Phe Met Leu Thr Gly Leu Met
               580                 585                 590
   Phe Val Asn Phe Leu Tyr Phe Leu Leu Met Ala Arg Trp Tyr Arg Tyr
           595                 600                 605
   Lys Gly Thr His Asp Glu Val Ser Tyr Val Val Asp Thr Val Asp Glu
       610                 615                 620
   Glu Thr Lys Gln Gln Asp Lys Asn Ser Val
   625                 630